(19) Europäisches Patentamt
European Patent Office
Office européen des brevets

(11) EP 3 392 249 B1

(12) EUROPEAN PATENT SPECIFICATION

(45) Date of publication and mention of the grant of the patent:
**25.08.2021 Bulletin 2021/34**

(51) Int Cl.:
*C07D 471/08* (2006.01) *C07D 487/08* (2006.01)
*A61K 31/529* (2006.01) *A61P 7/00* (2006.01)

(21) Application number: **18174593.6**

(22) Date of filing: **30.01.2015**

# (54) MACROCYCLES WITH HETEROCYCLIC P2' GROUPS AS FACTOR XIA INHIBITORS

MAKROZYKLEN MIT HETEROCYCLISCHEN P2-GRUPPEN ALS FAKTOR-XIA-HEMMER

MACROCYCLES A GROUPES HETEROCYCLIQUES P2' SERVANT D'INHIBITEURS DU FACTEUR XIA

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2014 US 201461933942 P**
**01.10.2014 US 201462058293 P**

(43) Date of publication of application:
**24.10.2018 Bulletin 2018/43**

(62) Document number(s) of the earlier application(s) in accordance with Art. 76 EPC:
**15705860.3 / 3 099 687**

(73) Proprietor: **Bristol-Myers Squibb Company**
**Princeton, NJ 08543 (US)**

(72) Inventors:
- **CORTE, James R.**
  **Pennington, New Jersey 08534 (US)**
- **DE LUCCA, Indawati**
  **Pennington, New Jersey 08534 (US)**
- **FANG, Tianan**
  **Pennington, New Jersey 08534 (US)**
- **YANG, Wu**
  **Pennington, New Jersey 08534 (US)**
- **WANG, Yufeng**
  **Pennington, New Jersey 08534 (US)**
- **DILGER, Andrew K.**
  **Pennington, New Jersey 08534 (US)**
- **PABBISETTY, Kumar Balashanmuga**
  **Pennington, New Jersey 08534 (US)**
- **EWING, William R.**
  **Pennington, New Jersey 08534 (US)**
- **ZHU, Yeheng**
  **Pennington, New Jersey 08534 (US)**
- **WEXLER, Ruth R.**
  **Pennington, New Jersey 08534 (US)**
- **PINTO, Donald J. P.**
  **Pennington, New Jersey 08534 (US)**
- **ORWAT, Michael J.**
  **Pennington, New Jersey 08534 (US)**
- **SMITH, Leon M. II**
  **Pennington, New Jersey 08534 (US)**

(74) Representative: **Dehns**
**St. Bride's House**
**10 Salisbury Square**
**London EC4Y 8JD (GB)**

(56) References cited:
**WO-A1-2013/022814** **WO-A1-2013/093484**
**WO-A1-2014/022766**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 392 249 B1

## Description

FIELD OF THE INVENTION

The present invention relates generally to novel macrocyclic compounds, which are factor XIa inhibitors or dual inhibitors of factor XIa and plasma kallikrein, compositions containing them, and methods of using them, for example, for the treatment or prophylaxis of thromboembolic disorders, or for the treatment of retinal vascular permeability associated with diabetic retinopathy and diabetic macular edema.

BACKGROUND OF THE INVENTION

Thromboembolic diseases remain the leading cause of death in developed countries despite the availability of anticoagulants such as warfarin (COUMADIN®), heparin, low molecular weight heparins (LMWH), and synthetic pentasaccharides and antiplatelet agents such as aspirin and clopidogrel (PLAVIX®). The oral anticoagulant warfarin, inhibits the post-translational maturation of coagulation factors VII, IX, X and prothrombin, and has proven effective in both venous and arterial thrombosis. However, its usage is limited due to its narrow therapeutic index, slow onset of therapeutic effect, numerous dietary and drug interactions, and a need for monitoring and dose adjustment. Thus discovering and developing safe and efficacious oral anticoagulants for the prevention and treatment of a wide range of thromboembolic disorders has become increasingly important.

One approach is to inhibit thrombin generation by targeting the inhibition of coagulation factor XIa (FXIa). Factor XIa is a plasma serine protease involved in the regulation of blood coagulation, which is initiated *in vivo* by the binding of tissue factor (TF) to factor VII (FVII) to generate factor VIIa (FVIIa). The resulting TF:FVIIa complex activates factor IX (FIX) and factor X (FX) that leads to the production of factor Xa (FXa). The generated FXa catalyzes the transformation of prothrombin into small amounts of thrombin before this pathway is shut down by tissue factor pathway inhibitor (TFPI). The process of coagulation is then further propagated via the feedback activation of Factors V, VIII and XI by catalytic amounts of thrombin. (Gailani, D. et al., Arterioscler. Thromb. Vasc. Biol., 27:2507-2513 (2007).) The resulting burst of thrombin converts fibrinogen to fibrin that polymerizes to form the structural framework of a blood clot, and activates platelets, which are a key cellular component of coagulation (Hoffman, M., Blood Reviews, 17:S1-S5 (2003)). Therefore, factor XIa plays a key role in propagating this amplification loop and is thus an attractive target for anti-thrombotic therapy.

An alternative way of initiation of coagulation is operative when blood is exposed to artificial surfaces. This process is also termed contact activation. Surface absorption of factor XII leads to a conformational change in the factor XII molecule, thereby facilitating activation to proteolytic active factor XII molecules (factor XIIa and factor XIIf). Factor XIIa (or XIIf) has a number of target proteins, including plasma prekallikrein and factor XI.

Plasma prekallikrein is a zymogen of a trypsin-like serine protease and is present in plasma at 35 to 50 $\mu$g/mL. The gene structure is similar to that of factor XI. Overall, the amino acid sequence of plasma kallikrein has 58% homology to factor XI. Plasma kallikrein is thought to play a role in a number of inflammatory disorders. The major inhibitor of plasma kallikrein is the serpin C1 esterase inhibitor. Patients who present with a genetic deficiency in C1 esterase inhibitor suffer from hereditary angioedema (HAE) which results in intermittent swelling of face, hands, throat, gastrointestinal tract and genitals. Blisters formed during acute episodes contain high levels of plasma kallikrein which cleaves high molecular weight kininogen liberating bradykinin leading to increased vascular permeability. Treatment with a large protein plasma kallikrein inhibitor has been shown to effectively treat HAE by preventing the release of bradykinin which causes increased vascular permeability (Lehmann, A., "Ecallantide (DX-88), a plasma kallikrein inhibitor for the treatment of hereditary angioedema and the prevention of blood loss in on-pump cardiothoracic surgery", Expert Opin. Biol. Ther., 8:1187-1199 (2008)).

The plasma kallikrein-kinin system is abnormally abundant in patients with advanced diabetic macular edema. It has been recently published that plasma kallikrein contributes to retinal vascular dysfunctions in diabetic rats (Clermont, A. et al., "Plasma kallikrein mediates retinal vascular dysfunction and induces retinal thickening in diabetic rats", Diabetes, 60:1590-1598 (2011)). Furthermore, administration of the plasma kallikrein inhibitor ASP-440 ameliorated both retinal vascular permeability and retinal blood flow abnormalities in diabetic rats. Therefore, a plasma kallikrein inhibitor should have utility as a treatment to reduce retinal vascular permeability associated with diabetic retinopathy and diabetic macular edema. Other complications of diabetes such as cerebral hemorrhage, nephropathy, cardiomyopathy and neuropathy, all of which have associations with plasma kallikrein may also be considered as targets for a plasma kallikrein inhibitor.

To date, no small molecule synthetic plasma kallikrein inhibitor has been approved for medical use. The large protein plasma kallikrein inhibitors present risks of anaphylactic reactions, as has been reported for Ecallantide. Thus there remains a need for compounds that inhibit plasma kallikrein, that do not induce anaphylaxis and that are orally available. Furthermore, the molecules in the known art feature a highly polar and ionizable guanidine or amidine functionality. It is well known that such functionalities may be limiting to gut permeability and therefore to oral availability.

SUMMARY OF THE INVENTION

The present invention provides novel macrocyclic compounds, including stereoisomers, tautomers, pharmaceutically acceptable salts, or solvates thereof, which are useful as selective factor XIa inhibitors or dual inhibitors of factor XIa and plasma kallikrein.

Also disclosed herein are processes and intermediates for making the compounds of the present invention.

The present invention also provides pharmaceutical compositions comprising a pharmaceutically acceptable carrier or diluent and at least one of the compounds of the present invention or stereoisomers, tautomers, pharmaceutically acceptable salts, or solvates thereof.

The compounds of the invention may be used in the treatment and/or prophylaxis of thromboembolic disorders.

The compounds of the invention may be used in the treatment of retinal vascular permeability associated with diabetic retinopathy and diabetic macular edema.

The compounds of the present invention may be used in therapy.

The compounds of the present invention may be used for the manufacture of a medicament for the treatment and/or prophylaxis of a thromboembolic disorder.

The compounds of the invention can be used alone, in combination with other compounds of the present invention, or in combination with one or more, preferably one to two other agent(s).

These and other features of the invention will be set forth in expanded form as the disclosure continues.

DETAILED DESCRIPTION OF THE INVENTION

I. COMPOUNDS OF THE INVENTION

In one aspect, the present invention provides compounds of Formula (IIb):

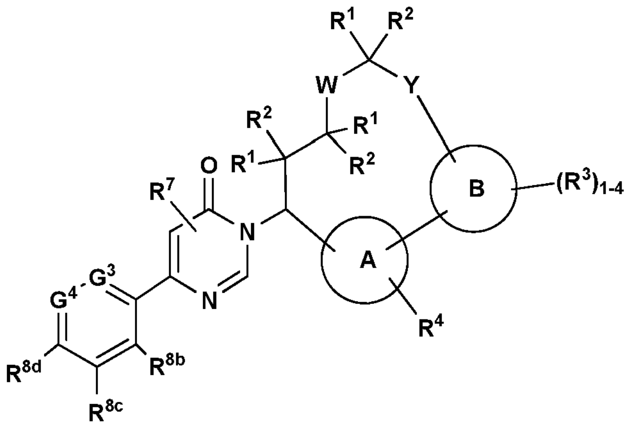

(IIb)

or stereoisomers, tautomers, pharmaceutically acceptable salts, or solvates thereof, wherein:

ring A is independently selected from phenyl and 5- to 6-membered heterocyclyl;
ring B is 5- to 6-membered heteroaryl comprising carbon atoms and 1-4 heteroatoms selected from N and $NR^{3c}$;
W is independently selected from $(CR^1R^2)_{1-2}$, O, NH, and $N(C_{1-4}$ alkyl);
Y is independently selected from $-CH_2NH-$, -NHC(=O)- and -C(=O)NH-;
$G^3$ is $CR^{8a}$;
$G^4$ is $CR^{8e}$;
$R^1$ and $R^2$ are independently selected from H, D, halogen, $CF_3$, $C_{1-6}$ alkyl, and hydroxyl;
$R^3$ is independently selected from H, halogen, $C_{1-4}$alkyl (optionally substituted with $R^6$), CN, $-(CH_2)_n-OR^5$, $-(CH_2)_n-C(=O)R^5$, and $-(CH_2)_n-C(=O)OR^5$;
$R^{3c}$ is independently selected from H, haloalkyl, $C_{1-4}$ alkyl (optionally substituted with $R^6$), $-(CH_2)_{1-2}-OH$, $C(=O)C_{1-4}$ alkyl, $-(CH_2)_{1-2}-C(=O)OH$, $-C(=O)OC_{1-4}$ alkyl, $S(=O)_pC_{1-6}$ alkyl, $-(CH_2)_n-C_{3-10}$ carbocyclyl and $-(CH_2)_n$-4- to 10-membered heterocyclyl, wherein said carbocyclyl and heterocyclyl are optionally substituted with $R^6$;
$R^4$ is independently selected from H, OH, F, Cl, Br, $C_{1-4}$ alkyl, $C_{1-4}$ alkoxy, $CF_3$, CN, $C(=O)NH_2$, $C_{3-6}$ cycloalkyl, aryl, and 5- to 6-membered heterocyclyl, wherein said cycloalkyl, aryl and heterocyclyl are optionally substituted with $R^6$;
$R^5$ is independently selected from H and $C_{1-4}$ alkyl;

$R^6$ is independently selected from H, $-(CH_2)_n$-OH, =O, $NH_2$, $-(CH_2)_n$-CN, halogen, $C_{1-6}$ alkyl, $-(CH_2)_n$-C(=O)OH, $-(CH_2)_n$-C(=O)O$C_{1-4}$ alkyl, $-(CH_2)_n$-O$C_{1-4}$ alkyl, $-(CH_2)_n$-$C_{3-6}$ cycloalkyl, $-(CH_2)_n$-4- to 10-membered heterocyclyl, and -O-$(CH_2)_n$-4- to 10-membered heterocyclyl, wherein said cycloalkyl and heterocyclyl are optionally substituted with $R^{10}$;

$R^7$ is independently selected from H, F, Cl, Br, and methyl;

$R^{8a}$ is independently selected from H, F, Cl, Br, I, $-(CH_2)_n$CN, $-(CH_2)_nNH_2$, $CH_3CHF_2$, $CCH_3F_2$, $CF_3$, OH, $OCH_3$, $OCF_3$, $OCHF_2$, C(=O)$CH_3$, C(=O)OH, C(=O)$OCH_3$, C(=O)$NH_2$, C(=O)NH$CH_2CF_3$, C(=O)NH$CH_2$Ph, NHC(=O)$OCH_3$, NHC(=O)$CF_3$,

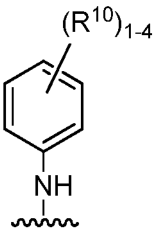 , 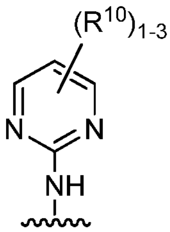 , 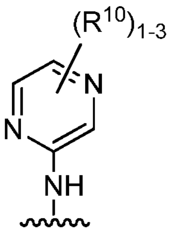 , 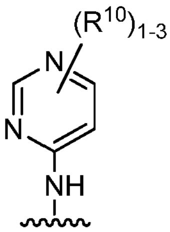 , 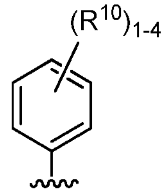 ,

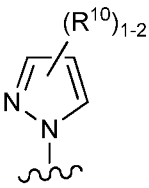 , 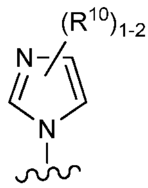 , 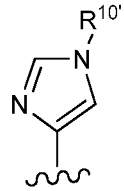 , 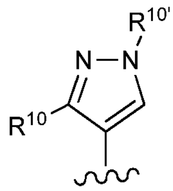 , 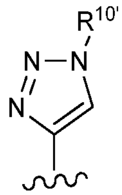 , 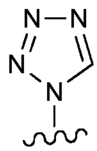 , 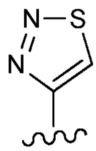 ,

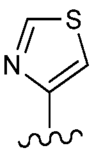 , 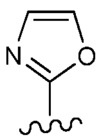 , 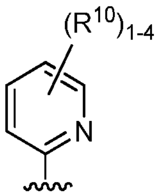 , 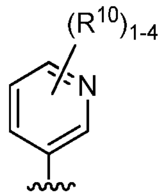 , 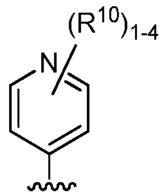 , 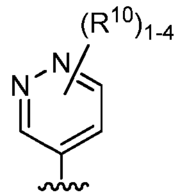 , 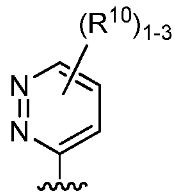 ,

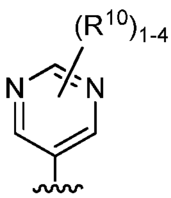 , 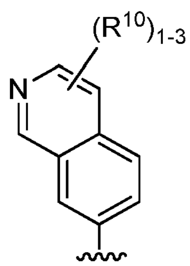 , 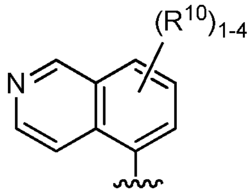 , and 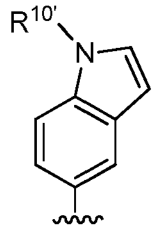 ;

$R^{8b}$ is independently selected from H and F;

$R^{8c}$ is independently selected from H, F, Cl, methyl, ethyl, isopropyl, $OCHF_2$ and $OCH_3$;

$R^{8d}$ is independently selected from H, F, and Cl;

$R^{8e}$ is independently selected from H, F, and Cl;

$R^{10}$ is independently selected from H, $C_{1-6}$ alkyl (optionally substituted with $R^{11}$), $C_{2-6}$ alkenyl, $C_{2-6}$ alkynyl, aryl (optionally substituted with $R^{11}$), $-(CH_2)_n$-$C_{3-6}$ cycloalkyl (optionally substituted with $R^{11}$), $-(CH_2)_n$-O-4- to 10-membered heterocyclyl (optionally substituted with $R^{11}$), F, Cl, Br, CN, $NO_2$, =O, CON$R^{12}R^{12}$, $-(CH_2)_n$-C(=O)O$R^{12}$, Si($C_{1-4}$alkyl)$_3$, $-(CH_2)_n$-O$R^{12}$, $-(CH_2)_n$-N$R^{12}R^{12}$, -S(=O)$_p$$C_{1-6}$ alkyl, N$R^{12}$S(=O)$_p$$C_{1-6}$ alkyl, and S(=O)$_p$N$R^{12}R^{12}$;

$R^{10'}$ is independently selected from H, $C_{1-6}$ alkyl (optionally substituted with $R^{11}$), aryl, $-(CH_2)_n$-$C_{3-6}$ cycloalkyl (optionally substituted with $R^{11}$), and $-(CH_2)_n$-O-4- to 10-membered heterocyclyl (optionally substituted with $R^{11}$);

$R^{11}$, at each occurrence, is independently selected from H, halogen, $C_{1-5}$ alkyl, $-(CH_2)_n$-OH, $C_{3-6}$ cycloalkyl, and phenyl;

$R^{12}$, at each occurrence, is independently selected from H, $C_{1-5}$ alkyl optionally substituted with $R^{11}$, $C_{3-6}$ cycloalkyl, phenyl, and heterocyclyl, or $R^{12}$ and $R^{12}$ together with the nitrogen atom to which they are both attached form a

heterocyclic ring optionally substituted with $C_{1-4}$alkyl;
n, at each occurrence, is an integer independently selected from 0, 1, and 2; and
p, at each occurrence, is an integer independently selected from 0, 1, and 2.

In another aspect, the present invention provides compounds of Formula (IIIb):

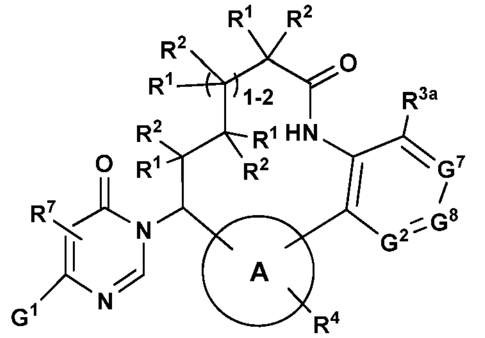

(IIIb)

or stereoisomers, tautomers, pharmaceutically acceptable salts, or solvates thereof, wherein:

ring A is independently selected from phenyl and 5- to 6-membered heterocyclyl;
$G^1$ is independently selected from aryl, $C_{3-6}$cycloalkyl and 5- to 6-membered heterocyclyl, wherein said aryl, cycloalkyl and heterocyclyl are substituted with 1-4 $R^8$;
$G^2$ is independently selected from N and $CR^{3b}$;
$G^7$ is independently selected from N and $CR^3$;
$G^8$ is independently selected from N and $CR^3$;
provided at least one of $G^2$, $G^7$ and $G^8$ is N;
$R^1$ and $R^2$ are independently selected from H, halogen, $CF_3$, $C_{1-6}$ alkyl, and hydroxyl;
$R^3$ is independently selected from H, halogen, haloalkyl, $C_{1-4}$alkyl (optionally substituted with $R^6$), $C_{2-4}$alkenyl (optionally substituted with $R^6$), CN, $NO_2$, $-(CH_2)_n-OR^5$, $-(CH_2)_n-NR^5R^5$, $-(CH_2)_n-C(=O)OR^5$, $-(CH_2)_n-NHC(=O)OR^5$, $-(CH_2)_n-NHC(=O)R^5$, $-(CH_2)_n-NHC(N-CN)NHR^5$, $-(CH_2)_n-NHC(NH)NHR^5$, $-(CH_2)_n-N=CHNR^5R^5$, $-(CH_2)_n-NHC(=O)NR^5R^5$, $-(CH_2)_n-C(=O)NR^5R^5$, $-(CH_2)_n-NHC(S)NR^9C(=O)R^5$, $-(CH_2)_n-S(=O)_pC_{1-6}$ alkyl optionally substituted with $R^{11}$, $-(CH_2)_n-S(=O)_pNR^5R^5$, $-(CH_2)_n-NHS(=O)_pNR^5R^5$, $-(CH_2)_n-NHS(=O)_pC_{1-6}$ alkyl optionally substituted with $R^{11}$, $-(CH_2)_n-C_{3-10}$ carbocyclyl and $-(CH_2)_n$-4- to 10-membered heterocyclyl, wherein said carbocyclyl and heterocyclyl are optionally substituted with $R^6$; optionally, two adjacent $R^3$ groups on the carbocyclyl and heterocyclyl may form a ring optionally substituted with $R^6$;
$R^{3a}$ is independently selected from H and halogen;
$R^{3b}$ is independently selected from H, halogen, methyl, and CN;
$R^4$ is independently selected from H, OH, F, Cl, Br, $C_{1-4}$ alkyl, $C_{1-4}$ alkoxy, $CF_3$, CN, $C_{3-6}$ cycloalkyl, aryl, and 5- to 6-membered heterocyclyl, wherein said cycloalkyl, aryl and heterocyclyl are optionally substituted with $R^6$;
$R^5$ is independently selected from H, $C_{1-4}$ alkyl (optionally substituted with halogen, hydroxyl, alkoxy, carboxy, alkoxycarbonyl, amino, substituted amino), $-(CH_2)_n-C_{3-10}$ carbocyclyl and $-(CH_2)_n$-4- to 10-membered heterocyclyl, wherein said carbocyclyl and heterocyclyl are optionally substituted with $R^6$;
$R^6$ is independently selected from $-(CH_2)_n-OH$, =O, $NH_2$, $-(CH_2)_n-CN$, halogen, $C_{1-6}$ alkyl, $-(CH_2)_n-C(=O)OH$, $-(CH_2)_n-C(=O)OC_{1-4}$ alkyl, $-(CH_2)_n-OC_{1-4}$ alkyl, $-(CH_2)_n-C_{3-6}$ cycloalkyl, $-(CH_2)_n$-4- to 10-membered heterocyclyl, and $-O-(CH_2)_n$-4- to 10-membered heterocyclyl, wherein said cycloalkyl and heterocyclyl are optionally substituted with $R^{10}$;
$R^7$ is independently selected from H, F, Cl, and methyl;
$R^8$ is independently selected from H, halogen, CN, $NH_2$, $C_{1-6}$ alkyl, haloalkyl, alkylcarbonyl, alkoxy, haloalkoxy, aryl, $C_{3-6}$ cycloalkyl, and 4- to 12-membered heterocyclyl, wherein said aryl, cycloalkyl, and heterocyclyl are optionally substituted with $R^{10}$;
$R^9$ is H or $C_{1-6}$ alkyl;
$R^{10}$ is independently selected from H, $C_{1-6}$ alkyl (optionally substituted with $R^{11}$), $C_{2-6}$ alkenyl, $C_{2-6}$ alkynyl, aryl, $-(CH_2)_n-C_{3-6}$ cycloalkyl (optionally substituted with $R^{11}$), $-(CH_2)_n$-O-4- to 10-membered heterocyclyl (optionally substituted with $R^{11}$), F, Cl, Br, CN, $NO_2$, =O, $C(=O)NR^{12}R^{12}$, $C(=O)OR^{12}$, $Si(C_{1-4}\ alkyl)_3$, $-(CH_2)_n-OR^{12}$, $-(CH_2)_n-NR^{12}R^{12}$, $-S(=O)_pC_{1-6}$ alkyl, $NR^{12}S(=O)_pC_{1-6}$ alkyl, and $S(=O)_pNR^{12}R^{12}$;
$R^{11}$, at each occurrence, is independently selected from H, halogen, $C_{1-5}$ alkyl, $-(CH_2)_n-OH$, $C_{3-6}$ cycloalkyl, and

phenyl;

$R^{12}$, at each occurrence, is independently selected from H, $C_{1-5}$ alkyl, $C_{3-6}$ cycloalkyl, phenyl, and heterocyclyl, or $R^{12}$ and $R^{12}$ together with the nitrogen atom to which they are both attached form a heterocyclic ring optionally substituted with $C_{1-4}$alkyl;

n, at each occurrence, is an integer independently selected from 0, 1, and 2; and

p, at each occurrence, is an integer independently selected from 0, 1, and 2.

In another aspect, the present invention provides compounds of Formula (IVa):

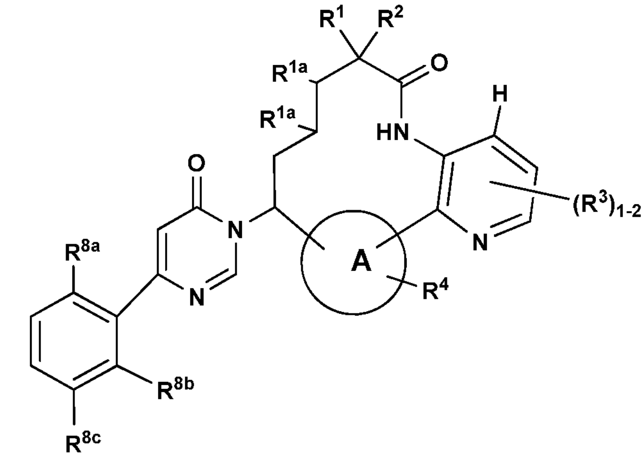

(IVa)

or stereoisomers, tautomers, pharmaceutically acceptable salts, or solvates thereof, wherein:

ring A is independently selected from

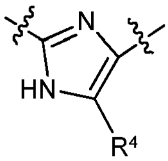 , 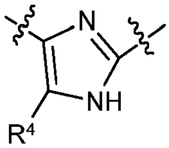 , 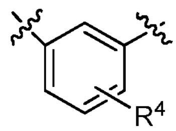 ,

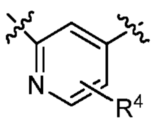 , 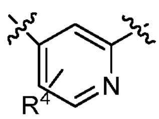 , 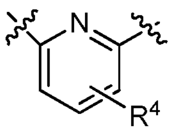 , 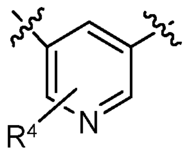 , 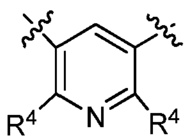 , 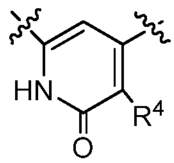 ,

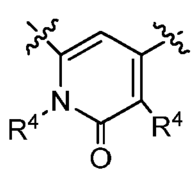 , 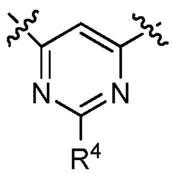 , 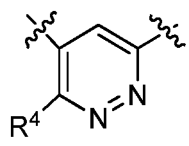 , and 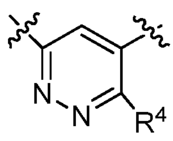 ;

$R^1$ and $R^2$ are independently selected from H, F, $C_{1-4}$ alkyl, and OH;

$R^{1a}$, at each occurrence, is independently selected from H, F, $CH_3$, and OH;

$R^3$ is independently selected from H, F, Cl, Br, I, $C_{2-4}$ alkenyl (optionally substituted with C(=O)OH), CN, and $-(CH_2)_n-OH$;

$R^4$ is independently selected from H, OH, F, $OC_{1-4}$ alkyl, $C_{1-4}$ alkyl, CN, $C_{3-6}$ cycloalkyl, aryl, and 5- to 6-membered heterocyclyl, wherein said cycloalkyl, aryl and heterocyclyl are optionally substituted with $R^6$;

$R^6$ is independently selected from OH, $NH_2$, halogen, $C_{1-6}$ alkyl, $C_{3-6}$ cycloalkyl, $-(CH_2)_n-C(=O)OH$,

$-(CH_2)_n-C(=O)OC_{1-4}$ alkyl, $-(CH_2)_n-OC_{1-4}$ alkyl, =O, $C_{3-6}$ cycloalkyl, 4- to 10-membered heterocyclyl, and -O-4- to 10-membered heterocyclyl, wherein said cycloalkyl and heterocyclyl are optionally substituted with $R^{10}$;
$R^{8a}$ is independently selected from H, F, Cl, Br, CN, $OCH_3$, $OCF_3$, $CH_3$, $C(=O)CH_3$, $CF_3$, $OCHF_2$, $NHC(=O)C_{1-4}$ alkyl, aryl, $C_{3-6}$ cycloalkyl, and 4- to 12-membered heterocyclyl, wherein said aryl, cycloalkyl, and heterocyclyl are optionally substituted with $R^{10}$;
$R^{8b}$ is independently selected from H and F;
$R^{8c}$ is independently selected from H, F, Cl, $CH_3$, and $OCH_3$;
$R^{10}$ is independently selected from $C_{1-6}$ alkyl, $-C_{3-6}$ cycloalkyl, F, Cl, Br, $CF_3$, $CHF_2$, CN, and $OC_{1-5}$ alkyl; and
n, at each occurrence, is an integer independently selected from 0, 1, and 2.

In another aspect, the present invention provides compounds of Formula (IIb), or stereoisomers, tautomers, pharmaceutically acceptable salts, or solvates thereof, wherein:

ring A is independently selected from

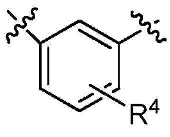, 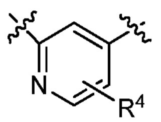, 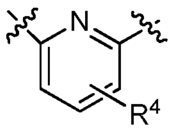,

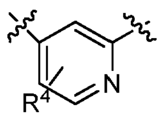, and 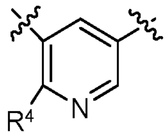;

ring B is independently selected from

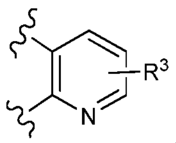, 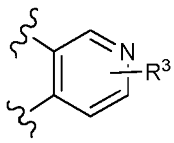, 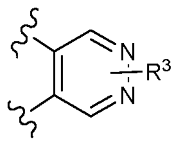,

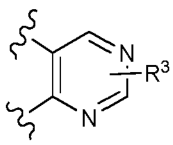, 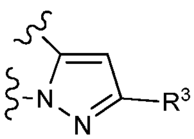, 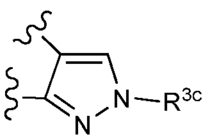, 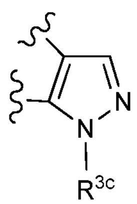

and

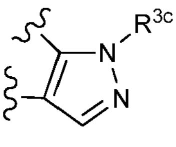;

W is independently selected from $CHR^{1a}$, O, NH, and $N(C_{1-4}$ alkyl);
$R^1$ is independently selected from H and $C_{1-4}$ alkyl;
$R^{1a}$ is independently selected from H, F, $CH_3$, and hydroxyl;
$R^2$ is independently selected from H and hydroxyl;
$R^3$ is independently selected from H, F, $CHF_2$, $CF_3$, $CH_3$, CN, $-(CH_2)_{0-2}-OH$, $OC_{1-4}$ alkyl, $C(=O)C_{1-4}$ alkyl, $-(CH_2)_{0-1}-C(=O)OH$, and $-C(=O)OC_{1-4}$ alkyl;
$R^{3c}$ is independently selected from H, $CF_2H$, $CF_3$, and $C_{1-4}$ alkyl (e.g. $CD_3$);

$R^4$ is independently selected from H and F;
$R^{8b}$ is independently selected from H and F;
$R^{8c}$ is independently selected from H and Cl;
$R^{10}$ is independently selected from H, $C_{1-6}$ alkyl (optionally substituted with $R^{11}$), aryl, $-(CH_2)_n-C_{3-6}$ cycloalkyl (optionally substituted with $R^{11}$), $-(CH_2)_n$-O-4- to 10-membered heterocyclyl, F, Cl, Br, CN, $C(=O)NR^{12}R^{12}$, $Si(C_{1-4}\ alkyl)_3$, and $-(CH_2)_n-OR^{12}$;
$R^{11}$, at each occurrence, is independently selected from H, halogen, and $C_{1-5}$ alkyl; and
n, at each occurrence, is an integer independently selected from 0, 1, and 2; and
other variables are as defined in Formula (IVa) above.

In another aspect, the present invention provides compounds of Formula (IIb), or stereoisomers, tautomers, pharmaceutically acceptable salts, or solvates thereof, wherein:

ring A is independently selected from

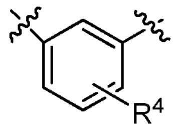 , 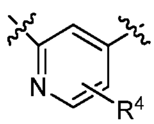 , 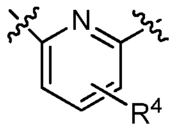 ,

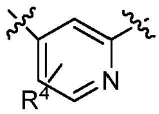 , and 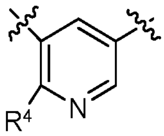 ;

ring B is independently selected from

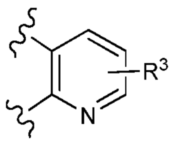 , 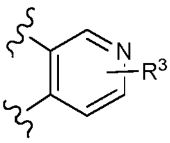 , 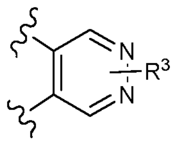 ,

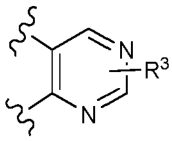 , 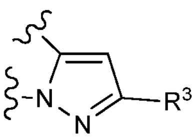 , 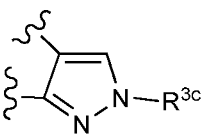 , 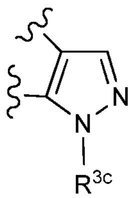

and

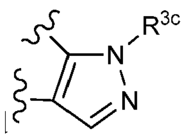 ;

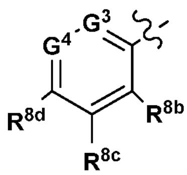

is independently selected from

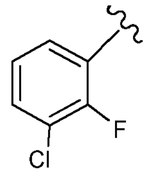 , 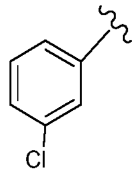 , 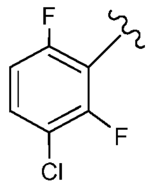 ,

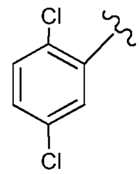 ,

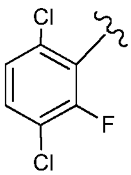 , 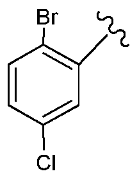 , 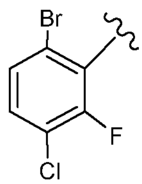 , 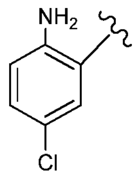 , 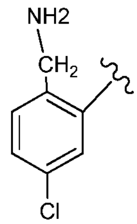 , 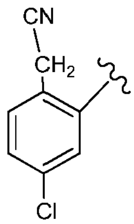 , 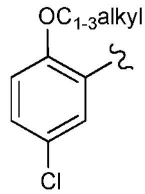 ,

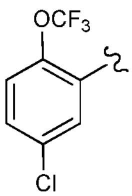 , 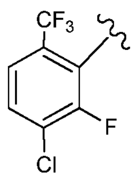 , 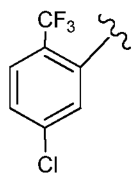 , 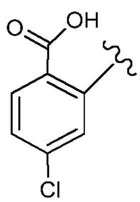 , 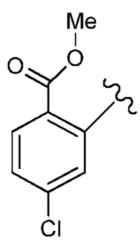 , 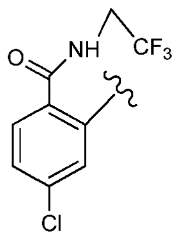 , 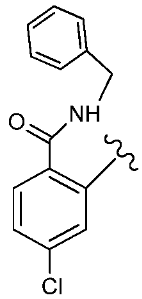 ,

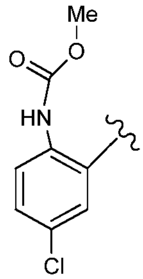 , 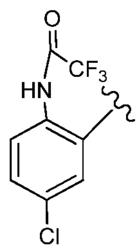 , 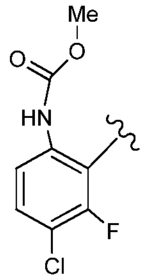 , 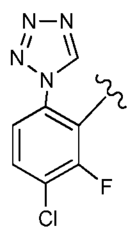 , 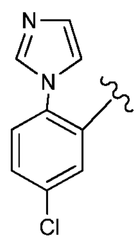 ,

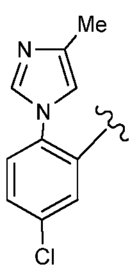 , 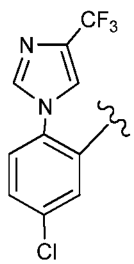 , 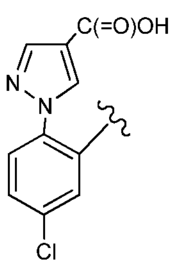 , 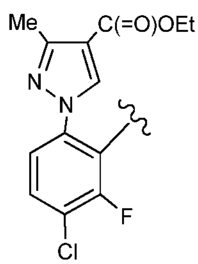 , 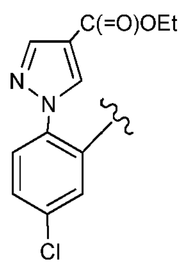 ,

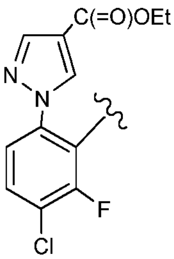, 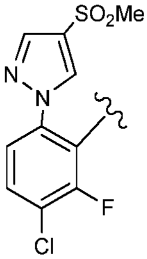, 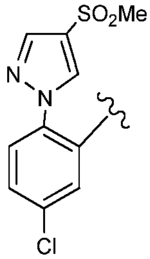, 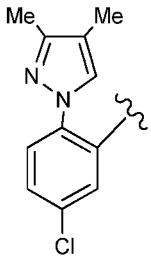, 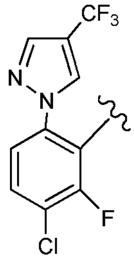, 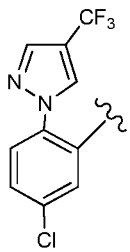, 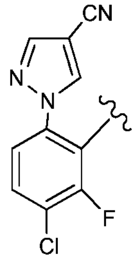,

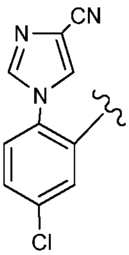, 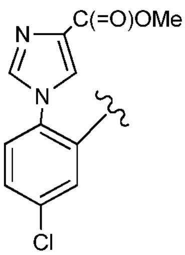, 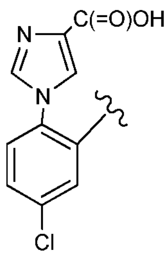, 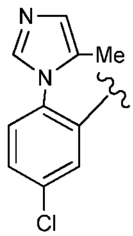, 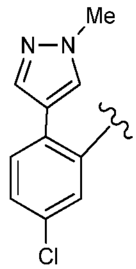, 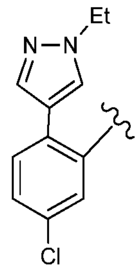, 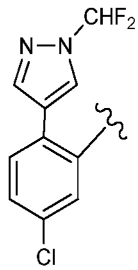,

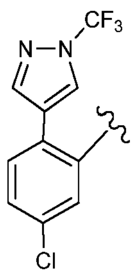, 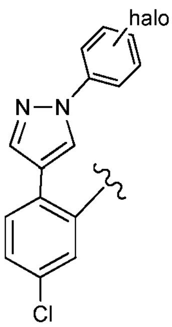, 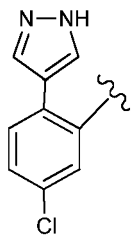,

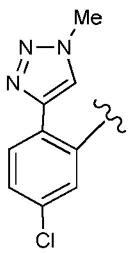, 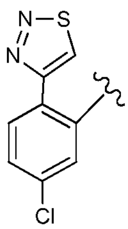, 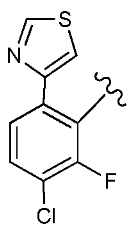, 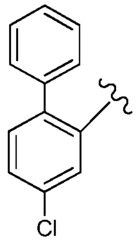, 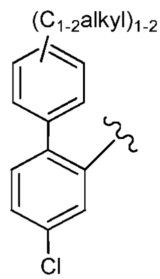, 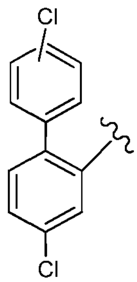, 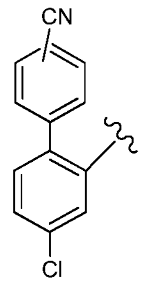,

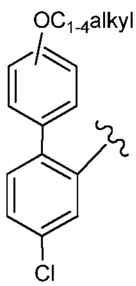, 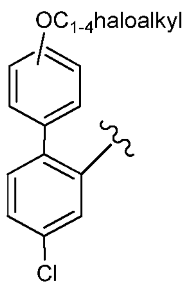, 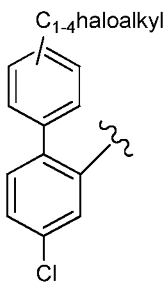, 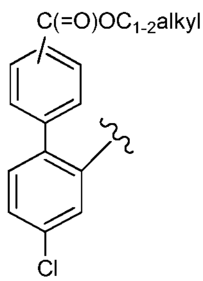, 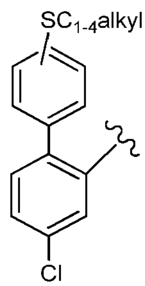, 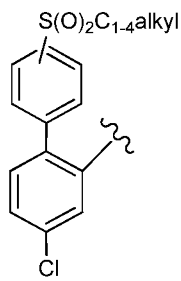,

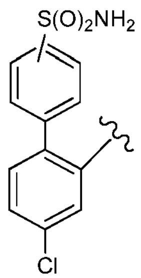 , 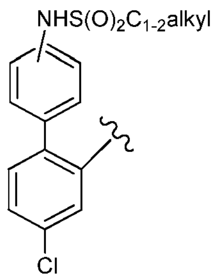 , 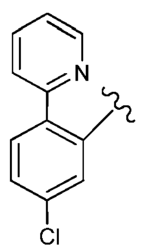 , 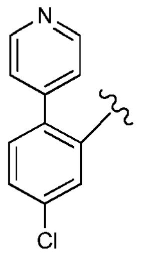 , 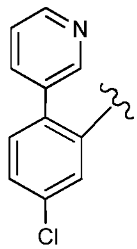 , 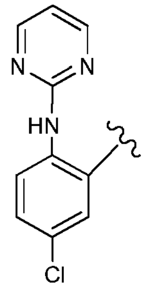 , 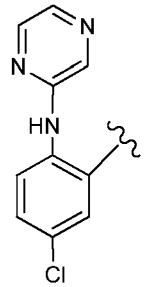 ,

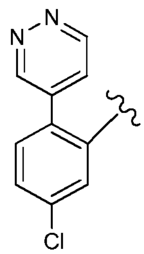 , 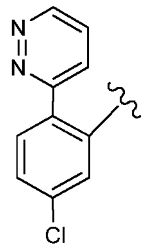 , 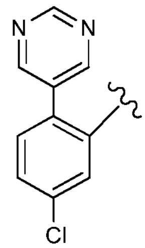 , 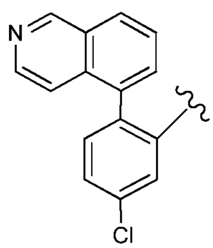 , and 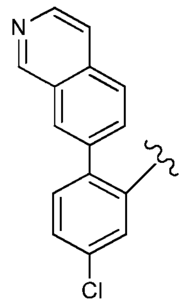 ;

W is independently selected from $CHR^1$, O, NH, and N($C_{1-4}$ alkyl);
Y is independently selected from $-CH_2NH-$, -NHC(=O)- and -C(=O)NH-;
$R^1$ and $R^2$ are independently selected from H, F, $C_{1-4}$ alkyl, and hydroxyl;
$R^3$ is independently selected from H, =O, F, $CHF_2$, $CF_3$, $CH_3$, CN, $-(CH_2)_{0-2}-OH$, $OC_{1-4}$ alkyl, $C(=O)C_{1-4}$ alkyl, $-(CH_2)_{0-1}-C(=O)OH$, and $-C(=O)OC_{1-4}$ alkyl, ;
$R^{3c}$ is independently selected from H, $CF_2H$, $CF_3$, and $C_{1-4}$ alkyl (e.g. $CD_3$);
$R^4$ is independently selected from H, F, and $C_{1-4}$ alkyl; and
$R^7$ is H; and
other variables are as defined in Formula (IIb) above.

In one embodiment of the compound of Formula (IIIb), $G^1$ is independently selected from the group consisting of

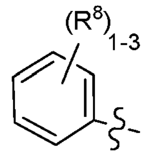 ,

wherein $R^8$ is, independently at each occurrence, selected from the group consisting of H, halogen, CN, $C_{1-6}$ alkyl, haloalkyl, alkoxy, haloalkoxy, and 4- to 6-membered heterocyclyl.

In another embodiment of the compound of Formula (IIIb), $G^1$ is

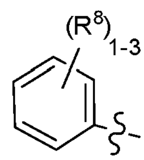 ,

wherein $R^8$ is, independently at each occurrence, selected from the group consisting of H, halogen, CN, methyl, ethyl, $CF_3$ $CHF_2$, OMe, OEt, $OCF_3$, $OCHF_2$, aryl, $C_{3-6}$ cycloalkyl, and 4- to 6-membered heterocyclyl.

In another embodiment of the compound of Formula (IIIb), $G^1$ is

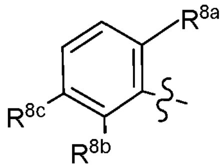

and selected from the group consisting of

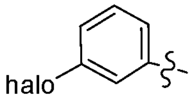, 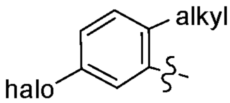, 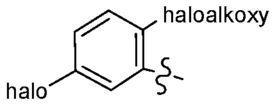,

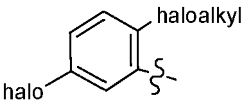, 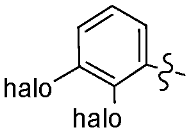, 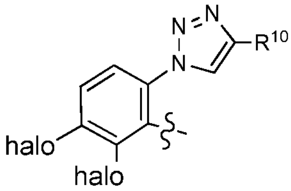, 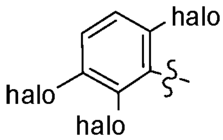,

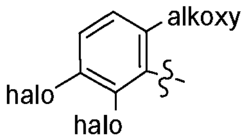, and 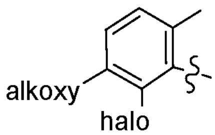.

In another embodiment of the compound of Formula (IIIb), $G^1$ is

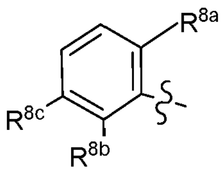,

wherein $R^{8a}$ is independently selected from the group consisting of H, F, $OCH_3$, $OCHF_2$, and

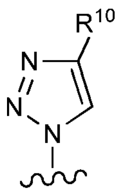.

In another embodiment, $R^{8b}$ is independently selected from the group consisting of H, F and Cl.

In another embodiment, $R^{8b}$ is independently selected from the group consisting of H and F.

In another embodiment, $R^{8c}$ is Cl.

In another embodiment of the compound of Formula (IIIb), $G^1$ is

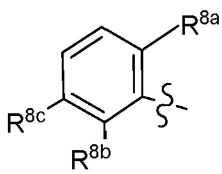

selected from the group consisting of 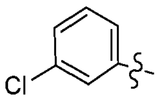, 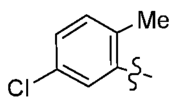, 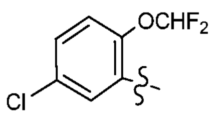, 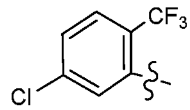, 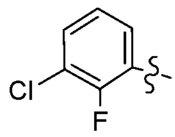, 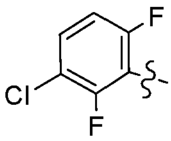, 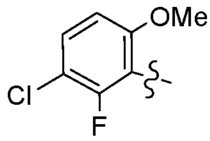, 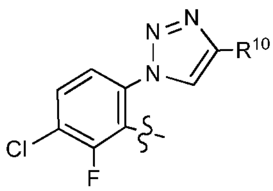, 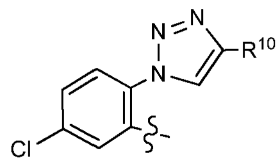,

and

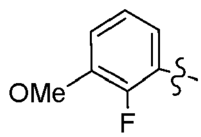.

In another embodiment of the compound of Formula (IIIb), $G^1$ is

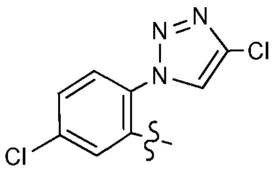.

In one embodiment, the present invention provides compounds of Formulae (IIb), (IIIb), or stereoisomers, tautomers, pharmaceutically acceptable salts, or solvates, wherein ring A is independently selected from the group consisting of imidazole, oxadiazole, pyridine, pyridinone, pyridazine, pyridazinone, and phenyl.

In another embodiment,

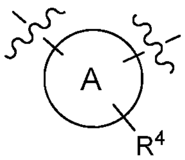

is independently selected from the group consisting of

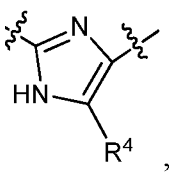, 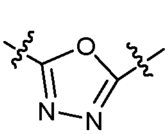, 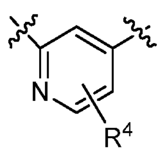, 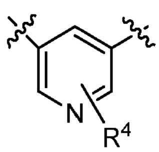, 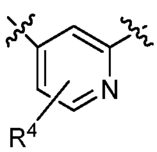,

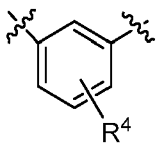, 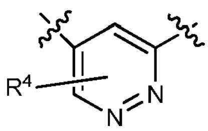, 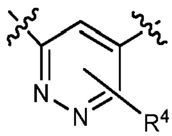 and 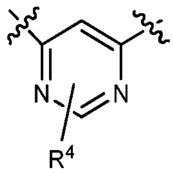.

In another embodiment,

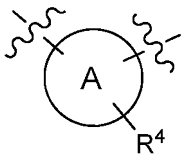

is independently selected from the group consisting of

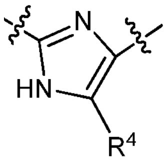, 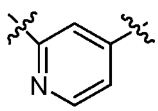, 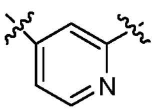, 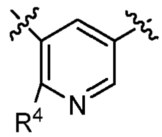, 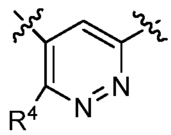,

and

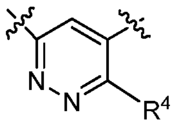.

In another embodiment,

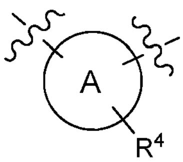

is independently selected from the group consisting of

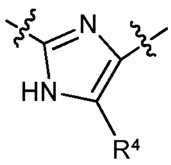, 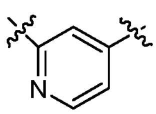, 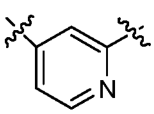, and 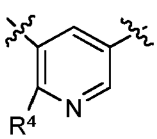.

In still another embodiment,

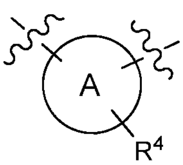

is independently selected from the group consisting of

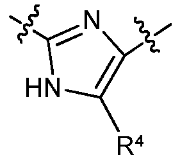 , 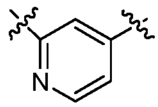 , and 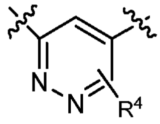 .

In another embodiment,

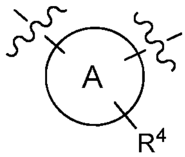 is 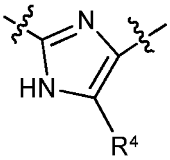 .

In another embodiment,

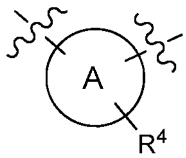 is 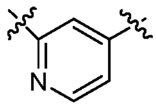 .

In another embodiment,

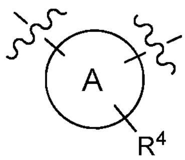 is 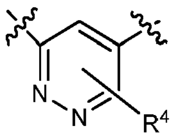 .

In another embodiment,

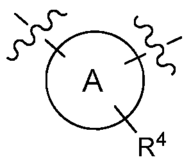 is 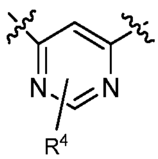 .

In another embodiment,

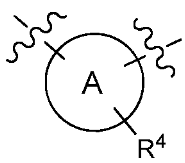 is 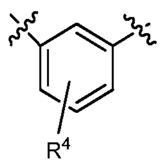 .

In another embodiment,

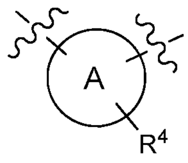 is 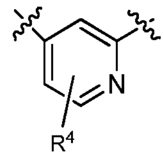 .

In another embodiment,

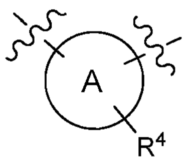 is 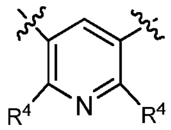 .

In another embodiment of the compound of Formula (IIb), ring B is independently selected from

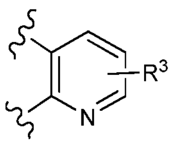 ,

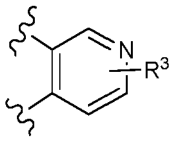 , 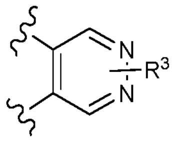 , 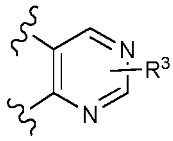 , 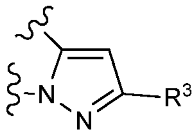 , 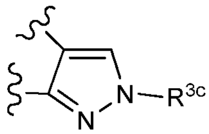 , 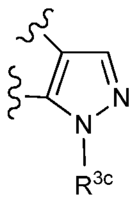

and

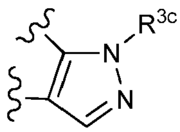 ;

In another embodiment of the compound of Formula (IIb), ring B is independently selected from

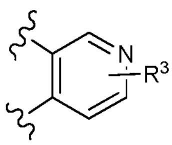 ,

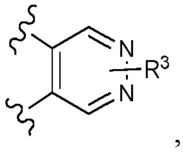 , and 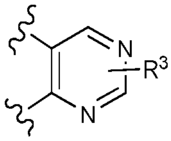 ;

In another embodiment of the compound of Formula (IIb), ring B is independently selected from,

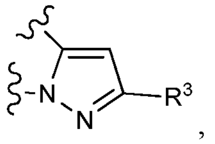

,

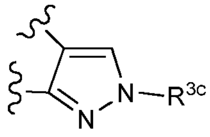

,

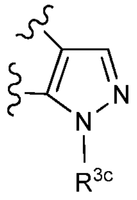

and

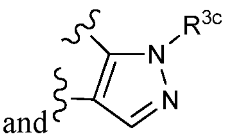

;

In another embodiment of the compound of Formula (IIb), ring B is

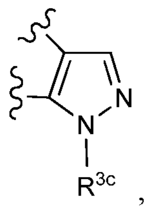

,

wherein $R^{3c}$ is independently selected from H, $CHF_2$, $CD_3$, $CH_3$, and $SO_2CH_3$.

In another embodiment, $R^1$ is independently selected from the group consisting of H, OH, F, and $C_{1-4}$ alkyl.

In another embodiment, $R^1$ is independently selected from the group consisting of H and methyl, ethyl, and isopropyl.

In one embodiment, $R^2$ is, independently at each occurrence, selected from the group consisting of H and $C_{1-4}$ alkyl.

In another embodiment, $R^2$ is, independently at each occurrence, selected from the group consisting of H and methyl.

In another embodiment, one of $R^1$ and $R^2$ is H and the other is methyl;

In another aspect, the present invention provides a compound selected from any subset list of compounds exemplified in the present application.

In another aspect, the present invention provides a compound selected from:

(9R,13S)-13-(4-{5-chloro-2-[(pyrimidin-2-yl)amino]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one;
2-(4-chloro-2-{1-[(9R,13S)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)acetonitrile;
(9*R*,13*S*)-13-{4-[5-chloro-2-(1-methyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one;
(9*R*,13*S*)-13-{4-[5-chloro-2-(1,3-dimethyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate;
(10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-4,5,8-triazatricyclo[13.3.1.$0^{2,7}$]nonadeca-1(19),2(7),3,5,15,17-hexaen-9-one;
1-(4-chloro-2-{1-[(9R,13S)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)-1H-pyrazole-4-carboxylic acid;
(9R,13S)-13-[4-(2-bromo-5-chlorophenyl)-5-chloro-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one;
(9R,13S)-13-{4-[3-chloro-2-fluoro-6-(1,3-thiazol-5-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-one;
(9*R*,13*S*)-13-{4-[3-chloro-6-(difluoromethyl)-2-fluorophenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate;
(9*R*,13*S*)-13-{4-[3-chloro-6-(1,1-difluoroethyl)-2-fluorophenyl]-6-oxo-1,6-ihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate;
(9*R*,13*S*)-13-[4-(3-chloro-2,6-difluorophenyl)-6-oxo-1,6-dihydropyrimidin-1-yl]-16-fluoro-3,9-dimethyl-3,4,7-triazatricyclo[12.3.1.0$^{2.6}$]octadeca-1(18),2(6),4,14,16-pentaen-8-one;
(10*R*,14*S*)-14-{4-[3-chloro-6-(4-chloro-1*H*-1,2,3-triazol-1-yl)-2-fluorophenyl]-6-oxo-1,6-dihydropyrimidin-1-yl}-10-methyl-3,8-diazatricyclo[13.3.1.0$^{2,7}$]nonadeca-1(19),2(7),3,5,15,17-hexaen-9-one trifluoroacetate;
(10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.0$^{2,7}$]nonadeca-1(19),2(7),3,5,15,17-hexaen-9-one;
(10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-4,10-dimethyl-3,8-diazatricyclo[13.3.1.0$^{2,7}$]nonadeca-1(19),2(7),3,5,15,17-hexaen-9-one;
(10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-5-methoxy-10-methyl-3,8-diazatricyclo[13.3.1.0$^{2,7}$] nonadeca-1(19),2(7),3,5,15,17-hexaen-9-one;
(10*R*,14*S*)-5-chloro-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.0$^{2,7}$] nonadeca-1(19),2(7),3,5,15,17-hexaen-9-one;
(9*R*,13*S*)-13-{4-[5-chloro-2-(1*H*-1,2,3,4-tetrazol-1-yl)phenyl]-6-oxo-1,6-dihydropyrimidin-1-yl}-3-(2-hydroxyethyl)-9-methyl-3,4,7,15-tetraazatricyclo [12.3.1.0$^{2,6}$]octadeca-1(18),2(6),4,14,16-pentaen-8-onetrifluoroacetate;
2-[(9*R*,13*S*)-13-{4-[5-chloro-2-(1*H*-1,2,3,4-tetrazol-1-yl)phenyl]-6-oxo-1,6-dihydropyrimidin-1-yl}-9-methyl-8-oxo-3,4,7,15-tetraazatricyclo[12.3.1.0$^{2.6}$]octadeca-1(18),2(6),4,14,16-pentaen-3-yl]acetic acid trifluoroacetate;
(10*R*,14*S*)-14-{4-[5-chloro-2-(4-chloro-1H-1,2,3-triazol-1-yl)phenyl]-6-oxo-1,6-dihydropyrimidin-1-yl}-10-methyl-3,8,16-triazatricyclo[13.3.1.0$^{2,7}$]nonadeca-1(19),2(7),3,5,15,17-hexaen-9-one, *bis*-trifluoroacetate;
(10*R*,14*S*)-14-{4-[3-chloro-6-(4-chloro-1H-1,2,3-triazol-1-yl)-2-fluorophenyl]-6-oxo-1,6-dihydropyrimidin-1-yl}-10-methyl-3,8,16-triazatricyclo[13.3.1.0$^{2,7}$]nonadeca-1(19),2(7),3,5,15,17-hexaen-9-one *bis*-trifluoroacetate;
(10*R*,14*S*)-14-(4-{5-chloro-2-[4-(trifluoromethyl)-1H-1,2,3-triazol-1-yl]phenyl}-6-oxo-1,6-dihydropyrimidin-1-yl)-10-methyl-3,8,16-triazatricyclo[13.3.1.0$^{2,7}$]nonadeca-1(19),2(7),3,5,15,17-hexaen-9-one, *bis*-trifluoroacetate;
*(9R*,13*S*)-13-[4-(3-chloro-2,6-difluorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate;
(9R,13S)-13-[4-(2-bromo-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
(9*R*,13*S*)-13-(4-{5-chloro-2-[1-(difluoromethyl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one;
(10*R*,14*S*)-14-(4-{5-chloro-2-[4-(trifluoromethyl)-1H-1,2,3-triazol-1-yl]phenyl}-6-oxo-1,6-dihydropyrimidin-1-yl)-10-methyl-9-oxo-3,8-diazatricyclo[13.3.1.0$^{2,7}$] nonadeca-1(19),2(7),3,5,15,17-hexaene-5-carbonitrile;
(9*R*,13*S*)-13-(4-{5-chloro-2-[4-(trifluoromethyl)-1H-pyrazol-1-yl]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one trifluoroacetate;
(9*R*,13*S*)-13-{4-[5-chloro-2-(pyridin-3-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
5-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)pyridine-3-carbonitrile;
methyl 4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}benzoate;
(9*R*,13*S*)-13-{4-[3-chloro-2-fluoro-6-(trifluoromethyl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one trifluoroacetate;
(9*R*,13*S*)-13-[4-(3-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
4-chloro-2-{1-[(9*R*,13*S*)-3,9-dimethyl-8-oxo-3,4,7,15-tetraazatricyclo[12.3.1.0$^{2,6}$] octadeca-1(18),2(6),4,14,16-pentaen-13-yl]-6-oxo-1,6-dihydropyrimidin-4-yl}benzoic acid;
(9*R*,13*S*)-13-[4-(3-chloro-2,6-difluorophenyl)-6-oxo-1,6-dihydropyrimidin-1-yl]-3-(difluoromethyl)-9-methyl-3,4,7,15-tetraazatricyclo[12.3.1.0$^{2,6}$]octadeca-1(18),2(6),4,14,16-pentaen-8-one;
(9*R*,13*S*)-13-[4-(5-chloro-2-phenylphenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
(9*R*,13*S*)-13-{4-[3-chloro-2-fluoro-6-(1H-1,2,3,4-tetrazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate;
(9*R*,13*S*)-13-{4-[3-chloro-2-fluoro-6-(1H-1,2,3,4-tetrazol-1-yl)phenyl]-6-oxo-1,6-dihydropyrimidin-1-yl}-3-(difluoromethyl)-9-methyl-3,4,7-triazatricyclo[12.3.1.0$^{2,6}$] octadeca-1(18),2(6),4,14,16-pentaen-8-one trifluoroacetate;
(9*R*,13*S*)-13-{4-[5-chloro-2-(1H-1,2,3,4-tetrazol-1-yl)phenyl]-6-oxo-1,6-dihydropyrimidin-1-yl}-3-(difluoromethyl)-9-methyl-3,4,7,15-tetraazatricyclo [12.3.1.0$^{2,6}$]octadeca-1(18),2(6),4,14,16-pentaen-8-onetrifluoroacetate;
(9*R*,13*S*)-13-{4-[5-chloro-2-(1H-1,2,3,4-tetrazol-1-yl)phenyl]-6-oxo-1,6-dihydropyrimidin-1-yl}-3-(difluoromethyl)-9-methyl-3,4,7-triazatricyclo[12.3.1.0$^{2.6}$] octadeca-1(18),2(6),4,14,16-pentaen-8-one;
(10*R*,14*S*)-14-(4-{5-chloro-2-[4-(trifluoromethyl)-1H-1,2,3-triazol-1-yl]phenyl}-6-oxo-1,6-dihydropyrimidin-1-yl)-10-methyl-5,8-diazatricyclo[13.3.1.0$^{2,7}$]nonadeca-1(19),2(7),3,5,15,17-hexaen-9-one;
(10*R*,14*S*)-14-{4-[5-chloro-2-(4-chloro-1H-1,2,3-triazol-1-yl)phenyl]-6-oxo-1,6-dihydropyrimidin-1-yl}-10-methyl-5,8-diazatricyclo[13.3.1.0$^{2,7}$]nonadeca-1(19),2(7),3,5,15,17-hexaen-9-one;
(9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
*N*-(4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-3-fluorophenyl)carbamate trifluoroacetate;
4-chloro-2-{1-[(9R,13S)-3,9-dimethyl-8-oxo-3,4,7,15-tetraazatricyclo[12.3.1.0$^{2,6}$] octadeca-1(18),2(6),4,14,16-pentaen-13-yl]-6-oxo-1,6-dihydropyrimidin-4-yl}-N-(2,2,2-trifluoroethyl)benzamide;
(9R,13S)-13-{4-[5-chloro-2-(1H-imidazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(10R,14S)-3-chloro-14-{4-[5-chloro-2-(4-chloro-1H-1,2,3-triazol-1-yl)phenyl]-6-oxo-1,6-dihydropyrimidin-1-yl}-10-methyl-5,8-diazatricyclo[13.3.1.0$^{2,7}$]nonadeca-1(19),2(7),3,5,15,17-hexaen-9-one;
(9R,13S)-13-{4-[5-chloro-2-(1H-pyrazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
N-benzyl-4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}benzamide;
1-(4-chloro-3-fluoro-2-{1-[(9R,13S)-3-($^{2}H_3$)methyl-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazole-4-carbonitrile;
1-(4-chloro-2-{1-[(9R,13S)-3-(difluoromethyl)-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile;
(9R,13S)-13-{4-[5-chloro-2-(1-propyl-1H-pyrazol-4-yl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carbonitrile;
N-(4-chloro-2-{1-[(9R,13S)-3-(difluoromethyl)-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide;
(9R,13S)-13-(4-{5-chloro-2-[1-(propan-2-yl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one;
(9R,13 S)-13-(4-{5-chloro-2-[1-(2-methylpropyl)-1H-pyrazol-4-yl]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
(9R,13S)-13-{4-[2-(1-benzyl-1H-pyrazol-4-yl)-5-chlorophenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13S)-13-{4-[5-chloro-2-(1-methyl-1H-1,2,3-triazol-4-yl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(10R,14S)-14-(4-{5-chloro-2-[4-(trifluoromethyl)-1H-1,2,3-triazol-1-yl]phenyl}-6-oxo-1,6-dihydropyrimidin-1-yl)-4-fluoro-10-methyl-5,8,16-triazatricyclo[13.3.1.0$^{2,7}$] nonadeca-1(19),2(7),3,5,15,17-hexaen-9-one;
(9R,13S)-13-{4-[5-chloro-2-(1-cyclopropyl-1H-pyrazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-(4-{5-chloro-2-[1-(2,2,2-trifluoroethyl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one;
(9R,13S)-13-{4-[5-chloro-2-(5-methyl-1H-imidazol-1-yl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one trifluoroacetate;
(9R,13S)-13-[4-(2-Amino-5-chlorophenyl)-6-oxo-1,6-dihydropyrimidin-1-yl]-3-(difluoromethyl)-9-methyl-3,4,7,15-tetraazatricyclo[12.3.1.0$^{2,6}$]octadeca-1(18),2(6),4,14,16-pentaen-8-one;
(9R,13 S)-13-(4-{3-chloro-6-[1-(difluoromethyl)-1H-pyrazol-4-yl]-2-fluorophenyl}-6-oxo-1,6-dihydropyrimidin-1-yl)-3,9-dimethyl-3,4,7,15-tetraazatricyclo [12.3.1.0$^{2,6}$]octadeca-1(18),2(6),4,14,16-pentaen-8-one;
(14S)-14-(4-{5-chloro-2-[4-(trifluoromethyl)-1H-1,2,3-triazol-1-yl]phenyl}-6-oxo-1,6-dihydropyrimidin-1-yl)-10-methyl-5,8,16-triazatricyclo[13.3.1.0$^{2,7}$]nonadeca-1(19),2(7),3,5,15,17-hexaen-9-one trifluoroacetate;
(9R,13S)-13-{4-[5-chloro-2-(4-methyl-1H-imidazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(1,3-oxazol-2-yl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13 S)-13-(4-{5-chloro-2-[(pyrazin-2-yl)amino]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
ethyl 1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-3-methyl-1H-pyrazole-4-carboxylate;
(9R,13S)-13-{4-[5-chloro-2-(3,4-dimethyl-1H-pyrazol-1-yl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
ethyl 1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-pyrazole-4-carboxylate;
(9R,13S)-13-[4-(5-chloro-2-hydroxyphenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
(9R,13S)-13-(4-{5-chloro-2-[4-(trifluoromethyl)-1H-imidazol-1-yl]phenyl}-6-oxo-1,6-dihydropyrimidin-1-yl)-3,9-dimethyl-3,4,7,15-tetraazatricyclo[12.3.1.0$^{2.6}$] octadeca-1(18),2(6),4,14,16-pentaen-8-one;
methyl 1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylate;
(9R,13S)-13-[4-(2,5-dichlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylic acid;
(10R,14S)-14-{4-[5-chloro-2-(4-chloro-1H-1,2,3-triazol-1-yl)phenyl]-6-oxo-1,6-dihydropyrimidin-1-yl}-10-methyl-3,5,8-triazatricyclo[13.3.1.0$^{2,7}$]nonadeca-1(19),2(7),3,5,15,17-hexaen-9-one;
(9R,13S)-13-{4-[5-chloro-2-(l,2,3-thiadiazol-4-yl)phenyl]-6-oxo-l,6-dihydropyrimidin-1-yl}-3-(difluoromethyl)-9-methyl-3,4,7,15-tetraazatricyclo [12.3.1.0$^{2,6}$]octadeca-1(18),2(6),4,14,16-pentaen-8-one;
(9R,13S)-13-(4-{3-chloro-2-fluoro-6-[4-(trifluoromethyl)-1H-pyrazol-1-yl]phenyl}-6-oxo-1,6-dihydropyrimidin-1-yl)-3,9-dimethyl-3,4,7,15-tetraazatricyclo [12.3.1.0$^{2,6}$]octadeca-1(18),2(6),4,14,16-pentaen-8-one;
(9R,13 S)-13-[4-(3-chloro-2,6-difluorophenyl)-6-oxo-1,6-dihydropyrimidin-1-yl]-9-methyl-3,4,7,15-tetraazatricyclo[12.3.1.0$^{2,6}$]octadeca-1(18),2(6),4,14,16-pentaen-8-one;
(9R,13S)-13-{4-[5-chloro-2-(l,2,3-thiadiazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-[4-(3-chloro-2,6-difluorophenyl)-6-oxo-1,6-dihydropyrimidin-1-yl]-9-methyl-4-(pyrimidin-5-yl)-3,4,7,15-tetraazatricyclo[12.3.1.0$^{2,6}$]octadeca-1(18),2,5,14,16-pentaen-8-one;
(9R,13S)-13-{4-[5-chloro-2-(pyridazin-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-5,8,17-triazatricyclo[13.3.1.0$^{2,7}$]nonadeca-1(19),2(7),3,5,15,17-hexaen-9-one;
1-(4-chloro-2-{1-[(9R,13 S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile;
N-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide;
(9R,13 S)-13-[4-(2-amino-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one;
(9R,13S)-13-(4-{5-chloro-2-[(pyrimidin-4-yl)amino]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
(9R,13S)-13-{4-[2-(aminomethyl)-5-chlorophenyl]-6-oxo-1,6-dihydropyrimidin-1-yl}-3-(difluoromethyl)-9-methyl-3,4,7,15-tetraazatricyclo[12.3.1.0$^{2.6}$]octadeca-1(18),2(6),4,14,16-pentaen-8-one;
(9R,13S)-13-{4-[5-chloro-2-(pyridin-2-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(4-methylphenyl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13S)-13-{4-[5-chloro-2-(3-chlorophenyl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13S)-13-{4-[5-chloro-2-(3-methoxyphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(2-methylphenyl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13 S)-13-(4-{5-chloro-2-[4-(trifluoromethoxy)phenyl]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
(9R,13S)-13-{4-[5-chloro-2-(2-chlorophenyl)phenyl]-6-oxo-1,6-dihydropyrimidin-1-yl}-3,9-dimethyl-3,4,7,15-tetraazatricyclo[12.3.1.0$^{2.6}$]octadeca-1(18),2(6),4,14,16-pentaen-8-one;
(9R,13 S)-13-(4-{5-chloro-2-[4-(trifluoromethyl)phenyl]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
(9R,13 S)-13-(4-{5-chloro-2-[4-(propan-2-ylsulfanyl)phenyl]phenyl}-6-oxo-1,6-dihydropyrimidin-1-yl)-3,9-dimethyl-3,4,7,15-tetraazatricyclo[12.3.1.0$^{2.6}$]octadeca-1(18),2(6),4,14,16-pentaen-8-one;
4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzene-1-sulfonamide;
(9R,13 S)-13-(4-{5-chloro-2-[4-(difluoromethoxy)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one;
N-[3-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)phenyl]methanesulfonamide;
3-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzonitrile;
(9R,13 S)-13-(4-{5-chloro-2-[3-(trifluoromethoxy)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(3-methanesulfonylphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
methyl 4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzoate;
(9R,13 S)-13-{4-[5-chloro-2-(3-methylphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzonitrile;
(9R,13S)-13-{4-[5-chloro-2-(1-methyl-1H-indol-5-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(isoquinolin-5-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
methyl 3-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzoate;
N-[4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)phenyl]methanesulfonamide;
(9R,13S)-13-(4-{5-chloro-2-[3-(trifluoromethyl)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(4-methoxyphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(4-chlorophenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(pyridin-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(isoquinolin-7-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(pyrimidin-5-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one;
(9R,13S)-13-{4-[5-chloro-2-(1-ethyl-1H-pyrazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one; and
(9R,13S)-13-(4-{5-chloro-2-[1-(4-fluorophenyl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one.

In another embodiment, the compounds of the present invention have Factor XIa or plasma kallikrein Ki values $\leq$ 10 $\mu$M.

In another embodiment, the compounds of the present invention have Factor XIa or plasma kallikrein Ki values $\leq$ 1 $\mu$M.

In another embodiment, the compounds of the present invention have Factor XIa or plasma kallikrein Ki values $\leq$ 0.5 $\mu$M.

In another embodiment, the compounds of the present invention have Factor XIa or plasma kallikrein Ki values $\leq$ 0.1 $\mu$M.

II. OTHER EMBODIMENTS OF THE INVENTION

In another embodiment, the present invention provides a composition comprising at least one of the compounds of the present invention or a stereoisomer, a tautomer, a pharmaceutically acceptable salt, or a solvate thereof.

In another embodiment, the present invention provides a pharmaceutical composition comprising a pharmaceutically acceptable carrier and at least one of the compounds of the present invention or a stereoisomer, a tautomer, a pharmaceutically acceptable salt, or a solvate, thereof.

In another embodiment, the present invention provides a pharmaceutical composition, comprising: a pharmaceutically acceptable carrier and a therapeutically effective amount of at least one of the compounds of the present

invention or a stereoisomer, a tautomer, a pharmaceutically acceptable salt, or a solvate thereof.

Also disclosed herein is a process for making a compound of the present invention.

Also disclosed herein is an intermediate for making a compound of the present invention.

In another embodiment, the present invention provides a pharmaceutical composition further comprising additional therapeutic agent(s). In a preferred embodiment, the present invention provides pharmaceutical composition, wherein the additional therapeutic agent(s) are an anti-platelet agent or a combination thereof. Preferably, the antiplatelet agent(s) are clopidogrel and/or aspirin, or a combination thereof.

In another embodiment, the present invention provides a compound of the invention for use in a method for the treatment and/or prophylaxis of a thromboembolic disorder comprising administering to a patient in need of such treatment and/or prophylaxis a therapeutically effective amount of at least one of the compounds of the present invention or a stereoisomer, a tautomer, a pharmaceutically acceptable salt, or a solvate thereof.

In another embodiment, the present invention provides a compound of the present invention or a stereoisomer, a tautomer, a pharmaceutically acceptable salt, or a solvate thereof, for use in therapy.

In another embodiment, the present invention provides a compound of the present invention or a stereoisomer, a tautomer, a pharmaceutically acceptable salt, or a solvate thereof, for use in therapy for the treatment and/or prophylaxis of a thromboembolic disorder.

In another embodiment, the present invention provides a compound of the invention for use in a method for treatment and/or prophylaxis of a thromboembolic disorder, comprising: administering to a patient in need thereof a therapeutically effective amount of a first and second therapeutic agent, wherein the first therapeutic agent is a compound of the present invention or a stereoisomer, a tautomer, a pharmaceutically acceptable salt, or a solvate thereof, and the second therapeutic agent is at least one agent selected from a factor Xa inhibitor such as apixaban, rivaroxaban, betrixaban, edoxaban, an anti-coagulant agent, an anti-platelet agent, a thrombin inhibiting agent such as dabigatran, a thrombolytic agent, and a fibrinolytic agent. Preferably, the second therapeutic agent is at least one agent selected from warfarin, unfractionated heparin, low molecular weight heparin, synthetic pentasaccharide, hirudin, argatroban, aspirin, ibuprofen, naproxen, sulindac, indomethacin, mefenamate, droxicam, diclofenac, eribaxaban, sulfinpyrazone, piroxicam, ticlopidine, clopidogrel, tirofiban, eptifibatide, abciximab, melagatran, desulfatohirudin, tissue plasminogen activator, modified tissue plasminogen activator, anistreplase, urokinase, and streptokinase. Preferably, the second therapeutic agent is at least one anti-platelet agent. Preferably, the anti-platelet agent(s) are clopidogrel and/or aspirin, or a combination thereof.

The thromboembolic disorder includes arterial cardiovascular thromboembolic disorders, venous cardiovascular thromboembolic disorders, arterial cerebrovascular thromboembolic disorders, and venous cerebrovascular thromboembolic disorders. Examples of the thromboembolic disorder include, but are not limited to, unstable angina, an acute coronary syndrome, atrial fibrillation, first myocardial infarction, recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis.

In another embodiment, the present invention provides a compound of the invention for use in a method for the treatment and/or prophylaxis of an inflammatory disorder comprising: administering to a patient in need of such treatment and/or prophylaxis a therapeutically effective amount of at least one of the compounds of the present invention or a stereoisomer, a tautomer, a pharmaceutically acceptable salt, or a solvate thereof. Examples of the inflammatory disorder include, but are not limited to, sepsis, acute respiratory distress syndrome, and systemic inflammatory response syndrome.

In another embodiment, the present invention provides a compound of the invention for use in a method for the prophylaxis of a disease or condition in which plasma kallikrein activity is implicated comprising administering to a patient in need of such treatment and/or prophylaxis a therapeutically effective amount of at least one of the compounds of the present invention or a stereoisomer, a tautomer, a pharmaceutically acceptable salt, or a solvate thereof.

The disease or condition in which plasma kallikrein activity is implicated includes, but not limited to, impaired visual acuity, diabetic retinopathy, diabetic macular edema, hereditary angioedema, diabetes, pancreatitis, nephropathy, cardio myopathy, neuropathy, inflammatory bowel disease, arthritis, inflammation, septic shock, hypotension, cancer, adult respiratory distress syndrome, disseminated intravascular coagulation, and cardiopulmonary bypass surgery.

In another embodiment, the present invention provides a combined preparation of a compound of the present invention and additional therapeutic agent(s) for simultaneous, separate or sequential use in therapy.

In another embodiment, the present invention provides a combined preparation of a compound of the present invention and additional therapeutic agent(s) for simultaneous, separate or sequential use in treatment and/or prophylaxis of a thromboembolic disorder.

This invention encompasses all combinations of preferred aspects of the invention noted herein. It is understood that any and all embodiments of the present invention may be taken in conjunction with any other embodiment or

embodiments to describe additional embodiments. It is also to be understood that each individual element of the embodiments is its own independent embodiment. Furthermore, any element of an embodiment is meant to be combined with any and all other elements from any embodiment to describe an additional embodiment.

III. CHEMISTRY

Throughout the specification and the appended claims, a given chemical formula or name shall encompass all stereo and optical isomers and racemates thereof where such isomers exist. Unless otherwise indicated, all chiral (enantiomeric and diastereomeric) and racemic forms are within the scope of the invention. Many geometric isomers of C=C double bonds, C=N double bonds, ring systems, and the like can also be present in the compounds, and all such stable isomers are contemplated in the present invention. *Cis*- and *trans-(or E-* and *Z-*) geometric isomers of the compounds of the present invention are described and may be isolated as a mixture of isomers or as separated isomeric forms. The present compounds can be isolated in optically active or racemic forms. Optically active forms may be prepared by resolution of racemic forms or by synthesis from optically active starting materials. When enantiomeric or diastereomeric products are prepared, they may be separated by conventional methods, for example, by chromatography or fractional crystallization. Depending on the process conditions the end products of the present invention are obtained either in free (neutral) or salt form. Both the free form and the salts of these end products are within the scope of the invention. If so desired, one form of a compound may be converted into another form. A free base or acid may be converted into a salt; a salt may be converted into the free compound or another salt; a mixture of isomeric compounds of the present invention may be separated into the individual isomers. Compounds of the present invention, free form and salts thereof, may exist in multiple tautomeric forms, in which hydrogen atoms are transposed to other parts of the molecules and the chemical bonds between the atoms of the molecules are consequently rearranged. It should be understood that all tautomeric forms, insofar as they may exist, are included within the invention.

The term "stereoisomer" refers to isomers of identical constitution that differ in the arrangement of their atoms in space. Enantiomers and diastereomers are examples of stereoisomers. The term "enantiomer" refers to one of a pair of molecular species that are mirror images of each other and are not superimposable. The term "diastereomer" refers to stereoisomers that are not mirror images. The term "racemate" or "racemic mixture" refers to a composition composed of equimolar quantities of two enantiomeric species, wherein the composition is devoid of optical activity.

The symbols "R" and "S" represent the configuration of substituents around a chiral carbon atom(s). The isomeric descriptors "R" and "S" are used as described herein for indicating atom configuration(s) relative to a core molecule and are intended to be used as defined in the literature (IUPAC Recommendations 1996, Pure and Applied Chemistry, 68:2193-2222 (1996)).

The term "chiral" refers to the structural characteristic of a molecule that makes it impossible to superimpose it on its mirror image. The term "homochiral" refers to a state of enantiomeric purity. The term "optical activity" refers to the degree to which a homochiral molecule or nonracemic mixture of chiral molecules rotates a plane of polarized light.

As used herein, the term "alkyl" or "alkylene" is intended to include both branched and straight-chain saturated aliphatic hydrocarbon groups having the specified number of carbon atoms. For example, "$C_1$ to $C_{10}$ alkyl" or "$C_{1-10}$ alkyl" (or alkylene), is intended to include $C_1$, $C_2$, $C_3$, $C_4$, $C_5$, $C_6$, $C_7$, $C_8$, $C_9$, and $C_{10}$ alkyl groups. Additionally, for example, "$C_1$ to $C_6$ alkyl" or "$C_1$-$C_6$ alkyl" denotes alkyl having 1 to 6 carbon atoms. Alkyl group can be unsubstituted or substituted with at least one hydrogen being replaced by another chemical group. Example alkyl groups include, but are not limited to, methyl (Me), ethyl (Et), propyl (*e.g.,* n-propyl and isopropyl), butyl (*e.g.,* n-butyl, isobutyl, *t*-butyl), and pentyl (*e.g.*, n-pentyl, isopentyl, neopentyl). When "$C_0$ alkyl" or "$C_0$ alkylene" is used, it is intended to denote a direct bond. "Alkyl" also includes deuteroalkyl such as $CD_3$.

"Alkenyl" or "alkenylene" is intended to include hydrocarbon chains of either straight or branched configuration having one or more, preferably one to three, carbon-carbon double bonds that may occur in any stable point along the chain. For example, "$C_2$ to $C_6$ alkenyl" or "$C_{2-6}$ alkenyl" (or alkenylene), is intended to include $C_2$, $C_3$, $C_4$, $C_5$, and $C_6$ alkenyl groups; such as ethenyl, propenyl, butenyl, pentenyl, and hexenyl.

"Alkynyl" or "alkynylene" is intended to include hydrocarbon chains of either straight or branched configuration having one or more, preferably one to three, carbon-carbon triple bonds that may occur in any stable point along the chain. For example, "$C_2$ to $C_6$ alkynyl" or "$C_{2-6}$ alkynyl" (or alkynylene), is intended to include $C_2$, $C_3$, $C_4$, $C_5$, and $C_6$ alkynyl groups; such as ethynyl, propynyl, butynyl, pentynyl, and hexynyl.

The term "alkoxy" or "alkyloxy" refers to an -O-alkyl group. "$C_1$ to $C_6$ alkoxy" or "$C_{1-6}$ alkoxy" (or alkyloxy), is intended to include $C_1$, $C_2$, $C_3$, $C_4$, $C_5$, and $C_6$ alkoxy groups. Example alkoxy groups include, but are not limited to, methoxy, ethoxy, propoxy (e.g., n-propoxy and isopropoxy), and *t*-butoxy. Alkoxy also includes deuteroalkoxy such as $OCD_3$. Similarly, "alkylthio" or "thioalkoxy" represents an alkyl group as defined above with the indicated number of carbon atoms attached through a sulphur bridge; for example methyl-S- and ethyl-S-.

"Halo" or "halogen" includes fluoro, chloro, bromo, and iodo. "Haloalkyl" is intended to include both branched and straight-chain saturated aliphatic hydrocarbon groups having the specified number of carbon atoms, substituted

with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" that is intended to include both branched and straight-chain saturated aliphatic hydrocarbon groups having the specified number of carbon atoms, substituted with 1 or more fluorine atoms.

"Haloalkoxy" or "haloalkyloxy" represents a haloalkyl group as defined above with the indicated number of carbon atoms attached through an oxygen bridge. For example, "$C_1$ to $C_6$ haloalkoxy" or "$C_{1-6}$ haloalkoxy", is intended to include $C_1$, $C_2$, $C_3$, $C_4$, $C_5$, and $C_6$ haloalkoxy groups. Examples of haloalkoxy include, but are not limited to, trifluoromethoxy, 2,2,2-trifluoroethoxy, and pentafluorothoxy. Similarly, "haloalkylthio" or "thiohaloalkoxy" represents a haloalkyl group as defined above with the indicated number of carbon atoms attached through a sulphur bridge; for example trifluoromethyl-S-, and pentafluoroethyl-S-.

The term "amino", as used herein, refers to $-NH_2$.

The term "substituted amino", as used herein, refers to the defined terms below having the suffix "amino" such as "arylamino", "alkylamino", "arylamino", etc.

The term "alkoxycarbonyl", as used herein, refers to an alkoxy group attached to the parent molecular moiety through a carbonyl group.

The term "alkoxycarbonylamino", as used herein, refers to an -NHR wherein R is an alkoxycarbonyl group.

The term "alkylamino", as used herein refers to -NHR, wherein R is an alkyl group.

The term "alkylcarbonyl", as used herein, refers to an alkyl group attached to the parent molecular moiety through a carbonyl group.

The term "alkylcarbonylamino", as used herein, refers to -NHR wherein R is an alkylcarbonyl group.

The term "aminosulfonyl", as used herein, refers to $-SO_2NH_2$.

The term "arylalkyl", as used herein, refers to an alkyl group substituted with one, two, or three aryl groups.

The term "arylamino", as used herein, refers to -NHR wherein R is an aryl group.

The term "arylcarbonyl", as used herein, refers to an aryl group attached to the parent molecular moiety through a carbonyl group.

The term "arylcarbonylamino", as used herein refers to -NHR wherein R is an arylcarbonyl group.

The term "cyano", as used herein, refers to -CN.

The term "cycloalkylamino", as used herein, refers to -NHR wherein R is a cycloalkyl group.

The term "cycloalkylcarbonyl", as used herein, refers to a cycloalkyl group attached to the parent molecular moiety through a carbonyl group.

The term "cycloalkylcarbonylamino", as used herein, refers to -NHR wherein R is a cycloalkylcarbonyl group.

The term "cycloalkyloxy", as used herein, refers to a cycloalkyl group attached to the parent molecular moiety through an oxygen atom.

The term "dialkylamino", as used herein, refers to $NR_2$, wherein each R is an alkyl group. The two alkyl groups are the same or different.

The term "haloalkoxy", as used herein, refers to a haloalkyl group attached to the parent molecular moiety through an oxygen atom.

The term "haloalkyl", as used herein, refers to an alkyl group substituted by one, two, three, or four halogen atoms.

The term "haloalkylamino", as used herein, refers to -NHR wherein R is a haloalkyl group.

The term "carbonyl" refers to C(=O) or C(O).

The term "carboxyl" or "carboxyl" refers to C(=O)OH.

The terms "carboxyl ester" and "oxycarbonyl" refer to the groups -C(O)O-alkyl, -C(O)O-substituted alkyl, -C(O)O-alkenyl, -C(O)O-substituted alkenyl, -C(O)O-alkynyl, C(O)O-substituted alkynyl, -C(O)O-cycloalkyl, -C(O)O-substituted cycloalkyl, -C(O)O-aryl, -C(O)O-substituted aryl, -C(O)O-heteroaryl, -C(O)O-substituted heteroaryl, -C(O)O-heterocyclic, and -C(O)O-substituted heterocyclic.

The term "aminoacyl" or "amide", or the prefix "carbamoyl", "carboxamide", "substituted carbamoyl" or "substituted carboxamide" refers to the group -C(O)NRR where each R is independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, heteroaryl, substituted heteroaryl, heterocyclic, and substituted heterocyclic.

The term "haloalkylcarbonyl", as used herein, refers to a haloalkyl group attached to the parent molecular moiety through a carbonyl group.

The term "haloalkylcarbonylamino", as used herein, refers to -NHR wherein R is a haloalkylcarbonyl group.

The terms "alkylcarbonyl" refer to an alkyl or substituted alkyl bonded to a carbonyl.

The term "alkoxycarbonyl", as used herein, refers to an alkoxy group attached to the parent molecular moiety through a carbonyl group.

The term "hydroxy" or "hydroxyl" refers to OH.

As used herein the term "thiol" means -SH. A thiol may be substituted with a substituent disclosed herein, in particular alkyl(thioalkyl), aryl(thioaryl), or alkoxy(thioalkoxy).

As used herein the term "sulfonyl", used alone or linked to other terms such as alkylsulfonyl or arylsulfonyl, refers to the divalent radicals $-SO_2-$. In aspects of the invention a sulfonyl group, the sulfonyl group may be attached to a substituted or unsubstituted hydroxyl, alkyl group, ether group, alkenyl group, alkynyl group, aryl group, cycloalkyl group, cycloalkenyl group, cycloalkynyl group, heterocyclic group, carbohydrate, peptide, or peptide derivative.

The term "cycloalkyl" refers to cyclized alkyl groups, including mono-, bi- or poly-cyclic ring systems. "$C_3$ to $C_7$ cycloalkyl" or "$C_{3-7}$ cycloalkyl" is intended to include $C_3$, $C_4$, $C_5$, $C_6$, and $C_7$ cycloalkyl groups. Example cycloalkyl groups include, but are not limited to, cyclopropyl, cyclobutyl, cyclopentyl, cyclohexyl, and norbornyl. Branched cycloalkyl groups such as 1-methylcyclopropyl and 2-methyl cyclopropyl are included in the definition of "cycloalkyl".

As used herein, "carbocycle", "carbocyclyl", or "carbocyclic residue" is intended to mean any stable 3-, 4-, 5-, 6-, 7-, or 8-membered monocyclic or bicyclic or 7-, 8-, 9-, 10-, 11-, 12-, or 13-membered bicyclic or tricyclic hydrocarbon ring, any of which may be saturated, partially unsaturated, unsaturated or aromatic. Examples of such carbocyclyls include, but are not limited to, cyclopropyl, cyclobutyl, cyclobutenyl, cyclopentyl, cyclopentenyl, cyclohexyl, cycloheptenyl, cycloheptyl, cycloheptenyl, adamantyl, cyclooctyl, cyclooctenyl, cyclooctadienyl, [3.3.0]bicyclooctane, [4.3.0]bicyclononane, [4.4.0]bicyclodecane (decalin), [2.2.2]bicyclooctane, fluorenyl, phenyl, naphthyl, indanyl, adamantyl, anthracenyl, and tetrahydronaphthyl (tetralin). As shown above, bridged rings are also included in the definition of carbocyclyl (e.g., [2.2.2]bicyclooctane). Preferred carbocyclyls, unless otherwise specified, are cyclopropyl, cyclobutyl, cyclopentyl, cyclohexyl, phenyl, and indanyl. When the term "carbocyclyl" is used, it is intended to include "aryl". A bridged ring occurs when one or more carbon atoms link two non-adjacent carbon atoms. Preferred bridges are one or two carbon atoms. It is noted that a bridge always converts a monocyclic ring into a tricyclic ring. When a ring is bridged, the substituents recited for the ring may also be present on the bridge.

As used herein, the term "bicyclic carbocyclyl" or "bicyclic carbocyclic group" is intended to mean a stable 9- or 10-membered carbocyclic ring system that contains two fused rings and consists of carbon atoms. Of the two fused rings, one ring is a benzo ring fused to a second ring; and the second ring is a 5- or 6-membered carbon ring which is saturated, partially unsaturated, or unsaturated. The bicyclic carbocyclic group may be attached to its pendant group at any carbon atom which results in a stable structure. The bicyclic carbocyclic group described herein may be substituted on any carbon if the resulting compound is stable. Examples of a bicyclic carbocyclic group are, but not limited to, naphthyl, 1,2-dihydronaphthyl, 1,2,3,4-tetrahydronaphthyl, and indanyl.

"Aryl" groups refer to monocyclic or polycyclic aromatic hydrocarbons, including, for example, phenyl, naphthyl, and phenanthranyl. Aryl moieties are well known and described, for example, in Lewis, R.J., ed., Hawley's Condensed Chemical Dictionary, 13th Edition, John Wiley & Sons, Inc., New York (1997).

"$C_6$ or Cio aryl" or "$C_{6-10}$ aryl" refers to phenyl and naphthyl. Unless otherwise specified, "aryl", "$C_6$ or Cio aryl" or "$C_{6-10}$ aryl" or "aromatic residue" may be unsubstituted or substituted with 1 to 5 groups, preferably 1 to 3 groups, OH, $OCH_3$, Cl, F, Br, I, CN, $NO_2$, $NH_2$, $N(CH_3)H$, $N(CH_3)_2$, $CF_3$, $OCF_3$, $C(=O)CH_3$, $SCH_3$, $S(=O)CH_3$, $S(=O)_2CH_3$, $CH_3$, $CH_2CH_3$, $CO_2H$, and $CO_2CH_3$.

The term "benzyl", as used herein, refers to a methyl group on which one of the hydrogen atoms is replaced by a phenyl group, wherein said phenyl group may optionally be substituted with 1 to 5 groups, preferably 1 to 3 groups, OH, $OCH_3$, Cl, F, Br, I, CN, $NO_2$, $NH_2$, $N(CH_3)H$, $N(CH_3)_2$, $CF_3$, $OCF_3$, $C(=O)CH_3$, $SCH_3$, $S(=O)CH_3$, $S(=O)_2CH_3$, $CH_3$, $CH_2CH_3$, $CO_2H$, and $CO_2CH_3$.

As used herein, the term "heterocycle", "heterocyclyl" or "heterocyclic ring" is intended to mean a stable 3-, 4-, 5-, 6-, or 7-membered monocyclic or bicyclic or 7-, 8-, 9-, 10-, 11-, 12-, 13-, or 14-membered polycyclic heterocyclic ring that is saturated, partially unsaturated, or fully unsaturated, and that contains carbon atoms and 1, 2, 3 or 4 heteroatoms independently selected from the group consisting of N, O and S; and including any polycyclic group in which any of the above-defined heterocyclic rings is fused to a benzene ring. The nitrogen and sulfur heteroatoms may optionally be oxidized (*i.e.,* $N \rightarrow O$ and $S(O)_p$, wherein p is 0, 1 or 2). The nitrogen atom may be substituted or unsubstituted (*i.e.,* N or NR wherein R is H or another substituent, if defined). The heterocyclic ring may be attached to its pendant group at any heteroatom or carbon atom that results in a stable structure. The heterocyclic rings described herein may be substituted on carbon or on a nitrogen atom if the resulting compound is stable. A nitrogen in the heterocyclyl may optionally be quaternized. It is preferred that when the total number of S and O atoms in the heterocyclyl exceeds 1, then these heteroatoms are not adjacent to one another. It is preferred that the total number of S and O atoms in the heterocyclyl is not more than 1. When the term "heterocyclyl" is used, it is intended to include heteroaryl.

Examples of heterocyclyls include, but are not limited to, acridinyl, azetidinyl, azocinyl, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2H,6H-1,5,2-dithiazinyl, dihydrofuro[2,3-*b*]tetrahydrofuran, furanyl, furazanyl, imidazolidinyl, imidazolinyl, imidazolyl, 1*H*-indazolyl, imidazolopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinylperimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4*H*-quinolizinyl, quinoxalinyl, quinuclidinyl, tetrazolyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridinyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl. Also included are fused ring and spiro compounds containing, for example, the above heterocyclyls.

Examples of 5- to 10-membered heterocyclyls include, but are not limited to, pyridinyl, furanyl, thienyl, pyrrolyl, pyrazolyl, pyrazinyl, piperazinyl, piperidinyl, imidazolyl, imidazolidinyl, indolyl, tetrazolyl, isoxazolyl, morpholinyl, oxazolyl, oxadiazolyl, oxazolidinyl, tetrahydrofuranyl, thiadiazinyl, thiadiazolyl, thiazolyl, triazinyl, triazolyl, benzimidazolyl, 1*H*-indazolyl, benzofuranyl, benzothiofuranyl, benztetrazolyl, benzotriazolyl, benzisoxazolyl, benzoxazolyl, oxindolyl, benzoxazolinyl, benzthiazolyl, benzisothiazolyl, isatinoyl, isoquinolinyl, octahydroisoquinolinyl, tetrahydroisoquinolinyl, tetrahydroquinolinyl, isoxazolopyridinyl, quinazolinyl, quinolinyl, isothiazolopyridinyl, thiazolopyridinyl, oxazolopyridinyl, imidazolopyridinyl, and pyrazolopyridinyl.

Examples of 5- to 6-membered heterocyclyls include, but are not limited to, pyridinyl, furanyl, thienyl, pyrrolyl, pyrazolyl, pyrazinyl, piperazinyl, piperidinyl, imidazolyl, imidazolidinyl, indolyl, tetrazolyl, isoxazolyl, morpholinyl, oxazolyl, oxadiazolyl, oxazolidinyl, tetrahydrofuranyl, thiadiazinyl, thiadiazolyl, thiazolyl, triazinyl, and triazolyl. Also included are fused ring and spiro compounds containing, for example, the above heterocyclyls.

As used herein, the term "bicyclic heterocyclyl" "bicyclic heterocyclyl" or "bicyclic heterocyclic group" is intended to mean a stable 9- or 10-membered heterocyclic ring system which contains two fused rings and consists of carbon atoms and 1, 2, 3, or 4 heteroatoms independently selected from the group consisting of N, O and S. Of the two fused rings, one ring is a 5- or 6-membered monocyclic aromatic ring comprising a 5-membered heteroaryl ring, a 6-membered heteroaryl ring or a benzo ring, each fused to a second ring. The second ring is a 5- or 6-membered monocyclic ring which is saturated, partially unsaturated, or unsaturated, and comprises a 5-membered heterocyclyl, a 6-membered heterocyclyl or a carbocyclyl (provided the first ring is not benzo when the second ring is a carbocyclyl).

The bicyclic heterocyclic group may be attached to its pendant group at any heteroatom or carbon atom which results in a stable structure. The bicyclic heterocyclic group described herein may be substituted on carbon or on a nitrogen atom if the resulting compound is stable. It is preferred that when the total number of S and O atoms in the heterocyclyl exceeds 1, then these heteroatoms are not adjacent to one another. It is preferred that the total number of S and O atoms in the heterocyclyl is not more than 1.

Examples of a bicyclic heterocyclic group are, but not limited to, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydroquinolinyl, 2,3-dihydrobenzofuranyl, chromanyl, 1,2,3,4-tetrahydroquinoxalinyl, and 1,2,3,4-tetrahydroquinazolinyl.

As used herein, the term "aromatic heterocyclic group" or "heteroaryl" is intended to mean stable monocyclic and polycyclic aromatic hydrocarbons that include at least one heteroatom ring member such as sulfur, oxygen, or nitrogen. Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrroyl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, purinyl, carbazolyl, benzimidazolyl, indolinyl, benzodioxolanyl, and benzodioxane. Heteroaryl groups are substituted or unsubstituted. The nitrogen atom is substituted or unsubstituted *(i.e.,* N or NR wherein R is H or another substituent, if defined). The nitrogen and sulfur heteroatoms may optionally be oxidized *(i.e.,* $N\rightarrow O$ and $S(O)_p$, wherein p is 0, 1 or 2).

Bridged rings are also included in the definition of heterocyclyl. A bridged ring occurs when one or more atoms *(i.e.,* C, O, N, or S) link two non-adjacent carbon or nitrogen atoms. Examples of bridged rings include, but are not limited to, one carbon atom, two carbon atoms, one nitrogen atom, two nitrogen atoms, and a carbon-nitrogen group. It is noted that a bridge always converts a monocyclic ring into a tricyclic ring. When a ring is bridged, the substituents recited for the ring may also be present on the bridge.

The term "counterion" is used to represent a negatively charged species such as chloride, bromide, hydroxide, acetate, and sulfate.

When a dotted ring is used within a ring structure, this indicates that the ring structure may be saturated, partially saturated or unsaturated.

As referred to herein, the term "substituted" means that at least one hydrogen atom is replaced with a non-hydrogen group, provided that normal valencies are maintained and that the substitution results in a stable compound. When a substituent is keto *(i.e.,* =O), then 2 hydrogens on the atom are replaced. Keto substituents are not present on aromatic moieties. When a ring system *(e.g.,* carbocyclic or heterocyclic) is said to be substituted with a carbonyl group or a double bond, it is intended that the carbonyl group or double bond be part *(i.e.,* within) of the ring. Ring double

bonds, as used herein, are double bonds that are formed between two adjacent ring atoms *(e.g.,* C=C, C=N, or N=N).

In cases wherein there are nitrogen atoms *(e.g.,* amines) on compounds of the present invention, these may be converted to N-oxides by treatment with an oxidizing agent *(e.g.,* mCPBA and/or hydrogen peroxides) to afford other compounds of this invention. Thus, shown and claimed nitrogen atoms are considered to cover both the shown nitrogen and its N-oxide ($N\rightarrow O$) derivative.

When any variable occurs more than one time in any constituent or formula for a compound, its definition at each occurrence is independent of its definition at every other occurrence. Thus, for example, if a group is shown to be substituted with 0-3 R groups, then said group may optionally be substituted with up to three R groups, and at each occurrence R is selected independently from the definition of R. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom in which such substituent is bonded to the rest of the compound of a given formula, then such substituent may be bonded via any atom in such substituent. Combinations of substituents and/or variables are permissible only if such combinations result in stable compounds.

The phrase "pharmaceutically acceptable" is employed herein to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 18th Edition, Mack Publishing Company, Easton, PA (1990), the disclosure of which is hereby incorporated by reference.

In addition, compounds of formula I may have prodrug forms. Any compound that will be converted *in vivo* to provide the bioactive agent *(i.e.,* a compound of formula I) is a prodrug. Various forms of prodrugs are well known in the art. For examples of such prodrug derivatives, see:

a) Bundgaard, H., ed., Design of Prodrugs, Elsevier (1985), and Widder, K. et al., eds., Methods in Enzymology, 112:309-396, Academic Press (1985);
b) Bundgaard, H., Chapter 5: "Design and Application of Prodrugs", A Textbook of Drug Design and Development, pp. 113-191, Krosgaard-Larsen, P. et al., eds., Harwood Academic Publishers (1991);
c) Bundgaard, H., Adv. Drug Deliv. Rev., 8:1-38 (1992);
d) Bundgaard, H. et al., J. Pharm. Sci., 77:285 (1988); and
e) Kakeya, N. et al., Chem. Pharm. Bull., 32:692 (1984).

Compounds containing a carboxy group can form physiologically hydrolyzable esters that serve as prodrugs by being hydrolyzed in the body to yield formula I compounds *per se.* Such prodrugs are preferably administered orally since hydrolysis in many instances occurs principally under the influence of the digestive enzymes. Parenteral administration may be used where the ester *per se* is active, or in those instances where hydrolysis occurs in the blood. Examples of physiologically hydrolyzable esters of compounds of formula I include $C_{1-6}$alkyl, $C_{1-6}$alkylbenzyl, 4-methoxybenzyl, indanyl, phthalyl, methoxymethyl, $C_{1-6}$ alkanoyloxy-$C_{1-6}$alkyl (*e.g.,* acetoxymethyl, pivaloyloxymethyl or propionyloxymethyl), $C_{1-6}$alkoxycarbonyloxy-$C_{1-6}$alkyl *(e.g.,* methoxycarbonyl-oxymethyl or ethoxycarbonyloxymethyl, glycyloxymethyl, phenylglycyloxymethyl, (5-methyl-2-oxo-1,3-dioxolen-4-yl)-methyl), and other well known physiologically hydrolyzable esters used, for example, in the penicillin and cephalosporin arts. Such esters may be prepared by conventional techniques known in the art.

Preparation of prodrugs is well known in the art and described in, for example, King, F.D., ed., Medicinal Chemistry: Principles and Practice, The Royal Society of Chemistry, Cambridge, UK (1994); Testa, B. et al., Hydrolysis in Drug and Prodrug Metabolism. Chemistry, Biochemistry and Enzymology, VCHA and Wiley-VCH, Zurich, Switzerland (2003); Wermuth, C.G., ed., The Practice of Medicinal Chemistry, Academic Press, San Diego, CA (1999).

The present invention is intended to include all isotopes of atoms occurring in the present compounds. Isotopes include those atoms having the same atomic number but different mass numbers. By way of general example and without limitation, isotopes of hydrogen include deuterium and tritium. Deuterium has one proton and one neutron in its nucleus and that has twice the mass of ordinary hydrogen. Deuterium can be represented by symbols such as "$^{2}H$" or "D". The term "deuterated" herein, by itself or used to modify a compound or group, refers to replacement of one or more hydrogen atom(s), which is attached to carbon(s), with a deuterium atom. Isotopes of carbon include $^{13}C$ and $^{14}C$.

Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds have a variety of potential uses, *e.g.,* as standards and reagents in determining the ability of a potential pharmaceutical compound to bind to target proteins or receptors, or for imaging compounds of this invention bound to biological receptors *in vivo* or *in vitro.*

"Stable compound" and "stable structure" are meant to indicate a compound that is sufficiently robust to survive isolation to a useful degree of purity from a reaction mixture, and formulation into an efficacious therapeutic agent. It is preferred that compounds of the present invention do not contain a N-halo, $S(O)_2H$, or S(O)H group.

The term "solvate" means a physical association of a compound of this invention with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvent molecules in the solvate may be present in a regular arrangement and/or a non-ordered arrangement. The solvate may comprise either a stoichiometric or nonstoichiometric amount of the solvent molecules. "Solvate" encompasses both solution-phase and isolable solvates. Exemplary solvates include, but are not limited to, hydrates, ethanolates, methanolates, and isopropanolates. Methods of solvation are generally known in the art.

Abbreviations as used herein, are defined as follows: "1 x" for once, "2 x" for twice, "3 x" for thrice, "°C" for degrees Celsius, "eq" for equivalent or equivalents, "g" for gram or grams, "mg" for milligram or milligrams, "L" for liter or liters, "mL" for milliliter or milliliters, "μL" for microliter or microliters, "N" for normal, "M" for molar, "mmol" for millimole or millimoles, "min" for minute or minutes, "h" for hour or hours, "rt" for room temperature, "RT" for retention time, "RBF" for round bottom flask, "atm" for atmosphere, "psi" for pounds per square inch, "conc." for concentrate, "RCM" for ring-closing metathesis, "sat" or "sat'd" for saturated, "SFC" for supercritical fluid chromatography "MW" for molecular weight, "mp" for melting point, "ee" for enantiomeric excess, "MS" or "Mass Spec" for mass spectrometry, "ESI" for electrospray ionization mass spectroscopy, "HR" for high resolution, "HRMS" for high resolution mass spectrometry, "LCMS" for liquid chromatography mass spectrometry, "HPLC" for high pressure liquid chromatography, "RP HPLC" for reverse phase HPLC, "TLC" or "tlc" for thin layer chromatography, "NMR" for nuclear magnetic resonance spectroscopy, "nOe" for nuclear Overhauser effect spectroscopy, "$^{1}H$" for proton, "δ" for delta, "s" for singlet, "d" for doublet, "t" for triplet, "q" for quartet, "m" for multiplet, "br" for broad, "Hz" for hertz, and "α", "β", "R", "S", "E", and "Z" are stereochemical designations familiar to one skilled in the art.

| | |
|---|---|
| Me | methyl |
| Et | ethyl |
| Pr | propyl |
| *i*-Pr | isopropyl |
| Bu | butyl |
| *i*-Bu | isobutyl |
| *t*-Bu | *tert*-butyl |
| Ph | phenyl |
| Bn | benzyl |
| Boc or BOC | *tert*-butyloxycarbonyl |
| $Boc_2O$ | di-*tert*-butyl dicarbonate |
| AcOH or HOAc | acetic acid |
| $AlCl_3$ | aluminum chloride |
| AIBN | azobisisobutyronitrile |
| aqueous | aq |
| $BBr_3$ | boron tribromide |
| $BCl_3$ | boron trichloride |
| BEMP | 2-*tert*-butylimino-2-diethylamino-1,3-dimethylperhydro-1,3,2-diazaphosphorine |
| BOP reagent | benzotriazol-1-yloxytris(dimethylamino)phosphonium hexafluorophosphate |

| | |
|---|---|
| Burgess reagent | 1-methoxy-N-triethylammoniosulfonyl-methanimidate |
| Cbz | carbobenzyloxy |
| DCM or $CH_2Cl_2$ | dichloromethane |
| $CH_3CN$ or ACN | acetonitrile |
| $CDCl_3$ | deutero-chloroform |
| $CHCl_3$ | chloroform |
| mCPBA or m-CPBA | *meta*-chloroperbenzoic acid |
| $Cs_2CO_3$ | cesium carbonate |
| $Cu(OAc)_2$ | copper (II) acetate |
| CuI | copper(I) iodide |
| $CuSO_4$ | copper(II) sulfate |
| $Cy_2NMe$ | N-cyclohexyl-N-methylcyclohexanamine |
| DBU | 1,8-diazabicyclo[5.4.0]undec-7-ene |
| DCE | 1,2-dichloroethane |
| DEA | diethylamine |
| Dess-Martin | 1,1,1-tris(acetyloxy)-1,1-dihydro-1,2-beniziodoxol-3-(1H)-one |
| DIC or DIPCDI | diisopropylcarbodiimide |
| DIEA, DIPEA or | diisopropylethylamine |
| Hunig's base DMAP | 4-dimethylaminopyridine |
| DME | 1,2-dimethoxyethane |
| DMF | dimethyl formamide |
| DMSO | dimethyl sulfoxide |
| cDNA | complimentary DNA |
| Dppp | (R)-(+)-1,2-bis(diphenylphosphino)propane |
| DuPhos | (+)-1,2-bis((2S,5S)-2,5-diethylphospholano)benzene |
| EDC | *N*-(3-dimethylaminopropyl)-*N'*-ethylcarbodiimide |
| EDCI | *N*-(3-dimethylaminopropyl)-*N'*-ethylcarbodiimide hydrochloride |
| EDTA | ethylenediaminetetraacetic acid |
| (*S,S*)-EtDuPhosRh(I) | (+)-1,2-bis((2S,5S)-2,5-diethylphospholano)benzene(1,5-cyclooctadiene)rhodium(I) trifluoromethanesulfonate |
| $Et_3N$ or TEA | triethylamine |
| EtOAc | ethyl acetate |
| $Et_2O$ | diethyl ether |
| EtOH | ethanol |
| GMF | glass microfiber filter |
| Grubbs II | (1,3-bis(2,4,6-trimethylphenyl)-2-imidazolidinylidene)dichloro (phenylmethylene)(triycyclohexylphosphine)ruthenium |
| HCl | hydrochloric acid |
| HATU | O-(7-azabenzotriazol-1-yl)-N,N,N',N'-tetramethyluronium hexafluorophosphate |
| HEPES | 4-(2-hydroxyethyl)piperazine-1-ethanesulfonic acid |
| Hex | hexane |
| HOBt or HOBT | 1-hydroxybenzotriazole |
| $H_2O_2$ | hydrogen peroxide |
| $H_2SO_4$ | sulfuric acid |
| IBX | 2-iodoxybenzoic acid |
| $InCl_3$ | Indium(III) chloride |
| Jones reagent | $CrO_3$ in aqueous $H_2SO_4$, 2 M |
| $K_2CO_3$ | potassium carbonate |
| $K_2HPO_4$ | potassium phosphate dibasic |
| $K_3PO_4$ | potassium phosphate tribasic |
| KOAc | potassium acetate |
| $K_3PO_4$ | potassium phosphate |
| LAH | lithium aluminum hydride |
| LG | leaving group |
| LiOH | lithium hydroxide |
| MeOH | methanol |
| $MgSO_4$ | magnesium sulfate |
| MsOH or MSA | methylsulfonic acid |

| | |
|---|---|
| NaCl | sodium chloride |
| NaH | sodium hydride |
| $NaHCO_3$ | sodium bicarbonate |
| $Na_2CO_3$ | sodium carbonate |
| NaOH | sodium hydroxide |
| $Na_2SO_3$ | sodium sulfite |
| $Na_2SO_4$ | sodium sulfate |
| NBS | N-bromosuccinimide |
| NCS | N-chlorosuccinimide |
| $NH_3$ | ammonia |
| $NH_4Cl$ | ammonium chloride |
| $NH_4OH$ | ammonium hydroxide |
| $NH_4COOH$ | ammonium formate |
| NMM | N-methylmorpholine |
| OTf | triflate or trifluoromethanesulfonate |
| $Pd_2(dba)_3$ | tris(dibenzylideneacetone)dipalladium(0) |
| $Pd(OAc)_2$ | palladium(II) acetate |
| Pd/C | palladium on carbon |
| $Pd(dppf)Cl_2$ | [1,1'-bis(diphenylphosphino)-ferrocene]dichloropalladium(II) |
| $Ph_3PCl_2$ | triphenylphosphine dichloride |
| PG | protecting group |
| $POCl_3$ | phosphorus oxychloride |
| i-PrOH or IPA | isopropanol |
| PS | Polystyrene |
| rt | room temperature |
| SEM-Cl | 2-(trimethysilyl)ethoxymethyl chloride |
| $SiO_2$ | silica oxide |
| $SnCl_2$ | tin(II) chloride |
| TBAI | tetra-*n*-butylammonium iodide |
| TBN | *t*-butyl nitrite |
| TFA | trifluoroacetic acid |
| THF | tetrahydrofuran |
| $TMSCHN_2$ | trimethylsilyldiazomethane |
| T3P® | propane phosphonic acid anhydride |
| TRIS | tris (hydroxymethyl) aminomethane |
| pTsOH | p-toluenesulfonic acid |

The compounds of the present invention can be prepared in a number of ways known to one skilled in the art of organic synthesis, which are described in more detail in Section VI.

## IV. BIOLOGY

While blood coagulation is essential to the regulation of an organism's hemostasis, it is also involved in many pathological conditions. In thrombosis, a blood clot, or thrombus, may form and obstruct circulation locally, causing ischemia and organ damage. Alternatively, in a process known as embolism, the clot may dislodge and subsequently become trapped in a distal vessel, where it again causes ischemia and organ damage. Diseases arising from pathological thrombus formation are collectively referred to as thromboembolic disorders and include acute coronary syndrome, unstable angina, myocardial infarction, atrial fibrillation, thrombosis in the cavity of the heart, ischemic stroke, deep vein thrombosis, peripheral occlusive arterial disease, transient ischemic attack, and pulmonary embolism. In addition, thrombosis occurs on artificial surfaces in contact with blood, including catheters, stents, artificial heart valves, and hemodialysis membranes.

Some conditions contribute to the risk of developing thrombosis. For example, alterations of the vessel wall, changes in the flow of blood, and alterations in the composition of the vascular compartment. These risk factors are collectively known as Virchow's triad. (Colman, R.W. et al., eds., Hemostasis and Thrombosis, Basic Principles and Clinical Practice, Fifth Edition, p. 853, Lippincott Williams & Wilkins (2006)).

Antithrombotic agents are frequently given to patients at risk of developing thromboembolic disease because of the presence of one or more predisposing risk factors from Virchow's triad to prevent formation of an occlusive thrombus (primary prevention). For example, in an orthopedic surgery setting *(e.g.,* hip and knee replacement), an

antithrombotic agent is frequently administered prior to a surgical procedure. The antithrombotic agent counterbalances the prothrombotic stimulus exerted by vascular flow alterations (stasis), potential surgical vessel wall injury, as well as changes in the composition of the blood due to the acute phase response related to surgery. Another example of the use of an antithrombotic agent for primary prevention is dosing with aspirin, a platelet activation inhibitor, in patients at risk for developing thrombotic cardiovascular disease. Well recognized risk factors in this setting include age, male gender, hypertension, diabetes mellitus, lipid alterations, and obesity.

Antithrombotic agents are also indicated for secondary prevention, following an initial thrombotic episode. For example, patients with mutations in factor V (also known as factor V Leiden) and additional risk factors *(e.g.,* pregnancy), are dosed with anticoagulants to prevent the reoccurrence of venous thrombosis. Another example entails secondary prevention of cardiovascular events in patients with a history of acute myocardial infarction or acute coronary syndrome. In a clinical setting, a combination of aspirin and clopidogrel (or other thienopyridines) may be used to prevent a second thrombotic event.

Antithrombotic agents are also given to treat the disease state *(i.e.,* by arresting its development) after it has already started. For example, patients presenting with deep vein thrombosis are treated with anticoagulants *(i.e.,* heparin, warfarin, or LMWH) to prevent further growth of the venous occlusion. Over time, these agents also cause a regression of the disease state because the balance between prothrombotic factors and anticoagulant/profibrinolytic pathways is changed in favor of the latter. Examples on the arterial vascular bed include the treatment of patients with acute myocardial infarction or acute coronary syndrome with aspirin and clopidogrel to prevent further growth of vascular occlusions and eventually leading to a regression of thrombotic occlusions.

Thus, antithrombotic agents are used widely for primary and secondary prevention *(i.e.,* prophylaxis or risk reduction) of thromboembolic disorders, as well as treatment of an already existing thrombotic process. Drugs that inhibit blood coagulation, or anticoagulants, are "pivotal agents for prevention and treatment of thromboembolic disorders" (Hirsh, J. et al., Blood, 105:453-463 (2005)).

An alternative way of initiation of coagulation is operative when blood is exposed to artificial surfaces *(e.g.,* during hemodialysis, "on-pump" cardiovascular surgery, vessel grafts, bacterial sepsis), on cell surfaces, cellular receptors, cell debris, DNA, RNA, and extracellular matrices. This process is also termed contact activation. Surface absorption of factor XII leads to a conformational change in the factor XII molecule, thereby facilitating activation to proteolytic active factor XII molecules (factor XIIa and factor XIIf). Factor XIIa (or XIIf) has a number of target proteins, including plasma prekallikrein and factor XI. Active plasma kallikrein further activates factor XII, leading to an amplification of contact activation. Alternatively, the serine protease prolylcarboxylpeptidase can activate plasma kallikrein complexed with high molecular weight kininogen in a multiprotein complex formed on the surface of cells and matrices (Shariat-Madar et al., Blood, 108:192-199 (2006)). Contact activation is a surface mediated process responsible in part for the regulation of thrombosis and inflammation, and is mediated, at least in part, by fibrinolytic-, complement-, kininogen/kinin-, and other humoral and cellular pathways (for review, Coleman, R., "Contact Activation Pathway", Hemostasis and Thrombosis, pp. 103-122, Lippincott Williams & Wilkins (2001); Schmaier, A.H., "Contact Activation", Thrombosis and Hemorrhage, pp. 105-128 (1998)). The biological relevance of the contact activation system for thromboembolic diseases is supported by the phenotype of factor XII deficient mice. More specifically, factor XII deficient mice were protected from thrombotic vascular occlusion in several thrombosis models as well as stroke models and the phenotype of the XII deficient mice was identical to XI deficient mice (Renne et al., J. Exp. Med., 202:271-281 (2005); Kleinschmitz et al., J. Exp. Med., 203:513-518 (2006)). The fact that factor XI is downstream from factor XIIa, combined with the identical phenotype of the XII and XI deficient mice suggest that the contact activation system could play a major role in factor XI activation *in vivo.*

Factor XI is a zymogen of a trypsin-like serine protease and is present in plasma at a relatively low concentration. Proteolytic activation at an internal R369-I370 bond yields a heavy chain (369 amino acids) and a light chain (238 amino acids). The latter contains a typical trypsin-like catalytic triad (H413, D464, and S557). Activation of factor XI by thrombin is believed to occur on negatively charged surfaces, most likely on the surface of activated platelets. Platelets contain high affinity (0.8 nM) specific sites (130-500/platelet) for activated factor XI. After activation, factor XIa remains surface bound and recognizes factor IX as its normal macromolecular substrate. (Galiani, D., Trends Cardiovasc. Med., 10:198-204 (2000)).

In addition to the feedback activation mechanisms described above, thrombin activates thrombin activated fibrinolysis inhibitor (TAFI), a plasma carboxypeptidase that cleaves C-terminal lysine and arginine residues on fibrin, reducing the ability of fibrin to enhance tissue-type plasminogen activator (tPA) dependent plasminogen activation. In the presence of antibodies to FXIa, clot lysis can occur more rapidly independent of plasma TAFI concentration. (Bouma, B.N. et al., Thromb. Res., 101:329-354 (2001).) Thus, inhibitors of factor XIa are expected to be anticoagulant and profibrinolytic.

Further evidence for the anti-thromboembolic effects of targeting factor XI is derived from mice deficient in factor XI. It has been demonstrated that complete fXI deficiency protected mice from ferric chloride ($FeCl_3$)-induced carotid artery thrombosis (Rosen et al., Thromb. Haemost., 87:774-777 (2002); Wang et al., J. Thromb. Haemost., 3:695-702 (2005)). Also, factor XI deficiency rescues the perinatal lethal phenotype of complete protein C deficiency

(Chan et al., Amer. J. Pathology, 158:469-479 (2001)). Furthermore, baboon cross-reactive, function blocking antibodies to human factor XI protect against baboon arterial - venous shunt thrombosis (Gruber et al., Blood, 102:953-955 (2003)). Evidence for an antithrombotic effect of small molecule inhibitors of factor XIa is also disclosed in published U.S. Patent Publication No. 2004/0180855 A1. Taken together, these studies suggest that targeting factor XI will reduce the propensity for thrombotic and thromboembolic diseases.

Genetic evidence indicates that factor XI is not required for normal homeostasis, implying a superior safety profile of the factor XI mechanism compared to competing antithrombotic mechanisms. In contrast to hemophilia A (factor VIII deficiency) or hemophilia B (factor IX deficiency), mutations of the factor XI gene causing factor XI deficiency (hemophilia C) result in only a mild to moderate bleeding diathesis characterized primarily by postoperative or posttraumatic, but rarely spontaneous hemorrhage. Postoperative bleeding occurs mostly in tissue with high concentrations of endogenous fibrinolytic activity *(e.g.,* oral cavity, and urogenital system). The majority of the cases are fortuitously identified by preoperative prolongation of aPTT (intrinsic system) without any prior bleeding history.

The increased safety of inhibition of XIa as an anticoagulation therapy is further supported by the fact that Factor XI knock-out mice, which have no detectable factor XI protein, undergo normal development, and have a normal life span. No evidence for spontaneous bleeding has been noted. The aPTT (intrinsic system) is prolonged in a gene dose-dependent fashion. Interestingly, even after severe stimulation of the coagulation system (tail transection), the bleeding time is not significantly prolonged compared to wild-type and heterozygous litter mates. (Gailani, D., Frontiers in Bioscience, 6:201-207 (2001); Gailani, D. et al., Blood Coagulation and Fibrinolysis, 8:134-144 (1997).) Taken together, these observations suggest that high levels of inhibition of factor XIa should be well tolerated. This is in contrast to gene targeting experiments with other coagulation factors, excluding factor XII.

*In vivo* activation of factor XI can be determined by complex formation with either C1 inhibitor or alpha 1 antitrypsin. In a study of 50 patients with acute myocardial infarction (AMI), approximately 25% of the patients had values above the upper normal range of the complex ELISA. This study can be viewed as evidence that at least in a subpopulation of patients with AMI, factor XI activation contributes to thrombin formation (Minnema, M.C. et al., Arterioscler. Thromb. Vasc. Biol., 20:2489-2493 (2000)). A second study establishes a positive correlation between the extent of coronary arteriosclerosis and factor XIa in complex with alpha 1 antitrypsin (Murakami, T. et al., Arterioscler. Thromb. Vasc. Biol., 15:1107-1113 (1995)). In another study, Factor XI levels above the 90th percentile in patients were associated with a 2.2-fold increased risk for venous thrombosis (Meijers, J.C.M. et al., N. Engl. J. Med., 342:696-701 (2000)).

Also, it is preferred to find new compounds with improved activity in *in vitro* clotting assays, compared with known serine protease inhibitors, such as the activated partial thromboplastin time (aPTT) or prothrombin time (PT) assay. (for a description of the aPTT and PT assays see, Goodnight, S.H. et al., "Screening Tests of Hemostasis", Disorders of Thrombosis and Hemostasis: A Clinical Guide, Second Edition, pp. 41-51, McGraw-Hill, New York (2001)).

It is also desirable and preferable to find compounds with advantageous and improved characteristics compared with known serine protease inhibitors, in one or more of the following categories that are given as examples, and are not intended to be limiting: (a) pharmacokinetic properties, including oral bioavailability, half life, and clearance; (b) pharmaceutical properties; (c) dosage requirements; (d) factors that decrease blood concentration peak-to-trough characteristics; (e) factors that increase the concentration of active drug at the receptor; (f) factors that decrease the liability for clinical drug-drug interactions; (g) factors that decrease the potential for adverse side-effects, including selectivity versus other biological targets; and (h) factors that improve manufacturing costs or feasibility.

Pre-clinical studies demonstrated significant antithrombotic effects of small molecule factor XIa inhibitors in rabbit and rat model of arterial and venous thrombosis, at doses that preserved hemostasis. (Wong P.C. et al., Journal of Thrombosis and Thrombolysis, 32(2): 129-137 (Aug. 2011); Schumacher, W. et al., Journal of Thrombosis and Haemostasis, 3(Suppl. 1):P1228 (2005); Schumacher, W.A. et al., Eur. J. Pharmacol., 167-174 (2007)). Furthermore, it was observed that *in vitro* prolongation of the aPTT by specific XIa inhibitors is a good predictor of efficacy in our thrombosis models. Thus, the *in vitro* aPTT test can be used as a surrogate for efficacy *in vivo.* Pre-clinical and clinical studies using FXI antisense (ASO) has been shown to be effective in various venous and arterial thrombosis models, comparable to warfarin or enoxaparin without increased bleeding (Bueller et al., DOI: 10.1056/NEJMoa1405760 (2014)).

As used herein, the term "patient" encompasses all mammalian species.

As used herein, "treating" or "treatment" cover the treatment of a disease-state in a mammal, particularly in a human, and include: (a) inhibiting the disease-state, *i.e.,* arresting it development; and/or (b) relieving the disease-state, *i.e.,* causing regression of the disease state.

As used herein, "prophylaxis" is the protective treatment of a disease state to reduce and/or minimize the risk and/or reduction in the risk of recurrence of a disease state by administering to a patient a therapeutically effective amount of at least one of the compounds of the present invention or a or a stereoisomer, a tautomer, a pharmaceutically acceptable salt, or a solvate thereof. Patients may be selected for prophylaxis therapy based on factors that are known to increase risk of suffering a clinical disease state compared to the general population. For prophylaxis treatment, conditions of the clinical disease state may or may not be presented yet. "Prophylaxis" treatment can be divided into (a) primary prophylaxis and (b) secondary prophylaxis. Primary prophylaxis is defined as treatment to reduce or minimize

the risk of a disease state in a patient that has not yet presented with a clinical disease state, whereas secondary prophylaxis is defined as minimizing or reducing the risk of a recurrence or second occurrence of the same or similar clinical disease state.

As used herein, "prevention" cover the preventive treatment of a subclinical disease-state in a mammal, particularly in a human, aimed at reducing the probability of the occurrence of a clinical disease-state. Patients are selected for preventative therapy based on factors that are known to increase risk of suffering a clinical disease state compared to the general population.

As used herein, "risk reduction" covers therapies that lower the incidence of development of a clinical disease state. As such, primary and secondary prevention therapies are examples of risk reduction.

"Therapeutically effective amount" is intended to include an amount of a compound of the present invention that is effective when administered alone or in combination to inhibit factor XIa and/or plasma kallikrein and/or to prevent or treat the disorders listed herein. When applied to a combination, the term refers to combined amounts of the active ingredients that result in the preventive or therapeutic effect, whether administered in combination, serially, or simultaneously.

The term "thrombosis", as used herein, refers to formation or presence of a thrombus (pl. thrombi); clotting within a blood vessel that may cause ischemia or infarction of tissues supplied by the vessel. The term "embolism", as used herein, refers to sudden blocking of an artery by a clot or foreign material that has been brought to its site of lodgment by the blood current. The term "thromboembolism", as used herein, refers to obstruction of a blood vessel with thrombotic material carried by the blood stream from the site of origin to plug another vessel. The term "thromboembolic disorders" entails both "thrombotic" and "embolic" disorders (defined above).

The term "thromboembolic disorders" as used herein includes arterial cardiovascular thromboembolic disorders, venous cardiovascular or cerebrovascular thromboembolic disorders, and thromboembolic disorders in the chambers of the heart or in the peripheral circulation. The term "thromboembolic disorders" as used herein also includes specific disorders selected from, but not limited to, unstable angina or other acute coronary syndromes, atrial fibrillation, first or recurrent myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis. The medical implants or devices include, but are not limited to: prosthetic valves, artificial valves, indwelling catheters, stents, blood oxygenators, shunts, vascular access ports, ventricular assist devices and artificial hearts or heart chambers, and vessel grafts. The procedures include, but are not limited to: cardiopulmonary bypass, percutaneous coronary intervention, and hemodialysis. In another embodiment, the term "thromboembolic disorders" includes acute coronary syndrome, stroke, deep vein thrombosis, and pulmonary embolism.

In another embodiment, the present invention provides a compound of the invention for use in a method for the treatment of a thromboembolic disorder, wherein the thromboembolic disorder is selected from unstable angina, an acute coronary syndrome, atrial fibrillation, myocardial infarction, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis. In another embodiment, the present invention provides a compound of the invention for use in a method for the treatment of a thromboembolic disorder, wherein the thromboembolic disorder is selected from acute coronary syndrome, stroke, venous thrombosis, atrial fibrillation, and thrombosis resulting from medical implants and devices.

In another embodiment, the present invention provides a compound of the invention for use in a method for the primary prophylaxis of a thromboembolic disorder, wherein the thromboembolic disorder is selected from unstable angina, an acute coronary syndrome, atrial fibrillation, myocardial infarction, ischemic sudden death, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis. In another embodiment, the present invention provides a compound of the invention for use in a method for the primary prophylaxis of a thromboembolic disorder, wherein the thromboembolic disorder is selected from acute coronary syndrome, stroke, venous thrombosis, and thrombosis resulting from medical implants and devices.

In another embodiment, the present invention provides a compound of the invention for use in a method for the secondary prophylaxis of a thromboembolic disorder, wherein the thromboembolic disorder is selected from unstable angina, an acute coronary syndrome, atrial fibrillation, recurrent myocardial infarction, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis. In another embodiment, the present invention provides a compound of the invention for use in a method for the secondary prophylaxis of a thromboembolic disorder, wherein the thromboembolic disorder is selected from acute coronary syndrome, stroke, atrial fibrillation and venous thrombosis.

The term "stroke", as used herein, refers to embolic stroke or atherothrombotic stroke arising from occlusive thrombosis in the carotid communis, carotid interna, or intracerebral arteries.

It is noted that thrombosis includes vessel occlusion (e.g., after a bypass) and reocclusion (e.g., during or after percutaneous transluminal coronary angioplasty). The thromboembolic disorders may result from conditions including but not limited to atherosclerosis, surgery or surgical complications, prolonged immobilization, arterial fibrillation, congenital thrombophilia, cancer, diabetes, effects of medications or hormones, and complications of pregnancy.

Thromboembolic disorders are frequently associated with patients with atherosclerosis. Risk factors for atherosclerosis include but are not limited to male gender, age, hypertension, lipid disorders, and diabetes mellitus. Risk factors for atherosclerosis are at the same time risk factors for complications of atherosclerosis, *i.e.,* thromboembolic disorders.

Similarly, arterial fibrillation is frequently associated with thromboembolic disorders. Risk factors for arterial fibrillation and subsequent thromboembolic disorders include cardiovascular disease, rheumatic heart disease, nonrheumatic mitral valve disease, hypertensive cardiovascular disease, chronic lung disease, and a variety of miscellaneous cardiac abnormalities as well as thyrotoxicosis.

Diabetes mellitus is frequently associated with atherosclerosis and thromboembolic disorders. Risk factors for the more common type 2 include but are not limited to are family history, obesity, physical inactivity, race/ethnicity, previously impaired fasting glucose or glucose tolerance test, history of gestational diabetes mellitus or delivery of a "big baby", hypertension, low HDL cholesterol, and polycystic ovary syndrome.

Risk factors for congenital thrombophilia include gain of function mutations in coagulation factors or loss of function mutations in the anticoagulant- or fibrinolytic pathways.

Thrombosis has been associated with a variety of tumor types, *e.g.,* pancreatic cancer, breast cancer, brain tumors, lung cancer, ovarian cancer, prostate cancer, gastrointestinal malignancies, and Hodgkins or non-Hodgkins lymphoma. Recent studies suggest that the frequency of cancer in patients with thrombosis reflects the frequency of a particular cancer type in the general population (Levitan, N. et al., Medicine (Baltimore), 78(5):285-291 (1999); Levine M. et al., N. Engl. J. Med., 334(11):677-681 (1996); Blom, J.W. et al., JAMA, 293(6):715-722 (2005)). Hence, the most common cancers associated with thrombosis in men are prostate, colorectal, brain, and lung cancer, and in women are breast, ovary, and lung cancer. The observed rate of venous thromboembolism (VTE) in cancer patients is significant. The varying rates of VTE between different tumor types are most likely related to the selection of the patient population. Cancer patients at risk for thrombosis may possess any or all of the following risk factors: (i) the stage of the cancer *(i.e.,* presence of metastases), (ii) the presence of central vein catheters, (iii) surgery and anticancer therapies including chemotherapy, and (iv) hormones and antiangiogenic drugs. Thus, it is common clinical practice to dose patients having advanced tumors with heparin or low molecular heparin to prevent thromboembolic disorders. A number of low molecular heparin preparations have been approved by the FDA for these indications.

There are three main clinical situations when considering the prevention of VTE in a medical cancer patient: (i) the patient is bedridden for prolonged periods of time; (ii) the ambulatory patient is receiving chemotherapy or radiation; and (iii) the patient is with indwelling central vein catheters. Unfractionated heparin (UFH) and low molecular weight heparin (LMWH) are effective antithrombotic agents in cancer patients undergoing surgery. (Mismetti, P. et al., British Journal of Surgery, 88:913-930 (2001).)

A. *In Vitro* Assays

The effectiveness of compounds of the present invention as inhibitors of the coagulation Factors XIa, VIIa, IXa, Xa, XIIa, plasma kallikrein or thrombin, can be determined using a relevant purified serine protease, respectively, and an appropriate synthetic substrate. The rate of hydrolysis of the chromogenic or fluorogenic substrate by the relevant serine protease was measured both in the absence and presence of compounds of the present invention. Hydrolysis of the substrate resulted in the release of pNA (para nitroaniline), which was monitored spectrophotometrically by measuring the increase in absorbance at 405 nm, or the release of AMC (amino methylcoumarin), which was monitored spectrofluorometrically by measuring the increase in emission at 460 nm with excitation at 380 nm. A decrease in the rate of absorbance or fluorescence change in the presence of inhibitor is indicative of enzyme inhibition. Such methods are known to one skilled in the art. The results of this assay are expressed as the inhibitory constant, $K_i$.

Factor XIa determinations were made in 50 mM HEPES buffer at pH 7.4 containing 145 mM NaCl, 5 mM KCl, and 0.1% PEG 8000 (polyethylene glycol; JT Baker or Fisher Scientific). Determinations were made using purified human Factor XIa at a final concentration of 25-200 pM (Haematologic Technologies) and the synthetic substrate S-2366 (pyroGlu-Pro-Arg-pNA; Chromogenix or AnaSpec) at a concentration of 0.0002-0.001 M.

Factor VIIa determinations were made in 0.005 M calcium chloride, 0.15 M sodium chloride, 0.05 M HEPES

buffer containing 0.1% PEG 8000 at a pH of 7.5. Determinations were made using purified human Factor VIIa (Haematologic Technologies) or recombinant human Factor VIIa (Novo Nordisk) at a final assay concentration of 0.5-10 nM, recombinant soluble tissue factor at a concentration of 10-40 nM and the synthetic substrate H-D-Ile-Pro-Arg-pNA (S-2288; Chromogenix or BMPM-2; AnaSpec) at a concentration of 0.001-0.0075 M.

Factor IXa determinations were made in 0.005 M calcium chloride, 0.1 M sodium chloride, 0.0000001 M Refludan (Berlex), 0.05 M TRIS base and 0.5% PEG 8000 at a pH of 7.4. Refludan was added to inhibit small amounts of thrombin in the commercial preparations of human Factor IXa. Determinations were made using purified human Factor IXa (Haematologic Technologies) at a final assay concentration of 20-100 nM and the synthetic substrate PCIXA2100-B (CenterChem) or Pefafluor IXa 3688 (H-D-Leu-Ph'Gly-Arg-AMC; CenterChem) at a concentration of 0.0004-0.0005 M.

Factor Xa determinations were made in 0.1 M sodium phosphate buffer at a pH of 7.5 containing 0.2 M sodium chloride and 0.5% PEG 8000. Determinations were made using purified human Factor Xa (Haematologic Technologies) at a final assay concentration of 150-1000 pM and the synthetic substrate S-2222 (Bz-Ile-Glu (gamma-OMe, 50%)-Gly-Arg-pNA; Chromogenix) at a concentration of 0.0002-0.00035 M.

Factor XIIa determinations were made in 0.05 M HEPES buffer at pH 7.4 containing 0.145 M NaCl, 0.05 M KCl, and 0.1% PEG 8000. Determinations were made using purified human Factor XIIa at a final concentration of 4 nM (American Diagnostica) and the synthetic substrate SPECTROZYME® #312 (H-D-CHT-Gly-L-Arg-pNA.2AcOH; American Diagnostica) at a concentration of 0.00015 M.

Plasma kallikrein determinations were made in 0.1 M sodium phosphate buffer at a pH of 7.5 containing 0.1-0.2 M sodium chloride and 0.5% PEG 8000. Determinations were made using purified human plasma kallikrein (Enzyme Research Laboratories) at a final assay concentration of 200 pM and the synthetic substrate S-2302 (H-(D)-Pro-Phe-Arg-pNA; Chromogenix) at a concentration of 0.00008-0.0004 M.

Thrombin determinations were made in 0.1 M sodium phosphate buffer at a pH of 7.5 containing 0.2 M sodium chloride and 0.5% PEG 8000. Determinations were made using purified human alpha thrombin (Haematologic Technologies or Enzyme Research Laboratories) at a final assay concentration of 200-250 pM and the synthetic substrate S-2366 (pyroGlu-Pro-Arg-pNA; Chromogenix or AnaSpec) at a concentration of 0.0002-0.0004 M.

The Michaelis constant, $K_m$, for substrate hydrolysis by each protease, was determined at 25 °C or 37 °C in the absence of inhibitor. Values of $K_i$ were determined by allowing the protease to react with the substrate in the presence of the inhibitor. Reactions were allowed to go for periods of 20-180 minutes (depending on the protease) and the velocities (rate of absorbance or fluorescence change versus time) were measured. The following relationships were used to calculate $K_i$ values:

$$(V_{max}*S)/(K_m+S);$$

$(v_o-v_s)/v_s = I/(K_i(1 + S/K_m))$ for a competitive inhibitor with one binding site; or
$v_s/v_o = A + ((B-A)/1 + ((IC_{50}/(I)_n)))$; and
$K_i = IC_{50}/(1 + S/K_m)$ for a competitive inhibitor

where:

- $v_o$ is the velocity of the control in the absence of inhibitor;
- $v_s$ is the velocity in the presence of inhibitor;
- $V_{max}$ is the maximum reaction velocity;
- I is the concentration of inhibitor;
- A is the minimum activity remaining (usually locked at zero);
- B is the maximum activity remaining (usually locked at 1.0);
- n is the Hill coefficient, a measure of the number and cooperativity of potential inhibitor binding sites;
- $IC_{50}$ is the concentration of inhibitor that produces 50% inhibition under the assay conditions;
- $K_i$ is the dissociation constant of the enzyme: inhibitor complex;
- S is the concentration of substrate; and
- $K_m$ is the Michaelis constant for the substrate.

The selectivity of a compound may be evaluated by taking the ratio of the $K_i$ value for a given protease with the $K_i$ value for the protease of interest *(i.e.,* selectivity for FXIa versus protease P = $K_i$ for protease P/ $K_i$ for FXIa). Compounds with selectivity ratios >20 are considered selective.

The effectiveness of compounds of the present invention as inhibitors of coagulation can be determined using a standard or modified clotting assay. An increase in the plasma clotting time in the presence of inhibitor is indicative of anticoagulation. Relative clotting time is the clotting time in the presence of an inhibitor divided by the clotting time in

the absence of an inhibitor. The results of this assay may be expressed as IC1.5x or IC2x, the inhibitor concentration required to increase the clotting time by 1.5 time or 2 times, respectively, relative to the clotting time in the absence of the inhibitor. The IC1.5x or IC2x is found by linear interpolation from relative clotting time versus inhibitor concentration plots using inhibitor concentration that spans the IC1.5x or IC2x.

Clotting times are determined using citrated normal human plasma as well as plasma obtained from a number of laboratory animal species *(e.g.,* rat, or rabbit). A compound is diluted into plasma beginning with a 10 mM DMSO stock solution. The final concentration of DMSO is less than 2%. Plasma clotting assays are performed in an automated coagulation analyzer (Sysmex, Dade-Behring, Illinois). Similarly, clotting times can be determined from laboratory animal species or humans dosed with compounds of the invention.

Activated Partial Thromboplastin Time (aPTT) is determined using ACTIN® (Dade-Behring, Illinois) following the directions in the package insert. Plasma (0.05 mL) is warmed to 37 °C for 1 minute. ACTIN® (0.05 mL) is added to the plasma and incubated for an additional 2 to 5 minutes. Calcium chloride (25 mM, 0.05 mL) is added to the reaction to initiate coagulation. The clotting time is the time in seconds from the moment calcium chloride is added until a clot is detected.

Prothrombin Time (PT) is determined using thromboplastin (Innovin, Dade-Behring, Illinois) following the directions in the package insert. Plasma (0.05 mL) is warmed to 37 °C for 1 minute. Thromboplastin (0.1 mL) is added to the plasma to initiate coagulation. The clotting time is the time in seconds from the moment thromboplastin is added until a clot is detected.

Chymotrypsin determinations were made in 50 mM HEPES buffer at pH 7.4 containing 145 mM NaCl, 5 mM KCl, and 0.1% PEG 8000 (polyethylene glycol; JT Baker or Fisher Scientific). Determinations were made using purified human chymotrypsin at a final concentration of 0.2-2 nM (Calbiochem) and the synthetic substrate S-2586 (Methoxy-Succinyl-Arg-Pro-Tyr-pNA; Chromogenix) at a concentration of 0.0005-0.005 M.

Trypsin determinations were made in 0.1 M sodium phosphate buffer at a pH of 7.5 containing 0.2 M sodium chloride and 0.5% PEG 8000. Determinations were made using purified human trypsin (Sigma) at a final assay concentration of 0.1-1 nM and the synthetic substrate S-2222 (Bz-Ile-Glu (gamma-OMe, 50%)-Gly-Arg-pNA; Chromogenix) at a concentration of 0.0005-0.005 M.

The exemplified Examples disclosed below were tested in the Factor XIa assay described above and found having Factor XIa inhibitory activity. A range of Factor XIa inhibitory activity (Ki values) of ≤ 10 μM (10000 nM) was observed.

The exemplified Examples disclosed below were tested in the Plasma Kallikrein assay described above, with some Examples having both Factor XIa and Plasma Kallikrein inhibitory activity. For those Examples where the Plasma Kallikrein inhibitory activity was observed as Ki values of ≤ 10 μM (10000 nM), the inhibitory activity is reported.

B. *In Vivo* Assays

The effectiveness of compounds of the present invention as antithrombotic agents can be determined using relevant *in vivo* thrombosis models, including *In Vivo* Electrically-induced Carotid Artery Thrombosis Models and *In Vivo* Rabbit Arteriovenous Shunt Thrombosis Models.

a. *In Vivo* Electrically-induced Carotid Artery Thrombosis (ECAT) Model

The rabbit ECAT model, described by Wong et al. (J. Pharmacol. Exp. Ther., 295:212-218 (2000)), can be used in this study. Male New Zealand White rabbits are anesthetized with ketamine (50 mg/kg + 50 mg/kg/h IM) and xylazine (10 mg/kg + 10 mg/kg/h IM). These anesthetics are supplemented as needed. An electromagnetic flow probe is placed on a segment of an isolated carotid artery to monitor blood flow. Test agents or vehicle will be given (i.v., i.p., s.c., or orally) prior to or after the initiation of thrombosis. Drug treatment prior to initiation of thrombosis is used to model the ability of test agents to prevent and reduce the risk of thrombus formation, whereas dosing after initiation is used to model the ability to treat existing thrombotic disease. Thrombus formation is induced by electrical stimulation of the carotid artery for 3 min at 4 mA using an external stainless-steel bipolar electrode. Carotid blood flow is measured continuously over a 90-min period to monitor thrombus-induced occlusion. Total carotid blood flow over 90 min is calculated by the trapezoidal rule. Average carotid flow over 90 min is then determined by converting total carotid blood flow over 90 min to percent of total control carotid blood flow, which would result if control blood flow had been maintained continuously for 90 min. The $ED_{50}$ (dose that increased average carotid blood flow over 90 min to 50% of the control) of compounds are estimated by a nonlinear least square regression program using the Hill sigmoid $E_{max}$ equation (DeltaGraph; SPSS Inc., Chicago, IL).

b. *In vivo* Rabbit Arteriovenous (AV) Shunt Thrombosis Model

The rabbit AV shunt model, described by Wong et al. (Wong, P.C. et al., J. Pharmacol. Exp. Ther. 292:351-357 (2000)), can be used in this study. Male New Zealand White rabbits are anesthetized with ketamine (50 mg/kg + 50 mg/kg/h IM) and xylazine (10 mg/kg + 10 mg/kg/h IM). These anesthetics are supplemented as needed. The femoral artery, jugular vein and femoral vein are isolated and catheterized. A saline-filled AV shunt device is connected between the femoral arterial and the femoral venous cannulae. The AV shunt device consists of an outer piece of tygon tubing (length = 8 cm; internal diameter = 7.9 mm) and an inner piece of tubing (length = 2.5 cm; internal diameter = 4.8 mm). The AV shunt also contains an 8-cm-long 2-0 silk thread (Ethicon, Somerville, NJ). Blood flows from the femoral artery via the AV-shunt into the femoral vein. The exposure of flowing blood to a silk thread induces the formation of a significant thrombus. Forty minutes later, the shunt is disconnected and the silk thread covered with thrombus is weighed. Test agents or vehicle will be given (i.v., i.p., s.c., or orally) prior to the opening of the AV shunt. The percentage inhibition of thrombus formation is determined for each treatment group. The $ID_{50}$ values (dose that produces 50% inhibition of thrombus formation) are estimated by a nonlinear least square regression program using the Hill sigmoid $E_{max}$ equation (DeltaGraph; SPSS Inc., Chicago, IL).

The anti-inflammatory effect of these compounds can be demonstrated in an Evans Blue dye extravasation assay using C1-esterase inhibitor deficient mice. In this model, mice are dosed with a compound of the present invention, Evans Blue dye is injected via the tail vein, and extravasation of the blue dye is determined by spectrophotometric means from tissue extracts.

The ability of the compounds of the current invention to reduce or prevent the systemic inflammatory response syndrome, for example, as observed during on-pump cardiovascular procedures, can be tested in *in vitro* perfusion systems, or by on-pump surgical procedures in larger mammals, including dogs and baboons. Read-outs to assess the benefit of the compounds of the present invention include for example reduced platelet loss, reduced platelet / white blood cell complexes, reduced neutrophil elastase levels in plasma, reduced activation of complement factors, and reduced activation and/or consumption of contact activation proteins (plasma kallikrein, factor XII, factor XI, high molecular weight kininogen, C1-esterase inhibitors).

The compounds of the present invention may also be useful as inhibitors of additional serine proteases, notably human thrombin, human plasma kallikrein and human plasmin. Because of their inhibitory action, these compounds are indicated for use in the prevention or treatment of physiological reactions, including blood coagulation, fibrinolysis, blood pressure regulation and inflammation, and wound healing catalyzed by the aforesaid class of enzymes. Specifically, the compounds have utility as drugs for the treatment of diseases arising from elevated thrombin activity of the aforementioned serine proteases, such as myocardial infarction, and as reagents used as anticoagulants in the processing of blood to plasma for diagnostic and other commercial purposes.

## V. PHARMACEUTICAL COMPOSITIONS, FORMULATIONS AND COMBINATIONS

The compounds of this invention can be administered in such oral dosage forms as tablets, capsules (each of which includes sustained release or timed release formulations), pills, powders, granules, elixirs, tinctures, suspensions, syrups, and emulsions. They may also be administered in intravenous (bolus or infusion), intraperitoneal, subcutaneous, or intramuscular form, all using dosage forms well known to those of ordinary skill in the pharmaceutical arts. They can be administered alone, but generally will be administered with a pharmaceutical carrier selected on the basis of the chosen route of administration and standard pharmaceutical practice.

The term "pharmaceutical composition" means a composition comprising a compound of the invention in combination with at least one additional pharmaceutically acceptable carrier or diluent. A "pharmaceutically acceptable carrier" refers to media generally accepted in the art for the delivery of biologically active agents to animals, in particular, mammals, including, *i.e.,* adjuvant, excipient or vehicle, such as diluents, preserving agents, fillers, flow regulating agents, disintegrating agents, wetting agents, emulsifying agents, suspending agents, sweetening agents, flavoring agents, perfuming agents, antibacterial agents, antifungal agents, lubricating agents and dispensing agents, depending on the nature of the mode of administration and dosage forms. Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and the therapeutic indication being targeted. Pharmaceutically acceptable carriers include both aqueous and non-aqueous liquid media, as well as a variety of solid and semi-solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Remington's Pharmaceutical Sciences, 18th Edition (1990).

The dosage regimen for the compounds of the present invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the thromboembolic disorder.

By way of general guidance, the daily oral dosage of each active ingredient, when used for the indicated effects, will range between about 0.001 to about 1000 mg/kg of body weight, preferably between about 0.01 to about 100 mg/kg of body weight per day, and most preferably between about 0.1 to about 20 mg/kg/day. Intravenously, the most preferred doses will range from about 0.001 to about 10 mg/kg/minute during a constant rate infusion. Compounds of this invention may be administered in a single daily dose, or the total daily dosage may be administered in divided doses of two, three, or four times daily.

Compounds of this invention can also be administered by parenteral administration (*e.g.*, intra-venous, intra-arterial, intramuscularly, or subcutaneously. When administered intra-venous or intra-arterial, the dose can be given continuously or intermittent. Furthermore, formulation can be developed for intramuscularly and subcutaneous delivery that ensure a gradual release of the active pharmaceutical ingredient. In one embodiment, the pharmaceutical composition is a solid formulation, *e.g.,* a spray-dried composition, which may be used as is, or whereto the physician or the patient adds solvents, and/or diluents prior to use.

Compounds of this invention can be administered in intranasal form via topical use of suitable intranasal vehicles, or via transdermal routes, using transdermal skin patches. When administered in the form of a transdermal delivery system, the dosage administration will, of course, be continuous rather than intermittent throughout the dosage regimen.

The compounds are typically administered in admixture with suitable pharmaceutical diluents, excipients, or carriers (collectively referred to herein as pharmaceutical carriers) suitably selected with respect to the intended form of administration, e.g., oral tablets, capsules, elixirs, and syrups, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar, bentonite, xanthan gum, and the like.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Compounds of the present invention may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxide-polylysine substituted with palmitoyl residues. Furthermore, the compounds of the present invention may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels. Solid dispersions are also called solid-state dispersions. In some embodiments, any compound described herein is formulated as a spray dried dispersion (SDD). An SDD is a single phase amorphous molecular dispersion of a drug in a polymer matrix. It is a solid solution prepared by dissolving the drug and a polymer in a solvent (e.g., acetone, methanol or the like) and spray drying the solution. The solvent rapidly evaporates from droplets which rapidly solidifies the polymer and drug mixture trapping the drug in amorphous form as an amorphous molecular dispersion.

Dosage forms (pharmaceutical compositions) suitable for administration may contain from about 1 milligram to about 1000 milligrams of active ingredient per dosage unit. In these pharmaceutical compositions the active ingredient will ordinarily be present in an amount of about 0.1-95% by weight based on the total weight of the composition.

Gelatin capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste

and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl-or propyl-paraben, and chlorobutanol.

Suitable pharmaceutical carriers are described in Remington's Pharmaceutical Sciences, Mack Publishing Company, a standard reference text in this field.

Where the compounds of this invention are combined with other anticoagulant agents, for example, a daily dosage may be about 0.1 to about 100 milligrams of the compound of the present invention and about 0.1 to about 100 milligrams per kilogram of patient body weight. For a tablet dosage form, the compounds of this invention generally may be present in an amount of about 5 to about 300 milligrams per dosage unit, and the second anti-coagulant in an amount of about 1 to about 500 milligrams per dosage unit.

Where the compounds of the present invention are administered in combination with an anti-platelet agent, by way of general guidance, typically a daily dosage may be about 0.01 to about 300 milligrams of the compound of the present invention and about 50 to about 150 milligrams of the anti-platelet agent, preferably about 0.1 to about 4 milligrams of the compound of the present invention and about 1 to about 3 milligrams of antiplatelet agents, per kilogram of patient body weight.

Where the compounds of the present invention are administered in combination with thrombolytic agent, typically a daily dosage may be about 0.1 to about 100 milligrams of the compound of the present invention, per kilogram of patient body weight and, in the case of the thrombolytic agents, the usual dosage of the thrombolytic agent when administered alone may be reduced by about 50-80% when administered with a compound of the present invention.

Particularly when provided as a single dosage unit, the potential exists for a chemical interaction between the combined active ingredients. For this reason, when the compound of the present invention and a second therapeutic agent are combined in a single dosage unit they are formulated such that although the active ingredients are combined in a single dosage unit, the physical contact between the active ingredients is minimized (that is, reduced). For example, one active ingredient may be enteric coated. By enteric coating one of the active ingredients, it is possible not only to minimize the contact between the combined active ingredients, but also, it is possible to control the release of one of these components in the gastrointestinal tract such that one of these components is not released in the stomach but rather is released in the intestines. One of the active ingredients may also be coated with a material that affects a sustained-release throughout the gastrointestinal tract and also serves to minimize physical contact between the combined active ingredients. Furthermore, the sustained-released component can be additionally enteric coated such that the release of this component occurs only in the intestine. Still another approach would involve the formulation of a combination product in which the one component is coated with a sustained and/or enteric release polymer, and the other component is also coated with a polymer such as a low viscosity grade of hydroxypropyl methylcellulose (HPMC) or other appropriate materials as known in the art, in order to further separate the active components. The polymer coating serves to form an additional barrier to interaction with the other component.

These as well as other ways of minimizing contact between the components of combination products of the present invention, whether administered in a single dosage form or administered in separate forms but at the same time by the same manner, will be readily apparent to those skilled in the art, once armed with the present disclosure.

In another embodiment, the present invention provides a pharmaceutical composition further comprising additional therapeutic agent(s) selected from potassium channel openers, potassium channel blockers, calcium channel blockers, sodium hydrogen exchanger inhibitors, antiarrhythmic agents, antiatherosclerotic agents, anticoagulants, antithrombotic agents, prothrombolytic agents, fibrinogen antagonists, diuretics, antihypertensive agents, ATPase inhibitors, mineralocorticoid receptor antagonists, phospodiesterase inhibitors, antidiabetic agents, anti-inflammatory agents, antioxidants, angiogenesis modulators, antiosteoporosis agents, hormone replacement therapies, hormone receptor modulators, oral contraceptives, antiobesity agents, antidepressants, antianxiety agents, antipsychotic agents, antiproliferative agents, antitumor agents, antiulcer and gastroesophageal reflux disease agents, growth hormone agents and/or growth hormone secretagogues, thyroid mimetics, anti-infective agents, antiviral agents, antibacterial agents, antifungal agents, cholesterol/lipid lowering agents and lipid profile therapies, and agents that mimic ischemic preconditioning and/or myocardial stunning, or a combination thereof.

In another embodiment, the present invention provides a pharmaceutical composition further comprising additional therapeutic agent(s) selected from an antiarrhythmic agent, an anti-hypertensive agent, an anti-coagulant agent, an anti-platelet agent, a thrombin inhibiting agent, a thrombolytic agent, a fibrinolytic agent, a calcium channel blocker, a potassium channel blocker, a cholesterol/lipid lowering agent, or a combination thereof.

In another embodiment, the present invention provides a pharmaceutical composition further comprising additional therapeutic agent(s) selected from warfarin, unfractionated heparin, low molecular weight heparin, synthetic pentasaccharide, hirudin, argatroban, aspirin, ibuprofen, naproxen, sulindac, indomethacin, mefenamate, dipyridamol, droxicam, diclofenac, sulfinpyrazone, piroxicam, ticlopidine, clopidogrel, tirofiban, eptifibatide, abciximab, melagatran, ximelagatran, disulfatohirudin, tissue plasminogen activator, modified tissue plasminogen activator, anistreplase, urokinase, and streptokinase, or a combination thereof.

In another embodiment, the present invention provides a pharmaceutical composition wherein the additional therapeutic agent is an antihypertensive agent selected from ACE inhibitors, AT-1 receptor antagonists, beta-adrenergic receptor antagonists, ETA receptor antagonists, dual ETA/AT-1 receptor antagonists, renin inhibitors (aliskiren) and vasopepsidase inhibitors, an antiarrythmic agent selected from $I_{Kur}$ inhibitors, an anticoagulant selected from thrombin inhibitors, antithrombin-III activators, heparin co-factor II activators, other factor XIa inhibitors, other kallikrein inhibitors, plasminogen activator inhibitor (PAI-1) antagonists, thrombin activatable fibrinolysis inhibitor (TAFI) inhibitors, factor VIIa inhibitors, factor IXa inhibitors, and factor Xa inhibitors, or an antiplatelet agent selected from GPIIb/IIIa blockers, GP Ib/IX blockers, protease activated receptor 1 (PAR-1) antagonists, protease activated receptor4 (PAR-4) antagonists, prostaglandin E2 receptor EP3 antagonists, collagen receptor antagonists, phosphodiesterase-III inhibitors, $P2Y_1$ receptor antagonists, $P2Y_{12}$ antagonists, thromboxane receptor antagonists, cyclooxygense-1 inhibitors, and aspirin, or a combination thereof.

In another embodiment, the present invention provides pharmaceutical composition, wherein the additional therapeutic agent(s) are an anti-platelet agent or a combination thereof.

In another embodiment, the present invention provides a pharmaceutical composition, wherein the additional therapeutic agent is the anti-platelet agent clopidogrel.

The compounds of the present invention can be administered alone or in combination with one or more additional therapeutic agents. By "administered in combination" or "combination therapy" it is meant that the compound of the present invention and one or more additional therapeutic agents are administered concurrently to the mammal being treated. When administered in combination, each component may be administered at the same time or sequentially in any order at different points in time. Thus, each component may be administered separately but sufficiently closely in time so as to provide the desired therapeutic effect.

Compounds that can be administered in combination with the compounds of the present invention include, but are not limited to, anticoagulants, anti-thrombin agents, anti-platelet agents, fibrinolytics, hypolipidemic agents, antihypertensive agents, and anti-ischemic agents.

Other anticoagulant agents (or coagulation inhibitory agents) that may be used in combination with the compounds of this invention include warfarin, heparin (either unfractionated heparin or any commercially available low molecular weight heparin, for example LOVENOX®), synthetic pentasaccharide, direct acting thrombin inhibitors including hirudin and argatroban, as well as other factor VIIa inhibitors, factor IXa inhibitors, factor Xa inhibitors *(e.g.,* ARIXTRA®, apixaban, rivaroxaban, LY-517717, DU-176b, DX-9065a, and those disclosed in WO 98/57951, WO 03/026652, WO 01/047919, and WO 00/076970), factor XIa inhibitors, and inhibitors of activated TAFI and PAI-1 known in the art.

The term anti-platelet agents (or platelet inhibitory agents), as used herein, denotes agents that inhibit platelet function, for example, by inhibiting the aggregation, adhesion or granule-content secretion of platelets. Such agents include, but are not limited to, the various known non-steroidal anti-inflammatory drugs (NSAIDs) such as acetaminophen, aspirin, codeine, diclofenac, droxicam, fentaynl, ibuprofen, indomethacin, ketorolac, mefenamate, morphine, naproxen, phenacetin, piroxicam, sufentanyl, sulfinpyrazone, sulindac, and pharmaceutically acceptable salts or prodrugs thereof. Of the NSAIDs, aspirin (acetylsalicylic acid or ASA) and piroxicam are preferred. Other suitable platelet inhibitory agents include glycoprotein IIb/IIIa antagonists *(e.g.,* tirofiban, eptifibatide, abciximab, and integrelin), thromboxane-A2-receptor antagonists *(e.g.,* ifetroban), thromboxane-A-synthetase inhibitors, phosphodiesterase-III (PDE-III) inhibitors (e.g., dipyridamole, cilostazol), and PDE-V inhibitors (such as sildenafil), protease-activated receptor 1 (PAR-1) antagonists *(e.g.,* E-5555, SCH-530348, SCH-203099, SCH-529153 and SCH-205831), and pharmaceutically acceptable salts or prodrugs thereof.

Other examples of suitable anti-platelet agents for use in combination with the compounds of the present invention, with or without aspirin, are ADP (adenosine diphosphate) receptor antagonists, preferably antagonists of the purinergic receptors $P2Y_1$ and $P2Y_{12}$, with $P2Y_{12}$ being even more preferred. Preferred $P2Y_{12}$ receptor antagonists include clopidogrel, ticlopidine, prasugrel, ticagrelor, and cangrelor, and pharmaceutically acceptable salts or prodrugs thereof. Ticlopidine and clopidogrel are also preferred compounds since they are known to be more gentle than aspirin on the gastrointestinal tract in use. Clopidogrel is an even more preferred agent.

A preferred example is a triple combination of a compound of the present invention, aspirin, and another anti-platelet agent. Preferably, the anti-platelet agent is clopidogrel or prasugrel, more preferably clopidogrel.

The term thrombin inhibitors (or anti-thrombin agents), as used herein, denotes inhibitors of the serine protease thrombin. By inhibiting thrombin, various thrombin-mediated processes, such as thrombin-mediated platelet activation (that is, for example, the aggregation of platelets, and/or the secretion of platelet granule contents including serotonin) and/or fibrin formation are disrupted. A number of thrombin inhibitors are known to one of skill in the art and these

inhibitors are contemplated to be used in combination with the present compounds. Such inhibitors include, but are not limited to, boroarginine derivatives, boropeptides, heparins, hirudin, argatroban, dabigatran, AZD-0837, and those disclosed in WO 98/37075 and WO 02/044145, and pharmaceutically acceptable salts and prodrugs thereof. Boroarginine derivatives and boropeptides include N-acetyl and peptide derivatives of boronic acid, such as C-terminal a-aminoboronic acid derivatives of lysine, ornithine, arginine, homoarginine and corresponding isothiouronium analogs thereof. The term hirudin, as used herein, includes suitable derivatives or analogs of hirudin, referred to herein as hirulogs, such as disulfatohirudin.

The term thrombolytic (or fibrinolytic) agents (or thrombolytics or fibrinolytics), as used herein, denotes agents that lyse blood clots (thrombi). Such agents include tissue plasminogen activator (TPA, natural or recombinant) and modified forms thereof, anistreplase, urokinase, streptokinase, tenecteplase (TNK), lanoteplase (nPA), factor VIIa inhibitors, thrombin inhibitors, inhibitors of factors IXa, Xa, and XIa, PAI-I inhibitors *(i.e.,* inactivators of tissue plasminogen activator inhibitors), inhibitors of activated TAFI, alpha-2-antiplasmin inhibitors, and anisoylated plasminogen streptokinase activator complex, including pharmaceutically acceptable salts or prodrugs thereof. The term anistreplase, as used herein, refers to anisoylated plasminogen streptokinase activator complex, as described, for example, in European Patent Application No. 028,489. The term urokinase, as used herein, is intended to denote both dual and single chain urokinase, the latter also being referred to herein as prourokinase.

Examples of suitable cholesterol/lipid lowering agents and lipid profile therapies for use in combination with the compounds of the present invention include HMG-CoA reductase inhibitors *(e.g.,* pravastatin, lovastatin, simvastatin, fluvastatin, atorvastatin, rosuvastatin, and other statins), low-density lipoprotein (LDL) receptor activity modulators *(e.g.,* HOE-402, PCSK9 inhibitors), bile acid sequestrants (*e.g.,* cholestyramine and colestipol), nicotinic acid or derivatives thereof *(e.g.,* NIASPAN®), GPR109B (nicotinic acid receptor) modulators, fenofibric acid derivatives *(e.g.,* gemfibrozil, clofibrate, fenofibrate and benzafibrate) and other peroxisome proliferator-activated receptors (PPAR) alpha modulators, PPARdelta modulators *(e.g.,* GW-501516), PPARgamma modulators *(e.g.,* rosiglitazone), compounds that have multiple functionality for modulating the activities of various combinations of PPARalpha, PPARgamma and PPARdelta, probucol or derivatives thereof *(e.g.,* AGI-1067), cholesterol absorption inhibitors and/or Niemann-Pick C1-like transporter inhibitors *(e.g.,* ezetimibe), cholesterol ester transfer protein inhibitors *(e.g.,* CP-529414), squalene synthase inhibitors and/or squalene epoxidase inhibitors or mixtures thereof, acyl coenzyme A: cholesteryl acyltransferase (ACAT) 1 inhibitors, ACAT2 inhibitors, dual ACAT1/2 inhibitors, ileal bile acid transport inhibitors (or apical sodium co-dependent bile acid transport inhibitors), microsomal triglyceride transfer protein inhibitors, liver-X-receptor (LXR) alpha modulators, LXRbeta modulators, LXR dual alpha/beta modulators, FXR modulators, omega 3 fatty acids *(e.g.,* 3-PUFA), plant stanols and/or fatty acid esters of plant stanols *(e.g.,* sitostanol ester used in BENECOL® margarine), endothelial lipase inhibitors, and HDL functional mimetics which activate reverse cholesterol transport *(e.g.,* apoAI derivatives or apoAI peptide mimetics).

The compounds of the present invention can also be combined with soluble guanylate cyclase inhibitors, Chymase inhibitors, ROMK inhibitors, ACE inhibitors, ATII inhibitors, ATR inhibitors, NEP inhibitors and other compounds to treat heart failure.

The compounds of the present invention are also useful as standard or reference compounds, for example as a quality standard or control, in tests or assays involving the inhibition of thrombin, Factor VIIa, IXa, Xa, XIa, and/or plasma kallikrein. Such compounds may be provided in a commercial kit, for example, for use in pharmaceutical research involving thrombin, Factor VIIa, IXa, Xa, XIa, and/or plasma kallikrein. XIa. For example, a compound of the present invention could be used as a reference in an assay to compare its known activity to a compound with an unknown activity. This would ensure the experimentor that the assay was being performed properly and provide a basis for comparison, especially if the test compound was a derivative of the reference compound. When developing new assays or protocols, compounds according to the present invention could be used to test their effectiveness.

The compounds of the present invention may also be used in diagnostic assays involving thrombin, Factor VIIa, IXa, Xa, XIa, and/or plasma kallikrein. For example, the presence of thrombin, Factor VIIa, IXa, Xa XIa, and/or plasma kallikrein in an unknown sample could be determined by addition of the relevant chromogenic substrate, for example S2366 for Factor XIa, to a series of solutions containing test sample and optionally one of the compounds of the present invention. If production of pNA is observed in the solutions containing test sample, but not in the presence of a compound of the present invention, then one would conclude Factor XIa was present.

Extremely potent and selective compounds of the present invention, those having $K_i$ values less than or equal to 0.001 $\mu$M against the target protease and greater than or equal to 0.1 $\mu$M against the other proteases, may also be used in diagnostic assays involving the quantitation of thrombin, Factor VIIa, IXa, Xa, XIa, and/or plasma kallikrein in serum samples. For example, the amount of Factor XIa in serum samples could be determined by careful titration of protease activity in the presence of the relevant chromogenic substrate, S2366, with a potent Factor XIa inhibitor of the present invention.

The present invention also encompasses an article of manufacture. As used herein, article of manufacture is intended to include, but not be limited to, kits and packages. The article of manufacture of the present invention, comprises: (a) a first container; (b) a pharmaceutical composition located within the first container, wherein the composition, comprises: a first therapeutic agent, comprising: a compound of the present invention or a pharmaceutically acceptable salt form thereof; and, (c) a package insert stating that the pharmaceutical composition can be used for the treatment of a thromboembolic and/or inflammatory disorder (as defined previously). In another embodiment, the package insert states that the pharmaceutical composition can be used in combination (as defined previously) with a second therapeutic agent to treat a thromboembolic and/or inflammatory disorder. The article of manufacture can further comprise: (d) a second container, wherein components (a) and (b) are located within the second container and component (c) is located within or outside of the second container. Located within the first and second containers means that the respective container holds the item within its boundaries.

The first container is a receptacle used to hold a pharmaceutical composition. This container can be for manufacturing, storing, shipping, and/or individual/bulk selling. First container is intended to cover a bottle, jar, vial, flask, syringe, tube *(e.g.,* for a cream preparation), or any other container used to manufacture, hold, store, or distribute a pharmaceutical product.

The second container is one used to hold the first container and, optionally, the package insert. Examples of the second container include, but are not limited to, boxes *(e.g.,* cardboard or plastic), crates, cartons, bags *(e.g.,* paper or plastic bags), pouches, and sacks. The package insert can be physically attached to the outside of the first container via tape, glue, staple, or another method of attachment, or it can rest inside the second container without any physical means of attachment to the first container. Alternatively, the package insert is located on the outside of the second container. When located on the outside of the second container, it is preferable that the package insert is physically attached via tape, glue, staple, or another method of attachment. Alternatively, it can be adjacent to or touching the outside of the second container without being physically attached.

The package insert is a label, tag, marker, etc. that recites information relating to the pharmaceutical composition located within the first container. The information recited will usually be determined by the regulatory agency governing the area in which the article of manufacture is to be sold *(e.g.,* the United States Food and Drug Administration). Preferably, the package insert specifically recites the indications for which the pharmaceutical composition has been approved. The package insert may be made of any material on which a person can read information contained therein or thereon. Preferably, the package insert is a printable material *(e.g.,* paper, plastic, cardboard, foil, adhesive-backed paper or plastic, etc.) on which the desired information has been formed *(e.g.,* printed or applied).

Other features of the invention will become apparent in the course of the following descriptions of exemplary embodiments that are given for illustration of the invention and are not intended to be limiting thereof. The following Examples have been prepared, isolated and characterized using the methods disclosed herein.

## VI. GENERAL SYNTHESIS INCLUDING SCHEMES

The compounds of the present invention may be synthesized by many methods available to those skilled in the art of organic chemistry (Maffrand, J.P. et al., Heterocycles, 16(1):35-37 (1981)). General synthetic schemes for preparing compounds of the present invention are described below. These schemes are illustrative and are not meant to limit the possible techniques one skilled in the art may use to prepare the compounds disclosed herein. Different methods to prepare the compounds of the present invention will be evident to those skilled in the art. Additionally, the various steps in the synthesis may be performed in an alternate sequence in order to give the desired compound or compounds.

Examples of compounds of the present invention prepared by methods described in the general schemes are given in the intermediates and examples section set out hereinafter. Preparation of homochiral examples may be carried out by techniques known to one skilled in the art. For example, homochiral compounds may be prepared by separation of racemic products by chiral phase preparative HPLC. Alternatively, the example compounds may be prepared by methods known to give enantiomerically enriched products. These include, but are not limited to, the incorporation of chiral auxiliary functionalities into racemic intermediates which serve to control the diastereoselectivity of transformations, providing enantio-enriched products upon cleavage of the chiral auxiliary.

The compounds of the present invention can be prepared in a number of ways known to one skilled in the art of organic synthesis. The compounds of the present invention can be synthesized using the methods described below, together with synthetic methods known in the art of synthetic organic chemistry, or by variations thereon as appreciated by those skilled in the art. Preferred methods include, but are not limited to, those described below. The reactions are performed in a solvent or solvent mixture appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the invention.

It will also be recognized that another major consideration in the planning of any synthetic route in this field is the judicious choice of the protecting group used for protection of the reactive functional groups present in the compounds

described in this invention. An authoritative account describing the many alternatives to the trained practitioner is Greene et al. (Protective Groups in Organic Synthesis, Fourth Edition, Wiley-Interscience (2006)).

Representative pyrimidinone compounds **1a** of this invention can be prepared as described in Scheme 1. Using a modified procedure described by Xiao (Organic Letters, 11:1421 (2009)), suitably substituted pyrimidin-4-ol derivatives **1b** can be coupled with an appropriately substituted macrocycle amine **1c** in the presence of HATU and DBU in a solvent such as $CH_3CN$ to provide pyrimidinone compounds **1a.** When ring A is a SEM-protected imidazole ring, an additional deprotection step employing 4N HCl in dioxane or TFA in DCM is used to afford compounds of this invention.

Scheme 1

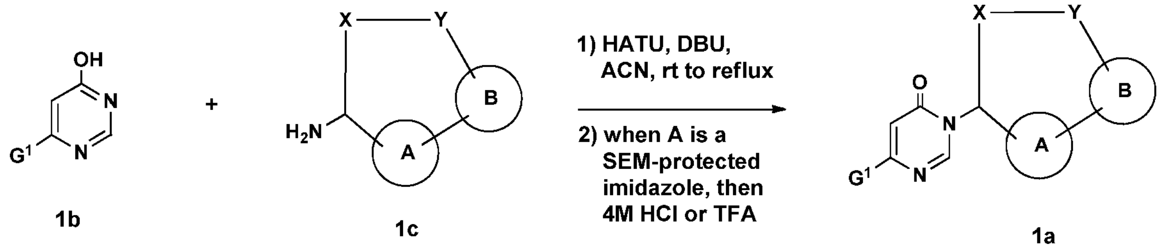


1b 1c 1a

Scheme 2 describes the synthesis of suitably substituted pyrimidin-4-ol derivatives **1b.** Suzuki-Miyaura coupling between 6-chloropyrimidin-4-ol (**2a**) and an appropriately substituted heteroaryl boronic acid or ester **2c** in the presence of a base such as Hunig's base or potassium phosphate tribasic, in a solvent mixture, such as toluene and ethanol, or THF, using a precatalyst such as $Pd(PPh_3)_4$ or 2nd generation XPhos provides **1b.** Alternatively, when 4-chloro-6-methoxypyrimidine **2b** is used, an additional deprotection step, employing aqueous HBr at elevated temperatures, is required to provide pyrimidin-4-ol derivatives **1b.**

Scheme 2

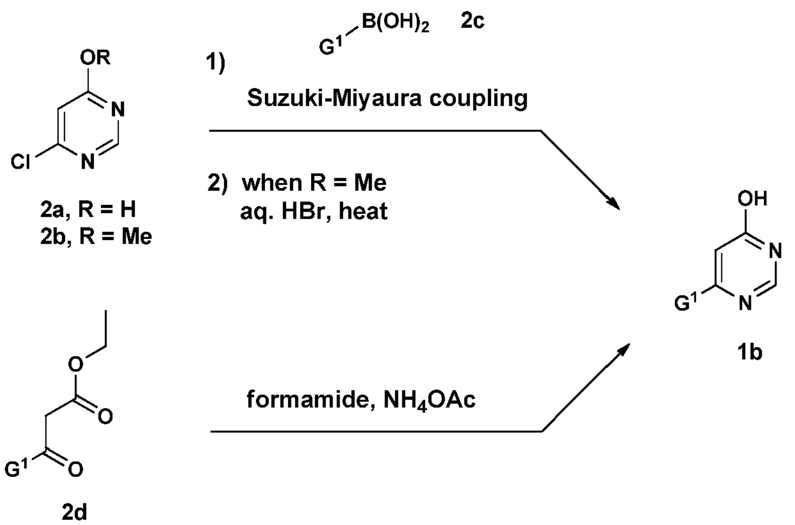


2c

2a, R = H
2b, R = Me

1b

2d

Intermediates for preparation of compounds of the present invention wherein ring A and B are a 6-membered heterocyclyl (example - pyridine) can be derived from appropriately substituted aldehydes **3a** according to the general method outlined in Scheme 3. Condensation of aldehyde **3a** (X= N, Y = Z = M = CH) prepared according to a modified procedure described by Negi (Synthesis, 991 (1996)), with (S)-2-methylpropane-2-sulfinamide in the presence of anhydrous copper sulfate or cesium carbonate in a solvent such as DCM gives the sulfinimine **3b** (Ellman, J., J. Org. Chem., 64:1278 (1999)). Using a modified procedure described by Kuduk (Tetrahedron Letters, 45:6641 (2004)), suitably substituted Grignard reagents, for example allylmagnesium bromide, can be added to sulfinimine **3b** to give a sulfinamide **3c,** as a mixture of diastereomers which can be separated at various stages of the sequence. The diastereoselectivity for the addition of allylmagnesium bromide to sulfinimine **3b** can be improved by employing indium(III) chloride according to a modified procedure of Xu (Xu, M-H, Organic Letters, 10(6):1259 (2008)). Suzuki-Miyaura coupling between 4-chloropyridine **3c** and an appropriately substituted heteroaryl boronic acid or ester **3e** in the presence of a base such as potassium phosphate, in a solvent mixture, such as DMSO and $H_2O$, or DMF, using a precatalyst such as

$Pd(dppf)Cl_2•CH_2Cl_2$ complex provides **3g.** Alternatively, the Suzuki-Miyaura coupling between boronic acid **3d** and an appropriately substituted heteroaryl halide **3f** can be used to prepare **3g.** Protecting group interconversion can be accomplished in two steps to give **3h.** Alternatively, the protecting group interconversion can take place initially on **3c** followed by the Suzuki-Miyaura coupling. The aniline **3h** can then be coupled with an appropriately substituted carboxylic acid **3i** using T3P® and a base, such as pyridine, to give the amide **3j.** Using a modified procedure described by Lovely (Tetrahedron Letters, 44:1379 (2003)), **3j,** following pretreatment with *p*-toluenesulfonic acid to form the pyridinium ion, can be cyclized via ring-closing metathesis using a catalyst, such as Second Generation Grubbs Catalyst in a suitable solvent, such as DCM, DCE, or toluene at elevated temperature, to give the pyridine-containing macrocycle **3k.** The alkene can be reduced with hydrogen over either palladium on carbon or platinum oxide, and subsequent deprotection with TFA in DCM or 4M HCl in dioxane provides amine **3l.** Compounds of the formula **3l** can be converted to compounds in this invention according to Scheme 1.

Scheme 3

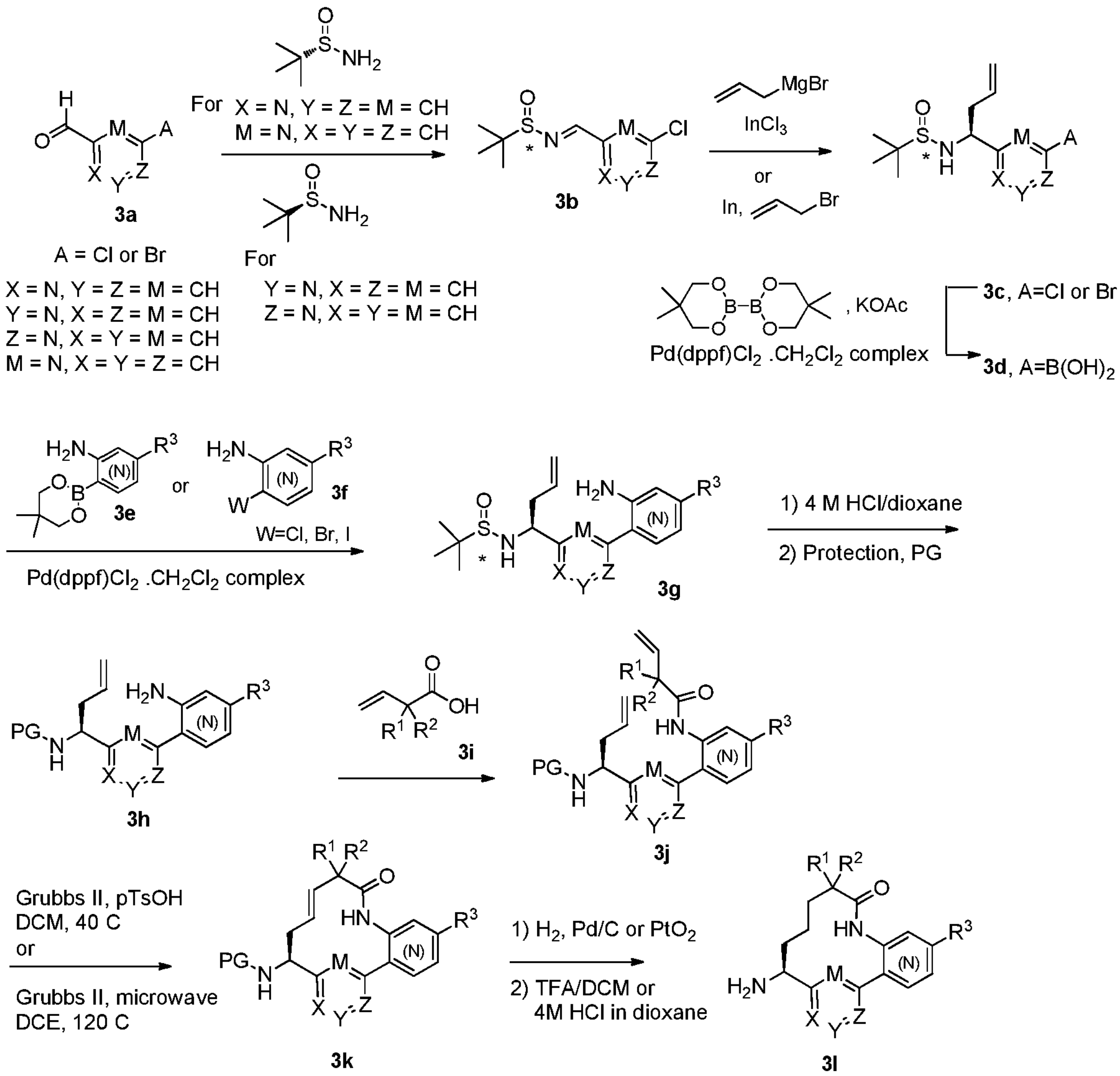


Methods for synthesis of a large variety of substituted pyridine compounds useful as starting materials for the preparation of compounds of the present invention are well known in the art and have been extensively reviewed. (For examples of methods useful for the preparation of pyridine starting materials see: Kroehnke, F., Synthesis, 1 (1976); Abramovitch, R.A., ed., "Pyridine and Its Derivatives", The Chemistry of Heterocyclic Compounds, 14(Suppl. 1-4), John Wiley & Sons, New York (1974); Boulton, A.J. et al., eds., Comprehensive Heterocyclic Chemistry, 2:165-524, Pergamon Press, New York (1984); McKillop, A., ed., Comprehensive Heterocyclic Chemistry, 5:1-300, Pergamon Press, New York (1996)).

In cases where suitably substituted boronic acids are not commercially available, a modification to this approach may be adopted wherein a heteroaryl halide is subjected to a palladium mediated coupling with a diboron species such as bis(pinacolato) diboron or bis(neopentyl glycolato)diboron to provide the corresponding 4,4,5,5-tetramethyl-[1,3,2]dioxaborolane or the 5,5-dimethyl-[1,3,2]dioxaborolane intermediates using the method of Ishiyama, T. et al. (J. Org. Chem., 60(23):7508-7510 (1995)). Alternately, this same intermediate can be prepared by reaction of the intermediate halide with the corresponding dialkoxyhydroborane as described by Murata et al. (J. Org. Chem., 62(19):6458-6459 (1997)). The boron pinacolate intermediates can be used in place of boronic acids for coupling to the aryl/heteroaryl halides or triflates or the boron pinacolate intermediate can be converted to the boronic acids. Alternately, the corresponding boronic acids can be prepared by metal-halogen exchange of the aryl/heteroaryl halide, quenching with a trialkoxyborate reagent, and aqueous workup to provide the boronic acids (Miyaura, N. et al., Chem. Rev., 95:2457 (1995)).

It is also realized that the scope of intermediate synthesis can be further extended outside the use of Suzuki-Miyaura coupling methodology since the precursor heteroaryl halides or triflates described above are also precursors for Stille, Negishi, Hiyama, and Kumada-type cross coupling methodologies (Tsuji, J., Transition Metal Reagents and Catalysts: Innovations in Organic Synthesis, John Wiley & Sons (2000); Tsuji, J., Palladium Reagents and Catalysts: Innovations in Organic Synthesis, John Wiley & Sons (1996)).

Intermediates for preparation of compounds of the present invention wherein ring A is an imidazole ring, can be prepared from an appropriately N-protected allylglycine **(4a)** according to the general method outlined in Scheme 4 (Contour-Galcera et al., Bioorg. Med. Chem. Lett., 11(5):741-745 (2001)). Condensation of **4a** with a suitably substituted alpha-bromo-ketone bearing a heteroaryl group **(4b)** in the presence of a suitable base such as potassium bicarbonate, potassium carbonate or cesium carbonate in a suitable solvent such as DMF provides a keto ester intermediate which can be cyclized to afford an imidazole **(4c)** by heating in the presence of excess ammonium acetate in a solvent such as toluene or xylene. This latter transformation can be conveniently carried out on small scale at 160 °C in a microwave reactor or on larger scale by refluxing the mixture while removing water via a Dean-Stark trap. The resulting imidazole intermediate **(4c)** is then protected by treatment with SEM-Cl in the presence of a base such as sodium hydride or dicyclohexylmethylamine in a solvent such as THF or DCM. The resulting heteroaryl bromide **(4d)** is then converted to the corresponding amino-heterocyclyl **(4e)** by heating in a sealed vessel with excess ammonium hydroxide, in the presence of copper iodide, a base such as potassium carbonate and a catalytic amount of proline in DMSO as solvent. Acylation of **4e** with the appropriate alkenoic acid and a coupling agent such as T3P® or BOP reagent, or alternately, by treatment with an alkenoic acid chloride in the presence of a base such as TEA, DIPEA, or pyridine provides diene **4f,** which undergoes ring closing metathesis by heating in dilute solution in the presence of *p*-toluene sulfonic acid and Second Generation Grubbs Catalyst in a suitable solvent such as DCM or DCE to provide the corresponding macrocycle **(4g).** Alternately, the RCM can be run in a microwave at elevated temperatures without *p*TsOH. Reduction of the double bond followed by bromination with NBS at room temperature affords **4h.** Suzuki-Miyaura coupling with methylboronic acid or tetramethylstannane and removal of the protecting group (PG), provides intermediate **4i.** Intermediate **4i** can be converted to compounds of the present invention following the steps described in Scheme 1.

Scheme 4

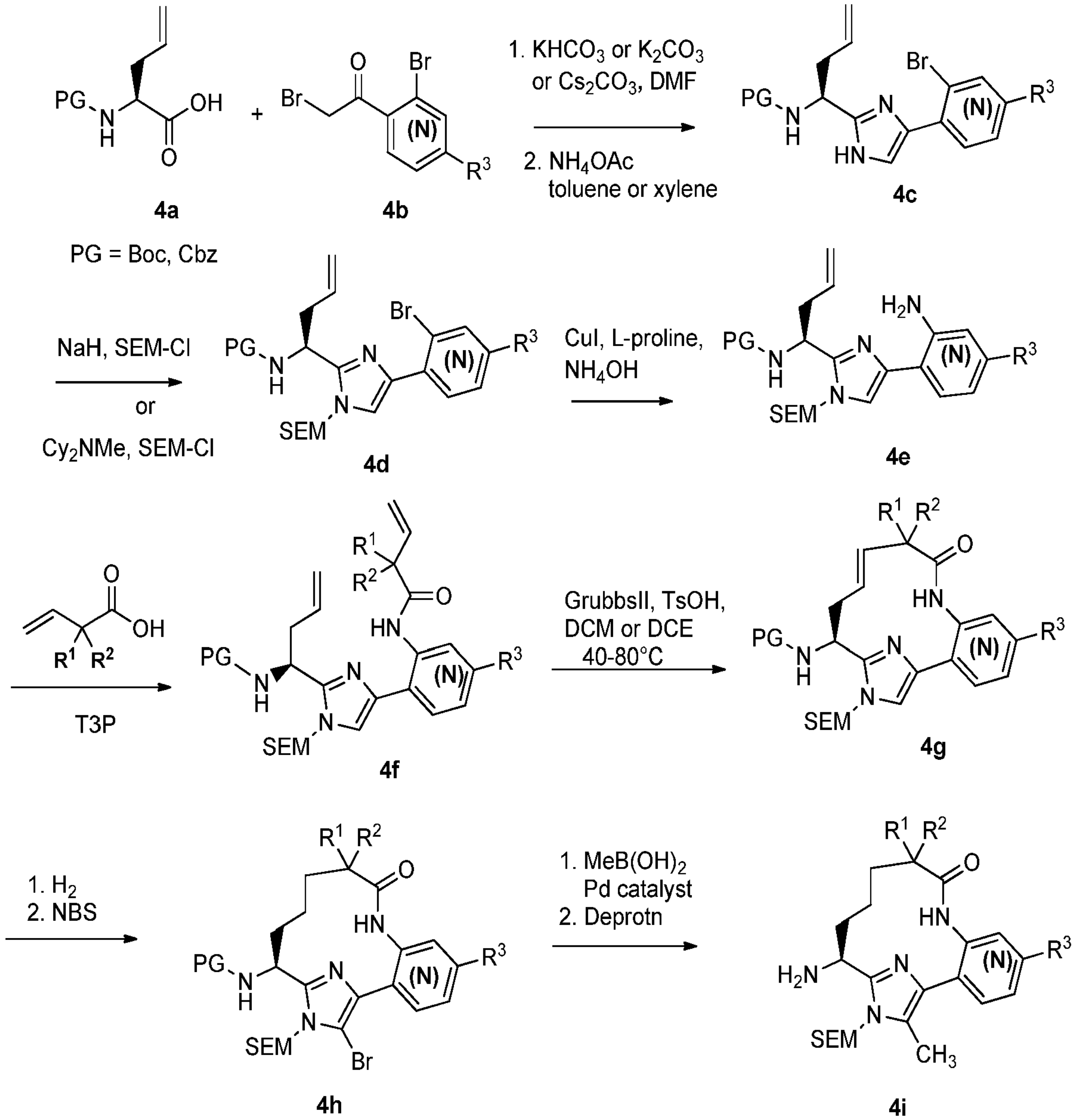


Scheme 5 describes the intermediates in the present invention where ring B is a heterocyclyl (example -pyrazole). Chloropyridine **3b** undergoes protecting group interconversion to provide **5a** which can be coupled to 4-nitropyrazoles **5b** upon heating with a Pd(II) salt such as $Pd(OAc)_2$ in the presence of a phosphine ligand and a base such as potassium carbonate in a solvent such as DMF or DMA, as described by Sames (Goikhman, R., Jacques, T.L. and Sames, D., J. Am. Chem. Soc., 131:3042 (2009)). Zinc/HOAc reduction of the nitropyrazole, **5c,** followed by amidation with an appropriately substituted carboxylic acid, **5d,** provides **5e.** Macrocyclization is then accomplished via ring-closing metathesis using the Grubb's second generation ruthenium catalyst to yield **5f.** Hydrogenation of the resulting olefin and protecting group cleavage yields amine **5g.** Compounds of the formulae **5g** can be converted to compounds in this invention upon coupling with an appropriately substituted pyrimidin-4-ol derivative, **1b,** according to Scheme 1.

Scheme 5

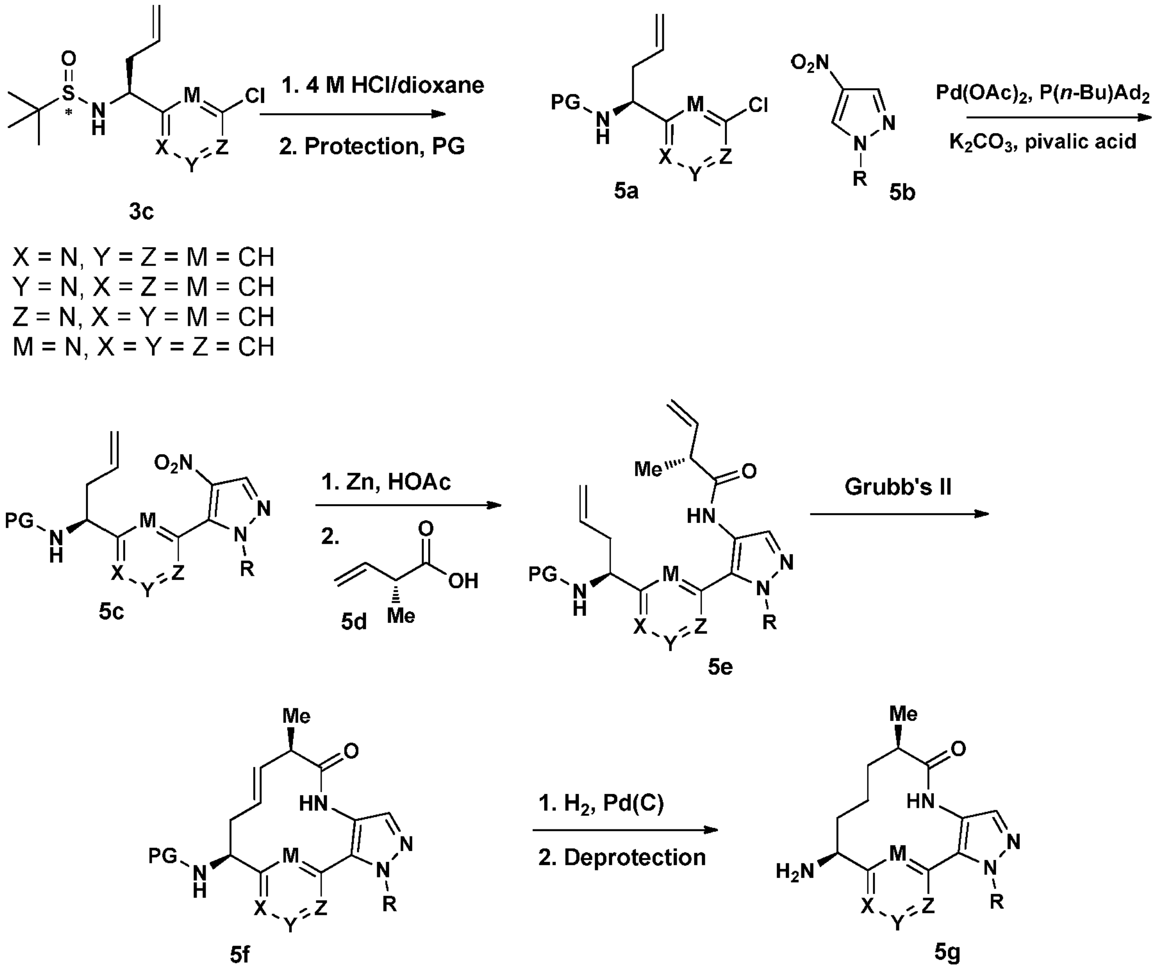


Compounds in this invention bearing alternate regiochemical pyrazole substitution can be synthesized as shown in Scheme 6. When R is an appropriate protective group (example - trimethylsilylethoxymethyl), deprotection of **6a** to **6b** can be followed by alkylation with an alkyl halide under basic conditions, upon reaction with a boronic acid in the presence of Cu(II) salts such as $Cu(OAc)_2$, or upon reaction with an aryl iodide in the presence of CuI and a diamine ligand. In most cases, the alkylation proceeds to give solely the product shown in **6c.** In select cases, products with the pyrazole regiochemistry shown in Scheme 5 are formed as a minor component.

Compounds in this invention bearing alternate regiochemical pyrazole substitution can be synthesized as shown in Scheme 6. When R is an appropriate protective group (example - trimethylsilylethoxymethyl), deprotection of **6a** to **6b** can be followed by alkylation with an alkyl halide under basic conditions, upon reaction with a boronic acid in the presence of Cu(II) salts such as $Cu(OAc)_2$, or upon reaction with an aryl iodide in the presence of CuI and a diamine ligand. In most cases, the alkylation proceeds to give solely the product shown in **6c.** In select cases, products with the pyrazole regiochemistry shown in Scheme 5 are formed as a minor component.

Scheme 6

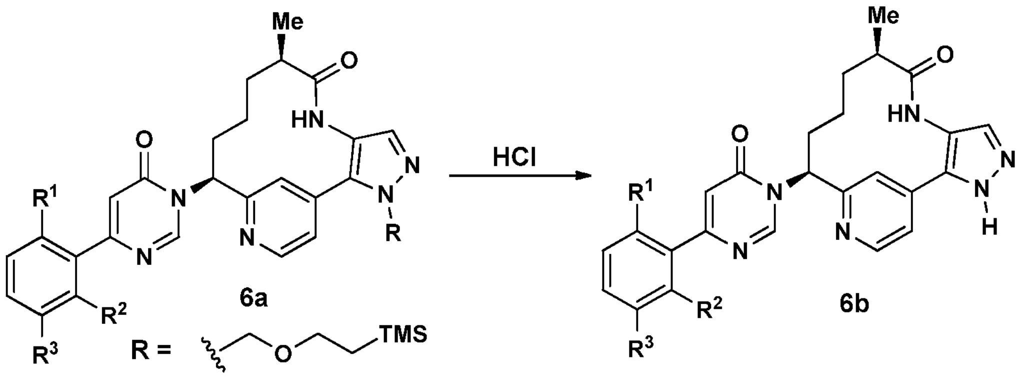


6a

6b

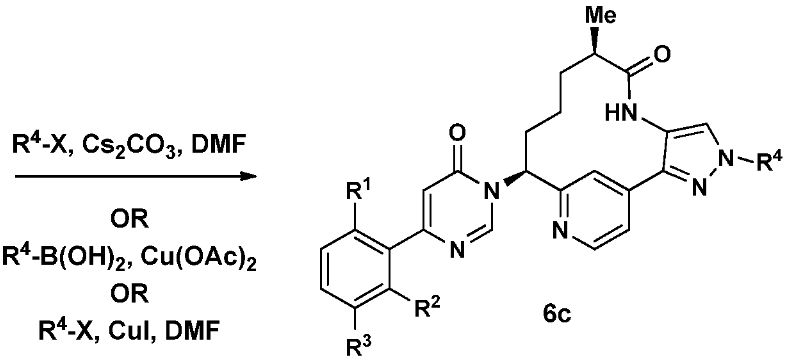


6c

Intermediates for preparation of compounds of the present invention wherein $R^{1a}$ is -F can be prepared according to Scheme 7. Olefin **5f** can be subjected to hydrofluorination, yielding as many as four isomeric alkyl fluorides. Following separation of the isomers, deprotection of the amine protecting group is accomplished by the action of either TFA or HCl, as previously shown in Schemes 3-5. The intermediate **7a** and **7b** can be elaborated to compounds of this invention according to the procedure described in Scheme 1.

Scheme 7

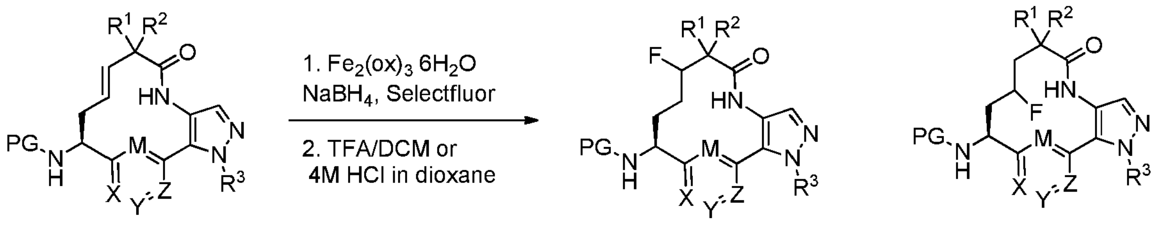


**5f** **7a** **7b**

X = N, Y = Z = M = CH
Y = N, X = Z = M = CH
Z = N, X = Y = M = CH
M = N, X = Y = Z = CH

Intermediates for preparation of compounds of the present invention corresponding to Formula V can be prepared according to Scheme 8. Chloropyridine **5a** is reacted with aqueous hydrazine to generate substituted hydrazine **8a.** This hydrazine can be cyclized upon treatment with α-cyanoketones **8b** to yield aminopyrazoles **8c.** These intermediates **(8c)** can be elaborated to compounds of this invention according to the procedures described in Schemes 1 and 3.

Scheme 8

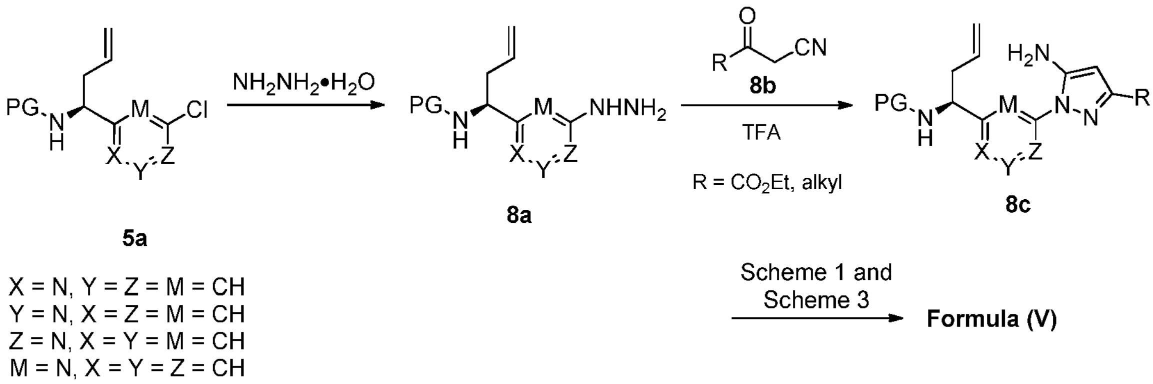


5a 8a 8c

Scheme 9 describes the synthesis of suitably substituted pyrimidin-4-ol derivatives where $G^1$ is a substituted phenyl. Aniline **9a** can be converted to a suitably substituted triazole **9b** in a one pot, two step sequence. Specifically, the aniline **9a** is converted to the aryl azide *in situ* followed by cycloaddition with a suitably substituted alkyne in the presence of a copper catalyst, such as $Cu_2O$, to provide **9b.** Demethylation of **9b** according to Scheme 2 provides the pyrimidin-4-ol derivatives **9c.** When $R^{10}$ is a trimethylsilyl group, the silyl moiety can be converted to a chloride at elevated temperature with NCS in the presence of silica gel. Aniline **9a** can be converted to the iodide **9d** with *p*-TsOH, $NaNO_2$, and NaI. Subjecting iodide **9d** to a variety of N-arylation or Suzuki-Miyaura couplings, followed by demethylation according to Scheme 2, gives additional pyrimidin-4-ol derivatives **9e.** When $R^8$ is tetrazole, intermediate **9g** can be prepared by first treatment of the aniline **9a** with trimethoxymethane and sodium azide followed by demethylation according to Scheme 2.

Scheme 9

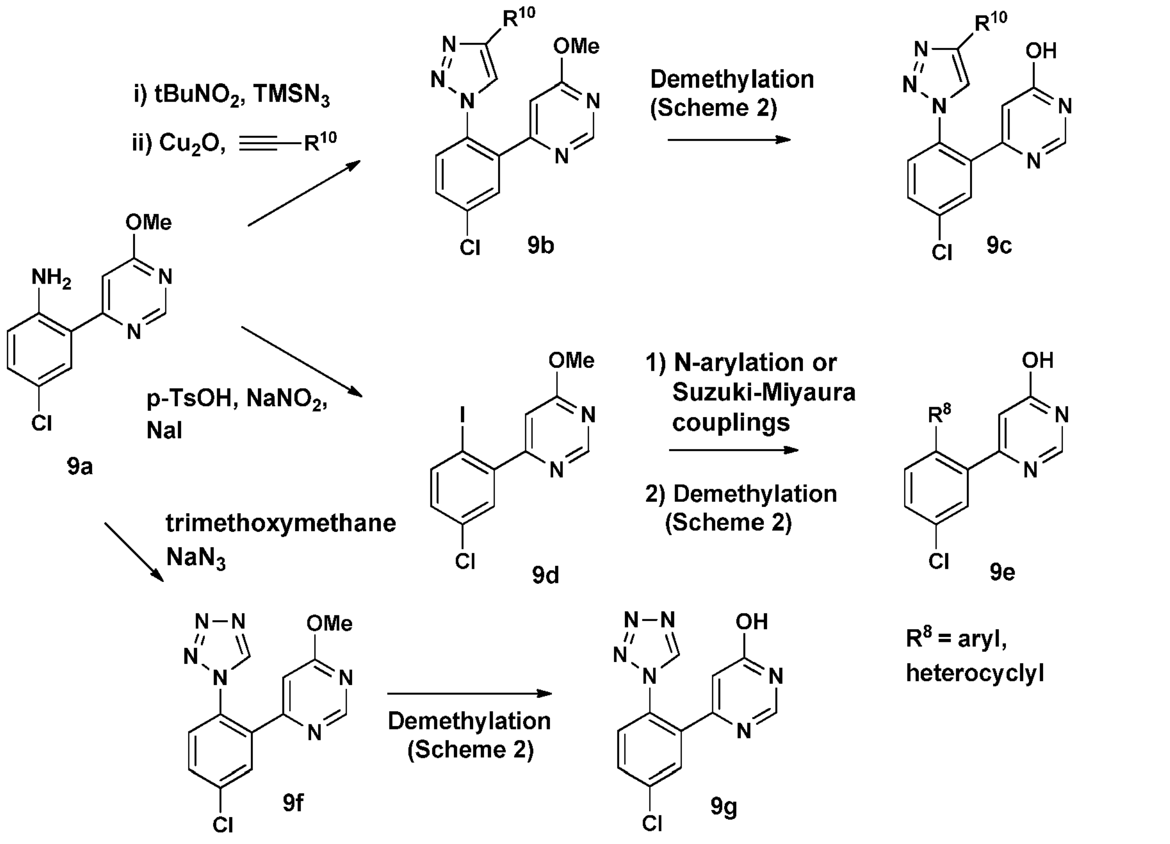


9a 9b 9c 9d 9e 9f 9g

Scheme 10 describes the synthesis of suitably substituted pyrimidin-4-ol derivatives where $R^8$ is a thiadiazole. Bromide 10a can be converted to acetyl compound **10b** by coupling with 1-(trimethylsilyl)ethanone with Pd catalyst. **10b**

can react with ethyl hydrazinecarboxylate to form 10c, which upon treatment with $SOCl_2$ to give thiadiazole compound **10d.** Intermediate **10e** can be obtained by demethylation of **10d** according to Scheme 2.

Scheme 10

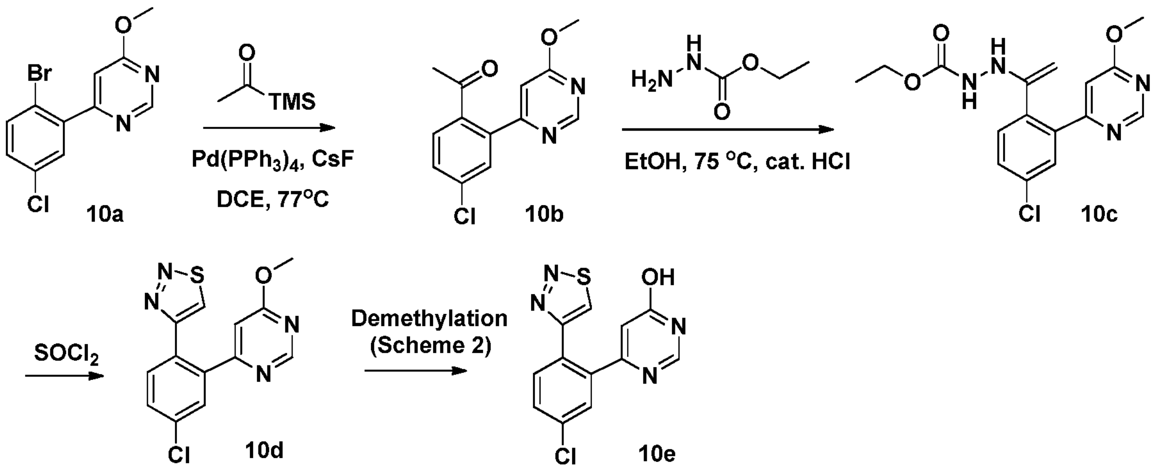


10a 10b 10c 10d 10e

Representative synthesis of compounds in this invention where ring A is methoxy-pyridine is outlined in Scheme 11. Benzaldehyde **11a** which was used in a Horner-Wadsworth-Emmons reaction with (S)-*tert*-butyl (1-(dimethoxyphosphoryl)-2-oxohex-5-en-3-yl)carbamate (synthesis previously described) to afford **11b.** Then, enone **11b** was converted into key intermediate **11c** by treatment with $NH_4OAc$ and separated by chiral chromatography to **11d1** and **11d2.** Methylation of chiral separation product **11d2** gave 2-methyoxy pyridine **11e**. Zn mediated reduction of nitro group afforded aniline **11f**. Coupling of aniline **11f** with the 2-methylbut-3-enoic acid by methods known in the art of synthesis resulted in formation of **11g**. The following ring closing metathesis formed two isomers **11h1** and **11h2**. Hydrogenation and deprotection of **11h1** and **11h2** gave the crucial intermediate **11l1** and **11l2** which can be coupled to afford compounds of this invention.

Scheme 11

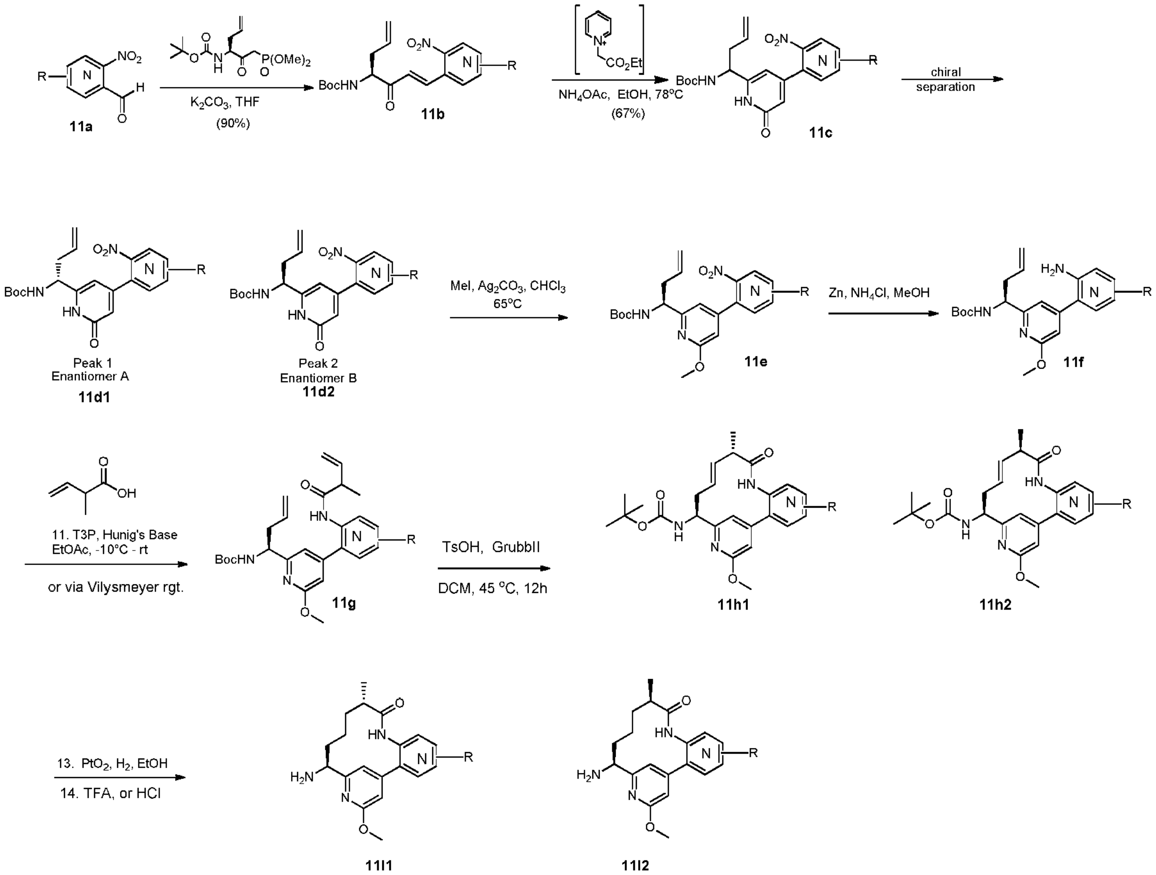


The corresponding pyridone compounds of this invention can also be prepared by the methodologies outlined in Schemes 12 to 14.

Scheme 12

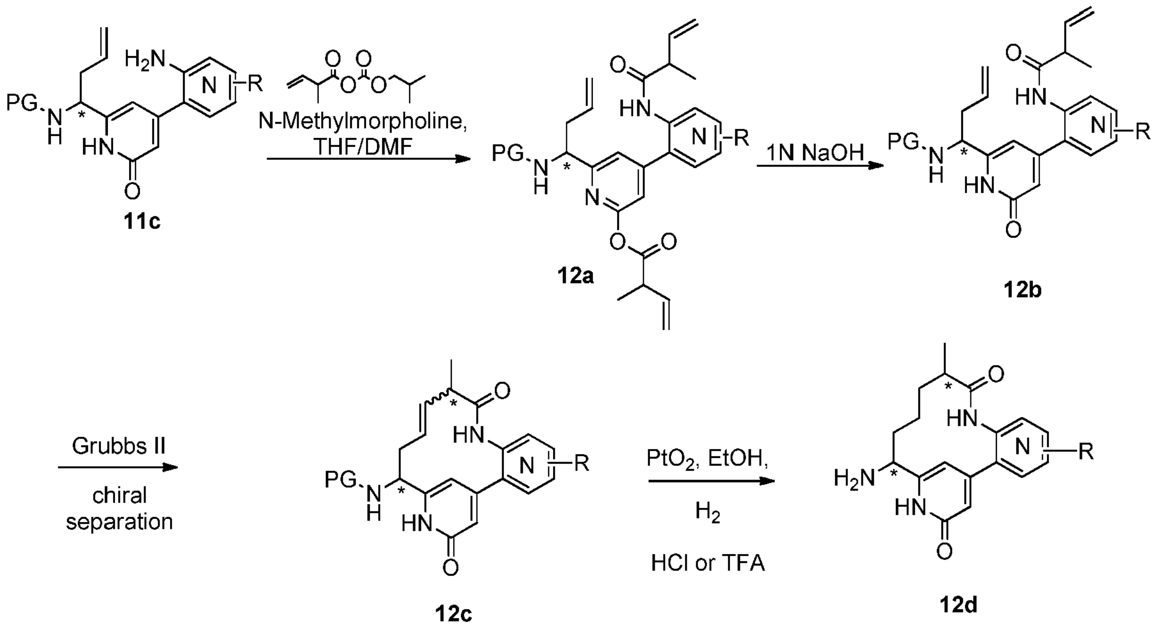

Scheme 13

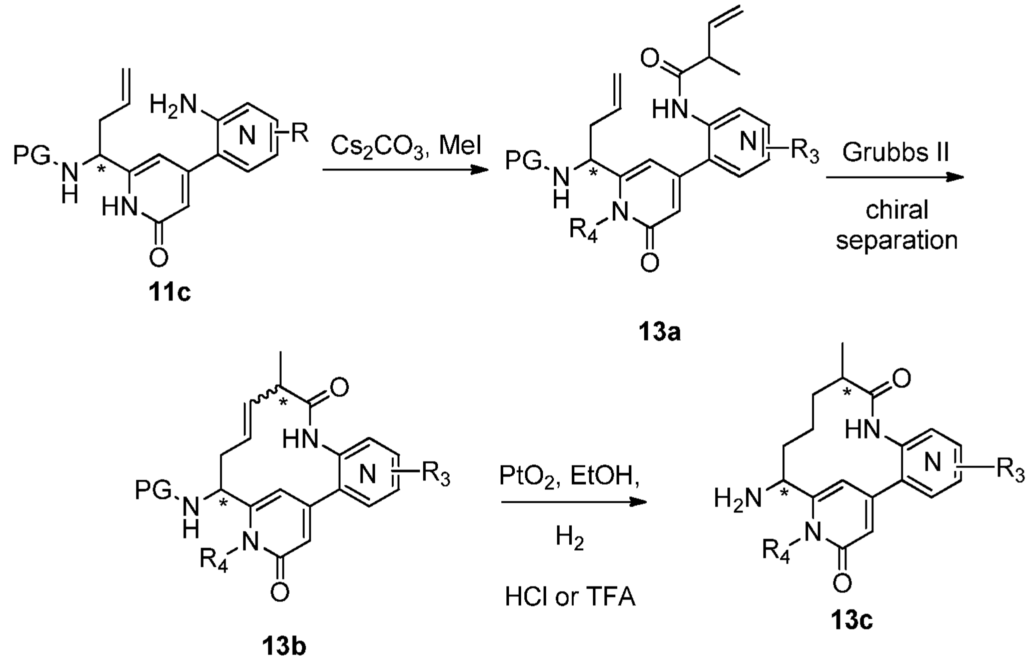


11c 13a 13b 13c

Scheme 14

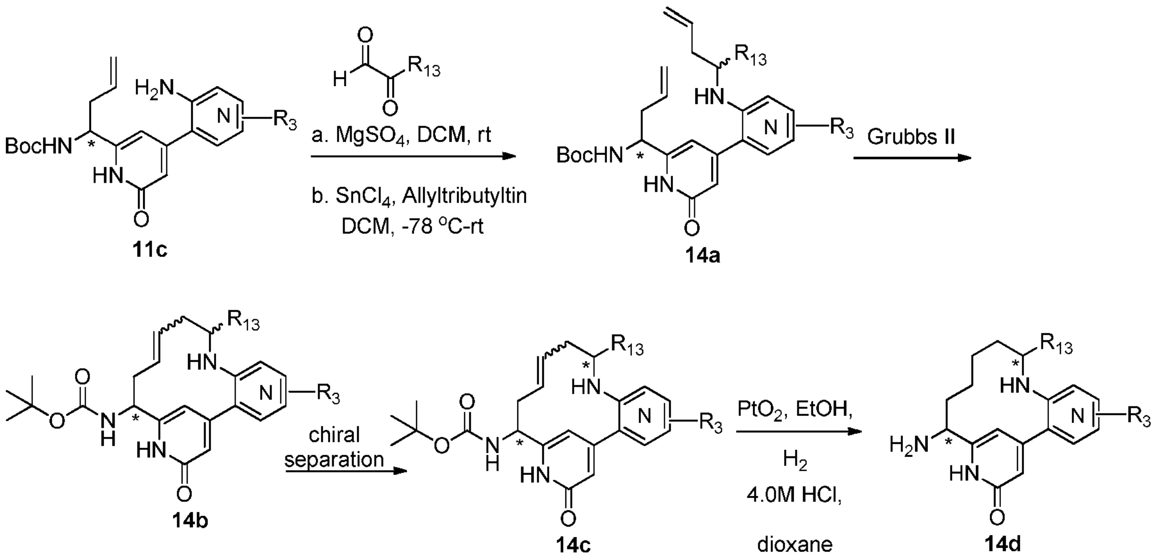


11c 14a 14b 14c 14d

Other azole containing compounds of this invention can also be accessed via the schematic shown in Scheme 15 by following the procedure outlined for the pyridine/one ring systems.

Scheme 15

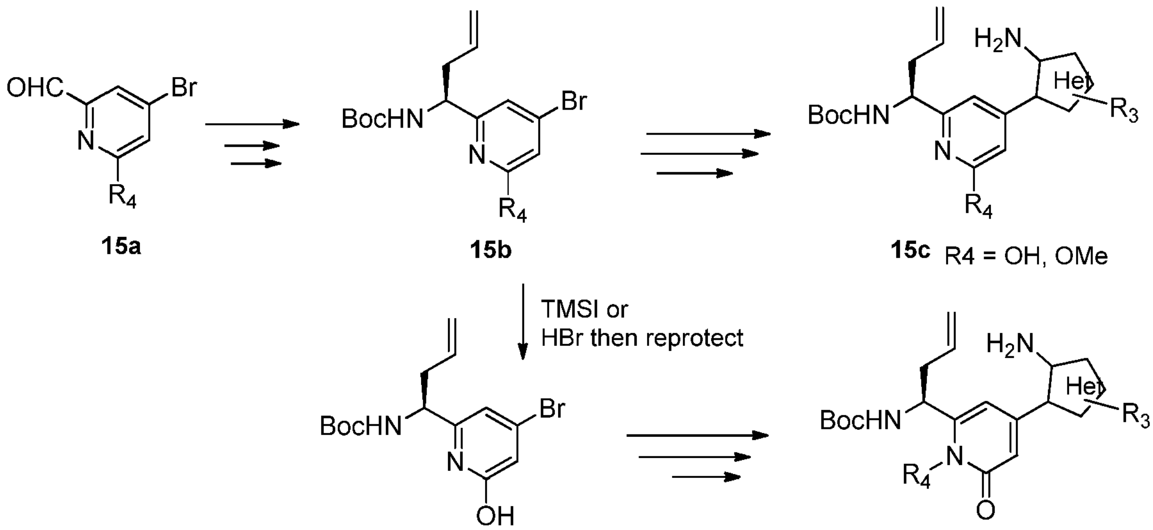


15a 15b 15c R4 = OH, OMe

15d 15e

Purification of intermediates and final products was carried out via either normal or reverse phase chromatography. Normal phase chromatography was carried out using pre-packed $SiO_2$ cartridges eluting with either gradients of hexanes and EtOAc or DCM and MeOH unless otherwise indicated. Reverse phase preparative HPLC was carried out using C18 columns eluting with gradients of Solvent A (90% water, 10% MeOH, 0.1% TFA) and Solvent B (10% water, 90% MeOH, 0.1% TFA, UV 220 nm) or with gradients of Solvent A (90% water, 10% ACN, 0.1% TFA) and Solvent B (10% water, 90% ACN, 0.1% TFA, UV 220 nm) or with gradients of Solvent A (98% water, 2% ACN, 0.05% TFA) and Solvent B (98% ACN, 2% water, 0.05% TFA, UV 220 nm) (or) SunFire Prep C18 OBD 5μ 30x100mm, 25 min gradient from 0-100% B. A = $H_2O$/ACN/TFA 90:10:0.1. B = ACN/$H_2O$/TFA 90:10:0.1

Unless otherwise stated, analysis of final products was carried out by reverse phase analytical HPLC.

Method A: Waters SunFire column (3.5 μm C18, 3.0 x 150 mm). Gradient elution (0.5 mL/min) from 10-100% Solvent B for 12 min and then 100% Solvent B for 3 min was used. Solvent A is (95% water, 5% acetonitrile, 0.05% TFA) and Solvent B is (5% water, 95% acetonitrile, 0.05% TFA, UV 254 nm).

Method B: Waters Acquity UPLC BEH C18, 2.1 x 50 mm, 1.7-μm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0-100% B over 3 minutes, then a 0.75-minute hold at 100% B; Flow: 1.11 mL/min.

Method C: Waters Acquity UPLC BEH C18, 2.1 x 50 mm, 1.7-μm particles; Mobile Phase A: 5:95 acetonitrile:water with 0.1% TFA; Mobile Phase B: 95:5 acetonitrile:water with 0.1% TFA; Temperature: 50 °C; Gradient: 0-100% B over 3 minutes, then a 0.75-minute hold at100% B; Flow: 1.11 mL/min

Method X: ZORBAX® SB C18 column (4.6x75mm). Gradient elution (2.5 mL/min) from 0-100% Solvent B for 8 min and then 100% Solvent B for 2 min was used. Solvent A is (90% water, 10% MeOH, 0.02% $H_3PO_4$) and Solvent B is (10% water, 90% MeOH, 0.02% $H_3PO_4$, UV 220 nm).

Intermediate 1

Preparation of *N*-(4-chloro-2-(6-hydroxypyrimidin-4-yl)phenyl)-2,2,2-trifluoroacetamide

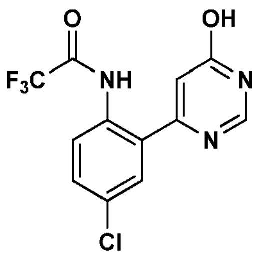

1A. Preparation of *N*-(4-chloro-2-(6-methoxypyrimidin-4-yl)phenyl)-2,2,2-trifluoroacetamide

$Et_3N$ (2.1 mL, 15.3 mmol) was added to a solution of 4-chloro-2-(6-methoxypyrimidin-4-yl)aniline (3 g, 12.7 mmol) and TFAA (2.2 mL, 15.3 mmol) in DCM (100 mL). The solution was stirred for 1 h at rt. The solution was then concentrated to about 15 mL volume and purified by normal phase silica gel chromatography (hexanes-EtOAc gradient) to yield *N*-(4-chloro-2-(6-methoxypyrimidin-4-yl)phenyl)-2,2,2-trifluoroacetamide (4 g, 12.06 mmol, 95% yield) as a white powder.

1B. Preparation of *N*-(4-chloro-2-(6-hydroxypyrimidin-4-yl)phenyl)-2,2,2-trifluoroacetamide

A clear, orange solution of *N*-(4-chloro-2-(6-methoxypyrimidin-4-yl)phenyl)-2,2,2-trifluoroacetamide (3.2 g, 9.65 mmol) in HOAc (20 ml) and 48% aq HBr (5.5 ml, 48.2 mmol) was warmed to 60 °C for 1.5 h. The reaction was cooled to rt and the solvents were removed *in vacuo.* EtOAc (100 mL) and sat aq $NaHCO_3$ were added to the residue. The aqueous layer was then extracted twice with EtOAc (50 mL). The combined organic layers were dried with $MgSO_4$ and concentrated. The residue was triturated with $Et_2O$ and filtered to yield *N*-(4-chloro-2-(6-hydroxypyrimidin-4-yl)phenyl)-2,2,2-trifluoroacetamide (1.2 g, 3.78 mmol, 39.2% yield) as a white powder.

Intermediate 2

Preparation of (*R*)-2-methylbut-3-enoic acid

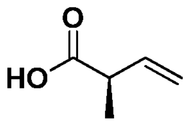

2A. Preparation of (*R*)-4-benzyl-3-((*R*)-2-methylbut-3-enoyl)oxazolidin-2-one

To the solution of 2-methylbut-3-enoic acid (5.59 g, 55.9 mmol) and NMM (6.14 mL, 55.9 mmol) in THF (62 mL) at 0 °C was added pivaloyl chloride (6.87 mL, 55.9 mmol) dropwise. The reaction mixture was cooled down to -78 °C, and stirred for ~2 h. In a separate flask: To the solution of (*R*)-4-benzyloxazolidin-2-one (8.25 g, 46.6 mmol) in THF (126 mL) at -78 °C was added 2.5 M nBuLi in hexane (20.49 mL, 51.2 mmol) dropwise. After 35 min, this reaction was transferred *via* cannula to the first reaction. The reaction mixture was stirred at -78 °C for 2 h, then the cold bath was removed, and the reaction was quenched with sat $NH_4Cl$. The reaction was diluted with water and extracted with EtOAc (3x). The combined organic layers were washed with brine, dried over $Na_2SO_4$, filtered, and concentrated to give a yellow oil (15 g). Purification by silica gel chromatography afforded (*R*)-4-benzyl-3-((*R*)-2-methylbut-3-enoyl)oxazolidin-2-one (6.59 g, 55%) as a colorless oil. MS(ESI) *m/z:* 282.1 $(M+Na)^+$. $^1H$ NMR (500 MHz, $CDCl_3$) $\delta$ 7.36 - 7.19 (m, 5H), 6.03 - 5.93 (m, 1H), 5.23 - 5.10 (m, 2H), 4.69 - 4.63 (m, 1H), 4.51 - 4.43 (m, 1H), 4.23 - 4.15 (m, 2H), 3.29 (dd, *J* = 13.5, 3.3 Hz, 1H), 2.79 (dd, *J* = 13.5, 9.6 Hz, 1H), 1.35 (d, *J* = 6.9 Hz, 3H) ppm. The other diastereomer (*R*)-4-benzyl-3-((*S*)-2-methylbut-3-enoyl)oxazolidin-2-one (4.6 g, 38%) was also obtained as a white solid. MS(ESI) *m/z:* 260.1 $(M+H)^+$.

2B. Preparation of (*R*)-2-methylbut-3-enoic acid

To a clear colorless solution of (*R*)-4-benzyl-3-((*R*)-2-methylbut-3-enoyl) oxazolidin-2-one (6.05 g, 23.33 mmol) in THF (146 mL) at 0 °C was added dropwise 30% aq $H_2O_2$ (9.53 mL, 93 mmol) followed by 2 N LiOH (23.33 mL, 46.7 mmol). After 30 min, the reaction was quenched with 25 mL of sat $Na_2SO_3$ and 25 mL of sat $NaHCO_3$. The reaction was

then concentrated to remove the THF. The residue was diluted with water and extracted with $CHCl_3$ (3x). The aqueous layer was acidified with conc. HCl to pH ~3 and then it was extracted with EtOAc (3x). The EtOAc layers were combined, washed with brine, dried over $MgSO_4$, filtered and concentrated to afford (R)-2-methylbut-3-enoic acid (2.15 g, 92%) as a colorless oil. $^1H$ NMR (500 MHz, $CDCl_3$) $\delta$ 10.84 (br. s., 1H), 5.94 (ddd, $J$ = 17.4, 10.1, 7.4 Hz, 1H), 5.22 - 5.13 (m, 2H), 3.23 - 3.15 (m, 1H), 1.31 (d, $J$ = 7.2 Hz, 3H) ppm.

Intermediate 3

Preparation of 6-(3-chloro-2-fluorophenyl)pyrimidin-4-ol

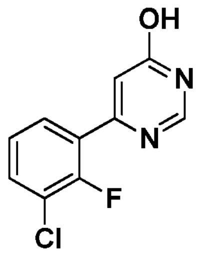

A microwave vial containing 6-chloropyrimidin-4-ol (0.100 g, 0.766 mmol), (3-chloro-2-fluorophenyl)boronic acid (0.534 g, 3.06 mmol), and $Pd(PPh_3)_4$ (0.089 g, 0.077 mmol) was purged with Ar for several min. Then degassed toluene (1.53 mL) and EtOH (1.53 mL) were added followed by DIEA (0.54 mL, 3.06 mmol). The vial was capped and the reaction was microwaved at 120 °C for 1 h. The resulting clear, orange solution was allowed to cool to rt and a precipitate formed. The yellow solid was removed by filtration, rinsing with 1:1 toluene/EtOH. A precipitate formed in the filtrate. The solid was collected by filtration, rinsed with cold 1:1 toluene/EtOH, air-dried, and dried under vacuum to give 6-(3-chloro-2-fluorophenyl)pyrimidin-4-ol (0.0357 g, 21% yield) as a white solid. MS(ESI) *m/z:* 225.1 $(M+H)^+$ and 227.1 $(M+2+H)^+$. $^1H$ NMR (500MHz, DMSO-$d_6$) $\delta$ 12.71 (br. s., 1H), 8.31 (d, $J$ = 1.1 Hz, 1H), 7.87 (ddd, $J$=8.0, 7.2, 1.7 Hz, 1H), 7.74 - 7.69 (m, 1H), 7.36 (td, $J$=8.0, 1.1 Hz, 1H), 6.72 (br. s, 1H). $^{19}F$ NMR (471MHz, DMSO-$d_6$) $\delta$ -117.48.

Intermediate 4

Preparation of 6-(3-chloro-2,6-difluorophenyl)pyrimidin-4-ol, hydrobromide

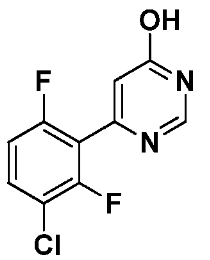

4A. Preparation of 4-(3-chloro-2,6-difluorophenyl)-6-methoxypyrimidine

A flask containing 4-chloro-6-methoxypyrimidine (1.0 g, 6.92 mmol), (3-chloro-2,6-difluorophenyl)boronic acid (1.996 g, 10.38 mmol), and 2nd generation XPhos precatalyst (0.272 g, 0.346 mmol) was purged with Ar for several min, then degassed THF (13.84 mL) and degassed 0.5 M $K_3PO_4$ (27.7 mL, 13.84 mmol) were added. The resulting cloudy, pink reaction mixture was stirred vigorously at rt. After 2 h, the reaction was diluted with water and extracted with EtOAc (2x). The organic layers were combined and washed with brine, dried over $Na_2SO_4$, filtered and concentrated to give an orange-brown residue weighing 1.5 g. Purification by normal phase chromatography gave 4-(3-chloro-2,6-difluorophenyl)-6-methoxypyrimidine (0.242 g, 13.6% yield) as an off-white solid. MS(ESI) *m/z:* 257.0 $(M+H)^+$ and 259.0 $(M+2+H)^+$. $^1H$ NMR (500MHz, $CD_3OD$) $\delta$ 8.86 (d, $J$=1.1 Hz, 1H), 7.68 - 7.63 (m, 1H), 7.17 (td, $J$=9.0, 1.8 Hz, 1H), 7.10

- 7.08 (m, 1H), 4.07 (s, 3H). $^{19}$F NMR (471MHz, $CD_3OD$) δ -115.84 (d, *J*=4.3 Hz), - 116.49 (d, *J*=5.7 Hz).

4B. Preparation of 6-(3-chloro-2,6-difluorophenyl)pyrimidin-4-ol

A clear, yellow solution of 4-(3-chloro-2,6-difluorophenyl)-6-methoxypyrimidine (0.240 g, 0.935 mmol) in AcOH (9.35 mL) and 48% aq HBr (5.29 mL, 46.8 mmol) was warmed to 85 °C. After 1 h, the reaction was cooled to rt and then it was concentrated to give a yellow solid. $Et_2O$ (10 mL) was added resulting in a suspension. The solid was collected by filtration, rinsed with $Et_2O$, air-dried, and then dried under vacuum to give 6-(3-chloro-2,6-difluorophenyl)pyrimidin-4-ol (0.258 g, 85% yield) as an off-white solid. MS(ESI) *m/z:* 243.0 $(M+H)^+$ and 245.0 $(M+2+H)^+$. $^1$H NMR (500MHz, DMSO-$d_6$) δ 8.33 (d, *J*=1.1 Hz, 1H), 7.77 (td, *J*=8.7, 5.6 Hz, 1H), 7.32 (td, *J*=9.1, 1.7 Hz, 1H), 6.63 (d, *J*=0.6 Hz, 1H). $^{19}$F NMR (471MHz, DMSO-$d_6$) δ -113.79 (d, *J*=4.3 Hz), -113.88 (d, J=5.7 Hz).

Intermediate 5

Preparation of 6-(5-chloro-2-fluorophenyl)pyrimidin-4-ol

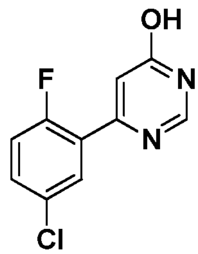

5A. Preparation of 4-(5-chloro-2-fluorophenyl)-6-methoxypyrimidine

A microwave vial containing 4-chloro-6-methoxypyrimidine (0.290 g, 2.007 mmol), (5-chloro-2-fluorophenyl)boronic acid (0.35 g, 2.007 mmol) and $Na_2CO_3$ (0.213 g, 2.007 mmol) in DME (10 mL), EtOH (1.250 mL) and water (1.250 mL) was purged with $N_2$ for several min. Then $PdCl_2$(dppf)-$CH_2Cl_2$ adduct (0.082 g, 0.100 mmol) was added and the vial was capped. The reaction was heated in a microwave at 100 °C for 1 h. The reaction mixture was then diluted with water and extracted with EtOAc. The organic layer was washed with brine and then concentrated to give an orange-brown residue. Purification by normal phase chromatography gave 4-(5-chloro-2-fluorophenyl)-6-methoxypyrimidine (400 mg, 84% yield) as white crystals. MS(ESI) *m/z:* 239.3 $(M+H)^+$. $^1$H NMR (400MHz, $CDCl_3$) δ 8.86 (s, 1H), 8.16 (dd, *J*=6.7, 2.8 Hz, 1H), 7.39 (ddd, *J*=8.8, 4.2, 2.9 Hz, 1H), 7.28 - 7.23 (m, 1H), 7.12 (dd, *J*=10.8, 8.8 Hz, 1H), 4.04 (s, 3H).

5B. Preparation of 6-(5-chloro-2-fluorophenyl)pyrimidin-4-ol

A clear, yellow solution of 4-(5-chloro-2-fluorophenyl)-6-methoxypyrimidine (300 mg, 1.257 mmol) in AcOH (12.57 mL) and 48% aq HBr (7 mL, 61.9 mmol) was warmed to 85 °C. After 0.5 h, the reaction was cooled to rt and concentrated under high vacuum to dryness. To the residue was added sat $NaHCO_3$ carefully to give a suspension. The solid was collected by filtration, rinsed with water, a small amount of acetone and air dried to give 6-(5-chloro-2-fluorophenyl)pyrimidin-4-ol (140 mg, 36.5% yield) as a white solid. MS(ESI) *m/z:* 225.2 $(M+H)^+$. $^1$H NMR (400MHz, DMSO-$d_6$) δ 12.73 (br. s., 1H), 8.33 (d, *J*=0.9 Hz, 1H), 7.99 (dd, *J*=6.6, 2.9 Hz, 1H), 7.61 (ddd, *J*=6.6, 4.3, 2.1 Hz, 1H), 7.43 (dd, *J*=11.1, 8.9 Hz, 1H), 6.76 (s, 1H).

Intermediate 6

Preparation of (*S*)-(2-(1-((*tert*-butoxycarbonyl)amino)but-3-en-1-yl)pyridin-4-yl) boronic acid

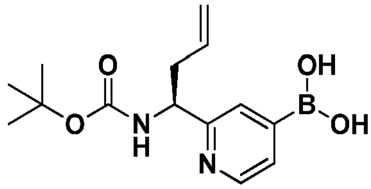

6A. Preparation of 4-chloro-2-[(*E*)-2-[(*S*)-2-methylpropane-2-sulfinyl]ethenyl]pyridine

To a solution of *S*-(-)-*t*-butyl-sulfinamide (0.856 g, 7.06 mmol) in DCM (14.1 mL) was added sequentially $CuSO_4$ (2.481 g, 15.54 mmol) and 4-chloropicolinaldehyde (1.0 g, 7.06 mmol). The resulting white suspension was stirred at rt. After 3 h, the brown suspension was filtered through CELITE®, eluting with DCM, to give a clear brown filtrate. Concentration of the filtrate gave a brown oil weighing 1.85 g. Purification by normal phase chromatography gave 1.31 g of 4-chloro-2-[(E)-2-[(S)-2-methylpropane-2-sulfinyl]ethenyl]pyridine as a clear, yellow oil. MS(ESI) *m/z:* 245.0 $(M+H)^+$.

6B. Preparation of (*S*)-*N*-((*S*)-1-(4-chloropyridin-2-yl)but-3-enyl)-2-methylpropane-2-sulfinamide

To a cooled (0 - 5 °C) mixture of $InCl_3$ (13.56 g, 61.3 mmol) in THF (170 mL) was added dropwise over 30 min 1 M allylmagnesium bromide in $Et_2O$ (62 mL, 61.3 mmol). The reaction was allowed to warm to rt. After 1 h at rt, a solution of chloro-2-[(*E*)-2-[(*S*)-2-methylpropane-2-sulfinyl] ethenyl]pyridine (10 g, 40.9 mmol) in EtOH (170 mL) was added. After 3 h, the reaction was concentrated under vacuum at 50-55 °C. The crude material was partitioned between EtOAc (200 mL) and water (50 mL) and the layers were separated. The aqueous layer was extracted with EtOAc (2 x 50 mL). The organic layers were combined and washed with brine (100 mL), dried over $Na_2SO_4$, filtered and concentrated to give (*S*)-*N*-((*S*)-1-(4-chloropyridin-2-yl)but-3-enyl)-2-methylpropane-2-sulfinamide (13.5 g, 106%) as a yellow oil. MS(ESI) *m*/*z:* 287.2 $(M+H)^+$.

6C. Preparation of (*S*)-*tert*-butyl 1-(4-chloropyridin-2-yl)but-3-enylcarbamate

(*S*)-*N*-((*S*)-1-(4-Chloropyridin-2-yl)but-3-enyl)-2-metliylpropane-2-sulfinamide (75 g, 261 mmol) was dissolved in MeOH (1500 mL). 6 N aq HCl (750 mL, 4.5 mol) was added. The reaction was stirred at rt for 3 h and then was concentrated. The residue was diluted with water (2 L), washed with EtOAc (500 mL). The aqueous layer was basified with sat $Na_2CO_3$ solution and then extracted with EtOAc (3 x 1 L). The combined organic layers were washed with water (1 L) and brine (1 L), dried over $Na_2SO_4$, filtered and concentrated under vacuum at 50-55 °C to give crude product (43 g, 90%). MS(ESI) *m*/*z:* 183.2 $(M+H)^+$. The crude product (42 g, 230 mmol) was dissolved in DCM (420 mL) and $Et_3N$ (32.1 mL, 230 mmol) was added followed by dropwise addition of $Boc_2O$ (53.4 mL, 230 mmol). The reaction was stirred at rt for 3 h. The reaction was diluted with DCM (1 L), washed with water (500 mL) and brine (500 mL). The organic layer was dried over $Na_2SO_4$, filtered, and concentrated. The crude product was then purified using silica gel chromatography to give (*S*)-*tert*-butyl 1-(4-chloropyridin-2-yl)but-3-enylcarbamate (61 g, 86%) as a pale yellow solid. MS(ESI) *m*/*z:* 283.2 $(M+H)^+$.

6D. Preparation of (*S*)-(2-(1-((*tert*-butoxycarbonyl)amino)but-3-en-1-yl)pyridin-4-yl)boronic acid trifluoroacetate

To a solution of 5,5,5',5'-tetramethyl-2,2'-bi(1,3,2-dioxaborinane) (1.198 g, 5.30 mmol) and (*S*)-*tert*-butyl 1-(4-chloropyridin-2-yl)but-3-enylcarbamate (1.0 g, 3.54 mmol), prepared as described in Intermediate 23, in DMSO (10 mL) was added KOAc (1.041 g, 10.61 mmol) and $PdCl_2(dppf)-CH_2Cl_2$ adduct (0.289 g, 0.354 mmol). The reaction was purged with Ar for 10 min. The reaction mixture was then sealed and stirred for 12 h at 85 °C. The reaction mixture was cooled to rt and then it was diluted with EtOAc and washed with water. The aqueous layer was extracted with EtOAc. The organic layers were combined and was washed with brine, dried over $Na_2SO_4$, filtered, and concentrated. Purification by reverse phase chromatography afforded the (*S*)-(2-(1-((*tert*-butoxycarbonyl)amino)but-3-en-1-yl)pyridin-4-yl)boronic acid trifluoroacetate (1.1 g, 77%) as a white solid. MS(ESI) *m*/*z:* 293.2 $(M+H)^+$. $^1H$ NMR (500 MHz, $CD_3OD$) δ 8.54 (d, *J* = 5.8 Hz, 1H), 8.11 (s, 1H), 8.02 (dd, *J* = 5.8, 0.6 Hz, 1H), 5.79 (ddt, *J* = 17.1, 10.2, 7.1 Hz, 1H), 5.11 - 5.03 (m, 2H), 4.86 (t, *J* = 7.0 Hz, 1H), 2.69 - 2.55 (m, 2H), 1.40 (br. s., 9H) ppm.

Intermediate 7

Preparation of 6-(3-chloro-2-fluoro-6-(1H-1,2,3-triazol-1-yl)phenyl)pyrimidin-4-ol

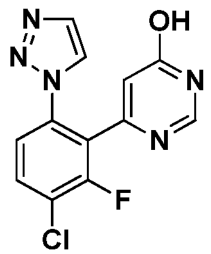

7A. Preparation of *N*-(4-chloro-3-fluorophenyl)-2,2,2-trifluoroacetamide

To a cooled (-10 °C) suspension of 4-chloro-3-fluoroaniline (10.67 g, 73.3 mmol) and $Na_2CO_3$ (13.21 g, 125 mmol) in $Et_2O$ (300 mL) was added dropwise TFAA (12.23 mL, 88 mmol). The mixture was allowed to warm to rt overnight. The mixture was diluted with hexane (300 mL) and filtered. The filtrate was washed with ice water, 10% aq $NaHCO_3$, and brine, dried over $Na_2SO_4$, filtered, and concentrated to give *N*-(4-chloro-3-fluorophenyl)-2,2,2-trifluoroacetamide (17 g, 96% yield), as a pale, yellow solid. MS(ESI) *m/z:* 242.1 $(M+H)^+$.

7B. Preparation of (3-chloro-2-fluoro-6-(2,2,2-trifluoroacetamido)phenyl)boronic acid

To a cooled (-78 °C) clear, colorless solution of *N*-(4-chloro-3-fluorophenyl)-2,2,2-trifluoroacetamide (0.500 g, 2.070 mmol) in THF (8.28 mL) was added dropwise 2.5 M nBuLi in hexane (1.74 mL, 4.35 mmol) over 15 min keeping the internal temperature below -65 °C. The resulting clear, yellow solution was stirred at -78 °C for 10 min. The reaction was allowed to warm to -50 °C over 1 h. The reaction was then cooled to -78 °C and $B(O\text{-}i\text{-}Pr)_3$ (1.051 mL, 4.55 mmol) was added dropwise. The reaction was stirred at -78 °C for 30 min and then the ice bath was removed and the reaction was allowed to warm to rt and stirred at rt for 1 h. After this time, the reaction was cooled to -5 °C and then quenched with the dropwise addition of 1.0 M HCl (5 mL) followed by the addition of water (5 mL). The resulting cloudy yellow reaction mixture was stirred at rt for 45 min. The reaction was diluted with EtOAc and the layers were separated. The organic layer was washed with brine, dried over $Na_2SO_4$, filtered and concentrated to give a pale, orange solid. The solid was partitioned between THF (10 mL) and 0.5 M HCl (20 mL) and stirred vigorously for 4 h. The layers were then separated and the clear, colorless aqueous layer was concentrated to give (3-chloro-2-fluoro-6-(2,2,2-trifluoroacetamido)phenyl)boronic acid (0.1599 g, 34.2% yield) as a white solid. MS(ESI) *m/z:* 189.9 $[M+H]^+$.

7C. Preparation of 4-chloro-3-fluoro-2-(6-methoxypyrimidin-4-yl)aniline

4-Chloro-3-fluoro-2-(6-methoxypyrimidin-4-yl)aniline was prepared according to the procedures described in Intermediate 3 using (3-chloro-2-fluoro-6-(2,2,2-trifluoroacetamido)phenyl)boronic acid. MS(ESI) *m/z:* 253.9 $(M+H)^+$. $^1H$ NMR (500MHz, $CD_3OD$) $\delta$ 8.82 (d, *J*=1.1 Hz, 1H), 7.18 (dd, *J*=8.8, 8.3 Hz, 1H), 7.01 (dd, *J=3.0,* 1.1 Hz, 1H), 6.61 (dd, *J*=8.9, 1.5 Hz, 1H), 4.04 (s, 3H). $^{19}F$ NMR (471MHz, $CD_3OD$) $\delta$ -119.92 (s, IF).

7D. Preparation of 4-(3-chloro-2-fluoro-6-(1H-1,2,3-triazol-1-yl)phenyl)-6-methoxypyrimidine trifluoroacetate

In a microwave vial, 4-chloro-3-fluoro-2-(6-methoxypyrimidin-4-yl)aniline (0.045 g, 0.177 mmol) in $CH_3CN$ (1.8 mL), cooled to 0 °C, was added isoamylnitrite (0.036 mL, 0.266 mmol), followed by the dropwise addition of $TMSN_3$ (0.035 mL, 0.266 mmol). Gas evolution was observed. After 5 min, the cold bath was removed, and the reaction was allowed to warm to rt. After 1 h, trimethylsilylacetylene (0.076 mL, 0.532 mmol) was added. The septum was replaced with a microwave cap and sealed. The reaction was heated in a microwave at 120 °C for a total of 4 h. The reaction was concentrated almost to dryness and then purified by reverse phase chromatography to give 4-(3-chloro-2-fluoro-6-(1H-1,2,3-triazol-1-yl)phenyl)-6-methoxypyrimidine (27 mg, 0.088 mmol) as a clear glass. MS(ESI) *m/z:* 306.3 $(M+H)^+$. $^1H$ NMR (400MHz, $CDCl_3$) $\delta$ 8.74 (d, *J*=0.4 Hz, 1H), 7.80 (d, *J*=0.9 Hz, 1H), 7.77 - 7.69 (m, 2H), 7.38 (dd, J=8.6, 1.5 Hz, 1H), 6.88 (s, 1H), 4.06 (s, 3H). $^{19}F$ NMR (376MHz, $CDCl_3$) $\delta$ -76.02 (s), - 112.27 (s).

7E. Preparation of 6-(3-chloro-2-fluoro-6-(1H-1,2,3-triazol-1-yl)phenyl)pyrimidin-4-ol

6-(3-Chloro-2-fluoro-6-(1H-1,2,3-triazol-1-yl)phenyl)pyrimidin-4-ol was prepared according to the procedures in described in Intermediate 5 for the synthesis of 6-(5-chloro-2-fluorophenyl)pyrimidin-4-ol, by replacing 4-(5-chloro-2-fluorophenyl)-6-methoxypyrimidine with 4-(3-chloro-2-fluoro-6-(1H-1,2,3-triazol-1-yl)phenyl)-6-methoxypyrimidine. MS(ESI) *m/z:* 292.3 $(M+H)^+$. $^1H$ NMR (400MHz, $CD_3OD$) δ 8.20 (d, *J*=1.1 Hz, 1H), 8.06 (d, *J*=0.7 Hz, 1H), 7.89 - 7.81 (m, 1H), 7.80 (d, *J*=0.9 Hz, 1H), 7.54 (dd, *J*=8.6, 1.5 Hz, 1H), 6.52 (s, 1H).

Intermediate 8

Preparation of 6-(5-chloro-2-(1H-1,2,3-triazol-1-yl)phenyl)pyrimidin-4-ol

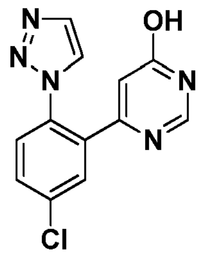

8A. Preparation of 4-chloro-2-(6-methoxypyrimidin-4-yl)aniline

4-Chloro-2-(6-methoxypyrimidin-4-yl)aniline was synthesized according to the procedure described in Intermediate 5, by replacing (5-chloro-2-fluorophenyl)boronic acid with 4-chloro-2-(4,4,5,5-tetramethyl-1,3,2-dioxaborolan-2-yl)aniline. MS(ESI) *m/z:* 236.3 $(M+H)^+$. $^1H$ NMR (400MHz, $CDCl_3$) δ 8.78 (s, 1H), 7.49 (d, *J*=2.4 Hz, 1H), 7.15 (dd, *J*=8.6, 2.4 Hz, 1H), 6.99 (d, *J*=0.9 Hz, 1H), 6.66 (d, *J*=8.6 Hz, 1H), 4.02 (s, 3H).

8B. Preparation of 6-(5-chloro-2-(1H-1,2,3-triazol-1-yl)phenyl)pyrimidin-4-ol

6-(5-Chloro-2-(1H-1,2,3-triazol-1-yl)phenyl)pyrimidin-4-ol was synthesized according to the procedures described for the synthesis of 6-(3-chloro-2-fluoro-6-(1H-1,2,3-triazol-1-yl)phenyl)pyrimidin-4-ol, by replacing 4-chloro-3-fluoro-2-(6-methoxypyrimidin-4-yl)aniline with 4-chloro-2-(6-methoxypyrimidin-4-yl)aniline. MS(ESI) *m/z:* 274.3 $(M+H)^+$. $^1H$ NMR (400MHz, $CD_3OD$) δ 8.51 (d, *J*=0.9 Hz, 1H), 8.35 (d, *J*=1.1 Hz, 1H), 7.92 (d, *J*=1.1 Hz, 1H), 7.88 (d, *J*=2.4 Hz, 1H), 7.83 - 7.78 (m, 1H), 7.74 - 7.69 (m, 1H), 6.39 (d, *J*=0.9 Hz, 1H).

Intermediate 9

Preparation of 6-[5-chloro-2-(4-chloro-1H-1,2,3-tri azol-1-yl)phenyl] pyrimidin-4-ol

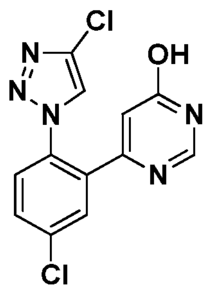

9A. Preparation of 4-chloro-2-(tetramethyl-1,3,2-dioxaborolan-2-yl)aniline

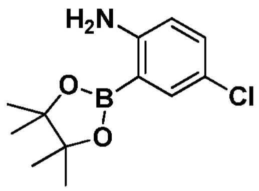

In a 20 mL microwave vial was added 2-bromo-4-chloroaniline (3 g, 14.53 mmol), 4,4,5,5-tetramethyl-2-(tetramethyl-1,3,2-dioxaborolan-2-yl)-1,3,2-dioxaborolane (5.53 g, 21.80 mmol), KOAc (3.66 g, 37.3 mmol), $Pd(dppf)Cl_2$-$CH_2Cl_2$ adduct (0.32 g, 0.44 mmol) and DMSO (9 mL). The resulting suspension was purged with $N_2$, capped and heated at 80 °C for 22 h. The reaction was cooled to rt. Water was added to dissolve the salts, then the reaction was filtered. The remaining solid was suspended in DCM and the insoluble solid was filtered. The filtrate was concentrated and then purified by normal phase chromatography to give 4-chloro-2-(tetramethyl-1,3,2-dioxaborolan-2-yl)aniline (3.15 g, 86% yield) as a white solid. MS(ESI) *m/z:* 172.3 $(M-C_6H_{10}+H)^+$. $^1H$ NMR (400MHz, $CDCl_3$) δ 7.54 (d, *J*=2.6 Hz, 1H), 7.13 (dd, *J*=8.8, 2.6 Hz, 1H), 6.52 (d, *J*=8.6 Hz, 1H), 4.72 (br. s., 2H), 1.34 (s, 12H).

9B. Preparation of 4-chloro-2-(6-methoxypyrimidin-4-yl)aniline

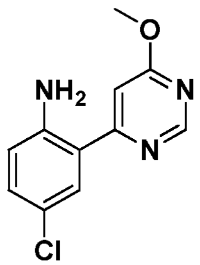

A RBF containing 4-chloro-6-methoxypyrimidine (3.13 g, 21.62 mmol), 4-chloro-2-(tetramethyl-1,3,2-dioxaborolan-2-yl)aniline (7.31 g, 21.62 mmol), $Na_2CO_3$ (2.29 g, 21.62 mmol), DME (86 ml), EtOH (10.81 ml) and water (10.81 ml) was equipped with a condenser. The mixture was purged with Ar for several min then $Pd(dppf)Cl_2$-$CH_2Cl_2$ adduct (1.77 g, 2.16 mmol) was added. The reaction was heated at 90 °C for 5 h. The reaction was cooled to rt, diluted with water and extracted with EtOAc. The organic layer was washed with brine, concentrated and purified by normal phase chromatography to give 4-chloro-2-(6-methoxypyrimidin-4-yl)aniline (2.86 g, 56.1% yield) as yellow solid. MS(ESI) *m/z:* 236.0 $(M+H)^+$. $^1H$ NMR (500MHz, $CDCl_3$) δ 8.78 (d, *J*=1.1 Hz, 1H), 7.49 (d, *J*=2.5 Hz, 1H), 7.15 (dd, *J*=8.8, 2.5 Hz, 1H), 6.99 (d, *J*=1.1 Hz, 1H), 6.67 (d, *J*=8.8 Hz, 1H), 5.89 (br. s., 2H), 4.03 (s, 3H).

9C. Preparation of 4-{5-chloro-2-[4-(trimethylsilyl)-1H-1,2,3-triazol-1-yl] phenyl}-6-methoxypyrimidine

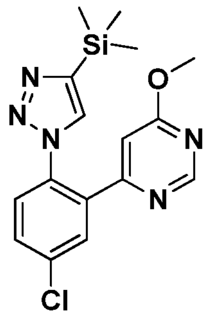

To a solution of 4-chloro-2-(6-methoxypyrimidin-4-yl)aniline (1.5 g, 6.36 mmol) in ACN (90 ml) at 0 °C was added 3-methylbutyl nitrite (1.28 ml, 9.55 mmol), followed by the dropwise addition of $TMSN_3$ (1.26 ml, 9.55 mmol). Gas evolution was observed. After 10 min, the ice bath was removed, and the reaction was allowed to warm to rt. After 1 h, ethynyltrimethylsilane (2.72 ml, 19.09 mmol) and $Cu_2O$ (0.09 g, 0.64 mmol) were added and the reaction was stirred for an additional 1 h. The reaction was partitioned in EtOAc and sat $NH_4Cl$, and the layers were separated. The organic layer was washed with brine, dried over $MgSO_4$, filtered and concentrated. Purification by normal phase chromatography gave 4-{5-chloro-2-[4-(trimethylsilyl)-1H-1,2,3-triazol-1-yl]phenyl}-6-methoxypyrimidine (2.13 g, 5.92 mmol, 93% yield) as a yellow solid. MS(ESI) *m/z:* 360.3 $(M+H)^+$. $^1H$ NMR (400MHz, $CDCl_3$) $\delta$ 8.71 (d, *J*=1.1 Hz, 1H), 7.82 (d, *J*=2.2 Hz, 1H), 7.61 - 7.56 (m, 1H), 7.54 - 7.48 (m, 2H), 6.20 (d, *J*=1.1 Hz, 1H), 3.92 (s, 3H), 0.32 - 0.28 (m, 9H).

9D. Preparation of 4-[5-chloro-2-(4-chloro-1H-1,2,3-triazol-1-yl)phenyl]-6-methoxypyrimidine

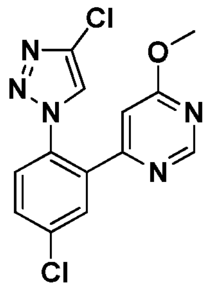

To a solution of 4-{5-chloro-2-[4-(trimethylsilyl)-1H-1,2,3-triazol-1-yl] phenyl}-6-methoxypyrimidine (1.56 g, 4.33 mmol) in ACN (28.9 ml) was added NCS (2.03 g, 15.17 mmol) and silica gel (6.51 g, 108 mmol). The reaction was stirred at 80 °C for 1 h. Then, the reaction was filtered to remove the silica gel and the collected silica gel was washed with EtOAc. The filtrate was washed with water (2x), brine and concentrated. Purification by normal phase chromatography gave 4-[5-chloro-2-(4-chloro-1H-1,2,3-triazol-1-yl)phenyl]-6-methoxypyrimidine (0.90 g, 64.5% yield) as a yellow foam. MS(ESI) *m/z:* 322.3 $(M+H)^+$. $^1H$ NMR (400MHz, $CDCl_3$) $\delta$ 8.70 (d, *J*=1.1 Hz, 1H), 7.75 (d, *J*=2.4 Hz, 1H), 7.66 - 7.55 (m, 2H), 7.50 (d, *J*=8.6 Hz, 1H), 6.52 (d, *J*=0.9 Hz, 1H), 3.98 (s, 3H).

9E. Preparation of 6-[5-chloro-2-(4-chloro-1H-1,2,3-triazol-1-yl)phenyl] pyrimidin-4-ol

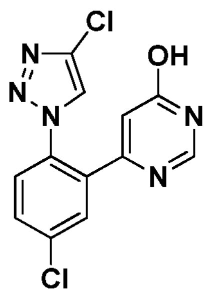

To a solution of 4-[5-chloro-2-(4-chloro-1H-1,2,3-triazol-1-yl)phenyl]-6-methoxypyrimidine (900 mg, 2.79 mmol) in AcOH (6 ml) was added 48% aq HBr (3 ml, 26.5 mmol). The mixture was stirred at 85 °C for 1 h. The reaction was concentrated to dryness and then partitioned between EtOAc and sat $NaHCO_3$. The mixture was separated and the aqueous layer was extracted with EtOAc (2x). The organic layers were combined, concentrated, and then the residue was purified by normal phase chromatography to give a white solid. The solid was suspended in $Et_2O$, filtered and washed with $Et_2O$ to give 6-[5-chloro-2-(4-chloro-1H-1,2,3-triazol-1-yl)phenyl] pyrimidin-4-ol (610 mg, 70.9% yield) as a white solid. MS(ESI) *m/z:* 308.3 $(M+H)^+$. $^1H$ NMR (400MHz, $CDCl_3$) $\delta$ 7.96 (s, 1H), 7.74 - 7.67 (m, 2H), 7.62 (dd, *J*=8.5, 2.3 Hz, 1H), 7.47 (d, *J*=8.4 Hz, 1H), 6.44 (d, *J*=0.9 Hz, 1H).

Intermediate 10

Preparation of 6-(3-chloro-6-(4-chloro-1H-1,2,3-triazol-1-yl)-2-fluorophenyl) pyrimidin-4-ol

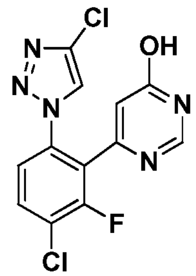

10A. Preparation of N-(4-chloro-3-fluorophenyl)-2,2,2-trifluoroacetamide

To a suspension of 4-chloro-3-fluoroaniline (10.67 g, 73.3 mmol) and $Na_2CO_3$ (24.5 g, 125 mmol) in $Et_2O$ (300 mL) at -10 °C under $N_2$ was added TFAA (12.23 mL, 88 mmol) dropwise. The mixture was allowed to warm to rt and then stirred for 18 h. The reaction mixture was diluted with hexane (300 mL) and filtered. The filtrate was washed with ice water, 10% aq $NaHCO_3$, and brine, dried over $Na_2SO_4$, and concentrated. A pale yellow solid obtained as N-(4-chloro-3-fluorophenyl)-2,2,2-trifluoroacetamide (17 g, 96% yield). MS(ESI) *m*/*z:* 242.1 $(M+H)^+$.

10B. Preparation of (6-amino-3-chloro-2-fluorophenyl)boronic acid

To a cooled (-78 °C) clear, colorless solution of N-(4-chloro-3-fluorophenyl)-2,2,2-trifluoroacetamide (5 g, 20.70 mmol) in THF (69.0 ml) was added dropwise 2.5 M BuLi in hexane (16.56 ml, 41.4 mmol) over 15 min, keeping the internal temperature below -60 °C. The resulting clear, yellow solution was stirred at -78 °C for 10 min, then the reaction was allowed to warm to -50 °C over 1 h. The resulting clear brown solution was cooled to -78 °C and then $B(O\text{-}i\text{Pr})_3$ (10.51 ml, 45.5 mmol) was added dropwise. The reaction was stirred at -78 °C for 10 min, and then the ice bath was removed and the reaction was allowed to warm to rt. The resulting orange suspension was stirred at rt for 2 h, then cooled in ice bath and quenched with 1 N HCl (40 ml). The reaction mixture was warmed to 40 °C for 1 h and then cooled to rt. The reaction was diluted with EtOAc and the layers were separated. The organic layer was washed with brine and concentrated. Purification by normal phase chromatography afforded (6-amino-3-chloro-2-fluorophenyl)boronic acid (3 g, 76.6% yield). MS(ESI) *m*/*z:* 190.1 $(M+H)^+$.

10C. Preparation of 4-chloro-3-fluoro-2-(6-methoxypyrimidin-4-yl)aniline

Reaction was done in a 350 ml pressure bottle. A solution of 4-chloro-6-methoxypyrimidine (1.784 g, 12.34 mmol), (6-amino-3-chloro-2-fluorophenyl)boronic acid (3.3 g, 12.34 mmol) in toluene (25 ml) and EtOH (25 ml) was purged with $N_2$ for several min. DIEA (4.31 ml, 24.68 mmol) followed by $Pd(Ph_3P)_4$ (1.426 g, 1.234 mmol) were added. The flask was capped and the reaction was heated at 120 °C for 2 h, then cooled to rt, and concentrated. Purification by normal phase chromatography afforded 4-chloro-3-fluoro-2-(6-methoxypyrimidin-4-yl)aniline (2 g, 45.2% yield) as a yellow solid. MS(ESI) *m*/*z:* 254.0 $(M+H)^+$.

10D. Preparation of 4-(3-chloro-2-fluoro-6-(4-(trimethylsilyl)-1H-1,2,3-triazol-1-yl)phenyl)-6-methoxypyrimidine

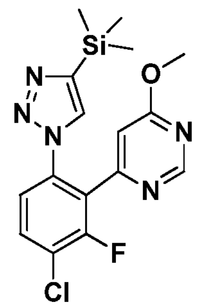

To a cooled (0 °C), clear, yellow solution of 4-chloro-3-fluoro-2-(6-methoxypyrimidin-4-yl)aniline (2.1 g, 8.28 mmol) in ACN (118 ml) was added isoamylnitrite (1.67 ml, 12.42 mmol), followed by the dropwise addition of $TMSN_3$ (1.63 ml, 12.42 mmol). After 10 min, the cold bath was removed, and the reaction was allowed to warm to rt. After 2 h, ethynyltrimethylsilane (3.54 ml, 24.84 mmol) and $Cu_2O$ (0.118 g, 0.83 mmol) were added, and the reaction was stirred at rt for 1.5 h. The reaction was then diluted with EtOAc and washed with sat $NH_4Cl$, brine, dried over $MgSO_4$, filtered and concentrated to give a brown oil. Purification by normal phase chromatography afforded 4-(3-chloro-2-fluoro-6-(4-(trimethylsilyl)-1H-1,2,3-triazol-1-yl)phenyl)-6-methoxypyrimidine (2.71 g, 87% yield) as a brown solid. MS(ESI) *m/z:* 378.1 $(M+H)^+$.

10E. Preparation of 4-(3-chloro-6-(4-chloro-1H-1,2,3-triazol-1-yl)-2-fluorophenyl)-6-methoxypyrimidine

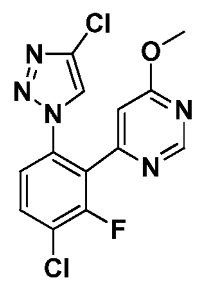

In a RBF equipped with stirring bar and condenser was added 4-(3-chloro-2-fluoro-6-(4-(trimethylsilyl)-1H-1,2,3-triazol-1-yl)phenyl)-6-methoxypyrimidine (2.71 g, 7.17 mmol), NCS (3.35 g, 25.1 mmol), and silica gel (10.77 g, 179 mmol), followed by ACN (47.8 ml). The reaction was heated at 80 °C for 1 h, and then cooled to rt. The reaction was filtered, and the filtrate was concentrated. The residue was redissolved in EtOAc and washed with sat $NaHCO_3$, water, brine, and concentrated. Purification by normal phase chromatography afforded 4-(3-chloro-6-(4-chloro-1H-1,2,3-triazol-1-yl)-2-fluorophenyl)-6-methoxypyrimidine (1.05 g, 43.0% yield) as a yellow solid. MS(ESI) *m/z:* 340.0 $(M+H)^+$. $^1H$ NMR (400MHz, $CDCl_3$) $\delta$ 8.68 (d, *J*=0.7 Hz, 1H), 7.71 - 7.62 (m, 2H), 7.37 (dd, *J*=8.6, 1.8 Hz, 1H), 6.84 (s, 1H), 4.02 (s, 3H).

10F. Preparation of 6-(3-chloro-6-(4-chloro-1H-1,2,3-triazol-1-yl)-2-fluorophenyl) pyrimidin-4-ol.

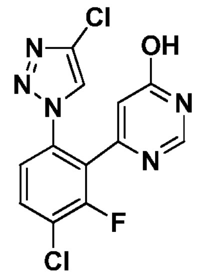

A clear, yellow solution of 4-(3-chloro-6-(4-chloro-1H-1,2,3-triazol-1-yl)-2-fluorophenyl)-6-methoxypyrimidine (1.05 g, 3.09 mmol) in HOAc (15.43 ml) and 48% aq HBr (17.46 ml, 154 mmol) was warmed to 65 °C for 3 h, and then

cooled to rt and concentrated. The yellow gum was suspended in EtOAc and washed with sat $NaHCO_3$ (2x), brine, dried over $Na_2SO_4$, filtered, and concentrated. To the residue was added $Et_2O$ (10 ml), and the resulting suspension was sonicated then filtered. The solid was rinsed with $Et_2O$ (2 ml), air-dried with suction to afford 6-(3-chloro-6-(4-chloro-1H-1,2,3-triazol-1-yl)-2-fluorophenyl)pyrimidin-4-ol (0.79 g, 78% yield) as a white solid. MS(ESI) *m/z:* 326.3 $(M+H)^+$. $^1$H NMR (400MHz, $CD_3OD$) $\delta$ 8.35 (s, 1H), 8.08 (d, *J*=0.7 Hz, 1H), 7.85 (dd, *J*=8.7, 7.6 Hz, 1H), 7.54 (dd, *J*=8.6, 1.5 Hz, 1H), 6.57 (s, 1H).

Intermediate 11

Preparation of 6-(3-chloro-2-fluoro-6-(4-(trifluoromethyl)-1H-1,2,3-triazol-1-yl)phenyl)pyrimidin-4-ol

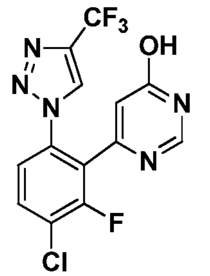

11A. Preparation of 4-(3-chloro-2-fluoro-6-(4-(trifluoromethyl)-1H-1,2,3-triazol-1-yl)phenyl)-6-methoxypyrimidine

To a cooled (0 °C), clear, yellow solution of 4-chloro-3-fluoro-2-(6-methoxypyrimidin-4-yl)aniline (0.2 g, 0.79 mmol) in ACN (11.26 ml) was added isoamylnitrite (0.16 mL, 1.18 mmol), followed by the dropwise addition of $TMSN_3$ (0.16 mL, 1.18 mmol). After 10 min, the cold bath was removed, and the reaction was allowed to warm to rt. After 2 h, $Cu_2O$ (0.011 g, 0.079 mmol) was added. 3,3,3-Trifluoroprop-1-yne (0.5 mL, 0.79 mmol) gas was bubbled in through the reaction for 5 min, then the reaction was capped and stirred at rt. After 1 h, the reaction was diluted with EtOAc and washed with sat $NH_4Cl$, brine, dried over $MgSO_4$, filtered and concentrated to give a brown oil. Purification by normal phase chromatography afforded 4-(3-chloro-2-fluoro-6-(4-(trifluoromethyl)-1H-1,2,3-triazol-1-yl)phenyl)-6-methoxypyrimidine (0.24 g, 81% yield) as a yellow solid. MS(ESI) *m/z:* 374.3 $(M+H)^+$.

11B. Preparation of 6-(3-chloro-2-fluoro-6-(4-(trifluoromethyl)-1H-1,2,3-triazol-1-yl)phenyl)pyrimidin-4-ol

A clear, yellow solution of 4-(3-chloro-2-fluoro-6-(4-(trifluoromethyl)-1H-1,2,3-triazol-1-yl)phenyl)-6-methoxypyrimidine (0.1 g, 0.268 mmol) in HOAc (1.34 ml) and 48% aq HBr (1.51 ml, 13.38 mmol) was warmed to 65 °C for 3 h, and then cooled to rt and concentrated. The yellow gum was suspended with EtOAc, washed with sat $NaHCO_3$ (2x), brine, dried over $Na_2SO_4$, filtered, and concentrated. To the residue was added $Et_2O$ (3 ml) and the resulting suspension was sonicated, then filtered. The solid was rinsed with $Et_2O$ (2 ml), air-dried with suction to afford 6-(3-chloro-2-fluoro-6-(4-(trifluoromethyl)-1H-1,2,3-triazol-1-yl)phenyl)pyrimidin-4-ol (0.07 g, 72.7% yield) as a white solid. MS(ESI) *m/z:* 360.0 $(M+H)^+$. $^1$H NMR (400MHz, $CD_3OD$) $\delta$ 8.84 (s, 1H), 8.03 (br. s., 1H), 7.91 - 7.84 (m, 1H), 7.58 (dd, *J*=8.8, 1.5 Hz, 1H), 6.61 (br. s., 1H).

Intermediate 12

Preparation of 1-(4-chloro-3-fluoro-2-(6-hydroxypyrimidin-4-yl)phenyl)-1H-1,2,3-triazole-4-carbonitrile

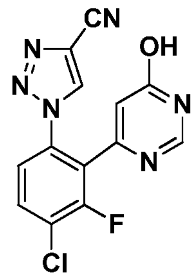

12A. Preparation of 1-(4-chloro-3-fluoro-2-(6-methoxypyrimidin-4-yl)phenyl)-1H-1,2,3-triazole-4-carboxamide

To a cooled (0 °C), clear, yellow solution of 4-chloro-3-fluoro-2-(6-methoxypyrimidin-4-yl)aniline (1 g, 3.94 mmol) in ACN (56.3 ml) was added isoamylnitrite (0.79 ml, 5.91 mmol), followed by the dropwise addition of $TMSN_3$ (0.79 ml, 5.91 mmol). After 10 min, the cold bath was removed, and the reaction was allowed to warm to rt and stirred at rt for 1h. Next, propiolamide (0.817 g, 11.83 mmol) and $Cu_2O$ (0.056 g, 0.394 mmol) were added. After 1 h, the yellow cloudy reaction was diluted with EtOAc, and washed with sat $NH_4Cl$, brine, dried over $MgSO_4$, filtered and concentrated to give a yellow solid. DCM (10 ml) was added and the resulting mixture was sonicated. The suspension was filtered and the solid was air-dried. A yellow solid obtained as 1-(4-chloro-3-fluoro-2-(6-methoxypyrimidin-4-yl)phenyl)-1H-1,2,3-triazole-4-carboxamide (1.003 g, 73.0% yield). MS(ESI) *m/z:* 349.0 $(M+H)^+$.

12B. Preparation of 1-(4-chloro-3-fluoro-2-(6-methoxypyrimidin-4-yl)phenyl)-1H-1,2,3-triazole-4-carbonitrile

To a suspension of 1-(4-chloro-3-fluoro-2-(6-methoxypyrimidin-4-yl)phenyl)-1H-1,2,3-triazole-4-carboxamide (1.003 g, 2.88 mmol) in EtOAc (13 ml) was added TEA (1.20 ml, 8.63 mmol), followed by the dropwise addition of T3P® (50% in EtOAc) (5.14 ml, 8.63 mmol). The reaction was microwaved at 120 °C for 30 min and then it was cooled to rt. The reaction was diluted with EtOAc, washed with sat $NaHCO_3$, brine, dried over $Na_2SO_4$, filtered, and concentrated to afford a brown solid. Purification by normal phase chromatography afforded 1-(4-chloro-3-fluoro-2-(6-methoxypyrimidin-4-yl)phenyl)-1H-1,2,3-triazole-4-carbonitrile (0.815 g, 86% yield) as a yellow solid. MS(ESI) *m/z:* 331.1 $(M+H)^+$. $^1H$ NMR (400MHz, $CDCl_3$) $\delta$ 8.62 (d, *J*=1.1 Hz, 1H), 8.21 (s, 1H), 7.72 (dd, *J*=8.6, 7.5 Hz, 1H), 7.39 (dd, *J*=8.6, 1.8 Hz, 1H), 6.89 (dd, *J*=1.9, 1.2 Hz, 1H), 4.03 (s, 3H).

12C. Preparation of 1-(4-chloro-3-fluoro-2-(6-hydroxypyrimidin-4-yl)phenyl)-1H-1,2,3-triazole-4-carbonitrile

To a suspension of 1-(4-chloro-3-fluoro-2-(6-methoxypyrimidin-4-yl)phenyl)-1H-1,2,3-triazole-4-carbonitrile (0.81 g, 2.449 mmol) in ACN (16.33 ml) was added TMSI (2.00 ml, 14.70 mmol) at rt then the clear solution was heated to 50 °C. After 18 h, the reaction was cooled to rt. The reaction was poured into a 10% $Na_2S_2O_3$ solution and extracted with EtOAc (3x). The combined organic layers were washed with sat $NaHCO_3$, brine, dried over $Na_2SO_4$, filtered, and concentrated to give a residue. The residue was suspended in DCM (20 ml), filtered, and the solid was rinsed with DCM, and air-dried to afford 1-(4-chloro-3-fluoro-2-(6-hydroxypyrimidin-4-yl)phenyl)-1H-1,2,3-triazole-4-carbonitrile (0.73 g, 94% yield) as a white solid. MS(ESI) *m/z:* 317.1 $(M+H)^+$. $^1H$ NMR (400MHz, $CD_3OD$) $\delta$ 8.97 (s, 1H), 8.04 (s, 1H), 7.91 - 7.85 (m, 1H), 7.58 (dd, *J*=8.8, 1.5 Hz, 1H), 6.62 (s, 1H). $^{19}F$ NMR (376MHz, $CD_3OD$) $\delta$ -114.93 (s, IF).

Intermediate 13

Preparation of 6-(5-chloro-2-(4-cyclopropyl-1H-1,2,3-triazol-1-yl)phenyl)pyrimidin-4-ol hydrobromide

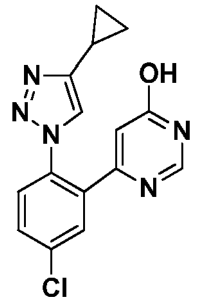

13A. Preparation of 4-(5-chloro-2-(4-cyclopropyl-1H-1,2,3-triazol-1-yl)phenyl)-6-methoxypyrimidine

To a cooled (0 °C), clear, yellow solution of 4-chloro-2-(6-methoxypyrimidin-4-yl)aniline (0.100 g, 0.42 mmol) in ACN (6.06 ml) was added isoamylnitrite (0.086 ml, 0.64 mmol), followed by the dropwise addition of $TMSN_3$ (0.084 ml, 0.64 mmol). After 10 min, the cold bath was removed, and the reaction was allowed to warm to rt and the reaction was stirred at rt for 1 h. Next, ethynylcyclopropane (0.120 g, 1.27 mmol) and $Cu_2O$ (6.07 mg, 0.042 mmol) were added. The flask was equipped with a reflux condenser and the reaction was heated to 50 °C for 1 h, then the reaction was cooled to rt. The reaction was diluted with DCM and washed with sat $NH_4Cl$, brine, dried over $MgSO_4$, filtered and concentrated to give a brown oil. Purification by normal phase chromatography then reverse phase chromatography afforded 4-(5-chloro-2-(4-cyclopropyl-1H-1,2,3-triazol-1-yl)phenyl)-6-methoxypyrimidine (0.024 g, 17.3% yield) as a yellow oil. MS(ESI) *m/z:* 328.1 $(M+H)^+$. $^1H$ NMR (400MHz, $CDCl_3$) $\delta$ 8.75 (d, *J*=0.9 Hz, 1H), 7.79 (d, *J*=2.2 Hz, 1H), 7.61 - 7.56 (m, 1H), 7.51 - 7.47 (m, 1H), 7.29 (s, 1H), 6.35 (d, *J*=0.9 Hz, 1H), 3.96 (s, 3H), 1.96 (tt, *J*=8.4, 5.0 Hz, 1H), 1.02 - 0.95 (m, 2H), 0.88 - 0.81 (m, 2H).

13B. Preparation of 6-(5-chloro-2-(4-cyclopropyl-1H-1,2,3-triazol-1-yl)phenyl) pyrimidin-4-ol hydrobromide.

A clear, yellow solution of 4-(5-chloro-2-(4-cyclopropyl-1H-1,2,3-triazol-1-yl)phenyl)-6-methoxypyrimidine (0.024 g, 0.073 mmol) in HOAc (0.73 ml) and 48% aq HBr (0.41 ml, 3.66 mmol) was warmed to 65 °C for 3 h, and then cooled to rt and concentrated. The yellow gum was suspended in EtOAc and washed with brine, dried over $Na_2SO_4$, filtered, and concentrated. To the residue was added $Et_2O$ (3 ml), sonicated, and filtered. The solid was rinsed with $Et_2O$ (2 ml), air-dried with suction to afford 6-(5-chloro-2-(4-cyclopropyl-1H-1,2,3-triazol-1-yl)phenyl)pyrimidin-4-ol hydrobromide (0.03 g, 100% yield) as a yellow solid. MS(ESI) *m/z:* 314.0 $(M+H)^+$. $^1H$ NMR (400MHz, $CD_3OD$) $\delta$ 8.67 (d, *J*=0.7 Hz, 1H), 8.22 (s, 1H), 7.89 (d, *J*=2.4 Hz, 1H), 7.82 (dd, *J*=8.6, 2.2 Hz, 1H), 7.74 (d, *J*=8.6 Hz, 1H), 6.48 (d, *J*=0.9 Hz, 1H), 2.11 - 2.01 (m, 1H), 1.11 - 1.04 (m, 2H), 0.91 - 0.84 (m, 2H).

Intermediate 14

Preparation of 6-(3-chloro-6-(4-cyclopropyl-1H-1,2,3-triazol-1-yl)-2-fluorophenyl)pyrimidin-4-ol

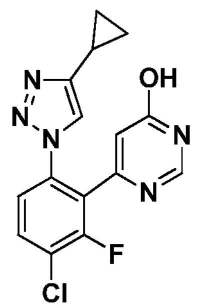

14A. Preparation of 4-(3-chloro-6-(4-cyclopropyl-1H-1,2,3-triazol-1-yl)-2-fluorophenyl)-6-methoxypyrimidine

To a cooled (0 °C), clear, yellow solution of 4-chloro-3-fluoro-2-(6-methoxypyrimidin-4-yl)aniline (0.100 g, 0.39 mmol) in ACN (5.6 ml) was added isoamylnitrite (0.079 ml, 0.59 mmol), followed by the dropwise addition of $TMSN_3$ (0.078 ml, 0.59 mmol). After 10 min, the cold bath was removed, and the reaction was allowed to warm to rt. After 1 h,

ethynylcyclopropane (0.112 g, 1.18 mmol) and $Cu_2O$ (5.64 mg, 0.039 mmol) were added. The flask was equipped with a reflux condenser and the reaction was heated to 50 °C for 1 h, then the reaction was cooled to rt. The reaction was diluted with DCM and washed with sat $NH_4Cl$, brine, dried over $MgSO_4$, filtered and concentrated to give a brown oil. Purification by normal phase chromatography afforded 4-(3-chloro-6-(4-cyclopropyl-1H-1,2,3-triazol-1-yl)-2-fluorophenyl)-6-methoxypyrimidine (0.05 g, 36.7% yield) as a yellow oil. MS(ESI) *m/z:* 346.0 $(M+H)^+$. $^1H$ NMR (400MHz, $CDCl_3$) $\delta$ 8.69 (d, *J*=0.9 Hz, 1H), 7.63 (dd, *J*=8.6, 7.5 Hz, 1H), 7.35 (dd, *J*=8.6, 1.5 Hz, 1H), 7.30 (s, 1H), 6.76 (t, *J*=1.2 Hz, 1H), 4.00 (s, 3H), 1.90 (tt, *J*=8.4, 5.0 Hz, 1H), 0.98 - 0.91 (m, 2H), 0.82 - 0.76 (m, 2H).

14B. Preparation of 6-(3-chloro-6-(4-cyclopropyl-1H-1,2,3-triazol-1-yl)-2-fluorophenyl) pyrimidin-4-ol

A clear, yellow solution of 4-(3-chloro-6-(4-cyclopropyl-1H-1,2,3-triazol-1-yl)-2-fluorophenyl)-6-methoxypyrimidine (0.05 g, 0.145 mmol) in HOAc (1.45 ml) and 48% aq HBr (0.82 ml, 7.23 mmol) was warmed to 65 °C for 3 h, and then the reaction was cooled to rt and concentrated. Purification by reverse phase chromatography afforded 6-(3-chloro-6-(4-cyclopropyl-1H-1,2,3-triazol-1-yl)-2-fluorophenyl)pyrimidin-4-ol (0.04 g, 83% yield) as a yellow solid. MS(ESI) *m/z:* 332.0 $(M+H)^+$. $^1H$ NMR (400MHz, $CD_3OD$) $\delta$ 8.09 (d, *J*=0.9 Hz, 1H), 7.91 (s, 1H), 7.82 (dd, *J*=8.6, 7.7 Hz, 1H), 7.49 (dd, *J*=8.8, 1.5 Hz, 1H), 6.50 - 6.47 (m, 1H), 1.97 (tt, *J*=8.5, 5.1 Hz, 1H), 1.01 - 0.95 (m, 2H), 0.81 - 0.75 (m, 2H). $^{19}F$ NMR (376MHz, $CD_3OD$) $\delta$ -115.39 (s).

Intermediate 15

Preparation of 6-{5-chloro-2-[4-(trifluoromethyl)-1H-1,2,3-triazol-1-yl]phenyl}pyrimidin-4-ol

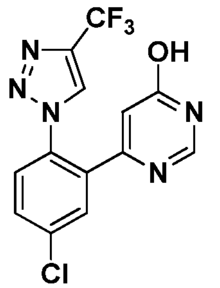

15A. Preparation of 4-{5-chloro-2-[4-(trifluoromethyl)-1H-1,2,3-triazol-1-yl]phenyl}-6-methoxypyrimidine

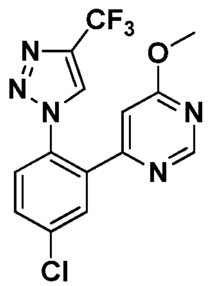

To a solution of 4-chloro-2-(6-methoxypyrimidin-4-yl)aniline (1.0 g, 4.24 mmol), prepared as described in Intermediate 9B, in ACN (60.6 ml) at 0 °C was added 3-methylbutyl nitrite (0.86 ml, 6.36 mmol) followed by the dropwise addition of $TMSN_3$ (0.84 ml, 6.36 mmol). Gas evolution was observed. After 10 min, the ice bath was removed, and the reaction was allowed to warm to rt. After 2 h, $Cu_2O$ (61 mg, 0.42 mmol) was added followed by a slow bubbling of 3,3,3-trifluoroprop-1-yne gas over a period of 5 min. After an additional 10 min, the reaction was partitioned between DCM and sat $NH_4Cl$ and then the layers were separated. The organic layer was washed with brine, dried over $MgSO_4$, filtered and concentrated. Purification by normal phase chromatography gave 4-{5-chloro-2-[4-(trifluoromethyl)-1H-1,2,3-triazol-1-yl]phenyl}-6-methoxypyrimidine (1.46 g, 97% yield) as a yellow solid. MS(ESI) *m/z:* 356.1 $(M+H)^+$. $^1H$ NMR (400MHz,

$CDCl_3$) δ 8.62 (d, *J*=1.1 Hz, 1H), 8.00 (d, *J*=0.7 Hz, 1H), 7.75 (d, *J*=2.4 Hz, 1H), 7.66 - 7.60 (m, 1H), 7.52 (d, *J*=8.6 Hz, 1H), 6.60 (d, *J*=1.1 Hz, 1H), 3.98 (s, 3H). $^{19}F$ NMR (376MHz, $CDCl_3$) δ -61.10 (s).

15B. Preparation of 6-{5-chloro-2-[4-(trifluoromethyl)-1H-1,2,3-triazol-1-yl]phenyl} pyrimidin-4-ol

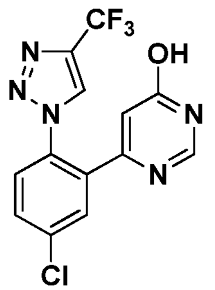

To a solution of 4-{5-chloro-2-[4-(trifluoromethyl)-1H-1,2,3-triazol-1-yl]phenyl}-6-methoxypyrimidine (1.46 g, 4.10 mmol) in AcOH (10ml) was added 48% aq HBr (5 ml, 44.2 mmol). The mixture was stirred at 85 °C for 1 h. The reaction was concentrated to dryness and then partitioned between EtOAc and sat $NaHCO_3$. The layers were separated and the aqueous layer was extracted with EtOAc (2x). The organic layers were combined and washed with sat $NaHCO_3$, brine, dried over $MgSO_4$, filtered and the solvent was reduced under vacuum until some solid started to form. The resulting suspension was triturated with $Et_2O$. The solid was filtered and washed with $Et_2O$ to give 6-{5-chloro-2-[4-(trifluoromethyl)-1H-1,2,3-triazol-1-yl]phenyl}pyrimidin-4-ol (1 g, 71.3% yield) as a pale yellow solid. MS(ESI) *m/z:* 342.0 $(M+H)^+$. $^1H$ NMR (400MHz, $CD_3OD$) δ 8.83 (d, *J*=0.7 Hz, 1H), 7.99 (d, *J*=0.9 Hz, 1H), 7.87 (d, *J*=2.2 Hz, 1H), 7.79 - 7.72 (m, 1H), 7.70 - 7.62 (m, 1H), 6.45 (d, *J*=0.9 Hz, 1H). $^{19}F$ NMR (376MHz, $CD_3OD$) δ -62.61 (s).

Intermediate 16

Preparation of 6-{5-chloro-2-[4-(difluoromethyl)-1H-1,2,3-triazol-1-yl]phenyl}pyrimidin-4-ol

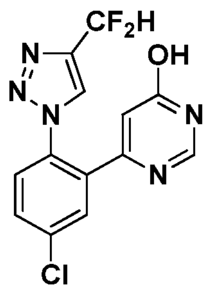

16A. Preparation of {1-[4-chloro-2-(6-methoxypyrimidin-4-yl)phenyl]-1H-1,2,3-triazol-4-yl}methanol

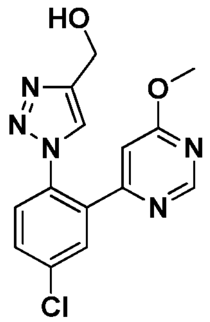

{1-[4-Chloro-2-(6-methoxypyrimidin-4-yl)phenyl]-1H-1,2,3-triazol-4-yl}methanol (0.44 g, 52.5% yield) was prepared in a similar manner as the procedure described for the preparation of 4-{5-chloro-2-[4-(trimethylsilyl)-1H-1,2,3-triazol-1-yl]phenyl}-6-methoxypyrimidine, as described in Intermediate 9C, by replacing ethynyltrimethylsilane with propargyl alcohol (0.38 ml, 6.36 mmol). MS(ESI) *m/z:* 318.3 $(M+H)^+$. $^1H$ NMR (400MHz, $CDCl_3$) δ 8.66 (d, *J*=1.1 Hz, 1H), 7.77 (d, *J*=2.2 Hz, 1H), 7.63 (s, 1H), 7.61 - 7.55 (m, 1H), 7.51 - 7.46 (m, 1H), 6.42 (d, *J*=1.1 Hz, 1H), 4.77 (d, *J*=5.9 Hz, 2H), 3.93 (s, 3H).

16B. Preparation of 1-[4-chloro-2-(6-methoxypyrimidin-4-yl)phenyl]-1H-1,2,3-triazole-4-carbaldehyde

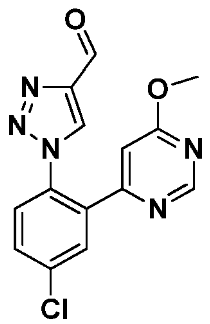

To a solution of {1-[4-chloro-2-(6-methoxypyrimidin-4-yl)phenyl]-1H-1,2,3-triazol-4-yl}methanol (95 mg, 0.3 mmol) in DMSO (1 mL) was added IBX (92 mg, 0.33 mmol) and the reaction was stirred at rt for 14 h. Water and sat $NaHCO_3$ were added and the mixture was extracted with EtOAc (2x). The organic layers were combined, concentrated and purified by normal phase chromatography to give 1-[4-chloro-2-(6-methoxy pyrimidin-4-yl)phenyl]-1H-1,2,3-triazole-4-carbaldehyde (82 mg, 87% yield) as a white solid. MS(ESI) *m/z:* 316.3 $(M+H)^+$. $^1H$ NMR (400MHz, $CDCl_3$) δ 10.16 (s, 1H), 8.62 (d, *J*=1.1 Hz, 1H), 8.21 (s, 1H), 7.76 (d, *J*=2.2 Hz, 1H), 7.64 (dd, *J*=8.5, 2.3 Hz, 1H), 7.53 (d, *J*=8.4 Hz, 1H), 6.59 (d, *J*=1.1 Hz, 1H), 3.97 (s, 3H).

16C. Preparation of 4-{5-chloro-2-[4-(difluoromethyl)-1H-1,2,3-triazol-1-yl]phenyl}-6-methoxypyrimidine

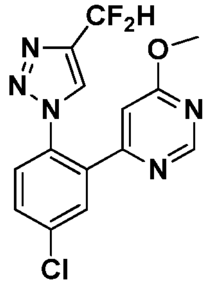

To a solution of 1-[4-chloro-2-(6-methoxypyrimidin-4-yl)phenyl]-1H-1,2,3-triazole-4-carbaldehyde (427 mg, 1.35 mmol) in DCM (30 ml) was added DAST (0.54 ml, 4.1 mmol) and the reaction was stirred overnight at rt. The reaction was quenched with water and extracted with DCM. The organic layer was concentrated and purified by normal phase chromatography to give 4-{5-chloro-2-[4-(difluoromethyl)-1H-1,2,3-triazol-1-yl]phenyl}-6-methoxypyrimidine (441 mg, 97% yield) as a yellow solid. MS(ESI) *m/z:* 338.3 $(M+H)^+$. $^1H$ NMR (400MHz, $CDCl_3$) δ 8.65 (d, *J*=0.9 Hz, 1H), 7.89 (s, 1H), 7.76 (d, *J*=2.4 Hz, 1H), 7.62 (dd, *J*=8.5, 2.3 Hz, 1H), 7.55 - 7.47 (m, 1H), 6.89 (t, J=54.6 Hz, 1H), 6.52 (d, *J*=1.1 Hz, 1H), 4.03 - 3.87 (m, 3H). $^{19}F$ NMR (376MHz, $CDCl_3$) δ -112.40 (s).

16D. Preparation of 6-{5-chloro-2-[4-(difluoromethyl)-1H-1,2,3-triazol-1-yl]phenyl} pyrimidin-4-ol

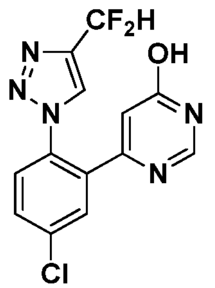

6-{5-Chloro-2-[4-(difluoromethyl)-1H-1,2,3-triazol-1-yl]phenyl}pyrimidin-4-ol (370 mg, 88% yield) was prepared in a similar manner as the procedure described for the preparation of 6-[5-chloro-2-(4-chloro-1H-1,2,3-triazol-1-yl)phenyl]pyrimidin-4-ol, as described in Intermediate 9E, by replacing 4-[5-chloro-2-(4-chloro-1H-1,2,3-triazol-1-yl)phenyl]-6-methoxypyrimidine with 4-{5-chloro-2-[4-(difluoromethyl)-1H-1,2,3-triazol-1-yl]phenyl}-6-methoxypyrimidine (441 mg, 1.31 mmol). MS(ESI) *m/z:* 324.3 $(M+H)^+$. $^1H$ NMR (400MHz, $CDCl_3$) δ 8.04 (s, 1H), 7.86 (s, 1H), 7.71 (d, *J*=2.2 Hz, 1H), 7.67 - 7.61 (m, 1H), 7.51 (d, *J*=8.6 Hz, 1H), 6.92 (t, *J*=54.6 Hz, 1H), 6.43 (d, *J*=0.7 Hz, 1H). $^{19}F$ NMR (376MHz, $CDCl_3$) δ -112.69 (s).

Intermediate 17

Preparation of 6-[3-chloro-2-fluoro-6-(1H-1,2,3,4-tetrazol-1-yl)phenyl]pyrimidin-4-ol

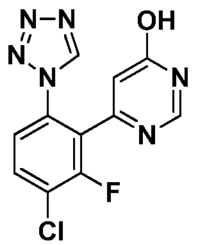

17A. Preparation of 4-[3-chloro-2-fluoro-6-(1H-1,2,3,4-tetrazol-1-yl)phenyl]-6-methoxypyrimidine

4-Chloro-3-fluoro-2-(6-methoxypyrimidin-4-yl)aniline (300 mg, 1.183 mmol) dissolved in AcOH (3 mL) was added trimethoxymethane (377 mg, 3.55 mmol), stirred at rt. After 30 min, $NaN_3$ (231 mg, 3.55 mmol) was added and stirred at rt for 16 h. To the reaction mixture was added water and a precipitate formed. The mixture was filtered to collect the solid residue, and filtrate was extracted with EtOAc, and the organic later was washed with brine, dried over $MgSO_4$, filtered and concentrated to give a crude solid, which was then combined with original solid residue collected. The crude material was purified by normal phase chromatography to afford 4-(3-chloro-2-fluoro-6-(1H-tetrazol-1-yl)phenyl)-6-methoxypyrimidine (367 mg, 100% yield). MS(ESI) *m/z:* 307.08 $(M+H)^+$. $^1H$ NMR (400MHz, $CDCl_3$) δ 8.78 (s, 1H), 8.59 (d, *J*=1.1 Hz, 1H), 7.71 (dd, *J*=8.7, 7.4 Hz, 1H), 7.38 (dd, *J*=8.6, 1.8 Hz, 1H), 6.86 (dd, *J*=1.9, 1.2 Hz, 1H), 3.98 (s, 3H).

17B. Preparation of 6-[3-chloro-2-fluoro-6-(1H-1,2,3,4-tetrazol-1-yl)phenyl]pyrimidin-4-ol

To a solution of 4-(3-chloro-2-fluoro-6-(1H-tetrazol-1-yl)phenyl)-6-methoxypyrimidine (50 mg, 0.163 mmol), NaI (244 mg, 1.630 mmol) dissolved in ACN (1.6 ml) was added TMSCl (0.2 ml, 1.630 mmol). The resulting reaction mixture was stirred at rt for 23 h. To the reaction mixture was added CELITE®, the slurry was filtered and the collected organics were concentrated to yield a crude solid. Purification by normal phase chromatography, followed by trituration with $Et_2O$, afforded 6-[3-chloro-2-fluoro-6-(1H-1,2,3,4-tetrazol-1-yl)phenyl]pyrimidin-4-ol (46 mg, 96% yield) as a white solid. MS(ESI) *m/z:* 293.08 $(M+H)^+$. $^1H$ NMR (400MHz, $CD_3OD$) δ 9.75 (s, 1H), 8.40 (s, 1H), 8.28 (dd, *J*=8.7, 7.6 Hz, 1H), 7.97 (dd, *J*=8.7, 1.7 Hz, 1H), 7.02 (s, 1H).

Intermediate 18

Preparation of 1-[4-chloro-2-(6-hydroxypyrimidin-4-yl)phenyl]-1H-1,2,3-triazole-4-carbonitrile

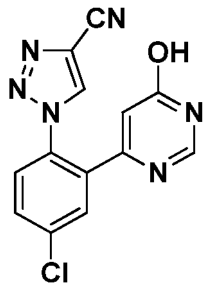

18A. Preparation of 1-[4-chloro-2-(6-methoxypyrimidin-4-yl)phenyl]-1H-1,2,3-triazole-4-carboxamide

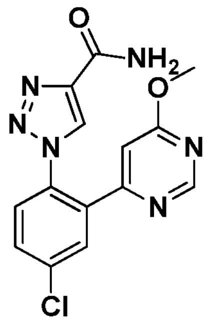

1-[4-Chloro-2-(6-methoxypyrimidin-4-yl)phenyl]-1H-1,2,3-triazole-4-carboxamide (300 mg, 80% yield) was prepared in a similar manner as the procedure described for the preparation of 4-{5-chloro-2-[4-(trimethylsilyl)-1H-1,2,3-triazol-1-yl] phenyl }-6-methoxypyrimidine, as described in Intermediate 9C, by replacing ethynyltrimethylsilane with prop-2-ynamide (176 mg, 2.55 mmol). MS(ESI) *m/z:* 331.4 $(M+H)^+$. $^1H$ NMR (400MHz, $CDCl_3$) δ 8.66 (d, *J*=0.7 Hz, 1H), 8.16 (s, 1H), 7.76 (d, *J*=2.4 Hz, 1H), 7.62 (dd, *J*=8.5, 2.3 Hz, 1H), 7.51 (d, *J*=8.6 Hz, 1H), 7.05 (br. s., 1H), 6.53 (d, *J*=0.9 Hz, 1H), 5.66 (br. s., 1H), 3.97 (s, 3H).

18B. Preparation of 1-[4-chloro-2-(6-methoxypyrimidin-4-yl)phenyl]-1H-1,2,3-triazole-4-carbonitrile

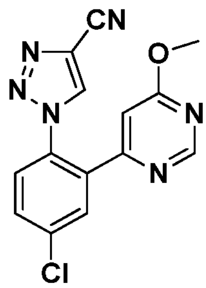

To a suspension of 1-[4-chloro-2-(6-methoxypyrimidin-4-yl)phenyl]-1H-1,2,3-triazole-4-carboxamide (91 mg, 0.28 mmol) and TEA (115 μl, 0.83 mmol) in EtOAc (6.88 ml) was added T3P® (50% in EtOAc) (0.49 ml, 0.83 mmol) dropwise. The reaction was microwaved at 120 °C for 1 h. Additional TEA (115 μl, 0.83 mmol) and T3P® (50% in EtOAc) (0.49 ml, 0.83 mmol) were added and the reaction was microwaved at 120 °C for an additional 30 min. The reaction was diluted with EtOAc and washed with water, sat $NaHCO_3$, brine, dried over $MgSO_4$, filtered, and concentrated. Purification by normal phase chromatography gave 1-[4-chloro-2-(6-methoxypyrimidin-4-yl)phenyl]-1H-1,2,3-triazole-4-carbonitrile (91 mg, 100% yield) as a white solid. MS(ESI) *m/z:* 313.3 $(M+H)^+$. $^1H$ NMR (400MHz, $CDCl_3$) δ 8.62 (d, *J*=0.9 Hz, 1H), 8.17 (s, 1H), 7.73 (d, *J*=2.4 Hz, 1H), 7.65 (dd, *J*=8.5, 2.3 Hz, 1H), 7.51 (d, *J*=8.6 Hz, 1H), 6.65 (d, *J*=1.1 Hz, 1H), 4.00 (s, 3H).

18C. Preparation of 1-[4-chloro-2-(6-hydroxypyrimidin-4-yl)phenyl]-1H-1,2,3-triazole-4-carbonitrile

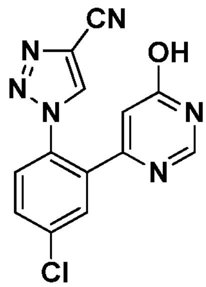

To a suspension of 1-[4-chloro-2-(6-methoxypyrimidin-4-yl)phenyl]-1H-1,2,3-triazole-4-carbonitrile (91 mg, 0.29 mmol) in ACN (3 mL) was added TMSI (0.2 mL, 1.47 mmol) at rt and the solution was heated at 50 °C for 15 h. The reaction was poured into 10% $Na_2S_2O_3$ and sat $NaHCO_3$ then extracted with EtOAc (3x). The combined organic layers were washed with brine. On standing, a solid precipitated out from the organic layer. The solid was filtered and rinsed with EtOAc and air-dried to give 1-[4-chloro-2-(6-hydroxypyrimidin-4-yl)phenyl]-1H-1,2,3-triazole-4-carbonitrile (60 mg, 69.0% yield) as a white solid. MS(ESI) *m/z:* 299.3 $(M+H)^+$. $^1H$ NMR (400MHz, $CDCl_3$) δ 8.22 (s, 1H), 7.91 (s, 1H), 7.72 (d, *J*=2.2 Hz, 1H), 7.66 (dd, *J*=8.5, 2.3 Hz, 1H), 7.49 (d, *J*=8.4 Hz, 1H), 6.55 (s, 1H).

Intermediate 19

Preparation of (9*R*,13*S*)-13-amino-9-methyl-3-{[2-(trimethylsilyl)ethoxy]methyl}-3,4,7,15-tetraazatricyclo[12.3.1.$0^{2,6}$]octadeca-1(18),2(6),4,14,16-pentaen-8-one

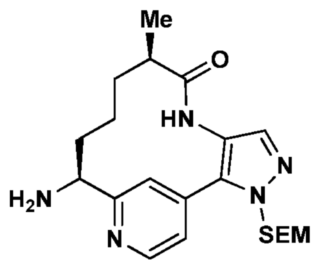

19A. Preparation of 4-nitro-1-((2-(trimethylsilyl)ethoxy)methyl)-1*H*-pyrazole

To a solution of 4-nitro-1*H*-pyrazole (5.0 g, 44.2 mmol) in THF (100 mL) at 0 °C was added N-cyclohexyl-N-methylcyclohexanamine (0.948 mL, 4.43 mmol) followed by dropwise addition of SEM-C1 (12.55 mL, 70.7 mmol). The reaction mixture was then allowed to gradually warm to rt and stirred overnight. The reaction mixture was concentrated and purified by normal phase chromatography to yield 4-nitro-1-((2-(trimethylsilyl)ethoxy)methyl)-1*H*-pyrazole as clear oil (2.4 g, 21% yield). $^1H$ NMR (500MHz, $CDCl_3$) δ 8.31 (s, 1H), 8.10 (s, 1H), 5.46 (s, 2H), 3.67 - 3.55 (m, 2H), 0.99 - 0.90 (m, 2H), 0.05 - 0.03 (m, 9H).

19B. Preparation of (*S*)-benzyl (1-(4-(4-nitro-1-((2-(trimethylsilyl)ethoxy)methyl)-1*H*-pyrazol-5-yl)pyridin-2-yl)but-3-en-1-yl)carbamate

To a $N_2$ flushed pressure vial was added (*S*)-benzyl (1-(4-chloropyridin-2-yl)but-3-en-1-yl)carbamate, prepared as described in Intermediate 23, (1.9 g, 6.00 mmol), 4-nitro-1-((2-(trimethylsilyl)ethoxy)methyl)-1*H*-pyrazole, prepared as described in Intermediate 41A, (1.6 g, 6.60 mmol), di(adamant-1-yl)(butyl)phosphine (0.323 g, 0.90 mmol), PvOH (0.209 mL, 1.80 mmol) and $K_2CO_3$ (2.48 g, 17.9 mmol). To the above mixture was then added N,N-dimethylacetamide (45 mL) and the vial was purged with $N_2$ for 5 min. To this mixture was then added $Pd(OAc)_2$ (0.135 g, 0.600 mmol). The reaction mixture was again purged with $N_2$. The vial was sealed and heated in microwave at 120 °C for 1 h. The reaction mixture was cooled to rt and partitioned between 10% aqueous LiCl (15 mL) and EtOAc (30 mL). The aqueous layer was extracted with EtOAc (2 x 20 mL) and the combined organic layers were washed with brine (15 mL) and dried over $MgSO_4$. The crude product was then purified using normal phase chromatography to yield (*S*)-benzyl (1-(4-(4-nitro-1-((2-(trimethylsilyl)ethoxy)methyl)-1*H*-pyrazol-5-yl)pyridin-2-yl)but-3-en-1-yl)carbamate (1.92 g, 58% yield) as a brown oil. MS(ESI) *m/z:* 524.2 $(M+H)^+$.

19C. Preparation of (*S*)-benzyl(1-(4-(4-amino-1-((2-(trimethylsilyl)ethoxy)methyl)-1*H*-pyrazol-5-yl)pyridin-2-yl)but-3-en-1-yl)carbamate

A solution of (*S*)-benzyl (1-(4-(4-nitro-1-((2-(trimethylsilyl)ethoxy)methyl)-1*H*-pyrazol-5-yl)pyridin-2-yl)but-3-en-1-yl)carbamate (1.92 g, 3.68 mmol), prepared as described in Intermediate 41B, in MeOH (20 mL) and AcOH (2 mL) was heated at 40 °C. To the above clear solution was then slowly added Zn (0.481 g, 7.35 mmol, in 3 portions (50:25:25%)) and allowed to stir at the same temperature for 5 min. The reaction mixture was monitored by LCMS and once complete, to the cooled reaction mixture was added 2.0 g of $K_2CO_3$ (1 g for 1 mL AcOH) and 2 mL water. The reaction mixture was stirred for 5 min then filtered over a pad of CELITE® and concentrated to yield the crude product. The crude product was then partitioned between EtOAc (30 mL) and sat $NaHCO_3$ (15 mL) solution. The organic layers are separated and dried over $MgSO_4$, filtered and concentrated. The crude product was then purified using normal phase chromatography to yield (*S*)-benzyl (1-(4-(4-amino-1-((2-(trimethylsilyl)ethoxy)methyl)-1*H*-pyrazol-5-yl)pyridin-2-yl)but-3-en-1-yl)carbamate (1.15 g, 63% yield) as pale yellow oil. MS(ESI) *m/z*: 494.4 $(M+H)^+$.

19D. Preparation of benzyl ((*S*)-1-(4-(4-((*R*)-2-methylbut-3-enamido)-1-((2-(trimethylsilyl)ethoxy)methyl)-1*H*-pyrazol-5-yl)pyridin-2-yl)but-3-en-1-yl)carbamate

To a $N_2$ flushed, 3-necked, 250 mL RBF was added a solution (*S*)-benzyl (1-(4-(4-amino-1-((2-(trimethylsilyl)ethoxy)methyl)-1*H*-pyrazol-5-yl)pyridin-2-yl)but-3-en-1-yl)carbamate (1.15 g, 2.33 mmol), prepared as described in Example 41C, and EtOAc (15 mL). The solution was cooled to -10 °C and (*R*)-2-methylbut-3-enoic acid, as prepared in Intermediate 2, (350 mg, 3.49 mmol), pyridine (0.564 mL, 6.99 mmol) and T3P® (2.77 mL, 4.66 mmol) were added. The cooling bath was removed and the solution was allowed to warm to rt and then stir over a period of 20 h. Water (20 mL) and EtOAc (20 mL) were added and the mixture was stirred for 30 min. The organic phase was separated and the aqueous layer was extracted with EtOAc (20 mL). The combined organic extracts were washed with brine (15 mL), dried

over $Na_2SO_4$, filtered and concentrated *in vacuo.* Purification by normal phase chromatography eluting with a gradient of hexanes/EtOAc gave benzyl ((*S*)-1-(4-(4-((*R*)-2-methylbut-3-enamido)-1 -((2-(trimethylsilyl)ethoxy) methyl)-1*H*-pyrazol-5-yl)pyridin-2-yl)but-3-en-1-yl)carbamate (1.12 g, 79% yield). MS(ESI) *m*/*z*: 576.4 $[M+H]^+$.

19E. Preparation of benzyl N-[(9*R*,10*E*,13*S*)-9-methyl-8-oxo-3-{[2-(trimethylsilyl) ethoxy]methyl}-3,4,7,15-tetraazatricyclo[12.3 .1.$0^{2,6}$]octadeca-1(18),2(6),4,10,14,16-hexaen-13-yl]carbamate

To a $N_2$ flushed, 250 mL, 3-necked, RBF was added a solution of benzyl ((*S*)-1-(4-(4-((*R*)-2-methylbut-3-enamido)-1-((2-(trimethylsilyl)ethoxy)methyl)-1*H*-pyrazol-5-yl)pyridin-2-yl)but-3-en-1-yl)carbamate (1.12 g, 1.945 mmol), prepared as described in Intermediate 41D, in DCE (18 mL). The solution was sparged with Ar for 15 min. Second Generation Grubbs Catalyst (662 mg, 0.778 mmol) was added in one portion. The reaction mixture was heated at 120 °C in microwave for 30 min. After cooling to rt, the solvent was removed and the residue was purified by normal phase chromatography eluting with a gradient of DCM/MeOH to yield benzyl N-[(9*R*,10*E*,13*S*)-9-methyl-8-oxo-3-{[2-(trimethylsilyl)ethoxy]methyl}-3,4,7,15-tetraazatricyclo[12.3.1.$0^{2,6}$]octadeca-1(18),2(6),4,10,14,16-hexaen-13-yl]carbamate (477 mg, 42% yield) as a tan solid. MS(ESI) *m*/*z*: 548.3 $[M+H]^+$.

19F. Preparation of (9*R*,13*S*)-13-amino-9-methyl-3-{[2-(trimethylsilyl)ethoxy]methyl}-3,4,7,15-tetraazatricyclo[12.3.1.$0^{2,6}$]octadeca-1(18),2(6),4,14,16-pentaen-8-one

Pd/C (0.93 g, 0.871 mmol) was added to a 250 mL Parr hydrogenation flask containing a solution of benzyl N-[(9*R*,10*E*,13*S*)-9-methyl-8-oxo-3-{[2-(trimethylsilyl) ethoxy]methyl}-3,4,7,15-tetraazatricyclo[12.3.1.$0^{2,6}$]octadeca-1(18),2(6),4,10,14,16-hexaen-13-yl]carbamate (477 mg, 0.871 mmol), prepared as described in Intermediate 41E, in EtOH (20 mL). The flask was purged with $N_2$ and pressurized to 55 psi of $H_2$ and allowed to stir for 4 h. The reaction was filtered through a pad of CELITE® and concentrated to yield (9R,13S)-13-amino-9-methyl-3-{[2-(trimethylsilyl)ethoxy]methyl}-3,4,7,15-tetraazatricyclo[12.3.1.$0^{2,6}$]octadeca-1(18),2(6),4,14,16-pentaen-8-one (245 mg, 64% yield) as a tan solid. MS(ESI) *m*/*z*: 416.4 $[M+H]^+$.

Intermediate 20

Preparation of 6-[5-chloro-2-(1H-1,2,3,4-tetrazol-1-yl)phenyl]pyrimidin-4-ol

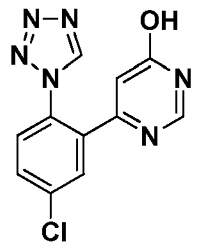

20A. Preparation of 4-[5-chloro-2-(1H-1,2,3,4-tetrazol-1-yl)phenyl]-6-methoxypyrimidine

To a solution of 4-chloro-2-(6-methoxypyrimidin-4-yl)aniline (0.507 g, 2.151 mmol) dissolved in AcOH (5.4 ml) was added trimethoxymethane (0.685 g, 6.45 mmol) and the resulting solution was stirred at rt for 30 min. After that time $NaN_3$ (0.420 g, 6.45 mmol) was added and the reaction mixture was stirred at rt for 16 h. Water was added to form a precipitate. The precipitate was collected by filtration, and filtrate was extracted with EtOAc, which was then washed with brine, dried over $MgSO_4$, filtered and concentrated to give a crude solid. The combined solid residue was purified by normal phase chromatography to afford 4-(5-chloro-2-(1H-tetrazol-1-yl)phenyl)-6-methoxypyrimidine (0.59 g, 95% yield) as an off-white solid. MS(ESI) *m/z:* 289.08 $(M+H)^+$. $^1H$ NMR (400MHz, $CDCl_3$) $\delta$ 8.76 (s, 1H), 8.62 (d, *J*=0.9 Hz, 1H), 7.74 (d, *J*=2.2 Hz, 1H), 7.66 (dd, *J*=8.5, 2.3 Hz, 1H), 7.52 (d, *J*=8.4 Hz, 1H), 6.65 (d, *J*=1.1 Hz, 1H), 3.99 (s, 3H).

20B. Preparation of 6-[5-chloro-2-(1H-1,2,3,4-tetrazol-1-yl)phenyl]pyrimidin-4-ol

To a solution of 4-(5-chloro-2-(1H-tetrazol-1-yl)phenyl)-6-methoxypyrimidine (0.59 g, 2.044 mmol), NaI (3.06 g, 20.44 mmol) in ACN (20.44 ml) was added TMSCl (2.6 ml, 20.44 mmol), and the reaction was stirred at rt for 16 h. CELITE® was added to the reaction mixture, the slurry was filtered, and concentrated to give a crude solid mixture. The

solid was purified by normal phase chromatography, then recrystallized from EtOAc to give 6-[5-chloro-2-(1H-1,2,3,4-tetrazol-1-yl)phenyl]pyrimidin-4-ol (370 mg, 66% yield) as a white solid. MS(ESI) *m/z*: 275.08 (M+H)$^+$. $^1$H NMR (400MHz, DMSO-$d_6$) δ 12.62 (br. s., 1H), 9.72 (s, 1H), 7.97 (d, *J*=0.7 Hz, 1H), 7.92 (d, *J*=2.2 Hz, 1H), 7.87 - 7.83 (m, 1H), 7.82 - 7.78 (m, 1H), 6.48 (d, *J*=0.7 Hz, 1H).

Intermediate 21

Preparation of 6-(3-chloro-6-(4-(difluoromethyl)-1H-1,2,3-triazol-1-yl)-2-fluorophenyl)pyrimidin-4-ol

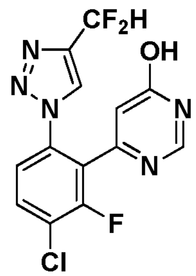

21A. Preparation of (1-(4-chloro-3-fluoro-2-(6-methoxypyrimidin-4-yl)phenyl)-1H-1,2,3-triazol-4-yl)methanol

To a cooled (0 °C), clear, yellow solution of 4-chloro-3-fluoro-2-(6-methoxypyrimidin-4-yl)aniline (1.058 g, 4.17 mmol) in ACN (59.6 ml) was added isoamylnitrite (0.84 ml, 6.26 mmol), followed by the dropwise addition of $TMSN_3$ (0.82 ml, 6.26 mmol). After 10 min, the cold bath was removed, and the reaction was allowed to warm to rt. Propargyl alcohol (0.75 ml, 12.51 mmol) and $Cu_2O$ (0.060 g, 0.42 mmol) were added. After 1 h, the reaction was diluted with EtOAc and washed with sat $NH_4Cl$, brine, dried over $MgSO_4$, filtered and concentrated to give a brown oil. The crude product was purified by normal phase chromatography to give (1-(4-chloro-3-fluoro-2-(6-methoxypyrimidin-4-yl)phenyl)-1H-1,2,3-triazol-4-yl)methanol (0.8 g, 57.1% yield) as a yellow foam. MS(ESI) *m/z*: 336.1 (M+H)$^+$. $^1$H NMR (400MHz, $CDCl_3$) δ 8.65 (d, *J*=1.1 Hz, 1H), 7.69 - 7.62 (m, 2H), 7.37 (dd, *J*=8.6, 1.5 Hz, 1H), 6.81 (t, *J*=1.2 Hz, 1H), 4.76 (d, *J*=5.9 Hz, 2H), 4.00 (s, 3H), 2.18 (t, *J*=6.1 Hz, 1H).

21B. Preparation of 1-(4-chloro-3-fluoro-2-(6-methoxypyrimidin-4-yl)phenyl)-1H-1,2,3-triazole-4-carbaldehyde

To the solution of (1-(4-chloro-3-fluoro-2-(6-methoxypyrimidin-4-yl)phenyl)-1H-1,2,3-triazol-4-yl)methanol (0.8 g, 2.38 mmol) in DMSO (9.53 ml) was added IBX (0.734 g, 2.62 mmol), and the reaction was stirred at rt. After 18 h, water and sat $NaHCO_3$ were added and the reaction mixture was extracted with EtOAc (2x). The organic layers were combined and dried over $Na_2SO_4$, filtered, and concentrated. Purification by normal phase chromatography afforded 1-(4-chloro-3-fluoro-2-(6-methoxypyrimidin-4-yl)phenyl)-1H-1,2,3-triazole-4-carbaldehyde (0.64 g, 80% yield) as a white solid. MS(ESI) *m/z*: 334.4 (M+H)$^+$. $^1$H NMR (400MHz, $CDCl_3$) δ 10.12 (s, 1H), 8.60 (d, *J*=1.1 Hz, 1H), 8.25 (s, 1H), 7.71 (dd, *J*=8.6, 7.5 Hz, 1H), 7.39 (dd, *J*=8.6, 1.8 Hz, 1H), 6.88 (dd, *J*=1.8, 1.1 Hz, 1H), 4.01 (s, 3H).

21C. Preparation of 4-(3-chloro-6-(4-(difluoromethyl)-1H-1,2,3-triazol-1-yl)-2-fluorophenyl)-6-methoxypyrimidine

To the solution of 1-(4-chloro-3-fluoro-2-(6-methoxypyrimidin-4-yl)phenyl)-1H-1,2,3-triazole-4-carbaldehyde (0.3 g, 0.9 mmol) in DCM (24 ml) was added DAST (0.54 ml, 4.09 mmol). The reaction was stirred at rt for 22 h. To the reaction was added water and the resulting mixture was extracted with DCM. The organic layer was washed with brine, dried over $Na_2SO_4$, filtered, and concentrated. Purification by normal phase chromatography afforded 4-(3-chloro-6-(4-(difluoromethyl)-1H-1,2,3-triazol-1-yl)-2-fluorophenyl)-6-methoxypyrimidine (0.256 g, 80% yield) as a white solid. MS(ESI) *m/z:* 356.1 (M+H)$^+$. $^1$H NMR (400MHz, $CDCl_3$) δ 8.62 (d, *J*=0.9 Hz, 1H), 7.94 (t, *J*=1.3 Hz, 1H), 7.69 (dd, *J*=8.6, 7.5 Hz, 1H), 7.39 (dd, *J*=8.6, 1.8 Hz, 1H), 7.00 - 6.69 (m, 2H), 4.00 (s, 3H).

21D. Preparation of 6-(3-chloro-6-(4-(difluoromethyl)-1H-1,2,3-triazol-1-yl)-2-fluorophenyl)pyrimidin-4-ol

A clear, yellow solution of 4-(3-chloro-6-(4-(difluoromethyl)-1H-1,2,3-triazol-1-yl)-2-fluorophenyl)-6-methoxypyrimidine (0.256 g, 0.72 mmol) in HOAc (3.6 ml) and 48% aq HBr (4.07 ml, 36.0 mmol) was warmed to 65 °C for 3 h, and then the reaction was cooled to rt and concentrated. The yellow gum was suspended in EtOAc and washed with sat

$NaHCO_3$ (2x), brine, dried over $Na_2SO_4$, filtered, and concentrated. The residue was suspended in $Et_2O$ (3 ml), sonicated, and filtered. The solid was rinsed with $Et_2O$ (2 ml), air-dried with suction to afford 6-(3-chloro-6-(4-(difluoromethyl)-1H-1,2,3-triazol-1-yl)-2-fluorophenyl)pyrimidin-4-ol (0.23 g, 94% yield) as a yellow solid. MS(ESI) *m/z*: 342.0 $(M+H)^+$. $^1H$ NMR (400MHz, $CD_3OD$) δ 8.56 (t, *J*=1.4 Hz, 1H), 8.05 (d, *J*=0.9 Hz, 1H), 7.86 (dd, *J*=8.6, 7.7 Hz, 1H), 7.57 (dd, *J*=8.7, 1.7 Hz, 1H), 6.98 (t, *J*=54.0 Hz, 1H), 6.58 (t, *J*=1.2 Hz, 1H). $^{19}F$ NMR (376MHz, $CD_3OD$) δ -114.68 (s), -115.20 (s).

Intermediate 23

Preparation of tert-butyl N-[(1*S*)-1-(4-chloropyridin-2-yl)but-3-en-1-yl]carbamate

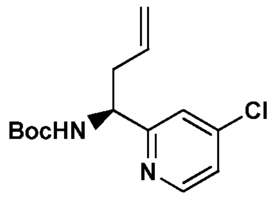

23A. Preparation of 4-chloro-2-[(E)-2-[(*S*)-2-methylpropane-2-sulfinyl]ethenyl]pyiridine

To a solution of *S*-(-)-*t*-butyl-sulfinamide (0.856 g, 7.06 mmol) in DCM (14.13 mL) was added sequentially $CuSO_4$ (2.481 g, 15.54 mmol) and 4-chloropicolinaldehyde (1.0 g, 7.06 mmol). The white suspension was stirred at rt. After 3 h, the brown suspension was filtered through CELITE®, eluting with DCM, to give a clear brown filtrate. Concentration gave crude product as a brown oil weighing 1.85 g. Purification by normal phase chromatography gave tert-butyl N-[(1*S*)-1-(4-chloropyridin-2-yl)but-3-en-1-yl]carbamate (1.31 g) as a clear, yellow oil. MS(ESI) *m/z*: 245.0 $(M+H)^+$.

23B. Preparation of (*R*)-N-[(1*S*)-1-(4-chloropyridin-2-yl)but-3-en-1-yl]-2-methylpropane-2-sulfinamide

To a cooled (0-5 °C) mixture of $InCl_3$ (13.56 g, 61.3 mmol) in THF (170 mL) was added dropwise over 30 min 1 M allylmagnesium bromide in $Et_2O$ (62 mL, 61.3 mmol). The reaction was allowed to warm to rt. After 1 h, a solution of 4-chloro-2-[(E)-2-[(S)-2-methylpropane-2-sulfinyl]ethenyl]pyridine (10 g, 40.9 mmol) in EtOH (170 mL) was added to the reaction mixture. After 2-3 h, the reaction was concentrated under vacuum at 50-55 °C. The crude material was partitioned between EtOAc (200ml) and water (50ml) and the layers were separated. The aqueous layer was extracted with EtOAc (2 x 50 ml). The organic layers were combined and washed with brine (100 ml), dried over $Na_2SO_4$, filtered and concentrated to give (*R*)-N-[(1*S*)-1-(4-chloropyridin-2-yl)but-3-en-1-yl]-2-methylpropane-2-sulfinamide (13.5 g, 106%) as a yellow oil. MS(ESI) *m/z:* 287.2 $(M+H)^+$.

23C. Preparation of (1*S*)-1-(4-chloropyridin-2-yl)but-3-en-1-amine

(R)-N-[(1*S*)-1-(4-Chloropyridin-2-yl)but-3-en-1-yl]-2-methylpropane-2-sulfinamide (75 g, 261 mmol) was dissolved in MeOH (1500 mL). 6 N HCl (750 ml, 4.5 mol) was added. The reaction was stirred at rt for 2-3 h and then was concentrated. The residue was diluted with water (2 L), washed with EtOAc (500 ml). The aqueous layer was basified with sat aq $Na_2CO_3$, then extracted into EtOAc (3 x 1 L). The combined organic layers were washed with water (1 L) and brine (1 L), dried over $Na_2SO_4$, filtered and conc. under vacuum at 50-55 °C to give (1*S*)-1-(4-chloropyridin-2-yl)but-3-en-1-amine (43g, 90%). MS(ESI) *m/z*: 183.2 $(M+H)^+$.

23D. Preparation of *tert*-butyl N-[(1*S*)-1-(4-chloropyridin-2-yl)but-3-en-1-yl]carbamate

(1*S*)-1-(4-Chloropyridin-2-yl)but-3-en-1-amine (42g, 230 mmol) was dissolved in DCM (420 mL), $Et_3N$ (32.1 mL, 230 mmol) was added followed by dropwise addition of $BOC_2O$ (53.4 mL, 230 mmol). The reaction was stirred at rt for 2-3 h. The reaction was diluted with excess DCM (1 L), washed with water (500 ml) and brine (500ml). The organic layer was dried over $Na_2SO_4$, filtered, and concentrated. The crude product was purified using silica gel chromatography to give *tert*-butyl N-[(1*S*)-1-(4-chloropyridin-2-yl)but-3-en-1-yl]carbamate (61 g, 86%) as a pale yellow solid. MS(ESI) *m/z*: 283.2 $(M+H)^+$. $^1H$ NMR (500 MHz, $CDCl_3$) δ 8.44 (d, 1H), 7.26-7.16 (dd, 2H), 5.69-5.61 (m, 1H), 5.59 (bs, 1H), 5.07-5.03 (m, 2H), 4.76 (bs, 1H), 2.62-2.55 (m, 2H), 1.42 (s, 9H).

Intermediate 24

Preparation of *tert*-butyl *N*-[(1S)-1-(3-bromophenyl)but-3-en-1-yl] carbamate

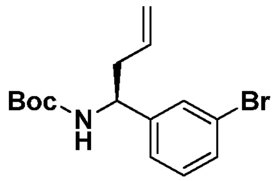

24A. Preparation of (R)-*N*-[(1E)-(3-bromophenyl)methylidene]-2-methylpropane-2-sulfinamide

To 3-bromobenzaldehyde (7.8 g, 42.2 mmol) was added (*R*)-2-methylpropane-2-sulfinamide (5.11 g, 42.2 mmol), $Cs_2CO_3$ (20.60 g, 63.2 mmol) in DCM (211 ml) and the resulting reaction mixture was stirred for 5 days. The reaction mixture was then partitioned with brine (50 ml) and DCM (50 ml). The aqueous layer was extracted with DCM (2 x 50 ml). The combined organic layers were washed with brine (25 ml), dried ($Na_2SO_4$), filtered and concentrated. Purification by normal phase chromatography using hexanes and EtOAc as eluents gave (R)-N-[(1E)-(3-bromophenyl)methylidene]-2-methylpropane-2-sulfinamide (11.8 g, 97%) as an amber oil. $^1H$ NMR (400MHz, $CDCl_3$) $\delta$ 8.53 (s, 1H), 8.02 (t, *J*=1.8 Hz, 1H), 7.74 (dt, *J*=7.7, 1.2 Hz, 1H), 7.64 (ddd, *J*=8.0, 2.0, 1.0 Hz, 1H), 7.36 (t, *J*=7.8 Hz, 1H), 1.34 - 1.22 (m, 9H). MS(ESI) *m/z*: 290 $(M+H)^+$.

24B. Preparation of (R)-*N*-[(1S)-1-(3-bromophenyl)but-3-en-1-yl]-2-methylpropane-2-sulfinamide

To (*R*)-N-[(1E)-(3-bromophenyl)methylidene]-2-methylpropane-2-sulfinamide (11.8 g, 40.9 mmol) in THF (190 ml), in a 3 neck flask, cooled to 0 °C, was added allyl bromide (3.90 ml, 45.0 mmol) and In (6.58 g, 57.3 mmol). After stirred at rt for 18 h, the reaction was heated to 50 °C for 6 h, then stirred at rt for 18 h. The reaction mixture was filtered through CELITE® and the filtrate was quenched with water (100 ml). A thick clear gelatinous material formed in the aqueous layer. The organics were extracted with EtOAc (4 x 75 ml). The combined organic layer was washed with brine, dried with $MgSO_4$, filtered and concentrated to give (R)-*N*-[(1S)-1-(3-bromophenyl)but-3-en-1-yl]-2-methylpropane-2-sulfinamide as a clear oil (9.6 g, 71%). $^1H$ NMR (400MHz, $CDCl_3$) $\delta$ 7.48 (t, *J*=1.8 Hz, 1H), 7.41 (dt, *J*=7.6, 1.6 Hz, 1H), 7.26 - 7.18 (m, 2H), 5.79 - 5.66 (m, 1H), 5.23 - 5.16 (m, 2H), 4.46 (ddd, *J*=8.1, 5.6, 2.0 Hz, 1H), 3.69 (s, 1H), 2.63 - 2.53 (m, 1H), 2.53 - 2.40 (m, 1H), 1.23 - 1.19 (m, 9H).

24C. Preparation of *tert*-butyl *N*-[(1S)-1-(3-bromophenyl)but-3-en-1-yl]carbamate

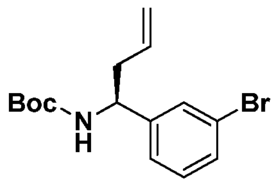

To (R)-*N*-[(1S)-1-(3-bromophenyl)but-3-en-1-yl]-2-methylpropane-2-sulfinamide (9.6 g, 29.1 mmol) in MeOH (300 ml) was added conc. HCl (4 ml). After 3 h, the reaction was concentrated and the residue was dissolved in DCM (300 ml), cooled to 0 °C, and then TEA (16.20 ml, 116 mmol) and $Boc_2O$ (6.75 ml, 29.1 mmol) in DCM (20 ml) were added. After 18 h, additional $Boc_2O$ (1 g) was added and the reaction was stirred 4 h. The reaction was quenched with water (100 ml) and extracted with DCM (3 x 50 ml). The combined organic layers were washed with brine (50 ml), dried ($Na_2SO_4$), filtered and concentrated. Purification by normal phase chromatography using hexanes and EtOAc as eluents gave *tert*-butyl N-[(1S)-1-(3-bromophenyl)but-3-en-1-yl]carbamate (7.3 g, 77%) as a white solid. MS(ESI) *m/z*: 326.08 $(M+H)^+$.

Intermediate 25

Preparation of N-[(1*S*)-1-(3-bromo-5-fluorophenyl)but-3-en-1-yl]carbamate

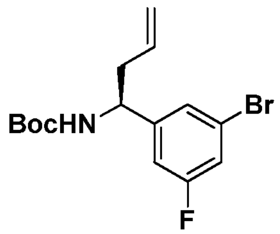

25A. Preparation of (*R*)-N-[(1E)-(3-bromo-5-fluorophenyl)methylidene]-2-methylpropane-2-sulfinamide

To 3-bromo-5-fluorobenzaldehyde (25g, 123 mol) dissolved in DCM (200 mL) was added (*R*)-2-methylpropane-2-sulfinamide (14.96 g, 123 mol) and $Cs_2CO_3$ (40.2 g, 123 mol). The reaction mixture was stirred at rt overnight. After this time, the reaction mixture was filtered and concentrated to give a yellow oil. The yellow oil was purified using a 120 g silica gel ISCO column eluted with hexanes and EtOAc to give (*R*)-N-[(1E)-(3-bromo-5-fluorophenyl)methylidene]-2-methylpropane-2-sulfinamide (35 g, 93%) as a yellow oil. $^1H$ NMR (500MHz, DMSO-$d_6$) $\delta$ 8.58 - 8.55 (m, 1H), 8.05 - 7.98 (m, 1H), 7.84 - 7.76 (m, 2H), 1.20 (s, 9H). LCMS *m/z* 306.1 (M+H).

25B. Preparation of (*R*)-N-[(1*S*)-1-(3-bromo-5-fluorophenyl)but-3-en-1-yl]-2-methylpropane-2-sulfinamide

N-[(1E)-(3-Bromo-5-fluorophenyl)methylidene]-2,2-dimethylpropanamide (35 g, 114 mol) was dissolved in THF (500 mL) in a large 3 neck RB flask and flushed with Ar. The solution was cooled to 0 °C and In powder (18.4 g, 160 mol) was added followed by dropwise addition of allylbromide (15.2 g, 126 mol). The reaction was stirred at 0 °C for 2 h, then the ice bath was removed and the reaction mixture was stirred at rt overnight. The reaction was quenched with water (2 L) and the gelatinous material was filtered through CELITE®. The filtrate was concentrated to an oily mass. The crude material was dissolved in water (2 L) and the organics were extracted with EtOAc (4 x 200 mL), dried over $MgSO_4$, filtered and concentrated to give an oil. The oily liquid was purified via a silica gel ISCO column and eluted with DCM/MeOH to afford (*R*)-N-[(1*S*)-1-(3-bromo-5-fluorophenyl)but-3-en-1-yl]-2-methylpropane-2-sulfinamide (34.9 g, 88% yield) as a semi solid mass. LCMS *m/z* 348.2 (M+H). $^1H$ NMR (500MHz, DMSO-$d_6$) $\delta$ 7.44 - 7.38 (m, 2H), 7.26 - 7.20 (m, 1H), 5.79 - 5.65 (m, 1H), 5.46 - 5.42 (m, 1H), 5.04 - 4.98 (m, 2H), 4.41 - 4.34 (m, 1H), 2.69 - 2.59 (m, 1H), 2.49-2.43 (m, 1H), 1.09 (s, 9H).

25C. Preparation of N-[(1*S*)-1-(3-bromo-5-fluorophenyl)but-3-en-1-yl]carbamate

To a cooled 0 °C solution of (R)-N-[(1S)-1-(3-bromo-5-fluorophenyl)but-3-en-1-yl]-2-methylpropane-2-sulfinamide (21.9 g, 100 mol) dissolved in MeOH (100 mL) was added conc. HCl (50 mL) dropwise and then the reaction was stirred at 0 °C for 48 h. After this time, the reaction mixture was concentrated to give a white solid mass. The residue was dissolved in water (1 L) and the organics were extracted with EtOAc (2 x 200 mL), dried over $MgSO_4$, filtered and concentrated to a brown oil (11.5 g). The aqueous layer was basified with NaOH and the organics were extracted with EtOAc (2 x 300 mL), dried over $MgSO_4$, filtered and concentrated to a brown oil (18 g). The combined oils were dissolved in DCM (500 mL) and to this was added $Boc_2O$ (22 g) followed by TEA (15 mL) and the reaction mixture was stirred at rt overnight. The reaction mixture was concentrated and purified via a 330g silica gel Isco column eluting with hexanes and EtOAc to give a white solid. The white solid was triturated with hexanes and the precipitate was collected by filtration to give N-[(1*S*)-1-(3-bromo-5-fluorophenyl)but-3-en-1-yl]carbamate (29.5 g, 87% yield).

Intermediate 26

Preparation of N-[(1*S*)-1-(5-bromopyridin-3-yl)but-3-en-1-yl]carbamate

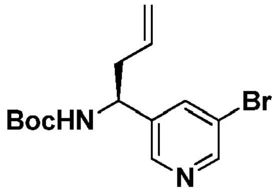

26A. Preparation of (*R*)-N-[(1E)-(5-chloropyridin-3-yl)methylidene]-2-methylpropane-2-sulfinamide

5-Bromonicotinaldehyde (6.6g, 35.9 mmol) was dissolved in DCM (200 mL). To the solution was added $Cs_2CO_3$ (11.68g, 35.9 mmol) and (*R*)-2-methylpropane-2-sulfinamide (4.34 g, 35.9 mol) and then the reaction mixture was stirred at rt overnight. The inorganics were filtered and the filtrate was concentrated to afford (*R*)-N-[(1E)-(5-chloropyridin-3-yl)methylidene]-2-methylpropane-2-sulfinamide as an oil (10.4g, 100% yield). LCMS *m*/*z* = 291.3.

26B. Preparation of (*R*)-N-[(1S)-1-(5-chloropyridin-3-yl)but-3-en-1-yl]-2-methylpropane-2-sulfinamide

To a solution of (*R*)-N-[(1E)-(5-chloropyridin-3-yl)methylidene]-2-methylpropane-2-sulfinamide (10.36 g, 35.8 mmol) in THF (150 mL) at 0 °C was added powdered In (5.76 g, 50.2 mmol) followed by allylbromide (3.72 mL, 43.0 mmol). The reaction mixture was sealed and was stirred vigorously at 0 °C for 1 h and then warmed to rt and stirred overnight. The reaction gradually turned from pale yellow to greenish yellow to dark greenish yellow with the indium metal forming fine particles. LCMS of the greenish black heterogenous solution showed the desired product peak and mass. The solution was filtered through a pad of CELITE® and washed with EtOAc. The solution was concentrated to afford a yellow solid mass. The solids were dissolved in MeOH (100 mL) and a solution of 4 N HCl in dioxane (25 mL) was added. The resultant solution was stirred at rt. After 6 h, conc. HCl (1 mL) was added and stirring was continued for 1 h. The reaction mixture was concentrated to give a yellow solid. The solid was dissolved in a mixture of THF and dioxane and DCM (1:1:1, 200 mL). To this solution was added TEA (20 mL) followed by $Boc_2O$ (8.1 g, 37.1 mmol) and the reaction mixture was stirred overnight. LCMS confirmed the desired product formation. To the reaction mixture was added water (200 mL) and the mixture was filtered through a pad of CELITE® and washed with EtOAc (200 mL). The aqueous layer was extracted with EtOAc (2 x 100 mL). The combined organic layer was dried over $MgSO_4$, filtered and concentrated to give a reddish brown oil. The crude material was purified via a 80 g silica gel ISCO column and eluted with hexanes and EtOAc. (*R*)-N-[(1S)-1-(5-Chloropyridin-3-yl)but-3-en-1-yl]-2-methylpropane-2-sulfinamide was obtained as a pale yellow semi solid mass (4.3 g, 36.7% yield). LCMS *m*/*z* 327.1(M+H). $^1$H NMR (400MHz, $CDCl_3$) δ 8.61 - 8.59 (m, 1H), 8.51 - 8.48 (m, 1H), 7.77 - 7.74 (m, 1H), 5.76 - 5.63 (m, 1H), 5.23 - 5.14 (m, 2H), 5.00 - 4.84 (m, 1H), 4.83 - 4.70 (m, 1H), 2.60 - 2.44 (m, 2H), 1.48 - 1.35 (m, 9H).

Intermediate 27

Preparation of *tert*-butyl N-[(1*S*)-1-(2-bromopyridin-4-yl)but-3-en-1-yl] carbamate

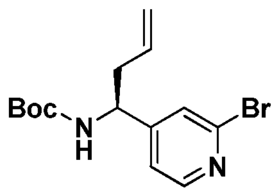

27A. Preparation of (*R*)-N-[(1E)-(2-bromopyridin-4-yl)methylidene]-2-methylpropane-2-sulfinamide

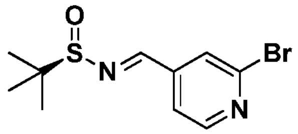

To a stirred suspension of (*R*)-2-methylpropane-2-sulfinamide (13.03 g, 108 mmol) and $Cs_2CO_3$ (52.5 g, 161 mmol) in DCM (400 ml) was added 2-bromopyridine-4-carbaldehyde (20 g, 108 mmol) over 10 min. The reaction mixture was then stirred for 18.5 h at rt. The reaction mixture was concentrated and the residue was diluted with EtOAc (50 ml) and washed with brine (3 x 20 ml). The organic layer was dried over $MgSO_4$, filtered and the filtrate concentrated. The residue was purified by normal phase chromatography using hexanes and EtOAc as eluents to afford (*R*)-N-[(1E)-(2-bromopyridin-4-yl)methylidene]-2-methylpropane-2-sulfinamide (27.2 g, 87%) as a white solid. MS(ESI) *m*/*z*: 289-291.0 $(M+H)^+$.

27B. Preparation of (*R*)-N-[(1*S*)-1-(2-bromopyridin-4-yl)but-3-en-1-yl]-2-methylpropane-2-sulfonamide

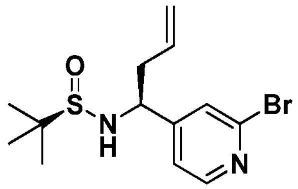

To a solution of (*R*)-N-[(1E)-(2-bromopyridin-4-yl)methylidene]-2-methylpropane-2-sulfinamide (0.73 g, 2.52 mmol) and In (0.435 g, 3.79 mmol) in THF (6 ml) was slowly added 3-bromoprop-1-ene (0.458 g, 3.79 mmol) and resulting solution was heated at 60 °C for 18 h. The reaction mixture was cooled, filtered through CELITE® and the filtrate was concentrated. To the residue was added EtOAc (100 ml) and 5% aq $NaHCO_3$ (1 L) and an emulsion formed immediately. The suspension was filtered through paper. The organic layer was washed with brine, dried over $Na_2SO_4$, filtered, and concentrated. The residue was purified by normal phase chromatography using hexanes and EtOAc as eluents to afford (0.62 g, 74%) of (*R*)-N-[(1*S*)-1-(2-bromopyridin-4-yl)but-3-en-1-yl]-2-methylpropane-2-sulfonamide as a yellow liquid. MS(ESI) *m*/*z*: 331-333.0 $(M+H)^+$.

27C. Preparation of tert-butyl N-[(1*S*)-1-(2-bromopyridin-4-yl)but-3-en-1-yl]carbamate

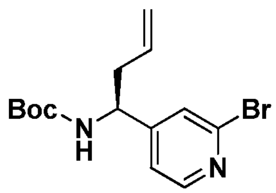

To a solution of (*R*)-N-[(1*S*)-1-(2-bromopyridin-4-yl)but-3-en-1-yl]-2-methylpropane-2-sulfinamide (1.38 g, 4.17 mmol) in MeOH (10 ml) was added 4 N HCl in dioxane (5.21 mL, 20.83 mmol). The reaction mixture was stirred for 1.5 h at rt, then was concentrated. To the resulting residue was added ACN (10 ml), TEA (5.8 ml, 41.7 mmol) and $Boc_2O$ (1.818 g, 8.33 mmol). After 18 h, the reaction mixture was concentrated and the residue was taken up in EtOAc, washed with water, brine, dried over $MgSO_4$, filtered and concentrated. The resulting residue was purified by normal phase chromatography using hexanes and EtOAc as eluents to afford *tert*-butyl N-[(1*S*)-1-(2-bromopyridin-4-yl)but-3-en-1-yl] carbamate (0.80 g, 58.7%) as a pale yellow oil. MS(ESI) *m*/*z*: 324-326.1 $(M+H)^+$.

Intermediate 28

Preparation of (9*R*,13*S*)-13-amino-3,9-dimethyl-3,4,7,18-tetraazatricyclo[12.3.1.$0^{2,6}$] octadeca-1(18),2(6),4,14,16-pentaen-8-one

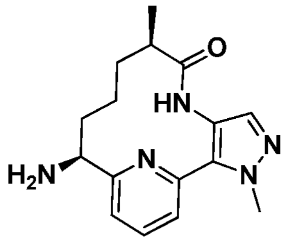

28A. Preparation of (*S*)-N-[(1E)-(6-chloropyridin-2-yl)methylidene]-2-methylpropane-2-sulfinamide

To a solution of (*S*)-2-methylpropane-2-sulfinamide (1.712 g, 14.13 mmol) in DCM (61.4 mL) was added $Cs_2CO_3$ (6.91 g, 21.19 mmol) and 6-chloropicolinaldehyde (2.0 g, 14.13 mmol). The resulting white suspension was stirred at rt. After 17 h, the reaction was filtered. The filtrate was diluted with EtOAc (100 ml) and washed with brine (3 x 50 mL). The organic layer was dried over $MgSO_4$, filtered and concentrated to give (*S*)-N-[(1E)-(6-chloropyridin-2-yl)methylidene]-2-methylpropane-2-sulfinamide (3.58g, 100%) as a yellow oil. $^1H$ NMR (400MHz, $CDCl_3$) $\delta$ 8.65 (s, 1H), 7.99 - 7.94 (m, 1H), 7.79 (t, *J*=7.7 Hz, 1H), 7.45 (dd, *J*=7.9, 0.7 Hz, 1H), 1.28 (s, 10H).

28B. Preparation of (S)-N-[(1S)-1-(6-chloropyridin-2-yl)but-3-en-1-yl]-2-methylpropane-2-sulfinamide, and

28C. Preparation of (S)-N-[(1R)-1-(6-chloropyridin-2-yl)but-3-en-1-yl]-2-methylpropane-2-sulfinamide

To a mixture of (*S*)-N-[(1E)-(6-chloropyridin-2-yl)methylidene]-2-methylpropane-2-sulfinamide (1.73 g, 7.07 mmol) and In (0.92 g, 10.60 mmol) in THF (17.7 ml) was slowly added 3-bromoprop-1-ene (0.92 g, 10.60 mmol). The reaction was heated at 60 °C overnight. The reaction mixture was cooled to rt, filtered through CELITE® and the filtrate was concentrated. The resulting residue was purified by normal phase chromatography, using hexanes and EtOAc, which gave a 5.6:1 of (*S*)-N-[(1*S*)-1-(6-chloropyridin-2-yl)but-3-en-1-yl]-2-methylpropane-2-sulfinamide:(*S*)-N-[(1*R*)-1-(6-chloropyridin-2-yl)but-3-en-1-yl]-2-methylpropane-2-sulfinamide (2.42 g, 58%) as a brown semi-solid. MS(ESI) *m/z:* 287.4 $(M+H)^+$.

28D. Preparation of (S)-2-methyl-N-[(1R)-1-[6-(1-methyl-4-nitro-1H-pyrazol-5-yl)pyridin-2-yl]but-3-en-1-yl]propane-2-sulfinamide (Diastereomer A), and

28E. Preparation of (S)-2-methyl-N-[(1S)-1-[6-(1-methyl-4-nitro-1H-pyrazol-5-yl)pyridin-2-yl]but-3-en-1-yl]propane-2-sulfinamide (Diastereomer B)

To a $N_2$ flushed pressure vial was added 5.6:1 of (*S*)-N-[(1*S*)-1-(6-chloropyridin-2-yl)but-3-en-1-yl]-2-methylpropane-2-sulfinamide: (*S*)-N-[(1*R*)-1-(6-chloropyridin-2-yl)but-3-en-1-yl]-2-methylpropane-2-sulfinamide (2.18 g, 7.60 mmol), 1-methyl-4-nitro-1*H*-pyrazole (0.966 g, 7.60 mmol), prepared as described in Intermediate 32A, di(adamant-1-yl)(butyl)phosphine (0.954 g, 2.66 mmol), PvOH (0.300 ml, 2.58 mmol), $K_2CO_3$ (3.62 g, 26.2 mmol), $Pd(OAc)_2$ (0.341 g, 1.52 mmol) and DMF (15.2 mL). The vial was purged with Ar. The vial was sealed and heated at 120 °C overnight. The reaction mixture was cooled to rt, partitioned between water and EtOAc, and the layers were separated. The aqueous layer was extracted with EtOAc (3x) and the organic layers were combined and concentrated. The crude product was purified using normal phase chromatography followed a second purification by reverse phase chromatography to give (S)-2-methyl-N-[(1R)-1-[6-(1-methyl-4-nitro-1H-pyrazol-5-yl)pyridin-2-yl]but-3-en-1-yl]propane-2-sulfinamide (Diastereomer A) (0.275 g, 13%), MS(ESI) *m/z*: 274.4 $(M+H)^+$; and (S)-2-methyl-N-[(1S)-1-[6-(1-methyl-4-nitro-1H-pyrazol-5-yl)pyridin-2-yl]but-3-en-1-yl]propane-2-sulfinamide (Diastereomer B) (1.2 g, 57%); MS(ESI) *m/z*: 274.4 $(M+H)^+$.

28F. Preparation of tert-butyl N-[(1*S*)-1-[6-(1-methyl-4-nitro-1H-pyrazol-5-yl)pyridin-2-yl]but-3-en-1-yl]carbamate

(1*S*)-1-(6-(1-Methyl-4-nitro-1H-pyrazol-5-yl)pyridin-2-yl)but-3-en-1-amine (Diastereomer B) (1.2 g, 3.18 mmol)

was dissolved in MeOH (5 mL) and dioxane (25 ml). 4 N HCl in dioxane (4.8 ml, 19.1 mmol) was added. The reaction was stirred at rt for 3 h and then was concentrated. The residue was coevaporated with toluene, dissolved in DCM (40 mL), and cooled to 0 °C. TEA (4.43 mL, 31.8 mmol) was added followed by $BOC_2O$ (0.738 mL, 3.18 mmol). The reaction was stirred at 0 °C for 15 min and then the reaction was allowed to warm to rt. After 2 h, the reaction was diluted with DCM, washed with sat $NaHCO_3$, brine, and concentrated. Purification by normal phase chromatography gave *tert*-butyl N-[(1*S*)-1-[6-(1-methyl-4-nitro-1H-pyrazol-5-yl)pyridin-2-yl]but-3-en-1-yl]carbamate (393 mg, 33% yield) as an orange oil. MS(ESI) *m*/*z*: 374.5 $(M+H)^+$. $^1H$ NMR (400MHz, $CDCl_3$) δ 8.19 (s, 1H), 7.84 (t, *J*=7.8 Hz, 1H), 7.55 (d, *J*=7.7 Hz, 1H), 7.38 (d, *J*=7.7 Hz, 1H), 5.77 - 5.58 (m, 1H), 5.40 (br. s., 1H), 5.13 - 5.01 (m, 2H), 4.92 (d, *J*=6.8 Hz, 1H), 3.86 (s, 3H), 2.71 - 2.51 (m, 2H), 1.43 (s, 9H).

28G. Preparation of *tert*-butyl N-[(1*S*)-1-[6-(4-amino-1-methyl-1H-pyrazol-5-yl)pyridin-2-yl]but-3-en-1-yl]carbamate

To a solution of *tert*-butyl N-[(1*S*)-1-[6-(1-methyl-4-nitro-1H-pyrazol-5-yl) pyridin-2-yl]but-3-en-1-yl]carbamate (393 mg, 1.05 mmol) in MeOH (6.4 mL) was added AcOH (0.64 mL). The reaction mixture was heated to 45 °C then Zn powder (206 mg, 3.16 mmol) was added portionwise. After 1 h, additional Zn (198 mg) was added. Upon completion of the reaction, the mixture was cooled to rt, partitioned between DCM and sat $NaHCO_3$, and the layers were separated. The aqueous layer was extracted with DCM (2x). The organic layers were combined and washed with brine, dried over $MgSO_4$, filtered and concentrated to give *tert*-butyl N-[(1*S*)-1-[6-(4-amino-1-methyl-1H-pyrazol-5-yl)pyridin-2-yl]but-3-en-1-yl]carbamate (343 mg, 95% yield) as a yellow foam. MS(ESI) *m*/*z*: 344.5 $(M+H)^+$. $^1H$ NMR (400MHz, $CDCl_3$) δ 7.74 (t, *J*=7.8 Hz, 1H), 7.39 (dd, *J*=7.8, 0.8 Hz, 1H), 7.25 - 7.18 (m, 1H), 7.14 (d, *J*=7.7 Hz, 1H), 5.70 (ddt, *J*=17.1, 10.2, 7.0 Hz, 1H), 5.46 (d, *J*=6.8 Hz, 1H), 5.13 - 4.99 (m, 2H), 4.89 (d, *J*=6.8 Hz, 1H), 4.01 (s, 3H), 2.71 - 2.53 (m, 2H), 1.49 - 1.30 (m, 9H).

28H. Preparation of *tert*-butyl N-[(1*S*)-1-(6-{1-methyl-4-[(2*R*)-2-methylbut-3-enamido]-1H-pyrazol-5-yl}pyridin-2-yl)but-3-en-1-yl]carbamate

To *tert*-butyl N-[(1*S*)-1-[6-(4-amino-1-methyl-1H-pyrazol-5-yl) pyridin-2-yl]but-3-en-1-yl]carbamate (343 mg, 0.999 mmol) in EtOAc (3.33 ml) was added a solution of (*R*)-2-methylbut-3-enoic acid (0.150 g, 1.498 mmol), prepared as described in Intermediate 2, in EtOAc (1 ml). The mixture was cooled to 0 °C and pyridine (0.24 ml, 3.0 mmol) was added, followed by the addition of a solution of 50% T3P® in EtOAc (1.19 ml, 1.50 mmol). After 2 h, the reaction was partitioned between sat $NaHCO_3$ and EtOAc, and the layers were separated. The aqueous layer was extracted with EtOAc (2x). The organic layers were combined and washed with brine and then concentrated. Purification by normal phase chromatography gave *tert*-butyl N-[(1*S*)-1-(6-{1-methyl-4-[(2*R*)-2-methylbut-3-enamido]-1H-pyrazol-5-yl}pyridin-2-yl) but-3-en-1-yl]carbamate (360 mg, 85%) as a yellow solid. MS(ESI) *m*/*z*: 426.5 $(M+H)^+$. $^1H$ NMR (400MHz, $CDCl_3$) δ 9.35 (br. s., 1H), 8.30 (s, 1H), 7.82 (t, *J*=7.8 Hz, 1H), 7.40 (d, *J*=7.9 Hz, 1H), 7.32 - 7.19 (m, 1H), 6.01 (ddd, *J*=17.4, 10.0, 7.6 Hz, 1H), 5.78 - 5.57 (m, 1H), 5.35 - 5.04 (m, 5H), 4.91 (br. s., 1H), 4.06 (s, 3H), 3.26 - 3.06 (m, 1H), 2.81 - 2.54 (m, 2H), 1.54 - 1.30 (m, 12H).

281. Preparation of *tert*-butyl N-[(9*R*,10E,13,5*S*)-3,9-dimethyl-8-oxo-3,4,7,18-tetraazatricyclo[12.3.1.$0^{2,6}$]octadeca-1(18),2(6),4,10,14,16-hexaen-13-yl]carbamate

A solution of *tert*-butyl N-[(1*S*)-1-(6-{1-methyl-4-[(2*R*)-2-methylbut-3-enamido]-1H-pyrazol-5-yl}pyridin-2-yl) but-3-en-1-yl]carbamate (140 mg, 0.329 mmol) in EtOAc (25 ml) was purged with Ar for 20 min. Second Generation Grubbs Catalyst (0.112 g, 0.132 mmol) was added and the reaction mixture was heated at 80 °C overnight. The reaction mixture was cooled to rt and concentrated. Purification by normal phase chromatography and then by reverse phase chromatography was done. The fractions containing the desired product were made basic (pH ~8) with sat $NaHCO_3$ and then concentrated. The residue was partitioned between water and EtOAc, and the layers were separated. The aqueous layer was extracted with DCM (3x) and EtOAc (3x). The organic layers were combined and washed with brine, dried $MgSO_4$, filtered and concentrated to give *tert*-butyl N-[(9*R*,10E,13*S*)-3,9-dimethyl-8-oxo-3,4,7,18-tetraazatricyclo[12.3.1.$0^{2,6}$]octadeca-1(18),2(6),4,10,14,16-hexaen-13-yl]carbamate (96mg, 66% yield). MS(ESI) *m*/*z*: 398.2 $(M+H)^+$. $^1H$ NMR (400MHz, $CDCl_3$) δ 11.12 (br. s., 1H), 8.08 (s, 1H), 7.84 (t, *J*=7.9 Hz, 1H), 7.39 (dd, *J*=7.9, 0.7 Hz, 1H), 7.32 - 7.24 (m, 1H), 5.98 - 5.83 (m, 1H), 5.55 (dd, *J*=15.7, 7.4 Hz, 1H), 5.41 (d, *J*=6.6 Hz, 1H), 5.04 (m, 1H), 4.10 - 4.03 (m, 3H), 3.15 (quin, *J*=7.3 Hz, 1H), 2.84 - 2.56 (m, 2H), 1.51 - 1.32 (m, 12H).

28J. Preparation of *tert*-butyl N-[(9*R*,13*S*)-3,9-dimethyl-8-oxo-3,4,7,18-tetraazatricyclo [12.3.1.$0^{2,6}$]octadeca-1(18),2(6),4,14,16-pentaen-13-yl]carbamate, and

28K. Preparation of *tert*-butyl N-[(9*R*,13*S*)-3,9-dimethyl-8-oxo-3,4,7,18-tetraazatricyclo [12.3.1.$0^{2,6}$]octadeca-2(6),4-dien-13-yl]carbamate

A solution of *tert*-butyl N-[(9*R*,10E,13*S*)-3,9-dimethyl-8-oxo-3,4,7,18-tetraazatricyclo[12.3.1.$0^{2,6}$]octadeca-1(18),2(6),4,10,14,16-hexaen-13-yl]carbamate (0.096 g, 0.024 mmol) in EtOH (4 ml) was hydrogenated at 20 psi $H_2$ in the presence of $PtO_2$ (20 mg) for 20 h. The mixture was filtered, washing with MeOH and EtOAc. The filtrate was concentrated and then purified by reverse phase chromatography to give, following neutralization of the fractions and extraction, *tert*-butyl N-[(9*R*,13*S*)-3,9-dimethyl-8-oxo-3,4,7,18-tetraazatricyclo[12.3.1.$0^{2,6}$]octadeca-2(6),4-dien-13-yl]carbamate (20 mg, 20.4% yield), MS(ESI) *m*/*z*: 406.2 $(M+H)^+$; and *tert*-butyl N-[(9*R*,13*S*)-3,9-dimethyl-8-oxo-3,4,7,18-tetraazatricyclo[12.3.1.$0^{2,6}$]octadeca-1(18),2(6),4,14,16-pentaen-13-yl]carbamate (68 mg, 70.5% yield), MS(ESI) *m*/*z*: 400.2 $(M+H)^+$.

28L. Preparation of (9*R*,13*S*)-13-amino-3,9-dimethyl-3,4,7,18-tetraazatricyclo [12.3.1.$0^{2,6}$]octadeca-1(18),2(6),4,14,16-pentaen-8-one

To a solution of *tert*-butyl N-[(9*R*,13*S*)-3,9-dimethyl-8-oxo-3,4,7,18-tetraazatricyclo[12.3.1.$0^{2,6}$]octadeca-1(18),2(6),4,14,16-pentaen-13-yl]carbamate (0.035 g, 0.088 mmol) in DCM (0.5 ml) was added TFA (0.2 mL, 2.60 mmol). After stirring for 1 h, the reaction mixture was concentrated to dryness, and coevaporated with $CH_3CN$. The residue was neutralized by dissolving in MeOH, passing through $NaHCO_3$ cartridge (StratoSpheres SPE; 500 mg, 0.90 mmol loading), and the filtrate concentrated to give (9*R*,13*S*)-13-amino-3,9-dimethyl-3,4,7,18-tetraazatricyclo[12.3.1.$0^{2,6}$]octadeca-1(18),2(6),4,14,16-pentaen-8-one (15 mg, 57% yield) as clear glass. MS(ESI) *m*/*z*: 300.5 $(M+H)^+$.

Intermediate 29

Preparation of (9*R*,13*S*)-13-amino-16-fluoro-3,9-dimethyl-3,4,7-triazatricyclo [12.3.1.$0^{2,6}$]octadeca-1(18),2(6),4,14,16-pentaen-8-one

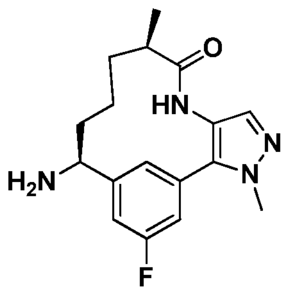

29A. Preparation of *tert*-butyl N-[(1*S*)-1-[3-fluoro-5-(1-methyl-4-nitro-1*H*-pyrazol-5-yl)phenyl]but-3-en-1-yl]carbamate

To *tert*-butyl N-[(1S)-1-(3-bromo-5-fluorophenyl)but-3-en-1-yl] carbamate (0.19 g, 0.552 mmol), 1-methyl-4-nitro-1*H*-pyrazole (0.070 g, 0.552 mmol), di(adamantan-1-yl)(butyl)phosphine (0.059 g, 0.166 mmol), pivalic acid (0.019 ml, 0.166 mmol), $K_2CO_3$ (0.229 g, 1.656 mmol) was added DMF (1.1 ml), and the mixture was purged with Ar. $Pd(OAc)_2$ (0.025 g, 0.110 mmol) was added and the reaction was heated at 120 °C for 18 h. The reaction was partitioned between water (15 ml) and EtOAc (30 ml). The aqueous layer was extracted with EtOAc (2 x 20 ml). The combined organic layers was washed with brine (15 ml), dried over $MgSO_4$, filtered and concentrated. The residue was purified by normal phase chromatography using hexanes and EtOAc as eluents to give *tert*-butyl N-[(1*S*)-1-[3-fluoro-5-(1-methyl-4-nitro-1*H*-pyrazol-5-yl)phenyl]but-3-en-1-yl]carbamate (0.123 g, 57%) as a yellow oil. $^1$H NMR (400MHz, $CDCl_3$) δ 8.23 - 8.17 (m, 1H), 7.22 - 7.16 (m, 1H), 7.10 (s, 1H), 7.01 (dt, *J*=8.5, 1.9 Hz, 1H), 5.76 - 5.60 (m, 1H), 5.22 - 5.11 (m, 2H), 4.90 (br. s., 1H), 4.78 (br. s., 1H), 3.78 - 3.69 (m, 3H), 2.60 - 2.48 (m, 2H), 1.41 (br. s., 9H).

29B. Preparation of *tert*-butyl *N*-[(1*S*)-1-[3-(4-amino-1-methyl-1*H*-pyrazol-5-yl)-5-fluorophenyl]but-3-en-1-yl]carbamate

To *tert*-butyl *N*-[(1S)-1-[3-fluoro-5-(1-methyl-4-nitro-1*H*-pyrazol-5-yl)phenyl]but-3-en-1-yl]carbamate (0.123 g, 0.315 mmol) dissolved in acetone (5 ml) / water (1 ml), cooled to 0 °C, and $NH_4Cl$ (0.084 g, 1.575 mmol) and Zn (0.206 g, 3.15 mmol) were added. The ice bath was removed. After 3 h, the reaction was filtered and filtrate was partitioned between water (10 ml) and EtOAc (30 ml). The aqueous layer was extracted with EtOAc (2 x 20 ml). The combined organic layers was washed with brine (10 ml), dried over $MgSO_4$, filtered and concentrated. The residue was purified by normal phase chromatography using DCM and 0-10% MeOH as eluents to give *tert*-butyl *N*-[(1*S*)-1-[3-(4-amino-1-methyl-1*H*-pyrazol-5-yl)-5-fluorophenyl]but-3-en-1-yl]carbamate (0.105 g, 92%). MS(ESI) *m*/*z*: 361.08 $(M+H)^+$.

29C. Preparation of *tert*-butyl *N*-[(1S)-1-(3-fluoro-5-{1-methyl-4-[(2R)-2-methylbut-3-enamido]-1*H*-pyrazol-5-yl}phenyl)but-3-en-1-yl]carbamate

To *tert*-butyl *N*-[(1S)-1-[3-(4-amino-1-methyl-1*H*-pyrazol-5-yl)-5-fluorophenyl]but-3-en-1-yl]carbamate (0.105 g, 0.291 mmol) in EtOAc (0.58 ml) was added (R)-2-methylbut-3-enoic acid (0.035 g, 0.350 mmol), prepared as described in Intermediate 2, in 0.3 ml EtOAc. The mixture was cooled to 0 °C and Hunig's Base (0.153 ml, 0.874 mmol) followed by a solution of 50% T3P® in EtOAc (0.347 ml, 0.583 mmol) were added. After 4 h, the reaction was partitioned with sat $NaHCO_3$ (5 ml) and EtOAc (5 ml). The aqueous layer was extracted with EtOAc (2 x 10 ml). The combined organic layers was washed with brine (5 ml), dried over $MgSO_4$, filtered and concentrated. The residue was purified by normal phase chromatography using hexanes and EtOAc as eluents to give the desired product (53.0 mg, 41%) as a yellow foam. MS(ESI) *m*/*z*: 443.5 $(M+H)^+$.

29D. Preparation of tert-butyl *N*-[(9*R*,10E,13*S*)-16-fluoro-3,9-dimethyl-8-oxo-3,4,7-triazatricyclo[12.3.1.0$^{2,6}$]octadeca-1(18),2(6),4,10,14,16-hexaen-13-yl]carbamate

A solution of *tert*-butyl *N*-[(1S)-1-(3-fluoro-5-{1-methyl-4-[(2R)-2-methylbut-3-enamido]-1*H*-pyrazol-5-yl}phenyl)but-3-en-1-yl]carbamate (0.053 g, 0.120 mmol) in degassed DCE (10 ml) was heated to 120 °C for 30 min in a microwave in the presence of Second Generation Grubbs Catalyst (0.041 g, 0.048 mmol). The reaction mixture was directly purified by normal phase chromatography using hexanes and EtOAc as eluents to give *tert*-butyl *N*-[(9*R*,10E,13*S*)-16-fluoro-3,9-dimethyl-8-oxo-3,4,7-triazatricyclo [12.3.1.0$^{2,6}$]octadeca-1(18),2(6),4,10,14,16-hexaen-13-yl]carbamate (27.0 mg, 54%) as a dark solid. MS(ESI) *m*/*z*: 415.4 $(M+H)^+$.

29E. Preparation of (9*R*,13S)-13-amino-16-fluoro-3,9-dimethyl-3,4,7-triazatricyclo [12.3.1.0$^{2,6}$]octadeca-1(18),2(6),4,14,16-pentaen-8-one

A solution of *tert*-butyl N-[(9R,10E,13S)-16-fluoro-3,9-dimethyl-8-oxo-3,4,7-triazatricyclo[12.3.1.0$^{2,6}$]octadeca-1(18),2(6),4,10,14,16-hexaen-13-yl]carbamate (0.027 g, 0.065 mmol) in EtOH (3 ml) was hydrogenated in the presence of $PtO_2$ (5 mg) for 6 h. After this time, the reaction was filtered through CELITE® and the filtrate was concentrated to *tert*-butyl N-[(9*R*,13*S*)-16-fluoro-3,9-dimethyl-8-oxo-3,4,7-triazatricyclo[12.3.1.0$^{2,6}$]octadeca-1(18),2(6),4,14,16-pentaen-13-yl]carbamate (19 mg). The Boc protecting group was removed by dissolving the material in 3 ml of 50% TFA/DCM. After 2 h, the reaction mixture was concentrated and the residue was taken up in DCM and MeOH, and filtered through a basic cartridge. Concentration of the filtrate afforded (9R,13S)-13-amino-16-fluoro-3,9-dimethyl-3,4,7-triazatricyclo[12.3.1.0$^{2,6}$] octadeca-1(18),2(6),4,14,16-pentaen-8-one (19 mg, 92%) as a dark solid. MS(ESI) *m*/*z*: 317.4 $(M+H)^+$.

Intermediate 30

Preparation of (9*R*,13*S*)-13-amino-3-(difluoromethyl)-9-methyl-3,4,7,15-tetraazatricyclo[12.3.1.0$^{2,6}$]octadeca-1(18),2(6),4,14,16-pentaen-8-one

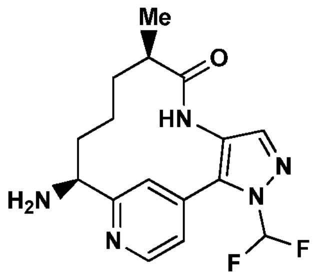

30A. Preparation of 1-(difluoromethyl)-4-nitro-1*H*-pyrazole

$Cs_2CO_3$ (14.41 g, 44.2 mmol) was suspended in a solution of 4-nitro-1H-pyrazole (5.00 g, 44.2 mmol) and DMF (40 mL). After heating to 120 °C for 5 min, solid sodium 2-chloro-2,2-difluoroacetate (13.48 g, 88 mmol) was added in 10 equal portions over 20 min. The reaction was complete after 10 min of additional heating. The mixture was added to a separatory funnel containing 100 mL water and extracted with $Et_2O$ (2 x 50 mL). The combined organic layers were concentrated. Purification by normal-phase chromatography eluting with a gradient of hexanes/EtOAc yielded 1-(difluoromethyl)-4-nitro-1H-pyrazole (6.99 g, 42.9 mmol, 97% yield) as a clear, colorless oil. $^1$H NMR (500MHz, $CDCl_3$) δ 8.58 (s, 1H), 8.22 (s, 1H), 7.39 - 7.05 (t, *J* = 60 Hz, 1H).

30B. Preparation of (*S*)-*tert*-butyl (1-(4-(1-(difluoromethyl)-4-nitro-1*H*-pyrazol-5-yl)pyridin-2-yl)but-3-en-1-yl)carbamate

To a $N_2$ flushed, 500 mL RBF was added (*S*)-*tert*-butyl (1-(4-chloropyridin-2-yl)but-3-en-1-yl)carbamate, prepared as described in Intermediate 23, (10 g, 35.4 mmol), 1-(difluoromethyl)-4-nitro-1*H*-pyrazole, prepared as described in Intermediate 30A, (6.34 g, 38.9 mmol) and dioxane (100 mL). The solution was bubbled with $N_2$ for 5 min and $Pd(OAc)_2$ (0.40 g, 1.7 mmol), di(adamantan-1-yl)(butyl)phosphine (1.27 g, 3.5 mmol), $K_2CO_3$ (14.7 g, 106 mmol) and PvOH (1.08 g, 10.61 mmol) were added. The reaction mixture was bubbled with $N_2$ for 5 min, then heated to 100 °C for 3 h. Water (200 mL) was added. The reaction mixture was then extracted with EtOAc (2 x 200 mL). The combined organic extracts were washed with water (200 mL), brine (200 mL), dried over $Na_2SO_4$, filtered and concentrated. Purification by normal phase chromatography eluting with a gradient of hexanes/EtOAc afforded (*S*)-*tert*-butyl (1-(4-(1-(difluoromethyl)-4-nitro-1*H*-pyrazol-5-yl)pyridin-2-yl)but-3-en-1-yl)carbamate (12.91 g, 31.5 mmol, 89% yield) as a yellowish oil. MS(ESI) *m/z*: 410.4 $[M+H]^+$. $^1$H NMR (400MHz, $CDCl_3$) δ 8.80 (dd, *J*=5.1, 0.7 Hz, 1H), 8.36 (s, 1H), 7.34 (s, 1H), 7.31 (dd, *J*=5.1, 1.5 Hz, 1H), 7.27 - 6.91 (t, *J*=58 Hz, 1H), 5.79 - 5.63 (m, 1H), 5.16 - 5.03 (m, 2H), 4.92 (d, *J*=5.9 Hz, 1H), 2.67 (t, *J*=6.4 Hz, 2H), 1.46 (br. s., 9H).

30C. Preparation of (*S*)-*tert*-butyl (1-(4-(4-amino-1-(difluoromethyl)-1*H*-pyrazol-5-yl)pyridin-2-yl)but-3-en-1-yl)carbamate

To a 100 mL, 3-necked RBF was added a solution of (*S*)-*tert*-butyl (1-(4-(1-(difluoromethyl)-4-nitro-1*H*-pyrazol-5-yl)pyridin-2-yl)but-3-en-1-yl)carbamate (0.78 g, 1.90 mmol) in MeOH (12 mL) and a solution of $NH_4Cl$ (1.02 g, 19 mmol) in water (3 mL). To the solution was added Fe (0.53 g, 9.49 mmol). The reaction mixture was heated to 65 °C for 3 h. Water (50 mL) was added. After cooling to rt, the mixture was filtered through a CELITE® pad and rinsed with MeOH (200 mL). The filtrate was concentrated. The residue was partitioned between EtOAc (100 mL) and water (100 mL). The organic phase was separated, washed with water (100 mL), brine (100 mL), dried over $Na_2SO_4$, filtered and concentrated *in vacuo.* Purification by normal phase chromatography eluting with a gradient of DCM/MeOH yielded (*S*)-*tert*-butyl (1-(4-(4-amino-1-(difluoromethyl)-1*H*-pyrazol-5-yl)pyridin-2-yl)but-3-en-1-yl)carbamate (0.585 g, 1.54 mmol, 81% yield) as an oil. MS(ESI) *m/z*: 380.1 $[M+H]^+$. $^1$H NMR (400MHz, $CDCl_3$) δ 8.70 (dd, *J*=5.0, 0.7 Hz, 1H), 7.43 (s, 1H), 7.36 (s, 1H), 7.32 (dd, *J*=5.1, 1.5 Hz, 1H), 7.28 - 6.97 (t, *J*=58 Hz, 1H), 5.80 - 5.66 (m, 1H), 5.65 - 5.53 (m, 1H), 5.13 - 5.03 (m, 2H), 4.87 (br. s., 1H), 3.22 (br. s., 2H), 2.65 (t, *J*=6.5 Hz, 2H), 1.52 - 1.37 (m, 9H).

30D. Preparation of *tert*-butyl ((*S*)-1-(4-(1-(difluoromethyl)-4-((*R*)-2-methylbut-3-enamido)-1*H*-pyrazol-5-yl)pyridin-2-yl)but-3-en-1-yl)carbamate

To a $N_2$ flushed, 3-necked, 250 mL RBF was added a solution of (*S*)-*tert*-butyl (1-(4-(4-amino-1-(difluoromethyl)-1*H*-pyrazol-5-yl)pyridin-2-yl)but-3-en-1-yl)carbamate (5 g, 13.18 mmol) and EtOAc (50 mL). The solution was cooled to -10 °C and (*R*)-2-methylbut-3-enoic acid, as prepared in Intermediate 2, (1.72 g, 17.13 mmol), pyridine (4.26 mL, 52.7 mmol), and T3P® (23.54 mL, 39.5 mmol) were added. The cooling bath was removed and the solution was allowed to warm to rt and then stir over a period of 20 h. Water (30 mL) and EtOAc (30 mL) were added and the mixture was stirred

for 30 min. The organic phase was separated and the aqueous layer was extracted with EtOAc (30 mL). The combined organic extracts were washed with brine (50 mL), dried over $Na_2SO_4$, filtered and concentrated. Purification by normal phase chromatography eluting with a gradient of hexanes/EtOAc gave *tert*-butyl ((*S*)-1-(4-(1-(difluoromethyl)-4-((*R*)-2-methylbut-3-enamido)-1*H*-pyrazol-5-yl)pyridin-2-yl)but-3-en-1-yl)carbamate (5.69 g, 12.33 mmol, 94% yield). MS(ESI) *m*/*z*: 462.2 $[M+H]^+$. $^1H$ NMR (400MHz, $CDCl_3$) δ 8.75 (dd, *J*=5.0, 0.6 Hz, 1H), 8.37 (s, 1H), 7.32 (t, *J*=59 Hz, 1H), 7.28 (br. s., 1H), 7.20 (s, 1H), 5.97 - 5.85 (m, 1H), 5.78 - 5.65 (m, 1H), 5.56 - 5.44 (m, 1H), 5.28 - 5.19 (m, 2H), 5.12 (d, *J*=2.0 Hz, 2H), 4.91 - 4.82 (m, 1H), 3.20 - 3.11 (m, 1H), 2.72 - 2.62 (m, 2H), 1.48 - 1.43 (s, 9H), 1.33 (d, *J*=6.8 Hz, 3H).

30E. Preparation of *tert*-butyl *N*-[(9*R*,10*E*,13*S*)-3-(difluoromethyl)-9-methyl-8-oxo-3,4,7,15-tetraazatricyclo[12.3.1.$0^{2,6}$]octadeca-1(18),2(6),4,10,14,16-hexaen-13-yl]carbamate

To a $N_2$ flushed, 2 L, 3-necked, RBF was added a solution of *tert*-butyl ((*S*)-1-(4-(1-(difluoromethyl)-4-((*R*)-2-methylbut-3-enamido)-1*H*-pyrazol-5-yl)pyridin-2-yl)but-3-en-1-yl)carbamate (3 g, 6.50 mmol) in EtOAc (1300 mL). The solution was sparged with Ar for 15 min. Second Generation Grubbs Catalyst (1.38 g, 1.63 mmol) was added in one portion. The reaction mixture was heated to reflux for 24 h. After cooling to rt, the solvent was removed and the residue was purified by normal phase chromatography eluting with a gradient of DCM/MeOH to yield *tert*-butyl *N*-[(9*R*,10*E*,13*S*)-3-(difluoromethyl)-9-methyl-8-oxo-3,4,7,15-tetraazatricyclo[12.3.1.$0^{2,6}$]octadeca-1(18),2(6),4,10,14,16-hexaen-13-yl]carbamate (2.13 g, 4.91 mmol, 76% yield) as a tan solid. MS(ESI) *m*/*z*: 434.4 $[M+H]^+$. $^1H$ NMR (400MHz, $CDCl_3$) δ 8.71 (d, *J*=5.1 Hz, 1H), 7.78 (s, 1H), 7.44 - 7.40 (m, 1H), 7.36 (br. s., 1H), 7.27 (t, *J*=58 Hz, 1H), 6.87 (s, 1H), 6.49 - 6.39 (m, 1H), 5.78 (s, 1H), 4.80 (br. s., 2H), 3.18 - 3.08 (m, 1H), 3.08 - 2.98 (m, 1H), 2.06 - 1.93 (m, 1H), 1.51 (s, 9H), 1.19 (d, *J*=6.6 Hz, 3H).

30F. Preparation of *tert*-butyl *N*-[(9*R*,13*S*)-3-(difluoromethyl)-9-methyl-8-oxo-3,4,7,15-tetraazatricyclo[12.3.1.$0^{2,6}$]octadeca-1(18),2(6),4,14,16-pentaen-13-yl]carbamate

Pd/C (0.60 g, 0.570 mmol) was added to a 250 mL Parr hydrogenation flask containing a solution of *tert*-butyl N-[(9*R*,10*E*,13*S*)-3-(difluoromethyl)-9-methyl-8-oxo-3,4,7,15-tetraazatricyclo[12.3.1.$0^{2,6}$]octadeca-1(18),2(6),4,10,14,16-hexaen-13-yl]carbamate (2.46 g, 5.68 mmol) in EtOH (100 mL). The flask was purged with $N_2$ and pressurized to 55 psi of $H_2$ allowed to stir for 18 h. The reaction was filtered through CELITE® and concentrated to yield *tert*-butyl N-[(9*R*,13*S*)-3-(difluoromethyl)-9-methyl-8-oxo-3,4,7,15-tetraazatricyclo[12.3.1.$0^{2,6}$]octadeca-1(18),2(6),4,14,16-pentaen-13-yl]carbamate (2.17 g, 88% yield) as a tan solid. MS(ESI) *m*/*z*: 436.3 $[M+H]^+$. $^1H$ NMR (400MHz, DMSO-$d_6$) δ 9.32 (s, 1H), 8.71 (d, *J*=5.0 Hz, 1H), 7.96 (t, *J*=58 Hz, 1H), 7.43 (s, 1H), 7.32 (d, *J*=4.8 Hz, 1H), 7.22 (d, *J*=7.3 Hz, 1H), 4.66 (d, *J*=8.3 Hz, 1H), 2.62 (br. s., 1H), 1.88 (d, *J*=12.8 Hz, 1H), 1.77 - 1.59 (m, 2H), 1.42 - 1.28 (m, 9H), 1.15 (d, *J*=18.2 Hz, 2H), 0.83 (d, *J*=7.0 Hz, 3H).

Example 30G. Preparation of (9*R*,13*S*)-13-amino-3-(difluoromethyl)-9-methyl-3,4,7,15-tetraazatricyclo[12.3.1.$0^{2,6}$]octadeca-1(18),2(6),4,14,16-pentaen-8-one

4 N HCl in dioxane (3.88 mL, 15.5 mmol) was added to a solution of *tert*-butyl *N*-[(9*R*,13*S*)-3-(difluoromethyl)-9-methyl-8-oxo-3,4,7,15-tetraazatricyclo[12.3.1.$0^{2,6}$] octadeca-1(18),2(6),4,14,16-pentaen-13-yl]carbamate (2.25 g, 5.2 mmol) in MeOH (10 mL). The reaction was allowed to stir at rt for 2 h. The reaction was cooled in an ice bath, and 7 N $NH_3$ in MeOH (13.3 mL, 93.0 mmol) was added. After 5 min, the reaction was diluted with $CH_2Cl_2$ (80 mL) and the solid that formed was filtered. The filtrate was concentrated to yield (9*R*,13*S*)-13-amino-3-(difluoromethyl)-9-methyl-3,4,7,15-tetraazatricyclo[12.3.1.$0^{2,6}$]octadeca-1(18),2(6),4,14,16-pentaen-8-one (1.3 g, 3.88 mmol, 75% yield). MS(ESI) *m*/*z*: 336.3 $[M+H]^+$. $^1H$ NMR (400MHz, DMSO-$d_6$) δ 9.33 (s, 1H), 8.71 (d, *J*=5.0 Hz, 1H), 7.94 (t, *J*=58 Hz, 1H), 7.85 (s, 1H), 7.40 (s, 1H), 7.32 (d, *J*=5.0 Hz, 1H), 4.01 (dd, *J*=10.2, 5.1 Hz, 1H), 2.63 - 2.53 (m, 1H), 1.90 - 1.69 (m, 2H), 1.53 - 1.36 (m, 2H), 1.16 - 1.00 (m, 1H), 0.85 (d, *J*=7.0 Hz, 3H).

Intermediate 31

Preparation of (9*R*,13*S*)-13-amino-3,9-dimethyl-3,4,7-triazatricyclo[12.3.1.$0^{2,6}$] octadeca-1(18),2(6),4,14,16-pentaen-8-one, hydrochloride

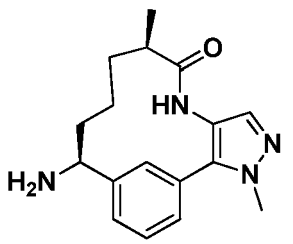

31A. Preparation of *tert*-butyl *N*-[(1*S*)-1-[3-(1-methyl-4-nitro-1*H*-pyrazol-5-yl)phenyl]but-3-en-1-yl]carbamate

To *tert*-butyl *N*-[(1*S*)-1-(3-bromophenyl)but-3-en-1-yl]carbamate (2 g, 6.13 mmol), 1-methyl-4-nitro-1*H*-pyrazole (0.779 g, 6.13 mmol), di(adamantan-1-yl)(butyl) phosphine (0.659 g, 1.839 mmol), pivalic acid (0.213 ml, 1.839 mmol), $K_2CO_3$ (2.54 g, 18.39 mmol) was added DMF (9 ml). The mixture was purged with Ar for 10 min and $Pd(OAc)_2$ (0.275 g, 1.226 mmol) was added. The reaction was heated at 120 °C for 15 h. The reaction was partitioned between water (50 ml) and EtOAc (50 ml) and solution was filtered through paper and the layers were separated. The aqueous layer was extracted with EtOAc (2 x 50 ml). The combined organic layers were washed with brine (50 ml), dried over $MgSO_4$, filtered and concentrated. The residue was purified by normal phase chromatography using hexanes and EtOAc as eluents to afford (*S*)-*tert*-butyl (1-(3-(1-methyl-4-nitro-1*H*-pyrazol-5-yl)phenyl)but-3-en-1-yl)carbamate (1.186 g, 3.18 mmol, 51.9% yield) as a yellow oil. MS(ESI) *m/z*: 371.1 $(M-H)^+$.

31B. Preparation of *tert*-butyl *N*-[(1S)-1-[3-(4-amino-1-methyl-1H-pyrazol-5-yl)phenyl] but-3-en-1-yl]carbamate

To *tert*-butyl *N*-[(1S)-1-[3-(1-methyl-4-nitro-1*H*-pyrazol-5-yl)phenyl]but-3-en-1-yl]carbamate (0.097 g, 0.260 mmol) in acetone (5ml) / water (1 ml), cooled to 0 °C, was added $NH_4Cl$ (0.070 g, 1.302 mmol) and Zn (0.170 g, 2.60 mmol). The ice bath was removed. After 3 h, the reaction was filtered and the filtrate was partitioned between water (10 ml) and EtOAc (30 ml). The aqueous layer was extracted with EtOAc (2 x 20 ml). The combined organic layers were washed with brine (10 ml), dried over $MgSO_4$, filtered and concentrated. The residue was purified by normal phase chromatography using DCM and 0-10% MeOH as eluents to afford *tert*-butyl *N*-[(1S)-1-[3-(4-amino-1-methyl-1H-pyrazol-5-yl)phenyl]but-3-en-1-yl]carbamate (76.6 mg, 86%). MS(ESI) *m/z*: 343.2 $(M+H)^+$.

31C. Preparation of *tert*-butyl *N*-[(1*S*)-1-(3-{1-methyl-4-[(2*R*)-2-methylbut-3-enamido]-1*H*-pyrazol-5-yl}phenyl)but-3-en-1-yl]carbamate

To *tert*-butyl *N*-[(1*S*)-1-[3-(4-amino-1-methyl-1*H*-pyrazol-5-yl)phenyl]but-3-en-1-yl]carbamate (0.076 g, 0.222 mmol) in EtOAc (0.58 ml) was added (R)-2-methylbut-3-enoic acid (0.027 g, 0.266 mmol), prepared as described in Intermediate 2, in 0.3 mL EtOAc. The mixture was cooled to 0 °C and Hunig's Base (0.116 ml, 0.666 mmol) followed by a solution of 50% T3P® in EtOAc (0.264 ml, 0.444 mmol) were added. After 3 h, the reaction was partitioned with sat $NaHCO_3$ (5 ml) and EtOAc (5 ml). The aqueous layer was extracted with EtOAc (2 x 10 ml). The combined organic layers were washed with brine (5 ml), dried over $MgSO_4$, filtered and concentrated. The residue was purified by normal phase chromatography using hexanes and EtOAc as eluents to afford *tert-butyl N*-[(1S)-1-(3-{1-methyl-4-[(2*R*)-2-methylbut-3-enamido]-1*H*-pyrazol-5-yl}phenyl)but-3-en-1-yl]carbamate (69 mg, 73%) as a yellow oil. MS(ESI) *m/z*: 425.2 $(M+H)^+$. $^1H$ NMR (400MHz, $CDCl_3$) δ 8.04 (s, 1H), 7.52 - 7.45 (m, 1H), 7.37 (d, *J*=7.9 Hz, 1H), 7.26 - 7.18 (m, 2H), 7.05 (br. s., 1H), 5.96 - 5.85 (m, 1H), 5.69 (ddt, *J*=17.0, 10.1, 7.0 Hz, 1H), 5.21 - 5.09 (m, 4H), 4.95 (br. s., 1H), 4.77 (br. s., 1H), 3.76 (s, 3H), 3.07 (quin, *J*=7.2 Hz, 1H), 2.61 - 2.48 (m, 2H), 1.45 - 1.38 (m, 9H), 1.30 (d, *J*=7.0 Hz, 3H).

31D. Preparation of *tert*-butyl *N*-[(9R,10E,13S)-3,9-dimethyl-8-oxo-3,4,7-triazatricyclo [12.3.1.$0^{2,6}$]octadeca-1(18),2(6),4,10,14,16-hexaen-13-yl]carbamate

A solution of *tert*-butyl *N*-[(1*S*)-1-(3-{1-methyl-4-[(2*R*)-2-methylbut-3-enamido]-1*H*-pyrazol-5-yl}phenyl)but-3-en-1-yl]carbamate (0.069 g, 0.163 mmol) in degassed DCE (10 ml) was heated to 120 °C for 30 min in a microwave in the presence of Second Generation Grubbs Catalyst (0.055 g, 0.065 mmol). The reaction mixture was directly purified by normal phase chromatography twice using hexanes and EtOAc as eluents to afford desired *tert*-butyl *N*-[(9R,10E,13S)-3,9-dimethyl-8-oxo-3,4,7-triazatricyclo [12.3.1.$0^{2,6}$]octadeca-1(18),2(6),4,10,14,16-hexaen-13-yl]carbamate (33 mg, 51.2%) as a dark solid. MS(ESI) *m/z*: 397.1 $(M+H)^+$. $^1H$ NMR (400MHz, $CDCl_3$) δ 7.61 - 7.52 (m, 1H), 7.46 - 7.40 (m, 1H), 7.33 - 7.25 (m, 1H), 7.20 (d, *J*=7.5 Hz, 1H), 6.93 (br. s., 1H), 6.83 (s, 1H), 5.63 (ddd, *J*=15.1, 9.4, 5.6 Hz, 1H), 5.18 (br. s., 1H), 4.89 (dd, *J*=15.2, 8.8 Hz, 1H), 4.69 (br. s., 1H), 3.93 - 3.86 (m, 3H), 3.09 - 2.99 (m, 1H), 2.69 - 2.58 (m, 1H), 2.17 - 2.08 (m, 1H), 1.53 - 1.32 (m, 9H), 1.18 (d, *J*=6.8 Hz, 3H).

31E. Preparation of *tert*-butyl *N*-[(9*R*,13*S*)-3,9-dimethyl-8-oxo-3,4,7-triazatricyclo [12.3.1.0^2,6^]octadeca-1(18),2(6),4,14,16-pentaen-13-yl]carbamate

A solution of *tert*-butyl N-[(9*R*,10E,13S)-3,9-dimethyl-8-oxo-3,4,7-triazatricyclo [12.3.1.0$^{2,6}$]octadeca-1(18),2(6),4,10,14,16-hexaen-13-yl]carbamate (0.089 g, 0.224 mmol) in EtOH (5 ml) was hydrogenated under a $H_2$ atmosphere at 55 psi for 3 h. The reaction mixture was filtered through small plug of CELITE® and rinsed with EtOH/MeOH/DCM to give *tert*-butyl N-[(9*R*,13*S*)-3,9-dimethyl-8-oxo-3,4,7-triazatricyclo[12.3.1.0$^{2,6}$]octadeca-1(18),2(6),4,14,16-pentaen-13-yl]carbamate (89 mg, 99%) as a white solid. MS(ESI) *m*/*z*: 399.4 (M+H)$^+$. $^1$H NMR (400MHz $CDCl_3$) δ 7.53 - 7.43 (m, 2H), 7.43 - 7.36 (m, 1H), 7.29 (s, 1H), 6.44 (s, 1H), 4.90 (br. s., 1H), 4.68 (br. s., 1H), 3.98 (s, 3H), 2.44 (br. s., 1H), 1.93 (d, *J*=7.7 Hz, 1H), 1.85 - 1.63 (m, 2H), 1.42 (br. s., 9H), 1.28 - 1.19 (m, 2H), 1.07 (d, *J*=6.8 Hz, 3H), 0.96 (br. s., 1H).

31F. Preparation of (9*R*,13,*S*)-13-amino-3,9-dimethyl-3,4,7-triazatricyclo[12.3.1.0$^{2,6}$] octadeca-1(18),2(6),4,14,16-pentaen-8-one hydrochloride

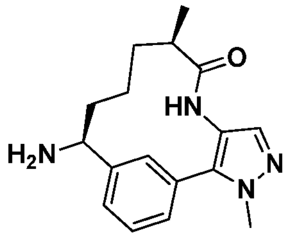

*tert*-Butyl N-[(9*R*,13S)-3,9-dimethyl-8-oxo-3,4,7-triazatricyclo[12.3.1.0$^{2,6}$] octadeca-1(18),2(6),4,14,16-pentaen-13-yl]carbamate (88 mg, 0.221 mmol) was deprotected with 4 N HCl in dioxane (3 ml) for 5 h. The reaction was concentrated to afford (70 mg, 95%) of (9*R*,13*S*)-13-amino-3,9-dimethyl-3,4,7-triazatricyclo[12.3.1.0$^{2,6}$] octadeca-1(18),2(6),4,14,16-pentaen-8-one hydrochloride as a dark solid. MS(ESI) *m*/*z*: 299.08 (M+H)$^+$. $^1$H NMR (500MHz, $CD_3OD$) δ 7.81 (s, 1H), 7.77 - 7.70 (m, 1H), 7.70 - 7.58 (m, 3H), 4.46 (dd, *J*=12.0, 4.5 Hz, 1H), 4.19 - 4.07 (m, 3H), 3.45 - 3.26 (m, 1H), 2.75 - 2.59 (m, 1H), 2.21 - 2.09 (m, 1H), 1.99 - 1.86 (m, 2H), 1.58 (td, *J*=14.3, 8.3 Hz, 1H), 1.29 - 1.17 (m, 1H), 1.03 (d, *J*=6.9 Hz, 3H), 0.94 - 0.82 (m, 1H).

Intermediate 32

Preparation of (9*R*,13*S*)-13-amino-3,9-dimethyl-3,4,7,15-tetraazatricyclo[12.3.1.0$^{2,6}$] octadeca-1(18),2(6),4,14,16-pentaen-8-one

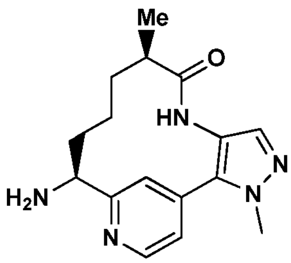

32A. Preparation of 1-methyl-4-nitro-1*H*-pyrazole

To a solution of 4-nitro-1*H*-pyrazole (2.5 g, 22.11 mmol) in THF (50 mL) was added NaH (0.973 g, 24.32 mmol) and the mixture was stirred at rt for 5 min. To this suspension was then added $CH_3I$ (1.382 mL, 22.11 mmol) and stirred at rt overnight. The reaction mixture was then diluted with EtOAc (2 x 25 mL) and washed with brine (25 mL). The organic layer was concentrated, followed by purification using normal phase chromatography to yield 1-methyl-4-nitro-1*H*-pyrazole as white solid (1.9 g, 80% yield). $^1$H NMR (400 MHz, $CDCl_3$) δ ppm 8.12 (s, 1H), 8.06 (s, 1H), 3.97 (s, 3H).

32B. Preparation of (*S*)-*tert*-butyl (1-(4-(1-methyl-4-nitro-1*H*-pyrazol-5-yl)pyridin-2-yl)but-3-en-1-yl)carbamate

To a $N_2$ flushed pressure vial was added (*S*)-*tert*-butyl (1-(4-chloropyridin-2-yl)but-3-en-1-yl)carbamate, prepared as described in Intermediate 23, (3.0 g, 10.61 mmol), 1-methyl-4-nitro-1*H*-pyrazole (1.348 g, 10.61 mmol), di(adamant-1-yl)(butyl) phosphine (1.141 g, 3.18 mmol), PvOH (0.369 ml, 3.18 mmol), $K_2CO_3$ (4.40 g, 31.8 mmol) and DMF (21 mL). The reaction mixture was purged with $N_2$ for 5 min and $Pd(OAc)_2$ (0.476 g, 2.122 mmol) was added. The reaction mixture was purged with $N_2$. The vial was sealed and heated at 120 °C for 4 h. The reaction mixture was cooled to rt and partitioned between 10% aqueous LiCl (15 mL) and EtOAc (30 mL). The aqueous layer was extracted with EtOAc (2 x 20 mL) and the combined organic layers were washed with brine (15 mL), dried over $MgSO_4$, filtered and concentrated. The crude product was then purified using normal phase chromatography to yield (*S*)-*tert*-butyl (1-(4-(1-methyl-4-nitro-1*H*-pyrazol-5-yl)pyridin-2-yl)but-3-en-1-yl)carbamate (1.2 g, 29% yield) as a brown oil. MS(ESI) *m*/*z*: 374.4 $(M+H)^+$.

32C. Preparation of (*S*)-*tert*-butyl (1-(4-(4-amino-1-methyl-1*H*-pyrazol-5-yl)pyridin-2-yl)but-3-en-1-yl)carbamate

A solution of (*S*)-*tert*-butyl (1-(4-(1-methyl-4-nitro-1*H*-pyrazol-5-yl)pyridin-2-yl)but-3-en-1-yl)carbamate (1.2 g, 3.21 mmol) in MeOH (10 mL) and AcOH (1 mL) was heated to 40 °C. To the above clear solution was then slowly added Zn (0.420 g, 6.43 mmol) in 3 portions (50:25:25%) and stirred at 40 °C for 5 min. The reaction mixture was monitored by LCMS and once complete, the solution was cooled to rt, and $K_2CO_3$ and 1 mL water were added. The reaction mixture was stirred for 5 min, then filtered through a pad of CELITE® and concentrated to yield the crude product. The crude product was partitioned between EtOAc (30 mL) and sat $NaHCO_3$ (15 mL). The organic layers were separated and dried over $MgSO_4$. The crude product was purified using normal phase chromatography to yield (*S*)-*tert*-butyl (1-(4-(4-amino-1-methyl-1*H*-pyrazol-5-yl)pyridin-2-yl)but-3-en-1-yl)carbamate (0.88 g, 76% yield) as pale brown oil. MS(ESI) *m*/*z*: 344.4 $(M+H)^+$.

32D. Preparation of *tert*-butyl ((*S*)-1-(4-(1-methyl-4-((*R*)-2-methylbut-3-enamido)-1*H*-pyrazol-5-yl)pyridin-2-yl)but-3-en-1-yl)carbamate

To a $N_2$ flushed, 3-necked, 250 mL RBF was added a solution of (*S*)-*tert*-butyl (1-(4-(4-amino-1-methyl-1*H*-pyrazol-5-yl)pyridin-2-yl)but-3-en-1-yl)carbamate (620 mg, 1.805 mmol) and EtOAc (15 mL). The solution was cooled to -10 °C and (R)-2-methylbut-3-enoic acid, as prepared in Intermediate 2, (271 mg, 2.71 mmol), pyridine (0.437 mL, 5.42 mmol) and T3P® (2.149 mL, 3.61 mmol) were added. The cooling bath was removed and the solution was allowed to warm to rt and then stir over a period of 20 h. Water (15 mL) and EtOAc (15 mL) were added and the mixture was stirred for 30 min. The organic phase was separated and the aqueous layer was extracted with EtOAc (15 mL). The combined organic extracts were washed with brine (15 mL), dried over $Na_2SO_4$, filtered and concentrated. Purification by normal phase chromatography eluting with a gradient of hexanes/EtOAc gave *tert*-butyl ((*S*)-1-(4-(1-methyl-4-((*R*)-2-methylbut-3-enamido)-1*H*-pyrazol-5-yl)pyridin-2-yl)but-3-en-1-yl)carbamate (0.26 g, 34% yield). MS(ESI) *m*/*z*: 426.5 $[M+H]^+$.

32E. Preparation of *tert*-butyl N-[(9*R*,10*E*,13*S*)-3,9-dimethyl-8-oxo-3,4,7,15-tetraazatricyclo[12.3.1.$0^{2,6}$]octadeca-1(18),2(6),4,10,14,16-hexaen-13-yl]carbamate

To a $N_2$ flushed, 250 mL, 3-necked RBF was added a solution of *tert*-butyl ((*S*)-1-(4-(1-methyl-4-((*R*)-2-methylbut-3-enamido)-1*H*-pyrazol-5-yl)pyridin-2-yl)but-3-en-1-yl)carbamate (266 mg, 0.625 mmol) in DCE (18 mL). The solution was sparged with Ar for 15 min. Second Generation Grubbs Catalyst (213 mg, 0.250 mmol) was added in one portion. The reaction mixture was heated to 120 °C in microwave for 30 min. After cooling to rt, the solvent was removed and the residue was purified by normal phase chromatography eluting with a gradient of DCM/MeOH to yield *tert*-butyl N-[(9*R*,10*E*,13*S*)-3,9-dimethyl-8-oxo-3,4,7,15-tetraazatricyclo[12.3.1.$0^{2,6}$]octadeca-1(18),2(6),4,10,14,16-hexaen-13-yl]carbamate (60 mg, 23% yield) as a tan solid. MS(ESI) *m*/*z*: 398.4 $[M+H]^+$.

32F. Preparation of *tert*-butyl N-[(9*R*,13*S*)-3,9-dimethyl-8-oxo-3,4,7,15-tetraazatricyclo [12.3.1.$0^{2,6}$]octadeca-1(18),2(6),4,14,16-pentaen-13-yl]carbamate

Pd/C (0.016 g, 0.015 mmol) was added to a 100 mL Parr hydrogenation flask containing a solution of *tert*-butyl N-[(9*R*,10*E*,13*S*)-3,9-dimethyl-8-oxo-3,4,7,15-tetraazatricyclo[12.3.1.$0^{2,6}$]octadeca-1(18),2(6),4,10,14,16-hexaen-13-yl]carbamate (60 mg, 0.151 mmol) in EtOH (6 mL). The flask was purged with $N_2$ and pressurized to 55 psi of $H_2$ and allowed to stir for 5 h. The reaction was filtered through a pad of CELITE® and concentrated to yield *tert*-butyl N-[(9*R*,13*S*)-3,9-dimethyl-8-oxo-3,4,7,15-tetraazatricyclo[12.3.1.$0^{2,6}$]octadeca-1(18),2(6),4,14,16-pentaen-13-yl]carbamate (48 mg, 76% yield) as a tan solid. MS(ESI) *m*/*z*: 400.5 $[M+H]^+$.

32G. Preparation of (9*R*,13*S*)-13-amino-3,9-dimethyl-3,4,7,15-tetraazatricyclo [$12.3.1.0^{2,6}$]octadeca-1(18),2(6),4,14,16-pentaen-8-one

To a solution of *tert*-butyl N-[(9*R*,13*S*)-3,9-dimethyl-8-oxo-3,4,7,15-tetraazatricyclo[$12.3.1.0^{2,6}$]octadeca-1(18),2(6),4,14,16-pentaen-13-yl]carbamate (48 mg, 0.120 mmol) in DCM (2.5 mL) was added TFA (0.6 mL, 7.79 mmol) and the reaction was stirred at rt for 1.5 h. The reaction mixture was then concentrated to give (9*R*,13*S*)-13-amino-3,9-dimethyl-3,4,7,15-tetraazatricyclo[$12.3.1.0^{2,6}$]octadeca-1(18),2(6),4,14,16-pentaen-8-one bis trifluoroacetate (63 mg, 94% yield) as a brown solid which was then dissolved in MeOH (1 mL) to give a clear, brown solution. The solution was added to a pre-rinsed AGILENT® StratoSpheres SPE PL-$HCO_3$ MP Resin cartridge. Gravity filtration, eluting with MeOH, gave a clear, slightly yellow filtrate. Concentration provided (9*R*,13*S*)-13-amino-3,9-dimethyl-3,4,7,15-tetraazatricyclo[$12.3.1.0^{2,6}$]octadeca-1(18),2(6),4,14,16-pentaen-8-one (25 mg, 93%) as a pale yellow solid. MS(ESI) *m/z:* 300.4 $[M+H]^+$.

Intermediate 33

Preparation of (9*R*,13*S*)-13-amino-3-($^2H_3$)methyl-9-methyl-3,4,7,15-tetraazatricyclo [$12.3.1.0^{2,6}$]octadeca-1(18),2(6),4,14,16-pentaen-8-one

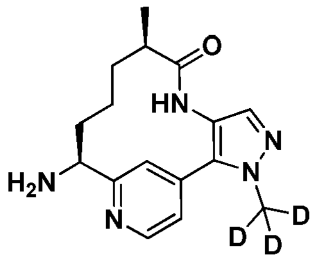

33A. Preparation of 1-($^2H_3$)methyl-4-nitro-1H-pyrazole

DIAD (5.59 mL, 28.7 mmol) was added to a solution of 4-nitro-1H-pyrazole (2.5 g, 22.11 mmol), $CD_3OD$ (0.898 mL, 22.11 mmol), and $Ph_3P$ (resin bound) (8.84 g, 26.5 mmol) in THF (40 ml) and stirred overnight. The reaction was quenched with water, extracted with EtOAc, washed with brine, dried over $Na_2SO_4$, filtered, and concentrated. The crude product was purified by normal phase chromatography eluting with a gradient of DCM/MeOH to afford 1-($^2H_3$)methyl-4-nitro-1H-pyrazole (1.92 g, 14.76 mmol, 66.7% yield) as a white solid. MS(ESI) *m/z*: 131.0 $(M+H)^+$. $^1H$ NMR (400MHz, $CDCl_3$) δ 8.13 (d, J=0.4 Hz, 1H), 8.05 (s, 1H).

33B. Preparation of *tert*-butyl *N*-[(1*S*)-1-{4-[1-(2H3)methyl-4-nitro-1H-pyrazol-5-yl]pyridin-2-yl}but-3-en-1-yl]carbamate

To a large microwave vial were added (S)-*tert*-butyl (1-(4-chloropyridin-2-yl)but-3-en-1-yl)carbamate (2.61 g, 9.22 mmol), 1-($^2H_3$)methyl-4-nitro-1H-pyrazole (1.0 g, 7.69 mmol), di(adamantan-1-yl)(butyl)phosphine (0.413 g, 1.15 mmol), $K_2CO_3$ (3.19 g, 23.06 mmol), pivalic acid (0.268 ml, 2.306 mmol) and DMF (15.37 ml). The reaction was purged with Ar for 10 min, $Pd(OAc)_2$ (0.173 g, 0.769 mmol) was added, the vial sealed, and stirred at 115 °C overnight. The reaction was then partitioned between EtOAc and $H_2O$. The aqueous layer was extracted with EtOAc (2x). The combined organic layer was washed with brine, dried over $MgSO_4$, filtered and concentrated. The residue was purified by normal phase chromatography eluting with a gradient of hexanes/EtOAc to give *tert*-butyl *N*-[(1*S*)-1-{4-[1-($^2H_3$)methyl-4-nitro-1H-pyrazol-5-yl]pyridin-2-yl}but-3-en-1-yl]carbamate (1.49 g, 3.96 mmol, 51.5% yield) as a lavender foam. MS(ESI) *m/z:* 377.0 $(M+H)^+$. $^1H$ NMR (400MHz, $CDCl_3$) δ 8.77 (d, J=4.8 Hz, 1H), 8.21 (s, 1H), 7.26 (s, 1H), 7.23 (dd, J=5.1, 1.5 Hz, 1H), 5.78 - 5.65 (m, 1H), 5.55 (d, J=6.8 Hz, 1H), 5.14 - 5.03 (m, 2H), 4.89 (d, J=6.8 Hz, 1H), 2.66 (t, J=6.6 Hz, 2H), 1.44 (s, 9H).

33C. Preparation of *tert*-butyl *N*-[(1*S*)-1-1{4-[4-amino-1-($^2H_3$)methyl-1H-pyrazol-5-yl]pyridin-2-yl}but-3-en-1-yl] carbamate

*tert*-Butyl N-[(1S)-1-{4-[1-($^2H_3$)methyl-4-nitro-1H-pyrazol-5-yl]pyridin-2-yl}but-3-en-1-yl]carbamate (1.45 g, 3.85 mmol) was dissolved in acetone (15ml)/ water (3 ml), cooled to 0 °C. $NH_4Cl$ (1.030 g, 19.26 mmol) and Zn (2.52

g, 38.5 mmol) were added and the ice bath was removed. After 1 h, the reaction was filtered and filtrate partitioned with water (30 ml) and EtOAc (50 ml). The aqueous layer was extracted with EtOAc (2 x 50 ml). The combined organic layers were washed with brine (20 ml), dried over $MgSO_4$, filtered, and concentrated. The residue was purified by normal phase eluting with a gradient of DCM/MeOH chromatography to afford tert-butyl *N*-[(1*S*)-1-{4-[4-amino-1-($^2H_3$)methyl-1H-pyrazol-5-yl]pyridin-2-yl}but-3-en-1-yl]carbamate (0.62 g, 46.5%). MS(ESI) *m/z*: 347.2 (M+H)$^+$. $^1$H NMR (400MHz, $CDCl_3$) δ 8.67 (dd, J=5.1, 0.7 Hz, 1H), 7.26 - 7.23 (m, 2H), 7.21 (dd, J=5.1, 1.5 Hz, 1H), 5.79 - 5.66 (m, 1H), 5.58 (d, J=7.3 Hz, 1H), 5.11 - 5.05 (m, 2H), 4.86 (q, J=6.6 Hz, 1H), 2.64 (t, J=6.7 Hz, 2H), 1.44 (s, 9H).

33D. Preparation of *tert*-butyl N-[(1S)-1-{4-[1-($^2H_3$)methyl-4-[(2R)-2-methylbut-3-enamido]-1H-pyrazol-5-yl]pyridin-2-yl}but-3-en-1-yl]carbamate

(R)-2-Methylbut-3-enoic acid (233 mg, 2.327 mmol), *tert*-butyl N-[(1S)-1-{4-[4-amino-1-($^2H_3$)methyl-1H-pyrazol-5-yl]pyridin-2-yl}but-3-en-1-yl]carbamate (620 mg, 1.79 mmol), pyridine (0.433 ml, 5.37 mmol) in EtOAc (17.900 ml) was cooled to -10 °C under Ar. T3P® (50%wt in EtOAc) (2.13 ml, 3.58 mmol) was added dropwise and then the reaction mixture was gradually warmed up to rt. After 3.5 h, the reaction mixture was diluted with EtOAc, washed with 1.5 M $K_2HPO_4$ followed by brine, dried over $Na_2SO_4$, filtered, and concentrated. The crude product was then purified by normal phase chromatography eluting with a gradient of hexanes/EtOAc to give *tert*-butyl N-[(1S)-1-{4-[1-($^2H_3$)methyl-4-[(2R)-2-methylbut-3-enamido]-1H-pyrazol-5-yl]pyridin-2-yl}but-3-en-1-yl]carbamate (529 mg, 1.234 mmol, 69.0% yield) as a yellow foam. MS(ESI) *m/z*: 429.2 (M+H)$^+$.

33E. Preparation of *tert*-butyl N-[(9R,10E,13S)-3-($^2H_3$)methyl-9-methyl-8-oxo-3,4,7,15-tetraazatricyclo[12.3.1.0$^{2,6}$]octadeca-1(18),2(6),4,10,14,16-hexaen-13-yl]carbamate

Five large microwave vials were charged in equal amounts with the following: *tert*-butyl N-[(1S)-1-{4-[1-($^2H_3$)methyl-4-[(2R)-2-methylbut-3-enamido]-1H-pyrazol-5-yl]pyridin-2-yl}but-3-en-1-yl]carbamate (0.51 g, 1.190 mmol) in degassed DCE (90 ml) was irradiated at 120 °C for 30 min in the presence of Second Generation Grubbs Catalyst (0.404 g, 0.476 mmol). The reactions were combined, concentrated, and the residue purified by normal phase column chromatography eluting with a gradient of hexanes/EtOAc to give *tert*-butyl N-[(9R,10E,13S)-3-($^2H_3$)methyl-9-methyl-8-oxo-3,4,7,15-tetraazatricyclo[12.3.1.0$^{2,6}$]octadeca-1(18),2(6),4,10,14,16-hexaen-13-yl]carbamate (0.124 g, 26.0%) as a brown solid. MS(ESI) *m/z*: 401.2 (M+H)$^+$. $^1$H NMR (400MHz, $CDCl_3$) δ 8.66 (d, J=5.1 Hz, 1H), 7.52 (s, 1H), 7.19 (d, J=4.8 Hz, 1H), 6.80 (s, 1H), 6.37 (d, J=7.5 Hz, 1H), 5.68 (t, J=11.2 Hz, 1H), 4.82 - 4.63 (m, 2H), 3.12 - 2.93 (m, 2H), 1.93 (q, J=11.1 Hz, 1H), 1.48 (s, 9H), 1.15 (d, J=5.9 Hz, 3H).

33F. Preparation of *tert*-butyl N-[(9R,13S)-3-($^2H_3$)methyl-9-methyl-8-oxo-3,4,7,15-tetraazatricyclo[12.3.1.0$^{2,6}$]octadeca-1(18),2(6),4,14,16-pentaen-13-yl]carbamate

$PtO_2$ (6.80 mg, 0.030 mmol) was added to a stirring solution of *tert*-butyl N-[(9R,10E,13 S)-3-($^2H_3$)methyl-9-methyl-8-oxo-3,4,7,15-tetraazatricyclo[12.3.1.0$^{2,6}$] octadeca-1(18),2(6),4,10,14,16-hexaen-13-yl]carbamate (0.120 g, 0.300 mmol) in EtOH (10 ml). The suspension was subjected to a $H_2$ atmosphere (55 psi) for 1 h. The catalyst was filtered off through a plug of CELITE® and the filtrate concentrated to give *tert*-butyl N-[(9R,13S)-3-($^2H_3$)methyl-9-methyl-8-oxo-3,4,7,15-tetraazatricyclo[12.3.1.0$^{2,6}$] octadeca-1(18),2(6),4,14,16-pentaen-13-yl]carbamate (0.104 g, 86%). MS(ESI) *m/z*: 403.2 (M+H)$^+$.

33G. Preparation of (9R,13S)-13-amino-3-($^2H_3$)methyl-9-methyl-3,4,7,15-tetraazatricyclo[12.3.1.0$^{2,6}$]octadeca-1(18),2(6),4,14,16-pentaen-8-one

4 M HCl in dioxane (1.62 ml) was added to a stirring solution of *tert*-butyl N-[(9R,13 S)-3-($^2H_3$)methyl-9-methyl-8-oxo-3,4,7,15-tetraazatricyclo[12.3.1.0$^{2,6}$]octadeca-1(18),2(6),4,14,16-pentaen-13-yl]carbamate (0.100 g, 0.248 mmol) in MeOH (3 ml) and stirred overnight. The reaction mixture was concentrated to dryness and placed under high vacuum. The hydrochloride salt was free based by dissolution in MeOH, passed through a resin bound $NaHCO_3$ cartridge (StratoSpheres SPE; 500 mg, 0.90 mmol loading) and filtrate concentrated to give (9R,13S)-13-amino-3-($^2H_3$)methyl-9-methyl-3,4,7,15-tetraazatricyclo[12.3.1.0$^{2,6}$]octadeca-1(18),2(6),4,14,16-pentaen-8-one. MS(ESI) *m/z*: 303.4 (M+H)$^+$.

Intermediate 34

Preparation of (9R,13S)-13-amino-3-($^2H_3$)methyl-9-methyl-3,4,7,17-tetraazatricyclo [12.3.1.0$^{2,6}$]octadeca-1(18),2(6),4,14,16-pentaen-8-one

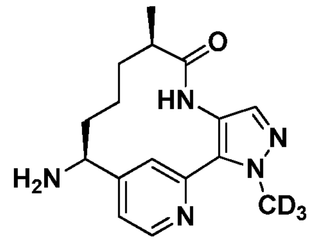

(9R,13S)-13-Amino-3-($^2H_3$)methyl-9-methyl-3,4,7,17-tetraazatricyclo[12.3.1.0$^{2,6}$] octadeca-1(18),2(6),4,14,16-pentaen-8-one was prepared in a similar manner as (9R,13S)-13-amino-3-c($^2H_3$)methyl-9-methyl-3,4,7,15-tetraazatricyclo[12.3.1.0$^{2,6}$] octadeca-1(18),2(6),4,14,16-pentaen-8-one, as described in Intermediate 33, replacing (S)-*tert*-butyl (1-(4-chloropyridin-2-yl)but-3-en-1-yl)carbamate, described in Intermediate 23, with (S)-*tert*-butyl (1-(2-bromopyridin-4-yl)but-3-en-1-yl)carbamate, described in Intermediate 27. MS(ESI) *m/z*: 303.3 $(M+H)^+$. $^1H$ NMR (400MHz, $CD_3OD$) δ 8.70 (d, J=5.3 Hz, 1H), 7.58 (s, 1H), 7.50 - 7.42 (m, 2H), 4.14 - 4.05 (m, 1H), 2.72 (td, J=6.7, 3.5 Hz, 1H), 2.06 - 1.94 (m, 2H), 1.65 - 1.50 (m, 2H), 1.41 - 1.26 (m, 1H), 1.02 (d, J=6.8 Hz, 3H), 0.70 - 0.53 (m, 1H).

Intermediate 35

Preparation of (9*R*,13*S*)-13-amino-3-(difluoromethyl)-9-methyl-3,4,7-triazatricyclo [12.3.1.0$^{2,6}$]octadeca-1(18),2(6),4,14,16-pentaen-8-one

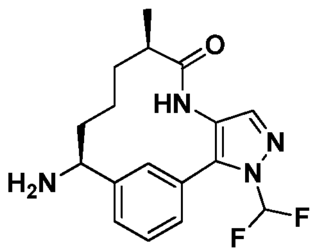

35A. Preparation of *tert*-butyl N-[(1*S*)-1-{3-[1-(difluoromethyl)-4-nitro-1H-pyrazol-5-yl]phenyl}but-3-en-1-yl]carbamate

To a solution of (*S*)-*tert*-butyl (1-(3-bromophenyl)but-3-en-1-yl)carbamate (4.0 g, 12.29 mmol), prepared as described in Intermediate 24, in DMF (40.9 ml), was added 1-(difluoromethyl)-4-nitro-1H-pyrazole (2.20 g, 13.49 mmol), di(adamantan-1-yl)(butyl) phosphine (0.659 g, 1.839 mmol), $K_2CO_3$ (5.08 g, 36.8 mmol) and pivalic acid (0.427 ml, 3.68 mmol). The resulting solution was purged with Ar for 10 min. $Pd(OAc)_2$ (0.275 g, 1.226 mmol) was added and the reaction mixture was stirred at 115 °C for 4 h. The reaction was cooled to rt, quenched with water (50 mL) and extracted with EtOAc (3 x 50 mL). The combined organic layers were washed with brine (50 mL), dried ($MgSO_4$), filtered, and concentrated. The residue was purified by normal phase column chromatography eluting with a gradient of heptane/EtOAc to give *tert*-butyl N-[(1*S*)-1-{3-[1-(difluoromethyl)-4-nitro-1H-pyrazol-5-yl]phenyl}but-3-en-1-yl]carbamate (4.0 g, 80.0%). MS(ESI) *m/z*: 407 $(M-H)^-$.

35B. Preparation of *tert*-butyl N-[(1*S*)-1-{3-[4-amino-1-(difluoromethyl)-1H-pyrazol-5-yl]phenyl}but-3-en-1-yl]carbamate

*tert*-Butyl N-[(1*S*)-1-{3-[1-(difluoromethyl)-4-nitro-1H-pyrazol-5-yl]phenyl}but-3-en-1-yl]carbamate (4.0 g, 9.79 mmol) was dissolved in acetone (100 ml)/$H_2O$ (24 ml) and then cooled to 0 °C. To the solution was added $NH_4Cl$ (2.62 g, 49.0 mmol) and Zn (6.40 g, 98 mmol) and the ice bath was removed. After 2 h, the reaction mixture was filtered and filtrate partitioned between water (30 ml) and EtOAc (50 ml). The aqueous layer was extracted with EtOAc (2 x 50 ml).

The combined organic phase was washed with brine (20 ml), dried ($MgSO_4$),filtered, and concentrated. The residue was purified by normal phase chromatography eluting with a gradient of DCM/MeOH to give of *tert*-butyl N-[(1*S*)-1-{3-[4-amino-1-(difluoromethyl)-1H-pyrazol-5-yl]phenyl}but-3-en-1-yl]carbamate (3.33 g, 8.80 mmol, 90%) as a yellow oil. MS(ESI) *m*/*z*: 379.2 $(M+H)^+$.

35C. Preparation of *tert*-butyl N-[(1*S*)-1-{3-[1-(difluoromethyl)-4-[(2R)-2-methylbut-3-enamido]-1H-pyrazol-5-yl]phenyl}but-3-en-1-yl]carbamate

To a solution of *tert*-butyl N-[(1S)-1-{3-[4-amino-1-(difluoromethyl)-1H-pyrazol-5-yl]phenyl}but-3-en-1-yl]carbamate (3.3 g, 8.72 mmol) in EtOAc (20 ml) at 0 °C was added (R)-2-methylbut-3-enoic acid (1.048 g, 10.46 mmol), prepared as described in Intermediate 2, in EtOAc (10 ml), pyridine (2.116 ml, 26.2 mmol), and T3P®/50% EtOAc (10.38 ml, 17.44 mmol). After 4 h, the reaction was diluted with EtOAc, and washed with a solution of $K_2HPO_4$, followed by brine. The organic layer was dried over $Na_2SO_4$, filtered, and concentrated. The residue was purified normal phase column chromatography eluting with a gradient of hexanes/EtOAc to give *tert*-butyl N-[(1S)-1-{3-[1-(difluoromethyl)-4-[(2R)-2-methylbut-3-enamido]-1H-pyrazol-5-yl]phenyl}but-3-en-1-yl]carbamate (3.10 g, 6.73 mmol, 77% yield) as a yellow foam. MS(ESI) *m/z:* 461.2 $(M+H)^+$.

35D. Preparation of *tert*-butyl N-[(9*R*,10E,13*S*)-3-(difluoromethyl)-9-methyl-8-oxo-3,4,7-triazatricyclo[12.3.1.$0^{2,6}$]octadeca-1(18),2(6),4,10,14,16-hexaen-13-yl]carbamate

To a solution of *tert*-butyl N-[(1S)-1-{3-[1-(difluoromethyl)-4-[(2R)-2-methylbut-3-enamido]-1H-pyrazol-5-yl]phenyl}but-3-en-1-yl]carbamate (3.0 g, 6.51 mmol) in degassed DCM (800 mL), was added Second Generation Grubbs Catalyst (2.212 g, 2.61 mmol) and the reaction was heated to 40 °C. After stirring overnight, the mixture was concentrated and the residue was purified by normal phase column chromatography eluting with a gradient of hexanes/EtOAc to give *tert*-butyl N-[(9*R*,10E,13*S*)-3-(difluoromethyl)-9-methyl-8-oxo-3,4,7-triazatricyclo[12.3.1.$0^{2,6}$]octadeca-1(18),2(6),4,10,14,16-hexaen-13-yl]carbamate (1.8 g, 63.9%). MS(ESI) *m*/*z*: 433.2 $(M+H)^+$.

35E. Preparation of *tert*-butyl N-[(9*R*,13*S*)-3-(difluoromethyl)-9-methyl-8-oxo-3,4,7-triazatricyclo[12.3.1.$0^{2,6}$]octadeca-1(18),2(6),4,14,16-pentaen-13-yl]carbamate

To a solution of *tert*-butyl N-[(9*R*,10E,13*S*)-3-(difluoromethyl)-9-methyl-8-oxo-3,4,7-triazatricyclo[12.3.1.$0^{2,6}$]octadeca-1(18),2(6),4,10,14,16-hexaen-13-yl]carbamate (1.3 g, 3.01 mmol) in EtOH (50 ml) was added $PtO_2$ (0.102 g, 0.451 mmol) and the reaction was hydrogenated at 55 psi for 4 h. The reaction mixture was filtered through a plug of CELITE® and the filtrate concentrated to give tert-butyl N-[(9*R*,13*S*)-3-(difluoromethyl)-9-methyl-8-oxo-3,4,7-triazatricyclo[12.3.1.$0^{2,6}$]octadeca-1(18),2(6),4,14,16-pentaen-13-yl]carbamate (0.973 g, 74.5%). MS(ESI) *m*/*z*: 435.2 $(M+H)^+$.

35F. Preparation of (9*R*,13*S*)-13-amino-3-(difluoromethyl)-9-methyl-3,4,7-triazatricyclo [12.3.1.$0^{2,6}$]octadeca-1(18),2(6),4,14,16-pentaen-8-one

To a solution of *tert*-butyl N-[(9*R*,13*S*)-3-(difluoromethyl)-9-methyl-8-oxo-3,4,7-triazatricyclo[12.3.1.$0^{2,6}$]octadeca-1(18),2(6),4,14,16-pentaen-13-yl]carbamate (0.973 g, 2.239 mmol) in DCM (50 ml) was added TFA (5.18 ml, 67.2 mmol). After 3 h, the reaction mixture was concentrated to dryness. The residue was partitioned between sat $NaHCO_3$ and EtOAc. The aqueous phase was extracted with EtOAc (3x), washed with brine, dried over $Na_2SO_4$, filtered, and concentrated to give (9*R*,13*S*)-13-amino-3-(difluoromethyl)-9-methyl-3,4,7-triazatricyclo[12.3.1.$0^{2,6}$]octadeca-1(18),2(6),4,14,16-pentaen-8-one (0.619 g, 83%). MS(ESI) *m*/*z*: 335 $(M+H)^+$.

Intermediate 36

Preparation of (9*R*,13,*S*)-13-amino-3-($^2H_3$)methyl-9-methyl-3,4,7-triazatricyclo [12.3.1.$0^{2,6}$]octadeca-1(18),2(6),4,14,16-pentaen-8-one

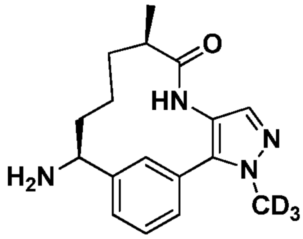

36A. Preparation of *tert*-butyl *N*-[(1*S*)-1-{3-[1-($^2$H3)methyl-4-nitro-1*H*-pyrazol-5-yl]phenyl}but-3-en-1-yl]carbamate

To a solution of *tert*-butyl *N*-[(1S)-1-(3-bromophenyl)but-3-en-1-yl]carbamate (3.8 g, 11.65 mmol) in DMF (35 ml), was added 1-($^2H_3$)methyl-4-nitro-1H-pyrazole (1.667 g, 12.81 mmol), di(adamantan-1-yl)(butyl)phosphine (0.626 g, 1.747 mmol), $K_2CO_3$ (4.83 g, 34.9 mmol) and pivalic acid (0.406 ml, 3.49 mmol). The reaction was purged with Ar and $Pd(OAc)_2$ (0.262 g, 1.165 mmol) was added. The reaction was heated to 115 °C. After 4 h, the reaction was diluted with 1:1 EtOAc/water (50 ml) and filtered through paper to remove Pd solids. The filtrate was extracted with EtOAc (2 x 50 ml). The combined organic layer was washed with water (20 ml), brine (20 ml), dried ($MgSO_4$), filtered and concentrated. The residue was purified by normal phase chromatography twice using hexanes and EtOAc as eluents to afford *tert*-butyl-*N*-[(1*S*)-1-{3-[1-($^2H_3$)methyl-4-nitro-1*H*-pyrazol-5-yl]phenyl}but-3-en-1-yl]carbamate (1.89g, 43.2%) as a brown oil. MS(ESI) *m*/*z*: 374.4 (M-H)$^+$.

36B. Preparation of *tert*-butyl *N*-[(1S)-1-{3-[4-amino-1-($^2H_3$)methyl-1H-pyrazol-5-yl]phenyl}but-3-en-1-yl]carbamate

To a cooled (0 °C) solution of *tert*-butyl *N*-[(1S)-1-{3-[1-($^2H_3$)methyl-4-nitro-1*H*-pyrazol-5-yl]phenyl}but-3-en-1-yl] carbamate (1.89 g, 5.03 mmol), dissolved in acetone (40 ml) / water (12 ml) was added $NH_4Cl$ (1.346 g, 25.2 mmol) and Zn (3.29 g, 50.3 mmol). The ice bath was removed and the solution was allowed to warm to rt. After 3 h, the reaction was filtered through paper and the filtrate was partitioned between water (20 ml) and EtOAc (75 ml). The aqueous layer was extracted with EtOAc (2 x 50 ml). The combined organic layers were washed with brine (25 ml), dried ($MgSO_4$), filtered and concentrated. The residue was purified by normal phase chromatography using hexanes and EtOAc and then DCM /0-10% MeOH as eluents to afford *tert*-butyl *N*-[(1S)-1-{3-[4-amino-1-($^2H_3$)methyl-1*H*-pyrazol-5-yl]phenyl}but-3-en-1-yl]carbamate (0.84g, 48.3%) as a light brown foam. MS(ESI) *m/z:* 346.5 (M+H)$^+$.

36C. Preparation of *tert*-butyl *N*-[(1*S*)-1-{3-[1-($^2H_3$)methyl-4-[(2*R*)-2-methylbut-3-enamido]-1*H*-pyrazol-5-yl]phenyl}but-3 -en-1-yl] carbamate

To *tert*-butyl *N*-[(1*S*)-1-{3-[4-amino-1-($^2H_3$)methyl-1*H*-pyrazol-5-yl]phenyl}but-3-en-1-yl]carbamate (0.7 g, 2.026 mmol) in EtOAc (6 ml) was added (R)-2-methylbut-3-enoic acid (0.26 g, 2.63 mmol), prepared as described in Intermediate 2, in 1 mL EtOAc. The mixture was cooled to 0 °C and pyridine (0.49 ml, 6.08 mmol) followed by a solution of 50% T3P® in EtOAc (2.41 ml, 4.05 mmol) were added. After 1 h, the reaction was partitioned with sat $NaHCO_3$ (30 ml) and EtOAc (50 ml). The aqueous layer was extracted with EtOAc (2 x 50 ml). The combined organic layers were washed with brine (25 ml), dried ($MgSO_4$),filtered and concentrated. The residue was purified by normal phase chromatography using hexanes and EtOAc as eluents to afford *tert*-butyl *N*-[(1*S*)-1-{3-[1-($^2H_3$)methyl-4-[(2*R*)-2-methylbut-3-enamido]-1*H*-pyrazol-5-yl]phenyl}but-3-en-1-yl]carbamate (0.69 g, 81%) as a rose oil. MS(ESI) *m*/*z*: 428.5 (M+H)$^+$. $^1$H NMR (400MHz, $CDCl_3$) $\delta$ 8.07 - 7.93 (m, 1H), 7.53 - 7.44 (m, 1H), 7.37 (d, J=7.9 Hz, 1H), 7.28 - 7.09 (m, 3H), 5.89 (ddd, J=17.4, 9.9, 7.9 Hz, 1H), 5.76 - 5.60 (m, 1H), 5.25 - 5.11 (m, 4H), 5.07 (d, J=7.0 Hz, 1H), 4.77 (br. s., 1H), 3.08 (quin, J=7.2 Hz, 1H), 2.62 - 2.47 (m, 2H), 1.41 (br. s., 9H), 1.30 (s, 3H).

36D. Preparation of *tert*-butyl N-[(9*R*,10E,13S)-3-($^2H_3$)methyl-9-methyl-8-oxo-3,4,7-triazatricyclo[12.3.1.0$^{2,6}$]octadeca-1(18),2(6),4,10,14,16-hexaen-13-yl]carbamate

To a degassed DCM (200 ml) solution of *tert*-butyl *N*-[(1S)-1-{3-[1-($^2H_3$)methyl-4-[(2R)-2-methylbut-3-enamido]-1*H*-pyrazol-5-yl]phenyl}but-3-en-1-yl]carbamate (0.699 g, 1.635 mmol) was added Second Generation Grubbs Catalyst (0.555 g, 0.654 mmol) and the resulting solution was heated to 40 °C for 24 h. The reaction mixture was concentrated and the residue was purified by normal phase chromatography using DCM and 0-10% MeOH as eluents to afford *tert*-butyl *N*-[(9R,10E,13S)-3-($^2H_3$)methyl-9-methyl-8-oxo-3,4,7-triazatricyclo[12.3.1.0$^{2,6}$]octadeca-1(18),2(6),4,10,14,16-hexaen-13-yl]carbamate (0.511 g, 78%) as a dark solid. MS(ESI) *m*/*z*: 400.2 (M+H)$^+$. $^1$H NMR (400MHz, $CDCl_3$) $\delta$ 7.65 - 7.56 (m, 1H), 7.51 - 7.44 (m, 1H), 7.30 (d, *J*=7.9 Hz, 1H), 7.23 (d, *J*=7.7 Hz, 1H), 6.85 (s, 1H), 6.68 (s, 1H), 5.66 (ddd, *J*=15.2, 9.3, 5.6 Hz, 1H), 5.20 - 5.06 (m, 1H), 4.94 (dd, *J*=15.3, 8.5 Hz, 1H), 4.78 - 4.66 (m, 1H),

3.08 - 2.99 (m, 1H), 2.71 - 2.58 (m, 1H), 2.23 - 2.12 (m, 1H), 1.43 (br. s., 9H), 1.25 - 1.19 (m, 3H).

36E. Preparation of tert-butyl *N*-[(9*R*,13*S*)-3-($^2H_3$)methyl-9-methyl-8-oxo-3,4,7-triazatricyclo[12.3.1.$0^{2,6}$]octadeca-1(18),2(6),4,14,16-pentaen-13-yl]carbamate

To a EtOH (20 ml) solution of *tert*-butyl *N*-[(9*R*,10E,13*S*)-3-($^2H_3$)methyl-9-methyl-8-oxo-3,4,7-triazatricyclo[12.3.1.$0^{2,6}$]octadeca-1(18),2(6),4,10,14,16-hexaen-13-yl]carbamate (0.40 g, 1.001 mmol) was added $PtO_2$ (0.023 g, 0.100 mmol). The reaction vessel was purged with $H_2$ and the reaction mixture was then hydrogenated at 55 psi. After 1.5 h under pressure, the reaction mixture was then allowed to sit overnight under $N_2$. The reaction was then filtered through CELITE® rinsing with DCM and EtOH. The filtrate was concentration of the afforded *tert*-butyl *N*-[(9*R*,13*S*)-3-($^2H_3$)methyl9-methyl-8-oxo-3,4,7-triazatricyclo[12.3.1.$0^{2,6}$]octadeca-1(18),2(6),4,14,16-pentaen-13-yl]carbamate (0.38 g, 95%) as a brown solid. MS(ESI) *m*/*z*: 402.5 $(M+H)^+$.

36F. Preparation of (9*R*,13*S*)-13-amino-3-($^2H_3$)methyl-9-methyl-3,4,7-triazatricyclo [12.3.1.$0^{2,6}$]octadeca-1(18),2(6),4,14,16-pentaen-8-one

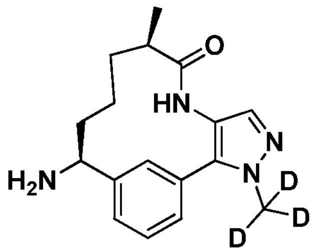

To a dioxane (2 ml) and MeOH (2 ml) solution of *tert*-butyl *N*-[(9*R*,13*S*)-3-($^2H_3$)methyl-9-methyl-8-oxo-3,4,7-triazatricyclo[12.3.1.$0^{2,6}$]octadeca-1(18),2(6),4,14,16-pentaen-13-yl]carbamate (0.38 g, 0.946 mmol) was added 4 N HCl in dioxane (2 ml). After 4 h, the reaction was concentrated to near dryness. The dry residue was dissolved in MeOH/DCM and filtered through 500 mg basic cartridge and the filtrate was concentrated to afford (9*R*,13*S*)-13-amino-3-($^2H_3$)methyl-9-methyl-3,4,7-triazatricyclo [12.3.1.$0^{2,6}$]octadeca-1(18),2(6),4,14,16-pentaen-8-one (0.28 g, 98%) as a gray solid. MS(ESI) *m*/*z*: 302.5 $(M+H)^+$.

Intermediate 37

Preparation of (9R,13S)-13-amino-3-(difluoromethyl)-9-methyl-3,4,7,17-tetraazatricyclo [12.3.1.$0^{2,6}$]octadeca-1(18),2(6),4,14,16-pentaen-8-one

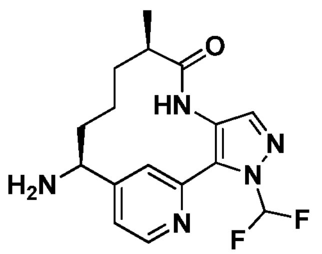

37A. Preparation of (S)-*tert*-butyl (1-(2-(1-(difluoromethyl)-4-nitro-1H-pyrazol-5-yl)pyridin-4-yl)but-3-en-1-yl)carbamate

To a large microwave vial was added (S)-*tert*-butyl (1-(2-bromopyridin-4-yl)but-3-en-1-yl)carbamate (1.5 g, 4.58 mmol), prepared as described for Intermediate 27, 1-(difluoromethyl)-4-nitro-1H-pyrazole (0.822 g, 5.04 mmol), prepared as described for Intermediate 30A, DMF (15.3 mL), di(adamantan-1-yl)(butyl)phosphine (0.247 g, 0.688 mmol), $K_2CO_3$ (1.901 g, 13.75 mmol) and pivalic acid (0.160 mL, 1.375 mmol). The mixture was purged with Ar for 15 min. $Pd(OAc)_2$ (0.103 g, 0.458 mmol) was added, the vial sealed and stirred at 115 °C. After 4h, the reaction was quenched with water

(50 mL) and extracted with EtOAc (3 x 50 mL). The combined organic layers were washed with brine (50 mL), dried ($MgSO_4$), filtered, and concentrated to give a dark brown oil. The crude product was purified by normal phase chromatography using heptane and EtOAc as eluents to give (S)-*tert*-butyl (1-(2-(1-(difluoromethyl)-4-nitro-1H-pyrazol-5-yl)pyridin-4-yl)but-3-en-1-yl)carbamate (973 mg, 52%) as a orange solid. MS(ESI) *m/z*: 410.1 $(M+H)^+$. $^1H$ NMR (500MHz, $CDCl_3$) δ 8.73 (d, J=5.2 Hz, 1H), 8.38 (s, 1H), 7.74 (br. s., 1H), 7.63 - 7.48 (m, 1H), 7.44 - 7.37 (m, 1H), 5.69 (ddt, J=17.0, 10.1, 7.0 Hz, 1H), 5.24 - 5.17 (m, 2H), 2.62 - 2.50 (m, 2H), 1.45 (s, 9H).

37B. Preparation of (S)-*tert*-butyl (1-(2-(4-amino-1-(difluoromethyl)-1H-pyrazol-5-yl)pyridin-4-yl)but-3-en-1-yl)carbamate

(S)-*tert*-Butyl (1-(2-(1-(difluoromethyl)-4-nitro-1H-pyrazol-5-yl)pyridin-4-yl)but-3-en-1-yl)carbamate (0.974 g, 2.379 mmol) was dissolved in acetone (15 mL) / water (3 mL), cooled to 0 °C, and $NH_4Cl$ (0.636 g, 11.90 mmol) and Zn (1.555 g, 23.79 mmol) were added. After stirring overnight at rt, the reaction mixture was filtered through a plug of CELITE® and the filtrate was concentrated. The residue was partitioned with water (30 mL) and EtOAc (50 mL). The aqueous layer was extracted with EtOAc (2 x 50 mL). The combined organic layers were washed with brine (20 mL) and dried ($MgSO_4$). The product was carried forward as is. MS(ESI) *m/z:* 380.1 $(M+H)^+$.

37C. Preparation of *tert*-butyl ((S)-1-(2-(1-(difluoromethyl)-4-((R)-2-methylbut-3-enamido)-1H-pyrazol-5-yl)pyridin-4-yl)but-3-en-1-yl)carbamate

To a stirring solution of (S)-*tert*-butyl (1-(2-(4-amino-1-(difluoromethyl)-1H-pyrazol-5-yl)pyridin-4-yl)but-3-en-1-yl)carbamate (0.900 g, 2.372 mmol) in EtOAc (7.91 mL) at 0 °C, (R)-2-methylbut-3-enoic acid (0.309 g, 3.08 mmol) in EtOAc (0.50 mL) T3P® / 50% EtOAc (2.82 mL, 4.74 mmol) and pyridine (0.576 mL, 7.12 mmol) were added. After 5 h, the reaction mixture concentrated and purified by normal phase chromatography using hexanes and EtOAc as eluents to afford tert-butyl ((S)-1-(2-(1-(difluoromethyl)-4-((R)-2-methylbut-3-enamido)-1H-pyrazol-5-yl)pyridin-4-yl)but-3-en-1-yl)carbamate (680 mg, 62.1%) as an oil. MS(ESI) *m/z*: 462.2 $(M+H)^+$. $^1H$ NMR (500MHz, $CDCl_3$-d) δ 10.74 (br. s., 1H), 8.60 (s, 1H), 8.57 (d, J=5.2 Hz, 1H), 7.84 (s, 1H), 7.58 - 7.42 (m, 1H), 7.23 - 7.20 (m, 1H), 6.00 (ddd, J=17.3, 10.1, 8.1 Hz, 1H), 5.72 - 5.62 (m, 1H), 5.36 - 5.31 (m, 2H), 5.21 - 5.15 (m, 2H), 3.22 (quin, J=7.2 Hz, 1H), 2.59 - 2.47 (m, 2H), 1.48 - 1.37 (m, 12H).

37D. Preparation of *tert*-butyl N-[(9R,10E,13S)-3-(difluoromethyl)-9-methyl-8-oxo-3,4,7,17-tetraazatricyclo[12.3.1.$0^{2,6}$]octadeca-1(18),2(6),4,10,14,16-hexaen-13-yl]carbamate

3 large microwave vials received equal portions of the following: *tert*-butyl ((S)-1-(2-(1-(difluoromethyl)-4-((R)-2-methylbut-3-enamido)-1H-pyrazol-5-yl)pyridin-4-yl)but-3-en-1-yl)carbamate (0.680 g, 1.473 mmol) in degassed DCE (61.4 mL) in the presence of Second Generation Grubbs Catalyst (0.500 g, 0.589 mmol) was irradiated to 120 °C for 30 min in a μwave. The reaction mixture was concentrated and purified by normal phase chromatography using hexanes and EtOAc as eluents to give *tert*-butyl N-[(9R,10E,13S)-3-(difluoromethyl)-9-methyl-8-oxo-3,4,7,17-tetraazatricyclo[12.3.1.$0^{2,6}$] octadeca-1(18),2(6),4,10,14,16-hexaen-13-yl]carbamate (158 mg, 24.7%) as a brown film. MS(ESI) *m/z*: 434.2 $(M+H)^+$. $^1H$ NMR (500MHz, $CDCl_3$-d) δ 8.69 (d, J=5.0 Hz, 1H), 8.05 - 7.89 (m, 1H), 7.83 (s, 1H), 7.10 (s, 1H), 6.77 (br. s., 1H), 5.73 (ddd, J=15.2, 9.7, 5.1 Hz, 1H), 5.13 - 5.05 (m, 2H), 3.19 - 3.10 (m, 1H), 2.73 (d, J=12.7 Hz, 1H), 2.24 - 2.15 (m, 1H), 1.46 (br. s., 9H), 1.30 - 1.24 (m, 4H).

37E. Preparation of *tert*-butyl N-[(9R,13S)-3-(difluoromethyl)-9-methyl-8-oxo-3,4,7,17-tetraazatricyclo[12.3.1.$0^{2,6}$]octadeca-1(18),2(6),4,14,16-pentaen-13-yl]carbamate

$PtO_2$ (8.28 mg, 0.036 mmol) was added to a solution of *tert*-butyl N-[(9R,10E,13 S)-3-(difluoromethyl)-9-methyl-8-oxo-3,4,7,17-tetraazatricyclo[12.3.1.$0^{2,6}$] octadeca-1(18),2(6),4,10,14,16-hexaen-13-yl]carbamate (0.158 g, 0.365 mmol) in EtOH (10mL) and subjected to a $H_2$ atmosphere (55 psi). After 3 h, the catalyst was filtered through a pad of CELITE® and filtrate concentrated to give *tert*-butyl N-[(9R,13S)-3-(difluoromethyl)-9-methyl-8-oxo-3,4,7,17-tetraazatricyclo[12.3.1.$0^{2,6}$]octadeca-1(18),2(6),4,14,16-pentaen-13-yl]carbamate. MS(ESI) *m/z*: 436.1 $(M+H)^+$.

37F. Preparation of (9R,13S)-13-amino-3-(difluoromethyl)-9-methyl-3,4,7,17-tetraazatricyclo[12.3.1.$0^{2,6}$]octadeca-1(18),2(6),4,14,16-pentaen-8-one

*tert*-Butyl N-[(9R,13S)-3-(difluoromethyl)-9-methyl-8-oxo-3,4,7,17-tetraazatricyclo[12.3.1.$0^{2,6}$]octadeca-1(18),2(6),4,14,16-pentaen-13-yl]carbamate (0.159 g, 0.365 mmol) was dissolved in MeOH (0.50 mL) and treated with 4 M HCl in dioxane (1.83 mL, 7.30 mmol). After stirring for 14 h, the reaction mixture was concentrated to dryness. The

amine HCl salt was free based by dissolving in MeOH and passing through 2 consecutive $NaHCO_3$ cartridges. The filtrate was concentrated (9R,13S)-13-amino-3-(difluoromethyl)-9-methyl-3,4,7,17-tetraazatricyclo[12.3.1.0$^{2,6}$]octadeca-1(18),2(6),4,14,16-pentaen-8-one (0.085 g, 69%). MS(ESI) *m/z*: 336.1 $(M+H)^+$.

Intermediate 38

Preparation of (10*R*,14*S*)-14-amino-10-methyl-3,8-diazatricyclo[13.3.1$^{2,7}$] nonadeca-1(19),2,4,6,15,17-hexaen-9-one.

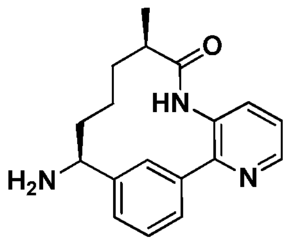

38A. Preparation of {3-[(1*S*)-1-{[(*tert*-butoxy)carbonyl]amino}but-3-en-1-yl]phenyl} boronic acid

To a solution of *tert*-butyl N-[(1*S*)-1-(3-bromophenyl)but-3-en-1-yl]carbamate, prepared as described in Intermediate 24, (2.36 g, 7.23 mmol) in dioxane (50 ml), was added 5,5,5',5'-tetramethyl-2,2'-bi(1,3,2-dioxaborinane) (1.798 g, 7.96 mmol), and KOAc (2.130 g, 21.70 mmol). The mixture was purged with Ar and $PdCl_2$(dppf)-$CH_2Cl_2$ adduct (0.295 g, 0.362 mmol) was added. The reaction mixture was heated to 90 °C for 18 h, then quenched with water (20 ml) and extracted with EtOAc (3 x 30 ml). The combined organic layers were washed with brine (20 ml), dried ($Na_2SO_4$), filtered and concentrated. The residue was absorbed on CELITE® and charged to a 100 g reverse phase cartridge which was eluted with a 25 min gradient from 10-100% Solvent B (Solvent A: 90% $H_{2O}$ - 10% MeCN - 0.05% TFA; Solvent B: 90% MeCN - 10% $H_2O$ - 0.05% TFA) to give {3-[(1S)-1-{[(*tert*-butoxy)carbonyl]amino}but-3-en-1-yl]phenyl}boronic acid as a tan solid. MS(ESI) *m/z:* 292.08 $(M+H)^+$.

38B. Preparation of *tert*-butyl N-[(1*S*)-1-[3-(3-aminopyridin-2-yl)phenyl]but-3-en-1-yl]carbamate

{3-[(1S)-1-{[(*tert*-Butoxy)carbonyl]amino}but-3-en-1-yl]phenyl}boronic acid (0.36 g, 1.236 mmol), 2-bromopyridin-3-amine (0.214 g, 1.236 mmol), and 2 M aq $Na_2CO_3$ (3.09 ml, 6.18 mmol) were added to dioxane (8 ml) and purged with a stream of Ar for 10 min. $Pd(PPh_3)_4$ (0.143 g, 0.124 mmol) was added and the reaction mixture was irradiated in microwave at 120 °C for 30 min. The reaction was quenched with water (20 ml) and extracted with EtOAc (3 x 30 ml). The combined organic layers were washed with brine (15 ml), dried ($MgSO_4$), filtered and concentrated. The residue was purified via Isco 40g column eluting with DCM/0-10% MeOH to give a tan foam (0.352g (84%). The reaction mixture was filtered and concentrated and the residue was purified by normal phase chromatography using DCM and 0-10% MeOH as eluents to afford *tert*-butyl N-[(1*S*)-1-[3-(3-aminopyridin-2-yl)phenyl]but-3-en-1-yl]carbamate (0.352 g, 84%) as a tan solid. MS(ESI) *m/z*: 340.5 $(M+H)^+$. $^1H$ NMR (400MHz, $CDCl_3$) δ 8.13 (dd, J=4.1, 1.9 Hz, 1H), 7.62 - 7.54 (m, 2H), 7.44 (t, J=7.6 Hz, 1H), 7.26 (s, 2H), 7.12 - 7.00 (m, 2H), 5.82 - 5.57 (m, 1H), 5.23 - 5.02 (m, 1H), 4.91 (br. s., 1H), 4.80 (br. s., 1H), 3.82 (br. s., 2H), 2.81 - 2.41 (m, 2H), 1.51 - 1.34 (m, 9H).

38C. Preparation of *tert*-butyl N-[(1*S*)-1-(3-{3-[(2*R*)-2-methylbut-3-enamido]pyridin-2-yl}phenyl)but-3-en-1-yl]carbamate

To a cooled (0 °C) EtOAc (6 mL) solution of *tert*-butyl N-[(1*S*)-1-[3-(3-minopyridin-2-yl)phenyl]but-3-en-1-yl]carbamate (0.334 g, 1.03 mmol) was added (R)-2-methylbut-3-enoic acid (0.135 g, 1.348 mmol), prepared as described in Intermediate 2, in 1 ml EtOAc, pyridine (0.252 ml, 3.11 mmol) and the dropwise addition of a 50% EtOAc solution of T3P® (1.235 ml, 2.074 mmol). After 1 h, the reaction was partitioned between sat $NaHCO_3$ (30 ml) and EtOAc (50 ml). The aqueous layer was extracted with EtOAc (2 x 50 ml). The combined organic layers were washed with brine (25 ml) dried ($MgSO_4$), filtered and concentrated. The residue was purified by normal phase chromatography using hexanes and EtOAc as eluents to afford *tert*-butyl N-[(1*S*)-1-(3-{3-[(2*R*)-2-methylbut-3-enamido]pyridin-2-yl}phenyl)but-3-en-1-yl]carbamate (0.334g, 76%) as a tan solid. MS(ESI) *m/z*: 428.5 $(M+H)^+$. $^1H$ NMR (400MHz, $CDCl_3$) δ 8.71 (dd, J=8.3, 1.2 Hz, 1H), 8.41 (dd, J=4.6, 1.5 Hz, 1H), 7.60 (br. s., 1H), 7.49 - 7.42 (m, 2H), 7.42 - 7.36 (m, 2H), 7.29 (dd, J=8.4, 4.8 Hz, 1H), 5.84 - 5.62 (m, 2H), 5.16 - 5.02 (m, 4H), 4.92 (br. s., 1H), 4.80 (br. s., 1H), 3.04 (quin, J=7.3 Hz, 1H), 2.62 - 2.48 (m, 2H), 1.51 - 1.35 (m, 9H), 1.32 - 1.25 (m, 3H).

38D. Preparation of *tert*-butyl N-[(10*R*,11E,14*S*)-10-methyl-9-oxo-3,8-diazatricyclo [13.3.1.$0^{2,7}$]nonadeca-1(19),2(7),3,5,11,15,17-heptaen-14-yl]carbamate trifluoroacetate

To a in degassed DCE (20 ml) solution of *tert*-butyl N-[(1*S*)-1-(3-{3-[(2*R*)-2-methylbut-3-enamido]pyridin-2-yl}phenyl)but-3-en-1-yl]carbamate (0.15 g, 0.356 mmol) was added Second Generation Grubbs Catalyst (0.121 g, 0.142 mmol) and the resulting solution was heated to 120 °C for 30 min in a microwave. The reaction was concentrated, and the residue purified by normal phase chromatography, then reverse phase preparative HPLC (PHENOMENEX® Luna Axia C18 5$\mu$ 30 x 100 mm column, 8-min gradient; Solvent A: 30% MeOH - 70% $H_2O$- 0.1% TFA; Solvent B: 90% MeOH - 10% $H_2O$ - 0.1% TFA) to afford *tert*-butyl N-[(10*R*,11E,14*S*)-10-methyl-9-oxo-3,8-diazatricyclo[13.3.1.$0^{2,7}$]nonadeca-1(19),2(7),3,5,11,15,17-heptaen-14-yl]carbamate trifluoroacetate (71 mg, 39%) as a clear residue. MS(ESI) *m/z:* 394.5 $(M+H)^+$. $^1H$ NMR (400MHz, $CDCl_3$) $\delta$ 8.73 (d, J=4.4 Hz, 1H), 8.44 (d, J=7.9 Hz, 1H), 7.99 - 7.89 (m, 1H), 7.69 - 7.59 (m, 1H), 7.59 - 7.48 (m, 2H), 7.17 - 7.08 (m, 1H), 5.84 - 5.67 (m, 1H), 4.67 - 4.53 (m, 1H), 4.53 - 4.38 (m, 1H), 3.28 - 3.17 (m, 1H), 2.77 - 2.66 (m, 1H), 2.04 (q, J=11.4 Hz, 1H), 1.46 (br. s., 9H), 1.18 - 1.09 (m, 3H).

38E. Preparation of *tert*-butyl N-[(10*R*,14*S*)-10-methyl-9-oxo-3,8-diazatricyclo [13.3.1.$0^{2,7}$]nonadeca-1(19),2(7),3,5,15,17-hexaen-14-yl]carbamate

To a EtOH (5 ml) solution of *tert*-butyl N-[(10*R*,11E,14S)-10-methyl-9-oxo-3,8-diazatricyclo[13.3.1.$0^{2,7}$]nonadeca-1(19),2(7),3,5,11,15,17-heptaen-14-yl]carbamate (0.37 g, 0.940 mmol) (free base was prepared as in Example 38D) was added $PtO_2$ (21 mg) and the reaction mixture was purged with $H_2$ and was hydrogenated at 20-30 psi for 4 h. The reaction was filtered through CELITE® and the filtrate was concentrated to afford *tert*-butyl N-[(10*R*,14,*S*)-10-methyl-9-oxo-3,8-diazatricyclo[13.3.1.$0^{2,7}$]nonadeca-1(19),2(7),3,5,15,17-hexaen-14-yl]carbamate (0.37g, 99%) as a dark solid. MS(ESI) *m*/*z*: 396.3 $(M+H)^+$.

38F. Preparation of (10*R*,14*S*)-14-amino-10-methyl-3,8-diazatricyclo[13.3.1.$0^{2,7}$] nonadeca-1(19),2(7),3,5,15,17-hexaen-9-one

*tert*-Butyl N-[(10*R*,14*S*)-10-methyl-9-oxo-3,8-diazatricyclo[13.3.1.$0^{2,7}$]nonadeca-1(19),2 (7),3,5,15,17-hexaen-14-yl]carbamate (0.18 g, 0.455 mmol) was dissolved in 4 N HCl in dioxane (2 ml) and MeOH (2 ml). After 2 h, the reaction was concentrated, the residue was dissolved in DCM/MeOH and free-based by passing through a 500 mg basic cartridge (2x). Concentration of the filtrate afforded (10*R*,14*S*)-14-amino-10-methyl-3,8-diazatricyclo[13.3.1.$0^{2,7}$]nonadeca-1(19),2(7),3,5,15,17-hexaen-9-one (0.11g, 49%) as a dark brown film. MS(ESI) *m*/*z*: 296.3 $(M+H)^+$.

Intermediate 39

Preparation of (10*R*,14*S*)-14-amino-10-methyl-3,8,16-triazatricyclo[13.3.1.$0^{2,7}$]nonadeca-1(19),2(7),3,5,15,17-hexaen-9-one

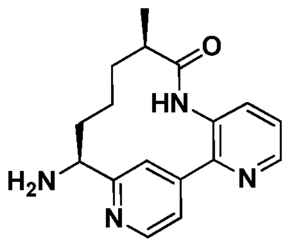

39A. Preparation of (S)-*tert*-butyl (1-(3-amino-[2,4'-bipyridin]-2'-yl)but-3-en-1-yl)carbamate

To a solution of (S)-(2-(1-((*tert*-butoxycarbonyl)amino)but-3-en-1-yl)pyridin-4-yl)boronic acid trifluoroacetate (0.60 g, 1.477 mmol) in dioxane (12 ml) was added 2-bromopyridin-3-amine (0.256 g, 1.477 mmol) and 2 M aq $Na_2CO_3$ (3.69 ml, 7.39 mmol). The reaction mixture was purged with a stream of Ar for 10 min. $Pd(PPh_3)_4$ (0.171 g, 0.148 mmol) was added and the mixture irradiated at 120 °C for 30 min. The reaction was partitioned between water and EtOAc. The organic layer was washed with brine, dried over $Na_2SO_4$, filtered and concentrated. The crude material was purified by normal phase column chromatography eluting with a gradient of DCM/MeOH to give (S)-*tert*-butyl (1-(3-amino-[2,4'-bipyridin]-2'-yl)but-3-en-1-yl)carbamate (0.500 g, 99% yield) as a brown oil. MS(ESI) *m*/*z*: 341.1 $(M+H)^+$.

39B. Preparation of *tert*-butyl ((S)-1-(3-((R)-2-methylbut-3-enamido)-[2,4'-bipyridin]-2'-yl)but-3-en-1-yl)carbamate

A solution of (R)-2-methylbut-3-enoic acid (0.191 g, 1.909 mmol), prepared as described in Intermediate 2, (S)-*tert*-butyl (1-(3-amino-[2,4'-bipyridin]-2'-yl)but-3-en-1-yl)carbamate (0.500 g, 1.469 mmol), and pyridine (0.356 ml, 4.41 mmol) in EtOAc (14.69 ml) was cooled down to 0 °C under Ar followed by addition of T3P® (50% wt in EtOAc) (1.75 ml, 2.94 mmol), then the reaction mixture was gradually warmed up to rt. After stirring overnight, the mixture was diluted with EtOAc, washed with 1.5 M $K_2HPO_4$ followed by brine, dried over $Na_2SO_4$, filtered, and concentrated. The crude product was then purified by normal phase chromatography eluting with a gradient of hexanes/EtOAc to give *tert*-butyl ((S)-1-(3-((R)-2-methylbut-3-enamido)-[2,4'-bipyridin]-2'-yl)but-3-en-1-yl)carbamate (0.458 g, 73.8% yield) as a yellow foam. MS(ESI) *m/z*: 423.2 $(M+H)^+$.

39C. Preparation of *tert*-butyl N-[(10*R,*1E,14*S*)-10-methyl-9-oxo-3,8,16-triazatricyclo [13.3.1.$0^{2,7}$]nonadeca-1(19),2(7),3,5,11,15,17-heptaen-14-yl]carbamate, *bis*-trifluoroacetate

*tert*-Butyl ((S)-1-(3-((R)-2-methylbut-3-enamido)-[2,4'-bipyridin]-2'-yl)but-3-en-1-yl)carbamate (100 mg, 0.237 mmol) in degassed DCE (14.79 ml) in the presence of Second Generation Grubbs Catalyst (0.080 g, 0.095 mmol) was irradiated at 120 °C for 30 min. The reaction mixture was concentrated and purified by reverse phase chromatography to give the *tert*-butyl N-[(10R,11E,14S)-10-methyl-9-oxo-3,8,16-triazatricyclo[13.3.1.$0^{2,7}$]nonadeca-1(19),2(7),3,5,11,15,17-heptaen-14-yl]carbamate, *bis*-trifluoroacetate (39 mg, 26.5% yield) as brown oil. MS(ESI) *m/z:* 395.2 $(M+H)^+$.

39D. Preparation of *tert*-butyl N-[(10*R*,14*S*)-10-methyl-9-oxo-3,8,16-triazatricyclo[13.3.1.$0^{2,7}$]nonadeca-1(19),2(7),3,5,15,17-hexaen-14-yl]carbamate

$PtO_2$ (2.245 mg, 9.89 μmol) was added to a stirring solution of *tert*-butyl N-[(10R,11E,14S)-10-methyl-9-oxo-3,8,16-triazatricyclo[13.3.1.$0^{2,7}$]nonadeca-1(19),2(7),3,5,11,15,17-heptaen-14-yl]carbamate, bis-trifluoroacetate (0.039 g, 0.099 mmol) in EtOH (10 ml) and subjected to a $H_2$ atmosphere (55 psi). After 4 h, the reaction mixture was filtered through a pad of CELITE® and the filtrate concentrated to give *tert*-butyl N-[(10*R*,14*S*)-10-methyl-9-oxo-3,8,16-triazatricyclo[13.3.1.$0^{2,7}$]nonadeca-1(19),2(7),3,5,15,17-hexaen-14-yl]carbamate. MS(ESI) *m/z*: 397.2 $(M+H)^+$.

39E. Preparation of (10*R*,14*S*)-14-amino-10-methyl-3,8,16-triazatricyclo[13.3.1.$0^{2,7}$] nonadeca-1(19),2(7),3,5,15,17-hexaen-9-one

TFA (0.15 mL, 1.967 mmol) was added to a solution of *tert*-butyl N-[(10R,14S)-10-methyl-9-oxo-3,8,16-triazatricyclo[13.3.1.$0^{2,7}$]nonadeca-1(19),2(7),3,5,15,17-hexaen-14-yl]carbamate (0.039 g, 0.098 mmol) in DCM (2.0 ml). After stirring for 4 h, the reaction mixture was concentrated to dryness, and placed under high vacuum for 12 h. The residue was neutral by dissolving in MeOH, passing through $NaHCO_3$ cartridge (StratoSpheres SPE; 500 mg, 0.90 mmol loading), and concentrating the filtrate to give (10*R*,14*S*)-14-amino-10-methyl-3,8,16-triazatricyclo[13.3.1.$0^{2,7}$]nonadeca-1(19),2(7),3,5,15,17-hexaen-9-one. MS(ESI) *m/z*: 297.5 $(M+H)^+$.

Intermediate 40

Preparation of (9*R*,13*S*)-13-amino-3-(2-hydroxyethyl)-9-methyl-3,4,7,15-tetraazatricyclo[12.3.1.$0^{2,6}$]octadeca-1(18),2(6),4,14,16-pentaen-8-one

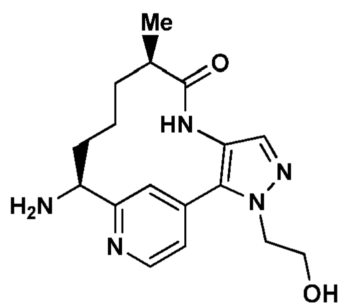

40A. Preparation of 1-(2-((*tert*-butyldimethylsilyl)oxy)ethyl)-4-nitro-1H-pyrazole

To a solution of 4-nitro-1*H*-pyrazole (4.0 g, 35.4 mmol) in DMF (50 mL) was added $CS_2CO_3$ (12.68 g, 38.9 mmol) and (2-bromoethoxy)(*tert*-butyl)dimethylsilane (8.35 mL, 38.9 mmol). The resulting suspension was heated to 60 °C for 2 h. The reaction mixture was then diluted with EtOAc (2 x 25 mL) and washed with 10% LiCl solution (25 mL). The organic layer was concentrated, and the residue purified using normal phase chromatography to yield 1-(2-((*tert*-butyldimethylsilyl)oxy)ethyl)-4-nitro-1*H*-pyrazole as white solid (8.6 g, 85% yield). MS(ESI) *m*/*z*: 272.4 $(M+H)^+$.

40B. Preparation of (S)-*tert*-butyl (1-(4-(1-(2-((*tert*-butyldimethylsilyl)oxy)ethyl)-4-nitro-1*H*-pyrazol-5-yl)pyridin-2-yl)but-3-en-1-yl)carbamate

To a $N_2$ flushed pressure vial was added (S)-*tert*-butyl (1-(4-chloropyridin-2-yl)but-3-en-1-yl)carbamate, prepared as described in Intermediate 23, (3.0 g, 10.61 mmol), 1-(2-((*tert*-butyldimethylsilyl)oxy)ethyl)-4-nitro-1*H*-pyrazole, prepared as described in Intermediate 40A, (2.88 g, 10.61 mmol), di(adamant-1-yl)(butyl)phosphine (0.571 g, 1.59 mmol), PvOH (0.369 ml, 3.18 mmol), $K_2CO_3$ (4.40 g, 31.8 mmol), and DMF (20 mL). The vial was purged with $N_2$ for 5 min and $Pd(OAc)_2$ (0.238 g, 1.061 mmol) was added. The reaction mixture was again briefly purged with $N_2$. The vial was sealed and heated at 120 °C for 4 h. The reaction mixture was cooled to rt and partitioned between 10% aqueous LiCl (15 mL) and EtOAc (30 mL). The aqueous layer was extracted with EtOAc (2 x 20 mL) and the combined organic layers were washed with brine (15 mL), dried over $MgSO_4$, filtered and concentrated. The crude product was purified using normal phase chromatography to yield (*S*)-*tert*-butyl (1-(4-(1-(2-((*tert*butyldimethylsilyl)oxy)ethyl)-4-nitro-1*H*-pyrazol-5-yl)pyridin-2-yl)but-3-en-1-yl)carbamate (1.4 g, 25% yield) as a brown oil. MS(ESI) *m*/*z:* 518.3 $(M+H)^+$.

40C. Preparation of (S)-*tert*-butyl (1-(4-(4-amino-1-(2-((*tert*-butyldimethylsilyl)oxy) ethyl)-1*H*-pyrazol-5-yl)pyridin-2-yl)but-3-en-1-yl)carbamate

A solution of (S)-*tert*-butyl (1-(4-(1-(2-((*tert*-butyldimethylsilyl)oxy)ethyl)-4-nitro-1*H*-pyrazol-5-yl)pyridin-2-yl)but-3-en-1-yl)carbamate (2.4 g, 4.64 mmol) in MeOH (25 mL) and $CH_3COOH$ (2.5 mL) was heated to 40 °C. To the resulting clear solution was then slowly added Zn (0.606 g, 9.27 mmol, in 3 portions (50:25:25%)) and the reaction was stirred at 40 °C for 5 min. Additional Zn was added to the reaction. The reaction mixture was monitored by LCMS and once complete, to the cooled reaction mixture was then added 2.5 g of $K_2CO_3$ (1 g for 1 mL AcOH) and 2.5 mL water. The reaction mixture was then stirred for 5 min. The reaction mixture was then filtered over a pad of CELITE® and concentrated to yield the crude product. The crude product was partitioned between EtOAc (40 mL) and sat $NaHCO_3$ (20 mL). The organic layers were separated, dried over $MgSO_4$, filtered and concentrated. The crude product was purified using normal phase chromatography to yield (S)-*tert*-butyl (1-(4-(4-amino-1-(2-((*tert*butyldimethylsilyl)oxy)ethyl)-1*H*-pyrazol-5-yl)pyridin-2-yl)but-3-en-1-yl)carbamate (1.9 g, 80% yield) as pale brown oil. MS(ESI) *m*/*z*: 488.6 $(M+H)^+$.

40D. Preparation of *tert*-butyl ((*S*)-1-(4-(1-(2-((*tert*-butyldimethylsilyl)oxy)ethyl)-4-((*R*)-2-methylbut-3-enamido)-1*H*-pyrazol-5-yl)pyridin-2-yl)but-3-en-1-yl)carbamate

To a $N_2$ flushed, 3-necked, 250 mL RBF was added (S)-*tert*-butyl (1-(4-(4-amino-1-(2-((*tert*-butyldimethylsilyl)oxy)ethyl)-1*H*-pyrazol-5-yl)pyridin-2-yl)but-3-en-1-yl)carbamate (1.9 g, 3.90 mmol) and EtOAc (25 mL). The solution was cooled to -10 °C and (*R*)-2-methylbut-3-enoic acid, as prepared in Intermediate 2, (390 mg, 3.90 mmol), pyridine (0.630 mL, 7.79 mmol) and T3P® (3.48 mL, 5.84 mmol) were added. The cooling bath was removed and the solution was allowed to warm to rt and then stirred for 20 h. Water (20 mL) and EtOAc (20 mL) were added and the mixture was stirred for 30 min. The organic phase was separated and the aqueous layer was extracted with EtOAc (20 mL). The combined organic extracts were washed with brine (15 mL), dried over $Na_2SO_4$, filtered and concentrated. Purification by normal phase chromatography eluting with a gradient of hexanes/EtOAc gave ((*S*)-1-(4-(1-(2-((*tert*-butyldimethylsilyl)oxy) ethyl)-4-((*R*)-2-methylbut-3-enamido)-1*H*-pyrazol-5-yl)pyridin-2-yl)but-3-en-1-yl)carbamate (0.68 g, 28% yield). MS(ESI) *m*/*z*: 570.1 $[M+H]^+$.

40E. Preparation of *tert*-butyl N-[(9*R*,10*E*,13*S*)-3-{2-[(*tert*-butyldimethylsilyl)oxy] ethyl}-9-methyl-8-oxo-3,4,7,15-tetraazatricyclo[12.3.1.0$^{2,6}$]octadeca-1(18),2(6),4,10,14,16-hexaen-13-yl]carbamate

To a $N_2$ flushed, 250 mL, 3-necked, RBF was added a solution of *tert*-butyl ((*S*)-1-(4-(1-(2-((*tert*-butyldimethylsilyl)oxy)ethyl)-4-((*R*)-2-methylbut-3-enamido)-1*H*-pyrazol-5-yl)pyridin-2-yl)but-3-en-1-yl)carbamate (680 mg, 1.193 mmol) in EtOAc (56 mL). The solution was sparged with Ar for 15 min. Second Generation Grubbs Catalyst (253 mg, 0.298 mmol) was added in one portion. The reaction mixture was heated to reflux temperature for overnight. After cooling to rt, the solvent was removed and the residue was purified by normal phase chromatography eluting with a gradient of

DCM/MeOH to yield *tert*-butyl N-[(9*R*,10*E*,13*S*)-3-{2-[(*tert*-butyldimethylsilyl)oxy] ethyl}-9-methyl-8-oxo-3,4,7,15-tetraazatricyclo[12.3.1.$0^{2,6}$]octadeca-1(18),2(6),4,10,14,16-hexaen-13-yl]carbamate (400 mg, 61% yield) as a tan solid. MS(ESI) *m*/*z*: 542.6 $[M+H]^+$.

40F. Preparation of *tert*-butyl N-[(9*R*,13*S*)-3-{2-[(*tert*-butyldimethylsilyl)oxy]ethyl}-9-methyl-8-oxo-3,4,7,15-tetraazatricyclo[12.3.1.$0^{2,6}$]octadeca-1(18),2(6),4,14,16-pentaen-13-yl]carbamate

Pd/C (0.078 g, 0.074 mmol) was added to a 250 mL Parr hydrogenation flask containing a solution of *tert*-butyl N-[(9*R*,10E,13*S*)-3-{2-[(*tert*-butyldimethylsilyl)oxy] ethyl}-9-methyl-8-oxo-3,4,7,15-tetraazatricyclo[12.3.1.$0^{2,6}$]octadeca-1(18),2(6),4,10,14,16-hexaen-13-yl]carbamate (400 mg, 0.738 mmol) in EtOH (20 mL). The flask was purged with $N_2$ and pressurized to 55 psi of $H_2$ and allowed to stir for 4 h. The reaction was filtered through a pad of CELITE® and concentrated to yield *tert*-butyl N-[(9*R*,13*S*)-3-{2-[(*tert*-butyldimethylsilyl)oxy]ethyl}-9-methyl-8-oxo-3,4,7,15-tetraazatricyclo[12.3.1.$0^{2,6}$]octadeca-1(18),2(6),4,14,16-pentaen-13-yl]carbamate (375 mg, 92% yield) as a tan solid. MS(ESI) *m*/*z*: 544.6 $[M+H]^+$.

40G. (9*R*,13*S*)-13-Amino-3-(2-hydroxyethyl)-9-methyl-3,4,7,15-tetraazatricyclo [12.3.1.$0^{2,6}$]octadeca-1(18),2(6),4,14,16-pentaen-8-one

To a solution of *tert*-butyl N-[(9*R*,13*S*)-3-{2-[(*tert*-butyldimethylsilyl)oxy]ethyl}-9-methyl-8-oxo-3,4,7,15-tetraazatricyclo[12.3.1.$0^{2,6}$]octadeca-1(18),2(6),4,14,16-pentaen-13-yl]carbamate (375 mg, 0.690 mmol) in MeOH (5 mL) was added 4 N HCl in dioxane (5 mL, 20.0 mmol) and the reaction mixture was stirred at rt for 1 h. The reaction mixture was then concentrated to give (9*R*,13*S*)-13-amino-3-(2-hydroxyethyl)-9-methyl-3,4,7,15-tetraazatricyclo [12.3.1.$0^{2,6}$]octadeca-1(18),2(6),4,14,16-pentaen-8-one, bishydrochloride (220 mg, 96% yield) as a pale yellow solid which was then dissolved in MeOH (4 mL) to give a clear, pale yellow solution. The solution was added to a pre-rinsed AGILENT® StratoSpheres SPE PL-$HCO_3$ MP Resin cartridge. Gravity filtration, eluting with MeOH, gave a clear, slightly yellow filtrate. Concentration provided (9*R*,13*S*)-13-amino-3-(2-hydroxyethyl)-9-methyl-3,4,7,15-tetraazatricyclo[12.3.1.$0^{2,6}$] octadeca-1(18),2(6),4,14,16-pentaen-8-one (170 mg, 96%) as a pale yellow solid. MS(ESI) *m*/*z*: 330.5 $[M+H]^+$.

Intermediate 41

Preparation of (9*R*,13*S*)-13-amino-16-fluoro-3,9-dimethyl-3,4,7-triazatricyclo [12.3.1.$0^{2,6}$]octadeca-1(18),2(6),4,14,16-pentaen-8-one

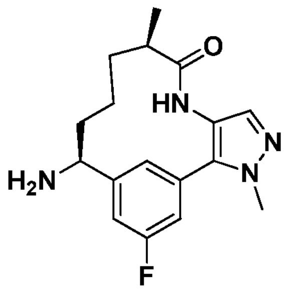

41A. Preparation of *tert*-butyl *N*-[(1*S*)-1-[3-fluoro-5-(1-methyl-4-nitro-1*H*-pyrazol-5-yl)phenyl]but-3-en-1-yl]carbamate

To a DMF (1 ml) solution of *tert*-butyl *N*-[(1*S*)-1-(3-bromo-5-fluorophenyl)but-3-en-1-yl]carbamate (0.19 g, 0.552 mmol), prepared as described in Intermediate 25, was added 1-methyl-4-nitro-1*H*-pyrazole (0.070 g, 0.552 mmol), di(adamantan-1-yl)(butyl) phosphine (0.059 g, 0.166 mmol), pivalic acid (0.019 ml, 0.166 mmol), $K_2CO_3$ (0.229 g, 1.656 mmol) and $Pd(OAc)_2$ (0.025 g, 0.110 mmol). The reaction mixture was purged with Ar, and heated at 120 °C. After 18 h, the reaction was partitioned between water (15 ml) and EtOAc (30 ml). The aqueous layer was extracted with EtOAc (2 x 20 ml). The combined organic layers were washed with brine (15 ml), dried ($MgSO_4$), filtered and concentrated. The residue was purified by normal phase chromatography and was eluted with hexanes and EtOAc to afford *tert*-butyl *N*-[(1*S*)-1-[3-fluoro-5-(1-methyl-4-nitro-1*H*-pyrazol-5-yl)phenyl]but-3-en-1-yl]carbamate as a yellow oil (0.123 g, 57%). $^1H$ NMR (400MHz, $CDCl_3$) $\delta$ 8.23 - 8.17 (m, 1H), 7.22 - 7.16 (m, 1H), 7.10 (s, 1H), 7.01 (dt, J=8.5, 1.9 Hz, 1H), 5.76 - 5.60 (m, 1H), 5.22 - 5.11 (m, 2H), 4.90 (br. s., 1H), 4.78 (br. s., 1H), 3.78 - 3.69 (m, 3H), 2.60 - 2.48 (m, 2H), 1.41 (br. s., 9H).

41B. Preparation of tert-butyl *N*-[(1*S*)-1-[3-(4-amino-1-methyl-1*H*-pyrazol-5-yl)-5-fluorophenyl]but-3 -en-1 -yl]carbamate

*tert*-Butyl *N*-[(1S)-1-[3-(4-amino-1-methyl-1*H*-pyrazol-5-yl)-5-fluorophenyl]but-3-en-1-yl]carbamate (0.123 g, 0.315 mmol) was dissolved in acetone (5 ml) / water (1 ml), cooled to 0 °C, and $NH_4Cl$ (0.084 g, 1.575 mmol) and Zn (0.206 g, 3.15 mmol) were added. The ice bath was removed and the reaction mixture was warmed to rt. After 3 h, the reaction was filtered and partitioned between water (10 ml) and EtOAc (30 ml). The aqueous layer was extracted with EtOAc (2 x 20 ml). The combined organic layers were washed with brine (10 ml), dried ($MgSO_4$), filtered and concentrated. The residue was purified by normal phase chromatography and was eluted with hexanes and EtOAc to afford *tert*-butyl *N*-[(1,5*S*)-1-[3-(4-amino-1-methyl-1*H*-pyrazol-5-yl)-5-fluorophenyl]but-3-en-1-yl]carbamate (0.105 g, 92%). MS(ESI) *m*/*z*: 361.08 $(M+H)^+$.

41C. Preparation of *tert*-butyl *N*-[(1*S*)-1-(3-fluoro-5-{1-methyl-4-[(2*R*)-2-methylbut-3-enamido]-1*H*-pyrazol-5-yl}phenyl)but-3 -en-1 -yl]carbamate

To *tert*-butyl *N*-[(1S)-1-[3-(4-amino-1-methyl-1*H*-pyrazol-5-yl)-5-fluorophenyl] but-3-en-1-yl]carbamate (0.105 g, 0.291 mmol) was added EtOAc (0.6 ml), (R)-2-methylbut-3-enoic acid (0.035 g, 0.350 mmol), prepared as described in Intermediate 2, in 0.3 ml EtOAc. The reaction mixture was cooled to 0 °C, and a 50% EtOAc solution of T3P® (0.347 ml, 0.583 mmol) and Hunig's Base (0.153 ml, 0.874 mmol) were added. After 4 h, the reaction was partitioned between sat $NaHCO_3$ (5 ml) and EtOAc (5 ml). The aqueous layer was extracted with EtOAc (2 x 10 ml). The combined organic layers were washed with brine (5 ml), dried ($MgSO_4$), filtered and concentrated. The residue was purified by normal phase chromatography and was eluted with hexanes and EtOAc to give *tert*-butyl *N*-[(1*S*)-1-(3-fluoro-5-{1-methyl-4-[(2*R*)-2-methylbut-3-enamido]-1*H*-pyrazol-5-yl}phenyl)but-3-en-1-yl]carbamate as a yellow foam (53 mg, 41%). MS(ESI) *m*/*z*: 443.5 $(M+H)^+$.

41D. Preparation of *tert*-butyl *N*-[(9*R*,10E,13*S*)-16-fluoro-3,9-dimethyl-8-oxo-3,4,7-triazatricyclo[12.3.1.$0^{2,6}$]octadeca-1(18),2(6),4,10,14,16-hexaen-13-yl]carbamate

To a degassed DCE (10 ml) solution of *tert*-butyl *N*-[(1S)-1-(3-fluoro-5-{1-methyl-4-[(2R)-2-methylbut-3-enamido]-1*H*-pyrazol-5-yl}phenyl)but-3-en-1-yl]carbamate (0.053 g, 0.120 mmol) was added Second Generation Grubbs Catalyst (0.041 g, 0.048 mmol) and the reaction mixture was heated to 120 °C for 30 min in a microwave. The reaction mixture was directly purified by normal phase chromatography eluting with hexanes and EtOAc to afford *tert*-butyl *N*-[(9*R*,10E,13*S*)-16-fluoro- 3,9-dimethyl-8-oxo-3,4,7-triazatricyclo[12.3.1.$0^{2,6}$]octadeca-1(18),2(6),4,10,14,16-hexaen-13-yl]carbamate as a dark solid (27 mg, 54%). MS(ESI) *m*/*z*: 415.4 $(M+H)^+$.

41E. Preparation of (9*R*,13*S*)-13-amino-16-fluoro-3,9-dimethyl-3,4,7-triazatricyclo [12.3.1.$0^{2,6}$]octadeca-1(18),2(6),4,14,16-pentaen-8-one

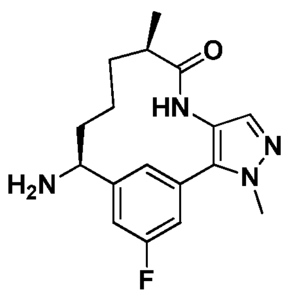

To an EtOH (3 ml) solution of *tert*-butyl N-[(9*R*,10E,13S)-16-fluoro-3,9-dimethyl-8-oxo-3,4,7-triazatricyclo[12.3.1.$0^{2,6}$]octadeca-1(18),2(6),4,10,14,16-hexaen-13-yl]carbamate (0.027 g, 0.065 mmol) was added $PtO_2$ (5 mg). The reaction mixture was purged with $H_2$ and was then hydrogenated at 55 psi. After 6 h, the reaction mixture was filtered through CELITE® and concentrated to give 19 mg of a dark solid MS(ESI) *m/z:* 417.08 $(M+H)^+$. The dark solid residue was dissolved in 50% TFA/DCM (3 ml). After 3 h, the reaction mixture was concentrated, the residue was dissolved in DCM/MeOH, passed through a basic cartridge and concentrated to give (9*R*,13*S*)-13-amino-16-fluoro-3,9-dimethyl-3,4,7-triazatricyclo[12.3.1.$0^{2,6}$]octadeca-1(18),2(6),4,14,16-pentaen-8-one a dark solid (19 mg, 92%). MS(ESI) *m*/*z*: 317.4 $(M+H)^+$.

Intermediate 42

Preparation of (9*R*,13*S*)-13-amino-3,9-dimethyl-3,4,7,17-tetraazatricyclo[12.3.1.$0^{2,6}$] octadeca-1(18),2(6),4,14,16-pentaen-8-one

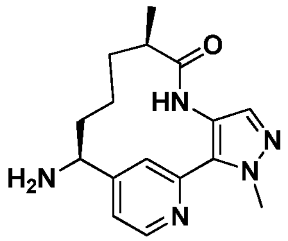

42A. Preparation of *tert*-butyl *N*-[(1S)-1-[2-(1-methyl-4-nitro-1*H*-pyrazol-5-yl)pyridin-4-yl]but-3-en-1-yl]carbamate

To a large microwave vial was added *tert*-butyl N-[(1*S*)-1-(2-bromopyridin-4-yl) but-3-en-1-yl]carbamate (1.0 g, 3.06 mmol), prepared as described in Intermediate 27, 1-methyl-4-nitro-1*H*-pyrazole (0.427 g, 3.36 mmol), dioxane (10 ml), di(adamantan-1-yl)(butyl)phosphine (0.164 g, 0.458 mmol), $K_2CO_3$ (1.267 g, 9.17 mmol) and pivalic acid (0.106 ml, 0.917 mmol). The reaction was purged with Ar. $Pd(OAc)_2$ (0.069 g, 0.306 mmol) was added and the reaction was stirred at 100 °C. After 4 h, heating was stopped and the reaction was stirred at rt for 72 h. The reaction was quenched with water (20 ml) and extracted with EtOAc (3 x 50 ml). The combined organic layers were washed with brine (20 ml), dried ($MgSO_4$), filtered, and concentrated. The residue was purified by normal phase chromatography using heptanes and EtOAc as eluents to give *tert*-butyl *N*-[(1S)-1-[2-(1-methyl-4-nitro-1*H*-pyrazol-5-yl)pyridin-4-yl]but-3-en-1-yl]carbamate (0.62 g, 54%) as a white foam. MS(ESI) *m/z*: 374.08 $(M+H)^+$. $^1H$ NMR (500MHz, $CDCl_3$) δ 8.73 (d, *J*=5.2 Hz, 1H), 8.28 - 8.15 (m, 1H), 7.66 - 7.54 (m, 1H), 7.43 - 7.34 (m, 1H), 5.76 - 5.63 (m, 1H), 5.26 - 5.16 (m, 2H), 4.99 (br. s., 1H), 4.83 (br. s., 1H), 3.97 - 3.85 (m, 3H), 2.66 - 2.46 (m, 2H), 1.45 (br. s., 9H).

42B. Preparation of *tert*-butyl *N*-[(1S)-1-[2-(4-amino-1-methyl-1*H*-pyrazol-5-yl)pyridin-4-yl]but-3-en-1-yl]carbamate

To a cooled (0 °C) acetone (40 ml) / water (12 ml) solution of *tert*-butyl *N*-[(1*S*)-1-[2-(1-methyl-4-nitro-1*H*-pyrazol-5-yl)pyridin-4-yl]but-3-en-1-yl]carbamate (0.62 g, 1.660 mmol) was added $NH_4Cl$ (0.444 g, 8.30 mmol) and Zn (1.086 g, 16.60 mmol). The ice bath was removed and the reaction was stirred 18 h. The reaction was filtered through paper and partitioned with water (20 ml) and EtOAc (75 ml). The aqueous layer was extracted with EtOAc (2 x 50 ml). The combined organic layers were washed with brine (25 ml), dried ($MgSO_4$), filtered and concentrated. The residue was purified by normal phase chromatography using DCM and 0-10% MeOH as eluents to give *tert*-butyl *N*-[(1S)-1-[2-(4-amino-1-methyl-1*H*-pyrazol-5-yl)pyridin-4-yl]but-3-en-1-yl]carbamate (0.46 g, 60%). MS(ESI) *m/z*: 344.5 $(M+H)^+$.

42C. Preparation of *tert*-butyl *N*-[(1*S*)-1-(2-{1-methyl-4-[(2*R*)-2-methylbut-3-enamido]-1*H*-pyrazol-5-yl}pyridin-4-yl)but-3-en-1-yl]carbamate

To *tert*-butyl *N*-[(1S)-1-[2-(4-amino-1-methyl-1*H*-pyrazol-5-yl)pyridin-4-yl]but-3-en-1-yl]carbamate (0.6 g, 1.747 mmol) was added (R)-2-methylbut-3-enoic acid (0.189 g, 1.893 mmol), prepared as described in Intermediate 2, in EtOAc (5.8 ml), cooled to 0 °C. Pyridine (0. 0.424 ml, 5.24 mmol) and a 50% EtOAc solution of T3P® (2.080 ml, 3.49 mmol) were added. After 24 h, the reaction was partitioned between sat $NaHCO_3$ (10 ml) and EtOAc (20 ml). The aqueous layer was extracted with EtOAc (2 x 20 ml). The combined organic layers were washed with brine (10 ml), dried ($MgSO_4$), filtered and concentrated. The residue was purified by normal phase chromatography using hexanes and EtOAc as eluents to give *tert*-butyl *N*-[(1*S*)-1-(2-{1-methyl-4-[(2*R*)-2-methylbut-3-enamido]-1*H*-pyrazol-5-yl}pyridin-4-yl)but-3-en-1-yl]carbamate (0.35g, 47%). MS(ESI) *m/z*: 426.1 $(M+H)^+$. $^1H$ NMR (500MHz, $CDCl_3$) δ 10.23 (br. s., 1H), 8.70 - 8.56 (m, 1H), 8.35 (d, *J*=1.1 Hz, 1H), 7.56 - 7.44 (m, 1H), 7.25 - 7.14 (m, 1H), 6.03 (ddd, *J*=17.2, 10.2, 8.0 Hz, 1H), 5.39 - 5.17 (m, 3H), 5.03 - 4.63 (m, 2H), 4.14 - 4.08 (m, 3H), 3.22 (quin, *J*=7.2 Hz, 1H), 2.66 - 2.49 (m, 1H), 1.84 - 1.72 (m, 1H), 1.50 - 1.40 (m, 9H), 1.42 - 1.37 (m, 3H), 1.06 - 0.93 (m, 1H).

42D. Preparation of *tert*-butyl *N*-[(9*R*,10E,13*S*)-3,9-dimethyl-8-oxo-3,4,7,17-tetraazatricyclo[12.3.1.$0^{2,6}$]octadeca-1(18),2(6),4,10,14,16-hexaen-13-yl]carbamate

To a degassed DCE (20 ml) solution of *tert*-butyl *N*-[(1*S*)-1-(2-{1-methyl-4-[(2*R*)-2-methylbut-3-enamido]-1*H*-pyrazol-5-yl} pyridin-4-yl)but-3-en-1-yl]carbamate (0.160 g, 0.376 mmol) was added Second Generation Grubbs Catalyst (0.096 g, 0.113 mmol) and the reaction mixture was heated to 120 °C for 30 min in a microwave. The reaction mixture was concentrated and the residue was purified by normal phase chromatography using DCM and MeOH as eluents to afford desired product (29 mg, 19%) as a green film. MS(ESI) *m*/*z*: 398.3 $(M+H)^+$. $^1$H NMR (500MHz, $CDCl_3$) δ 8.71 (d, *J*=4.7 Hz, 1H), 7.58 (s, 1H), 7.23 (d, *J*=13.8 Hz, 1H), 7.03 - 6.94 (m, 1H), 6.61 (s, 1H), 5.82 - 5.71 (m, 1H), 5.19 - 5.09 (m, 2H), 4.75 (br. s., 1H), 4.15 - 4.09 (m, 3H), 3.19 - 3.10 (m, 1H), 2.67 (br. s., 1H), 2.28 - 2.15 (m, 2H), 1.54 - 1.39 (m, 9H), 1.34 - 1.28 (m, 3H).

42E. Preparation of (9*R*,13*S*)-13-amino-3,9-dimethyl-3,4,7,17-tetraazatricyclo [12.3.1.$0^{2,6}$]octadeca-1(18),2(6),4,14,16-pentaen-8-one

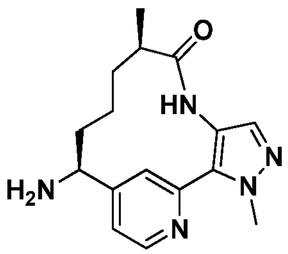

To an EtOH (3 mL) solution of *tert*-butyl *N*-[(9*R*,10E,13*S*)-3,9-dimethyl-8-oxo-3,4,7,17-tetraazatricyclo[12.3.1.$0^{2,6}$]octadeca-1(18),2(6),4,10,14,16-hexaen-13-yl]carbamate (29 mg, 0.073 mmol) was added $PtO_2$ (4 mg). The reaction mixture was purged with $H_2$, then was hydrogenated at 55 psi. After 3 h, the reaction mixture was filtered through a 0.45μM filter and concentrated to afford a dark solid (MS(ESI) *m*/*z:* 400.3 $(M+H)^+$). The dark solid residue was dissolved in 4 N HCl in dioxane (1 ml) and MeOH (1 ml). After 3 h, the mixture was concentrated and resultant HCl salt was dissolved in DCM/MeOH and passed through a basic cartridge to afford (9*R*,13*S*)-13-amino-3,9-dimethyl-3,4,7,17-tetraazatricyclo[12.3.1.$0^{2,6}$]octadeca-1(18),2(6),4,14,16-pentaen-8-one as a dark solid (21 mg, 96%). MS(ESI) *m*/*z:* 300.2 $(M+H)^+$.

Intermediate 43

Preparation of (9*R*,13*S*)-13-amino-3-(difluoromethyl)-9-methyl-3,4,7,16-tetraazatricyclo [12.3.1.$0^{2,6}$]octadeca-1(18),2(6),4,14,16-pentaen-8-one

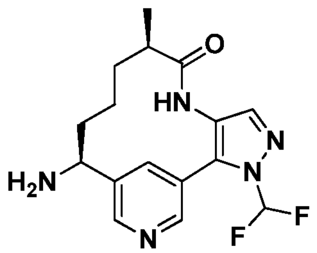

43A. Preparation of (*S*)-*tert*-butyl (1-(5-(1-(difluoromethyl)-4-nitro-1H-pyrazol-5-yl)pyridin-3-yl)but-3 -en-1 -yl)carbamate

To a large microwave vial was added (*S*)-*tert*-butyl (1-(5-bromopyridin-3-yl)but-3-en-1-yl)carbamate (1.0 g, 3.06 mmol), prepared a described in Intermediate 26, 1-(difluoromethyl)-4-nitro-1H-pyrazole (0.548 g, 3.36 mmol), DMF (10.19 ml), di(adamantan-1-yl)(butyl)phosphine (0.164 g, 0.458 mmol), $K_2CO_3$ (1.267 g, 9.17 mmol) and pivalic acid

(0.106 ml, 0.917 mmol). The reaction mixture was purged with Ar. After 10 min, $Pd(OAc)_2$ (0.069 g, 0.306 mmol) was added, the vessel sealed, and stirred at 115 °C. After 4 h, the reaction was quenched with $H_2O$ (50 mL) and extracted with EtOAc (3 x 50 mL). The combined organic phase was washed with brine (50 mL), dried ($MgSO_4$), filtered, and concentrated. The crude material was purified by normal phase chromatography eluting with a gradient of heptane/EtOAc to give (*S*)-*tert*-butyl (1-(5-(1-(difluoromethyl)-4-nitro-1H-pyrazol-5-yl)pyridin-3-yl)but-3-en-1-yl)carbamate (1.25 g, 100%). MS(ESI) *m*/*z*: 410.2 $(M+H)^+$.

43B. Preparation of (*S*)-*tert*-butyl (1-(5-(4-amino-1-(difluoromethyl)-1H-pyrazol-5-yl)pyridin-3 -yl)but-3 -en-1 -yl)carbamate

(*S*)-*tert*-Butyl (1-(5-(1-(difluoromethyl)-4-nitro-1H-pyrazol-5-yl)pyridin-3-yl)but-3-en-1-yl)carbamate (1.27 g, 3.10 mmol) was dissolved in acetone (15ml) / water (3 ml), cooled to 0 °C, and $NH_4Cl$ (0.830 g, 15.51 mmol) and Zn (2.028 g, 31.0 mmol) were added. The ice bath was removed. After 2 h, the reaction mixture was filtered and filtrate partitioned with water (30 ml) and EtOAc (50 ml). The aqueous layer was extracted with EtOAc (2 x 50 ml). The combined organic phase was washed with brine (20 ml), dried ($MgSO_4$), filtered, and concentrated. The residue was purified by normal phase chromatography eluting with a gradient of DCM/MeOH to give (*S*)-*tert*-butyl (1-(5-(4-amino-1-(difluoromethyl)-1H-pyrazol-5-yl)pyridin-3-yl)but-3-en-1-yl)carbamate (0.720 g, 61.2% yield) as a solid. MS(ESI) *m*/*z*: 380 $(M+H)^+$.

43C. Preparation of tert-butyl ((*S*)-1-(5-(1-(difluoromethyl)-4-((*R*)-2-methylbut-3-enamido)-1H-pyrazol-5-yl)pyridin-3-yl)but-3-en-1-yl)carbamate

A solution of (*S*)-*tert*-butyl (1-(5-(4-amino-1-(difluoromethyl)-1H-pyrazol-5-yl)pyridin-3-yl)but-3-en-1-yl)carbamate (0.720 g, 1.898 mmol) in EtOAc (20 ml) was cooled to 0 °C and (*R*)-2-methylbut-3-enoic acid (0.228 g, 2.277 mmol), prepared as described in Intermediate 2, in EtOAc (10 ml), pyridine (0.460 ml, 5.69 mmol), and T3P® (50% wt in EtOAc) (2.259 ml, 3.80 mmol) were added. After 6 h, the reaction was partitioned with 1.5 M $K_2PO_4$ (50 mL) and EtOAc (50 mL). The aqueous layer was extracted with EtOAc (2 x 20 mL). The combined organic phase was washed with brine (50 mL), dried ($MgSO_4$), filtered, and concentrated. The residue was purified by normal chromatography eluting with a gradient of hexanes/EtOAc to give *tert*-butyl ((*S*)-1-(5-(1-(difluoromethyl)-4-((*R*)-2-methylbut-3-enamido)-1H-pyrazol-5-yl)pyridin-3-yl)but-3-en-1-yl)carbamate (0.386 g, 44.1% yield) as a yellow foam. MS(ESI) *m*/*z*: 462.2 $(M+H)^+$.

43D. Preparation of tert-butyl *N*-[(9*R*,10*E*,13*S*)-3-(difluoromethyl)-9-methyl-8-oxo-3,4,7,16-tetraazatricyclo[12.3.1.$0^{2,6}$]octadeca-1(18),2(6),4,10,14,16-hexaen-13-yl]carbamate

To a RBF was added *tert*-butyl ((*S*)-1-(5-(1-(difluoromethyl)-4-((*R*)-2-methylbut-3-enamido)-1H-pyrazol-5-yl)pyridin-3-yl)but-3-en-1-yl)carbamate (0.190 g, 0.412 mmol), *p*TsOH (0.086 g, 0.453 mmol), and degassed DCE (103 ml). The clear yellow solution was warmed to 40 °C and degassed with Ar for 1 h. Second Generation Grubbs Catalyst (0.140 g, 0.165 mmol) was added and reaction stirred at 40 °C overnight. Additional Second Generation Grubbs Catalyst (0.2 eq.) was added and stirring continued. After stirring for a total of 48 h, the reaction mixture was cooled to rt, washed with sat $NaHCO_3$, brine, dried over $MgSO_4$, filtered, and concentrated. The crude product was purified by normal phase chromatography eluting with a gradient of DCM/MeOH to give *tert*-butyl *N*-[(9*R*,10*E*,13*S*)-3-(difluoromethyl)-9-methyl-8-oxo-3,4,7,16-tetraazatricyclo[12.3.1.$0^{2,6}$]octadeca-1(18),2(6),4,10,14,16-hexaen-13-yl]carbamate (0.020 g, 11.2%) as a brown oil. MS(ESI) *m*/*z*: 434.3 $(M+H)^+$.

43E. Preparation of *tert*-butyl *N*-[(9*R*,13*S*)-3-(difluoromethyl)-9-methyl-8-oxo-3,4,7,16-tetraazatricyclo[12.3.1.$0^{2,6}$]octadeca-1(18),2(6),4,14,16-pentaen-13-yl]carbamate

To an EtOH (3 mL) solution of *tert*-butyl *N*-[(9*R*,10*E*,13*S*)-3-(difluoromethyl)-9-methyl-8-oxo-3,4,7,16-tetraazatricyclo[12.3.1.$0^{2,6}$]octadeca-1(18),2(6),4,10,14,16-hexaen-13-yl]carbamate (0.020, 0.046 mmol) was added $PtO_2$ (1.048 mg, 4.61 μmol) and the reaction was purged with $H_2$. The reaction mixture was subjected to a $H_2$ atmosphere (55 psi). After 2 h, the catalyst was filtered off through a plug of CELITE® and the filtrate concentrated to give *tert*-butyl *N*-[(9*R*,13*S*)-3-(difluoromethyl)-9-methyl-8-oxo-3,4,7,16-tetraazatricyclo[12.3.1.$0^{2,6}$]octadeca-1(18),2(6),4,14,16-pentaen-13-yl]carbamate. MS(ESI) *m*/*z*: 436.2 $(M+H)^+$.

43F. Preparation of (9*R*,13*S*)-13-amino-3-(difluoromethyl)-9-methyl-3,4,7,16-tetraazatricyclo[12.3.1.$0^{2,6}$]octadeca-1(18),2(6),4,14,16-pentaen-8-one

*tert*-Butyl *N*-[(9*R*,13*S*)-3-(difluoromethyl)-9-methyl-8-oxo-3,4,7,16-tetraazatricyclo[12.3.1.$0^{2,6}$]octadeca-1(18),2(6),4,14,16-pentaen-13-yl]carbamate (0.020 g, 0.046 mmol) was dissolved in 4 N HCl in dioxane (0.230 ml, 0.919 mmol). A minimum amount of MeOH was added to aid dissolution. After 1 h, the reaction mixture was concentrated to dryness. The residue was dissolved in MeOH, passed through a NaHCO3 cartridge (StratoSpheres SPE; 500 mg, 0.90 mmol loading), concentrated to give (9*R*,13*S*)-13-amino-3-(difluoromethyl)-9-methyl-3,4,7,16-tetraazatricyclo [12.3.1.0$^{2,6}$]octadeca-1(18),2(6),4, 14, 16-pentaen-8-one. MS(ESI) *m/z*: 336.2 $(M+H)^+$.

Intermediate 44

Preparation of 6-(2-bromo-5-chlorophenyl)pyrimidin-4-ol

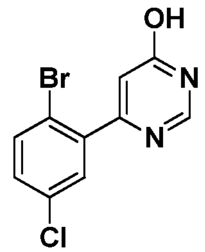

44A. Preparation of 4-(2-bromo-5-chlorophenyl)-6-methoxypyrimidine

To a suspension of 4-chloro-2-(6-methoxypyrimidin-4-yl)aniline (100 mg, 0.424 mmol) and $TsOH{\cdot}H_2O$ (97 mg, 0.509 mmol) in $CH_3CN$ (20 mL) was added $CuBr_2$ (9.48 mg, 0.042 mmol). Then *t*-butyl nitrite (0.067 mL, 0.509 mmol) was added followed by tetrabutylammonium bromide (274 mg, 0.849 mmol) and the reaction was stirred at rt. After 2 h, water was added and the mixture was extracted with $CH_2Cl_2$ (2x). The organic layers were combined, dried over $MgSO_4$, filtered, and concentrated. Purification by normal phase chromatography gave 4-(2-bromo-5-chlorophenyl)-6-methoxypyrimidine (115mg, 90% yield) as a white solid. MS(ESI) *m/z*: 299.2 $(M+H)^+$. $^1H$ NMR (400MHz, $CDCl_3$) $\delta$ 8.88 (d, *J*=1.1 Hz, 1H), 7.61 (d, *J*=8.6 Hz, 1H), 7.56 (d, *J*=2.6 Hz, 1H), 7.30-7.24 (m, 1H), 7.04 (d, *J*=1.1 Hz, 1H), 4.05 (s, 3H).

44B. Preparation of 6-(2-bromo-5-chlorophenyl)pyrimidin-4-ol

6-(2-Bromo-5-chlorophenyl)pyrimidin-4-ol was prepared according to the procedures described in Intermediate 5 for the synthesis of 6-(5-chloro-2-fluorophenyl) pyrimidin-4-ol, by replacing 6-(5-chloro-2-fluorophenyl)pyrimidin-4-ol with 4-(2-bromo-5-chlorophenyl)-6-methoxypyrimidine. MS(ESI) *m/z*: 285.2 $(M+H)^+$. $^1H$ NMR (400MHz, DMSO-$d_6$) $\delta$ 8.19 (s, 1H), 7.72 (d, *J*=8.6 Hz, 1H), 7.52 (d, *J*=2.6 Hz, 1H), 7.41 (dd, *J*=8.6, 2.6 Hz, 1H), 6.21 (s, 1H).

Example 45

Preparation of (9R,13S)-13-(4-{5-chloro-2-[(pyrimidin-2-yl)amino]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one

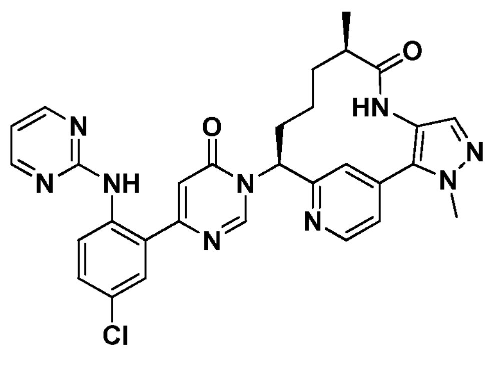

(9*R*,13*S*)-13-(4-{5-Chloro-2-[(pyrimidin-2-yl)amino]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one, 2 trifluoroacetate (2.75 mg, 19% yield) was prepared in a similar manner as the procedure described in Example 314, by replacing 4-bromopyrimidine hydrochloride (6.78 mg, 0.035 mmol) with 2-bromopyrimidine (5.51 mg, 0.035 mmol). MS(ESI) *m/z*: 582.5 $(M+H)^+$. $^1H$ NMR (400MHz, $CD_3OD$) δ 9.12 (s, 1H), 8.74 (d, *J*=5.1 Hz, 1H), 8.46 - 8.42 (m, 3H), 7.73 (s, 1H), 7.66 (d, *J*=2.6 Hz, 1H), 7.54 - 7.48 (m, 2H), 7.43 (dd, *J*=8.9, 2.5 Hz, 1H), 6.85 (t, *J*=5.0 Hz, 1H), 6.79 (s, 1H), 6.06 (dd, *J*=12.7, 4.3 Hz, 1H), 4.05 (s, 3H), 2.78 - 2.67 (m, 1H), 2.42 - 2.31 (m, 1H), 2.16 - 2.02 (m, 2H), 1.69 - 1.44 (m, 2H), 1.02 (d, *J*=6.8 Hz, 3H), 0.80 - 0.63 (m, 1H). Analytical HPLC (Method A): RT = 8.33 min, 97.9% purity; Factor XIa Ki = 2,000 nM.

Example 46 (reference)

Preparation of ethyl 2-[4-(4-chloro-2-{1-[(9R,13S)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazol-1-yl]acetate

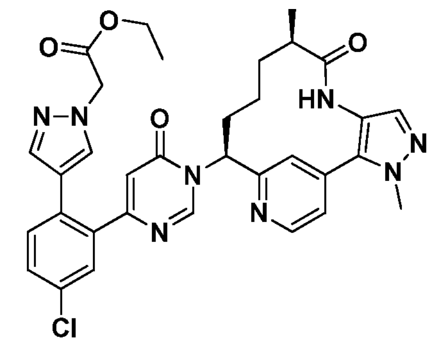

Ethyl 2-[4-(4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyiimidin-4-yl}phenyl)-1H-pyrazol-1-yl]acetate trifluoroacetate (3.67 mg, 15% yield) was prepared in a similar manner as the procedure described in Example 49, by replacing 1-methyl-4-(4,4,5,5-tetramethyl-1,3,2-dioxaborolan-2-yl)-1H-pyrazole with ethyl 2-(4-(4,4,5,5-tetramethyl-1,3,2-dioxaborolan-2-yl)-1H-pyrazol-1-yl)acetate (13.67 mg, 0.049 mmol). MS(ESI) *m/z*: 641.5 $(M+H)^+$. $^1H$ NMR (400MHz, $CD_3OD$) δ 8.96 (s, 1H), 8.74 (d, *J*=5.3 Hz, 1H), 7.72 (s, 1H), 7.62 (s, 1H), 7.56 - 7.52 (m, 2H), 7.51 - 7.49 (m, 3H), 7.45 (s, 1H), 6.41 (s, 1H), 6.00 (dd, *J*=12.7, 4.1 Hz, 1H), 4.94 (s, 2H), 4.18 (q, *J*=7.1 Hz, 2H), 4.05 (s, 3H), 2.76 - 2.66 (m, 1H), 2.40 - 2.28 (m, 1H), 2.14 - 2.03 (m, 2H), 1.67 - 1.42 (m, 2H), 1.24 (t, *J*=7.2 Hz, 3H), 1.02 (d, *J*=6.8 Hz, 3H), 0.80 - 0.65 (m, 1H). Analytical HPLC (Method A): RT = 7.92 min, 99.6% purity; Factor XIa Ki = 25 nM, Plasma Kallikrein Ki = 7,000 nM.

Example 48

Preparation of 2-(4-chloro-2-{1-[(9R,13 S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)acetonitrile

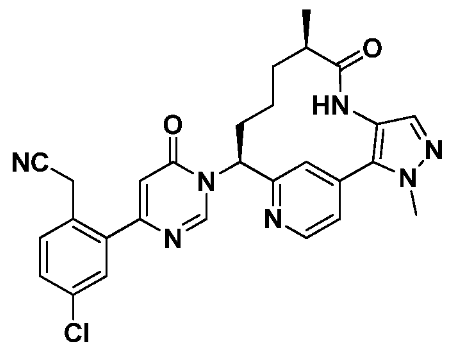

2-(4-Chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)acetonitrile trifluoroacetate (2.2 mg, 11%

yield) was prepared in a similar manner as the procedure described in Example 49, by replacing 1-methyl-4-(4,4,5,5-tetramethyl-1,3,2-dioxaborolan-2-yl)-1H-pyrazole with 4-(4,4,5,5-tetramethyl-1,3,2-dioxaborolan-2-yl)isoxazole (9.52 mg, 0.049 mmol). MS(ESI) *m/z:* 528.35 $(M+H)^+$. $^1H$ NMR (500MHz, $CD_3OD$) δ 9.04 (br. s., 1H), 8.74 (d, *J*=5.2 Hz, 1H), 7.73 (s, 1H), 7.59 - 7.49 (m, 5H), 6.65 (s, 1H), 6.06 (d, *J*=9.6 Hz, 1H), 4.18 - 4.08 (m, 2H), 4.06 (s, 3H), 2.78 - 2.68 (m, 1H), 2.42 - 2.33 (m, 1H), 2.16 - 2.03 (m, 2H), 1.68 - 1.58 (m, 1H), 1.55 - 1.45 (m, 1H), 1.02 (d, *J*=6.9 Hz, 3H), 0.79 - 0.65 (m, 1H). Analytical HPLC (Method C): RT = 1.46 min, 100% purity; Factor XIa Ki = 16 nM, Plasma Kallikrein Ki = 850 nM.

Example 49

Preparation of (9*R*,13*S*)-13-{4-[5-chloro-2-(1-methyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

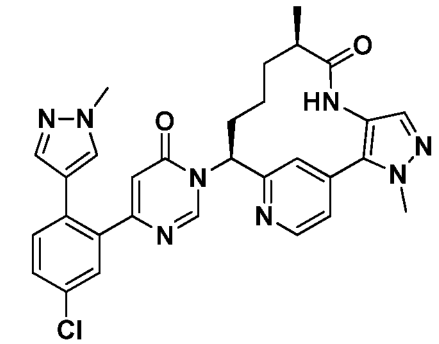

To a degassed solution of (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one (15 mg, 0.024 mmol), prepared as described in Example 211, 1-methyl-4-(4,4,5,5-tetramethyl-1,3,2-dioxaborolan-2-yl)-1H-pyrazole (7.61 mg, 0.037 mmol), and $K_2CO_3$ (8.43 mg, 0.061 mmol) in 1,4-dioxane (0.6 ml) and water (0.2 ml) was added $Pd(Ph_3P)_4$ (2.82 mg, 2.440 μmol). The reaction was microwaved at 120 °C for 0.5 h, and then cooled to rt and concentrated. Purification by reverse phase chromatography afforded (9*R*,13*S*)-13-{4-[5-chloro-2-(1-methyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (7.9 mg, 47% yield) as an off-white solid. MS(ESI) *m/z*: 569.6 $(M+H)^+$. $^1H$ NMR (500MHz, $CD_3OD$) δ 8.97 (s, 1H), 8.75 (d, *J*=5.2 Hz, 1H), 7.73 (s, 1H), 7.59 (s, 1H), 7.55 - 7.52 (m, 2H), 7.50 - 7.45 (m, 3H), 7.33 (s, 1H), 6.40 (d, *J*=0.6 Hz, 1H), 6.02 (dd, *J*=12.7, 3.9 Hz, 1H), 4.05 (s, 3H), 3.84 (s, 3H), 2.75 - 2.68 (m, 1H), 2.39 - 2.30 (m, 1H), 2.13 - 2.02 (m, 2H), 1.66 - 1.45 (m, 2H), 1.02 (d, *J*=7.2 Hz, 3H), 0.78 - 0.65 (m, 1H). Analytical HPLC (Method A): RT = 7.01 min, 98.4% purity; Factor XIa Ki = 14 nM, Plasma Kallikrein Ki = 930 nM.

Example 50

Preparation of (9*R*,{4-[5-chloro-2-(1,3-dimethyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14, 16-pentaen-8-one trifluoroacetate

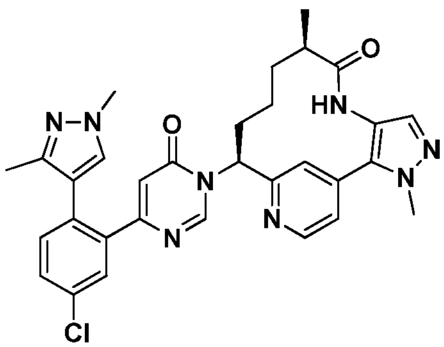

(9*R*,13*S*)-13-{4-[5-Chloro-2-(1,3-dimethyl-1H-pyrazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (11.3 mg, 49% yield) was prepared in a similar manner as the procedure described in Example 49, by replacing 1-methyl-4-(4,4,5,5-tetramethyl-1,3,2-dioxaborolan-2-yl)-1H-pyrazole with 1,3-dimethyl-4-(4,4,5,5-tetramethyl-1,3,2-dioxaborolan-2-yl)-1H-pyrazole (10.84 mg, 0.049 mmol). MS(ESI) *m/z*: 583.5 $(M+H)^+$. $^1$H NMR (400MHz, $CD_3OD$) $\delta$ 8.95 (s, 1H), 8.73 (d, *J*=5.1 Hz, 1H), 7.73 - 7.69 (m, 2H), 7.55 - 7.47 (m, 4H), 7.33 (d, *J*=8.4 Hz, 1H), 6.24 (d, *J*=0.7 Hz, 1H), 5.97 (dd, *J*=12.7, 4.3 Hz, 1H), 4.05 (s, 3H), 3.81 (s, 3H), 2.76 - 2.65 (m, 1H), 2.39 - 2.28 (m, 1H), 2.13 - 1.97 (m, 2H), 1.90 (s, 3H), 1.66 - 1.42 (m, 2H), 1.01 (d, *J*=6.8 Hz, 3H), 0.80 - 0.63 (m, 1H). Analytical HPLC (Method A): RT = 7.15 min, 99.6% purity; Factor XIa Ki = 270 nM, Plasma Kallikrein Ki = 5,200 nM.

Example 51

Preparation of (10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-4,5,8-triazatricyclo [13.3.1.0$^{2,7}$]nonadeca-1(19),2(7),3,5,15,17-hexaen-9-one

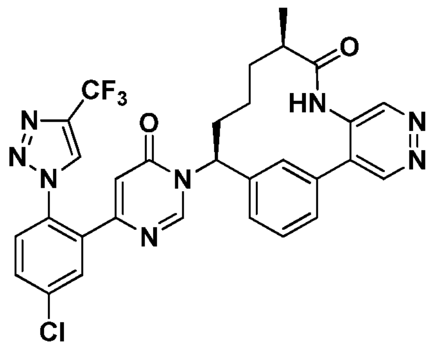

51A. Preparation of 5-bromopyridazin-4-amine

*tert*-Butyl N-(5-bromopyridazin-4-yl)carbamate (400 mg, 1.183 mmol) in DCM (15 mL) was added TFA (4.56 mL, 59.2 mmol). The reaction was stirred at rt overnight. Concentration gave 5-bromopyridazin-4-amine.trifluoroacetate as a dark brownish solid. MS(ESI) *m/z*: 174.2 $(M+H)^+$.

51B. Preparation of (10R,14S)-14-amino-10-methyl-4,5,8-triazatricyclo[13.3.1.0$^{2,7}$] nonadeca-1(19),2,4,6,15,17-hexaen-9-one

(10R,14S)-14-Amino-10-methyl-4,5,8-triazatricyclo[13.3.1.0$^{2,7}$]nonadeca-1(19),2,4,6,15,17-hexaen-9-one was prepared in a similar manner as the procedure described in Intermediate 38, by replacing 2-bromopyridin-3-amine with 5-bromopyridazin-4-amine. MS(ESI) *m/z:* 297.5 $(M+H)^+$.

51C. Preparation of (10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-4,5,8-triazatricyclo [13.3.1.0$^{2,7}$]nonadeca-1(19),2(7),3,5,15,17-hexaen-9-one

(10R,14S)-14-(4-{5-Chloro-2-[4-(trifluoromethyl)-1H-1,2,3-triazol-1-yl]phenyl}-6-oxo-1,6-dihydropyrimidin-1-yl)-10-methyl-4,5,8-triazatricyclo[13.3.1.0$^{2,7}$]nonadeca-1(19),2(7),3,5,15,17-hexaen-9-one trifluoroacetate (3.8 mg, 32.7% yield) was prepared in a similar manner as the procedure described in Example 56 by using (10R,14S)-14-amino-10-methyl-4,5,8-triazatricyclo[13.3.1.0$^{2,7}$]nonadeca-1(19),2,4,6,15,17-hexaen-9-one (4.5 mg, 0.015 mmol). $^1$H NMR (400MHz, $CD_3OD$) $\delta$ 9.42 (s, 1H), 9.14 (s, 1H), 8.82 - 8.77 (m, 1H), 8.42 (s, 1H), 7.90 - 7.84 (m, 1H), 7.81 (s, 1H), 7.77 - 7.72 (m, 1H), 7.70-7.65 (m, 1H), 7.65 - 7.61 (m, 2H), 7.30 - 7.24 (m, 1H), 6.45 (d, J=0.7 Hz, 1H), 5.77 (dd, J=12.9, 4.3 Hz, 1H), 2.70 - 2.59 (m, 1H), 2.36 - 2.23 (m, 1H), 2.13 - 2.00 (m, 1H), 2.00-1.89 (m, 1H), 1.67 - 1.35 (m, 2H), 1.26 - 1.13 (m, 1H), 1.07 (d, J=6.8 Hz, 3H). MS(ESI) *m/z*: 621.0 $(M+H)^+$. Analytical HPLC (Method A): RT = 8.24 min, purity = 100%; Factor XIa Ki = 5 nM, Plasma Kallikrein Ki = 18 nM.

Example 52

Preparation of 1-(4-chloro-2-{1-[(9R,13 S)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)-1H-pyrazole-4-carboxylic acid

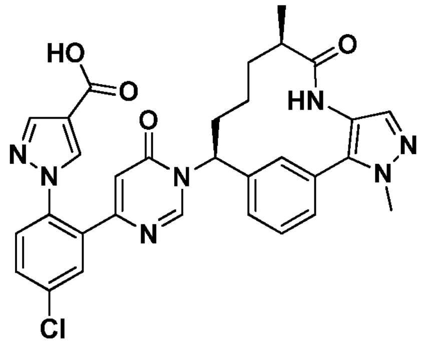

To a solution of ethyl 1-(4-chloro-2-{1-[(9R,13S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)-1H-pyrazole-4-carboxylate trifluoroacetate (7 mg, 0.011 mmol) in THF (56 μl) was added a solution of LiOH·$H_2O$ (4.7 mg, 0.112 mmol) in water (56 μl). To the resulting cloudy mixture was added MeOH (1 drop). The reaction was stirred vigorously at rt for 3.5 h. The solution was acidified to pH 5 with 1.0 N HCl and then purified by reverse phase chromatography to give 1-(4-chloro-2-{1-[(9R,13S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)-1H-pyrazole-4-carboxylic acid trifluoroacetate (0.0024 g, 30% yield) as a white solid. MS(ESI) *m*/*z*: 599.1 $(M+H)^+$. $^1H$ NMR (400MHz, $CD_3CN$) δ 8.73 - 8.66 (m, 2H), 8.17 (s, 1H), 7.94 (s, 1H), 7.81 (d, *J*=2.2 Hz, 1H), 7.73 - 7.62 (m, 3H), 7.61 - 7.55 (m, 1H), 7.47 - 7.38 (m, 2H), 6.16 (d, *J*=0.9 Hz, 1H), 5.98 (dd, *J*=12.7, 3.9 Hz, 1H), 4.02 (s, 3H), 2.65 (m, 1H), 2.30 - 2.19 (m, 1H), 2.15 - 2.02 (m, 1H), 1.64 - 1.39 (m, 2H), 0.98 (d, *J*=6.8 Hz, 3H), 0.61 (m, 1H). Analytical HPLC (Method A): SunFire, RT = 6.48 min, 99.3% purity; Factor XIa Ki = 5 nM, Plasma Kallikrein Ki = 2,400 nM.

Example 53

Preparation of (9R,13S)-13-[4-(2-bromo-5-chlorophenyl)-5-chloro-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one

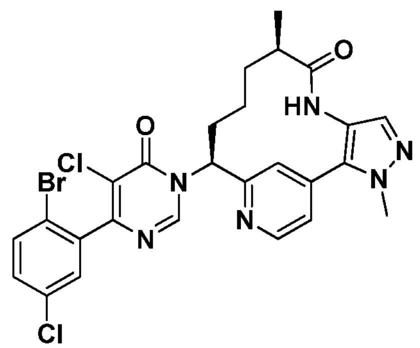

53A. Preparation of 6-(2-bromo-5-chlorophenyl)-5-chloropyrimidin-4-ol

To a suspension of 6-(2-bromo-5-chlorophenyl)pyrimidin-4-ol, prepared as described in Intermediate 44, (40 mg, 0.140 mmol) in MeCN (1401 μl) was added NCS (20.58 mg, 0.154 mmol). The reaction was heated at 60 °C for 4 h. The reaction mixture was concentrated and the crude residue was purified using normal phase chromatography to yield 6-(2-bromo-5-chlorophenyl)-5-chloropyrimidin-4-ol (42 mg, 94%) as a white solid. MS(ESI) *m*/*z*: 320.9 $(M+H)^+$.

53B. Preparation of (9R,13S)-13-[4-(2-bromo-5-chlorophenyl)-5-chloro-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one

(9R,13S)-13-[4-(2-Bromo-5-chlorophenyl)-5-chloro-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one trifluoroacetate (8.3 mg, 41.1% yield) was prepared in a similar manner as the procedure described in Example 56, by using 6-(2-bromo-5-chlorophenyl)-5-chloropyrimidin-4-ol (8.6 mg, 0.027 mmol) and (9*R*,13*S*)-13-amino-3,9-dimethyl-3,4,7,15-tetraazatricyclo[12.3.1.$0^{2,6}$]octadeca-1(18),2(6),4,14,16-pentaen-8-one(8mg, 0.027 mmol), prepared as described in Intermediate 32. MS(ESI) *m/z:* 603.0 $(M+H)^+$. $^1H$ NMR (400MHz, $CD_3OD$) $\delta$ 8.99 (s, 1H), 8.75 (d, J=5.1 Hz, 1H), 7.75 (s, 1H), 7.70 (d, J=8.6 Hz, 1H), 7.54 (dd, J=5.1, 1.5 Hz, 1H), 7.50 (s, 1H), 7.45 - 7.38 (m, 2H), 6.06 (dd, J=12.4, 4.1 Hz, 1H), 4.05 (s, 3H), 2.72 (td, J=6.7, 3.1 Hz, 1H), 2.45 - 2.31 (m, 1H), 2.19 - 2.03 (m, 2H), 1.70 - 1.43 (m, 2H), 1.02 (d, J=7.0 Hz, 3H), 0.73 (br. s., 1H). Analytical HPLC (Method A): RT = 9.24 min, 100% purity; Factor XIa Ki = 8 nM, Plasma Kallikrein Ki = 1,200 nM.

Example 54

Preparation of (9R,13S)-13-{4-[3-chloro-2-fluoro-6-(1,3-thiazol-5-yl)phenyl]-6-oxo-1,6-dihydropyrimidin-1-yl}-3-(difluoromethyl)-9-methyl-3,4,7,15-tetraazatricyclo [12.3.1.$0^{2,6}$] octadeca-1(18),2(6),4,14,16-pentaen-8-one

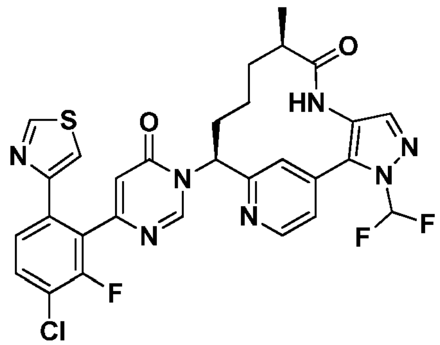

54A. Preparation of 4-(3-chloro-2-fluoro-6-iodophenyl)-6-methoxypyrimidine

4-Chloro-3-fluoro-2-(6-methoxypyrimidin-4-yl)aniline, prepared as described in Intermediate 10C (1 g, 3.94 mmol) in ACN (26.3 ml) was cooled to 0 °C and *p*TsOH.$H_2O$ (1.875 g, 9.86 mmol) was added followed by addition of $NaNO_2$ (0.544 g, 7.88 mmol) and NaI (1.477 g, 9.86 mmol) in water (13.14 ml). After 1 h, the reaction was warmed to rt and stirred overnight. After this time, the reaction was partially concentrated to remove the ACN and NaHCO3 was then added to neutralize the solution. The resulting solution was extracted with EtOAc. The combined organic layer was washed with sat $Na_2S_2O_3$ and brine, dried over $MgSO_4$, filtered, and concentrated to yield a solid, which was purified by normal phase chromatography to give 4-(3-chloro-2-fluoro-6-iodophenyl)-6-methoxypyrimidine (0.934 g, 65% yield). MS(ESI) *m/z*: 365.2 $(M+H)^+$.

54B. Preparation of 6-(3-chloro-2-fluoro-6-iodophenyl)pyrimidin-4-ol

6-(3-Chloro-2-fluoro-6-iodophenyl)pyrimidin-4-ol was prepared according to the procedures as described in Intermediate 4B for the synthesis of 6-(3-chloro-2,6-difluorophenyl)pyrimidin-4-ol, by replacing 4-(3-chloro-2,6-difluorophenyl)-6-methoxypyrimidine, prepared as described in Intermediate 4A, with 4-(3-chloro-2-fluoro-6-iodophenyl)-6-methoxypyrimidine. MS(ESI) *m/z*: 350.8 $(M+H)^+$.

54C. Preparation of (9R,13S)-13-[4-(3-chloro-2-fluoro-6-iodophenyl)-6-oxo-1,6-dihydropyrimidin-1-yl]-3-(difluoromethyl)-9-methyl-3,4,7,15-tetraazatricyclo[12.3.1.$0^{2,6}$] octadeca-1(18),2(6),4,14,16-pentaen-8-one

(9R,13S)-13-[4-(3-Chloro-2-fluoro-6-iodophenyl)-6-oxo-1,6-dihydropyrimidin-1-yl]-3-(difluoromethyl)-9-methyl-3,4,7,15-tetraazatricyclo[12.3.1.$0^{2,6}$]octadeca-1(18),2(6),4,14,16-pentaen-8-one was prepared in a similar manner as the procedure described in Example 56, using 6-(3-chloro-2-fluoro-6-iodophenyl)pyrimidin-4-ol (62.7 mg, 0.179 mmol) and (9R,13S)-13-amino-3-(difluoromethyl)-9-methyl-3,4,7,15-tetraazatricyclo[12.3.1.$0^{2,6}$]octadeca-1(18),2(6),4,14,16-pentaen-8-one (60 mg, 0.179 mmol), prepared as described in Intermediate 30. MS(ESI) *m/z*: 667.1 $(M+H)^+$.

54D. Preparation of (9R,13S)-13-{4-[3-chloro-2-fluoro-6-(1,3-thiazol-5-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-one

To a microwave tube was added (9R,13S)-13-[4-(3-chloro-2-fluoro-6-iodophenyl)-6-oxo-1,6-dihydropyrimidin-1-yl]-3-(difluoromethyl)-9-methyl-3,4,7,15-tetraazatricyclo[12.3.1.$0^{2,6}$]octadeca-1(18),2(6),4,14,16-pentaen-8-one (20 mg, 0.030 mmol), 5-(4,4,5,5-tetramethyl-1,3,2-dioxaborolan-2-yl)thiazole (9.47 mg, 0.045 mmol), $K_3PO_4$ (29.9 μl, 0.090 mmol) and THF (299 μl). The solution was bubbled through with Ar for several min then (DtBPF)$PdCl_2$ (0.974 mg, 1.495 μmol) was added. The reaction was sealed and heated at 90 °C overnight. The solution was cooled to rt and Ar was again bubbled through the solution for several minutes and additional 5-(4,4,5,5-tetramethyl-1,3,2-dioxaborolan-2-yl)thiazole (9.47 mg, 0.045 mmol) and $Pd(PPh_3)_4$ (3.46 mg, 2.99 μmol) were added. The solution was heated in a microwave at 120 °C for 30 min. The solution was then filtered and the residue was purified by reverse phase chromatography to give (9R,13S)-13-{4-[3-chloro-2-fluoro-6-(1,3-thiazol-5-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-one (0.67 mg, 2.9%). $^1$H NMR (400MHz, $CD_3OD$) δ 9.02 (s, 1H), 8.95 (s, 1H), 8.77 (d, *J*=5.1 Hz, 1H), 7.80 (s, 1H), 7.74 (s, 1H), 7.72 - 7.60 (m, 3H), 7.59 - 7.50 (m, 2H), 7.46 (dd, *J*=8.4, 1.3 Hz, 1H), 6.55 (s, 1H), 6.05 (dd, *J*=12.9, 4.3 Hz, 1H), 2.71 (dt, *J*=6.6, 3.3 Hz, 1H), 2.40 - 2.26 (m, 1H), 2.12 - 1.97 (m, 2H), 1.70 - 1.41 (m, 2H), 1.00 (d, *J*=7.0 Hz, 3H), 0.66 (br. s., 1H). MS(ESI) *m/z*: 625.9 $(M+H)^+$. Analytical HPLC (Method A): RT = 8.51 min, purity = 96.4%; Factor XIa Ki = 1.7 nM, Plasma Kallikrein Ki = 230 nM.

Example 56 (reference)

Preparation of (9*R*,13*S*)-13-(4-{5-chloro-2-[4-(trifluoromethyl)-1H-1,2,3-triazol-1-yl] phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one trifluoroacetate

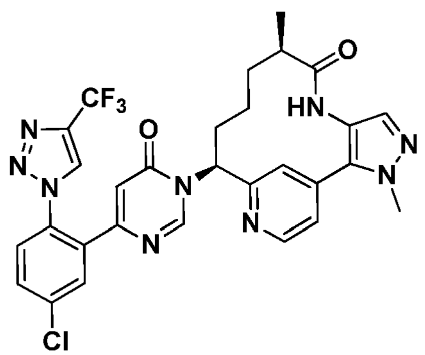

To a scintillation vial containing 6-{5-chloro-2-[4-(trifluoromethyl)-1H-1,2,3-triazol-1-yl]phenyl}pyrimidin-4-ol (22.8 mg, 0.067 mmol), prepared as described in Intermediate 15, HATU (33.0 mg, 0.087 mmol) in anhydrous ACN (0.5 mL) was added DBU (15 mL, 0.100 mmol). After 30 min, a solution of (9R,13S)-13-amino-3,9-dimethyl-3,4,7,15-tetraazatricyclo[12.3.1.$0^{2,6}$]octadeca-1(18),2(6),4,14,16-pentaen-8-one (20 mg, 0.067 mmol), prepared as described in Intermediate 32, in 0.5ml $CH_3CN$ and DMF (0.1 ml) was added. The resulting solution was stirred at rt for 2 h then purified by reverse phase chromatography to give (9*R*,13*S*)-13-(4-{5-chloro-2-[4-(trifluoromethyl)-1H-1,2,3-triazol-1-yl]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one trifluoroacetate (26.98 mg, 53.1% yield) as a white solid. MS(ESI) *m/z*: 624.3 $(M+H)^+$. $^1$H NMR (400MHz, $CD_3OD$) d 8.81 (d, *J*=0.7 Hz, 1H), 8.75 (s, 1H), 8.70 (d, *J*=5.3 Hz, 1H), 7.89 (d, *J*=2.4 Hz, 1H), 7.77 - 7.72 (m, 1H), 7.72 - 7.66 (m, 2H), 7.53 (dd, *J*=5.1, 1.5 Hz, 1H), 7.49 (s, 1H), 6.43 (s, 1H), 6.02 - 5.93 (m, 1H), 4.04 (s, 3H), 2.70 (td, *J*=6.7, 3.3 Hz, 1H), 2.27 (tt, *J*=12.7, 4.4 Hz, 1H), 2.12 - 1.94 (m, 2H), 1.66 - 1.52 (m, 1H), 1.45 (ddd, *J*=15.0, 9.8, 5.0 Hz, 1H), 1.00 (d, *J*=7.0 Hz, 3H), 0.69 (br. s., 1H). $^{19}$F NMR (376MHz, $CD_3OD$) d -62.54 (s), -77.44 (s). Analytical HPLC (Method A): RT = 11.02 min, purity = 96.7%; Factor XIa Ki = 1.4 nM, Plasma Kallikrein Ki = 24 nM.

Example 61

Preparation of (9*R*,13*S*)-13-{4-[3-chloro-6-(difluoromethyl)-2-fluorophenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate

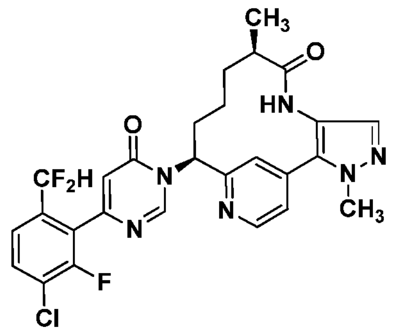

61A. Preparation of 2-(3-chloro-6-(difluoromethyl)-2-fluorophenyl)-4,4,5,5-tetramethyl-1,3,2-dioxaborolane

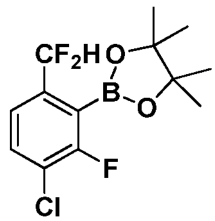

To a dry RBF was added 1-chloro-4-(difluoromethyl)-2-fluorobenzene (180 mg, 0.997 mmol) and THF (3 mL). The reaction was cooled to -78 °C and 2 M LDA in THF (0.498 mL, 0.997 mmol) was added dropwise. The reaction turned to dark red immediately after the addition. The reaction was stirred at -78 °C for 5 min and 2-isopropoxy-4,4,5,5-tetramethyl-1,3,2-dioxaborolane (371 mg, 1.994 mmol) was added in one portion. The reaction was stirred at -78 °C for 20 min. The colored changed to pale yellow. The reaction was partitioned between EtOAc (30 mL) and water (20 mL). The organic layer was separated, washed with water (20 mL) and brine (20 mL), dried over $MgSO_4$, filtered and concentrated. The residue was purified using ISCO system (0-30% EtOAc/Hex gradient) to give 2-(3-chloro-6-(difluoromethyl)-2-fluorophenyl)-4,4,5,5-tetramethyl-1,3,2-dioxaborolane (125 mg, 0.408 mmol, 40.9% yield) as a light brown oil. $^1H$ NMR (400MHz, $CDCl_3$) $\delta$ 7.45 (t, *J*=7.8 Hz, 1H), 7.29 (d, *J*=8.4 Hz, 1H), 7.10 - 6.74 (m, 1H), 1.31 (s, 12H).

61B. Preparation of 4-(3-chloro-6-(difluoromethyl)-2-fluorophenyl)-6-methoxypyrimidine

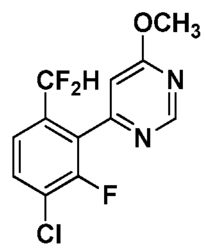

To a microwave vial was added 2-(3-chloro-6-(difluoromethyl)-2-fluorophenyl)-4,4,5,5-tetramethyl-1,3,2-dioxaborolane (120 mg, 0.391 mmol), 4-chloro-6-methoxypyrimidine (56.6 mg, 0.391 mmol), toluene (2 mL), EtOH (1 mL) and 2 M $Na_2CO_3$ (0.587 mL, 1.174 mmol). The reaction was purged with Ar and $Pd(PPh_3)_4$ (45 mg, 0.039 mmol) was

added. The reaction was sealed and stirred in a microwave at 120 °C for 1 h. The reaction was partitioned between EtOAc (20 mL) and water (20 mL). The organic layer was separated, washed with water (10 mL) and brine (15 mL), dried over $MgSO_4$, filtered and concentrated. The residue was purified using ISCO system (0-30% EtOAc/Hex gradient) to give 4-(3-chloro-6-(difluoromethyl)-2-fluorophenyl)-6-methoxypyrimidine (40 mg, 0.139 mmol, 35.4% yield) as a white solid. $^1H$ NMR (400MHz, $CDCl_3$) δ 8.88 (d, *J*=0.9 Hz, 1H), 7.64 - 7.57 (m, 1H), 7.56 - 7.50 (m, 1H), 7.11 - 6.76 (m, 2H), 4.06 (s, 3H).

61C. Preparation of 6-(3-chloro-6-(difluoromethyl)-2 fluorophenyl)pyrimidin-4-ol

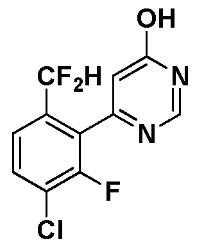

To a RBF was added 4-(3-chloro-6-(difluoromethyl)-2-fluorophenyl)-6-methoxypyrimidine (40 mg, 0.139 mmol), AcOH (0.5 mL) and 48% HBr (0.784 mL, 6.93 mmol). The reaction was stirred at 85 °C for 45 min. Then toluene (25 mL) was added and the reaction was concentrated. The residue was then partitioned between EtOAc (25 mL) and sat aq NaHCO3 (25 mL). The organic layer was separated, washed with water (15 mL) and brine (15 mL), dried over $MgSO_4$, filtered and concentrated. The residue was purified using ISCO system (0-100% EtOAc/Hex gradient) to give 6-(3-chloro-6-(difluoromethyl)-2-fluorophenyl)pyrimidin-4-ol (36 mg, 0.131 mmol, 95% yield) as a white solid. $^1H$ NMR (400MHz, $CDCl_3$) δ 13.25 (br. s., 1H), 8.28 (s, 1H), 7.70 - 7.57 (m, 1H), 7.53 (d, *J*=8.4 Hz, 1H), 7.16 - 6.79 (m, 1H), 6.72 (br. s., 1H).

61D. Preparation of (9*R*,13*S*)-13-{4-[3-chloro-6-(difluoromethyl)-2-fluorophenyl]-6-oxo-1,6-dihydropyrimidin-1-yl}-3,9-dimethyl-3,4,7,15-tetraazatricyclo[12.3.1.0$^{2,6}$] octadeca-1(18),2(6),4,14,16-pentaen-8-one trifluoroacetate

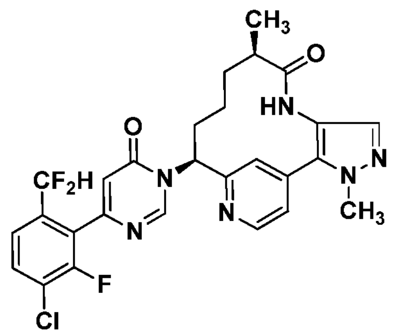

(9R,13S)-13-{4-[3-Chloro-6-(difluoromethyl)-2-fluorophenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (3.1 mg, 4.44 μmol, 7.38% yield) was prepared in a similar manner as the procedure described in Example 56 using 6-(3-chloro-6-(difluoromethyl)-2-fluorophenyl)pyrimidin-4-ol (16.51 mg, 0.060 mmol). $^1H$ NMR (400MHz, $CD_3OD$) δ 8.78 (d, *J*=5.3 Hz, 1H), 7.80 - 7.74 (m, 2H), 7.60 (d, *J*=8.6 Hz, 1H), 7.56 (dd, *J*=5.3, 1.5 Hz, 1H), 7.53 (s, 1H), 7.16 - 6.84 (m, 1H), 6.67 (s, 1H), 6.08 (dd, *J*=12.8, 4.2 Hz, 1H), 4.08 (s, 3H), 2.75 (td, *J*=6.8, 3.2 Hz, 1H), 2.40 (tt, *J*=12.7, 4.6 Hz, 1H), 2.19 - 2.05 (m, 2H), 1.71 - 1.60 (m, 1H), 1.53 (ddd, *J*=15.0, 9.9, 5.5 Hz, 1H), 1.05 (d, *J*=7.0 Hz, 3H), 0.76 (br. s., 1H); MS(ESI) *m/z*: 557.1 $(M+H)^+$. Analytical HPLC (Method A): RT = 7.516 min, purity = 96.0%; Factor XIa Ki = 2.5 nM, Plasma Kallikrein Ki = 45 nM.

Example 62

Preparation of (9*R*,13*S*)-13-{4-[3-chloro-6-(1,1-difluoroethyl)-2-fluorophenyl]-6-oxo-1,6-ihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate

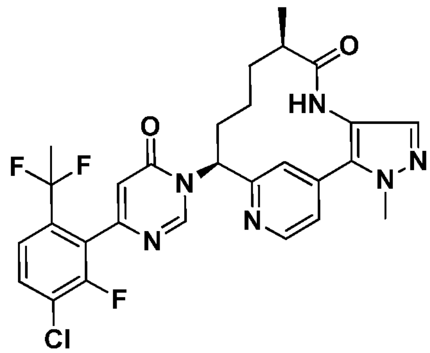

62A. Preparation of 1-chloro-4-(1,1-difluoroethyl)-2-fluorobenzene

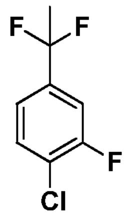

To a sealed tube was added 1-(4-chloro-3-fluorophenyl)ethanone (1 g, 5.79 mmol), $CH_2Cl_2$ (10 mL) and DAST (2.297 mL, 17.38 mmol). The reaction was sealed and stirred at 45 °C for 8 h. The reaction was carefully quenched with cold sat $NaHCO_3$ over 30 min until the pH was greater than 7. The organic layer was separated, washed with water, dried over $MgSO_4$, filtered and concentrated. The reside was purified using ISCO system (0-10% EtOAC/Hex gradient) to give 1-chloro-4-(1,1-difluoroethyl)-2-fluorobenzene (300 mg, 1.54 mmol, 26.6% yield) as a light brown liquid. $^1$H NMR (400MHz, $CDCl_3$) δ 7.49 - 7.42 (m, 1H), 7.32 - 7.27 (m, 1H), 7.25 - 7.20 (m, 1H), 1.90 (t, *J*=18.2 Hz, 3H).

62B. Preparation of 2-(3-chloro-6-(1,1-difluoroethyl)-2-fluorophenyl)-4,4,5,5-tetramethyl-1,3,2-dioxaborolane

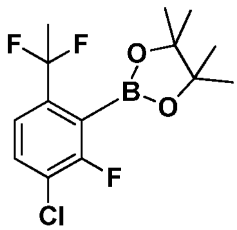

To a dry RBF was added 1-chloro-4-(1,1-difluoroethyl)-2-fluorobenzene (230 mg, 1.182 mmol) and THF (3 mL). The reaction was cooled to -78 °C and 2 M LDA solution (0.71 mL, 1.418 mmol) was added dropwise. The reaction turned to red after the addition. The reaction was stirred at -78 °C for 5 min and then 2-isopropoxy-4,4,5,5-tetramethyl-1,3,2-dioxaborolane (440 mg, 2.364 mmol) was added in one portion. The reaction was stirred at -78 °C for additional 20 min. The color changed to pale yellow. The reaction was partitioned between EtOAc (30 mL) and water (20 mL). The organic layer was separated, washed with water (20 mL) and brine (20 mL), dried over $MgSO_4$, filtered and concentrated.

The residue was purified using ISCO system (0-30% EtOAc/Hex gradient) to give 2-(3-chloro-6-(1,1-difluoroethyl)-2-fluorophenyl)-4,4,5,5-tetramethyl-1,3,2-dioxaborolane (330 mg, 1.030 mmol, 87% yield) as a clear oil. $^1$H NMR (400MHz, $CDCl_3$) δ 7.43 (t, *J*=7.9 Hz, 1H), 7.18 (d, *J*=8.4 Hz, 1H), 1.93 (t, *J*=18.3 Hz, 3H), 1.38 (s, 12H).

62C. Preparation of 4-(3-chloro-6-(1,1-difluoroethyl)-2-fluorophenyl)-6-methoxypyrimidine

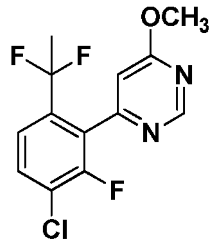

To a microwave vial was added 2-(3-chloro-6-(1,1-difluoroethyl)-2-fluorophenyl)-4,4,5,5-tetramethyl-1,3,2-dioxaborolane (325 mg, 1.014 mmol), 4-chloro-6-methoxypyrimidine (147 mg, 1.014 mmol), toluene (4 mL), EtOH (2 mL) and 2 M $Na_2CO_3$ (1.52 mL, 3.04 mmol). The reaction was purged with Ar and $Pd(PPh_3)_4$ (116.7 mg, 0.101 mmol) was added. The reaction was sealed and stirred in microwave at 120 °C for 1 h. The reaction was partitioned between EtOAc (20 mL) and water (20 mL). The organic layer was separated, washed with water (10 mL) and brine (15 mL), dried over $MgSO_4$, filtered and concentrated. The residue was purified using ISCO system (0-30% EtOAc/Hex gradient) to give 4-(3-chloro-6-(1,1-difluoroethyl)-2-fluorophenyl)-6-methoxypyrimidine (40 mg, 0.132 mmol, 13.03% yield) as a white solid. $^1$H NMR (400MHz, $CDCl_3$) δ 8.84 (d, *J*=1.1 Hz, 1H), 7.52 (d, *J*=7.3 Hz, 1H), 7.36 (dd, *J*=8.6, 1.3 Hz, 1H), 6.81 (s, 1H), 4.05 (s, 3H), 1.91 (t, *J*=18.6 Hz, 3H); MS(ESI) *m*/*z*: 303.0, 305.0 $(M+H)^+$.

62D. Preparation of 6-(3-chloro-6-(1,1-difluoroethyl)-2-fluorophenyl)pyrimidin-4-ol

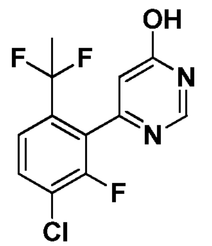

To a RBF was added 4-(3-chloro-6-(1,1-difluoroethyl)-2-fluorophenyl)-6-methoxypyrimidine (35 mg, 0.116 mmol), AcOH (0.5 mL) and HBr (0.654 mL, 5.78 mmol). The reaction was stirred at 85 °C for 45 min. Then toluene (25 mL) was added and the reaction was concentrated. The residue was partitioned between EtOAc (25 mL) and sat NaHCO3 (25 mL). The organic layer was separated, washed with water (15 mL) and brine (15 mL), dried over $MgSO_4$, filtered and concentrated. The residue was purified using ISCO system (0-100% EtOAc/Hex gradient) to give 6-(3-chloro-6-(1,1-difluoroethyl)-2-fluorophenyl)pyrimidin-4-ol (28 mg, 0.097 mmol, 84% yield) as a white solid. MS(ESI) *m*/*z*: 289, 291.0 $(M+H)^+$.

62E. Preparation of (9R,13S)-13-{4-[3-chloro-6-(1,1-difluoroethyl)-2-fluorophenyl]-6-oxo-1,6-ihydropyrimidin-1-yl}-3,9-dimethyl-3,4,7,15-tetraazatricyclo[12.3.1.0$^{2,6}$] octadeca-1(18),2(6),4,14,16-pentaen-8-one trifluoroacetate

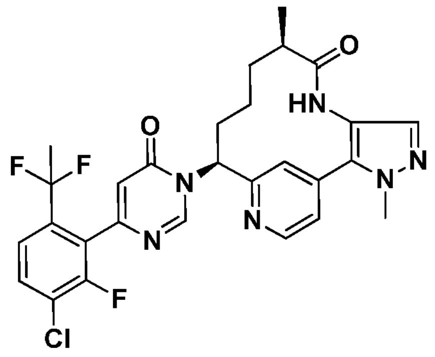

(9R,13S)-13-{4-[3-Chloro-6-(1,1-difluoroethyl)-2-fluorophenyl]-6-oxo-1,6-ihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (30 mg, 0.042 mmol, 69.9% yield) was prepared in a similar manner as the procedure described in Example 56 by replacing 6-{5-chloro-2-[4-(trifluoromethyl)-1H-1,2,3-triazol-1-yl]phenyl}pyrimidin-4-ol with 6-(3-chloro-6-(1,1-difluoroethyl)-2-fluorophenyl)pyrimidin-4-ol (22.2 mg, 0.06 mmol). $^1$H NMR (400MHz, $CD_3OD$) δ 8.99 (br. s., 1H), 8.74 (d, *J*=4.2 Hz, 1H), 7.78 (s, 1H), 7.65 (t, *J*=7.8 Hz, 1H), 7.58 (d, *J*=3.7 Hz, 1H), 7.50 - 7.39 (m, 2H), 6.53 (s, 1H), 6.01 (d, *J*=9.9 Hz, 1H), 4.02 (s, 3H), 2.67 (br. s., 1H), 2.43 - 2.31 (m, 1H), 2.06 (br. s., 2H), 1.89 (t, *J*=18.6 Hz, 3H), 1.66 - 1.53 (m, 1H), 1.45 (br. s., 1H), 0.98 (d, *J*=6.8 Hz, 3H), 0.73 (br. s., 1H); MS(ESI) *m/z*: 636.2 (M+H)$^+$. Analytical HPLC (Method A): RT = 11.078 min, purity = 96.0%; Factor XIa Ki = 16 nM, Plasma Kallikrein Ki = 240 nM.

Example 64

Preparation of (9*R*,13*S*)-13-[4-(3-chloro-2,6-difluorophenyl)-6-oxo-1,6-dihydropyrimidin-1-yl]-16-fluoro-3,9-dimethyl-3,4,7-triazatricyclo [12.3.1.0$^{2,6}$]octadeca-1(18),2(6),4,14,16-pentaen-8-one

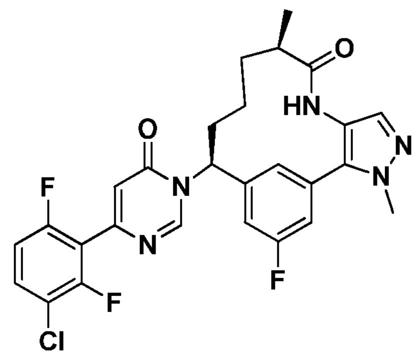

To a solution of 6-(3-chloro-2,6-difluorophenyl)pyrimidin-4-ol hydrobromide (0.017 g, 0.053 mmol), prepared as described in Intermediate 4, in $CH_3CN$ (1 ml), was added HATU (0.026 g, 0.068 mmol) and DBU (0.028 mL, 0.184 mmol). After 1 h, (9*R*,13*S*)-13-amino-16-fluoro-3,9-dimethyl-3,4,7-triazatricyclo[12.3.1.0$^{2,6}$]octadeca-1(18),2(6),4,14,16-pentaen-8-one, (0.019 g, 0.053 mmol), prepared as described in Intermediate 29, was added. After 18 h, the reaction was diluted with DMF, filtered and concentrated. The residue was purified by reverse phase HPLC, then preparative LCMS to give (9*R*,13*S*)-13-[4-(3-chloro-2,6-difluorophenyl)-6-oxo-1,6-dihydropyrimidin-1-yl]-16-fluoro-3,9-dimethyl-3,4,7-triazatricyclo[12.3.1.0$^{2,6}$]octadeca-1(18),2(6),4,14,16-pentaen-8-one (3.4 mg, 11.9%). MS(ESI) *m/z*: 542.1 (M+H)$^+$. $^1$H NMR ((500MHz, DMSO-$d_6$) δ 9.23 (s, 1H), 8.69 (br. s., 1H), 7.84 - 7.73 (m, 1H), 7.61 - 7.50 (m, 2H), 7.45 (s, 1H), 7.38 - 7.32 (m, 1H), 7.29 - 7.19 (m, 1H), 6.74 (s, 1H), 5.66 (d, *J*=12.5 Hz, 1H), 3.98 (s, 3H), 2.42 (br. s., 1H), 2.10 - 1.97 (m, 1H), 1.88 (br. s., 1H), 1.45 (d, *J*=7.3 Hz, 1H), 1.21 (br. s., 1H), 1.11 (br. s., 1H), 0.97 (d, *J*=6.4 Hz, 3H). Analytical HPLC (Method C) RT = 1.50 min., purity = 99%; Factor XIa Ki = 26 nM, Plasma Kallikrein Ki = 450 nM.

Example 69

Preparation of (10*R*,14*S*)-14-{4-[3-chloro-6-(4-chloro-1*H*-1,2,3-triazol-1-yl)-2-fluorophenyl]-6-oxo-1,6-dihydropyrimidin-1-yl}-10-methyl-3,8-diazatricyclo [13.3.1.0$^{2,7}$]nonadeca-1(19),2(7),3,5,15,17-hexaen-9-one trifluoroacetate

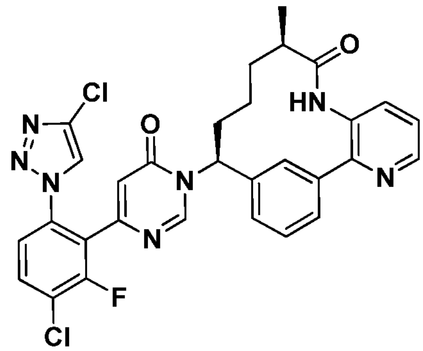

To a solution of 6-[3-chloro-6-(4-chloro-1*H*-1,2,3-triazol-1-yl)-2-fluorophenyl] pyrimidin-4-ol (4.42 mg, 0.014 mmol), prepared as described in Intermediate 10, in $CH_3CN$ (0.2 ml) was added HATU (6.69 mg, 0.018 mmol) and DBU (3.06 µl, 0.020 mmol). After 30 min, (10*R*,14*S*)-14-amino-10-methyl-3,8-diazatricyclo[13.3.1.0$^{2,7}$] nonadeca-1(19),2(7),3,5,15,17-hexaen-9-one (0.004 g, 0.014 mmol), prepared as described in Intermediate 38, was added with DMF (0.2 ml). After 18 h, the reaction was diluted with DMF, filtered and concentrated. The residue was purified by reverse phase HPLC using PHENOMENEX® Luna 5U 30x100mm (10:90 ACN/$H_2O$ to 90:10 ACN/$H_2O$, 0.1% TFA) (20% B start, 14 min gradient) to afford (10*R*,14*S*)-14-{4-[3-chloro-6-(4-chloro-1*H*-1,2,3-triazol-1-yl)-2-fluorophenyl]-6-oxo-1,6-dihydropyrimidin-1-yl}-10-methyl-3,8-diazatricyclo[13.3.1.0$^{2,7}$]nonadeca-1(19),2(7),3,5,15,17-hexaen-9-one trifluoroacetate (2 mg, 20% yield) as a white solid. MS(ESI) *m/z:* 604.4 $(M+H)^+$. $^1$H NMR (400MHz, $CD_3OD$) δ 8.69 - 8.65 (m, 1H), 8.38 - 8.30 (m, 2H), 8.00 - 7.91 (m, 2H), 7.91 - 7.84 (m, 1H), 7.74 (d, *J*=7.9 Hz, 1H), 7.69 - 7.63 (m, 2H), 7.55 (dd, *J*=8.6, 1.5 Hz, 1H), 7.36 (d, *J*=7.7 Hz, 1H), 6.64 (s, 1H), 5.83 (dd, *J*=12.9, 3.4 Hz, 1H), 2.55 (t, *J*=6.7 Hz, 1H), 2.40 - 2.29 (m, 1H), 2.21 - 2.10 (m, 1H), 1.89 (br. s., 1H), 1.65 - 1.51 (m, 2H), 1.33 (d, *J*=9.7 Hz, 1H), 1.15 (d, *J*=6.8 Hz, 3H). Analytical HPLC (Method A) RT = 6.32 min. Purity = 100%; Factor XIa Ki = 1.1 nM, Plasma Kallikrein Ki = 54 nM.

Example 76

Preparation of (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo [13.3.1.0$^{2,7}$]nonadeca-1(19),2(7),3,5,15,17-hexaen-9-one

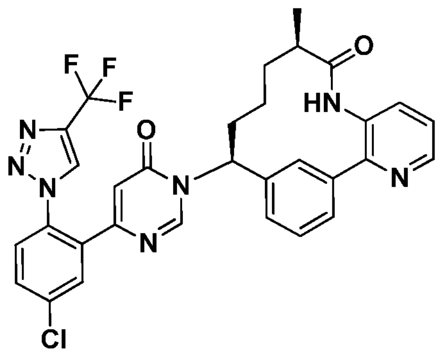

To a solution of 6-{5-chloro-2-[4-(trifluoromethyl)-1*H*-1,2,3-triazol-1-yl]phenyl} pyrimidin-4-ol (0.035 g, 0.102 mmol), prepared as described in Intermediate 15, in $CH_3CN$ (0.4 ml) was added HATU (0.050 g, 0.132 mmol) and DBU (0.023 mL, 0.152 mmol). After 30 min, (10*R*,14*S*)-14-amino-10-methyl-3,8-diazatricyclo[13.3.1.0$^{2,7}$] nonadeca-1(19),2(7),3,5,15,17-hexaen-9-one (0.030 g, 0.102 mmol), prepared as described in Intermediate 38, was added with

DMF (0.6 ml). After 18 h, the reaction was diluted with DMF, filtered and concentrated. The residue was purified by preparative LCMS using (5:95 ACN/$H_2O$ to 95:5 ACN/$H_2O$, 0.1% TFA) to afford (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.$0^{2,7}$]nonadeca-1(19),2(7),3,5,15,17-hexaen-9-one (8.6 mg, 11.1%). MS(ESI) *m*/*z*: 620.08 $(M+H)^+$. $^1H$ NMR (400MHz, $CD_3OD$) δ 8.81 - 8.72 (m, 1H), 8.59 (d, *J*=4.3 Hz, 1H), 8.16 (s, 1H), 7.88 - 7.83 (m, 2H), 7.78 - 7.65 (m, 4H), 7.58 - 7.53 (m, 1H), 7.53 - 7.48 (m, 1H), 7.28 (d, *J*=7.6 Hz, 1H), 6.43 (s, 1H), 5.88 - 5.72 (m, 1H), 2.49 (br. s., 1H), 2.27 (d, *J*=10.4 Hz, 1H), 2.10 (d, *J*=10.1 Hz, 1H), 1.85 (d, *J*=8.9 Hz, 1H), 1.55 (d, *J*=9.8 Hz, 2H), 1.28 (br. s., 1H), 1.13 (d, *J*=6.7 Hz, 3H). Analytical HPLC (Method C) RT = 1.55 min, purity = 97%; Factor XIa Ki = 1.7 nM, Plasma Kallikrein Ki = 130 nM.

Example 80

Preparation of (10*R*,14*S*)-14-(4-{5-chloro-2-[4-(trifluoromethyl)-1*H*-1,2,3-triazol-1-yl] phenyl}-6-oxo-1,6-dihydropyrimidin-1-yl)-4,10-dimethyl-3,8-diazatricyclo [13.3.1.$0^{2,7}$]nonadeca-1(19),2(7),3,5,15,17-hexaen-9-one

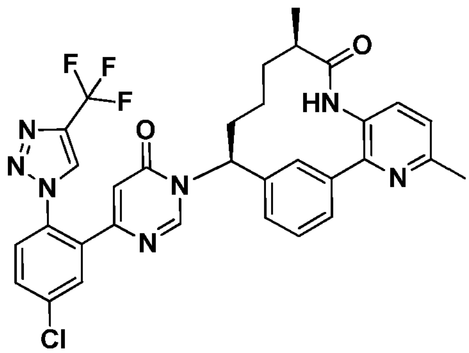

80A. Preparation of *tert*-butyl *N*-[(1S)-1-[3-(5,5-dimethyl-1,3,2-dioxaborinan-2-yl) phenyl]but-3-en-1-yl]carbamate

To a RBF was added *tert*-butyl *N*-[(1S)-1-(3-bromophenyl)but-3-en-1-yl] carbamate (2.6 g, 7.97 mmol), 5,5,5',5'-tetramethyl-2,2'-bi(1,3,2-dioxaborinane (1.980 g, 8.77 mmol), and KOAc (2.347 g, 23.91 mmol) in dioxane (35 ml). The mixture was purged with Ar for 10 min, then $PdCl_2$(dppf)-DCM adduct (0.325 g, 0.398 mmol) was added and the reaction was stirred at 90 °C for 4 h. The reaction was partitioned between EtOAc (50 ml) and water (40 ml). The organic layer was separated, washed with water (15 ml), brine (30 ml), dried over $MgSO_4$, filtered and concentrated. The residue was purified by normal phase chromatography using hexanes and EtOAc as eluents to afford *tert*-butyl *N*-[(1S)-1-[3-(5,5-dimethyl-1,3,2-dioxaborinan-2-yl)phenyl]but-3-en-1-yl]carbamate (2.62 g, 92%) as a white solid. MS(ESI) *m*/*z*: 292.08 $(M+H)^+$.

80B. Preparation of *tert*-butyl *N*-[(1S)-1-[3-(3-amino-6-methylpyridin-2-yl)phenyl]but-3-en-1-yl]carbamate

*tert*-Butyl *N*-[(1S)-1-[3-(5,5-dimethyl-1,3,2-dioxaborinan-2-yl)phenyl]but-3-en-1-yl]carbamate (0.67 g, 1.865 mmol), 2-bromo-6-methylpyridin-3-amine (0.349 g, 1.865 mmol), and 2 M aq $Na_2CO_3$ (4 mL, 8.00 mmol) were added to dioxane (9 ml) and the solution was purged with a stream of Ar for 10 min. $Pd(PPh_3)_4$ (0.108 g, 0.093 mmol) was added and the mixture was irradiated in a microwave at 120 °C for 30 min. The reaction was quenched with water (20 ml) and extracted with EtOAc (3 x 30 ml). The combined organic layers were washed with brine (15 ml), dried ($MgSO_4$), filtered and concentrated. The residue was purified by normal phase chromatography using DCM and 0-10% MeOH as eluents to afford *tert*-butyl *N*-[(1*S*)-1-[3-(3-amino-6-methylpyridin-2-yl)phenyl]but-3-en-1-yl]carbamate (0.94g, 100%, 70% purity) as a brown oil. MS(ESI) *m*/*z*: 354.5 $(M+H)^+$.

80C. Preparation of *tert*-butyl *N*-[(1S)-1-(3-{6-methyl-3-[(2*R*)-2-methylbut-3-enamido] pyridin-2-yl}phenyl)but-3-en-1-yl]carbamate

To a solution of *tert*-butyl *N*-[(1*S*)-1-[3-(3-amino-6-methylpyridin-2-yl)phenyl] but-3-en-1-yl]carbamate (0.65 g, 1.83 mmol) in EtOAc (0.58 ml), was added (R)-2-methylbut-3-enoic acid (0.239 g, 2.391 mmol), prepared as described in Intermediate 2, in 0.3 ml EtOAc. The resulting solution was cooled to 0 °C and pyridine (0.446 ml, 5.52 mmol) and a 50% EtOAc solution of T3P® (2.189 ml, 3.68 mmol) were added. After 3 h, the reaction was quenched with sat $NaHCO_3$

(15 ml) and extracted with EtOAc (3 x 20 ml). The combined organic layers were washed with brine (15 ml) and dried ($MgSO_4$). The mixture was filtered and concentrated and the residue was purified by normal phase chromatography using DCM and 0-10% MeOH as eluents to afford *tert*-butyl *N*-[(1S)-1-(3-{6-Methyl-3-[(2*R*)-2-methylbut-3-enamido]pyridin-2-yl}phenyl)but-3-en-1-yl] carbamate (0.65 g, 82%) as a tan foam. MS(ESI) *m/z*: 436.08 $(M+H)^+$. $^1H$ NMR (500MHz, $CDCl_3$) δ 8.56 (d, *J*=8.5 Hz, 1H), 7.54 - 7.46 (m, 2H), 7.42 (s, 1H), 7.37 (d, *J*=1.4 Hz, 2H), 7.17 (d, *J*=8.5 Hz, 1H), 5.90 - 5.67 (m, 2H), 5.22 - 5.03 (m, 4H), 5.00-4.75 (m, 3H), 3.05 (t, *J*=7.3 Hz, 1H), 2.62 (br. s. and m, 4H), 1.50 - 1.39 (m, 9H), 1.28 (d, *J*=7.2 Hz, 3H).

80D. Preparation of *tert*-butyl *N*-[(10*R*,11E,14*S*)-4,10-dimethyl-9-oxo-3,8-diazatricyclo [13.3.1.0$^{2,7}$]nonadeca-1(19),2(7),3,5,11,15,17-heptaen-14-yl]carbamate

To a solution of *tert*-butyl *N*-[(1S)-1-(3-{6-methyl-3-[(2*R*)-2-methylbut-3-enamido]pyridin-2-yl}phenyl)but-3-en-1-yl]carbamate (0.2 g, 0.459 mmol) in degassed DCE (20 ml) was added Second Generation Grubbs Catalyst (0.156 g, 0.184 mmol) and the resulting reaction mixture was heated to 120 °C for 30 min in a microwave. The reaction mixture was directly purified by normal phase chromatography using DCM and 0-10% MeOH as eluents to afford *tert*-butyl *N*-[(10*R*,11E,14*S*)-4,10-dimethyl-9-oxo-3,8-diazatricyclo[13.3.1.0$^{2,7}$]nonadeca-1(19),2(7),3,5,11,15,17-heptaen-14-yl]carbamate (0.19g, 100%) as a dark solid. MS(ESI) *m/z*: 408.08 $(M+H)^+$.

80E. Preparation of (10*R*,14*S*)-14-amino-4,10-dimethyl-3,8-diazatricyclo[13.3.1.0$^{2,7}$] nonadeca-1(19),2(7),3,5,15,17-hexaen-9-one

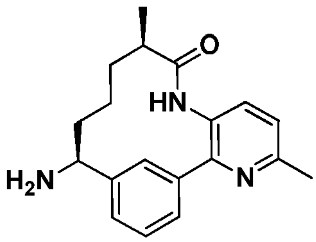

*tert*-Butyl *N*-[(10*R*,11*E*,14*S*)-4,10-dimethyl-9-oxo-3,8-diazatricyclo[13.3.1.0$^{2,7}$] nonadeca-1(19),2(7),3,5,11,15,17-heptaen-14-yl]carbamate (0.187 g, 0.459 mmol) was hydrogenated in EtOH (3 ml) in the presence of $PtO_2$ at 20-30 psi. After 4 h, the reaction mixture was filtered through CELITE® and concentrated to afford a dark solid (MS(ESI) *m/z:* 410.3 $(M+H)^+$) which was then deprotected with 4 N HCl in dioxane (2 ml) and MeOH (2 ml). The resultant HCl salt was dissolved in DCM/MeOH and passed through a basic cartridge to afford (0.16g, 118%) of a crude dark solid containing as the major component, (10*R*,14*S*)-14-amino-4,10-dimethyl-3,8-diazatricyclo[13.3.1.0$^{2,7}$]nonadeca-1(19),2(7),3,5,15,17-hexaen-9-one, which was used in next step without purification. MS(ESI) *m/z*: 310.3 $(M+H)^+$.

80F. Preparation of (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-4,10-dimethyl-3,8-diazatricyclo[13.3.1.0$^{2,7}$] nonadeca-1(19),2(7),3,5,15,17-hexaen-9-one

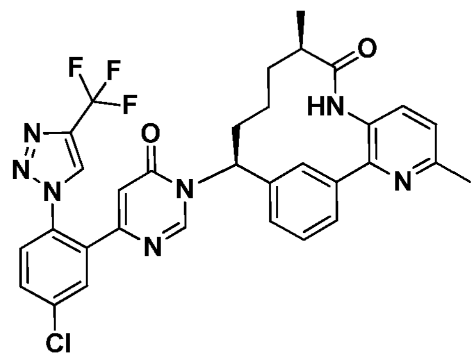

To 6-{5-chloro-2-[4-(trifluoromethyl)-1*H*-1,2,3-triazol-1-yl]phenyl}pyrimidin-4-ol (0.033 g, 0.097 mmol), prepared as described in Intermediate 15, and HATU (0.048 g, 0.126 mmol) in a small vial was added DBU (0.022 mL, 0.145 mmol) in ACN (0.4 ml). After 30 min, (10R,14S)-14-amino-4,10-dimethyl-3,8-diazatricyclo[13.3.1.0$^{2,7}$]nonadeca-1(19),2(7),3,5,15,17-hexaen-9-one (0.033 g, 0.097 mmol) was added with DMF (0.2 ml). The reaction was stirred for 18 h. The reaction was diluted with DMF, filtered and purified by preparative LCMS using (5:95 ACN/$H_2O$ to 95:5 ACN/$H_2O$, 10 mM $NH_4OAc$) to afford (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-4,10-dimethyl-3,8-diazatricyclo[13.3.1.0$^{2,7}$] nonadeca-1(19),2(7),3,5,15,17-hexaen-9-one (5.8 mg, 7.7% yield) as a white solid. MS(ESI) *m/z*: 634.4 $(M+H)^+$. $^1H$ NMR (500MHz, $CD_3OD$) δ 8.75 (s, 1H), 8.12 (s, 1H), 7.86 - 7.82 (m, 2H), 7.77 - 7.64 (m, 3H), 7.62 - 7.54 (m, 2H), 7.34 (d, *J*=8.2 Hz, 1H), 7.29 (d, *J*=7.9 Hz, 1H), 6.43 (s, 1H), 5.86 - 5.73 (m, 1H), 2.61 (s, 3H), 2.45 (br. s., 1H), 2.26 (d, *J*=9.5 Hz, 1H), 2.11 (br. s., 1H), 1.82 (br. s., 1H), 1.64 - 1.49 (m, 2H), 1.28 - 1.21 (m, 1H), 1.13 (d, *J*=6.7 Hz, 3H). Analytical HPLC (Method C) RT = 1.54 min, purity = 97%; Factor XIa Ki = 2.2 nM, Plasma Kallikrein Ki = 260 nM.

Example 81

Preparation of (10*R*,14*S*)-14-(4-{5-chloro-2-[4-(trifluoromethyl)-1*H*-1,2,3-triazol-1-yl] phenyl}-6-oxo-1,6-dihydropyrimidin-1-yl)-5-methoxy-10-methyl-3,8-diazatricyclo [13.3.1.0$^{2,7}$]nonadeca-1(19),2(7),3,5,15,17-hexaen-9-one

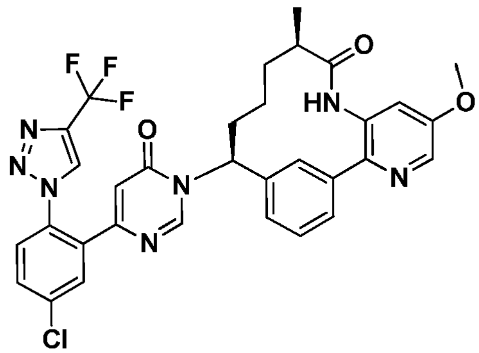

81A. Preparation of *tert*-butyl *N*-[(1S)-1-[3-(3-amino-5-methoxypyridin-2-yl)phenyl]but-3-en-1-yl]carbamate

*tert*-Butyl *N*-[(*S*)-1-[3-(5,5-dimethyl-1,3,2-dioxaborinan-2-yl)phenyl]but-3-en-1-yl]carbamate (0.348 g, 0.969 mmol), 2-bromo-5-methoxypyridin-3-amine (0.197 g, 0.969 mmol), and 2.0 M aq $Na_2CO_3$ (2.422 mL, 4.84 mmol) were added to dioxane (8 ml) and the solution was purged with a stream of Ar for 10 min. $Pd(PPh_3)_4$ (0.056 g, 0.048 mmol) was added and the mixture irradiated in microwave at 120 °C for 30 min. The reaction was quenched with water (20 ml) and extracted with EtOAc (3 x 30 ml). The combined organic layers were washed with brine (15 ml), dried ($MgSO_4$), filtered and concentrated. The residue was purified by normal phase chromatography using DCM and 0-10% MeOH as eluents to afford *tert*-butyl *N*-[(1*S*)-1-[3-(3-amino-5-methoxypyridin-2-yl) phenyl]but-3-en-1-yl]carbamate (0.391g, 100%) as a tan foam. MS(ESI) *m/z:* 370.08 $(M+H)^+$. $^1H$ NMR (500MHz, $CDCl_3$) δ 7.89 (d, *J*=2.5 Hz, 1H), 7.60 - 7.52 (m, 2H), 7.45 (t, *J*=7.6 Hz, 1H), 7.33 - 7.29 (m, 1H), 6.62 (d, *J*=2.5 Hz, 1H), 5.73 (ddt, *J*=17.1, 10.1, 7.0 Hz, 1H), 5.21 - 5.06 (m, 2H), 4.93 (br. s., 1H), 4.81 (br. s., 1H), 3.92 - 3.86 (m, 4H), 3.77 - 3.71 (m, 1H), 2.64 - 2.52 (m, 2H), 1.44 (br. s., 9H).

81B. Preparation of *tert*-butyl *N*-[(1S)-1-(3-{5-methoxy-3-[(2*R*)-2-methylbut-3-enamido]pyridin-2-yl}phenyl)but-3-en-1-yl]carbamate.

To *tert*-butyl *N*-[(1*S*)-1-[3-(3-amino-5-methoxypyridin-2-yl)phenyl]but-3-en-1-yl]carbamate (0.358 g, 0.972 mmol) was added (R)-2-methylbut-3-enoic acid (0.126 g, 1.263 mmol), prepared as described in Intermediate 2, in EtOAc (3 ml). The resulting solution was cooled to 0 °C. Pyridine (0.236 ml, 2.91 mmol) and a 50% EtOAc solution of T3P® (1.157 ml, 1.943 mmol) were added. The reaction was partitioned between sat NaHCO3 (10 ml) and EtOAc (20 ml). The aqueous layer was extracted with EtOAc (2 x 20 ml). The combined organic layers were washed with brine (25 ml), dried ($MgSO_4$), filtered and concentrated. The residue was purified by normal phase chromatography using heptanes and EtOAc as eluents to afford *tert*-butyl *N*-[(1S)-1-(3-{5-methoxy-3-[(2*R*)-2-methylbut-3-enamido]pyridin-2-yl}phenyl)but-3-en-1-yl]carbamate (0.347g, 79%) as a white foam. MS(ESI) *m/z*: 452.08 $(M+H)^+$. $^1H$ NMR (500MHz, $CDCl_3$) δ 8.47 (d, *J*=2.8 Hz, 1H), 8.16 (d, *J*=2.8 Hz, 1H), 7.65 (br. s., 1H), 7.53 - 7.46 (m, 1H), 7.46 - 7.41 (m, 1H), 7.37 (dt,

*J*=7.5, 1.9 Hz, 2H), 5.86 - 5.65 (m, 2H), 5.19 - 5.07 (m, 4H), 4.93 (br. s., 1H), 4.82 (br. s., 1H), 3.93 (s, 3H), 3.07 (quin, *J*=7.3 Hz, 1H), 2.62 - 2.51 (m, 2H), 1.50 - 1.41 (m, 9H), 1.32 - 1.29 (m, 3H).

81C. Preparation of *tert*-butyl *N*-[(10*R*,11*E*,14*S*)-5-methoxy-10-methyl-9-oxo-3,8-diazatricyclo[13.3.1.0$^{2,7}$]nonadeca-1(19),2(7),3,5,11,15,17-heptaen-14-yl]carbamate

A solution of *tert*-butyl *N*-[(1S)-1-(3-{5-methoxy-3-[(2*R*)-2-methylbut-3-enamido]pyridin-2-yl} phenyl)but-3-en-1-yl]carbamate (0.189 g, 0.419 mmol) in degassed DCE (20 ml) in the presence of Second Generation Grubbs Catalyst (0.107 g, 0.126 mmol) was heated to 120 °C for 30 min in a microwave. The reaction mixture was directly purified by normal phase chromatography using DCM and 0-10% MeOH as eluents to afford *tert*-butyl *N*-[(10*R*,11*E*,14*S*)-5-methoxy-10-methyl-9-oxo-3,8-diazatricyclo[13.3.1.0$^{2,7}$]nonadeca-1(19),2(7),3,5,11,15,17-heptaen-14-yl]carbamate (0.17g, 96%) as a dark brown oil. MS(ESI) *m/z*: 424.1 (M+H)$^+$.

81D. Preparation of (10*R*,14*S*)-14-amino-5-methoxy-10-methyl-3,8-diazatricyclo [13.3.1.0$^{2,7}$]nonadeca-1(19),2(7),3,5,15,17-hexaen-9-one

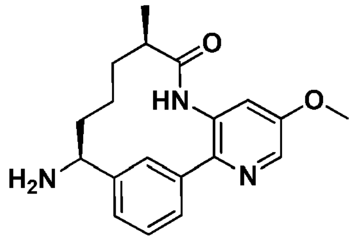

*tert*-Butyl *N*-[(10*R*,11*E,*14*S*)-5-methoxy-10-methyl-9-oxo-3,8-diazatricyclo [13.3.1.0$^{2,7}$]nonadeca-1(19),2(7),3,5,11,15,17-heptaen-14-yl]carbamate (0.177 g, 0.418 mmol) was hydrogenated in EtOH (3 ml) in the presence of $PtO_2$ (20 mg) over 8 h. The resultant thick sludge was filtered through CELITE® and rinsed with DCM, MeOH and EtOH to afford 0.121g of a dark solid. MS(ESI) *m/z*: 426.4 (M+H)$^+$. Deprotection was performed with 4 N HCl in dioxane (2 ml) in MeOH (4 ml) over 3 h. The reaction mixture was concentrated and the residue was taken up in DCM/MeOH and filtered through a basic cartridge to give (10*R*,14*S*)-14-amino-5-methoxy-10-methyl-3,8-diazatricyclo[13.3.1.0$^{2,7}$]nonadeca-1(19),2(7),3,5,15,17-hexaen-9-one as a dark solid (0.108g, 79%). MS(ESI) *m/z*: 326.4 (M+H)$^+$.

81E. Preparation of (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-5-methoxy-10-methyl-3,8-diazatricyclo [13.3.1.0$^{2,7}$]nonadeca-1(19),2(7),3,5,15,17-hexaen-9-one

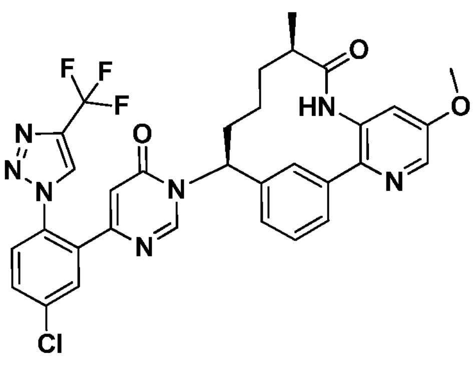

To 6-{5-chloro-2-[4-(trifluoromethyl)-1*H*-1,2,3-triazol-1-yl]phenyl}pyrimidin-4-ol (0.032 g, 0.092 mmol), prepared as described in Intermediate 15, and HATU (0.046 g, 0.120 mmol) in a small vial was added DBU (0.021 mL, 0.138 mmol) in ACN (0.4 ml). After 30 min, (10*R*,14*S*)-14-amino-5-methoxy-10-methyl-3,8-diazatricyclo[13.3.1.0$^{2,7}$] nonadeca-1(19),2(7),3,5,15,17-hexaen-9-one (0.030 g, 0.092 mmol) was added with DMF (0.4 ml). After 18 h, the reaction was diluted with DMF, filtered and concentrated. The residue was purified twice by reverse phase HPLC using PHENOMENEX® Luna 5U 30x 100mm (10:90 ACN/$H_2O$ to 90:10 ACN/$H_2O$, 0.1% TFA) (20% B start, 14 min gradient) to afford (10*R*,14*S*)-14-(4-{5-chloro-2-[4-(trifluoromethyl)-1*H*-1,2,3-triazol-1-yl]phenyl}-6-oxo-1,6-dihydropyrimidin-1-yl)-5-methoxy-10-methyl-3,8-diazatricyclo [13.3.1.$0^{2,7}$]nonadeca-1(19),2(7),3,5,15,17-hexaen-9-one (3.7 mg, 4.8%) as a white solid. MS(ESI) *m/z*: 650.3 $(M+H)^+$. $^1$H NMR (400MHz, $CD_3OD$) δ 8.82 (d, *J*=0.7 Hz, 1H), 8.39 (d, *J*=2.6 Hz, 1H), 8.25 (s, 1H), 7.89 (d, *J*=2.2 Hz, 1H), 7.86 (s, 1H), 7.80 - 7.74 (m, 1H), 7.71 - 7.63 (m, 2H), 7.62 - 7.55 (m, 2H), 7.29 (d, *J*=7.9 Hz, 1H), 6.47 (d, *J*=0.7 Hz, 1H), 5.81 (dd, *J*=13.0, 3.5 Hz, 1H), 4.03 - 4.01 (m, 3H), 2.60 - 2.49 (m, 1H), 2.34 - 2.26 (m, 1H), 2.18 - 2.08 (m, 1H), 1.94 - 1.85 (m, 1H), 1.62 - 1.46 (m, 2H), 1.33 (d, *J*=9.7 Hz, 1H), 1.15 (d, *J*=6.8 Hz, 3H). Analytical HPLC (Method A) RT = 8.44, purity = 93%; Factor XIa Ki = 22 nM, Plasma Kallikrein Ki = 950 nM.

Example 82

Preparation of (10*R*,14*S*)-5-chloro-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.$0^{2,7}$]nonadeca-1(19),2(7),3,5,15,17-hexaen-9-one

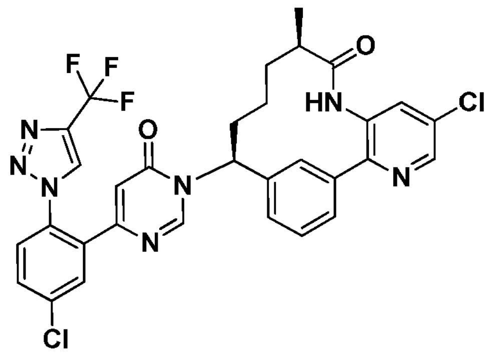

82A. Preparation of *tert*-butyl *N*-[(1*S*)-1-[3-(3-amino-5-chloropyridin-2-yl)phenyl]but-3-en-1-yl]carbamate

*tert*-Butyl *N*-[(1*S*)-1-[3-(5,5-dimethyl-1,3,2-dioxaborinan-2-yl)phenyl]but-3-en-1-yl]carbamate (0.339 g, 0.944 mmol), 2-bromo-5-chloropyridin-3-amine (0.196 g, 0.944 mmol), and 2.0 M aq $Na_2CO_3$ (2.36 mL, 4.72 mmol) were added to dioxane (8 ml) and the resulting solution was purged with a stream of Ar for 10 min. $Pd(PPh_3)_4$ (0.055 g, 0.047 mmol) was added and the mixture irradiated on microwave at 120 °C for 30 min. The reaction was quenched with water (20 ml) and extracted with EtOAc (3 x 30 ml). The combined organic layers were washed with brine (15 ml), dried ($Na_2SO_4$), filtered and concentrated. The residue was purified by normal phase chromatography using DCM and 0-10% MeOH as eluents to afford *tert*-butyl N-[(1*S*)-1-[3-(3-amino-5-chloropyridin-2-yl) phenyl]but-3-en-1-yl] carbamate (0.375g, 106%) as a tan foam. MS(ESI) *m/z*: 374.3 $(M+H)^+$. $^1$H NMR (500MHz, $CDCl_3$) δ 8.08 (d, *J*=2.2 Hz, 1H), 7.60 - 7.52 (m, 2H), 7.48-7.43 (m, 1H), 7.34 (d, *J*=7.7 Hz, 1H), 7.07 (d, *J*=1.9 Hz, 1H), 5.72 (ddt, *J*=17.1, 10.1, 7.0 Hz, 1H), 5.21 - 5.09 (m, 2H), 4.93 (br. s., 1H), 4.81 (br. s., 1H), 3.94 (br. s., 2H), 2.63-2.51 (m, 2H), 1.43 (br. s., 9H).

82B. Preparation of *tert*-butyl *N*-[(1S)-1-(3-{5-chloro-3-[(2*R*)-2-methylbut-3-enamido] pyridin-2-yl}phenyl)but-3-en-1-yl]carbamate

To *tert-butyl N*-[(1S)-1-[3-(3-amino-5-chloropyridin-2-yl)phenyl]but-3-en-1-yl]carbamate (0.358 g, 0.958 mmol) was added (R)-2-methylbut-3-enoic acid (0.125 g, 1.245 mmol), prepared as described in Intermediate 2, in 3 ml EtOAc, and the resulting solution was cooled to 0 °C. Pyridine (0.232 ml, 2.87 mmol) and a 50% EtOAc solution of T3P® (1.140 ml, 1.915 mmol) were then added. After 4 h, the reaction was partitioned with sat NaHCO3 (10 ml) and EtOAc (10 ml). The aqueous layer was extracted with EtOAc (2 x 20 ml). The combined organic layers were washed with brine (10 ml), dried ($Na_2SO_4$), filtered and concentrated. The residue was purified by normal phase chromatography using heptanes and EtOAc as eluents to give *tert*-butyl *N*-[(1S)-1-(3-{5-chloro-3-[(2*R*)-2-methylbut-3-enamido]pyridin-2-yl}phenyl)but-3-en-1-yl]carbamate (0.31 g, 71%) as a white foam. MS(ESI) *m/z*: 456.08 $(M+H)^+$. $^1$H NMR (500MHz, $CDCl_3$) δ 8.88 (d, *J*=2.2 Hz, 1H), 8.38 (d, *J*=2.2 Hz, 1H), 7.65 (br. s., 1H), 7.56 - 7.49 (m, 1H), 7.45 - 7.40 (m, 2H), 7.39 - 7.33 (m, 1H), 5.82 - 5.66 (m, 2H), 5.21 - 5.11 (m, 2H), 5.11 - 5.06 (m, 2H), 4.93 (br. s., 1H), 4.82 (br. s., 1H), 3.08 (quin, *J*=7.2 Hz, 1H), 2.64-2.50 (m, 2H), 1.46 - 1.41 (m, 9H), 1.30 (d, *J*=7.2 Hz, 3H).

82C. Preparation of *tert*-butyl *N*-[(10*R*,11*E*,14*S*)-5-chloro-10-methyl-9-oxo-3,8-diazatricyclo[13.3.1.$0^{2,7}$]nonadeca-1(19),2(7),3,5,11,15,17-heptaen-14-yl]carbamate

A solution of Second Generation Grubbs Catalyst (0.107 g, 0.126 mmol) and *tert*-butyl *N*-[(1S)-1-(3-{5-chloro-3-[(2R)-2-methylbut-3-enamido]pyridin-2-yl}phenyl)but-3-en-1-yl]carbamate (0.191 g, 0.419 mmol) in degassed DCE (20 ml) was heated to 120 °C for 30 min in a microwave. The reaction mixture was concentrated and the crude material was directly purified by normal phase chromatography using DCM and 0-10% MeOH as eluents to afford *tert*-butyl *N*-[(10*R*,11*E*,14*S*)-5-chloro-10-methyl-9-oxo-3,8-diazatricyclo [13.3.1.$0^{2,7}$]nonadeca-1(19),2(7),3,5,11,15,17-heptaen-14-yl]carbamate (0.17 g, 95%) as a dark brown oil. MS(ESI) *m*/*z*: 428.2 $(M+H)^+$.

82D. Preparation of (10*R*,14*S*)-14-amino-5-chloro-10-methyl-3,8-diazatricyclo [13.3.1.$0^{2,7}$]nonadeca-1(19),2(7),3,5,15,17-hexaen-9-one

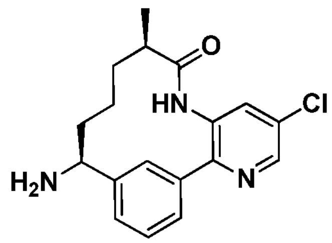

*tert*-Butyl *N*-[(10*R*,11*E*,14*S*)-5-chloro-10-methyl-9-oxo-3,8-diazatricyclo [13.3.1.$0^{2,7}$]nonadeca-1(19),2(7),3,5,11,15,17-heptaen-14-yl]carbamate (0.17 g, 0.397 mmol) was hydrogenated in EtOH (3 ml) in the presence of $PtO_2$ (20 mg) for 8 h. MS(ESI) *m/z:* 430.3 $(M+H)^+$. The thick sludge was filtered through CELITE® and rinsed with DCM/MeOH/EtOH to afford 0.169 g of a dark solid. Deprotection was carried out with 4 N HCl in dioxane (2 ml) and MeOH (2 ml) over 4 h. After this time the solution was concentrated and the residue was taken up in DCM/MeOH and passed through a basic cartridge to give (10*R*,14*S*)-14-amino-5-chloro-10-methyl-3,8-diazatricyclo [13.3.1.$0^{2,7}$]nonadeca-1(19),2(7),3,5,15,17-hexaen-9-one as a crude product (0.159g, 121%) that was used 'as is'. MS(ESI) *m*/*z*: 330.08 $(M+H)^+$.

82E. Preparation of (10*R*,14*S*)-5-chloro-14-({5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.$0^{2,7}$]nonadeca-1(19),2(7),3,5,15,17-hexaen-9-one

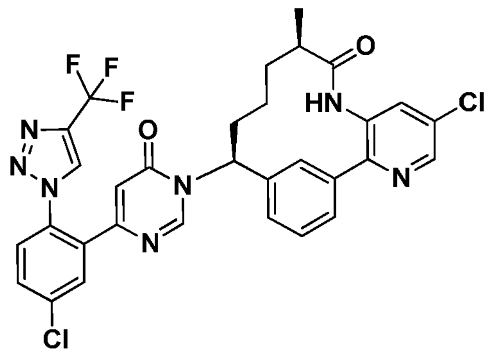

To HATU (0.045 g, 0.118 mmol) and 6-{5-chloro-2-[4-(trifluoromethyl)-1*H*-1,2,3-triazol-1-yl]phenyl}pyrimidin-4-ol (0.031 g, 0.091 mmol), prepared as described in Intermediate 15, in a small vial was added DBU (0.021 mL, 0.136 mmol) in $CH_3CN$ (0.4 ml). After 30 min, *tert*-butyl N-[(10*R*,11*E*,14*S*)-5-chloro-10-methyl-9-oxo-3,8-diazatricyclo[13.3.1.$0^{2,7}$]nonadeca-1(19),2(7),3,5,11,15,17-heptaen-14-yl]carbamate (0.030 g, 0.091 mmol) was added with DMF (0.4 ml). After 18 h, the reaction was diluted with DMF, filtered and concentrated The residue was purified twice by reverse phase HPLC using PHENOMENEX® Luna 5U 30x 100mm (10:90 ACN/$H_2O$ to 90:10 ACN/$H_2O$, 0.1% TFA) (20% B start, 14 min gradient) to afford (10*R*,14*S*)-5-chloro-14-(4-{5-chloro-2-[4-(trifluoromethyl)-1*H*-1,2,3-triazol-1-yl]phenyl}-6-oxo-1,6-dihydropyrimidin-1-yl)-10-methyl-3,8-diazatricyclo[13.3.1.0$^{2,7}$]nonadeca-1(19),2(7),3,5,15,17-hexaen-9-one (3.0 mg, 4.2% yield) as a tan solid. MS(ESI) *m/z:* 654.3 $(M+H)^+$. $^1$H NMR (400MHz, $CD_3OD$) δ 8.81 (d, *J*=0.7 Hz, 1H), 8.67 - 8.62 (m, 1H), 8.23 (s, 1H), 7.93 - 7.84 (m, 3H), 7.79 - 7.67 (m, 3H), 7.63 - 7.54 (m, 1H), 7.29 (d, *J*=7.9 Hz, 1H), 6.47 (d, *J*=0.9 Hz, 1H), 5.82 (dd, *J*=12.9, 3.6 Hz, 1H), 2.58 - 2.48 (m, 1H), 2.40 - 2.24 (m, 1H), 2.11 (d, *J*=9.7 Hz, 1H), 1.99 - 1.87 (m, 1H), 1.55 (d, *J*=9.0 Hz, 2H), 1.33 (d, *J*=9.7 Hz, 1H), 1.15 (d, *J*=6.8 Hz, 3H). Analytical HPLC (Method A) RT = 9.88 min, purity = 98%; Factor XIa Ki = 3.5 nM, Plasma Kallikrein Ki = 240 nM.

Example 90 (reference)

Preparation of (9*R*,13*S*)-13-14-[3-chloro-6-(4-chloro-1*H*-1,2,3-triazol-1-yl)-2-fluorophenyl]-6-oxo-1,6-dihydropyrimidin-1-yl}-3-(2-hydroxyethyl)-9-methyl-3,4,7,15-tetraazatricyclo[12.3.1.0$^{2,6}$]octadeca-1(18),2(6),4,14,16-pentaen-8-one trifluoroacetate

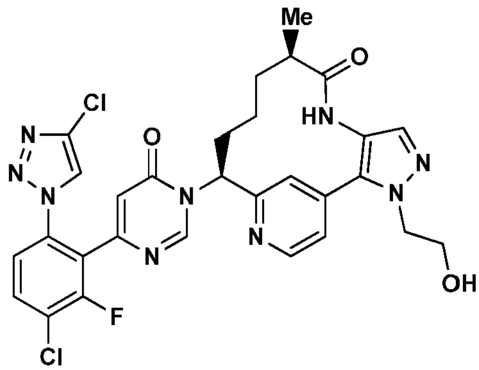

(9*R*,13*S*)-13-{4-[3-Chloro-6-(4-chloro-1*H*-1,2,3-triazol-1-yl)-2-fluorophenyl]-6-oxo-1,6-dihydropyrimidin-1-yl}-3-(2-hydroxyethyl)-9-methyl-3,4,7,15-tetraazatricyclo [12.3.1.0$^{2,6}$]octadeca-1(18),2(6),4,14,16-pentaen-8-one trifluoroacetate was prepared in a similar manner as the procedures described in Example 56, by using 6-(3-chloro-6-(4-chloro-1*H*-1,2,3-triazol-1-yl)-2-fluorophenyl)pyrimidin-4-ol (0.054 g, 0.167 mmol), prepared as described in Intermediate 10, and (9*R*,13*S*)-13-amino-3-(2-hydroxyethyl)-9-methyl-3,4,7,15-tetraazatricyclo[12.3.1.0$^{2,6}$]octadeca-1(18),2(6),4,14,16-pentaen-8-one (0.055 g, 0.167 mmol), prepared as described in Intermediate 40. The crude product was purified by prep HPLC to give (9*R*,13*S*)-13-{4-[3-chloro-6-(4-chloro-1*H*-1,2,3-triazol-1-yl)-2-fluorophenyl]-6-oxo-1,6-dihydropyrimidin-1-yl}-3-(2-hydroxyethyl)-9-methyl-3,4,7,15-tetraazatricyclo[12.3.1.0$^{2,6}$]octadeca-1(18),2(6),4,14,16-pentaen-8-one trifluoroacetate. (45 mg, 34% yield) as tan solid. $^1$H NMR (400MHz, $CD_3OD$) δ 8.82 (s, 1H), 8.75 (d, *J*=5.3 Hz, 1H), 8.36 - 8.32 (m, 1H), 7.87 (dd, *J*=8.6, 7.7 Hz, 1H), 7.81 (dd, *J*=5.1, 1.5 Hz, 1H), 7.73 (s, 1H), 7.61 - 7.53 (m, 2H), 6.00 (dd, *J*=12.7, 4.3 Hz, 1H), 4.51 - 4.33 (m, 1H), 4.13 - 3.86 (m, 2H), 3.39 - 3.35 (m, 2H), 2.71 (td, *J*=6.8, 3.1 Hz, 1H), 2.43 - 2.25 (m, 1H), 2.13 - 1.97 (m, 1H), 1.68 - 1.40 (m, 2H), 1.03 (d, *J*=6.8 Hz, 6H), 0.75 (br. s., 1H). MS(ESI) *m/z*: 638.5 $[M+H]^+$. Analytical HPLC (Method A): RT = 7.32 min, purity = >95.0%; Factor XIa Ki = 0.34 nM, Plasma Kallikrein Ki = 28 nM.

Example 93

Preparation of (9*R*,13*S*)-13-{4-[5-chloro-2-(1*H*-1,2,3,4-tetrazol-1-yl)phenyl]-6-oxo-1,6-dihydropyrimidin-1-yl}-3-(2-hydroxyethyl)-9-methyl-3,4,7,15-tetraazatricyclo [12.3.1.0$^{2,6}$]octadeca-1(18),2(6),4,14,16-pentaen-8-one trifluoroacetate

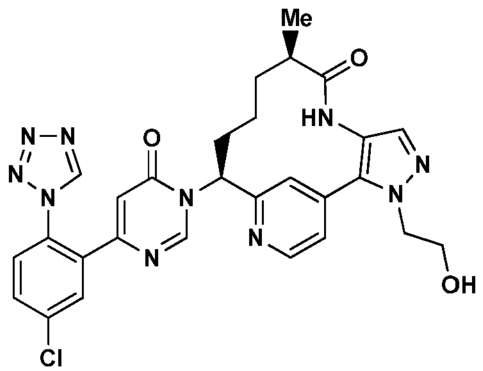

(9*R*,13*S*)-13-{4-[5-Chloro-2-(1*H*-1,2,3,4-tetrazol-1-yl)phenyl]-6-oxo-1,6-dihydropyrimidin-1-yl}-3-(2-hydroxyethyl)-9-methyl-3,4,7,15-tetraazatricyclo [12.3.1.$0^{2,6}$]octadeca-1(18),2(6),4,14,16-pentaen-8-one trifluoroacetate was prepared in a similar manner as the procedures described in Example 56, by using 6-(5-chloro-2-(1*H*-tetrazol-1-yl)phenyl)pyrimidin-4-ol (0.037 g, 0.137 mmol), prepared as described in Intermediate 20, and (9*R*,13*S*)-13-amino-3-(2-hydroxyethyl)-9-methyl-3,4,7,15-tetraazatricyclo[12.3.1.$0^{2,6}$]octadeca-1(18),2(6),4,14,16-pentaen-8-one (0.045 g, 0.137 mmol), prepared as described in Intermediate 40. The crude product was purified by prep HPLC to yield (9*R*,13*S*)-13-{4-[5-chloro-2-(1*H*-1,2,3,4-tetrazol-1-yl)phenyl]-6-oxo-1,6-dihydropyrimidin-1-yl}-3-(2-hydroxyethyl)-9-methyl-3,4,7,15-tetraazatricyclo [12.3.1.$0^{2,6}$]octadeca-1(18),2(6),4,14,16-pentaen-8-one (52 mg, 52% yield) as a pale white solid. $^1$H NMR (400MHz, $CD_3OD$) δ 9.42 (s, 1H), 8.68 (d, *J*=5.1 Hz, 2H), 7.88 (d, *J*=2.2 Hz, 1H), 7.78 - 7.72 (m, 2H), 7.67 (d, *J*=8.4 Hz, 2H), 7.55 (s, 1H), 6.49 (s, 1H), 5.93 (d, *J*=8.8 Hz, 1H), 4.45 - 4.29 (m, 2H), 4.08 - 3.88 (m, 3H), 3.32 (s, 1H), 2.66 (d, *J*=7.0 Hz, 1H), 2.24 (t, *J*=13.0 Hz, 1H), 2.08 - 1.88 (m, 2H), 1.65 - 1.49 (m, 1H), 1.41 (br. s., 1H), 0.98 (d, *J*=6.8 Hz, 3H), 0.67 (br. s., 1H). MS(ESI) *m/z*: 587.5 $[M+H]^+$. Analytical HPLC (Method A): RT = 6.40 min, purity = >95.0%; Factor XIa Ki = 0.65 nM, Plasma Kallikrein Ki = 45 nM.

Example 95 (reference)

Preparation of 2-[(9*R*,13*S*)-13-{4-[3-chloro-6-(4-chloro-1*H*-1,2,3-triazol-1-yl)-2-fluorophenyl]-6-oxo-1,6-dihydropyrimidin-1-yl}-9-methyl-8-oxo-3,4,7,15-tetraazatricyclo[12.3.1.$0^{2,6}$]octadeca-1(18),2(6),4,14,16-pentaen-3-yl] acetic acid trifluoroacetate

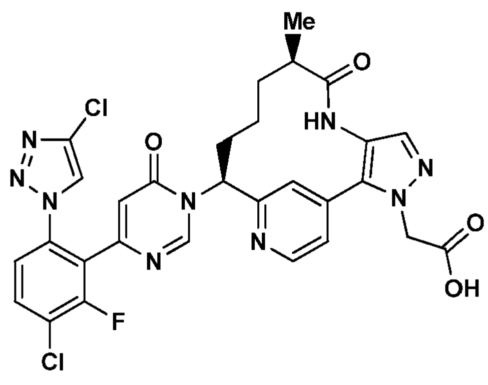

A solution of (9*R*,13*S*)-13-{4-[3-chloro-6-(4-chloro-1*H*-1,2,3-triazol-1-yl)-2-fluorophenyl]-6-oxo-1,6-dihydropyrimidin-1-yl}-3-(2-hydroxyethyl)-9-methyl-3,4,7,15-tetraazatricyclo[12.3.1.$0^{2,6}$]octadeca-1(18),2(6),4,14,16-pentaen-8-one trifluoroacetate (0.034 g, 0.053 mmol), prepared as described in Example 90, in acetone (2 mL) was cooled to 0 °C. To this cooled mixture was then added 2.86 M solution of Jones reagent (0.037 mL, 0.107 mmol) and the resulting reaction mixture was allowed to warm to rt over a period of 2 h. The reaction mixture was then quenched with 0.5 mL of IPA and concentrated. The resulting residue was purified by prep HPLC purification to afford 2-[(9*R*,13*S*)-13-{4-[3-chloro-6-(4-chloro-1*H*-1,2,3-triazol-1-yl)-2-fluorophenyl]-6-oxo-1,6-dihydropyrimidin-1-yl}-9-methyl-8-oxo-3,4,7,15-tetraazatricyclo[12.3.1.$0^{2,6}$]octadeca-1(18),2(6),4,14,16-pentaen-3-yl]acetic acid trifluoroacetate (8 mg, 19% yield) as a white solid. $^1$H NMR (400MHz, $CD_3OD$) δ 8.85 (s, 1H), 8.74 (d, *J*=5.1 Hz, 1H), 8.34 (s, 1H), 7.87 (dd, *J*=8.6, 7.5 Hz, 1H), 7.72 (s, 1H), 7.61 - 7.53 (m, 2H), 7.49 (dd, *J*=5.1, 1.5 Hz, 1H), 6.62 (s, 1H), 6.04 (dd, *J*=12.3, 4.2 Hz, 1H), 5.27 - 5.06

(m, 2H), 2.72 (dt, *J*=6.7, 3.4 Hz, 1H), 2.30 (t, *J*=12.7 Hz, 1H), 2.15 - 1.97 (m, 2H), 1.69 - 1.41 (m, 2H), 1.03 (d, *J*=7.0 Hz, 3H), 0.71 (br. s., 1H). MS(ESI) *m/z*: 652.2 $[M+H]^+$. Analytical HPLC (Method A): RT = 7.45 min, purity = >95.0%; Factor XIa Ki = 0.11 nM, Plasma Kallikrein Ki = 12 nM.

Example 98

Preparation of 2-[(9*R*,13*S*)-13-{4-[5-chloro-2-(1*H*-1,2,3,4-tetrazol-1-yl)phenyl]-6-oxo-1,6-dihydropyrimidin-1-yl}-9-methyl-8-oxo-3,4,7,15-tetraazatricyclo[12.3.1.0$^{2,6}$] octadeca-l(18),2(6),4,14,16-pentaen-3-yl]acetic acid trifluoroacetate

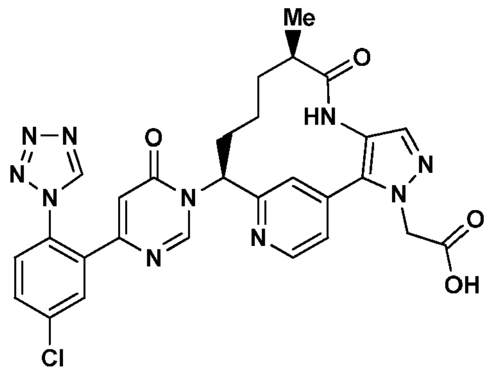

2-[(9R,13S)-13-{4-[5-Chloro-2-(1*H*-1,2,3,4-tetrazol-1-yl)phenyl]-6-oxo-1,6-dihydropyrimidin-1-yl}-9-methyl-8-oxo-3,4,7,15-tetraazatricyclo[12.3.1.0$^{2,6}$]octadeca-1(18),2(6),4,14,16-pentaen-3-yl]acetic acid trifluoroacetate was prepared in a similar manned as the procedure described in Example 95, using (9*R*,13*S*)-13-{4-[5-chloro-2-(1*H*-1,2,3,4-tetrazol-1-yl)phenyl]-6-oxo-1,6-dihydropyrimidin-1-yl}-3-(2-hydroxyethyl)-9-methyl-3,4,7,15-tetraazatricyclo[12.3.1.0$^{2,6}$]octadeca-1(18),2(6),4,14,16-pentaen-8-one trifluoroacetate (0.020 g, 0.034 mmol), prepared as described in Example 93, to yield 2-[(9*R*,13*S*)-13-{4-[5-chloro-2-(1*H*-1,2,3,4-tetrazol-1-yl)phenyl]-6-oxo-1,6-dihydropyrimidin-1-yl}-9-methyl-8-oxo-3,4,7,15-tetraazatricyclo[12.3.1.0$^{2,6}$]octadeca-1(18),2(6),4,14,16-pentaen-3-yl]acetic acid trifluoroacetate (5.1 mg, 20% yield) as a white solid. $^1$H NMR (400MHz, $CD_3OD$) δ 9.45 (s, 1H), 8.80 - 8.68 (m, 2H), 7.95 - 7.90 (m, 1H), 7.83 - 7.76 (m, 1H), 7.74 - 7.67 (m, 2H), 7.59 (s, 1H), 7.49 (dd, *J*=5.1, 1.3 Hz, 1H), 6.53 (s, 1H), 6.01 (dd, *J*=12.5, 4.0 Hz, 1H), 5.28 - 5.05 (m, 2H), 2.71 (m, 1H), 2.27 (m, 1H), 2.13 - 1.95 (m, 2H), 1.66 - 1.39 (m, 2H), 1.03 (d, *J*=7.0 Hz, 3H), 0.72 (br. s., 1H). MS(ESI) *m/z*: 601.3 $[M+H]^+$. Analytical HPLC (Method A): RT = 6.62 min, purity = >95.0%; Factor XIa Ki = 0.16 nM, Plasma Kallikrein Ki = 20 nM.

Example 114

Preparation of (10*R*,14*S*)-14-{4-[5-chloro-2-(4-chloro-1H-1,2,3-triazol-1-yl)phenyl]-6-oxo-1,6-dihydropyrimidin-1-yl}-10-methyl-3,8,16-triazatricyclo[13.3.1.0$^{2,7}$]nonadeca-1(19),2(7),3,5,15,17-hexaen-9-one, *bis*-trifluoroacetate

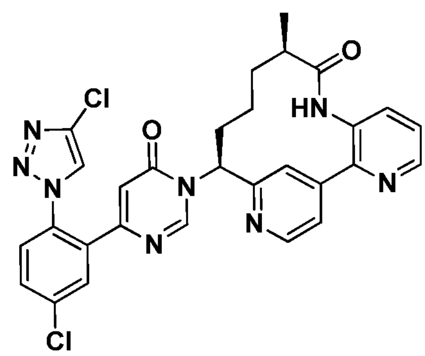

To a vial (4 ml) containing a suspension 6-(5-chloro-2-(4-chloro-1H-1,2,3-triazol-1-yl)phenyl)pyrimidin-4-ol

(0.021 g, 0.067 mmol), prepared as described in Intermediate 9, in ACN (1 ml) was added HATU (0.033 g, 0.088 mmol) and DBU (0.015 ml, 0.101 mmol). After 30 min, (10*R*,14*S*)-14-amino-10-methyl-3,8,16-triazatricyclo[13.3.1.0$^{2,7}$] nonadeca-1(19),2(7),3,5,15,17-hexaen-9-one (0.020 g, 0.067 mmol), prepared as described in Intermediate 38, in DMF (1.0 ml) was added at rt. After 4 h, the crude mixture was purified by reverse phase chromatography (PHENOMENEX® Luna Axia C18 5μ 30 x 100mm column, Solvent A: 20% ACN - 80% $H_2O$ - 0.1% TFA; Solvent B: 80% ACN - 20% $H_2O$ - 0.1% TFA) to give (10*R*,14*S*)-14-{4-[5-chloro-2-(4-chloro-1H-1,2,3-triazol-1-yl)phenyl]-6-oxo-1,6-dihydropyrimidin-1-yl}-10-methyl-3,8,16-triazatricyclo [13.3.1.0$^{2,7}$]nonadeca-1(19),2(7),3,5,15,17-hexaen-9-one, *bis*-trifluoroacetate (3.7 mg, 6.72%) as a white solid. MS(ESI) *m/z*: 587.1 $(M+H)^+$. $^1H$ NMR (500MHz, $CD_3OD$) δ 9.02 (s, 1H), 8.77 (d, J=4.8 Hz, 1H), 8.66 (d, J=4.8 Hz, 1H), 8.37 (s, 1H), 7.94 - 7.88 (m, 2H), 7.79 - 7.74 (m, 2H), 7.71 - 7.67 (m, 2H), 7.59 (dd, J=8.1, 4.8 Hz, 1H), 6.39 (s, 1H), 6.09 (dd, J=12.8, 5.1 Hz, 1H), 2.76 (br. s., 1H), 2.28 (t, J=12.8 Hz, 1H), 2.10 - 2.03 (m, 2H), 1.62 - 1.53 (m, 2H), 1.01 (d, J=7.0 Hz, 3H), 0.66 (br. s., 1H). Analytical HPLC (Method A): RT = 7.60 min, purity = 99.0%; Factor XIa Ki = 3.3 nM, Plasma Kallikrein Ki = 200 nM.

Example 118

Preparation of (10*R*,14*S*)-14-{4-[3-chloro-6-(4-chloro-1H-1,2,3-triazol-1-yl)-2-fluorophenyl]-6-oxo-1,6-dihydropyrimidin-1-yl}-10-methyl-3,8,16-triazatricyclo [13.3.1.0$^{2,7}$]nonadeca-1(19),2(7),3,5,15,17-hexaen-9-one *bis*-trifluoroacetate

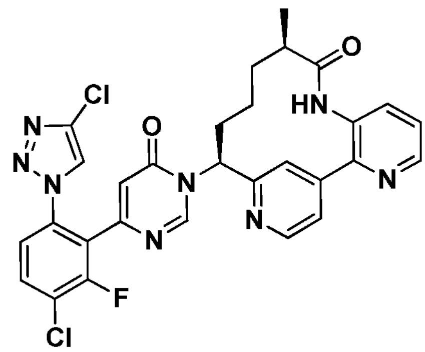

To a vial (4 ml) containing a suspension 6-[3-chloro-6-(4-chloro-1H-1,2,3-triazol-1-yl)-2-fluorophenyl]pyrimidin-4-ol (0.028 g, 0.084 mmol), prepared as described in Intermediate 10, in ACN (1 ml) was added HATU (0.042 g, 0.11 mmol) and DBU (0.019 ml, 0.127 mmol). After 30 min, 10*R*,14*S*)-14-amino-10-methyl-3,8,16-triazatricyclo [13.3.1.0$^{2,7}$]nonadeca-1(19),2(7),3,5,15,17-hexaen-9-one (0.025 g, 0.084 mmol), prepared as described in Intermediate 39, in DMF (1.0 ml) was added at rt. After stirring overnight, the crude mixture was purified by reverse phase chromatography (PHENOMENEX® Luna Axia C18 5μ 30 x 100mm column, Solvent A: 20% ACN - 80% $H_2O$ - 0.1% TFA; Solvent B: 80% ACN - 20% $H_2O$ - 0.1% TFA) to give, after concentration and lyophilization (10*R*,14*S*)-14-{4-[3-chloro-6-(4-chloro-1H-1,2,3-triazol-1-yl)-2-fluorophenyl]-6-oxo-1,6-dihydropyrimidin-1-yl}-10-methyl-3,8,16-triazatricyclo [13.3.1.0$^{2,7}$]nonadeca-1(19),2(7),3,5,15,17-hexaen-9-one, *bis*-trifluoroacetate (13 mg, 17.6%) as a white solid. MS(ESI) *m/z*: 611.2 $(M+H)^+$ and 613.1 $(M+2+H)^+$. $^1H$ NMR (400MHz, $CD_3OD$) δ $^1H$ NMR (500MHz, $CD_3OD-d_4$) d 8.98 (s, 1H), 8.86 - 8.83 (m, 1H), 8.75 - 8.71 (m, 1H), 8.37 (s, 1H), 7.98 (s, 1H), 7.95 - 7.87 (m, 2H), 7.79 - 7.71 (m, 2H), 7.59 - 7.57 (m, 1H), 6.64 (s, 1H), 6.08 (dd, J=12.4, 5.0 Hz, 1H), 2.77 (br. s., 1H), 2.35 - 2.28 (m, 1H), 2.13 - 2.02 (m, 2H), 1.61 - 1.55 (m, 2H), 1.29 (d, J=6.9 Hz, 1H), 1.00 (d, J=7.2 Hz, 3H), 0.66 (br. s., 1H). Analytical HPLC (Method A): RT = 7.11 min, purity = 97.5%; Factor XIa Ki = 1.4 nM, Plasma Kallikrein Ki = 73 nM.

Example 121

Preparation of (10*R*,14*S*)-14-(4-{5-chloro-2-[4-(trifluoromethyl)-1H-1,2,3-triazol-1-yl] phenyl}-6-oxo-1,6-dihydropyrimidin-1-yl)-10-methyl-3,8,16-triazatricyclo[13.3.1.0$^{2,7}$] nonadeca-1(19),2(7),3,5,15,17-hexaen-9-one, *bis*-trifluoroacetate

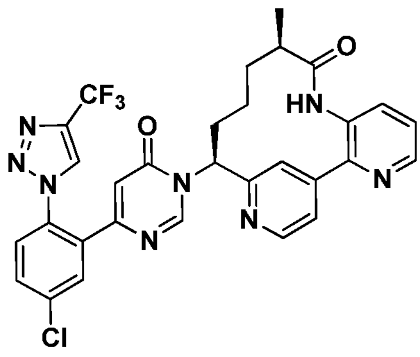

To a vial (4 ml) containing a suspension 6-{5-chloro-2-[4-(trifluoromethyl)-1H-1,2,3-triazol-1-yl]phenyl}pyrimidin-4-ol (0.027 g, 0.078 mmol), prepared as described in Intermediate 15, in ACN (2 ml) was added HATU (0.038 g, 0.101 mmol) and DBU (0.018 ml, 0.116 mmol). After 30 min, (10*R*,14*S*)-14-amino-10-methyl-3,8,16-triazatricycl0[13.3.1.$0^{2,7}$]nonadeca-1(19),2(7),3,5,15,17-hexaen-9-one (0.023 g, 0.078 mmol), prepared as described in Intermediate 39, in ACN (1.0 ml) was added at rt. After 4 h, the crude mixture was purified by reverse phase chromatography (PHENOMENEX® Luna Axia C18 5$\mu$ 30 x 100mm column, Solvent A: 20% ACN - 80% $H_2O$ - 0.1% TFA; Solvent B: 80% ACN - 20% $H_2O$ - 0.1% TFA) to give (10*R*,14*S*)-14-(4-{5-chloro-2-[4-(trifluoromethyl)-1H-1,2,3-triazol-1-yl]phenyl}-6-oxo-1,6-dihydropyrimidin-1-yl)-10-methyl-3,8,16-triazatricyclo[13.3.1.$0^{2,7}$]nonadeca-1(19),2(7),3,5,15,17-hexaen-9-one, bis-trifluoroacetate (18 mg, 26.8%) as a white solid. MS(ESI) *m/z:* 621.2 $(M+H)^+$ and 623.1 $(M+2+H)^+$. $^1H$ NMR (500MHz, $CD_3OD$) $\delta$ 8.82 (s, 1H), 8.72 (d, J=0.8 Hz, 1H), 8.63 (d, J=5.0 Hz, 1H), 8.54 (dd, J=5.0, 1.4 Hz, 1H), 7.80 - 7.78 (m, 2H), 7.70 (dd, J=8.1, 1.5 Hz, 1H), 7.68 - 7.64 (m, 1H), 7.61 - 7.57 (m, 2H), 7.51 (dd, J=8.0, 5.0 Hz, 1H), 6.33 (d, J=0.8 Hz, 1H), 5.95 (dd, J=12.7, 4.7 Hz, 1H), 2.66 - 2.61 (m, 1H), 2.13 (t, J=12.7 Hz, 1H), 1.91 (t, J=9.9 Hz, 2H), 1.47 - 1.39 (m, 2H), 0.87 (d, J=7.2 Hz, 3H). Analytical HPLC (Method A): RT = 8.18 min, purity = 98.3%; Factor XIa Ki = 2.8 nM, Plasma Kallikrein Ki = 190 nM.

Example 132

Preparation of (9*R*,13*S*)-13-[4-(3-chloro-2,6-difluorophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one trifluoroacetate.

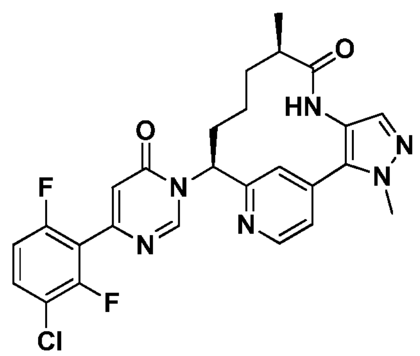

To a 1-dram vial containing a white suspension of 6-(3-chloro-2,6-difluorophenyl) pyrimidin-4-ol (0.013 g, 0.054 mmol), prepared as described in Intermediate 4, and HATU (0.027 g, 0.070 mmol) in ACN (0.54 ml) was added DBU (0.012 ml, 0.081 mmol). The resulting bright yellow solution was stirred at rt for 20 min. Over time this solution became a dull yellow-orange color. Then a clear, brown solution of (9*R*,13*S*)-13-amino-3,9-dimethyl-3,4,7,15-tetraazatricyclo[12.3.1.$0^{2,6}$]octadeca-1(18),2(6),4,14,16-pentaen-8-one, 2 HCl (0.020 g, 0.054 mmol), prepared as described in Intermediate 32, and DBU (0.016 ml, 0.107 mmol) in DMF (0.54 ml) was added. After 2.5 h, the reaction was stopped. Purification by reverse phase chromatography gave (9*R*,13*S*)-13-[4-(3-chloro-2,6-difluorophenyl)-6-oxo-1,6-dihydropyrimidin-1-yl]-3,9-dimethyl-3,4,7,15-tetraazatricyclo[12.3.1.$0^{2}$,]octadeca-1(18),2(6),4,14,16-pentaen-8-one trifluoroacetate (0.0152 g, 43% yield) as an off-white solid. MS(ESI) *m/z*: 525.1 $(M+H)^+$. $^1H$ NMR (500MHz, $CD_3OD$) $\delta$ 9.04 (s, 1H), 8.75 (d, *J*=5.0 Hz, 1H), 7.73 (s, 1H), 7.63 (ddd, *J*=9.1, 8.3, 5.5 Hz, 1H), 7.55 - 7.51 (m, 1H), 7.50 (s, 1H), 7.14 (td, *J*=9.1,

1.7 Hz, 1H), 6.66 (d, J=0.6 Hz, 1H), 6.10 - 6.02 (m, 1H), 4.05 (s, 3H), 2.76 - 2.68 (m, 1H), 2.37 (tt, *J*=12.8, 4.5 Hz, 1H), 2.15 - 2.04 (m, 2H), 1.68 - 1.58 (m, 1H), 1.56 - 1.45 (m, 1H), 1.02 (d, *J*=6.9 Hz, 3H), 0.80 - 0.65 (m, 1H). $^{19}F$ NMR (471MHz, $CD_3OD$) δ -77.53 (s), -114.79 (d, *J*=4.3 Hz), -115.50 (d, *J*=4.3 Hz). Analytical HPLC (Method A): RT = 7.37 min, purity = 99.7%; Factor XIa Ki = 33 nM, Plasma Kallikrein Ki = 290 nM.

Example 138

Preparation of (9R,13S)-13-[4-(2-bromo-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one

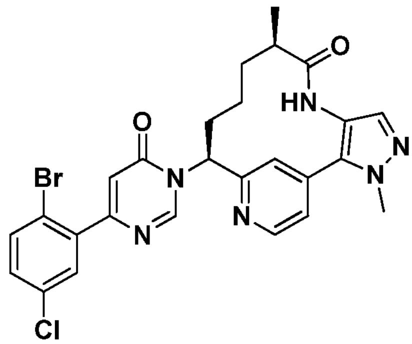

(9*R*,13*S*)-13-[4-(2-Bromo-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate (160 mg, 46.8% yield) was prepared in a similar manner as the procedure described in Example 56, by replacing 6-{5-chloro-2-[4-(trifluoromethyl)-1H-1,2,3-triazol-1-yl]phenyl}pyrimidin-4-ol (22.8 mg, 0.067 mmol), prepared as described in Intermediate 15, with 6-(2-bromo-5-chlorophenyl)pyrimidin-4-ol (143 mg, 0.501 mmol), prepared as described in Intermediate 44. MS(ESI) *m/z*: 567.1 $(M+H)^+$. $^1H$ NMR (400MHz, $CD_3OD$) δ 9.04 (s, 1H), 8.75 (d, *J*=5.3 Hz, 1H), 7.76 (s, 1H), 7.70 (d, *J*=8.6 Hz, 1H), 7.61 - 7.54 (m, 2H), 7.50 (s, 1H), 7.38 (dd, *J*=8.6, 2.4 Hz, 1H), 6.70 (s, 1H), 6.05 (dd, *J*=12.5, 4.0 Hz, 1H), 4.05 (s, 3H), 2.78 - 2.66 (m, 1H), 2.44 - 2.33 (m, 1H), 2.16 - 2.03 (m, 2H), 1.70 - 1.43 (m, 2H), 1.02 (d, *J*=6.8 Hz, 3H), 0.82 - 0.66 (m, 1H). Analytical HPLC (Method A): RT = 8.45 min, 99.9% purity; Factor XIa Ki = 23 nM, Plasma Kallikrein Ki = 420 nM.

Example 140

Preparation of (9*R*,13*S*)-13-(4-{5-chloro-2-[1-(difluoromethyl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one

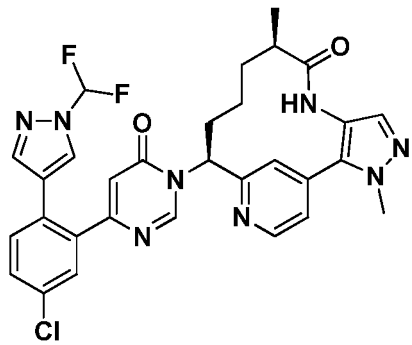

140A. Preparation of 4-(5-chloro-2-(1-(difluoromethyl)-1H-pyrazol-4-yl)phenyl)-6-methoxypyrimidine

To a sealable vial was added 4-(2-bromo-5-chlorophenyl)-6-methoxypyrimidine (0.08 g, 0.267 mmol), 1-(difluoromethyl)-4-(4,4,5,5-tetramethyl-1,3,2-dioxaborolan-2-yl)-1H-pyrazole (0.072 g, 0.294 mmol), 3 M aq $KH_2PO_4$ (0.27 ml, 0.81 mmol) and THF (2.67 ml). Ar was bubbled through the reaction mixture for several min and (DtBPF)$PdCl_2$ (8.70

mg, 0.013 mmol) was added. The vial was sealed and heated at 90 °C for 15 h. The reaction was cooled to rt, diluted with EtOAc, washed with brine, dried over $Na_2SO_4$, filtered, and concentrated. Purification by normal phase chromatography afforded 4-(5-chloro-2-(1-(difluoromethyl)-1H-pyrazol-4-yl)phenyl)-6-methoxypyrimidine (0.05 g, 56% yield) as a white solid. MS(ESI) *m/z*: 337.2 $(M+H)^+$. $^1H$ NMR (400MHz, $CDCl_3$) δ 8.81 (d, *J*=0.9 Hz, 1H), 7.69 (s, 1H), 7.54 (d, *J*=2.2 Hz, 1H), 7.48 - 7.43 (m, 1H), 7.41 - 7.35 (m, 2H), 7.17 (t, *J*=54.0 Hz, 1H), 6.61 (d, *J*=1.1 Hz, 1H), 3.99 (s, 3H).

140B. Preparation of 6-(5-chloro-2-(1-(difluoromethyl)-1H-pyrazol-4-yl)phenyl) pyrimidin-4-ol

A clear solution of 4-(5-chloro-2-(1-(difluoromethyl)-1H-pyrazol-4-yl)phenyl)-6-methoxypyrimidine (0.05 g, 0.148 mmol) in HOAc (0.742 ml) and 48% aq HBr (0.84 ml, 7.42 mmol) was warmed to 65 °C. After 3 h, the reaction was cooled to rt and concentrated. The residue was dissolved in EtOAc, washed with sat $NaHCO_3$, brine, dried over $Na_2SO_4$, filtered, and concentrated. $Et_2O$ (3 ml) was added and the mixture was sonicated. The solid was collected by filtration and rinsed with $Et_2O$ (2 ml), air-dried to give 6-(5-chloro-2-(1-(difluoromethyl)-1H-pyrazol-4-yl)phenyl)pyrimidin-4-ol (0.03 g, 63% yield) as a white solid. MS(ESI) *m/z*: 323.2 $(M+H)^+$. $^1H$ NMR (400MHz, $CD_3OD$) δ 8.20 (d, *J*=1.1 Hz, 1H), 8.05 (s, 1H), 7.61 - 7.27 (m, 5H), 6.40 (d, *J*=1.1 Hz, 1H).

140C. Preparation of (9*R*,13*S*)-13-(4-{5-chloro-2-[1-(difluoromethyl)-1H-pyrazol-4-yl] phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one trifluoroacetate

(9*R*,13*S*)-13-(4-{5-Chloro-2-[1-(difluoromethyl)-1H-pyrazol-4-yl]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one trifluoroacetate (10 mg, 32% yield) was prepared in a similar manner as the procedure described in Example 56, by using 6-(5-chloro-2-(1-(difluoromethyl)-1H-pyrazol-4-yl)phenyl)pyrimidin-4-ol (14.01 mg, 0.043 mmol). MS(ESI) *m/z*: 605.2 $(M+H)^+$. $^1H$ NMR (400MHz, $CD_3OD$) δ 8.95 (s, 1H), 8.74 (d, *J=5.3* Hz, 1H), 8.04 (s, 1H), 7.72 (s, 1H), 7.60 - 7.25 (m, 7H), 6.42 (s, 1H), 6.02 (dd, *J*=12.7, 4.3 Hz, 1H), 4.05 (s, 3H), 2.76 - 2.66 (m, 1H), 2.39 - 2.27 (m, 1H), 2.14 - 1.99 (m, 2H), 1.67 - 1.42 (m, 2H), 1.02 (d, *J*=6.8 Hz, 3H), 0.81 - 0.65 (m, 1H). Analytical HPLC (Method A): RT = 8.18 min, 98.5% purity; Factor XIa Ki = 9 nM, Plasma Kallikrein Ki = 910 nM.

Example 148

Preparation of (10*R*,14*S*)-14-(4-{5-chloro-2-[4-(trifluoromethyl)-1H-1,2,3-triazol-1-yl] phenyl}-6-oxo-1,6-dihydropyrimidin-1-yl)-10-methyl-9-oxo-3,8-diazatricyclo[13.3.1.$0^{2,7}$] nonadeca-1(19),2(7),3,5,15,17-hexaene-5-carbonitrile

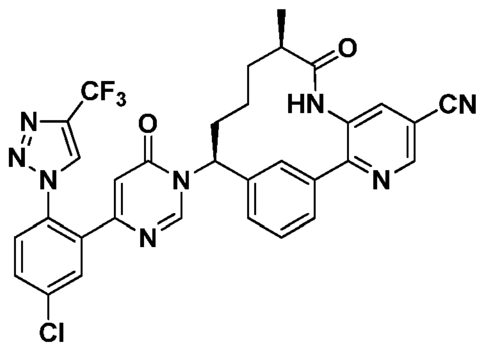

148A. Preparation of *tert*-butyl N-[(10R,14S)-5-bromo-10-methyl-9-oxo-3,8-diazatricyclo[13.3.1.$0^{2,7}$]nonadeca-1(19),2(7),3,5,15,17-hexaen-14-yl]carbamate

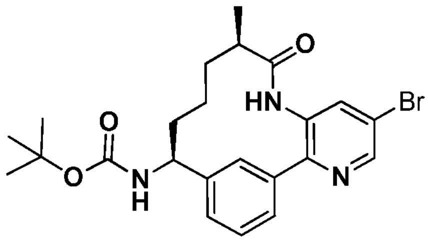

*tert*-Butyl N-[(10R,14S)-5-bromo-10-methyl-9-oxo-3,8-diazatricyclo[13.3.1.$0^{2,7}$] nonadeca-1(19),2(7),3,5,15,17-hexaen-14-yl]carbamate was prepared (0.1 g, 49% yield, dark solid) in a similar manner as Example 81C replacing 2-bromo-5-methoxypyridin-3-amine with 2,5-dibromopyridin-3-amine. LCMS $(M+H)^+$ 474-476.08.

148B. Preparation of *tert*-butyl N-[(10R,14S)-5-cyano-10-methyl-9-oxo-3,8-diazatricyclo[13.3.1.$0^{2,7}$]nonadeca-1(19),2(7),3,5,15,17-hexaen-14-yl]carbamate

To *tert*-butyl *N*-[(10R,14S)-5-bromo-10-methyl-9-oxo-3,8-diazatricyclo [13.3.1.$0^{2,7}$]nonadeca-1(19),2(7),3,5,15,17-hexaen-14-yl]carbamate (0.1 g, 0.211 mmol) in a microwave tube was added $Zn(CN)_2$ (0.037 g, 0.316 mmol), Zn (4.13 mg, 0.063 mmol) and DMF (2.1 ml). The mixture was purged with Ar for several min. $Pd(t\text{-}Bu_3P)_2$ (10.77 mg, 0.021 mmol) was added. The reaction was sealed and heated at 80 °C for 18 h. The reaction was partitioned with water (10 ml) and EtOAc (30 ml). The aqueous layer was extracted with EtOAc (2 x 10 ml). The combined organic layers were washed with brine (10 ml), dried ($MgSO_4$), filtered and concentrated. The residue was purified normal phase chromatography using 100% DCM to 10% MeOH as eluents to give tert-butyl N-[(10R,14S)-5-cyano-10-methyl-9-oxo-3,8-diazatricyclo[13.3.1.$0^{2,7}$]nonadeca-1(19),2(7),3,5,15,17 -hexaen-14-yl]carbamate (20 mg, 22.56% yield). LCMS $(M+H)^+$ 421.3.

148C. Preparation of (10R,14S)-14-amino-10-methyl-9-oxo-3,8-diazatricyclo [13.3.1.$0^{2,7}$]nonadeca-1(19),2(7),3,5,15,17-hexaene-5-carbonitrile

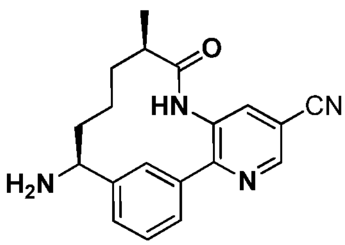

*tert*-Butyl N-[(10R,14S)-5-cyano-10-methyl-9-oxo-3,8-diazatricyclo[13.3.1.$0^{2,7}$] nonadeca-1(19),2(7),3,5,15,17-hexaen-14-yl]carbamate (0.02 g, 0.048 mmol), Example 148B, was deprotected and the free-base was produced in a similar manner as Example 81D to afford (10R,14S)-14-amino-10-methyl-9-oxo-3,8-diazatricyclo[13.3.1.$0^{2,7}$] nonadeca-1(19),2(7),3,5,15,17-hexaene-5-carbonitrile (13 mg, 85%) as a tan solid. LCMS $(M+H)^+$ 321.3.

148D. Preparation of (10*R*,14*S*)-14-(4-{5-chloro-2-[4-(trifluoromethyl)-1H-1,2,3-triazol-1-yl]phenyl}-6-oxo-1,6-dihydropyrimidin-1-yl)-10-methyl-9-oxo-3,8-diazatricyclo [13.3.1.$0^{2,7}$]nonadeca-1(19),2(7),3,5,15,17-hexaene-5-carbonitrile

To 6-{5-chloro-2-[4-(trifluoromethyl)-1*H*-1,2,3-triazol-1-yl]phenyl}pyrimidin-4-ol, described in Intermediate 15, (0.014 g, 0.041 mmol) and HATU (0.020 g, 0.053 mmol) in a small vial was added DBU (9.17 μl, 0.061 mmol) in ACN (0.2 mL). After 30min, (10R,14S)-14-amino-10-methyl-9-oxo-3,8-diazatricyclo[13.3.1.$0^{2,7}$]nonadeca-1(19),2(7),3,5,15,17-hexaene-5-carbonitrile was added in DMF (0.4 ml). After 18 h, the reaction was diluted with DMF, filtered and concentrated. The residue was purified by reverse phase HPLC using PHENOMENEX® Luna 5U 30x100mm (10:90 $ACN/H_2O$ to 90:10 $ACN/H_2O$, 0.1% TFA) (20% B start, 14 min gradient) to afford (10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-9-oxo-3,8-diazatricyclo[13.3.1.$0^{2,7}$]nonadeca-1(19),2(7),3,5,15,17-hexaene-5-carbonitrile (4.9 mg, 14%) as a white solid. MS(ESI) *m*/*z*: 645.4 $(M+H)^+$. $^1H$ NMR (400MHz, $CD_3OD$) δ 8.98 - 8.94 (m, 1H), 8.80 (s, 1H), 8.30 - 8.24 (m, 1H), 8.18 - 8.13 (m, 1H), 7.96 - 7.91 (m, 1H), 7.81 - 7.76 (m, 2H), 7.74 - 7.68 (m, 1H), 7.60 (t, *J*=7.7 Hz, 1H), 7.32 (s, 1H), 6.49 - 6.46 (m, 1H), 5.81

(dd, *J*=12.7, 3.4 Hz, 1H), 2.56 (d, *J*=7.0 Hz, 1H), 2.31 (d, *J*=5.1 Hz, 1H), 2.16 - 2.08 (m, 1H), 1.93 (d, *J*=7.3 Hz, 1H), 1.58 (d, *J*=7.3 Hz, 1H), 1.46 (d, *J*=6.8 Hz, 1H), 1.37 (br. s., 1H), 1.26 - 1.21 (m, 1H), 1.17 (s, 3H). Analytical HPLC (Method A) RT = 9.37 min, purity = 90%; Factor XIa Ki = 1.8 nM, Plasma Kallikrein Ki = 120 nM.

Example 152

Preparation of (9*R*,13*S*)-13-(4-{5-chloro-2-[4-(trifluoromethyl)-1H-pyrazol-1-yl]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14, 16-pentaen-8-one trifluoroacetate

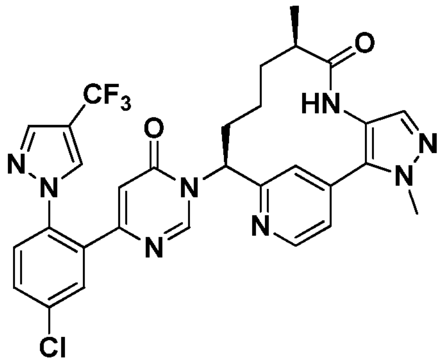

152A. Preparation of 4-{5-chloro-2-[4-(trifluoromethyl)-1H-pyrazol-1-yl]phenyl}-6-methoxypyrimidine

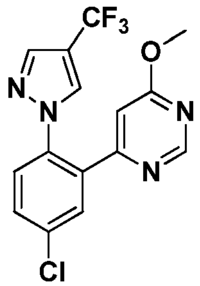

To a solution of 4-(trifluoromethyl)-1H-pyrazole (34.2 mg, 0.251 mmol) in DMF (698 μl) was added NaH (12.57 mg, 0.314 mmol) in 2 portions and the reaction was stirred at rt for 30 min. 4-(5-Chloro-2-fluorophenyl)-6-methoxypyrimidine (50 mg, 0.210 mmol), prepared as described in Intermediate 5A, was added and the solution was stirred at rt for 2 h and then heated at 85 °C overnight. The reaction mixture was quenched with water and MeOH, then concentrated. Purification by normal phase chromatography gave 4-{5-chloro-2-[4-(trifluoromethyl)-1H-pyrazol-1-yl]phenyl}-6-methoxypyrimidine (60 mg, 32.3% yield). MS(ESI) *m/z*: 355.3 (M+H)$^+$. $^1$H NMR (400MHz, $CDCl_3$) δ 8.73 (d, *J*=0.9 Hz, 1H), 7.81 (s, 1H), 7.77 (d, *J*=2.4 Hz, 1H), 7.70 (s, 1H), 7.59 - 7.53 (m, 1H), 7.51 - 7.47 (m, 1H), 6.36 (d, *J*=1.1 Hz, 1H), 3.98 - 3.94 (m, 3H).

152B. Preparation of 6-(5-chloro-2-(4-(trifluoromethyl)-1H-pyrazol-1-yl)phenyl) pyrimidin-4-ol.

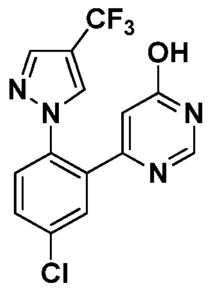

6-{5-Chloro-2-[4-(trifluoromethyl)-1H-pyrazol-1-yl]phenyl}pyrimidin-4-ol (23 mg, 25.5% yield) was prepared in a similar manner as the procedure described in Intermediate 9E, by using 4-{5-chloro-2-[4-(trifluoromethyl)-1H-pyrazol-1-yl]phenyl}-6-methoxypyrimidine (60 mg, 0.169 mmol). MS(ESI) *m/z:* 341.3(M+H)$^+$. $^1$H NMR (400MHz, $CDCl_3$) δ 7.97 (s, 1H), 7.90 (d, *J*=0.7 Hz, 1H), 7.82 (s, 1H), 7.70 (d, *J*=2.2 Hz, 1H), 7.63 - 7.55 (m, 1H), 7.53 - 7.47 (m, 1H), 6.27 (d, *J*=0.9 Hz, 1H).

152C. Preparation of (9*R*,13*S*)-13-(4-{5-chloro-2-[4-(trifluoromethyl)-1H-pyrazol-1-yl] phenyl}-6-oxo-1,6-dihydropyrimidin-1-yl)-3,9-dimethyl-3,4,7,15-tetraazatricyclo [12.3.1.0$^{2,6}$]octadeca-1(18),2(6),4,14,16-pentaen-8-one trifluoroacetate

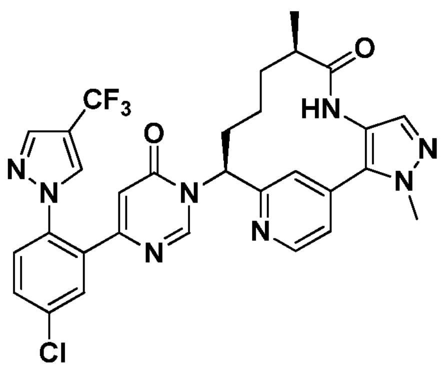

(9*R*,13*S*)-13-(4-{5-Chloro-2-[4-(trifluoromethyl)-1H-pyrazol-1-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one trifluoroacetate (20 mg, 40.2% yield) was prepared in a similar manner as the procedure described in Example 56, by using 6-{5-chloro-2-[4-(trifluoromethyl)-1H-pyrazol-1-yl]phenyl}pyrimidin-4-ol (22.76 mg, 0.067 mmol). MS(ESI) *m*/*z*: 623.2 (M+H)$^+$. $^1$H NMR (400MHz, $CD_3OD$) δ 8.85 (s, 1H), 8.71 (d, *J*=5.1 Hz, 1H), 8.32 (s, 1H), 7.90 - 7.80 (m, 2H), 7.71 - 7.64 (m, 2H), 7.62 - 7.57 (m, 1H), 7.52 (dd, *J*=5.2, 1.7 Hz, 1H), 7.49 (s, 1H), 6.20 (d, *J*=0.7 Hz, 1H), 5.98 (dd, *J*=12.7, 4.3 Hz, 1H), 4.05 (s, 3H), 2.70 (td, *J*=6.7, 3.2 Hz, 1H), 2.30 (tt, *J*=12.7, 4.4 Hz, 1H), 2.13 - 1.94 (m, 2H), 1.68 - 1.53 (m, 1H), 1.47 (ddd, *J*=14.9, 9.8, 5.3 Hz, 1H), 1.01 (d, *J*=6.8 Hz, 3H), 0.70 (m, 1H). $^{19}$F NMR (376MHz, $CD_3OD$) δ -57.99 (s), -77.75 (s). Analytical HPLC (Method A): RT = 8.98 min, purity = 99.1%; Factor XIa Ki = 10 nM, Plasma Kallikrein Ki = 4,900 nM.

Example 161

Preparation of (9*R*,13*S*)-13-{4-[5-chloro-2-(pyridin-3-yl)phenyl]-6-oxo-1,6-dihydropyrimidin-1-yl}-3,9-dimethyl-3,4,7,15-tetraazatricyclo[12.3.1.0$^{2,6}$] octadeca-1(18),2(6),4,14,16-pentaen-8-one

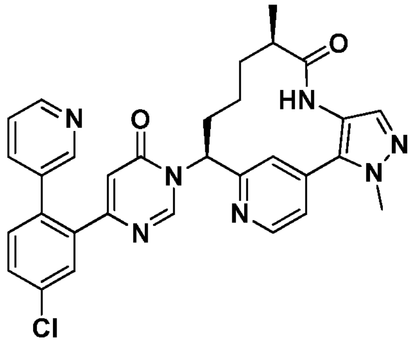

In a microwave vial was added (9*R*,13*S*)-13-[4-(2-bromo-5-chlorophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one (0.02 g, 0.035 mmol), prepared in Example 138, pyridin-3-ylboronic acid (4.76 mg, 0.039 mmol), 3 M aq $K_3PO_4$ (0.035 ml, 0.106 mmol) and THF (1 ml). Ar was bubbled through the reaction for several min and then (DtBPF)$PdCl_2$ (1.15 mg, 1.761 μmol) was added. The reaction mixture was microwaved at 150 °C for 1 h, cooled to rt, and concentrated. Purification by reverse phase chromatography afforded (9*R*,13*S*)-13-{4-[5-chloro-2-(pyridin-3-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (3.21 mg, 12% yield) as a yellow solid. MS(ESI) *m/z*: 566.2 $(M+H)^+$. $^1H$ NMR (500MHz, $CD_3OD$) δ 8.79 - 8.68 (m, 4H), 8.34 (dt, *J*=8.2, 1.7 Hz, 1H), 7.90 (dd, *J*=8.0, 5.8 Hz, 1H), 7.81 (d, *J*=2.2 Hz, 1H), 7.71 - 7.66 (m, 2H), 7.57 (d, *J*=8.3 Hz, 1H), 7.53 - 7.47 (m, 2H), 6.52 (d, *J*=0.5 Hz, 1H), 5.98 - 5.90 (m, 1H), 4.04 (s, 3H), 2.74 - 2.64 (m, 1H), 2.30 - 2.19 (m, 1H), 2.10 - 1.93 (m, 2H), 1.63 - 1.53 (m, 1H), 1.50 - 1.40 (m, 1H), 1.00 (d, *J*=6.9 Hz, 3H), 0.77 - 0.61 (m, 1H). Analytical HPLC (Method A): RT = 4.22 min, 99.8% purity; Factor XIa Ki = 100 nM, Plasma Kallikrein Ki = 5,700 nM.

Example 162 (reference)

Preparation of (9*R*,13S)-13-(4-{5-chloro-2-[4-(2-hydroxypropan-2-yl)-1H-1,2,3-triazol-1-yl]phenyl}-6-oxo-1,6-dihydropyrimidin-1-yl)-3-(difluoromethyl)-9-methyl-3,4,7,15-tetraazatricyclo[12.3.1.$0^{2,6}$]octadeca-1(18),2(6),4,14,16-pentaen-8-one

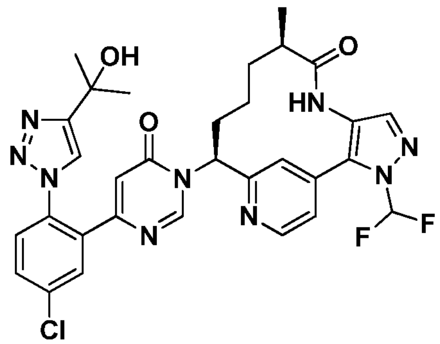

162A. Preparation of 6-{5-chloro-2-[4-(2-hydroxypropan-2-yl)-1H-1,2,3-triazol-1-yl] phenyl}pyrimidin-4-ol

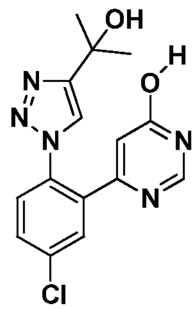

6-{5-Chloro-2-[4-(2-hydroxypropan-2-yl)-1H-1,2,3-triazol-1-yl]phenyl} pyrimidin-4-ol was prepared by the condensation of an ACN (5 ml) solution consisting of 6-(2-azido-5-chlorophenyl)pyrimidin-4-ol (0.9g, 3.44 mmol) and 2-methylbut-3-yn-2-ol (0.289 g, 3.44 mmol) in the presence $Cu_2O$ (0.05 g). The product, after ISCO silica gel chromatography with hexane:EtOAc as eluants, was obtained as an oil (0.5 g). $^1H$ NMR (400MHz, $CDCl_3$) $\delta$ 8.60 - 8.57 (m, 1H), 7.65 - 7.59 (m, 1H), 7.56 - 7.53 (m, 1H), 7.37 - 7.32 (m, 1H), 6.76 - 6.72 (m, 1H), 3.95 (s, 3H), 3.43 - 3.30 (m, 1H), 1.52 (s, 6H). The oil was then dissolved in AcOH (1 ml) and to this was added 48% aq HBr (0.5 ml) and the reaction was sealed. The reaction mixture was heated at 80 °C for 2 h and then concentrated to a gummy solid and water (10 ml) was added. A solid precipitated and was collected by decanting the solution. The solid was washed several times with water and the residue was dissolved in MeOH and the solution was concentrated to give 6-{5-chloro-2-[4-(2-hydroxypropan-2-yl)-1H-1,2,3-triazol-1-yl]phenyl}pyrimidin-4-ol (0.15 g, 13%) as a foam. LCMS *m/z* = 332.1$(M+H)^+$. $^1H$ NMR (400MHz, $CDCl_3$) $\delta$ 11.51 - 11.06 (m, 1H), 8.33 - 8.09 (m, 1H), 7.90 - 7.78 (m, 1H), 7.71 (t, J=7.9 Hz, 1H), 7.39 - 7.29 (m, 1H), 6.87 - 6.68 (m, 1H), 5.80 - 5.66 (m, 1H), 5.27 - 5.17 (m, 1H), 2.12 (s, 6H).

162B. Preparation of (9*R*,13S)-13-(4-{5-chloro-2-[4-(2-hydroxypropan-2-yl)-1H-1,2,3-triazol-1-yl]phenyl}-6-oxo-1,6-dihydropyrimidin-1-yl)-3-(difluoromethyl)-9-methyl-3,4,7,15-tetraazatricyclo[12.3.1.$0^{2,6}$]octadeca-1(18),2(6),4,14,16-pentaen-8-one

(9*R*,13S)-13-(4-{5-Chloro-2-[4-(2-hydroxypropan-2-yl)-1H-1,2,3-triazol-1-yl] phenyl}-6-oxo-1,6-dihydropyrimidin-1-yl)-3-(difluoromethyl)-9-methyl-3,4,7,15-tetraazatricyclo[12.3.1.$0^{2,6}$]octadeca-1(18),2(6),4,14,16-pentaen-8-one was prepared (4.2 mg, 21% yield) as a solid via the coupling of 6-{5-chloro-2-[4-(2-hydroxypropan-2-yl)-1H-1,2,3-triazol-1-yl]phenyl}pyrimidin-4-ol (0.01 g, 0.030 mmol) and (9R,13S)-13-amino-3-(difluoromethyl)-9-methyl-3,4,7,15-tetraazatricyclo[12.3.1.$0^{2,6}$]octadeca-1(18),2(6),4,14,16-pentaen-8-one (0.010 g, 0.030 mmol) using the HATU, DBU coupling methodology as described in Example 56. MS *m/z* = 651.1$(M+H)^+$. $^1H$ NMR (500MHz, DMSO-$d_6$) $\delta$ 9.46 - 9.40 (m, 1H), 8.88 - 8.82 (m, 1H), 8.72 - 8.65 (m, 1H), 8.40 - 8.36 (m, 1H), 8.01 - 7.93 (m, 2H), 7.89 (s, 1H), 7.72 - 7.68 (m, 1H), 7.68 - 7.61 (m, 1H), 7.46 - 7.40 (m, 1H), 6.63 - 6.57 (m, 1H), 5.94 - 5.85 (m, 1H), 5.70 - 5.64 (m, 1H), 5.15 - 5.08 (m, 1H), 2.71 - 2.61 (m, 1H), 2.56(s, 3H), 2.35 - 2.16 (m, 1H), 2.08 - 2.04 (m, 4H), 2.07 - 1.79 (m, 1H), 1.56 - 1.26 (m, 1H), 0.93 - 0.79 (d, 3H), 0.44 - 0.24 (m, 1H). Analytical HPLC (Method B): RT = 1.75 min, purity = 97%; Factor XIa Ki = 1 nM, Plasma Kallikrein Ki = 230 nM.

Example 166

Preparation of 5-(4-chloro-2-{1-[(9R,13S)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)pyridine-3-carbonitrile.

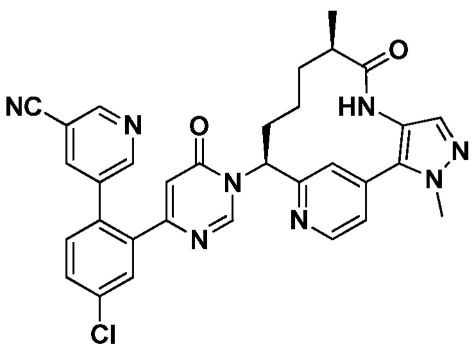

5-(4-Chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)pyridine-3-carbonitrile, 2 trifluoroacetate (1.5 mg, 5% yield) was prepared in a similar manner as the procedure described in Example 161, by replacing pyridin-3-ylboronic acid (4.76 mg, 0.039 mmol) with (5-cyanopyridin-3-yl)boronic acid (5.73 mg, 0.039 mmol). MS(ESI) *m*/*z*: 591.2 $(M+H)^+$. $^1H$ NMR (500MHz, $CD_3OD$) δ 8.84 (d, *J*=1.9 Hz, 1H), 8.79 (s, 1H), 8.72 (d, *J*=5.2 Hz, 1H), 8.64 (d, *J*=2.2 Hz, 1H), 8.08 (t, *J*=2.1 Hz, 1H), 7.75 (d, *J*=2.2 Hz, 1H), 7.67 - 7.62 (m, 2H), 7.54 - 7.47 (m, 3H), 6.43 (d, *J*=0.8 Hz, 1H), 6.00 - 5.94 (m, 1H), 4.04 (s, 3H), 2.74 - 2.66 (m, 1H), 2.31 - 2.23 (m, 1H), 2.11 - 1.94 (m, 2H), 1.64 - 1.54 (m, 1H), 1.51 - 1.42 (m, 1H), 1.00 (d, *J*=6.9 Hz, 3H), 0.75 - 0.60 (m, 1H). Analytical HPLC (Method A): RT = 6.86 min, 99.5% purity; Factor XIa Ki = 58 nM, Plasma Kallikrein Ki = 6,500 nM.

Example 168

Preparation of methyl 4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}benzoate

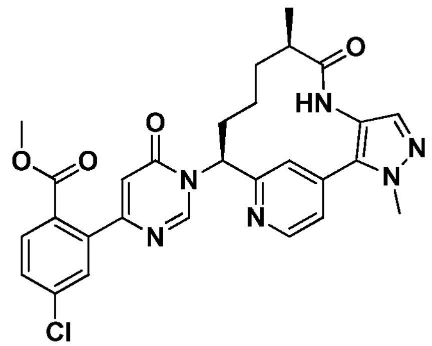

168A. Preparation of methyl 4-chloro-2-(6-methoxypyrimidin-4-yl)benzoate, and

168B. Preparation 4-chloro-2-(6-methoxypyrimidin-4-yl)benzoic acid

A suspension of 4-chloro-6-methoxypyrimidine (0.067 g, 0.466 mmol) and (5-chloro-2-(methoxycarbonyl)phenyl)boronic acid (0.1 g, 0.466 mmol) in ACN (1.8 ml) was purged with Ar for several min, then 2 M $Na_2CO_3$ aq (0.47 ml, 0.94 mmol) was added, followed by $Pd(Ph_3P)_4$ (0.027 g, 0.023 mmol). The vial was capped and microwaved at 130 °C for 0.5 h, then cooled to rt. The reaction was diluted with EtOAc, washed with brine, dried over $Na_2SO_4$, filtered, and concentrated. Purification by normal phase chromatography afforded methyl 4-chloro-2-(6-methoxypyrimidin-4-yl)benzoate (0.086 g, 66% yield) as a colorless oil. MS(ESI) *m*/*z:* 279.0 $(M+H)^+$. The aqueous layer from the work-up was neutralized with 1 N HCl to afford a white cloudy suspension. The mixture was filtered, and the solid was rinsed with water and air-dried to afford 4-chloro-2-(6-methoxypyrimidin-4-yl)benzoic acid (0.026 g, 21% yield) as a white solid. MS(ESI) *m*/*z*: 265.0 $(M+H)^+$.

168C. Preparation of methyl 4-chloro-2-(6-hydroxypyrimidin-4-yl)benzoate

Methyl 4-chloro-2-(6-hydroxypyrimidin-4-yl)benzoate (0.046 g, 56% yield) was prepared in a similar manner as the procedure described in Example 140B, by replacing 4-(5-chloro-2-(1-(difluoromethyl)-1H-pyrazol-4-yl)phenyl)-6-methoxypyrimidine with methyl 4-chloro-2-(6-methoxypyrimidin-4-yl)benzoate (0.086 g, 0.309 mmol). MS(ESI) *m*/*z*: 265.1 $(M+H)^+$. $^1H$ NMR (500MHz, $CD_3OD$) δ 8.19 (d, *J*=1.1 Hz, 1H), 7.83 (d, *J*=8.3 Hz, 1H), 7.62 - 7.57 (m, 2H), 6.57 (d, *J*=0.8 Hz, 1H), 3.76 (s, 3H).

168D. Preparation of methyl 4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}benzoate trifluoroacetate

Methyl 4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}benzoate trifluoroacetate (0.067 g, 58% yield) was prepared in a similar manner as the procedure described in Example 56, by using methyl 4-chloro-2-(6-hydroxypyrimidin-4-yl)benzoate (0.046 g, 0.174 mmol), prepared as described in Example 168C. MS(ESI) *m*/*z*: 547.2 $(M+H)^+$. $^1H$ NMR

(500MHz, $CD_3OD$) δ 8.99 (s, 1H), 8.74 (d, *J*=5.2 Hz, 1H), 7.83 (d, *J*=8.3 Hz, 1H), 7.75 (s, 1H), 7.62 (d, *J*=1.9 Hz, 1H), 7.59 (dd, *J*=8.3, 2.2 Hz, 1H), 7.55 (dd, *J*=5.2, 1.7 Hz, 1H), 7.50 (s, 1H), 6.62 (s, 1H), 6.07 (dd, *J*=12.7, 4.1 Hz, 1H), 4.05 (s, 3H), 3.75 (s, 3H), 2.76 - 2.68 (m, 1H), 2.41 - 2.33 (m, 1H), 2.14 - 2.03 (m, 2H), 1.67 - 1.58 (m, 1H), 1.55 - 1.45 (m, 1H), 1.02 (d, *J*=6.9 Hz, 3H), 0.79 - 0.66 (m, 1H). Analytical HPLC (Method A): RT = 6.69 min, 99.9% purity; Factor XIa Ki = 27 nM, Plasma Kallikrein Ki = 650 nM.

Example 171

Preparation of (9*R*,13*S*)-13-{4-[3-chloro-2-fluoro-6-(trifluoromethyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate

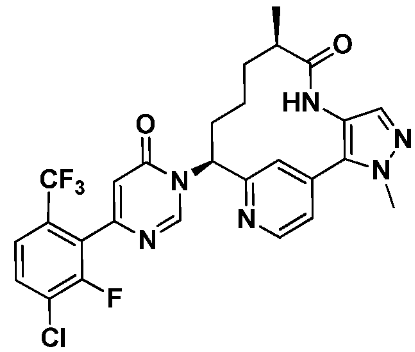

(9*R*,13*S*)-13-{4-[3-Chloro-2-fluoro-6-(trifluoromethyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (0.016 g, 40.1% yield) was prepared in a similar manner as the procedure described in Example 162, using 6-(3-chloro-2-fluoro-6-(trifluoromethyl)phenyl)pyrimidin-4-ol hydrobromide (0.021 g, 0.057 mmol) and (9*R*,13*S*)-13-amino-3,9-dimethyl-3,4,7,15-tetraazatricyclo[12.3.1.0$^{2,6}$]octadeca-1(18),2(6),4,14,16-pentaen-8-one (0.02 g, 0.057 mmol) prepared as described in Intermediate 32. MS(ESI) *m/z*: 575.3 $(M+H)^+$. $^1H$ NMR (400MHz, $CD_3OD$) δ 9.05 (s, 1H), 8.77 (d, *J*=5.1 Hz, 1H), 7.85 - 7.79 (m, 1H), 7.75 (s, 1H), 7.66 (d, *J*=8.6 Hz, 1H), 7.55 (dd, *J*=5.3, 1.5 Hz, 1H), 7.50 (s, 1H), 6.60 (s, 1H), 6.08 (dd, *J*=12.7, 4.3 Hz, 1H), 4.06 (s, 3H), 2.77 - 2.67 (m, 1H), 2.45 - 2.34 (m, 1H), 2.16 - 2.04 (m, 2H), 1.70 - 1.44 (m, 2H), 1.02 (d, *J*=7.0 Hz, 3H), 0.81 - 0.64 (m, 1H). $^{19}F$ NMR (376MHz, $CD_3OD$) δ -59.04 (s), -77.76 (s), -115.37 (s). Analytical HPLC (Method A): RT = 8.30 min, 99.3% purity; Factor XIa Ki = 12 nM, Plasma Kallikrein Ki = 190 nM.

Example 180

Preparation of (9*R*,13*S*)-13-[4-(3-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one

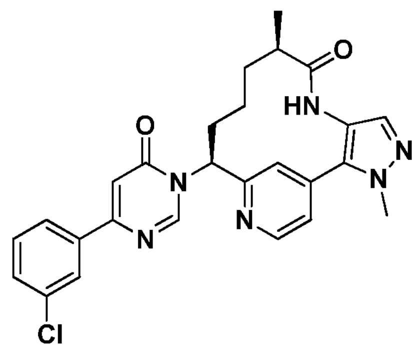

(9*R*,13*S*)-13-[4-(3-Chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate (3.35 mg, 15% yield) was prepared in a similar

manner as the procedure described in Example 161, by replacing pyridin-3-ylboronic acid (4.76 mg, 0.039 mmol) with lithium 4-methyl-1-(1-methyl-1H-1,2,3-triazol-4-yl)-2,6,7-trioxa-1-borabicyclo [2.2.2]octan-1-uide (8.41 mg, 0.039 mmol). MS(ESI) *m/z*: 489.3 (M+H)+. $^1$H NMR (500MHz, $CD_3OD$) δ 9.01 (s, 1H), 8.73 (d, *J*=5.2 Hz, 1H), 8.08 (t, *J*=1.7 Hz, 1H), 7.94 (dt, *J*=7.5, 1.6 Hz, 1H), 7.73 (s, 1H), 7.54 - 7.44 (m, 4H), 6.93 (d, *J*=0.5 Hz, 1H), 6.04 (dd, *J*=12.5, 4.0 Hz, 1H), 4.05 (s, 3H), 2.76 - 2.68 (m, 1H), 2.42 - 2.32 (m, 1H), 2.15 - 2.02 (m, 2H), 1.67 - 1.57 (m, 1H), 1.55 - 1.45 (m, 1H), 1.02 (d, *J*=6.9 Hz, 3H), 0.81 - 0.65 (m, 1H). Analytical HPLC (Method A): RT = 7.33 min, 99.0% purity; Factor XIa Ki = 360 nM, Plasma Kallikrein Ki = 6,800 nM.

Example 181

Preparation of 4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}benzoic acid

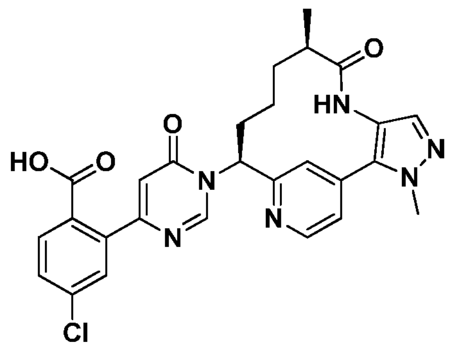

To the solution of methyl 4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}benzoate trifluoroacetate (0.02 g, 0.030 mmol), prepared as described in Example 168, in DCM (0.5 ml) at 0 °C was added $BBr_3$ (0.029 ml, 0.30 mmol). The reaction became a yellow suspension. After 10 min, the cold bath was removed, and the reaction was stirred at rt. After 18 h, the reaction was cooled to 0 °C and carefully quenched with MeOH. The reaction was warmed to rt and then concentrated. Purification by reverse phase chromatography afforded 4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}benzoic acid trifluoroacetate (0.011 g, 56% yield) as a white solid. MS(ESI) *m/z*: 533.1 (M+H)+. $^1$H NMR (400MHz, $CD_3OD$) δ 9.00 (s, 1H), 8.74 (d, *J*=5.1 Hz, 1H), 7.90 (d, *J*=9.0 Hz, 1H), 7.73 (s, 1H), 7.61 - 7.56 (m, 2H), 7.53 (dd, *J*=5.3, 1.5 Hz, 1H), 7.50 (s, 1H), 6.58 (s, 1H), 6.07 (dd, *J*=12.8, 4.2 Hz, 1H), 4.05 (s, 3H), 2.77 - 2.67 (m, 1H), 2.41 - 2.30 (m, 1H), 2.16 - 2.00 (m, 2H), 1.68 - 1.57 (m, 1H), 1.55 - 1.43 (m, 1H), 1.01 (d, *J*=7.0 Hz, 3H), 0.79 - 0.62 (m, 1H). Analytical HPLC (Method A): RT = 5.54 min, 99.9% purity; Factor XIa Ki = 500 nM.

Example 192

Preparation of (9*R*,13*S*)-13-[4-(3-chloro-2,6-difluorophenyl)-6-oxo-1,6-dihydropyrimidin-1-yl]-3-(difluoromethyl)-9-methyl-3,4,7,15-tetraazatricyclo [12.3.1.0$^{2,6}$]octadeca-1(18),2(6),4,14,16-pentaen-8-one

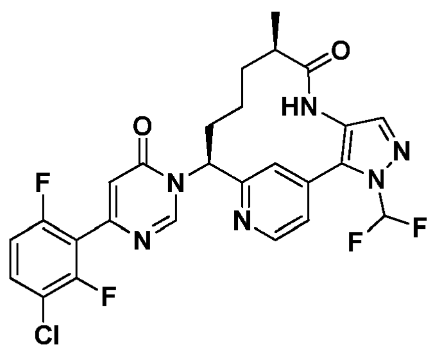

192A. Preparation of 6-(3-chloro-2,6-difluorophenyl)pyrimidin-4-ol

6-(3-Chloro-2,6-difluorophenyl)pyrimidin-4-ol hydrobromide, prepared as described in Intermediate 4, was partitioned between EtOAc and sat $NaHCO_3$. The layers were separated and the organic layer was washed with brine, dried over $Na_2SO_4$, filtered and concentrated to give a white solid. The solid was suspended in $Et_2O$ and sonicated. The solid was collected by filtration, rinsed with $Et_2O$, and dried under vacuum to give 6-(3-chloro-2,6-difluorophenyl)pyrimidin-4-ol as a white solid. MS(ESI) *m/z*: 243.0 $(M+H)^+$ and 245.0 $(M+2+H)^+$.

192B. Preparation of (9*R*,13*S*)-13-[4-(3-chloro-2,6-difluorophenyl)-6-oxo-1,6-dihydropyrimidin-1-yl]-3-(difluoromethyl)-9-methyl-3,4,7,15-tetraazatricyclo[12.3.1.$0^{2,6}$] octadeca-1(18),2(6),4,14,16-pentaen-8-one

(9*R*,13*S*)-13-[4-(3-Chloro-2,6-difluorophenyl)-6-oxo-1,6-dihydropyrimidin-1-yl 3-(difluoromethyl)-9-methyl-3,4,7,15-tetraazatricyclo[12.3.1.$0^{2,6}$]octadeca-1(18),2(6),4,14,16-pentaen-8-one trifluoroacetate (0.0092 g, 30%) was prepared in a similar manner as the procedures described in Example 56, by using 6-(3-chloro-2,6-difluorophenyl)pyrimidin-4-ol (10.8 mg, 0.045 mmol) and (9*R*,13*S*)-13-amino-3-(difluoromethyl)-9-methyl-3,4,7,15-tetraazatricyclo [12.3.1.$0^{2,6}$]octadeca-1(18),2(6),4,14,16-pentaen-8-one (0.015 g, 0.045 mmol), prepared as described in Intermediate 30. MS(ESI) *m/z*: 561.1 $(M+H)^+$ and 563.1 $(M+2+H)^+$. $^1H$ NMR (500MHz, $CD_3OD$) δ 9.09 (s, 1H), 8.77 (d, *J*=5.0 Hz, 1H), 7.79 - 7.72 (m, 2.25H), 7.67 - 7.60 (m, 1.5H), 7.56 - 7.52 (m, 1.25H), 7.14 (dt, *J*=9.1, 1.9 Hz, 1H), 6.67 (s, 1H), 6.11 - 6.04 (m, 1H), 2.76 - 2.69 (m, 1H), 2.42 - 2.33 (m, 1H), 2.13 - 2.02 (m, 2H), 1.66 - 1.56 (m, 1H), 1.56 - 1.46 (m, 1H), 1.01 (d, *J*=6.9 Hz, 3H), 0.73 - 0.60 (m, 1H). $^{19}F$ NMR (471MHz, $CD_3OD$) δ -114.75 (d, *J*=4.3 Hz), -115.47 (d, *J*=4.3 Hz), -77.66 (s). Analytical HPLC (Method A): RT = 8.38 min, purity = 99.7%; Factor XIa Ki = 30 nM, Plasma Kallikrein Ki = 670 nM.

Example 193

Preparation of (9*R*,13*S*)-13-[4-(5-chloro-2-phenylphenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one

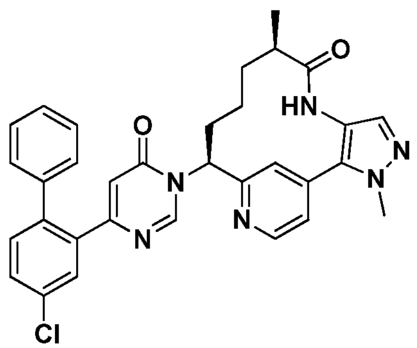

(9*R*,13*S*)-13-[4-(5-Chloro-2-phenylphenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one trifluoroacetate (5 mg, 21% yield) was prepared in a similar manner as the procedure described in Example 161, by replacing pyridin-3-ylboronic acid (4.76 mg, 0.039 mmol) with phenylboronic acid (4.72 mg, 0.039 mmol). MS(ESI) *m/z*: 565.4 $(M+H)^+$. $^1H$ NMR (500MHz, $CD_3OD$) δ 8.92 (s, 1H), 8.72 (d, *J*=5.0 Hz, 1H), 7.71 (d, *J*=2.2 Hz, 1H), 7.66 (s, 1H), 7.55 - 7.50 (m, 2H), 7.48 (s, 1H), 7.41 (d, *J*=8.3 Hz, 1H), 7.34 - 7.26 (m, 3H), 7.24 - 7.20 (m, 2H), 6.08 (d, *J*=0.8 Hz, 1H), 5.94 (dd, *J*=12.5, 4.0 Hz, 1H), 4.04 (s, 3H), 2.73 - 2.65 (m, 1H), 2.34 - 2.24 (m, 1H), 2.10 - 1.94 (m, 2H), 1.63 - 1.54 (m, 1H), 1.52 - 1.41 (m, 1H), 1.00 (d, *J*=6.9 Hz, 3H), 0.74 - 0.59 (m, 1H). Analytical HPLC (Method A): RT = 12.43 min, 98.2% purity; Factor XIa Ki = 160 nM, Plasma Kallikrein Ki = 4,700 nM.

Example 196 (reference)

Preparation of (9*R*,13S)-13-(4-{5-chloro-2-[4-(trifluoromethyl)-1H-1,2,3-triazol-1-yl] phenyl}-6-oxo-1,6-dihydropyrimidin-1-yl)-9-methyl-3,4,7,15-tetraazatricyclo [12.3.1.$0^{2,6}$]octadeca-1(18),2(6),4,14,16-pentaen-8-one

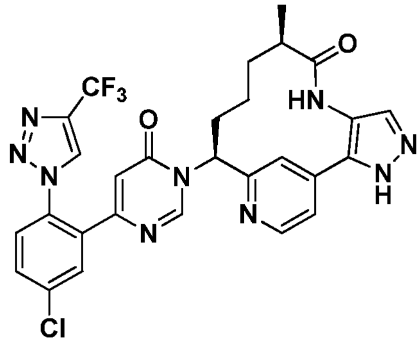

196A. Preparation of (9*R*,13*S*)-13-(4-{5-chloro-2-[4-(trifluoromethyl)-1H-1,2,3-triazol-1-yl]phenyl}-6-oxo-1,6-dihydropyrimidin-1-yl)-9-methyl-3-{[2-(trimethylsilyl)ethoxy] methyl}-3,4,7,15-tetraazatricyclo[12.3.1.$0^{2,6}$]octadeca-1(18),2(6),4,14,16-pentaen-8-one

(9*R*,13*S*)-13-(4-{5-Chloro-2-[4-(trifluoromethyl)-1H-1,2,3-triazol-1-yl]phenyl}-6-oxo-1,6-dihydropyrimidin-1-yl)-9-methyl-3-{[2-(trimethylsilyl)ethoxy]methyl}-3,4,7,15-tetraazatricyclo[12.3.1.$0^{2,6}$]octadeca-1(18),2(6),4,14,16-pentaen-8-one (760 mg, 0.975 mmol, 74% yield) was prepared in a similar manner as the procedures described in Example 56, by using 6-(5-chloro-2-(4-(trifluoromethyl)-1H-1,2,3-triazol-1-yl)phenyl) pyrimidin-4-ol (0.452 g, 1.323 mmol), prepared as described in Intermediate 15, and (9*R*,13*S*)-9-methyl-3-{[2-(trimethylsilyl)ethoxy]methyl}-3,4,7,15-tetraazatricyclo [12.3.1.$0^{2,6}$]octadeca-1(18),2(6),4,14,16-pentaen-8-one (0.550 g, 1.323 mmol), prepared as described in Intermediate 19. MS(ESI) *m/z:* 740.6 $[M+H]^+$.

196B. Preparation of (9*R*,13*S*)-13-(4-{5-chloro-2-[4-(trifluoromethyl)-1H-1,2,3-triazol-1-yl]phenyl}-6-oxo-1,6-dihydropyrimidin-1-yl)-9-methyl-3,4,7,15-tetraazatricyclo [12.3.1.$0^{2,6}$]octadeca-1(18),2(6),4,14,16-pentaen-8-onetrifluoroacetate

To a solution of (9*R*,13*S*)-13-(4-{5-chloro-2-[4-(trifluoromethyl)-1H-1,2,3-triazol-1-yl]phenyl}-6-oxo-1,6-dihydropyrimidin-1-yl)-9-methyl-3-{[2-(trimethylsilyl)ethoxy] methyl}-3,4,7,15-tetraazatricyclo[12.3.1.$0^{2,6}$]octadeca-1(18),2(6),4,14,16-pentaen-8-one (760 mg, 1.027 mmol), in DCM (4.0 mL) was added TFA (1.0 mL, 12.98 mmol) and the resulting solution was stirred at rt for 30 min. The reaction mixture was then concentrated and the residue was purified by prep HPLC purification to give (9*R*,13*S*)-13-(4-{5-chloro-2-[4-(trifluoromethyl)-1H-1,2,3-triazol-1-yl]phenyl}-6-oxo-1,6-dihydropyrimidin-1-yl)-9-methyl-3,4,7,15-tetraazatricyclo[12.3.1.$0^{2,6}$]octadeca-1(18),2(6),4,14,16-pentaen-8-one trifluoroacetate (480 mg, 92% yield). $^1$H NMR (500MHz, DMSO-$d_6$) $\delta$ 9.26 (s, 1H), 9.21 (s, 1H), 8.63 (br. s., 1H), 8.49 (br. s., 1H), 7.96 (d, *J*=1.5 Hz, 1H), 7.87 - 7.78 (m, 2H), 7.47 (br. s., 1H), 7.24 - 6.98 (m, 1H), 6.48 (s, 1H), 5.97 (br. s., 1H), 3.45 - 3.36 (m, 2H), 2.72 (br. s., 1H), 2.32 - 2.15 (m, 2H), 1.80 (br. s., 1H), 1.52 (br. s., 1H), 1.38 (br. s., 1H), 0.92 (d, *J*=6.7 Hz, 3H), 0.59 (br. s., 1H). MS(ESI) *m/z*: 610.1 $[M+H]^+$. Analytical HPLC (Method B): RT = 1.57 min, purity = 97.0%; Factor XIa Ki = 1.9 nM, Plasma Kallikrein Ki = 205 nM.

Example 197

Preparation of (9*R*,13*S*)-13-{4-[3-chloro-2-fluoro-6-(1H-1,2,3,4-tetrazol-1-yl)phenyl]-6-oxo-1,6-dihydropyrimidin-1-yl}-3,9-dimethyl-3,4,7,15-tetraazatricyclo[12.3.1.$0^{2,6}$] octadeca-1(18),2(6),4,14,16-pentaen-8-one trifluoroacetate

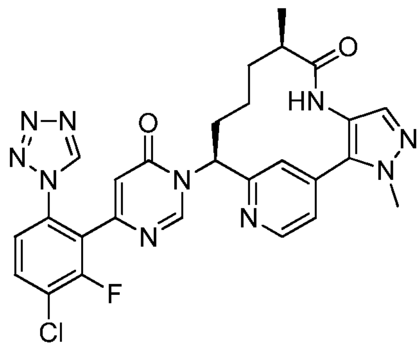

(9*R*,13*S*)-13-{4-[3-Chloro-2-fluoro-6-(1H-1,2,3,4-tetrazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (6.9 mg, 16% yield) was prepared in a similar manner as the procedure described in Example 196 by using (9R,13S)-13-amino-3,9-dimethyl-3,4,7,15-tetraazatricyclo[12.3.1.0$^{2,6}$]octadeca-1(18),2(6),4,14,16-pentaen-8-one, prepared as described in Intermediate 32. MS(ESI) *m/z*: 575.2 (M+H)$^+$. $^1$H NMR (400MHz, $CD_3OD$) δ 9.46 (br. s., 1H), 8.76 (d, J=8.1 Hz, 2H), 7.91 (d, J=6.8 Hz, 1H), 7.80 (br. s., 1H), 7.62 (d, J=7.0 Hz, 2H), 7.53 (br. s., 1H), 6.70 (br. s., 1H), 5.95 (br. s., 1H), 4.09 (br. s., 3H), 2.71 (br. s., 1H), 2.32 (br. s., 1H), 2.05 (br. s., 2H), 1.62 (br. s., 1H), 1.46 (br. s., 1H), 1.03 (br. s., 3H), 0.77 (br. s., 1H) Analytical HPLC (Method A): RT = 6.71 min, purity = > 99%; Factor XIa Ki = 0.1 nM, Plasma Kallikrein Ki = 8 nM.

Example 198

Preparation of (9*R*,13*S*)-13-{4-[3-chloro-2-fluoro-6-(1H-1,2,3,4-tetrazol-1-yl)phenyl]-6-oxo-1,6-dihydropyrimidin-1-yl}-3-(difluoromethyl)-9-methyl-3,4,7-triazatricyclo [12.3.1.0$^{2,6}$]octadeca-1(18),2(6),4,14,16-pentaen-8-one trifluoroacetate

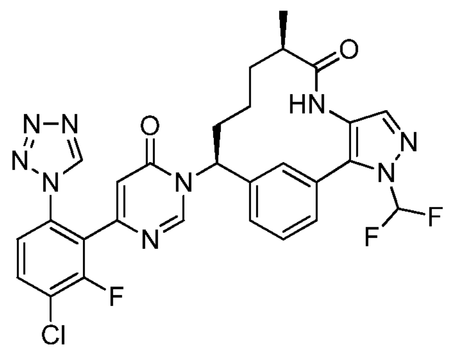

(9*R*,13*S*)-13-{4-[3-Chloro-2-fluoro-6-(1H-1,2,3,4-tetrazol-1-yl)phenyl]-6-oxo-1,6-dihydropyrimidin-1-yl}-3-(difluoromethyl)-9-methyl-3,4,7-triazatricyclo[12.3.1.0$^{2,6}$] octadeca-1(18),2(6),4,14,16-pentaen-8-one trifluoroacetate (9.7 mg, 33% yield) was prepared in a similar manner as the procedure described in Example 196 by using (9R,13S)-13-amino-3-(difluoromethyl)-9-methyl-3,4,7-triazatricyclo[12.3.1.0$^{2,6}$] octadeca-1(18),2(6),4,14,16-pentaen-8-one, prepared as described in Intermediate 35. MS(ESI) *m/z:* 610.3 (M+H)$^+$. $^1$H NMR (400MHz, $CD_3OD$) δ 9.42 (s, 1H), 8.17 (s, 1H), 7.89 (dd, *J*=8.7, 7.6 Hz, 1H), 7.81 - 7.75 (m, 2H), 7.65 - 7.46 (m, 4H), 7.38 (d, *J*=7.7 Hz, 1H), 6.71 (d, *J=09* Hz, 1H), 5.81 (dd, *J*=12.9, 3.4 Hz, 1H), 2.56 - 2.44 (m, 1H), 2.40 - 2.25 (m, 1H), 2.17 - 2.05 (m, 1H), 1.94 - 1.82 (m, 1H), 1.63 - 1.47 (m, 2H), 1.27 - 1.17 (m, 1H), 1.15 (d, *J*=6.8 Hz, 3H). Analytical HPLC (Method A): RT = 8.62 min, purity = > 99%; Factor XIa Ki = 0.1 nM, Plasma Kallikrein Ki = 4 nM.

Example 200

Preparation of (9*R*,13*S*)-13-{4-[5-chloro-2-(1H-1,2,3,4-tetrazol-1-yl)phenyl]-6-oxo-1,6-dihydropyrimidin-1-yl}-3-(difluoromethyl)-9-methyl-3,4,7,15-tetraazatricyclo [12.3.1.0$^{2,6}$]octadeca-1(18),2(6),4, 14,16-pentaen-8-one trifluoroacetate

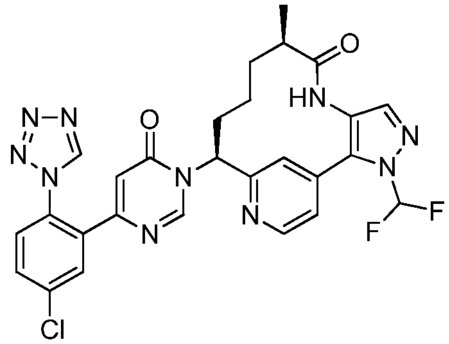

(9*R*,13*S*)-13-{4-[5-Chloro-2-(1H-1,2,3,4-tetrazol-1-yl)phenyl]-6-oxo-1,6-dihydropyrimidin-1-yl}-3-(difluoromethyl)-9-methyl-3,4,7,15-tetraazatricyclo [12.3.1.0$^{2,6}$]octadeca-1(18),2(6),4,14,16-pentaen-8-one trifluoroacetate (22.1 mg, 44% yield) was prepared in a similar manner as the procedure described in Example 198 by replacing 6-[3-chloro-2-fluoro-6-(1H-1,2,3,4-tetrazol-1-yl)phenyl]pyrimidin-4-ol with 6-[5-chloro-2-(1H-1,2,3,4-tetrazol-1-yl)phenyl]pyrimidin-4-ol, prepare as described in Intermediate 20. MS(ESI) *m/z:* 593.2 $(M+H)^+$. $^1H$ NMR (400MHz, $CD_3OD$) δ 9.38 (s, 1H), 8.73 - 8.65 (m, 2H), 7.84 (d, *J*=2.2 Hz, 1H), 7.79 - 7.42 (m, 6H), 6.46 (s, 1H), 5.88 (dd, *J*=12.5, 4.2 Hz, 1H), 2.71 - 2.58 (m, 1H), 2.32 - 2.19 (m, 1H), 2.04 - 1.89 (m, 2H), 1.60 - 1.47 (m, 1H), 1.45 - 1.30 (m, 1H), 0.94 (d, *J*=6.8 Hz, 3H), 0.60 (br. s., 1H). Analytical HPLC (Method A): RT = 7.93 min, purity = > 99%; Factor XIa Ki = 0.1 nM, Plasma Kallikrein Ki = 10 nM.

Example 201

Preparation of (9*R*,13*S*)-13-{4-[5-chloro-2-(1H-1,2,3,4-tetrazol-1-yl)phenyl]-6-oxo-1,6-dihydropyrimidin-1-yl}-3-(difluoromethyl)-9-methyl-3,4,7-triazatricyclo[12.3.1.0$^{2,6}$] octadeca-1(18),2(6),4,14,16-pentaen-8-one

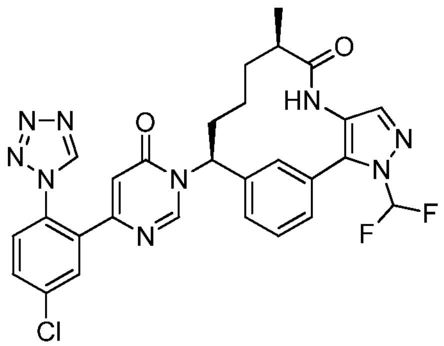

(9*R*,13*S*)-13-{4-[5-Chloro-2-(1H-1,2,3,4-tetrazol-1-yl)phenyl]-6-oxo-1,6-dihydropyrimidin-1-yl}-3-(difluoromethyl)-9-methyl-3,4,7-triazatricyclo[12.3.1.0$^{2,6}$] octadeca-1(18),2(6),4,14,16-pentaen-8-one (10.5 mg, 40% yield) was prepared in a similar manner as the procedure described in Example 200 by replacing (9R,13S)-13-amino-3-(difluoromethyl)-9-methyl-3,4,7,15-tetraazatricyclo[12.3.1.0$^{2,6}$]octadeca-1(18),2(6),4,14, 16-pentaen-8-one with (9R,13S)-13-amino-3-(difluoromethyl)-9-methyl-3,4,7-triazatricyclo[12.3.1.0$^{2,6}$]octadeca-1(18),2(6),4,14,16-pentaen-8-one, prepare as described in Intermediate 35. MS(ESI) *m/z:* 592.3 $(M+H)^+$. $^1H$ NMR (400MHz, $CD_3OD$) δ 9.39 (s, 1H), 8.09 (s, 1H), 7.86 (d, J=2.4 Hz, 1H), 7.78 - 7.42 (m, 7H), 7.34 (d, J=7.5 Hz, 1H), 6.51 (s, 1H), 5.76 (dd, J=12.8, 3.1 Hz, 1H), 2.51 - 2.38 (m, 1H), 2.36 - 2.21 (m, 1H), 2.11 - 1.99 (m, 1H), 1.90 - 1.77 (m, 1H), 1.58 - 1.41 (m, 2H), 1.21 - 1.03 (m, 4H) Analytical HPLC (Method A): RT = 8.50 min, purity = > 99%; Factor XIa Ki = 0.1 nM, Plasma Kallikrein Ki = 6 nM.

Example 205

Preparation of (10*R*,14*S*)-14-(4-{5-chloro-2-[4-(trifluoromethyl)-1H-1,2,3-triazol-1-yl] phenyl-6-oxo-1,6-dihydropyrimidin-1-yl)-10-methyl-5,8-diazatricyclo[13.3.1.0$^{2,7}$] nonadeca-1(19),2(7),3,5,15,17-hexaen-9-one

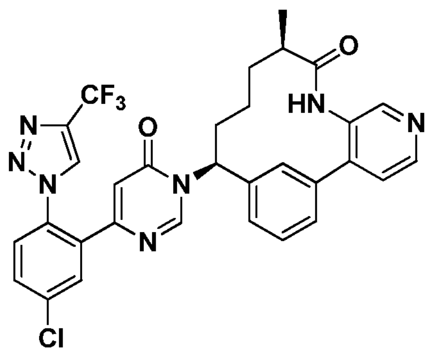

(10R,14S)-14-(4-{5-Chloro-2-[4-(trifluoromethyl)-1H-1,2,3-triazol-1-yl]phenyl}-6-oxo-1,6-dihydropyrimidin-1-yl)-10-methyl-5,8-diazatricyclo[13.3.1.$0^{2,7}$]nonadeca-1(19),2(7),3,5,15,17-hexaen-9-one trifluoroacetate (0.0076 g, 36%) was prepared according to the procedures described in Example 46 by using 6-{5-chloro-2-[4-(trifluoromethyl)-1H-1,2,3-triazol-1-yl]phenyl}pyrimidin-4-ol, prepared as described in Example 15B, (10R,14S)-14-amino-10-methyl-5,8-diazatricyclo[13.3.1.$0^{2,7}$]nonadeca-1(19),2(7),3,5,15,17-hexaen-9-one, prepared as described in Intermediate 38, and 4-bromopyridin-3-amine in Intermediate 38B. MS(ESI) *m/z:* 620.1 $(M+H)^+$. $^1H$ NMR (400MHz, $CD_3OD$) δ 8.81 (d, *J*=0.7 Hz, 1H), 8.77 (s, 2H), 8.29 (s, 1H), 8.08 (d, *J*=5.7 Hz, 1H), 7.91 - 7.84 (m, 2H), 7.82 - 7.60 (m, 4H), 7.42 - 7.34 (m, 1H), 6.49 (d, *J*=0.4 Hz, 1H), 5.80 (dd, *J*=13.0, 3.5 Hz, 1H), 2.64 - 2.51 (m, 1H), 2.39 - 2.26 (m, 1H), 2.18 - 2.06 (m, 1H), 1.99 - 1.88 (m, 1H), 1.63 - 1.30 (m, 3H), 1.16 (d, *J*=6.8 Hz, 3H). Analytical HPLC (Method A): RT = 6.87 min, purity = 90%; Factor XIa Ki = 0.11 nM, Plasma Kallikrein Ki = 11 nM.

Example 206

Preparation of (10*R*,14*S*)-14-{4-[5-chloro-2-(4-chloro-1H-1,2,3-triazol-1-yl)phenyl]-6-oxo-1,6-dihydropyrimidin-1-yl}-10-methyl-5,8-diazatricyclo[13.3.1.$0^{2,7}$]nonadeca-1(19),2(7),3,5,15,17-hexaen-9-one

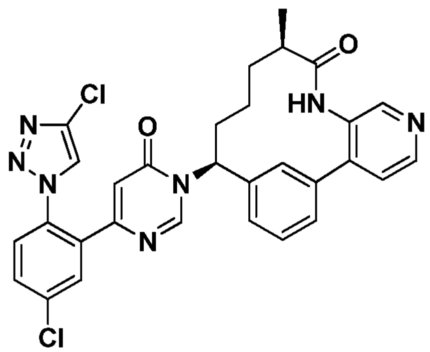

(10*R*,14*S*)-14-{4-[5-Chloro-2-(4-chloro-1H-1,2,3-triazol-1-yl)phenyl]-6-oxo-1,6-dihydropyrimidin-1-yl}-10-methyl-5,8-diazatricyclo[13.3.1.$0^{2,7}$]nonadeca-1(19),2(7),3,5,15,17-hexaen-9-one trifluoroacetate (0.0062 g, 31%) was prepared according to the procedures described in Example 205 by using 6-[5-chloro-2-(4-chloro-1H-1,2,3-triazol-1-yl)phenyl] pyrimidin-4-ol, prepared as described in Intermediate 9E. MS(ESI) *m/z:* 586.1 $(M+H)^+$. $^1H$ NMR (400MHz, $CD_3OD$) δ 8.70 (d, *J*=5.3 Hz, 1H), 8.64 (s, 1H), 8.37 - 8.28 (m, 2H), 7.93 - 7.84 (m, 3H), 7.75 (dd, *J*=8.6, 2.2 Hz, 1H), 7.68 - 7.61 (m, 3H), 7.43 - 7.35 (m, 1H), 6.42 (s, 1H), 5.82 (dd, *J*=13.0, 3.5 Hz, 1H), 2.63 - 2.52 (m, 1H), 2.41 - 2.28 (m, 1H), 2.20 - 2.08 (m, 1H), 1.98 - 1.87 (m, 1H), 1.65 - 1.45 (m, 2H), 1.41 - 1.29 (m, 1H), 1.17 (d, *J*=7.0 Hz, 3H). Analytical HPLC (Method A): RT = 6.26 min, purity = 90%; Factor XIa Ki = 0.2 nM, Plasma Kallikrein Ki = 17 nM.

Example 211

Preparation of (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one

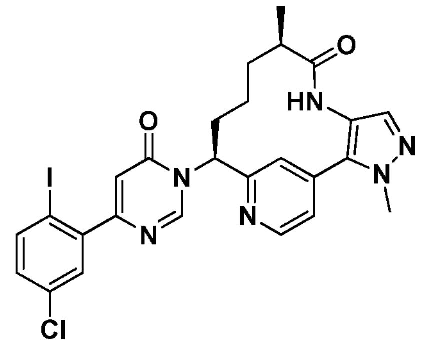

211A. Preparation of 4-(5-chloro-2-iodophenyl)-6-methoxypyrimidine

The solution of 4-chloro-2-(6-methoxypyrimidin-4-yl)aniline (2 g, 8.49 mmol), prepared as described in Intermediate 8A, in ACN (56.6 ml) was cooled to 0 °C, then *p*-TsOH·$H_2O$ (4.04 g, 21.22 mmol) was added. A solution of $NaNO_2$ (1.171 g, 16.97 mmol) and NaI (3.18 g, 21.22 mmol) in water (28.3 ml) was added slowly and the reaction turned into a dark brown solution. After a few min, the reaction turned cloudy. The reaction was stirred at 0 °C for 1 h, then the reaction was warmed to rt. After 18 h, the reaction was quenched with sat $NaHCO_3$ and extracted with EtOAc. The organic layer was washed with sat aq $Na_2S_2O_3$, brine, dried over $Na_2SO_4$, filtered, and concentrated to yield a solid. Purification by normal phase chromatography afforded 4-(5-chloro-2-iodophenyl)-6-methoxypyrimidine (2.13 g, 72% yield) as a white solid. MS(ESI) *m/z:* 347.2 $(M+H)^+$. $^1H$ NMR (500MHz, $CDCl_3$) d 8.87 (d, *J*=1.1 Hz, 1H), 7.88 (d, *J*=8.3 Hz, 1H), 7.44 (d, *J*=2.8 Hz, 1H), 7.11 (dd, *J*=8.5, 2.8 Hz, 1H), 6.93 (d, *J*=1.4 Hz, 1H), 4.06 (s, 3H).

211B. Preparation of 6-(5-chloro-2-iodophenyl)pyrimidin-4-ol

6-(5-Chloro-2-iodophenyl)pyrimidin-4-ol (0.24 g, 100% yield) was prepared in a similar manner as the procedure described in Example 140B, by replacing 4-(5-chloro-2-(1-(difluoromethyl)-1H-pyrazol-4-yl)phenyl)-6-methoxypyrimidine with 4-(5-chloro-2-iodophenyl)-6-methoxypyrimidine (0.25 g, 0.721 mmol), and the reaction time was 1 h at 85 °C. MS(ESI) *m/z:* 333.0 $(M+H)^+$. $^1H$ NMR (400MHz, $CD_3OD$) δ 8.27 (d, *J*=0.9 Hz, 1H), 7.95 (d, *J*=8.6 Hz, 1H), 7.47 (d, *J*=2.6 Hz, 1H), 7.21 (dd, *J*=8.6, 2.6 Hz, 1H), 6.54 (d, *J*=0.9 Hz, 1H).

211C. Preparation of (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate

(9*R*,13*S*)-13-[4-(5-Chloro-2-iodophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate (125 mg, 68% yield) was prepared in a similar manner as the procedure described in Example 56, by using 6-(5-chloro-2-iodophenyl)pyrimidin-4-ol (100 mg, 0.301 mmol). MS(ESI) *m/z:* 615.4 $(M+H)^+$. $^1H$ NMR (400MHz, $CD_3OD$) δ 9.05 (s, 1H), 8.75 (d, *J*=5.3 Hz, 1H), 7.95 (d, *J*=8.4 Hz, 1H), 7.74 (s, 1H), 7.54 (dd, *J*=5.1, 1.5 Hz, 1H), 7.51 - 7.49 (m, 2H), 7.21 (dd, *J*=8.5, 2.5 Hz, 1H), 6.60 (s, 1H), 6.07 (dd, *J*=12.8, 4.4 Hz, 1H), 4.05 (s, 3H), 2.77 - 2.68 (m, 1H), 2.38 (tt, *J*=12.7, 4.3 Hz, 1H), 2.16 - 2.02 (m, 2H), 1.68 - 1.44 (m, 2H), 1.02 (d, *J*=6.8 Hz, 3H), 0.79 - 0.64 (m, 1H). Analytical HPLC (Method A): RT = 8.71 min, 99.6% purity; Factor XIa Ki = 12 nM, Plasma Kallikrein Ki = 140 nM.

Example 213

Preparation of N-(4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-3-fluorophenyl)carbamate trifluoroacetate

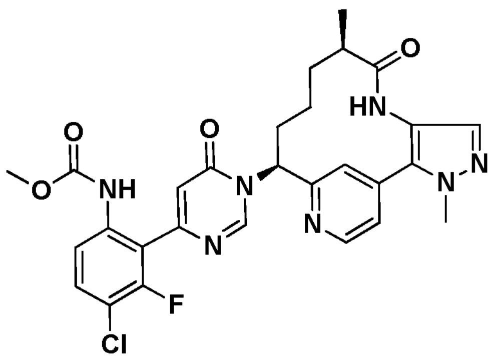

213A. Preparation of N-[4-chloro-3-fluoro-2-(6-methoxypyrimidin-4-yl)phenyl] carbamate

4-Chloro-3-fluoro-2-(6-methoxypyrimidin-4-yl)aniline (0.024 g, 0.095 mmol), was dissolved in DCM (5ml). To this solution was added sequentially TEA (1 mL) followed by methyl chloroformate (8.94 mg, 0.095 mmol) and the solution was stirred at rt. After 2 h, the reaction was concentrated *in vacuo* to an oil and quenched with dilute HCl (5 mL). The organics were extracted with EtOAc (2x25 mL), dried with $MgSO_4$ and evaporated to an oil. LCMS *m/z*

312.1(M+H). $^1$H NMR (400MHz, $CDCl_3$) δ 10.30 - 10.24 (m, 1H), 8.84 - 8.81 (m, 1H), 8.00 - 7.92 (m, 1H), 7.40 - 7.32 (m, 1H), 7.05 - 7.01 (m, 1H), 3.98 (s, 3H), 3.67 (s, 3H). The crude product was taken in a small vial and dissolved in AcOH (1 mL) and to this was added 48% HBr (0.1 mL) and heated at 80 °C until reaction was complete. The reaction mixture was concentrated and quenched with water (25 mL), extracted with EtOAc (2x25 mL). The combined organic layers was dried and evaporated to give N-[4-chloro-3-fluoro-2-(6-methoxypyrimidin-4-yl)phenyl] carbamate as a white film (13 mg). LCMS *m*/*z* 299.1(M+H).

213B. Preparation of N-(4-chloro-2-{1-[(9*R*,13*S*)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}-3-fluorophenyl)carbamate

N-(4-Chloro-2-{1-[(9*R*,13*S*)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}-3-fluorophenyl)carbamate (1.1 mg, 4.21% yield) was prepared following the procedure described in Example 56 by using N-[4-chloro-3-fluoro-2-(6-hydroxypyrimidin-4-yl)phenyl]carbamate (13 mg, 0.044 mmol), (9*R*,13*S*)-13-amino-3,9-dimethyl-3,4,7,15-tetraazatricyclo[12.3.1.$0^{2,6}$]octadeca-1(18),2(6),4,14,16-pentaen-8-one (15 mg, 0.044 mmol). LCMS *m*/*z* 580.1(M+H). $^1$H NMR (500MHz, DMSO-$d_6$) δ 9.39 - 9.34 (m, 1H), 9.24 - 9.21 (m, 1H), 9.05 - 9.02 (m, 1H), 8.74 - 8.70 (m, 1H), 7.74 - 7.57 (m, 3H), 7.49 (s, 1H), 6.61 - 6.55 (m, 1H), 5.99 - 5.87 (m, 1H), 4.04 - 3.98 (m, 3H), 3.65 - 3.59 (m, 3H), 2.70 - 2.62 (m, 1H), 2.40 - 2.32 (m, 1H), 2.18 - 2.09 (m, 1H), 1.96 - 1.85 (m, 1H), 1.53 - 1.30 (m, 2H), 0.94 - 0.87 (m, 3H), 0.56 - 0.36 (m, 1H). Ortho RT. 1.605 min purity 97%; Factor XIa Ki = 110 nM.

Example 216 (reference)

Preparation of (9R,13S)-13-{4-[5-chloro-2-(4-chloro-1H-1,2,3-triazol-1-yl)phenyl]-6-oxo-1,6-dihydropyrimidin-1-yl}-9-methyl-4-(pyridin-3-yl)-3,4,7,15-tetraazatricyclo[12.3.1.$0^{2,6}$]octadeca-1(18),2,5,14,16-pentaen-8-one

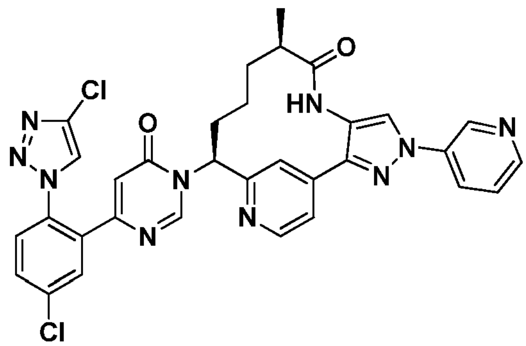

(9*R*,13*S*)-13-{4-[5-Chloro-2-(4-chloro-1*H*-1,2,3-triazol-1-yl)phenyl]-6-oxo-1,6-dihydropyrimidin-1-yl}-9-methyl-3,4,7,15-tetraazatricyclo[12.3.1.$0^{2,6}$]octadeca-1(18),2(6),4,14,16-pentaen-8-one, prepared as described in Example 101, (0.09 g, 0.156 mmol), (1*R*,2*R*)-*N*1,*N*2-dimethylcyclohexane-1,2-diamine (0.022 g, 0.156 mmol), $C_{S2}CO_3$ (0.102 g, 0.312 mmol), and 3-iodopyridine (0.032 g, 0.156 mmol) were added to a 5 mL microwave vial. DMF (2 mL) was added and the vial was purged with Ar (3x). CuI (2 mg, 10.50 μmol) was added, the vial was sealed with a microwave vial cap and the reaction was heated in a microwave reactor at 120 °C for 30 min. The reaction was then purified by reverse phase chromatography to yield (9*R*,13*S*)-13-{4-[5-chloro-2-(4-chloro-1*H*-1,2,3-triazol-1-yl)phenyl]-6-oxo-1,6-dihydropyrimidin-1-yl}-9-methyl-4-(pyridin-3-yl)-3,4,7,15-tetraazatricyclo[12.3.1.$0^{2,6}$]octadeca-1(18),2,5,14,16-pentaen-8-one trifluoroacetate (52 mg, 42% yield) as a tan solid. MS(ESI) *m/z:* 653.6 $(M+H)^+$. $^1$H NMR (400MHz, $CD_3OD$) δ 9.34 - 9.21 (m, 1H), 8.81 - 8.73 (m, 1H), 8.72 - 8.64 (m, 2H), 8.60 (s, 2H), 8.37 (s, 1H), 8.02 - 7.96 (m, 1H), 7.96 - 7.89 (m, 1H), 7.87 - 7.80 (m, 1H), 7.80 - 7.73 (m, 2H), 7.72 - 7.63 (m, 1H), 6.47 - 6.35 (m, 1H), 6.20 - 6.03 (m, 1H), 2.96 - 2.82 (m, 1H), 2.42 - 2.22 (m, 2H), 2.17 - 2.01 (m, 1H), 1.83 - 1.70 (m, 1H), 1.70 - 1.52 (m, 1H), 1.42 - 1.26 (m, 1H), 1.11 (d, *J*=7.0 Hz, 3H), 0.97 - 0.85 (m, 2H). Analytical HPLC (Method A): RT = 6.69 min, purity = 97.5%; Factor XIa Ki = 0.57 nM, Plasma Kallikrein Ki = 10 nM.

Example 225

Preparation of 4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-N-(2,2,2-trifluoroethyl)benzamide

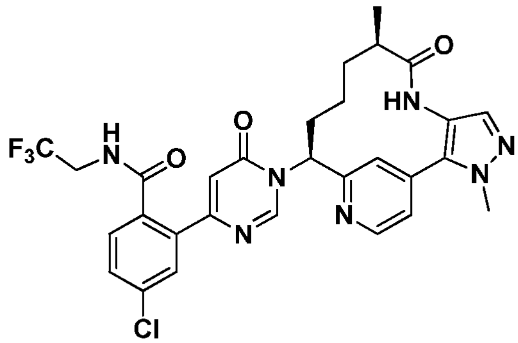

225A. Preparation of 4-chloro-2-(6-methoxypyrimidin-4-yl)-N-(2,2,2-trifluoroethyl) benzamide

To a RBF was added 4-chloro-2-(6-methoxypyrimidin-4-yl)benzoic acid (0.374 g, 1.413 mmol), prepared as described in Example 168B, EtOAc (7.07 ml), 2,2,2-$CF_3CH_2NH_2$ (0.14 g, 1.413 mmol), and pyridine (0.229 ml, 2.83 mmol). The solution was cooled in a MeOH/ice bath and 50%w/w 2,4,6-tripropyl-1,3,5,2,4,6-trioxatriphosphorinane-2,4,6-trioxide in EtOAc (1.26 ml, 2.120 mmol) was added. The reaction was allowed to warm to rt. After 18 h, the reaction was diluted with EtOAc, washed with sat $NaHCO_3$, brine, dried over $Na_2SO_4$, filtered, and concentrated. DCM (~10ml) was added, plus a few drops of MeOH to afford a yellow suspension. The solid was filtered off, and the filtrate was purified by normal phase chromatography to afford 4-chloro-2-(6-methoxypyrimidin-4-yl)-N-(2,2,2-trifluoroethyl)benzamide (0.155 g, 32% yield) as a white solid. MS(ESI) *m/z:* 346.4 $(M+H)^+$.

225B. Preparation of 4-chloro-2-(6-hydroxypyrimidin-4-yl)-N-(2,2,2-trifluoroethyl) benzamide

To a solution of 4-chloro-2-(6-methoxypyrimidin-4-yl)-N-(2,2,2-trifluoroethyl) benzamide (0.05 g, 0.145 mmol) in ACN (0.96 ml) was added TMSI (0.118 ml, 0.868 mmol). The reaction was heated to 50 °C. After 6 h, the reaction was cooled to rt, poured into a 10% aq $Na_2S_2O_3$, and extracted with EtOAc (3x). The combined organic layers were washed with sat $NaHCO_3$, brine, dried over $Na_2SO_4$, filtered, and concentrated. Purification by normal phase chromatography afforded 4-chloro-2-(6-hydroxypyrimidin-4-yl)-N-(2,2,2-trifluoroethyl)benzamide (0.02 g, 42% yield) as a white solid. MS(ESI) *m/z:* 332.3 $(M+H)^+$. $^1H$ NMR (400MHz, $CD_3OD$) δ 8.14 (d, *J*=0.9 Hz, 1H), 7.69 (d, *J*=2.0 Hz, 1H), 7.59 - 7.55 (m, 1H), 7.54 - 7.49 (m, 1H), 6.63 (d, *J*=0.9 Hz, 1H), 3.98 (q, *J*=9.5 Hz, 2H). $^{19}F$ NMR (376MHz, $CD_3OD$) δ -73.22 (s).

225C. Preparation of 4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}-N-(2,2,2-trifluoroethyl)benzamide trifluoroacetate

4-Chloro-2-{1-[(9*R*,13*S*)-3,9-dimethyl-8-oxo-3,4,7,15-tetraazatricyclo[12.3.1.0$^{2,6}$] octadeca-1(18),2(6),4,14,16-pentaen-13-yl]-6-oxo-1,6-dihydropyrimidin-4-yl}-N-(2,2,2-trifluoroethyl)benzamide trifluoroacetate (4.8 mg, 11% yield) was prepared in a similar manner as the procedure described in Example 56, by replacing 6-{5-chloro-2-[4-(trifluoromethyl)-1H-1,2,3-triazol-1-yl]phenyl}pyrimidin-4-ol with 4-chloro-2-(6-hydroxypyrimidin-4-yl)-N-(2,2,2-trifluoroethyl)benzamide (0.02 g, 0.060 mmol). MS(ESI) *m/z:* 614.5 $(M+H)^+$. $^1H$ NMR (400MHz, $CD_3OD$) δ 8.95 (s, 1H), 8.73 (d, *J*=5.3 Hz, 1H), 7.72 -7.71 (m, 2H), 7.59 - 7.55 (m, 1H), 7.54 - 7.48 (m, 3H), 6.66 (s, 1H), 6.05 (dd, *J*=12.5, 4.2 Hz, 1H), 4.08 - 3.90 (m, 5H), 2.76 - 2.67 (m, 1H), 2.39 - 2.28 (m, 1H), 2.15 - 1.98 (m, 2H), 1.68 - 1.42 (m, 2H), 1.01 (d, *J*=7.0 Hz, 3H), 0.79 - 0.62 (m, 1H). $^{19}F$ NMR (376MHz, $CD_3OD$) δ -73.10 (s), -77.67 (s). Analytical HPLC (Method A): RT = 6.78 min, 98.2% purity; Factor XIa Ki = 23 nM, Plasma Kallikrein Ki = 3,400 nM.

Example 231

Preparation of (9R,13S)-13-{4-[5-chloro-2-(1H-imidazol-1-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

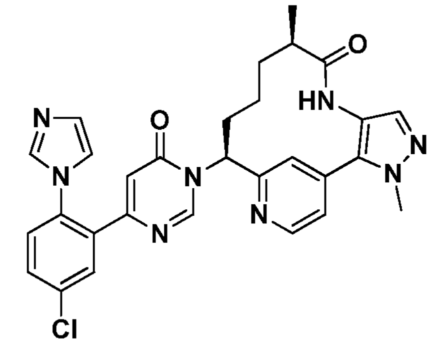

To the solution of 1H-imidazole (8.86 mg, 0.130 mmol) and (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one trifluoroacetate (0.02 g, 0.033 mmol), prepared as described in Example 211, in DMSO (1 ml) was added CuI (0.62 mg, 3.25 $\mu$mol), L-proline (0.75 mg, 6.51 $\mu$mol), and $K_2CO_3$ (0.013 g, 0.098 mmol). The reaction was heated at 80 °C for 3 h and cooled to rt. Purification by reverse phase chromatography afforded (9*R*,13*S*)-13-{4-[5-chloro-2-(1H-imidazol-1-yl)phenyl]-6-oxo-1,6-dihydropyrimidin-1-yl}1-3,9-dimethyl-3,4,7,15-tetraazatricyclo[12.3.1.$0^{2,6}$] octadeca-1(18),2(6),4,14,16-pentaen-8-one trifluoroacetate (11 mg, 43% yield) as a yellow solid. MS(ESI) *m/z:* 555.5 $(M+H)^+$. $^1H$ NMR (500MHz, $CD_3OD$) $\delta$ 9.19 (s, 1H), 8.77 - 8.67 (m, 2H), 7.92 (d, *J*=2.5 Hz, 1H), 7.81 - 7.75 (m, 1H), 7.73 - 7.64 (m, 4H), 7.54 - 7.48 (m, 2H), 6.62 (s, 1H), 6.00 - 5.91 (m, 1H), 4.04 (s, 3H), 2.74 - 2.64 (m, 1H), 2.32 - 2.21 (m, 1H), 2.11 - 1.92 (m, 2H), 1.64 - 1.39 (m, 2H), 1.01 (d, *J*=6.9 Hz, 3H), 0.79 - 0.63 (m, 1H). Analytical HPLC (Method A): RT = 4.41 min, 99.9% purity; Factor XIa Ki = 23 nM, Plasma Kallikrein Ki = 1,100 nM.

Example 234

Preparation of (10R,14S)-3-chloro-14-{4-[5-chloro-2-(4-chloro-1H-1,2,3-triazol-1-yl) phenyl]-6-oxo-1,6-dihydropyrimidin-1-yl}-10-methyl-5,8-diazatricyclo[13.3.1.$0^{2,7}$] nonadeca-1(19),2(7),3,5,15,17-hexaen-9-one

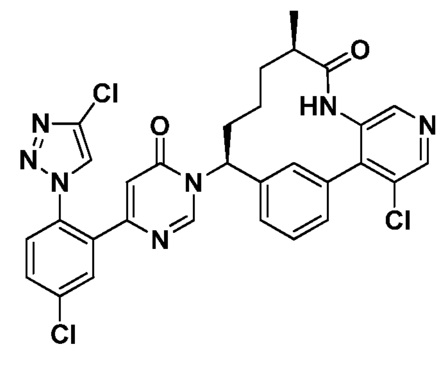

(10*R*,14*S*)-3-Chloro-14-{4-[5-chloro-2-(4-chloro-1H-1,2,3-triazol-1-yl)phenyl]-6-oxo-1,6-dihydropyrimi din-1-yl}-10-methyl-5,8-diazatricyclo[13.3.1.$0^{2,7}$]nonadeca-1(19),2(7),3,5,15,17-hexaen-9-one trifluoroacetate (0.0069 g, 44%) was prepared according to the procedures described in Example 206 by using (10R,14S)-14-amino-3-chloro-10-methyl-5,8-diazatricyclo[13.3.1.$0^{2,7}$]nonadeca-1(19),2(7),3,5,15,17-hexaen-9-one, prepared as described in Intermediate 38, by replacing 2-bromopyridin-3-amine with 4-bromo-5-chloropyridin-3-amine in Intermediate 38B. MS(ESI) *m/z:* 620.1 $(M+H)^+$. $^1H$ NMR (500MHz, $CD_3OD$, 60 °C) $\delta$ 9.00 (s, 1H), 8.75 (s, 1H), 8.71 - 8.60 (m, 1H), 8.56 (s, 1H), 8.16 (d, *J*=2.5 Hz, 1H), 8.03 (dd, *J*=8.4, 2.3 Hz, 1H), 7.97 - 7.82 (m, 4H), 7.73 (br. s., 1H), 6.70 (s, 1H), 6.04 (dd, *J*=12.8, 3.2 Hz, 1H), 2.73 - 2.55 (m, 2H), 2.52 - 2.41 (m, 1H), 2.00 (d, *J*=9.1 Hz, 2H), 1.85 - 1.58 (m, 1H), 1.47 (d, *J*=6.6 Hz, 3H), 1.45 -

1.23 (m, 1H). Analytical HPLC (Method A): RT = 9.14 min, purity > 99%; Factor XIa Ki = 0.29 nM, Plasma Kallikrein Ki = 80 nM.

Example 235

Preparation of (9R,13S)-13-{4-[5-chloro-2-(1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

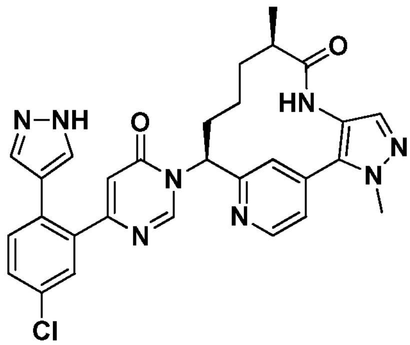

(9*R*,13*S*)-13-{4-[5-Chloro-2-(1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (2.97 mg, 14% yield) was prepared in a similar manner as the procedure described in Example 49, by replacing 1-methyl-4-(4,4,5,5-tetramethyl-1,3,2-dioxaborolan-2-yl)-1H-pyrazole with *tert*-butyl 4-(4,4,5,5-tetramethyl-1,3,2-dioxaborolan-2-yl)-1H-pyrazole-1-carboxylate (14.35 mg, 0.049 mmol). MS(ESI) *m/z:* 555.5 $(M+H)^+$. $^1H$ NMR (400MHz, $CD_3OD$) δ 8.97 (s, 1H), 8.75 (d, *J*=5.1 Hz, 1H), 7.70 (s, 1H), 7.57 - 7.47 (m, 7H), 6.37 (s, 1H), 6.02 (dd, *J*=12.5, 4.4 Hz, 1H), 4.05 (s, 3H), 2.76 - 2.67 (m, 1H), 2.39 - 2.28 (m, 1H), 2.15 - 1.98 (m, 2H), 1.67 - 1.43 (m, 2H), 1.01 (d, *J*=7.0 Hz, 3H), 0.80 - 0.63 (m, 1H). Analytical HPLC (Method A): RT = 6.50 min, 99.7% purity; Factor XIa Ki = 14 nM, Plasma Kallikrein Ki = 550 nM.

Example 236

Preparation of N-benzyl-4-chloro-2-{1-[(9R,13S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}benzamide

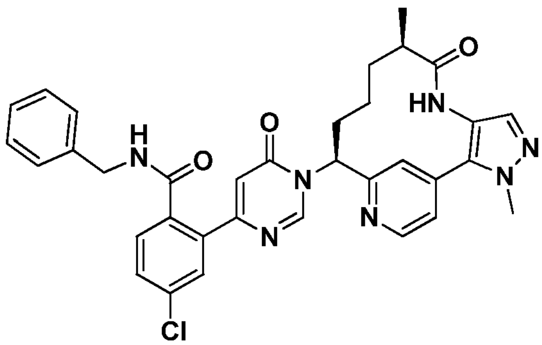

To a cooled (0 °C) solution of phenylmethanamine (0.081 ml, 0.741 mmol) in DCE (1.48 ml) was added dropwise a 2.0 M $Al(Me)_3$ in hexane (0.36 ml, 0.72 mmol). A white plume of gas formed above the reaction mixture. Gas evolution was observed in the solution. The resulting clear solution was stirred at 0 °C for 15 min, and then warmed to rt for 2 h. Next a solution of methyl 4-chloro-2-{1-[(9*R*,13*S*)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}benzoate (0.049 g, 0.074 mmol), prepared as described in Example 168, in DCE (1 ml) was added and the resulting clear, yellow reaction was heated to 40 °C. After 5 h, the reaction was cooled to rt and the reaction was added to a cold (0 °C), vigorously stirred suspension of DCM/sat Rochelle's salt. The biphasic mixture was stirred for 10-15 min and then the layers were separated. The aqueous layer

was extracted with DCM. The combined organic layers were washed with 1.0 N HCl, brine, dried over $Na_2SO_4$, filtered and concentrated. Purification by reverse phase chromatography afforded N-benzyl-4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}benzamide trifluoroacetate (6 mg, 11% yield) as a yellow solid. MS(ESI) *m/z:* 622.6 $(M+H)^+$. $^1H$ NMR (400MHz, $CD_3OD$) δ 8.80 (s, 1H), 8.71 (d, *J*=5.1 Hz, 1H), 7.72 (s, 1H), 7.69 (s, 1H), 7.55 - 7.49 (m, 4H), 7.34 - 7.15 (m, 5H), 6.66 (s, 1H), 6.02 (dd, *J*=12.5, 4.2 Hz, 1H), 4.51 - 4.39 (m, 2H), 4.05 (s, 3H), 2.78 - 2.67 (m, 1H), 2.34 - 2.21 (m, 1H), 2.16 - 2.05 (m, 1H), 2.03 - 1.92 (m, 1H), 1.69 - 1.42 (m, 2H), 1.02 (d, *J*=6.8 Hz, 3H), 0.77 - 0.59 (m, 1H). Analytical HPLC (Method A): RT = 7.27 min, 97.9% purity; Factor XIa Ki = 23 nM, Plasma Kallikrein Ki = 1,600 nM.

Example 238

Preparation of 1-(4-chloro-3-fluoro-2-{1-[(9*R*,13S)-3-($^2H_3$)methyl-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazole-4-carbonitrile

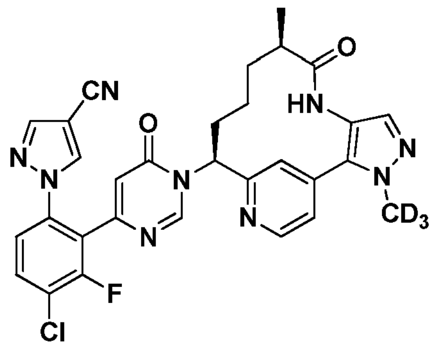

238A. Preparation of 4-(3-chloro-2-fluoro-6-iodophenyl)-6-methoxypyrimidine

To a suspension of 4-chloro-3-fluoro-2-(6-methoxypyrimidin-4-yl)aniline (2 g, 7.88 mmol) in $CH_3CN$ (90 ml) at 0 °C was added *p*TsOH·$H_2O$ (3.75 g, 19.71 mmol) followed by the dropwise addition of a solution of $NaNO_2$ (1.088 g, 15.77 mmol) and NaI (2.95 g, 19.71 mmol) in water (22.5 ml). The reaction was allowed to warm to rt and stir overnight. The reaction was quenched with sat $NaHCO_3$, and extracted with EtOAc. The organic layer was washed with sat $Na_2S_2O_3$, brine, dried with $MgSO_4$, filtered, and concentrated. Purification by normal phase chromatography gave 4-(3-chloro-2-fluoro-6-iodophenyl)-6-methoxypyrimidine (2.18 g, 76% yield) as viscous, yellow oil. MS(ESI) *m/z:* 365.1 $(M+H)^+$. $^1H$ NMR (400MHz, $CDCl_3$) δ 8.92 (d, *J*=0.9 Hz, 1H), 7.68 (dd, *J*=8.4, 1.5 Hz, 1H), 7.20 (dd, *J*=8.5, 7.4 Hz, 1H), 6.78 (s, 1H), 4.07 (s, 3H).

238B. Preparation of 6-(3-chloro-2-fluoro-6-iodophenyl)pyrimidin-4-ol

To a suspension of 4-(3-chloro-2-fluoro-6-iodophenyl)-6-methoxypyrimidine (0.22 g, 0.603 mmol) in ACN (6.03 ml) was added TMSI (0.411 ml, 3.02 mmol). The resulting clear, yellow solution was heated to 50 °C for 15 h. Additional TMSI (0.4 ml) was added and the reaction was heated 50 °C for 7 h. The reaction was poured into a 10% $Na_2S_2O_3$ and sat $NaHCO_3$, extracted with EtOAc (2x), and a mixture of DCM and MeOH. The organic layers were combined and concentrated. Purification by normal phase chromatography gave 6-(3-chloro-2-fluoro-6-iodophenyl)pyrimidin-4-ol (190 mg, 90% yield) as a yellow glass. MS(ESI) *m/z:* 351.1 $(M+H)^+$. $^1H$ NMR (400MHz, $CD_3OD$) δ 8.33 (d, *J*=1.1 Hz, 1H), 7.77 (dd, *J*=8.6, 1.5 Hz, 1H), 7.34 (dd, *J*=8.6, 7.5 Hz, 1H), 6.51 (d, *J*=0.9 Hz, 1H), 4.85 (br. s., 1H).

238C. Preparation of 1-(4-chloro-3-fluoro-2-(6-hydroxypyrimidin-4-yl)phenyl)-1H-pyrazole-4-carbonitrile

To a suspension of 1H-pyrazole-4-carbonitrile (50.5 mg, 0.542 mmol), $K_3PO_4$ (233 mg, 1.084 mmol) and 6-(3-chloro-2-fluoro-6-iodophenyl)pyrimidin-4-ol (190 mg, 0.542 mmol) in dioxane (0.24 mL) was added (1R,2R)-N1,N2-dimethylcyclohexane-1,2-diamine (38.6 mg, 0.271 mmol). The vial was then purged with Ar, CuI (5.16 mg, 0.027 mmol) was added, and the vial was sealed. The reaction mixture was heated at 80 °C. After 16 h, the reaction was cooled to rt, filtered, and the filtrate was concentrated. Purification by reverse phase chromatography gave 1-(4-chloro-3-fluoro-2-(6-hydroxypyrimidin-4-yl)phenyl)-1H-pyrazole-4-carbonitrile (74 mg, 43% yield) as an off-white solid. MS(ESI) *m/z:*

316.3 $(M+H)^+$. $^1H$ NMR (400MHz, $CD_3OD$) δ 8.54 (s, 1H), 8.09 (d, *J*=0.9 Hz, 1H), 7.97 (s, 1H), 7.80 (dd, *J*=8.7, 7.8 Hz, 1H), 7.50 (dd, *J*=8.7, 1.7 Hz, 1H), 6.51 (t, *J*=1.1 Hz, 1H).

238D. Preparation of 1-(4-chloro-3-fluoro-2-{1-[(9*R*,13S)-3-($^2H_3$)methyl-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazole-4-carbonitrile, trifluoroacetate

1-(4-Chloro-3-fluoro-2-{1-[(9R,13S)-3-($^2H_3$)methyl-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazole-4-carbonitrile trifluoroacetate (9.5 mg, 41% yield) was prepared in a similar manner as the procedure described in Example 56, by using 1-(4-chloro-3-fluoro-2-(6-hydroxypyrimidin-4-yl)phenyl)-1H-pyrazole-4-carbonitrile (10 mg, 0.032 mmol) and (9*R*,13*S*)-13-amino-3-($^2H_3$)methyl-9-methyl-3,4,7,15-tetraazatricyclo[12.3.1.0$^{2,6}$]octadeca-1(18),2(6),4,14,16-pentaen-8-one(9.58mg, 0.032 mmol), prepared as described in Intermediate 33. MS(ESI) *m/z:* 601.0 $(M+H)^+$. $^1H$ NMR (400MHz, $CD_3OD$) δ 8.81 (s, 1H), 8.76 (d, *J*=5.1 Hz, 1H), 8.46 (s, 1H), 7.94 (s, 1H), 7.80 (dd, *J*=8.6, 7.7 Hz, 1H), 7.69 (s, 1H), 7.55 - 7.48 (m, 3H), 6.54 (s, 1H), 6.00 (dd, *J*=13.0, 4.2 Hz, 1H), 2.70 (dt, *J*=6.7, 3.2 Hz, 1H), 2.36 - 2.25 (m, 1H), 2.15 - 1.95 (m, 2H), 1.67 - 1.55 (m, 1H), 1.55 - 1.41 (m, 1H), 1.01 (d, *J*=6.8 Hz, 3H), 0.72 (m., 1H). Analytical HPLC (Method A): RT = 7.58 min, purity = 98.5%; Factor XIa Ki = 2.4 nM, Plasma Kallikrein Ki = 1,500 nM.

Example 239

Preparation of 1-(4-chloro-2-{1-[(9R,13S)-3-(difluoromethyl)-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile

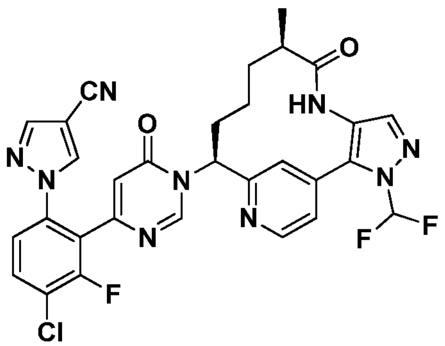

1-(4-Chloro-2-{1-[(9R,13S)-3-(difluoromethyl)-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile was prepared in a similar manner as the procedure described in Example 238, by replacing (9*R*,13*S*)-13-amino-3-($^2H_3$)methyl-9-methyl-3,4,7,15-tetraazatricyclo[12.3.1.0$^{2,6}$]octadeca-1(18),2(6),4,14,16-pentaen-8-one, prepared as described in Intermediate 33, with (9*R*,13*S*)-13-amino-3-(difluoromethyl)-9-methyl-3,4,7,15-tetraazatricyclo[12.3.1.0$^{2,6}$] octadeca-1(18),2(6),4,14,16-pentaen-8-one (10.62 mg, 0.032 mmol), prepared as described in Example 30G. MS(ESI) *m/z:* 634.0 $(M+H)^+$. $^1H$ NMR (400MHz, $CD_3OD$) δ 8.86 (s, 1H), 8.78 (d, *J*=5.1 Hz, 1H), 8.47 (s, 1H), 7.94 (s, 1H), 7.83 - 7.77 (m, 1H), 7.74 (s, 1H), 7.70 (s, 1H), 7.65 (s, 1H), 7.52 (dd, *J*=8.7, 1.4 Hz, 2H), 6.55 (s, 1H), 6.01 (dd, *J*=12.7, 4.1 Hz, 1H), 2.76 - 2.65 (m, 1H), 2.31 (t, *J*=13.0 Hz, 1H), 2.10 - 1.95 (m, 2H), 1.65 - 1.42 (m, 2H), 1.00 (d, *J*=7.0 Hz, 3H), 0.67 (m., 1H). Analytical HPLC (Method A): SunFire, RT = 8.75 min, 98.8% purity; Factor XIa Ki = 1.0 nM, Plasma Kallikrein Ki = 1,100 nM.

Example 240

Preparation of (9R,13S)-13-{4-[5-chloro-2-(1-propyl-1H-pyrazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

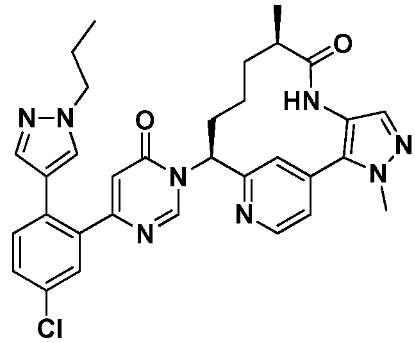

(9*R*,13*S*)-13-{4-[5-Chloro-2-(1-propyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (14.3 mg, 62% yield) was prepared in a similar manner as the procedure described in Example 49, by replacing 1-methyl-4-(4,4,5,5-tetramethyl-1,3,2-dioxaborolan-2-yl)-1H-pyrazole with 1-propyl-4-(4,4,5,5-tetramethyl-1,3,2-dioxaborolan-2-yl)-1H-pyrazole (11.52 mg, 0.049 mmol). MS(ESI) *m/z:* 597.4 $(M+H)^+$. $^1H$ NMR (400MHz, $CD_3OD$) δ 9.01 (s, 1H), 8.75 (d, *J*=5.1 Hz, 1H), 7.74 (s, 1H), 7.58 - 7.52 (m, 3H), 7.50 - 7.47 (m, 3H), 7.42 (s, 1H), 6.40 (d, *J*=0.4 Hz, 1H), 6.02 (dd, *J*=12.7, 4.3 Hz, 1H), 4.07 - 4.01 (m, 5H), 2.76 - 2.67 (m, 1H), 2.40 - 2.29 (m, 1H), 2.13 - 2.00 (m, 2H), 1.77 (sxt, *J*=7.2 Hz, 2H), 1.67 - 1.43 (m, 2H), 1.02 (d, *J*=6.8 Hz, 3H), 0.79 (t, *J*=7.4 Hz, 3H), 0.75 - 0.66 (m, 1H). Analytical HPLC (Method A): RT = 8.12 min, 100% purity; Factor XIa Ki = 35 nM, Plasma Kallikrein Ki = 3,800 nM.

Example 243

Preparation of 1-(4-chloro-2-{1-[(9R,13S)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)-1H-imidazole-4-carbonitrile

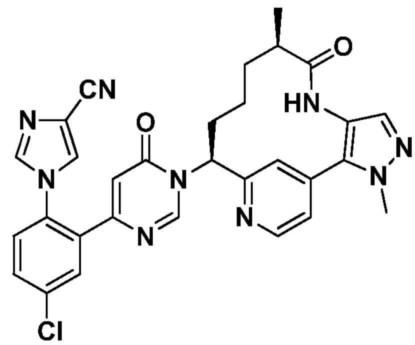

1-(4-Chloro-2-{1-[(9*R*,13*S*)-3,9-dimethyl-8-oxo-3,4,7,15-tetraazatricyclo [12.3.1.$0^{2,6}$]octadeca-1(18),2(6),4,14,16-pentaen-13-yl]-6-oxo-1,6-dihydropyrimidin-4-yl}phenyl)-1H-imidazole-4-carbonitrile trifluoroacetate (9.7 mg, 37% yield) was prepared in a similar manner as the procedure described in Example 231, by replacing 1H-imidazole with 1H-imidazole-4-carbonitrile (0.012 g, 0.130 mmol). MS(ESI) *m/z:* 580.20 $(M+H)^+$. $^1H$ NMR (500MHz, DMSO-$d_6$) δ 9.22 (s, 1H), 8.82 (s, 1H), 8.69 (d, *J*=5.2 Hz, 1H), 8.29 (s, 1H), 8.04 (s, 1H), 7.88 (d, *J*=2.4 Hz, 1H), 7.78 (dd, *J*=8.5, 2.1 Hz, 1H), 7.70 - 7.66 (m, 2H), 7.59 (d, *J*=4.9 Hz, 1H), 7.48 (s, 1H), 6.37 (s, 1H), 5.89 (d, *J*=10.7 Hz, 1H), 4.01 (s, 3H), 2.69 - 2.61 (m, 1H), 2.39 - 2.27 (m, 1H), 2.11 (t, *J*=12.5 Hz, 1H), 1.89 - 1.80 (m, 1H), 1.53 - 1.42 (m, 1H), 1.39 - 1.29 (m, 1H), 0.89 (d, *J*=7.0 Hz, 3H), 0.50 - 0.34 (m, 1H). Analytical HPLC (Method B): RT = 8.12 min, 100% purity; Factor XIa Ki = 53 nM, Plasma Kallikrein Ki = 3,400 nM

Example 244

Preparation of N-(4-chloro-2-{1-[(9R,13S)-3-(difluoromethyl)-9-methyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)-2,2,2-trifluoroacetamide

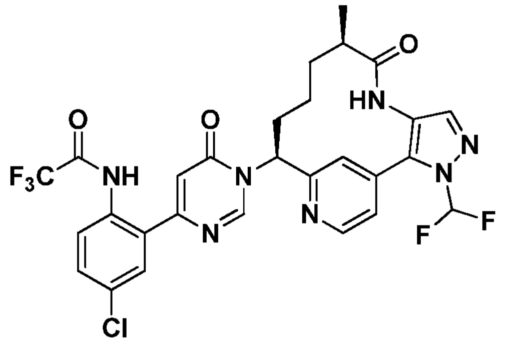

*N*-(4-Chloro-2-{1-[(9*R*,13*S*)-3-(difluoromethyl)-9-methyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide was prepared in a similar manner as the procedure described in Example 56, using *N*-(4-chloro-2-(6-hydroxypyrimidin-4-yl)phenyl)-2,2,2-trifluoroacetamide (0.19 g, 0.60 mmol), prepared as described in Intermediate 1, and (9*R*,13*S*)-13-amino-3-(difluoromethyl)-9-methyl-3,4,7,15-tetraazatricyclo[12.3.1.0$^{2,6}$]octadeca-1(18),2(6),4,14,16-pentaen-8-one(0.20g, 0.60 mmol), prepared as described in Intermediate 30, to give *N*-(4-chloro-2-{1-[(9*R*,13*S*)-3-(difluoromethyl)-9-methyl-8-oxo-3,4,7,15-tetraazatricyclo[12.3.1.0$^{2,6}$] octadeca-1(18),2(6),4,14,16-pentaen-13-yl]-6-oxo-1,6-dihydropyrimidin-4-yl}phenyl)-2,2,2-trifluoroacetamide (222 mg, 58%) as white powder. MS(ESI) *m/z:* 636.5 (M+H)$^+$. $^1$H NMR (400MHz, $CD_3OD$) δ 9.23 (s, 1H), 8.89 (s, 1H), 8.56 (d, *J*=5.1 Hz, 1H), 7.74 - 7.69 (m, 3H), 7.55 (s, 1H), 7.46 - 7.39 (m, 1H), 7.24 (d, *J*=5.1 Hz, 1H), 6.74 (s, 1H), 5.83 - 5.70 (m, 1H), 2.55 - 2.40 (m, 1H), 2.27 - 2.12 (m, 1H), 1.89 (s, 1H), 1.81 - 1.66 (m, 1H), 1.38 - 1.24 (m, 1H), 1.24 - 1.11 (m, 1H), 0.70 (d, *J*=7.0 Hz, 3H). Analytical HPLC (Method A): RT = 10.89 min, purity = 99%; Factor XIa Ki = 3.9 nM, Plasma Kallikrein Ki = 260 nM.

Example 245

Preparation of (9R,13S)-13-(4-{5-chloro-2-[1-(propan-2-yl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one

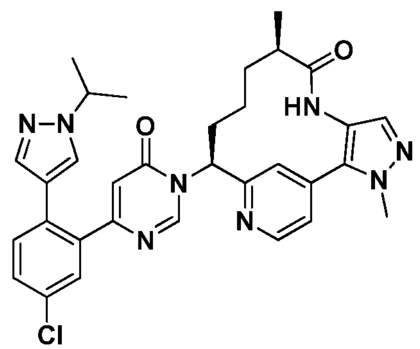

(9*R*,13*S*)-13-(4-{5-Chloro-2-[1-(propan-2-yl)-1H-pyrazol-4-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one trifluoroacetate (15.5 mg, 67% yield) was prepared in a similar manner as the procedure described in Example 49, by replacing 1-methyl-4-(4,4,5,5-tetramethyl-1,3,2-dioxaborolan-2-yl)-1H-pyrazole with 1-isopropyl-4-(4,4,5,5-tetramethyl-1,3,2-dioxaborolan-2-yl)-1H-pyrazole (11.52 mg, 0.049 mmol). MS(ESI) *m/z:* 597.4 (M+H)$^+$. $^1$H NMR (400MHz, $CD_3OD$) δ 9.02 (s, 1H), 8.75 (d, *J*=5.1 Hz, 1H), 7.74 (s, 1H), 7.62 (s, 1H), 7.56 (dd, *J*=5.2, 1.7 Hz, 1H), 7.54 - 7.52 (m, 1H), 7.50 - 7.47 (m, 3H), 7.39 (s, 1H), 6.40 (d, *J*=0.4 Hz, 1H), 6.02 (dd, *J*=12.7, 4.3 Hz, 1H), 4.46 (spt, *J*=6.7 Hz, 1H), 4.05 (s, 3H), 2.76 - 2.66 (m, 1H), 2.41 - 2.30 (m, 1H), 2.14 - 2.00 (m, 2H), 1.67 - 1.37 (m, 8H), 1.02 (d, *J*=6.8 Hz, 3H), 0.80 - 0.64 (m, 1H). Analytical HPLC (Method A): RT = 8.07 min, 100% purity; Factor XIa Ki = 44 nM, Plasma Kallikrein Ki = 5,600 nM

Example 248

Preparation of (9R,13S)-13-(4-{5-chloro-2-[1-(2-methylpropyl)-1H-pyrazol-4-yl] phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one

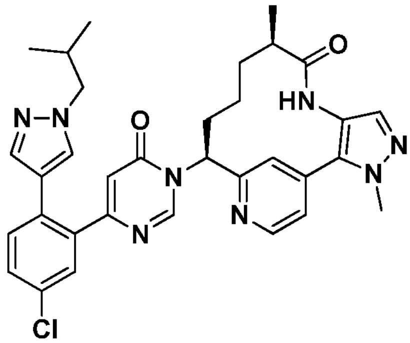

(9*R*,13*S*)-13-(4-{5-Chloro-2-[1-(2-methylpropyl)-1H-pyrazol-4-yl]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one trifluoroacetate (7.6 mg, 32% yield) was prepared in a similar manner as the procedure described in Example 49, by replacing 1-methyl-4-(4,4,5,5-tetramethyl-1,3,2-dioxaborolan-2-yl)-1H-pyrazole with 1-isobutyl-4-(4,4,5,5-tetramethyl-1,3,2-dioxaborolan-2-yl)-1H-pyrazole (12.20 mg, 0.049 mmol). MS(ESI) *m/z:* 611.4 $(M+H)^+$. $^1H$ NMR (400MHz, $CD_3OD$) $\delta$ 9.00 (s, 1H), 8.75 (d, *J*=5.3 Hz, 1H), 7.72 (s, 1H), 7.57 - 7.47 (m, 6H), 7.42 (s, 1H), 6.37 (d, *J*=0.4 Hz, 1H), 6.02 (dd, *J*=12.8, 4.2 Hz, 1H), 4.05 (s, 3H), 3.91 - 3.81 (m, 2H), 2.76 - 2.67 (m, 1H), 2.34 (tt, *J*=12.7, 4.3 Hz, 1H), 2.14 - 1.98 (m, 3H), 1.67 - 1.43 (m, 2H), 1.01 (d, *J*=6.8 Hz, 3H), 0.83 - 0.63 (m, 7H). Analytical HPLC (Method A): RT = 8.63 min, 100% purity; Factor XIa Ki = 73 nM, Plasma Kallikrein Ki = 8,900 nM.

Example 249

Preparation of (9R,13S)-13-{4-[2-(1-benzyl-1H-pyrazol-4-yl)-5-chlorophenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

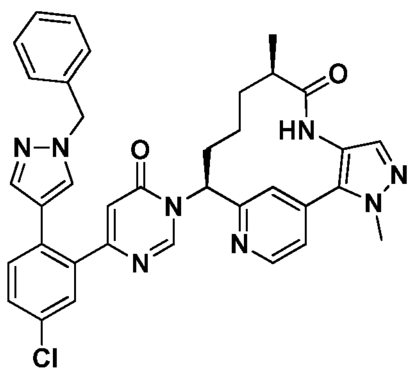

(9*R*,13*S*)-13-{4-[2-(1-Benzyl-1H-pyrazol-4-yl)-5-chlorophenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (2.68 mg, 11% yield) was prepared in a similar manner as the procedure described in Example 49, by replacing 1-methyl-4-(4,4,5,5-tetramethyl-1,3,2-dioxaborolan-2-yl)-1H-pyrazole with 1-benzyl-4-(4,4,5,5-tetramethyl-1,3,2-dioxaborolan-2-yl)-1H-pyrazole (13.86 mg, 0.049 mmol). MS(ESI) *m/z:* 645.4 $(M+H)^+$. $^1H$ NMR (400MHz, $CD_3OD$) $\delta$ 8.95 (s, 1H), 8.67 (d, *J*=5.1 Hz, 1H), 7.70 (s, 1H), 7.59 (s, 1H), 7.54 - 7.45 (m, 6H), 7.29 - 7.19 (m, 3H), 7.09 - 7.05 (m, 2H), 6.36 (d, *J*=0.7 Hz, 1H), 6.00 (dd, *J*=12.7, 4.1 Hz, 1H), 5.27 (s, 2H), 4.02 (s, 3H), 2.77 - 2.68 (m, 1H), 2.34 - 2.23 (m, 1H), 2.15 - 2.05 (m, 1H), 2.01 - 1.90 (m, 1H), 1.67 - 1.42 (m, 2H), 1.02 (d, *J*=6.8 Hz, 3H), 0.76 - 0.60 (m, 1H). Analytical HPLC (Method A): RT = 8.90 min, 100% purity; Factor XIa Ki = 100 nM.

Example 250

Preparation of (9R,13S)-13-{4-[5-chloro-2-(1-methyl-1H-1,2,3-triazol-4-yl)phenyl]-6-oxo-1,6-dihydropyrimidin-1-yl}-3,9-dimethyl-3,4,7,15-tetraazatricyclo [12.3.1.0$^{2,6}$]octadeca-1(18),2(6),4,14,16-pentaen-8-one

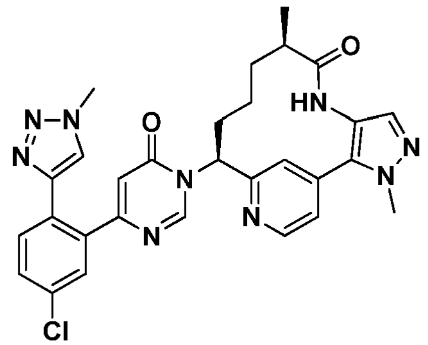

250A. Preparation of 4-(5-chloro-2-ethynylphenyl)-6-methoxypyrimidine

A flame-dried flask containing 4-(5-chloro-2-iodophenyl)-6-methoxypyrimidine (0.520 g, 1.50 mmol), prepared as described in Example 211, and $Pd(PPh_3)_4$ (0.087 g, 0.075 mmol) was purged with Ar for several min. Next, degassed THF (7.50 ml) and tributylstannylacetylene (0.651 ml, 2.25 mmol) were added. The resulting clear, burgundy solution was stirred at rt. After 15 h, the dark purple reaction was diluted with EtOAc and washed with brine, dried over $Na_2SO_4$, filtered and concentrated to give a purple solid. Purification by normal phase chromatography, using 0-10% EtOAc/Hex, gave 4-(5-chloro-2-ethynylphenyl)-6-methoxypyrimidine (0.176 g, 48%) as an off-white solid. MS(ESI) *m/z:* 244.9 $(M+H)^+$.

250B. Preparation of 4-(5-chloro-2-{1-[(trimethylsilyl)methyl]-1H-1,2,3-triazol-4-yl} phenyl)-6-methoxypyrimidine

A sealed vial containing a mixture of $TMSN_3$ (0.091 ml, 0.61 mmol), 4-(5-chloro-2-ethynylphenyl)-6-methoxypyrimidine (0.050 g, 0.20 mmol), sodium ascorbate (8.10 mg, 0.041 mmol), and $CuSO_4$ (3.26 mg, 0.020 mmol) was stirred at 60 °C. After 2 h, the dark black reaction was cooled to rt. The mixture was diluted with EtOAc (15 mL), and the turbid solution was washed water (3x), brine, dried over $Na_2SO_4$, filtered and concentrated to give 0.110 g of crude product as a black oil. Purification by normal phase chromatography using 0-40% EtOAc/Hex, gave 4-(5-chloro-2-{1-[(trimethylsilyl) methyl]-1H-1,2,3-triazol-4-yl}phenyl)-6-methoxypyrimidine (0.0191 g, 25%) as a yellow residue. MS(ESI) *m/z:* 374.0 $(M+H)^+$.

250C. Preparation of 4-[5-chloro-2-(1-methyl-1H-1,2,3-triazol-4-yl)phenyl]-6-methoxypyrimidine

To a cooled (0 °C) clear yellow solution of 4-(5-chloro-2-(1-((trimethylsilyl) methyl)-1H-1,2,3-triazol-4-yl)phenyl)-6-methoxypyrimidine (0.019 g, 0.051 mmol) in THF (0.508 ml) and water (1.831 μl, 0.102 mmol) was added dropwise 1.0 M TBAF in THF (0.061 ml, 0.061 mmol). The reaction was stirred at 0 °C for 1 h and then warmed to rt. After 2 h, additional 1.0 M TBAF in THF (0.061 ml, 0.061 mmol) was added. After 51 h, additional 1.0 M TBAF in THF (0.51 ml, 0.51 mmol) was added. After 44 h, the reaction was diluted with EtOAc and washed with sat $NH_4Cl$, brine, dried over $Na_2SO_4$, filtered and concentrated to give 4-(5-chloro-2-(1-methyl-1H-1,2,3-triazol-4-yl)phenyl)-6-methoxypyrimidine (0.015 g, 98%) as a yellow residue. MS(ESI) *m/z:* 302.0 $(M+H)^+$.

250D. Preparation of 6-[5-chloro-2-(1-methyl-1H-1,2,3-triazol-4-yl)phenyl]pyrimidin-4-ol

A clear, yellow solution of 4-(5-chloro-2-(1-methyl-1H-1,2,3-triazol-4-yl)phenyl)-6-methoxypyrimidine (0.015 g, 0.050 mmol) in AcOH (0.50 ml) and 48% aq HBr (0.28 ml, 2.486 mmol) was warmed to 85 °C. After 1 h, the reaction was cooled to rt and then concentrated to give a brown solid. The brown solid was suspended in EtOAc and filtered to give and off-white solid. Purification by reverse phase chromatography gave, after free-basing with sat $NaHCO_3$, 6-[5-chloro-2-(1-methyl-1H-1,2,3-triazol-4-yl) phenyl]pyrimidin-4-ol (0.0090 g, 63%) as a white solid. MS(ESI) *m/z:* 288.0 $(M+H)^+$. $^1H$ NMR (500MHz, $CD_3OD$) δ 8.21 (s, 1H), 7.90 (s, 1H), 7.72 (d, *J*=8.3 Hz, 1H), 7.60 (d, *J*=2.2 Hz, 1H), 7.58 (dd, *J*=8.3, 2.2 Hz, 1H), 6.38 (s, 1H), 4.09 (s, 3H).

250E. Preparation of (9R,13S)-13-{4-[5-chloro-2-(1-methyl-1H-1,2,3-triazol-4-yl) phenyl]-6-oxo-1,6-dihydropyrimidin-1-yl}-3,9-dimethyl-3,4,7,15-tetraazatricyclo [12.3.1.0$^{2,6}$]octadeca-1(18),2(6),4,14,16-pentaen-8-one trifluoroacetate

(9R,13S)-13-{4-[5-Chloro-2-(1-methyl-1H-1,2,3-triazol-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (0.0055 g, 25%) was prepared in a similar manner as the procedure described in Example 56, by replacing 6-{5-chloro-2-[4-(trifluoromethyl)-1H-1,2,3-triazol-1-yl]phenyl}pyrimidin-4-ol with 6-[5-chloro-2-(1-methyl-1H-1,2,3-triazol-4-yl)phenyl]pyrimidin-4-ol. MS(ESI) *m/z:* 570.1 $(M+H)^+$ and 572.0 $(M+2+H)^+$. $^1H$ NMR (500MHz, $CD_3OD$) δ 8.94 (s, 1H), 8.75 (d, *J*=5.2 Hz, 1H), 7.90 (s, 1H), 7.74 (s, 1H), 7.70 (d, *J*=8.3 Hz, 1H), 7.63 (d, *J*=2.2 Hz, 1H), 7.59 - 7.54 (m, 2H), 7.50 (s, 1H), 6.40 (d, *J*=0.6 Hz, 1H), 6.04 - 5.99 (m, 1H), 4.08 (s, 3H), 4.05 (s, 3H), 2.76 - 2.67 (m, 1H), 2.40 - 2.29 (m, 1H), 2.15 - 2.01 (m, 2H), 1.68 - 1.56 (m, 1H), 1.55 - 1.43 (m, 1H), 1.02 (d, *J*=7.2 Hz, 3H), 0.79 - 0.64 (m, 1H). Analytical HPLC (Method A): RT = 6.30 min, purity = 100%; Factor XIa Ki = 11 nM, Plasma Kallikrein Ki = 2,000 nM.

Example 251

Preparation of (10R,14S)-14-(4-{5-chloro-2-[4-(trifluoromethyl)-1H-1,2,3-triazol-1-yl] phenyl}-6-oxo-1,6-dihydropyrimidin-1-yl)-4-fluoro-10-methyl-5,8,16-triazatricyclo[13.3.1.0$^{2,7}$]nonadeca-1(19),2(7),3,5,15,17-hexaen-9-one

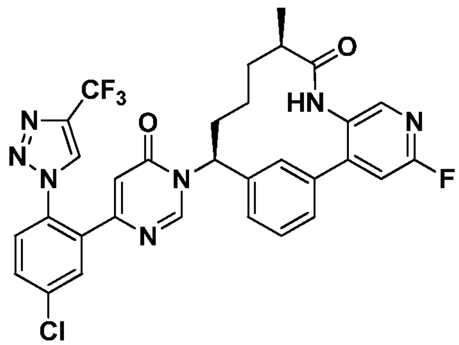

251A. Preparation of *tert*-butyl N-(6-fluoro-4-iodopyridin-3-yl)carbamate

BuLi (11.31 ml, 28.3 mmol) was added dropwise to a stirred, cooled (-78 °C) solution of tert-butyl (6-fluoropyridin-3-yl)carbamate (2 g, 9.42 mmol) and TMEDA (4.27 ml, 28.3 mmol) in $Et_2O$ (47.1 ml). The mixture was allowed to warm to -10 °C and stirred for 2 h. The mixture was recooled to -78 °C and a cooled (-10 °C) solution of $I_2$ (4.90 g, 19.32 mmol) in $Et_2O$ (25 mL) was added dropwise. The mixture was allowed to warm to rt and stirred for 2 days. Sat aq $NH_4Cl$ was added and the mixture was extracted with $Et_2O$ and EtOAc. The combined organic fractions were washed with $Na_2S_2O_3$, brine, dried over $MgSO_4$, filtered and concentrated to give a brownish oil, which was purified by normal phase chromatography to give *tert*-butyl N-(6-fluoro-4-iodopyridin-3-yl)carbamate (0.859 g, 27% yield). MS(ESI) *m/z:* 338.9 $(M+H)^+$.

251B. Preparation of 6-fluoro-4-iodopyridin-3-amine

To a solution of N-(6-fluoro-4-iodopyridin-3-yl)carbamate (400 mg, 1.183 mmol) in DCM (15 mL) was added TFA (4.56 mL, 59.2 mmol). The reaction was stirred at rt for 1 h. Concentration gave 6-fluoro-4-iodopyridin-3-amine.trifluoroacetate (551 mg, 100%) as a pale yellow solid. MS(ESI) *m/z:* 238.9 $(M+H)^+$.

251C. Preparation of (10R,14S)-14-amino-4-fluoro-10-methyl-5,8,16-triazatricyclo [13.3.1.0$^{2,7}$]nonadeca-1(19),2,4,6,15,17-hexaen-9-one

(10R,14S)-14-Amino-4-fluoro-10-methyl-5,8,16-triazatricyclo[13.3.1.0$^{2,7}$] nonadeca-1(19),2,4,6,15,17-hexaen-9-one was prepared in a similar manner as the procedure described in Intermediate 39, by replacing 2-bromopyridin-3-amine with 6-fluoro-4-iodopyridin-3-amine. MS(ESI) *m/z:* 315.4 $(M+H)^+$.

251D. Preparation of (10R,14S)-14-(4-{5-chloro-2-[4-(trifluoromethyl)-1H-1,2,3-triazol-1-yl]phenyl}-6-oxo-1,6-dihydropyrimidin-1-yl)-4-fluoro-10-methyl-5,8,16-triazatricyclo [13.3.1.$0^{2,7}$]nonadeca-1(19),2(7),3,5,15,17-hexaen-9-one

(10R,14S)-14-(4-{5-Chloro-2-[4-(trifluoromethyl)-1H-1,2,3-triazol-1-yl]phenyl}-6-oxo-1,6-dihydropyrimidin-1-yl)-4-fluoro-10-methyl-5,8,16-triazatricyclo[13.3.1.$0^{2,7}$] nonadeca-1(19),2(7),3,5,15,17-hexaen-9-one trifluoroacetate (7.3 mg, 33.7% yield) was prepared in a similar manner as the procedure described in Example 56 by using (10R,14S)-14-amino-4-fluoro-10-methyl-5,8,16-triazatricyclo[13.3.1.$0^{2,7}$]nonadeca-1(19),2,4,6,15,17-hexaen-9-one (8.8 mg, 0.028 mmol). $^1$H NMR (400MHz, $CD_3OD$) δ 8.91 (s, 1H), 8.83 - 8.79 (m, 1H), 8.71 (d, J=5.1 Hz, 1H), 8.13 - 8.09 (m, 1H), 7.89 (d, J=2.4 Hz, 1H), 7.78 - 7.65 (m, 3H), 7.48 (dd, J=5.1, 1.8 Hz, 1H), 7.37 (d, J=2.2 Hz, 1H), 6.42 (d, J=0.7 Hz, 1H), 6.04 (dd, J=12.5, 4.8 Hz, 1H), 2.79 - 2.65 (m, 1H), 2.27 - 2.14 (m, 1H), 2.07 - 1.92 (m, 2H), 1.60 - 1.37 (m, 2H), 0.95 (d, J=6.8 Hz, 3H), 0.57 (br. s., 1H). MS(ESI) *m/z:* 639.0 (M+H)$^+$. Analytical HPLC (Method A): RT = 9.64 min, purity = 100%; Factor XIa Ki = 0.53 nM, Plasma Kallikrein Ki = 71 nM.

Example 254

Preparation of (9R,13S)-13-{4-[5-chloro-2-(1-cyclopropyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$] octadeca-1(18),2(6),4,14,16-pentaen-8-one

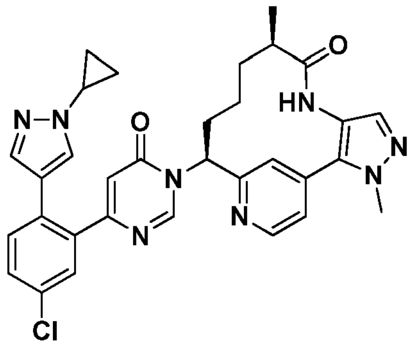

(9*R*,13,*S*)-13-{4-[5-Chloro-2-(1-cyclopropyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (10 mg, 43% yield) was prepared in a similar manner as the procedure described in Example 49, by replacing 1-methyl-4-(4,4,5,5-tetramethyl-1,3,2-dioxaborolan-2-yl)-1H-pyrazole with 1-cyclopropyl-4-(4,4,5,5-tetramethyl-1,3,2-dioxaborolan-2-yl)-1H-pyrazole (11.42 mg, 0.049 mmol). MS(ESI) *m/z:* 595.4 (M+H)$^+$. $^1$H NMR (400MHz, $CD_3OD$) δ 8.99 (s, 1H), 8.76 (d, *J*=5.3 Hz, 1H), 7.73 (s, 1H), 7.64 (s, 1H), 7.57 - 7.52 (m, 2H), 7.50 (s, 1H), 7.47 - 7.46 (m, *J*=1.1 Hz, 2H), 7.33 (d, *J*=0.7 Hz, 1H), 6.39 (s, 1H), 6.03 (dd, *J*=12.5, 4.2 Hz, 1H), 4.05 (s, 3H), 3.63 - 3.56 (m, 1H), 2.76 - 2.67 (m, 1H), 2.35 (tt, *J*=12.7, 4.3 Hz, 1H), 2.14 - 2.01 (m, 2H), 1.67 - 1.44 (m, 2H), 1.04 - 0.97 (m, 7H), 0.79 - 0.64 (m, 1H). Analytical HPLC (Method A): RT = 7.82 min, 99.9% purity; Factor XIa Ki = 8 nM, Plasma Kallikrein Ki = 1,700 nM.

Example 255

Preparation of (9R,13S)-13-(4-{5-chloro-2-[1-(2,2,2-trifluoroethyl)-1H-pyrazol-4-yl] phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one

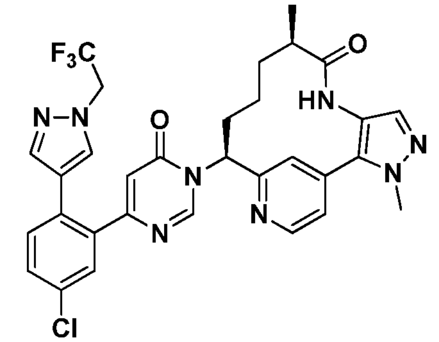

255A. Preparation of 4-(4,4,5,5-tetramethyl-1,3,2-dioxaborolan-2-yl)-1-(2,2,2-trifluoroethyl)-1H-pyrazole

To a stirred solution of 4-(4,4,5,5-tetramethyl-1,3,2-dioxaborolan-2-yl)-1H-pyrazole (100 mg, 0.515 mmol) in DMF (1 ml) were added $Cs_2CO_3$ (252 mg, 0.773 mmol) and 2,2,2-trifluoroethyl trifluoromethanesulfonate (0.144 ml, 1.031 mmol) at rt. After stirring at 100 °C for 2 h, the reaction mixture was evaporated to dryness, partitioned between EtOAc and water, and the layers were separated. The organic layer was dried over $Na_2SO_4$, filtered, and concentrated to afford 4-(4,4,5,5-tetramethyl-1,3,2-dioxaborolan-2-yl)-1-(2,2,2-trifluoroethyl)-1H-pyrazole (0.111 g, 78% yield). MS(ESI) *m*/*z:* 277.4 $(M+H)^+$.

255B. Preparation of (9*R*,13*S*)-13-(4-{5-chloro-2-[1-(2,2,2-trifluoroethyl)-1H-pyrazol-4-yl]phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-onetrifluoroacetate

(9*R*,13*S*)-13-(4-{5-Chloro-2-[1-(2,2,2-trifluoroethyl)-1H-pyrazol-4-yl]phenyl}-6-oxo-1,6-dihydropyrimidin-1-yl)-3,9-dimethyl-3,4,7,15-tetraazatricyclo[12.3.1.$0^{2,6}$] octadeca-1(18),2(6),4,14,16-pentaen-8-one trifluoroacetate (10.6 mg, 43% yield) was prepared in a similar manner as the procedure described in Example 49, by replacing 1-methyl-4-(4,4,5,5-tetramethyl-1,3,2-dioxaborolan-2-yl)-1H-pyrazole with 4-(4,4,5,5-tetramethyl-1,3,2-dioxaborolan-2-yl)-1-(2,2,2-trifluoroethyl)-1H-pyrazole (13.47 mg, 0.049 mmol), prepared as described in Example 255A. MS(ESI) *m*/*z:* 637.5 $(M+H)^+$. $^1H$ NMR (400MHz, $CD_3OD$) δ 8.97 (s, 1H), 8.74 (d, *J*=5.3 Hz, 1H), 7.74 - 7.67 (m, 2H), 7.56 - 7.49 (m, 6H), 6.40 (s, 1H), 6.03 (dd, *J*=12.7, 4.3 Hz, 1H), 4.92 - 4.82 (m, 2H), 4.05 (s, 3H), 2.76 - 2.67 (m, 1H), 2.39 - 2.28 (m, 1H), 2.14 - 1.99 (m, 2H), 1.67 - 1.43 (m, 2H), 1.02 (d, *J*=7.0 Hz, 3H), 0.79 - 0.64 (m, 1H). Analytical HPLC (Method A): RT = 8.26 min, 99.5% purity; Factor XIa Ki = 52 nM, Plasma Kallikrein Ki = 5,500 nM.

Example 257

Preparation of (9R,13S)-13-{4-[5-chloro-2-(5-methyl-1H-imidazol-1-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate

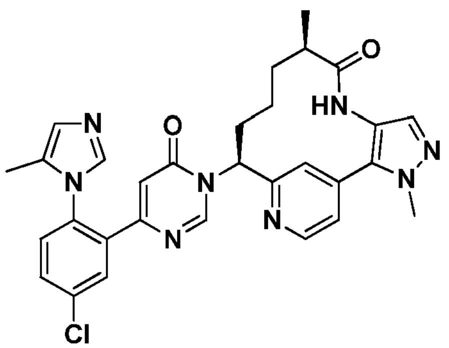

(9*R*,13*S*)-13-{4-[5-Chloro-2-(5-methyl-1H-imidazol-1-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (2.3 mg, 9% yield) was prepared in a similar manner as the procedure described in Example 231, by replacing 1H-imidazole with 4-methyl-1H-imidazole (10.68 mg, 0.130 mmol). MS(ESI) *m*/*z:* 569.20 $(M+H)^+$. $^1H$ NMR (500MHz, DMSO-$d_6$) δ 9.22 (s, 1H), 8.89

(br. s., 1H), 8.78 (s, 1H), 8.67 (d, *J*=4.9 Hz, 1H), 8.01 (d, *J*=2.4 Hz, 1H), 7.86 (dd, *J*=8.5, 2.1 Hz, 1H), 7.75 (d, *J*=8.5 Hz, 1H), 7.66 (s, 1H), 7.59 (d, *J*=4.9 Hz, 1H), 7.50 - 7.40 (m, 2H), 6.41 (br. s., 1H), 5.86 (d, *J*=10.4 Hz, 1H), 4.01 (s, 3H), 2.68 - 2.60 (m, 1H), 2.32 - 2.22 (m, 1H), 2.16 - 2.05 (m, 1H), 2.01 (s, 3H), 1.88 - 1.76 (m, 1H), 1.52 - 1.27 (m, 2H), 0.88 (d, *J*=6.7 Hz, 3H), 0.50 - 0.37 (m, 1H). Analytical HPLC (Method B): RT = 1.49 min, 100% purity; Factor XIa Ki = 7,500 nM.

Example 261

Preparation of (9R,13S)-13-[4-(2-amino-5-chlorophenyl)-6-oxo-1,6-dihydropyrimidin-1-yl]-3-(difluoromethyl)-9-methyl-3,4,7,15-tetraazatricyclo[12.3.1.$0^{2,6}$]octadeca-1(18),2(6),4,14,16-pentaen-8-one

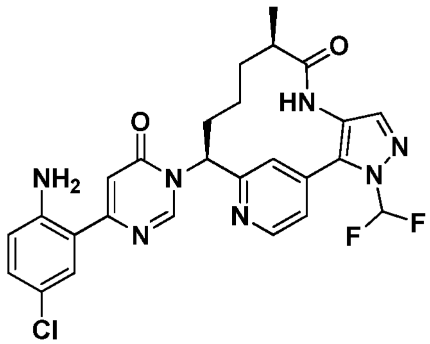

N-(4-Chloro-2-{1-[(9R,13S)-3-(difluoromethyl)-9-methyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)-2,2,2-trifluoroacetamide, prepared as described in Example 244, (0.2 g, 0.31 mmol) was dissolved in 1.25 M HCl in MeOH (5 ml, 6.25 mmol). The reaction was heated to 75 °C for 1 h, then cooled to rt and concentrated to dryness. The product was purified by recrystallizing from $CH_3CN$-$H_2O$ to give (9*R*,13*S*)-13-[4-(2-amino-5-chlorophenyl)-6-oxo-1,6-dihydropyrimidin-1-yl]-3-(difluoromethyl)-9-methyl-3,4,7,15-tetraazatricyclo[12.3.1.$0^{2,6}$]octadeca-1(18),2(6),4,14,16-pentaen-8-one (160 mg, 89%) as a yellow solid. MS(ESI) *m/z:* 540.5 $(M+H)^+$. $^1H$ NMR (500MHz, DMSO-$d_6$) $\delta$ 9.40 (s, 1 H), 9.00 (s, 1 H), 8.75 (d, *J*=5.09 Hz, 1 H), 7.96 (t, *J*=57.75 Hz, 1 H), 7.90 (s, 1 H), 7.73 (s, 1 H), 7.46 (d, *J*=2.49 Hz, 1 H), 7.43 (dd, *J*=4.92, 0.85 Hz, 1 H), 7.14 (dd, *J*=8.80, 2.51 Hz, 1 H), 6.76 (d, *J*=8.73 Hz, 1 H), 6.70 (s, 1 H), 6.43 (s, 2 H), 5.95 (dd, 12.17, 3.13 Hz, 1 H), 2.67 (m, 1 H), 2.34 (tm, *J*=12.83 Hz, 1 H), 2.08 (tm, *J*=13.09 Hz, 1 H), 1.93 (m, 1 H), 1.48 (m, 1 H), 1.37 (m, 1 H), 0.89 (d, *J*=6.87 Hz, 3 H), 0.39 (br-s, 1 H). Analytical HPLC (Method A): RT = 7.75 min, purity = 94%; Factor XIa Ki = 170 nM, Plasma Kallikrein Ki = 5,700 nM.

Example 262

Preparation of (9R,13S)-13-(4-{3-chloro-6-[1-(difluoromethyl)-1H-pyrazol-4-yl]-2-fluorophenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one

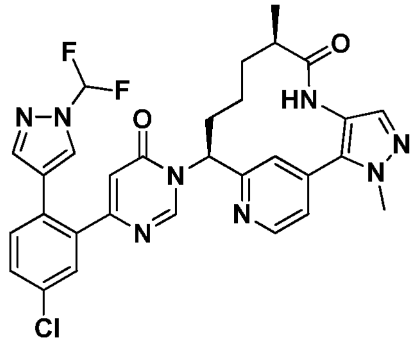

262A. Preparation of 6-(3-chloro-6-(1-(difluoromethyl)-1H-pyrazol-4-yl)-2-fluorophenyl)pyrimidin-4-ol

6-(3-Chloro-6-(1-(difluoromethyl)-1H-pyrazol-4-yl)-2-fluorophenyl)pyrimidin-4-ol (0.032 g, 85% yield) was prepared in a similar manner as the procedures described in Example 140A and 140B, by replacing 4-(2-bromo-5-chlorophenyl)-6-methoxypyrimidine (0.08 g, 0.267 mmol) with 4-(6-bromo-3-chloro-2-fluorophenyl)-6-methoxypyrimidine (0.1 g, 0.315 mmol). MS(ESI) *m/z:* 341.4 $(M+H)^+$. $^1H$ NMR (500MHz, $CD_3OD$) δ 8.35 (s, 1H), 8.06 (s, 1H), 7.67 - 7.60 (m, 2H), 7.45 (t, *J*=59.4 Hz, 1H), 7.40 (dd, *J*=8.3, 1.4 Hz, 1H), 6.52 (s, 1H).

262B. Preparation of (9*R*,13*S*)-13-{3-chloro-6-[1-(difluoromethyl)-1H-pyrazol-4-yl]-2-fluorophenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-onetrifluoroacetate

(9*R*,13*S*)-13-(4-{3-Chloro-6-[1-(difluoromethyl)-1H-pyrazol-4-yl]-2-fluorophenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one trifluoroacetate (11.7 mg, 36% yield) was prepared in a similar manner as the procedure described in Example 56, by replacing 6-{5-chloro-2-[4-(trifluoromethyl)-1H-1,2,3-triazol-1-yl]phenyl}pyrimidin-4-ol (22.8 mg, 0.067 mmol) with 6-(3-chloro-6-(1-(difluoromethyl)-1H-pyrazol-4-yl)-2-fluorophenyl)pyrimidin-4-ol (14.79 mg, 0.043 mmol). MS(ESI) *m/z:* 623.6 $(M+H)^+$. $^1H$ NMR (500MHz, $CD_3OD$) δ 8.99 (s, 1H), 8.75 (d, *J*=5.2 Hz, 1H), 8.00 (s, 1H), 7.73 (s, 1H), 7.64 - 7.58 (m, 2H), 7.55 - 7.28 (m, 4H), 6.51 (s, 1H), 6.04 (dd, *J*=12.7, 3.9 Hz, 1H), 4.05 (s, 3H), 2.75 - 2.67 (m, 1H), 2.36 (tt, *J*=12.7, 4.3 Hz, 1H), 2.13 - 2.02 (m, 2H), 1.66-1.45 (m, 2H), 1.02 (d, *J*=7.2 Hz, 3H), 0.83 - 0.66 (m, 1H). $^{19}F$ NMR (471MHz, $CD_3OD$) δ -77.75 (s), -96.25 (s), -117.96 (s). Analytical HPLC (Method A): RT = 8.18 min, 98.0% purity; Factor XIa Ki = 4.5 nM, Plasma Kallikrein Ki = 340 nM.

Example 265

Preparation of (14S)-14-(4-{5-chloro-2-[4-(trifluoromethyl)-1H-1,2,3-triazol-1-yl] phenyl}-6-oxo-1,6-dihydropyrimidin-1-yl)-10-methyl-5,8,16-tiiazatiicyclo[13.3.1.$0^{2,7}$] nonadeca-1(19),2(7),3,5,15,17-hexaen-9-one trifluoroacetate

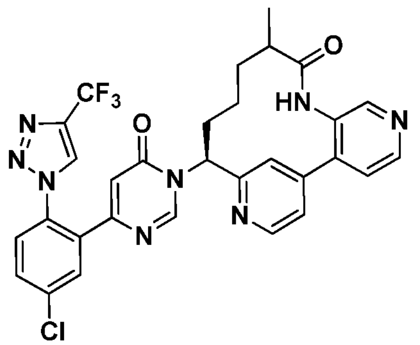

265A. Preparation of (S)-(2-(1-((*tert*-butoxycarbonyl)amino)but-3-en-1-yl)pyridin-4-yl)boronic acid, trifluoroacetate

To a solution of 5,5,5′,5′-tetramethyl-2,2′-bi(1,3,2-dioxaborinane) (1.198 g, 5.30 mmol) and (S)-*tert*-butyl (1-(4-chloropyridin-2-yl)but-3-en-1-yl)carbamate (1.0 g, 3.54 mmol), prepared as described in Intermediate 23, in DMSO (10 mL) was added KOAc (1.041 g, 10.61 mmol) under Ar. $PdCl_2(dppf)\cdot CH_2Cl_2$ adduct (0.289 g, 0.354 mmol) and the mixture was purged with Ar for an additional 10 min then stirred at 85 °C. After 12 h, the reaction mixture was diluted with EtOAc and washed with water. The aqueous layer was extracted with EtOAc. The combined organic layers were washed with brine, dried over $Na_2SO_4$, filtered, and concentrated. The dark oil purified by reverse phase chromatography to give, after concentration and lyophilization, (S)-(2-(1-((*tert*-butoxycarbonyl)amino)but-3-en-1-yl)pyridin-4-yl)boronic acid trifluoroacetate (1.05 g, 2.59 mmol, 73.1% yield) as a white solid. MS(ESI) *m/z:* 293.2 $(M+H)^+$.

265B. Preparation of (S)-*tert*-butyl (1-(3′-amino-[4,4′-bipyridin]-2-yl)but-3-en-1-yl) carbamate.

To a solution of (S)-(2-(1-((*tert*-butoxycarbonyl)amino)but-3-en-1-yl)pyridin-4-yl)boronic acid, trifluoroacetate (1.0 g, 2.462 mmol) and 4-bromopyridin-3-amine (0.511 g, 2.95 mmol) in dioxane (17 mL) was added 2 M aq $Na_2CO_3$ (4.92 mL, 9.85 mmol). The mixture was purged with a stream of Ar for 5 min. $Pd(PPh_3)_4$ (0.285 g, 0.246 mmol) was

added and the reaction was irradiated at 120 °C for 1 h. The reaction was quenched with water (40 mL) and extracted with EtOAc (2x). The combined organic layers were washed with brine, dried over $MgSO_4$, filtered, and concentrated. The crude material was purified by normal phase column chromatography eluting with a gradient of DCM/MeOH to give (S)-*tert*-butyl (1-(3'-amino-[4,4'-bipyridin]-2-yl)but-3-en-1-yl)carbamate (0.736 g, 88% yield) as a brown oil. MS(ESI) *m/z:* 341.2 $(M+H)^+$. $^1H$ NMR (500MHz, $CDCl_3$) $\delta$ 8.68 (d, J=4.7 Hz, 1H), 8.19 (s, 1H), 8.10 (d, J=5.0 Hz, 1H), 7.35 (s, 1H), 7.29 (dd, J=5.1, 1.5 Hz, 1H), 7.02 (d, J=5.0 Hz, 1H), 5.79 - 5.68 (m, 1H), 5.62 - 5.52 (m, 1H), 5.11 - 5.04 (m, 2H), 4.90 - 4.80 (m, 1H), 3.83 (br. s., 2H), 2.64 (t, J=6.7 Hz, 2H), 1.45 (s, 9H).

265C. Preparation of *tert*-butyl ((*S*)-1-(3'-((R)-2-methylbut-3-enamido)-[4,4'-bipyridin]-2-yl)but-3-en-1-yl)carbamate

To a solution of (S)-*tert*-butyl (1-(3'-amino-[4,4'-bipyridin]-2-yl)but-3-en-1-yl) carbamate (736 mg, 2.16 mmol) in EtOAc (21.6 mL) was added (R)-2-methylbut-3-enoic acid (303 mg, 3.03 mmol). After cooling to 0 °C, pyridine (0.525 mL, 6.49 mmol) was added followed by T3P® / 50% EtOAc (2.57 mL, 4.32 mmol) dropwise. The reaction was slowly warmed to rt overnight. The reaction mixture was diluted with water and extracted with EtOAc (2x). The combined organic layers were washed with brine, dried over $MgSO_4$, filtered, and concentrated. The crude material was purified by normal phase column chromatography eluting with a gradient of DCM/MeOH to give of *tert*-butyl((S)-1-(3'-((R)-2-methylbut-3-enamido)-[4,4'-bipyridin]-2-yl)but-3-en-1-yl) carbamate (815 mg, 89%). MS(ESI) *m/z:* 423.2 $(M+H)^+$. $^1H$ NMR (500MHz, $CDCl_3$) $\delta$ 9.45 (s, 1H), 8.70 (d, J=5.0 Hz, 1H), 8.49 (d, J=5.0 Hz, 1H), 7.24 (br. s., 1H), 7.21 (s, 1H), 7.18 -7.14 (m, 2H), 5.80 (ddd, J=17.1, 10.1, 8.3 Hz, 1H), 5.74 - 5.66 (m, 1H), 5.54-5.48 (m, 1H), 5.15 - 5.05 (m, 4H), 4.92 - 4.85 (m, 1H), 3.08 (quin, J=7.2 Hz, 1H), 2.69-2.61 (m, 2H), 1.45 (s, 9H), 1.31 - 1.28 (m, 3H).

265D. Preparation of tert-butyl N-[(11E,14S)-10-methyl-9-oxo-5,8,16-triazatricyclo [13.3.1.$0^{2,7}$]nonadeca-1(19),2(7),3,5,11,15,17-heptaen-14-yl]carbamate

To a solution of *tert*-butyl((S)-1-(3'-((R)-2-methylbut-3-enamido)-[4,4'-bipyridin]-2-yl)but-3-en-1-yl)carbamate (400 mg, 0.947 mmol) in freshly opened and degassed DCM (500 mL) was added *p*-TsOR·$H_2O$ (378 mg, 1.988 mmol). A stream of $N_2$ was bubbled for 10 min before heating the solution at 40 °C under a nitrogen atmosphere. After 1 h, Second Generation Grubbs Catalyst (201 mg, 0.237 mmol) dissolved in degassed DCM (20 ml) was added to the reaction mixture dropwise and heating continued at 40 °C overnight. The reaction mixture was quenched with 1.5 M $K_2HPO_3$ (30 mL) followed by separation of the organic phase and concentration. The crude residue was purified by reverse phase chromatography and concentration of product fractions. The residue was dissolved in MeOH, neutralized by passing through $NaHCO_3$ resin cartridges (2x) and concentrating the filtrate to afford *tert*-butyl N-[(11E,14S)-10-methyl-9-oxo-5,8,16-triazatricyclo[13.3.1.$0^{2,7}$]nonadeca-1(19),2(7),3,5,11,15,17-heptaen-14-yl]carbamate (24.6 mg, 6.59% yield) as brown film. MS(ESI) *m/z:* 395 $(M+H)^+$.

265E. Preparation of *tert*-butyl N-[(14S)-10-methyl-9-oxo-5,8,16-triazatricyclo [13.3.1.$0^{2,7}$]nonadeca-1(19),2(7),3,5,15,17-hexaen-14-yl]carbamate

$PtO_2$ (0.018 g, 0.079 mmol) was added to a stirring solution of *tert*-butyl N-[(11E,14S)-10-methyl-9-oxo-5,8,16-triazatricyclo[13.3.1.$0^{2,7}$]nonadeca-1(19),2(7),3,5,11,15,17-heptaen-14-yl]carbamate (0.031 g, 0.079 mmol) in EtOH (3 mL) and subjected to a $H_2$ atmosphere (55 psi) for 3 h. The catalyst was filtered off through a plug of CELITE® and filtrate concentrated to give tert-butyl N-[(14S)-10-methyl-9-oxo-5,8,16-triazatricyclo[13.3.1.$0^{2,7}$]nonadeca-1(19),2(7),3,5,15,17-hexaen-14-yl]carbamate as a brown film (yield assumed quantitative). MS(ESI) *m/z:* 397 $(M+H)^+$.

265F. Preparation of (14S)-14-amino-10-methyl-5,8,16-triazatricyclo[13.3.1.$0^{2,7}$] nonadeca-1(19),2(7),3,5,15,17-hexaen-9-one

TFA (0.18 mL, 2.35 mmol) was added to a stirring solution of *tert*-butyl N-[(14S)-10-methyl-9-oxo-5,8,16-triazatricyclo[13.3.1.$0^{2,7}$]nonadeca-1(19),2(7),3,5,15,17-hexaen-14-yl]carbamate (31 mg, 0.078 mmol). After 6 h, the reaction mixture was concentrated and the residue placed under vacuum overnight. The residue was dissolved in MeOH, passed a $NaHCO_3$ resin cartridge, and concentrated to give (14S)-14-amino-10-methyl-5,8,16-triazatricyclo[13.3.1.$0^{2,7}$]nonadeca-1(19),2(7),3,5,15,17-hexaen-9-one as a brown film. The material was carried forward to the next reaction without further purification. MS(ESI) *m/z:* 297.3 $(M+H)^+$.

265G. Preparation of (14S)-14-(4-{5-chloro-2-[4-(trifluoromethyl)-1H-1,2,3-triazol-1-yl] phenyl}-6-oxo-1,6-dihydropyrimidin-1-yl)-10-methyl-5,8,16-triazatricyclo[13.3.1.$0^{2,7}$] nonadeca-1(19), 2(7),3,5,15,17-hexaen-9-onetrifluoracetate

(14S)-14-(4-{5-Chloro-2-[4-(trifluoromethyl)-1H-1,2,3-triazol-1-yl]phenyl}-6-oxo-1,6-dihydropyrimidin-1-yl)-10-methyl-5,8,16-triazatricyclo[13.3.1.0$^{2,7}$]nonadeca-1(19),2(7),3,5,15,17-hexaen-9-one, trifluoroacetate was prepared (10.4 mg, 16.5%) in a similar manner as Example 56 using (14S)-14-amino-10-methyl-5,8,16-triazatricyclo [13.3.1.0$^{2,7}$]nonadeca-1(19),2(7),3,5,15,17-hexaen-9-one and 6-{5-chloro-2-[4-(trifluoromethyl)-1H-1,2,3-triazol-1-yl]phenyl}pyrimidin-4-ol, prepared as described in Intermediate 15. The compound exists as an apparent diastereomeric mixture according to NMR data. MS(ESI) *m/z:* 621.2 (M+H)$^+$. $^1$H NMR (400MHz, $CD_3OD$) δ 8.88 - 8.81 (m, 4H), 8.75 - 8.69 (m, 3H), 8.07 (br. s., 1H), 8.03 (br. s., 1H), 7.96 (d, J=2.2 Hz, 1H), 7.95 - 7.93 (m, 1H), 7.84 - 7.82 (m, 1H), 7.80 - 7.79 (m, 1H), 7.76 - 7.73 (m, 2H), 7.59 (dd, J=5.0, 1.7 Hz, 1H), 7.47 (dd, J=5.1, 1.8 Hz, 1H), 7.27 (s, 1H), 6.49 - 6.45 (m, 2H), 6.09 (dd, J=12.4, 4.7 Hz, 1H), 5.94 - 5.83 (m, 2H), 4.61 (dd, J=15.2, 9.7 Hz, 1H), 2.94-2.86 (m, 1H), 2.79 - 2.70 (m, 1H), 2.26 (s, 1H), 2.03 (d, J=13.0 Hz, 1H), 1.35 - 1.28 (m, 1H), 1.15 (d, J=6.6 Hz, 3H), 1.00 (d, J=6.8 Hz, 2H). Analytical HPLC (Method A): RT = 5.22 min, purity = 98%.

Example 271

Preparation of (9R,13S)-13-{4-[5-chloro-2-(4-methyl-1H-imidazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

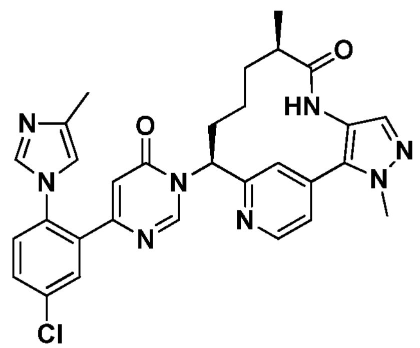

(9*R*,13*S*)-13-{4-[5-Chloro-2-(4-methyl-1H-imidazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (11.7 mg, 45% yield) was a major product isolated from the preparation of (9R,13S)-13-{4-[5-chloro-2-(5-methyl-1H-imidazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate, Example 257. MS(ESI) *m*/*z:* 569.20 (M+H)$^+$. $^1$H NMR (500MHz, DMSO-$d_6$) δ 9.23 (s, 1H), 9.14 (br. s., 1H), 8.77 (s, 1H), 8.68 (d, *J*=4.9 Hz, 1H), 7.94 (d, *J*=2.1 Hz, 1H), 7.85 (dd, *J*=8.4, 2.3 Hz, 1H), 7.76 (d, *J*=8.5 Hz, 1H), 7.67 (s, 1H), 7.59 (d, *J*=5.2 Hz, 1H), 7.52 - 7.47 (m, 2H), 6.60 (s, 1H), 5.90 (d, *J*=9.8 Hz, 1H), 4.01 (s, 3H), 2.69 - 2.61 (m, 1H), 2.35 - 2.24 (m, 4H), 2.16 - 2.06 (m, 1H), 1.88 - 1.78 (m, 1H), 1.52 - 1.42 (m, 1H), 1.38-1.28 (m, 1H), 0.89 (d, *J*=7.0 Hz, 3H), 0.53 - 0.38 (m, 1H). Analytical HPLC (Method C): RT = 1.14 min, 100% purity; Factor XIa Ki = 90 nM, Plasma Kallikrein Ki = 4,600 nM.

Example 274

Preparation of (9R,13S)-13-{4-[5-chloro-2-(1,3-oxazol-2-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

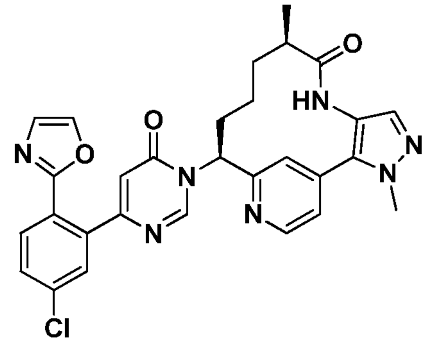

To a degassed dioxane (1 ml) solution of (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.$0^{2,6}$] octadeca-1(18),2(6),4,14,16-pentaen-8-one (20 mg, 0.033 mmol), prepared as described in Example 211, was added 2-(tributylstannyl)oxazole (11.65 mg, 0.033 mmol), followed by addition of $Pd(Ph_3P)_4$ (3.76 mg, 3.25 μmol). The reaction was stirred at 90 °C for 2 h, then cooled to rt and concentrated. Purification by reverse phase chromatography afforded (9*R*,13*S*)-13-{4-[5-chloro-2-(1,3-oxazol-2-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (2.38 mg, 11% yield) as a white solid. MS(ESI) *m/z:* 556.4 $(M+H)^+$. $^1H$ NMR (400MHz, $CD_3OD$) δ 8.89 (s, 1H), 8.74 (d, *J*=5.1 Hz, 1H), 7.92 (d, *J*=8.4 Hz, 1H), 7.87 (s, 1H), 7.71 (s, 1H), 7.69 (d, *J*=2.2 Hz, 1H), 7.65 (dd, *J*=8.4, 2.2 Hz, 1H), 7.52 (dd, *J*=5.1, 1.5 Hz, 1H), 7.49 (s, 1H), 7.23 (s, 1H), 6.50 (s, 1H), 6.05 (dd, *J*=12.7, 4.1 Hz, 1H), 4.05 (s, 3H), 2.77 - 2.67 (m, 1H), 2.38 - 2.27 (m, 1H), 2.15 - 1.98 (m, 2H), 1.68 - 1.42 (m, 2H), 1.01 (d, *J*=7.0 Hz, 3H), 0.77 - 0.62 (m, 1H). Analytical HPLC (Method A): RT = 7.13 min, 99.0% purity; Factor XIa Ki = 100 nM, Plasma Kallikrein Ki = 4,500 nM.

Example 275

Preparation of (9R,13S)-13-(4-{5-chloro-2-[(pyrazin-2-yl)amino]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one

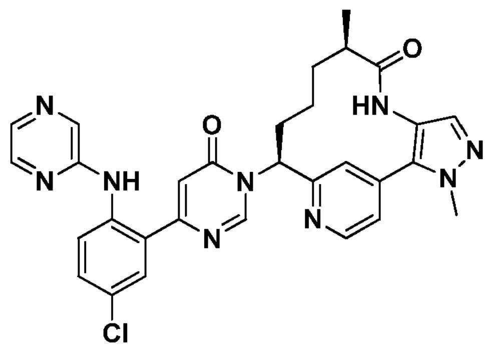

A sealed microwave vial containing (9*R*,13*S*)-13-[4-(2-amino-5-chlorophenyl)-6-oxo-1,6-dihydropyrimidin-1-yl]-3,9-dimethyl-3,4,7,15-tetraazatricyclo[12.3.1.$0^{2,6}$] octadeca-1(18),2(6),4,14,16-pentaen-8-one hydrochloride (0.01 g, 0.017 mmol), prepared as described in Example 313, 2-bromopyrazine (5.51 mg, 0.035 mmol) and EtOH (1 ml) was heated in a microwave at 150 °C for 30 min, cooled to rt, and concentrated. To the residue were added $Cs_2CO_3$ (0.030 g, 0.093 mmol), $Pd(OAc)_2$ (1.05 mg, 4.66 μmol), Xantphos (5.39 mg, 9.31 μmol), and 2-bromopyrazine (5.51 mg, 0.035 mmol), followed by dioxane (0.931 ml). The reaction mixture was degassed with Ar for 10 min. The vial was sealed and heated at 85 °C. After 4 h, the reaction was cooled to rt and concentrated. Purification by reverse phase chromatography afforded (9*R*,13*S*)-13-(4-{5-chloro-2-[(pyrazin-2-yl)amino]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one trifluoroacetate (2.95 mg, 20% yield) as a yellow solid. MS(ESI) *m/z:* 582.5 $(M+H)^+$. $^1H$ NMR (400MHz, $CD_3OD$) δ 9.09 (s, 1H), 8.73 (d, *J*=5.1 Hz, 1H), 8.23 - 8.19 (m, 2H), 8.08 (dd, J=2.8, 1.4 Hz, 1H), 7.89 (d, *J*=2.6 Hz, 1H), 7.73 (s, 1H), 7.67 (d, *J*=2.6 Hz, 1H), 7.53 (dd, *J*=5.1, 1.5 Hz, 1H), 7.50 (s, 1H), 7.43 (dd, *J*=8.9, 2.5 Hz, 1H), 6.77 (s, 1H), 6.02 (dd, *J*=12.7, 4.3 Hz, 1H), 4.05 (s, 3H), 2.76 - 2.67 (m, 1H), 2.43 - 2.32 (m, 1H), 2.15 - 2.01 (m, 2H), 1.68 - 1.44 (m, 2H), 1.02 (d, *J*=6.8 Hz, 3H), 0.81 - 0.65 (m, 1H). Analytical HPLC (Method A): RT = 7.06 min, 94.0% purity; Factor XIa Ki = 560 nM.

Example 277

Preparation of ethyl 1-(4-chloro-2-{1-[(9R,13S)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-3-methyl-1H-pyrazole-4-carboxylate

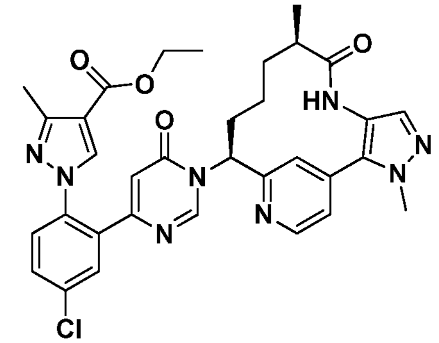

To a suspension of ethyl 3-methyl-1H-pyrazole-4-carboxylate (3.51 mg, 0.023 mmol), $K_3PO_4$ (9.79 mg, 0.046 mmol) and (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.0$^{2,6}$] octadeca-1(18),2(6),4,14,16-pentaen-8-one (14 mg, 0.023 mmol) in dioxane (0.24 mL) was added (1R,2R)-N1,N2-dimethylcyclohexane-1,2-diamine (1.619 mg, 0.011 mmol). The mixture was purged with Ar, CuI (0.434 mg, 2.277 $\mu$mol) was added, and the vial was sealed. The reaction was heated at 80 °C and stirred overnight. The solution was concentrated and the residue purified by reverse phase chromatography to give ethyl 1-(4-chloro-2-{1-[(9R,13S)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-3-methyl-1H-pyrazole-4-carboxylate trifluoroacetate (3.21 mg, 17% yield) as a white solid. MS(ESI) *m/z:* 641.2 $(M+H)^+$. $^1$H NMR (400MHz, $CD_3OD$) $\delta$ 8.86 (s, 1H), 8.71 (d, *J*=5.1 Hz, 1H), 8.20 (s, 1H), 7.84 (d, *J*=2.2 Hz, 1H), 7.68 (s, 1H), 7.67 - 7.61 (m, 1H), 7.60 - 7.54 (m, 1H), 7.53 - 7.47 (m, 2H), 6.20 (s, 1H), 6.02 - 5.95 (m, 1H), 4.30 - 4.21 (m, 2H), 4.07 - 4.01 (m, 3H), 2.70 (td, *J*=6.7, 3.2 Hz, 1H), 2.36 (s, 3H), 2.33 - 2.24 (m, 1H), 2.13 - 1.95 (m, 2H), 1.65 - 1.53 (m, 1H), 1.51 - 1.40 (m, 1H), 1.34 - 1.26 (m, 3H), 1.00 (d, *J*=7.0 Hz, 3H), 0.69 (m, 1H). Analytical HPLC (Method A) : RT =7.46 min, 94% purity; Factor XIa Ki = 170 nM, Plasma Kallikrein Ki = 2,700 nM.

Example 278

Preparation of (9R,13S)-13-{4-[5-chloro-2-(3,4-dimethyl-1H-pyrazol-1-yl)phenyl]-6-oxo-1,6-dihydropyrimidin-1-yl}-3,9-dimethyl-3,4,7,15-tetraazatricyclo[12.3.1.0$^{2,6}$] octadeca-1(18),2(6),4,14,16-pentaen-8-one

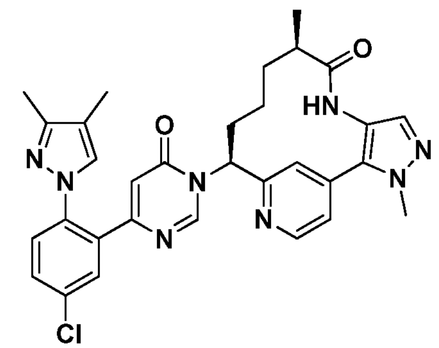

(9R,13S)-13-{4-[5-Chloro-2-(3,4-dimethyl-1H-pyrazol-1-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (0.85mg, 5% yield) was prepared in a similar manner as the procedure described in Example 277, by replacing ethyl 3-methyl-1H-pyrazole-4-carboxylate with 3,4-dimethyl-1H-pyrazole (2.189 mg, 0.023 mmol). MS(ESI) *m/z:* 583.2 $(M+H)^+$. $^1$H NMR (400MHz, $CD_3OD$) $\delta$ 8.91 (s, 1H), 8.72 (d, *J*=5.1 Hz, 1H), 7.86 (d, *J*=2.4 Hz, 1H), 7.68 (s, 1H), 7.60 (dd, *J*=8.6, 2.4 Hz, 1H), 7.51 (dd, *J*=5.1, 1.5 Hz, 1H), 7.49 - 7.46 (m, 2H), 7.39 (s, 1H), 6.02 - 5.94 (m, 2H), 4.04 (s, 3H), 2.71 (d, *J*=3.1 Hz, 1H), 2.37

- 2.24 (m, 1H), 2.14 (s, 3H), 2.00 (s, 3H), 1.93 (s, 1H), 1.66 - 1.54 (m, 1H), 1.48 (m, 1H), 1.01 (d, *J*=6.8 Hz, 3H), 0.68 (m, 1H). Analytical HPLC (Method A) : RT = 6.78 min, 92% purity; Factor XIa Ki = 540 nM.

Example 279

Preparation of ethyl 1-(4-chloro-2-{1-[(9R,13S)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-pyrazole-4-carboxylate

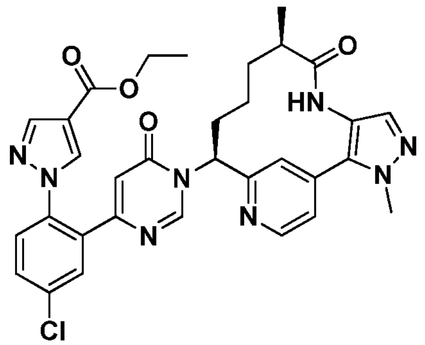

Ethyl 1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-pyrazole-4-carboxylate trifluoroacetate (9.9 mg, 56% yield) was prepared in a similar manner as the procedure described in Example 277, by replacing ethyl 3-methyl-1H-pyrazole-4-carboxylate with ethyl 1H-pyrazole-4-carboxylate (3.19 mg, 0.023 mmol). MS(ESI) *m/z:* 627.1 $(M+H)^+$. $^1H$ NMR (400MHz, methanol-$d_4$) δ 8.86 (s, 1H), 8.72 (d, *J*=5.3 Hz, 1H), 8.34 (s, 1H), 7.96 (s, 1H), 7.85 (d, *J*=2.2 Hz, 1H), 7.69 (s, 1H), 7.68 - 7.64 (m, 1H), 7.61 - 7.57 (m, 1H), 7.53 (dd, *J*=5.2, 1.7 Hz, 1H), 7.49 (s, 1H), 6.18 (s, 1H), 5.98 (dd, *J*=12.7, 4.3 Hz, 1H), 4.28 (q, *J*=7.1 Hz, 2H), 4.04 (s, 3H), 2.70 (td, *J*=6.7, 3.3 Hz, 1H), 2.30 (tt, *J*=12.7, 4.4 Hz, 1H), 2.12 - 1.94 (m, 2H), 1.66 - 1.53 (m, 1H), 1.46 (ddd, *J*=15.0, 9.8, 5.5 Hz, 1H), 1.32 (t, *J*=7.2 Hz, 3H), 1.00 (d, *J*=6.8 Hz, 3H), 0.69 (m, 1H). Analytical HPLC (Method A) : RT = 7.06 min, 99% purity; Factor XIa Ki = 2.2 nM, Plasma Kallikrein Ki = 960 nM.

Example 280

Preparation of (9R,13S)-13-[4-(5-chloro-2-hydroxyphenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one

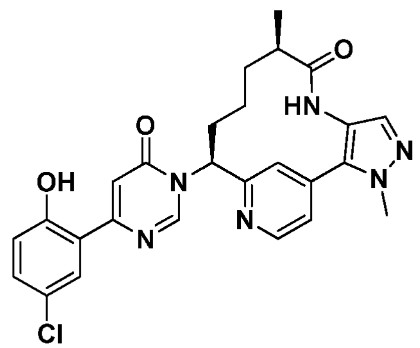

The mixture of 1H-imidazole (2.21 mg, 0.033 mmol), $Pd(OAc)_2$ (0.73 mg, 3.25 μmol) and CuI (0.012 g, 0.065 mmol) in DMF (1.63 ml) was purged with Ar (3x), and then (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one (0.02 g, 0.033 mmol), prepared as described in Example 211, was added. The reaction was sealed and heated at 140 °C. After 5 h, the reaction was cooled to rt. The solution was concentrated and the residue was purified by reverse phase chromatography afforded (9*R*,13*S*)-13-[4-(5-chloro-2-hydroxyphenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate (1.76 mg, 9% yield) as a yellow solid. MS(ESI) *m/z:* 505.6 (M+H)$^+$. $^1$H NMR (400MHz, $CD_3OD$) δ 9.11 (s, 1H), 8.74 (d, *J*=5.1 Hz, 1H), 7.84 (d, *J*=2.6 Hz, 1H), 7.72 (s, 1H), 7.52 (dd, *J*=5.1, 1.5 Hz, 1H), 7.50 (s, 1H), 7.30 (dd, *J*=8.8, 2.6 Hz, 1H), 7.07 (s, 1H), 6.90 (d, *J*=8.8 Hz, 1H), 6.06 (dd, *J*=12.9, 4.5 Hz, 1H), 4.05 (s, 3H), 2.78 - 2.68 (m, 1H), 2.35 (tt, *J*=12.8, 4.3 Hz, 1H), 2.16 - 2.01 (m, 2H), 1.68 - 1.45 (m, 2H), 1.01 (d, *J*=7.0 Hz, 3H), 0.76 - 0.61 (m, 1H). Analytical HPLC (Method A): RT = 8.47 min, 100% purity; Factor XIa Ki = 57 nM, Plasma Kallikrein Ki = 180 nM.

Example 281

Preparation of (9R,13S)-13-(4-{5-chloro-2-[4-(trifluoromethyl)-1H-imidazol-1-yl] phenyl}-6-oxo-1,6-dihydropyrimidin-1-yl)-3,9-dimethyl-3,4,7,15-tetraazatricyclo [12.3.1.0$^{2,6}$]octadeca-1(18),2(6),4,14,16-pentaen-8-one

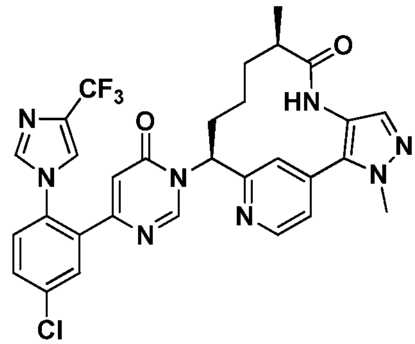

(9*R*,13*S*)-13-(4-{5-Chloro-2-[4-(trifluoromethyl)-1H-imidazol-1-yljphenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one trifluoroacetate (4 mg, 15% yield) was prepared in a similar manner as the procedure described in Example 231, by replacing 1H-imidazole with 4-(trifluoromethyl)-1H-imidazole (0.018 g, 0.130 mmol). MS(ESI) *m/z:* 623.25 (M+H)$^+$. $^1$H NMR (500MHz, $CD_3OD$) δ 8.84 (s, 1H), 8.70 (d, *J*=5.2 Hz, 1H), 7.86 (s, 1H), 7.83 (d, *J*=2.5 Hz, 1H), 7.74 (s, 1H), 7.71 - 7.66 (m, 2H), 7.58 (d, *J*=8.5 Hz, 1H), 7.52 - 7.47 (m, 2H), 6.40 (s, 1H), 5.99 (dd, *J*=12.5, 3.4 Hz, 1H), 4.04 (s, 3H), 2.70 (dt, *J*=6.6, 3.3 Hz, 1H), 2.33 - 2.23 (m, 1H), 2.12 - 1.92 (m, 2H), 1.65 - 1.55 (m, 1H), 1.52 - 1.41 (m, 1H), 1.01 (d, *J*=6.9 Hz, 3H), 0.76 - 0.60 (m, 1H). Analytical HPLC (Method C): RT = 1.58 min, 100% purity; Factor XIa Ki = 73 nM, Plasma Kallikrein Ki = 4,700 nM.

Example 284

Preparation of methyl 1-(4-chloro-2-{1-[(9R,13S)-3,9-dimethyl-8-oxo-3,4,7,15-tetraazatricyclo[12.3.1.0$^{2,5}$]octadeca-1(18),2(6),4,14,16-pentaen-13-yl]-6-oxo-1,6-dihydropyrimidin-4-yl}phenyl)-1H-imidazole-4-carboxylate

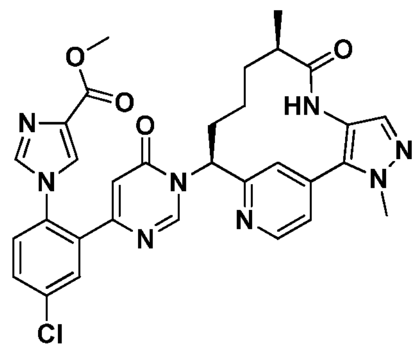

Methyl 1-(4-chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylate trifluoroacetate (10 mg, 24% yield) was prepared in a similar manner as the procedure described in Example 231, by replacing 1H-imidazole with 1H-imidazole-4-carboxylate hydrochloride (0.025 g, 0.195 mmol). MS(ESI) *m/z:* 613.5 (M+H)$^+$. $^1$H NMR

(400MHz, $CD_3OD$) δ 8.80 (s, 1H), 8.68 (d, *J*=5.3 Hz, 1H), 8.29 (s, 1H), 8.06 (s, 1H), 7.87 (d, *J*=2.2 Hz, 1H), 7.72 (dd, *J*=8.6, 2.4 Hz, 1H), 7.68 (s, 1H), 7.62 (d, *J*=8.4 Hz, 1H), 7.51 (dd, *J*=5.1, 1.5 Hz, 1H), 7.49 (s, 1H), 6.47 (s, 1H), 5.96 (dd, *J*=12.4, 3.9 Hz, 1H), 4.04 (s, 3H), 3.87 (s, 3H), 2.75 - 2.65 (m, 1H), 2.33-2.22 (m, 1H), 2.11 - 1.92 (m, 2H), 1.64 - 1.39 (m, 2H), 1.00 (d, *J*=6.8 Hz, 3H), 0.67 (br. s., 1H). Analytical HPLC (Method A): RT = 6.10 min, 99.9% purity; Factor XIa Ki = 16 nM, Plasma Kallikrein Ki = 1,800 nM.

Example 285

Preparation of (9R,13S)-13-[4-(2,5-dichlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one

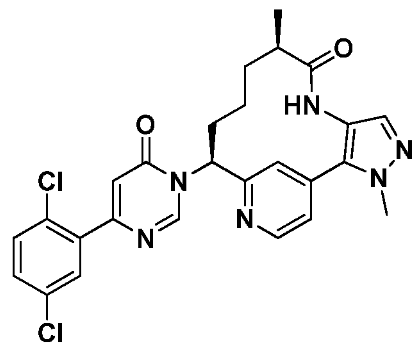

(9*R*,13*S*)-13-[4-(2,5-Dichlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate (4.3 mg, 13% yield) was a minor product isolated from the preparation of methyl 1-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylate, Example 284. MS(ESI) *m/z:* 523.5 $(M+H)^+$. $^1H$ NMR (400MHz, $CD_3OD$) δ 9.04 (s, 1H), 8.74 (d, *J*=5.3 Hz, 1H), 7.73 (s, 1H), 7.68 (d, *J*=2.4 Hz, 1H), 7.55 - 7.49 (m, 3H), 7.49 - 7.44 (m, 1H), 6.77 (d, *J*=0.7 Hz, 1H), 6.06 (dd, *J*=12.7, 4.1 Hz, 1H), 4.05 (s, 3H), 2.78 - 2.68 (m, 1H), 2.38 (tt, *J*=12.8, 4.3 Hz, 1H), 2.16 - 2.02 (m, 2H), 1.68 - 1.45 (m, 2H), 1.02 (d, *J*=6.8 Hz, 3H), 0.70 (br. s., 1H). Analytical HPLC (Method A): RT = 8.29 min, 98.6% purity; Factor XIa Ki = 50 nM, Plasma Kallikrein Ki = 770 nM.

Example 288

Preparation of 1-(4-chloro-2-{1-[(9R,13 S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylic acid

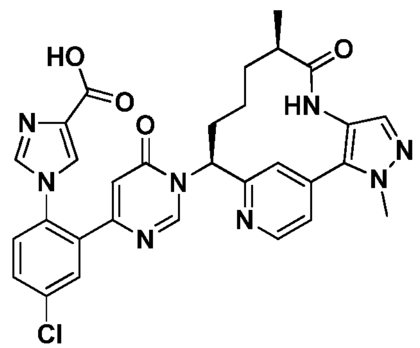

To the solution of methyl 1-(4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-1H-imidazole-4-carboxylate (0.008 g, 9.51 μmol), prepared as described in Example 284, in MeOH (1 ml) was added 1 N NaOH (0.057 ml, 0.057 mmol). After stirring at rt for 24 h, the reaction was quenched with a few drops of TFA. Purification by reverse

phase chromatography afforded 1-(4-chloro-2-{1-[(9*R*,13*S*)-3,9-dimethyl-8-oxo-3,4,7,15-tetraazatricyclo[12.3.1.$0^{1,6}$]octadeca-1(18),2(6),4,14,16-pentaen-13-yl]-6-oxo-1,6-dihydropyrimidin-4-yl}phenyl)-1H-imidazole-4-carboxylic acid trifluoroacetate (4.8 mg, 61% yield) as a white solid. MS(ESI) *m/z:* 599.5 (M+H)$^+$. $^1$H NMR (400MHz, $CD_3OD$) δ 8.76 (s, 1H), 8.71 - 8.66 (m, 2H), 8.11 (br. s., 1H), 7.89 (d, *J*=2.2 Hz, 1H), 7.77 - 7.72 (m, 1H), 7.70 - 7.65 (m, 2H), 7.51 - 7.48 (m, 2H), 6.58 (s, 1H), 5.97 (dd, *J*=12.5, 4.2 Hz, 1H), 4.04 (s, 3H), 2.75-2.64 (m, 1H), 2.34 - 2.22 (m, 1H), 2.12 - 1.93 (m, 2H), 1.65 - 1.39 (m, 2H), 1.00 (d, *J*=6.8 Hz, 3H), 0.79 - 0.62 (m, 1H). Analytical HPLC (Method A): RT = 5.13 min, 99.5% purity; Factor XIa Ki = 3.7 nM, Plasma Kallikrein Ki = 450 nM.

Example 290

Preparation of (10R,14S)-14-{4-[5-chloro-2-(4-chloro-1H-1,2,3-triazol-1-yl)phenyl]-6-oxo-1,6-dihydropyrimidin-1-yl}-10-methyl-3,5,8-triazatricyclo[13.3.1.$0^{2,7}$]nonadeca-1(19),2(7),3,5,15,17-hexaen-9-one

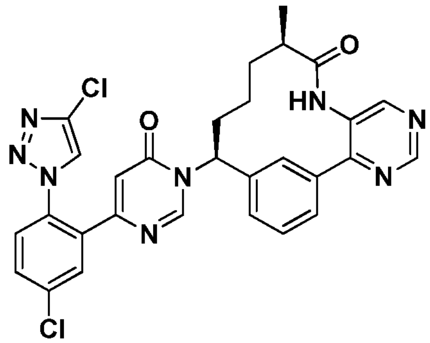

(10R,14S)-14-{4-[5-Chloro-2-(4-chloro-1H-1,2,3-triazol-1-yl)phenyl]-6-oxo-1,6-dihydropyrimidin-1-yl}-10-methyl-3,5,8-triazatricyclo[13.3.1.$0^{2,7}$]nonadeca-1(19),2(7),3,5,15,17-hexaen-9-one trifluoroacetate (0.004 g, 28%) was prepared according to the procedures described in Example 206 by using (10R,14S)-14-amino-10-methyl-3,5,8-triazatricyclo[13.3.1.$0^{2,7}$]nonadeca-1(19),2,4,6,15,17-hexaen-9-one, prepared as described in Intermediate 38, and 4-bromopyrimidin-5-amine in prepared as described in Intermediate 38B. MS(ESI) *m/z:* 587.1 (M+H)$^+$. $^1$H NMR (400MHz, $CD_3OD$) δ 9.18 (s, 1H), 8.71 (s, 1H), 8.35 - 8.31 (m, 2H), 8.01 (s, 1H), 7.87 (d, *J*=2.4 Hz, 1H), 7.86 - 7.83 (m, 1H), 7.76 - 7.71 (m, 1H), 7.67 - 7.62 (m, 2H), 7.35 (d, *J*=7.9 Hz, 1H), 6.42 (d, *J*=0.7 Hz, 1H), 5.84 (dd, *J*=13.0, 3.7 Hz, 1H), 2.72 - 2.59 (m, 1H), 2.40-2.27 (m, 1H), 2.18 - 2.07 (m, 1H), 2.03 - 1.94 (m, 1H), 1.68 - 1.56 (m, 1H), 1.46 - 1.33 (m, 2H), 1.15 (d, *J*=7.0 Hz, 3H). Analytical HPLC (Method A): RT = 8.12 min, purity > 99%; Factor XIa Ki = 2.4 nM, Plasma Kallikrein Ki = 150 nM.

Example 297

Preparation of (9R,13S)-13-{4-[5-chloro-2-(1,2,3-thiadiazol-4-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-one

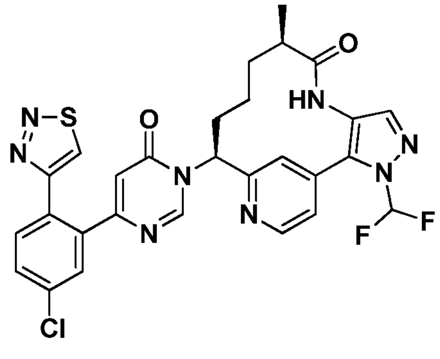

297A. Preparation of 1-[4-chloro-2-(6-methoxypyrimidin-4-yl)phenyl]ethan-1-one

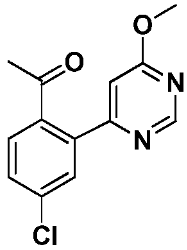

A solution of 4-(2-bromo-5-chlorophenyl)-6-methoxypyrimidine (0.173 g, 0.578 mmol), CeF (0.351 g, 2.310 mmol) in $ClCH_2CH_2Cl$ (3 mL) was purged with Ar, and $Pd(PPh_3)_4$ (0.033 g, 0.029 mmol) and 1-(trimethylsilyl)ethanone (0.165 mL, 1.155 mmol) were added. The reaction mixture was purged with Ar, sealed and heated at 75 °C for 2 days then cooled down to rt. Hexane (1 ml) was added, reaction mixture was filtered through a pad of CELITE®, rinsed with 10 ml EtOAc, filtrate concentrated. Purification by normal phase chromatography gave 1-[4-chloro-2-(6-methoxypyrimidin-4-yl)phenyl] ethan-1-one (0.057 g, 38% yield). MS(ESI) *m/z:* 263.08 $(M+H)^+$.

297B. Preparation of N'-{1-[4-chloro-2-(6-methoxypyrimidin-4-yl)phenyl]ethenyl} ethoxycarbohydrazide

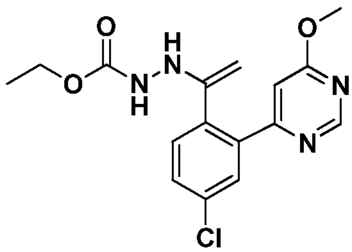

To a solution of 1-(4-chloro-2-(6-methoxypyrimidin-4-yl)phenyl)ethanone (0.057 g, 0.217 mmol) and ethyl hydrazinecarboxylate (0.022 g, 0.217 mmol) in EtOH (3 mL) was added 2 drops of conc. aq HCl. The reaction was heated at 75 °C for 2 h. After this time, the reaction mixture was concentrated to yield crude solid N'-{1-[4-chloro-2-(6-methoxypyrimidin-4-yl)phenyl]ethenyl}ethoxycarbohydrazide. MS(ESI) *m/z:* 349.4 $(M+H)^+$.

297C. Preparation of 4-[5-chloro-2-(1,2,3-thiadiazol-4-yl)phenyl]-6-methoxypyrimidine

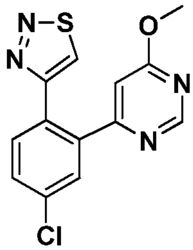

To N'-{1-[4-chloro-2-(6-methoxypyrimidin-4-yl)phenyl]ethenyl} ethoxycarbohydrazide (0.076 g, 0.217 mmol) in a vial was added $SOCl_2$ (0.32 ml, 4.34 mmol), and the resulting solution was stirred at rt for 30 min, then heated at 60 °C for 1 h. After this time, the solution was cooled to rt. To the reaction mixture was added MeOH, and the solution was concentrated. The residue was purified by reverse phase chromatography to afford 4-(4-chloro-2-(6-methoxypyrimidin-4-yl)phenyl)-1,2,3-thiadiazole (0.017 g, 26% yield) as a yellow solid. MS(ESI) *m/z:* 305.0 $(M+H)^+$. $^1H$ NMR (400MHz, $CD_3OD$) δ 8.87 (s, 1H), 8.63 (d, *J*=1.1 Hz, 1H), 7.86 (d, *J*=8.6 Hz, 1H), 7.76 (d, *J*=2.0 Hz, 1H), 7.71 (dd, *J*=8.4, 2.2 Hz, 1H), 6.82 (d, *J*=1.1 Hz, 1H), 4.02 (s, 3H).

297D. Preparation of 6-[5-chloro-2-(1,2,3-thiadiazol-4-yl)phenyl]pyrimidin-4-ol

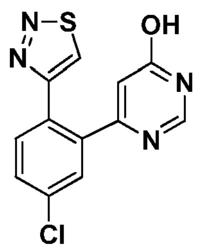

4-(4-Chloro-2-(6-methoxypyrimidin-4-yl)phenyl)-1,2,3-thiadiazole (0.059 g, 0.194 mmol) in AcOH (2 ml) was added 48% aq HBr (1.1 ml, 9.68 mmol), and the solution was heated at 85 °C for 1 h then cooled to rt. The reaction mixture was concentrated. The residue was dissolved in EtOAc, washed with sat aq $NaHCO_3$, brine, dried over $MgSO_4$, filtered and concentrated to give 6-(5-chloro-2-(1,2,3-thiadiazol-4-yl) phenyl)pyrimidin-4-ol (0.053 g, 94% yield) as an off-white solid. MS(ESI) *m/z:* 291.0 $(M+H)^+$. $^1H$ NMR (400MHz, $CD_3OD$) δ 8.84 (s, 1H), 7.94 (s, 1H), 7.70 (d, *J*=8.1 Hz, 1H), 7.61 (d, *J*=2.0 Hz, 1H), 7.55 (dd, *J*=8.4, 2.2 Hz, 1H), 6.27 (d, *J*=0.7 Hz, 1H).

297E. Preparation of (9R,13S)-13-{4-[5-chloro-2-(1,2,3-thiadiazol-4-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-onetrifluoroacetate

(9R,13S)-13-{4-[5-Chloro-2-(1,2,3-thiadiazol-4-yl)phenyl]-6-oxo-1,6-dihydropyrimidin-1-yl}-3-(difluoromethyl)-9-methyl-3,4,7,15-tetraazatricyclo [12.3.1.$0^{2,6}$]octadeca-1(18),2(6),4,14,16-pentaen-8-one trifluoroacetate (0.006 g, 7.7%) was prepared according to the procedures described in Example 56 by using (9R,13S)-13-amino-3-(difluoromethyl)-9-methyl-3,4,7,15-tetraazatricyclo[12.3.1.$0^{2,6}$]octadeca-1(18),2(6),4,14,16-pentaen-8-one, prepared as described in Intermediate 30, and 6-[5-chloro-2-(1,2,3-thiadiazol-4-yl)phenyl]pyrimidin-4-ol, MS(ESI) *m/z:* 609.1 $(M+H)^+$. $^1H$ NMR (400MHz, $CD_3OD$ δ 8.93 (s, 1H), 8.87 (s, 1H), 8.74 (d, *J*=5.1 Hz, 1H), 7.82 - 7.63 (m, 6H), 7.54 - 7.50 (m, 1H), 6.38 (s, 1H), 6.02 (dd, *J*=12.8, 4.8 Hz, 1H), 2.78 - 2.65 (m, 1H), 2.35 - 2.22 (m, 1H), 2.10 - 1.96 (m, 2H), 1.65 - 1.54 (m, 1H), 1.53 - 1.42 (m, 1H), 0.99 (d, *J*=7.0 Hz, 3H), 0.63 (br. s., 1H). Analytical HPLC (Method A): RT = 8.80 min, purity > 96%; Factor XIa Ki = 1.6 nM, Plasma Kallikrein Ki = 520 nM.

Example 298

Preparation of (9R,13S)-13-(4-{3-chloro-2-fluoro-6-[4-(trifluoromethyl)-1H-pyrazol-1-yl]phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one

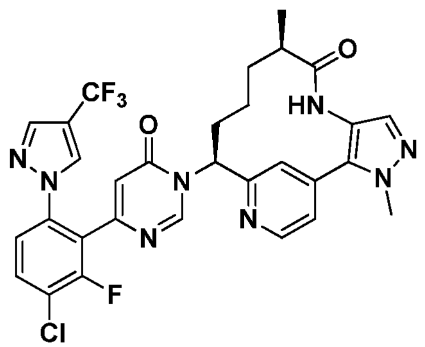

298A. Preparation of 4-(3-chloro-2-fluoro-6-(4-(trifluoromethyl)-1H-pyrazol-1-yl) phenyl)-6-methoxypyrimidine

4-(3-Chloro-2-fluoro-6-(4-(trifluoromethyl)-1H-pyrazol-1-yl)phenyl)-6-methoxypyrimidine (42 mg, 25% yield) was prepared in a similar manner as the procedure described in Example 238C, by replacing 1H-pyrazole-4-carbonitrile with 4-(trifluoromethyl)-1H-pyrazole (37.3 mg, 0.274 mmol). MS(ESI) *m/z:* 373.3 $(M+H)^+$. $^1H$ NMR (400MHz, $CDCl_3$) δ 8.68 (d, *J*=1.1 Hz, 1H), 7.76 (s, 1H), 7.70 (s, 1H), 7.62 (dd, *J*=8.6, 7.7 Hz, 1H), 7.36 (dd, *J*=8.7, 1.7 Hz, 1H), 6.78 (t, *J*=1.2 Hz, 1H), 4.01 (s, 3H).

298B. Preparation of 6-(3-chloro-2-fluoro-6-(4-(trifluoromethyl)-1H-pyrazol-1-yl) phenyl)pyrimidin-4-ol

6-(3-Chloro-2-fluoro-6-(4-(trifluoromethyl)-1H-pyrazol-1-yl)phenyl)pyrimidin-4-ol (0.017 g, 42.1% yield) was prepared in a similar manner as the procedure described for the preparation of 1-[4-chloro-2-(6-hydroxypyrimidin-4-yl)phenyl]-1H-1,2,3-triazole-4-carbonitrile as described in Intermediate 18C, by replacing 1-[4-chloro-2-(6-methoxypyrimidin-4-yl)phenyl]-1H-1,2,3-triazole-4-carbonitrile with 4-(3-chloro-2-fluoro-6-(4-(trifluoromethyl)-1H-pyrazol-1-yl)phenyl)-6-methoxypyrimidine (0.042 g, 0.113 mmol). MS(ESI) *m/z:* 359.4 $(M+H)^+$. $^1H$ NMR (400MHz, $CD_3OD$) δ 8.36 (d, *J*=09 Hz, 1H), 8.09 (d, *J*=1.1 Hz, 1H), 7.85 (s, 1H), 7.81 - 7.74 (m, 1H), 7.50 (dd, *J*=8.7, 1.7 Hz, 1H), 6.50 (t, *J*=1.1 Hz, 1H).

298C. Preparation of (9R,13 S)-13-(4-{3-chloro-2-fluoro-6-[4-(trifluoromethyl)-1H-pyrazol-1-yl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one

(9R,13S)-13-(4-{3-Chloro-2-fluoro-6-[4-(trifluoromethyl)-1H-pyrazol-1-yl] phenyl}-6-oxo-1,6-dihydropyrimidin-1-yl)-3,9-dimethyl-3,4,7,15-tetraazatricyclo [12.3.1.0$^{2,6}$]octadeca-1(18),2(6),4,14,16-pentaen-8-one (13 mg, 37% yield) was prepared in a similar manner as the procedure described in Example 56, by replacing 6-{5-chloro-2-[4-(trifluoromethyl)-1H-1,2,3-triazol-1-yl]phenyl}pyrimidin-4-ol with 6-(3-chloro-2-fluoro-6-(4-(trifluoromethyl)-1H-pyrazol-1-yl)phenyl)pyrimidin-4-ol (17 mg, 0.047 mmol). MS(ESI) *m/z:* 641.2 $(M+H)^+$. $^1H$ NMR (400MHz, $CD_3OD$) δ 9.40 (s, 1H), 8.85 (br. s., 1H), 8.72 (d, *J*=4.8 Hz, 1H), 8.31 (s, 1H), 7.85 - 7.74 (m, 2H), 7.69 (s, 1H), 7.55 - 7.44 (m, 3H), 6.53 (s, 1H), 6.02 (dd, *J*=12.4, 3.9 Hz, 1H), 4.05 (s, 3H), 2.70 (td, *J*=6.6, 3.1 Hz, 1H), 2.37 - 2.22 (m, 1H), 2.15 - 1.91 (m, 2H), 1.69 - 1.54 (m, 1H), 1.53 - 1.39 (m, 1H), 1.01 (d, *J*=6.8 Hz, 3H), 0.70 (m., 1H). Analytical HPLC (Method A): RT = 8.81 min, purity = 99%; Factor XIa Ki = 3.8 nM, Plasma Kallikrein Ki = 1,200 nM.

Example 299

Preparation of (9R,13S)-13-[4-(3-chloro-2,6-difluorophenyl)-6-oxo-1,6-dihydropyrimidin-1-yl]-9-methyl-3,4,7,15-tetraazatricyclo[12.3.1.0$^{2,6}$]octadeca-1(18),2(6),4,14,16-pentaen-8-one

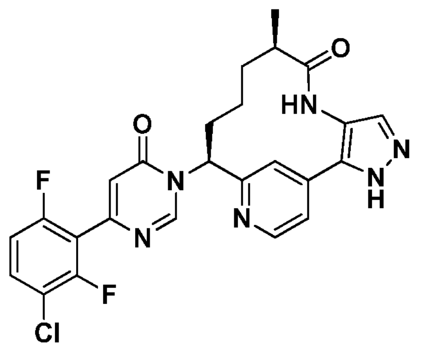

299A. Preparation of (9*R*,13*S*)-13-[4-(3-chloro-2,6-difluorophenyl)-6-oxo-1,6-dihydropyrimidin-1-yl]-9-methyl-3-{[2-(trimethylsilyl)ethoxy]methyl}-3,4,7,15-tetraazatricyclo[12.3.1.0$^{2,6}$]octadeca-1(18),2(6),4,14,16-pentaen-8-one

(9*R*,13*S*)-13-[4-(3-Chloro-2,6-difluorophenyl)-6-oxo-1,6-dihydropyrimidin-1-yl]-9-methyl-3-{[2-(trimethylsilyl)ethoxy]methyl}-3,4,7,15-tetraazatricyclo[12.3.1.0$^{2,6}$] octadeca-1(18),2(6),4,14,16-pentaen-8-one (78 mg, 89% yield) was prepared in a similar manner as the procedures described in Example 56, by using 6-(3-chloro-2,6-difluorophenyl)pyrimidin-4-ol (0.033 g, 0.137 mmol), prepared as described in Intermediate 4, and (9*R*,13*S*)-9-methyl-3-{[2-(trimethylsilyl)ethoxy]methyl}-3,4,7,15-tetraazatricyclo[12.3.1.0$^{2,6}$]octadeca-1(18),2(6),4,14,16-pentaen-8-one (0.057 g, 0.137 mmol), prepared as described in Intermediate 19. MS(ESI) *m/z:* 641.6 $[M+H]^+$.

299B. Preparation of (9*R*,13*S*)-13-[4-(3-chloro-2,6-difluorophenyl)-6-oxo-1,6-dihydropyrimidin-1-yl]-9-methyl-3,4,7,15-tetraazatricyclo[12.3.1.$0^{2,6}$]octadeca-1(18),2(6),4,14,16-pentaen-8-one trifluoroacetate

To a solution of (9*R*,13*S*)-13-[4-(3-chloro-2,6-difluorophenyl)-6-oxo-1,6-dihydropyrimidin-1-yl]-9-methyl-3-{[2-(trimethylsilyl)ethoxy]methyl}-3,4,7,15-tetraazatricyclo[12.3.1.$0^{2,6}$]octadeca-1(18),2(6),4,14,16-pentaen-8-one (78 mg, 0.122 mmol), in DCM (1.6 mL) was added TFA (0.4 mL, 5.19 mmol) and the resulting solution was stirred at rt for 30 min. The reaction mixture was then concentrated and the residue was purified by prep HPLC purification to give (9*R*,13*S*)-13-[4-(3-chloro-2,6-difluorophenyl)-6-oxo-1,6-dihydropyrimidin-1-yl]-9-methyl-3,4,7,15-tetraazatricyclo [12.3.1.$0^{2,6}$]octadeca-1(18),2(6),4,14,16-pentaen-8-one trifluoroacetate (70 mg, 0.112 mmol, 92% yield). $^1$H NMR (500MHz, DMSO-$d_6$) δ 9.31 (s, 1H), 8.95 (s, 1H), 8.57 (br. s., 1H), 7.92 - 7.71 (m, 3H), 7.48 (d, *J*=4.0 Hz, 1H), 7.31 (t, *J*=8.9 Hz, 1H), 6.70 (s, 1H), 5.99 (br. s., 1H), 2.73 (br. s., 1H), 2.30 (br. s., 2H), 2.01 - 1.88 (m, 1H), 1.60 - 1.37 (m, 2H), 0.93 (d, *J*=6.7 Hz, 3H), 0.64 (br. s., 1H). MS(ESI) *m/z:* 511.3 $[M+H]^+$. Analytical HPLC (Method B): RT = 1.47 min, purity = 94.0%; Factor XIa Ki = 970 nM.

Example 300

Preparation of (9R,13S)-13-{4-[5-chloro-2-(1,2,3-thiadiazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one.

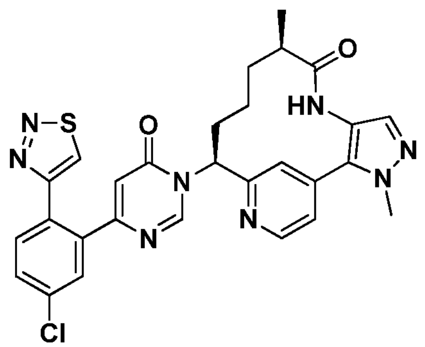

(9R,13S)-13-{4-[5-Chloro-2-(1,2,3-thiadiazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (0.0072 g, 37.8%) was prepared in a similar manner as the procedure described in Example 297 using (9*R*,13*S*)-13-amino-3,9-dimethyl-3,4,7,15-tetraazatricyclo[12.3.1.$0^{2,6}$]octadeca-1(18),2(6),4,14,16-pentaen-8-one, prepared as described in Intermediate 32. MS(ESI) *m/z:* 573.2 $(M+H)^+$. $^1$H NMR (400MHz, $CD_3OD$) δ 8.98 (s, 1H), 8.85 (s, 1H), 8.76 (d, *J*=5.3 Hz, 1H), 7.82 (d, *J*=8.1 Hz, 1H), 7.78 (s, 1H), 7.76 (d, *J*=2.2 Hz, 1H), 7.68 (dd, *J*=8.1, 2.2 Hz, 1H), 7.61 (dd, *J*=5.3, 1.5 Hz, 1H), 7.52 (s, 1H), 6.40 (s, 1H), 5.99 (dd, *J*=12.8, 4.4 Hz, 1H), 4.07 (s, 3H), 2.77 - 2.67 (m, 1H), 2.39 - 2.27 (m, 1H), 2.14 - 2.00 (m, 2H), 1.69 - 1.56 (m, 1H), 1.55-1.41 (m, 1H), 1.03 (d, *J*=7.0 Hz, 3H), 0.76 (br. s., 1H). Analytical HPLC (Method A): RT = 7.46 min, purity > 99%; Factor XIa Ki = 1.5 nM, Plasma Kallikrein Ki = 280 nM.

Example 302

Preparation of (9R,13S)-13-[4-(3-chloro-2,6-difluorophenyl)-6-oxo-1,6-dihydropyrimidin-1-yl]-9-methyl-4-(pyrimidin-5-yl)-3,4,7,15-tetraazatricyclo [12.3.1.$0^{2,6}$]octadeca-1(18),2,5,14,16-pentaen-8-one

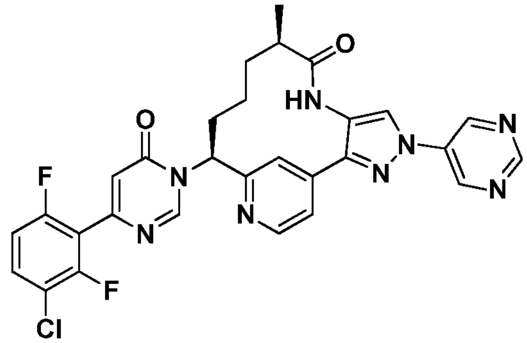

(9*R*,13*S*)-13-[4-(3-Chloro-2,6-difluorophenyl)-6-oxo-1,6-dihydropylimidin-1-yl]-9-methyl-4-(pyrimidin-5-yl)-3,4,7,15-tetraazatricyclo[12.3.1.$0^{2,6}$]octadeca-1(18),2,5,14,16-pentaen-8-one trifluoroacetate (13.7 mg, 0.019 mmol, 33% yield) was prepared in a similar manner as the procedure described in Example 216, by using 5-iodopyrimidine (24 mg, 0.117 mmol) and (9*R*,13*S*)-13-[4-(3-chloro-2,6-difluorophenyl)-6-oxo-1,6-dihydropyrimidin-1-yl]-9-methyl-3,4,7,15-tetraazatricyclo[12.3.1.$0^{2,6}$] octadeca-1(18),2(6),4,14,16-pentaen-8-one trifluoroacetate (30 mg, 0.059 mmol), as described in Example 299. $^1$H NMR (500MHz, DMSO-$d_6$) δ 9.58 (s, 1H), 9.35 (s, 2H), 9.15 (s, 1H), 8.97 (s, 1H), 8.76 (s, 1H), 8.63 (d, *J*=4.9 Hz, 1H), 7.92 (s, 1H), 7.74 (td, *J*=8.7, 5.8 Hz, 1H), 7.61 (d, *J*=4.9 Hz, 1H), 7.29 (t, *J*=9.0 Hz, 1H), 6.69 (s, 1H), 6.01 (d, *J*=11.6 Hz, 1H), 2.77 (br. s., 1H), 2.28 (br. s., 2H), 1.99 - 1.88 (m, 1H), 1.54 (br. s., 1H), 1.43 (br. s., 1H), 0.91 (d, *J*=6.7 Hz, 3H), 0.55 (br. s., 1H). MS(ESI) *m/z:* 589.2 $[M+H]^+$. Analytical HPLC (Method B): RT = 1.64 min, purity = 100.0%; Factor XIa Ki = 100 nM, Plasma Kallikrein Ki = 530 nM.

Example 303

Preparation of (9R,13S)-13-{4-[5-chloro-2-(pyridazin-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

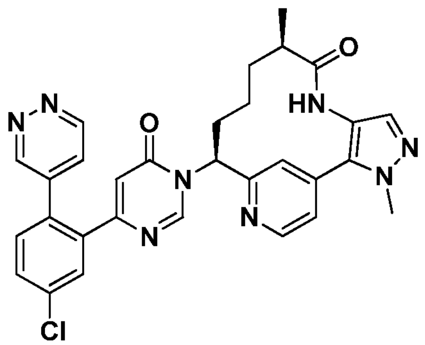

To a degassed solution of (9*R*,13*S*)-13-[4-(5-chloro-2-iodophenyl)-6-oxo-1,6-dihydropyrimidin-1-yl]-3,9-dimethyl-3,4,7,15-tetraazatricyclo[12.3.1.$0^{2,6}$]octadeca-1(18),2(6),4,14,16-pentaen-8-one (20 mg, 0.033 mmol), prepared as described in Example 211, 4-(tributylstannyl)pyridazine (18.01 mg, 0.049 mmol), CuI (1.24 mg, 6.51 μmol), and CsF (9.88 mg, 0.065 mmol) in ACN (1 ml) was added $Pd(Ph_3P)_4$ (3.76 mg, 3.25 μmol). After stirring at 45 °C for 2 h, the reaction was cooled to rt and concentrated. Purification by reverse phase chromatography afforded (9*R*,13*S*)-13-{4-[5-chloro-2-(pyridazin-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one trifluoroacetate (14.9 mg, 67% yield). MS(ESI) *m/z:* 567.35 $(M+H)^+$. $^1$H NMR (500MHz, DMSO-$d_6$) δ 9.27 - 9.19 (m, 2H), 9.09 (br. s., 1H), 8.76 (s, 1H), 8.67 (d, *J*=5.2 Hz, 1H), 7.80 (d, *J*=1.8 Hz, 1H), 7.74 (dd, *J*=8.2, 2.1 Hz, 1H), 7.68 - 7.56 (m, 4H), 7.48 (s, 1H), 6.50 (s, 1H), 5.92 - 5.83 (m, 1H), 4.01 (s, 3H), 2.69 - 2.60 (m, 1H), 2.31 - 2.22 (m, 1H), 2.14 - 2.04 (m, 1H), 1.89 - 1.78 (m, 1H), 1.53 - 1.42 (m, 1H), 1.38 - 1.26 (m, 1H), 0.88 (d, *J*=6.7 Hz, 3H), 0.50 - 0.32 (m, 1H). Analytical HPLC (Method C): RT = 1.28 min, 100% purity; Factor XIa Ki = 110 nM, Plasma Kallikrein Ki = 9,300 nM.

Example 305

Preparation of (10R,14S)-14-(4-{5-chloro-2-[4-(trifluoromethyl)-1H-1,2,3-triazol-1-yl] phenyl}-6-oxo-1,6-dihydropyrimidin-1-yl)-10-methyl-5,8,17-triazatricyclo [13.3.1.0$^{2,7}$]nonadeca-1(19),2(7),3,5,15,17-hexaen-9-one

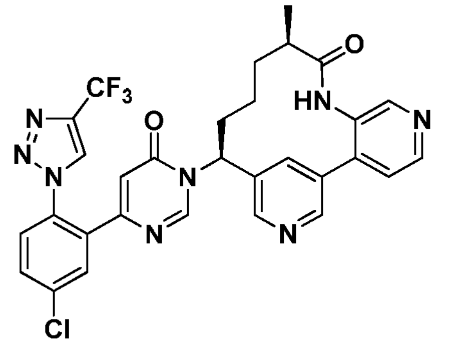

305A. Preparation of (S)-*tert*-butyl (1-(5-(5,5-dimethyl-1,3,2-dioxaborinan-2-yl)pyridin-3-yl)but-3-en-1-yl)carbamate

To a solution of 5,5,5',5'-tetramethyl-2,2'-bi(1,3,2-dioxaborinane) (2.9 g, 12.84 mmol) and (S)-*tert*-butyl (1-(5-bromopyridin-3-yl)but-3-en-1-yl)carbamate (3.0 g, 9.17 mmol) in toluene (38.8 mL) were added KOAc (2.70 g, 27.5 mmol) and Pd (dppf) $Cl_2.CH_2Cl_2$ Adduct (0.599 g, 0.733 mmol). The reaction was purged with Ar for 10 min and then heated at 90 °C for 12 h. The reaction mixture was diluted with EtOAc and then filtered through CELITE®. The filtrate was washed with water, brine, dried over $Na_2SO_4$, filtered, and concentrated. MS(ESI) *m*/*z:* 293 $(M-C_6H_{10}+H)^+$.

305B. Preparation of (S)-*tert*-butyl (1-(3'-amino-[3,4'-bipyridin]-5-yl)but-3-en-1-yl)carbamate

(S)-*tert*-Butyl (1-(5-(5,5-dimethyl-1,3,2-dioxaborinan-2-yl)pyridin-3-yl)but-3-en-1-yl)carbamate (1.5 g, 4.16 mmol), 4-bromopyridin-3-amine (0.72 g, 4.16 mmol), 4 M $NaHCO_3$ (3.12 mL, 12.49 mmol) were added to dioxane (5 mL) and purged with Ar. After 15 min, $Pd(PPh_3)_4$ (0.241 g, 0.208 mmol) was added and the mixture heated at 90 °C overnight. The reaction mixture was diluted with water (100ml) and extracted with EtOAc (2x50ml), washed with brine, dried and evaporated to a black oil and was carried forward to the next reaction. MS(ESI) *m*/*z:* 341.2 $(M+H)^+$.

305C. Preparation of *tert*-butyl ((S)-1-(3'-((R)-2-methylbut-3-enamido)-[3,4'-bipyridin]-5-yl)but-3-en-1-yl)carbamate

(R)-2-Methylbut-3-enoic acid (0.576 g, 5.76 mmol), (S)-*tert*-butyl (1-(3'-amino-[3,4'-bipyridin]-5-yl)but-3-en-1-yl)carbamate (1.4 g, 4.11 mmol), pyridine (0.998 ml, 12.34 mmol) in EtOAc (43.8 ml) was cooled to 0 °C. T3P® (50%wt in EtOAc) (5.23 g, 8.23 mmol) was added and the solution allowed to gradually come to rt. After 3 h, reaction mixture was concentrated and the residue purified by normal phase chromatography using EtOAc and MeOH as eluants to give *tert*-butyl ((S)-1-(3'-((R)-2-methylbut-3-enamido)-[3,4'-bipyridin]-5-yl)but-3-en-1-yl)carbamate (747 mg, 43%) as a brown oil. MS(ESI) *m*/*z:* 423.2 $(M+H)^+$. $^1H$ NMR (500MHz, $CDCl_3$) $\delta$ 9.38 (br. s., 1H), 8.66 (s, 1H), 8.57 - 8.47 (m, 2H), 7.62 (br. s., 1H), 7.44 (br. s., 1H), 7.20 (d, J=4.1 Hz, 1H), 5.86 - 5.76 (m, 1H), 5.75 - 5.65 (m, 1H), 5.21 - 5.10 (m, 3H), 3.71 (d, J=11.0 Hz, 3H), 3.09 (t, J=7.3 Hz, 1H), 2.66 - 2.46 (m, 2H), 1.44 - 1.39 (m, 9H), 1.28 (d, J=7.2 Hz, 3H).

305D. Preparation of *tert*-butyl N-[(10R,11E,14S)-10-methyl-9-oxo-5,8,17-triazatricyclo [13.3.1.0$^{2,7}$]nonadeca-1(19),2(7),3,5,11,15,17-heptaen-14-yl]carbamate

*tert*-Butyl ((S)-1-(3'-((R)-2-methylbut-3-enamido)-[3,4'-bipyridin]-5-yl)but-3-en-1-yl)carbamate (0.747 g, 1.768 mmol) and pTsOH (0.689 g, 3.62 mmol) were added to EtOAc (1040 mL) and heated to 60 °C while purging with Ar. After 1 h, Second Generation Grubbs Catalyst (0.600 g, 0.707 mmol) was added and the mixture stirred at 60 °C overnight. The reaction was quenched with sat $NaHCO_3$ (150 mL) and extracted with EtOAc (2 x 50 mL). The combined organic layers were washed with brine (100 mL), dried ($Na_2SO_4$), filtered and concentrated. The crude material was purified by normal phase chromatography DCM and MeOH as eluants to give *tert*-butyl N-[(10R,11E,14S)-10-methyl-9-oxo-5,8,17-triazatricyclo[13.3.1.0$^{2,7}$]nonadeca-1(19),2(7),3,5,11,15,17-heptaen-14-yl]carbamate (0.180 g, 25.8% yield) as a tan solid. MS(ESI) *m*/*z:* 395.2 $(M+H)^+$.

305E. Preparation of *tert*-butyl N-[(10R,14S)-10-methyl-9-oxo-5,8,17-triazatricyclo [13.3.1.0$^{2,7}$]nonadeca-1(19),2(7),3,5,15,17-hexaen-14-yl]carbamate

$PtO_2$ (10.36 mg, 0.046 mmol) was added to a solution of *tert*-butyl N-[(10R,11E,14S)-10-methyl-9-oxo-5,8,17-triazatricyclo[13.3.1.0$^{2,7}$]nonadeca-1(19),2(7),3,5,11,15,17-heptaen-14-yl]carbamate (0.180 g, 0.456 mmol) in EtOH (20 mL) and subjected to a $H_2$ atmosphere (55 psi). After 3 h, the suspension was filtered through a plug of CELITE® and the filtrate concentrated and carried forward to the next reaction. MS(ESI) *m/z:* 397.2 $(M+H)^+$.

305F. Preparation of (10R,14S)-14-amino-10-methyl-5,8,17-triazatricyclo[13.3.1.0$^{2,7}$] nonadeca-1(19),2(7),3,5,15,17-hexaen-9-one

TFA (0.70 mL, 9.08 mmol) was added to a stirring solution of *tert*-butyl N-[(10R,14S)-10-methyl-9-oxo-5,8,17-triazatricyclo[13.3.1.0$^{2,7}$]nonadeca-1(19),2(7),3,5,15,17-hexaen-14-yl]carbamate (0.180 g, 0.454 mmol) in DCM (5 mL) at rt. After 2 h, the reaction mixture was concentrated to dryness. The residue was partitioned between EtOAc and sat $NaHCO_3$. The aqueous layer was extracted with EtOAc (2x). The combined organic layer was washed with brine, dried over $Na_2SO_4$, filtered, and concentrated to give (10R,14S)-14-amino-10-methyl-5,8,17-triazatricyclo [13.3.1.0$^{2,7}$]nonadeca-1(19),2(7),3,5,15,17-hexaen-9-one (0.037 g, 27.5%) as a brown film. MS(ESI) *m/z*: 297.2 $(M+H)^+$.

305G. Preparation of (10R,14S)-14-(4-{5-chloro-2-[4-(trifluoromethyl)-1H-1,2,3-triazol-1-yl]phenyl}-6-oxo-1,6-dihydropyrimidin-1-yl)-10-methyl-5,8,17-triazatricyclo [13.3.1.0$^{2,7}$]nonadeca-1(19),2,4,6,15,17-hexaen-9-one, *bis*-trifluoroacetate

(10R,14S)-14-(4-{5-Chloro-2-[4-(trifluoromethyl)-1H-1,2,3-triazol-1-yl]phenyl}-6-oxo-1,6-dihydropyrimidin-1-yl)-10-methyl-5,8,17-triazatricyclo[13.3.1.0$^{2,7}$]nonadeca-1(19),2,4,6,15,17-hexaen-9-one, *bis*-trifluoroacetate was prepared (5.2 mg, 11%) in a similar manner as Example 56 using (10R,14S)-14-amino-10-methyl-5,8,17-triazatricyclo[13.3.1.0$^{2,7}$]nonadeca-1(19),2(7),3,5,15,17-hexaen-9-one and 6-{5-chloro-2-[4-(trifluoromethyl)-1H-1,2,3-triazol-1-yl]phenyl}pyrimidin-4-ol, prepared as described in Intermediate 15. MS(ESI) *m/z:* 621.2 $(M+H)^+$. $^1H$ NMR (400MHz, DMSO-$d_6$) $\delta$ 9.69 (s, 1H), 9.19 - 9.13 (m, 1H), 8.64 - 8.55 (m, 2H), 8.49 (s, 1H), 8.41 - 8.38 (m, 1H), 8.03 (s, 1H), 7.87 - 7.84 (m, 1H), 7.79 - 7.73 (m, 2H), 7.64 (d, J=4.6 Hz, 1H), 6.42 (s, 1H), 5.51 (d, J=12.8 Hz, 1H), 1.90 - 1.84 (m, 1H), 1.75 - 1.67 (m, 1H), 1.39 - 1.32 (m, 1H), 1.14 - 1.02 (m, 2H), 0.95 - 0.85 (m, 3H). Analytical HPLC (Method A): RT = 6.96 min, purity = 93%; Factor XIa Ki = 0.17 nM, Plasma Kallikrein Ki = 25 nM.

Example 307

Preparation of 1-(4-chloro-2-{1-[(9R,13 S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile

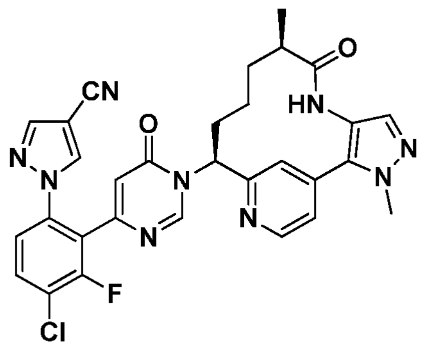

1-(4-Chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}-3-fluorophenyl)-1H-pyrazole-4-carbonitrile trifluoroacetate (2 mg, 43% yield) was prepared in a similar manner as the procedure described in Example 56, by replacing 6-{5-chloro-2-[4-(trifluoromethyl)-1H-1,2,3-triazol-1-yl]phenyl}pyrimidin-4-ol with 1-(4-chloro-3-fluoro-2-(6-hydroxypyrimidin-4-yl)phenyl)-1H-pyrazole-4-carbonitrile (2 mg, 6.34 µmol) prepared as described in Example 238C. MS(ESI) *m/z:* 598.1 $(M+H)^+$. $^1H$ NMR (400MHz, $CD_3OD$) $\delta$ 8.93 - 8.67 (m, 2H), 8.47 (s, 1H), 7.94 (s, 1H), 7.80 (dd, *J*=8.6, 7.7 Hz, 1H), 7.70 (s, 1H), 7.57 - 7.45 (m, 3H), 6.54 (s, 1H), 6.00 (dd, *J*=12.8, 4.2 Hz, 1H), 4.05 (s, 3H), 2.71 (td, *J*=6.6, 3.3 Hz, 1H),

2.37 - 2.23 (m, 1H), 2.13 - 1.96 (m, 2H), 1.69 - 1.55 (m, 1H), 1.54 - 1.41 (m, 1H), 1.01 (d, *J*=7.0 Hz, 3H), 0.71 (m, 1H). Analytical HPLC (Method A): RT = 7.58 min, purity = 97.1%; Factor XIa Ki = 2 nM, Plasma Kallikrein Ki = 1,500 nM.

Example 310

Preparation of N-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide

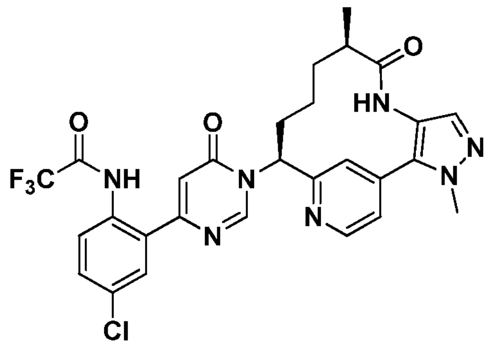

310A. Preparation of N-(4-chloro-2-(6-methoxypyrimidin-4-yl)phenyl)-2,2,2-trifluoroacetamide

TEA (0.71 ml, 5.09 mmol) was added to a solution of 4-chloro-2-(6-methoxypyrimidin-4-yl)aniline (1 g, 4.24 mmol), prepared as described in Intermediate 8A, and TFAA (0.72 ml, 5.09 mmol) in DCM (25 ml). After stirring at rt for 1 h, the reaction was diluted with DCM, washed with sat $NaHCO_3$, brine, dried over $Na_2SO_4$, filtered, and concentrated. A yellow solid was obtained as N-(4-chloro-2-(6-methoxypyrimidin-4-yl)phenyl)-2,2,2-trifluoroacetamide (1.4 g, 99% yield). MS(ESI) *m/z:* 332.0 $(M+H)^+$.

310B. Preparation of N-(4-chloro-2-(6-hydroxypyrimidin-4-yl)phenyl)-2,2,2-trifluoroacetamide

A clear yellow solution of N-(4-chloro-2-(6-methoxypyrimidin-4-yl)phenyl)-2,2,2-trifluoroacetamide (1.4 g, 4.22 mmol) in HOAc (10 ml) and 48% aq HBr (2.39 ml, 21.10 mmol) was warmed to 60 °C for 3 h, then cooled to rt, and the reaction was concentrated. EtOAc (~400 ml) was added to the residue, followed by sat $NaHCO_3$. The layers were separated and the organic layer was washed with sat $NaHCO_3$, brine, dried over $MgSO_4$, filtered and concentrated. The residue was suspended in DCM, and the solid was filtered off. The filtrate was purified by normal phase chromatography to afford N-(4-chloro-2-(6-hydroxypyrimidin-4-yl)phenyl)-2,2,2-trifluoroacetamide (0.095 g, 7% yield) as a white solid. MS(ESI) *m/z:* 318.0 $(M+H)^+$.

310C. Preparation of N-(4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide trifluoroacetate

N-(4-Chloro-2-{1-[(9*R*,13*S*)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide trifluoroacetate (0.113 g, 63% yield) was prepared in a similar manner as the procedure described in Example 56, by using N-(4-chloro-2-(6-hydroxypyrimidin-4-yl)phenyl)-2,2,2-trifluoroacetamide (0.095 g, 0.301 mmol). MS(ESI) *m/z:* 600.0 $(M+H)^+$. $^1H$ NMR (400MHz, DMSO-$d_6$) δ 12.37 (s, 1H), 9.23 (s, 1H), 9.07 (s, 1H), 8.70 (d, *J*=5.1 Hz, 1H), 7.96 - 7.89 (m, 2H), 7.71 (s, 1H), 7.67 - 7.58 (m, 2H), 7.49 (s, 1H), 6.89 (br. s., 1H), 5.95 (d, *J*=9.5 Hz, 1H), 4.02 (s, 3H), 2.72 - 2.62 (m, 1H), 2.44 - 2.32 (m, 1H), 2.20 - 2.07 (m, 1H), 1.98 - 1.85 (m, 1H), 1.58 - 1.30 (m, 2H), 0.91 (d, *J*=6.8 Hz, 3H), 0.58 - 0.39 (m, 1H). Analytical HPLC (Method A): RT = 9.82 min, 100% purity; Factor XIa Ki = 1.7 nM, Plasma Kallikrein Ki = 180 nM.

Example 313

Preparation of (9R,13S)-13-[4-(2-amino-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one

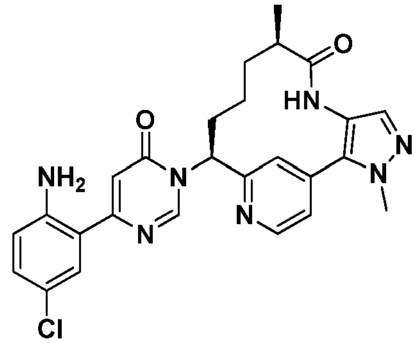

To the solution of N-(4-chloro-2-{1-[(9*R*,13*S*)-3,9-dimethyl-8-oxo-3,4,7,15-tetraazatricyclo[12.3.1.0$^{2,6}$]octadeca-1(18),2(6),4,14,16-pentaen-13-yl]-6-oxo-1,6-dihydropyrimidin-4-yl}phenyl)-2,2,2-trifluoroacetamide (0.111 g, 0.185 mmol), prepared as described in Example 310, in MeOH (2 ml) was added 1.25 M HCl in MeOH (0.5 ml, 0.625 mmol). After stirring at 75 °C for 1 h, the reaction was cooled to rt, concentrated and lyophilized overnight to give (9*R*,13*S*)-13-[4-(2-amino-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one hydrochloride (0.1 g, 94% yield) as a yellow solid. From this material, 10 mg was purified by reverse phase chromatography to afford (9*R*,13*S*)-13-[4-(2-amino-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one trifluoroacetate. MS(ESI) *m/z:* 504.4 (M+H)$^+$. $^1$H NMR (500MHz, $CD_3OD$) δ 8.89 (br. s., 1H), 8.63 (d, *J*=5.0 Hz, 1H), 7.63 (s, 1H), 7.43 - 7.38 (m, 2H), 7.33 (s, 1H), 7.04 (d, *J*=8.5 Hz, 1H), 6.68 (d, *J*=8.8 Hz, 1H), 6.59 (s, 1H), 5.95 (d, *J*=9.9 Hz, 1H), 3.95 (s, 3H), 2.67 - 2.60 (m, 1H), 2.31 - 2.21 (m, 1H), 2.06 - 1.91 (m, 2H), 1.59 - 1.34 (m, 2H), 0.92 (d, *J*=6.9 Hz, 3H), 0.69 - 0.54 (m, 1H). Analytical HPLC (Method B): RT = 1.45 min, 100% purity; Factor XIa Ki = 57 nM, Plasma Kallikrein Ki = 2,400 nM.

Example 314

Preparation of (9R,13S)-13-(4-{5-chloro-2-[(pyrimidin-4-yl)amino]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one

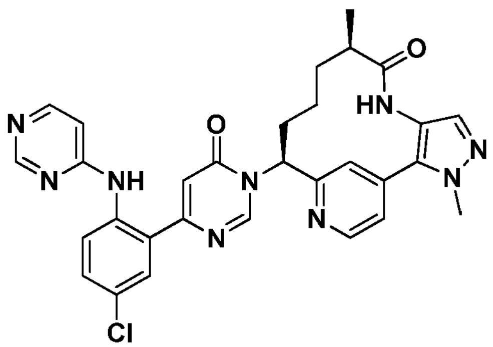

A mixture of (9*R*,13*S*)-13-[4-(2-amino-5-chlorophenyl)-6-oxo-1,6-dihydropyrimidin-1-yl]-3,9-dimethyl-3,4,7,15-tetraazatricyclo[12.3.1.0$^{2,6}$]octadeca-1(18),2(6),4,14,16-pentaen-8-one hydrochloride (0.01 g, 0.017 mmol), 4-bromopyrimidine hydrochloride (6.78 mg, 0.035 mmol), prepared as described in Example 313, in EtOH (1 ml) was microwaved at 150 °C for 30 min, cooled to rt and concentrated. Purification by reverse phase chromatography afforded (9*R*,13*S*)-13-(4-{5-chloro-2-[(pyrimidin-4-yl)amino]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one trifluoroacetate (6.3 mg, 45% yield). MS(ESI) *m/z:* 582.2 (M+H)$^+$. $^1$H NMR (500MHz, $CD_3OD$) δ 8.88 (s, 1H), 8.61 (d, *J*=5.2 Hz, 1H), 8.56 (s, 1H), 8.15 (d, *J*=6.1 Hz, 1H), 7.73 - 7.67 (m, 2H), 7.62 (s, 1H), 7.48 (dd, *J*=8.5, 2.5 Hz, 1H), 7.43 - 7.39 (m, 2H), 6.85 (d, *J*=6.9 Hz, 1H), 6.61 (s, 1H), 5.89 (d, *J*=12.9 Hz, 1H), 3.97 - 3.92 (m, 3H), 2.66 - 2.59 (m, 1H), 2.28 - 2.19 (m, 1H), 2.03 - 1.89 (m, 2H), 1.56 - 1.34 (m, 2H), 0.92 (d, *J*=7.2 Hz, 3H), 0.71 - 0.56 (m, 1H). Analytical HPLC (Method C): RT = 1.16 min, 100% purity; Factor XIa Ki = 6,000 nM.

Example 315

Preparation of (9R,13S)-13-{4-[2-(aminomethyl)-5-chlorophenyl]-6-oxo-1,6-dihydropyrimidin-1-yl}-3-(difluoromethyl)-9-methyl-3,4,7,15-tetraazatricyclo [12.3.1.0$^{2,6}$]octadeca-1(18),2(6),4,14,16-pentaen-8-one

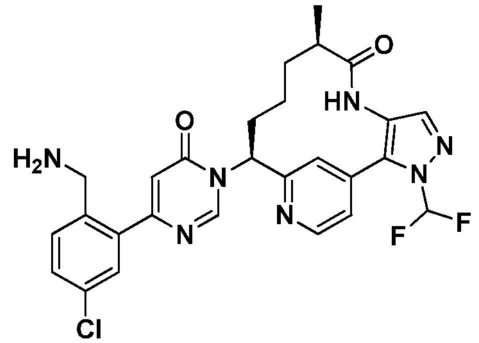

315A. Preparation of *tert*-butyl 4-chloro-2-(6-hydroxypyrimidin-4-yl)benzylcarbamate

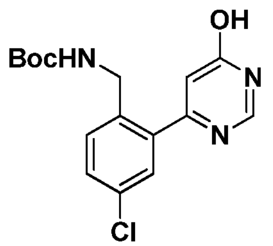

In a microwave vial, was taken *tert*-butyl 2-bromo-4-chlorobenzylcarbamate (0.78 g, 2.43 mmol) and dissolved in dioxane (10 ml) and the solution was purged with Ar for 0.5 h. To this solution was then added 4,4,4',4',5,5,5',5'-octamethyl-2,2'-bi(1,3,2-dioxaborolane) (0.93 g, 3.64 mmol), followed by KOAc (0.64 g, 6.56 mmol) and Pd(dppf)$Cl_2$·$CH_2Cl_2$ adduct (0.06 g, 0.07 mmol) and the reaction was sealed. The microwave vial was heated at 80 °C overnight. LCMS confirmed the formation of desired boronate/boronic acid and the reaction was cooled to rt. To this was added chloromethoxypyrimidine (0.351 g, 2.43 mmol) followed by addition of 2 M aq $Na_2CO_3$ (3.04 ml) and the reaction mixture was purged with Ar for 0.5 h followed by the addition of Pd(dppf)$Cl_2$·$CH_2Cl_2$ adduct (0.06 g, 0.07 mmol) and the reaction was again sealed. The reaction was heated at 120 °C for 1 h then cooled to rt and the reaction was quenched with water (100 ml). The organics were extracted with EtOAc (2x200ml), dried and evaporated to a blackish oil. Purified via a 40g silica gel ISCO column and eluting with Hex:EtOAc gave pure product as an oily mass. LCMS *m/z* = 350.08 $(M+H)^+$.

315B. Preparation of *tert*-butyl 4-chloro-2-(6-hydroxypyrimidin-4-yl)benzylcarbamate

The *tert*-butyl 4-chloro-2-(6-methoxypyrimidin-4-yl)benzylcarbamate was taken in a small vial and to this was added AcOH (1 ml) followed by 48% aq HBr (0.1 ml), sealed and heated at 80 °C for 1 h. LCMS confirmed product peak and a mass of 336 $(M+H)^+$. The solution was cooled and concentrated under a stream of $N_2$ to a oily mass and dioxane (3 ml) was added at which point solid precipitated. The solution was decanted and the residue was dissolved in DMF (3 ml) and transferred to the dioxane (2 ml) solution. To this was added $Boc_2O$ (0.1 g) followed by TEA (2 ml) and the solution was stirred at rt overnight. To this solution was added NaOH solution (IN, 5 ml) and the reaction was stirred at rt for 0.5 h. After this time, the reaction mixture was extracted with EtOAc (2x50ml). The combined organic layer was dried over $MgSO_4$, filtered and concentrated to a dark brown oil. Purification via prep HPLC using MeOH/water/TFA gradient afforded *tert*-butyl 4-chloro-2-(6-hydroxypyrimidin-4-yl)benzylcarbamate (0.05 g). $^1H$ NMR (400MHz, $CDCl_3$) δ 8.46 - 8.32 (m, 1H), 7.54 - 7.41 (m, 3H), 6.76 - 6.67 (m, 1H), 4.31 (s, 2H), 1.44 (s, 9H). MS *m/z* = 236.1 $(M+H)^+$.

315C. Preparation of (9R,13S)-13-{4-[2-(aminomethyl)-5-chlorophenyl]-6-oxo-1,6-dihydropyrimidin-1-yl}-3-(difluoromethyl)-9-methyl-3,4,7,15-tetraazatricyclo [12.3.1.0$^{2,6}$]octadeca-1(18),2(6),4,14,16-pentaen-8-one

(9R,13S)-13-{4-[2-(Aminomethyl)-5-chlorophenyl]-6-oxo-1,6-dihydropyrimidin-1-yl}-3-(difluoromethyl)-9-methyl-3,4,7,15-tetraazatricyclo[12.3.1.0$^{2,6}$]octadeca-1(18),2(6),4,14,16-pentaen-8-one (4 mg, 42% yield) was prepared as a solid, via the coupling of N-{[4-chloro-2-(6-hydroxypyrimidin-4-yl)phenyl]methyl}carbamate (0.005 g, 0.015 mmol) and (9R,13S)-13-amino-3-(difluoromethyl)-9-methyl-3,4,7,15-tetraazatricyclo[12.3.1.0$^{2,6}$]octadeca-1(18),2(6), 4,14,16-pentaen-8-one (0.005 g, 0.015 mmol) using the HATU, DBU coupling methodology described in Example 56. MS *m/z* = 554.1 (M+H)$^+$. $^1$H NMR (500MHz, DMSO-d$_6$) δ 9.53 - 9.43 (m, 1H), 9.11 - 9.04 (m, 1H), 8.82 - 8.71 (m, 1H), 8.42 - 8.23 (m, 1H), 7.98 - 7.83 (m, 1H), 7.79 - 7.70 (m, 1H), 7.68 - 7.59 (m, 1H), 7.37 - 7.01 (m, 2H), 6.84 - 6.74 (m, 1H), 6.08 - 5.91 (m, 1H), 4.19 - 3.94 (m, 2H), 2.77 - 2.63 (m, 1H), 2.34 - 2.22 (m, 1H), 2.13 - 1.86 (m, 2H), 1.57 - 1.30 (m, 2H), 0.97 - 0.74 (d 3H), 0.53 - 0.27 (m, 1H). Analytical HPLC (Method B) RT = 1.17 min, purity = 96%: Factor XIa Ki = 43 nM, Plasma Kallikrein Ki = 4,900 nM.

Example 316

Preparation of (9R,13 S)-13-{4-[5-chloro-2-(pyridin-2-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

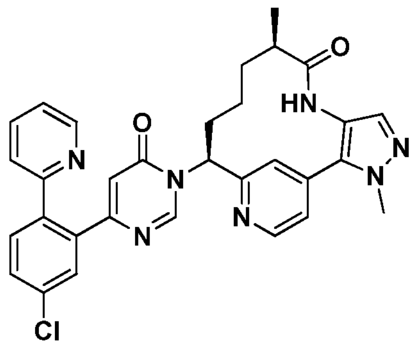

(9*R*,13*S*)-13-{4-[5-Chloro-2-(pyridin-2-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one trifluoroacetate (13.3 mg, 51% yield) was prepared in a similar manner as the procedure described in Example 303, by replacing 4-(tributylstannyl)pyridazine with 2-(tributylstannyl)pyridine (17.96 mg, 0.049 mmol), and the reaction time was 2 h at 45 °C and then 6 h at 90 °C. MS(ESI) *m/z:* 566.15 (M+H)$^+$. $^1$H NMR (500MHz, DMSO-d$_6$) δ 9.23 (s, 1H), 8.72 (br. s., 1H), 8.67 (d, *J*=4.9 Hz, 1H), 8.52 (d, *J*=4.0 Hz, 1H), 7.82 (t, *J*=7.8 Hz, 1H), 7.72 (s, 1H), 7.67 - 7.62 (m, 3H), 7.56 (d, *J*=4.9 Hz, 1H), 7.49 - 7.36 (m, 3H), 6.25 (s, 1H), 5.86 (d, *J*=10.1 Hz, 1H), 4.00 (s, 3H), 2.68 - 2.59 (m, 1H), 2.30 - 2.21 (m, 1H), 2.14 - 2.03 (m, 1H), 1.86 - 1.76 (m, 1H), 1.50 - 1.40 (m, 1H), 1.37 - 1.26 (m, 1H), 0.87 (d, *J*=7.0 Hz, 3H), 0.52 - 0.33 (m, 1H). Analytical HPLC (Method C): RT = 1.12 min, 100% purity; Factor XIa Ki = 350 nM.

Preparation of Examples 322 to 352

The following compounds were made in a parallel manner using the following procedure: Reagents were weighed into BIOTAGE® 0.5 - 2 mL microwave vials. Stock solutions were made for reagent addition : Dissolved 472.9 mg core in 18.6 mL 1,4-dioxane (0.04M). Dissolved 265.7 mg potassium carbonate in 6.2 mL water 0.3 M). To each microwave vial containing reagent was added Si-DPP-Pd (12.40 mg, 3.72 μmol) via ArgoScoop, 0.600 mL core solution, and 0.200 mL potassium carbonate solution. Queued the reactions to run on the BIOTAGE® Initiator (400 W) microwave for 30 min at 120 °C with 10 seconds of prestirring and using a fixed hold time.

Upon completion of the microwave run, reaction mixtures were concentrated, then redissolved in 1.8 mL DMF and filtered through a 45 μM syringe filter. Resulting clear solutions were purified via preparative LC/MS with the following conditions: Column: XBridge C18, 19 x 100 mm, 5-μm particles; Mobile Phase A: 5:95 acetonitrile: water with 0.1% trifluoroacetic acid; Mobile Phase B: 95:5 acetonitrile: water with 0.1% trifluoroacetic acid; Gradient: 40-80% B over 10 min, then a 5-minute hold at 100% B; Flow: 20 mL/min. Fractions containing the desired product were combined and dried via centrifugal evaporation. Gradient varied for each reaction depending on polarity of compound.

Compound purity was assigned based on the methods below.

Method A: Column: Waters Acquity UPLC BEH C18, 2.1 x 50 mm, 1.7-μm particles; Mobile Phase A: 5:95 acetonitrile:water with 10 mM ammonium acetate; Mobile Phase B: 95:5 acetonitrile:water with 10 mM ammonium acetate; Temperature: 50 °C; Gradient: 0-100% B over 3 min, then a 0.75-minute hold at 100% B; Flow: 1.11 mL/min; Detection: UV at 220 nm.

Method B: Column: Waters Acquity UPLC BEH C18, 2.1 x 50 mm, 1.7-μm particles; Mobile Phase A: 5:95 acetonitrile:water with 0.1% trifluoroacetic acid; Mobile Phase B: 95:5 acetonitrile:water with 0.1% trifluoroacetic acid; Temperature: 50 °C; Gradient: 0-100% B over 3 min, then a 0.75-minute hold at 100% B; Flow: 1.11 mL/min; Detection: UV at 220 nm.

Example 322

Preparation of (9R,13S)-13-{4-[5-chloro-2-(4-methylphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

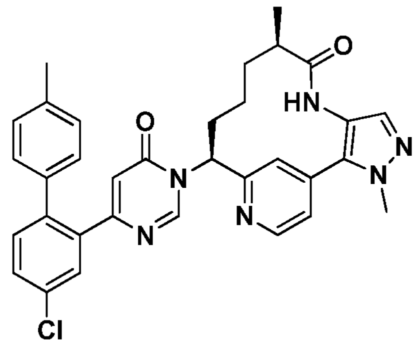

MS(ESI) *m/z:* 579.1 (M+H)$^+$. $^1$H NMR (500MHz, DMSO-d$_6$) δ 9.14 (s, 1H), 8.82 (s, 1H), 8.62 (d, *J*=5.2 Hz, 1H), 7.61 (d, *J*=2.4 Hz, 1H), 7.57 (s, 1H), 7.53 - 7.49 (m, 2H), 7.40 (s, 1H), 7.34 (d, *J*=8.5 Hz, 1H), 7.10 - 7.03 (m, 4H), 5.99 (s, 1H), 5.79 (d, *J*=10.1 Hz, 1H), 3.94 (s, 3H), 2.61 - 2.53 (m, 1H), 2.26 - 2.17 (m, 4H), 2.07 - 1.99 (m, 1H), 1.80 - 1.72 (m, 1H), 1.44 - 1.35 (m, 1H), 1.31 - 1.21 (m, 1H), 0.81 (d, *J*=6.7 Hz, 3H), 0.41 - 0.26 (m, 1H). Analytical HPLC (Method A): RT =2.02 min, purity =98.7%; Factor XIa Ki = 180 nM.

Example 323

Preparation of (9R,13S)-13-{4-[5-chloro-2-(3-chlorophenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

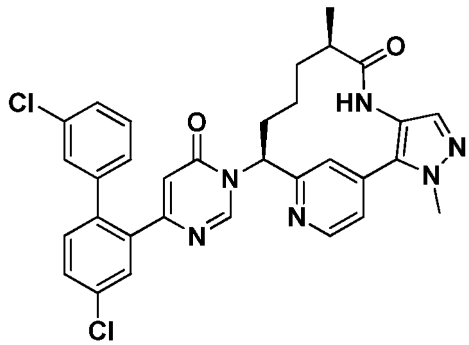

MS(ESI) *m/z:* 599.1 (M+H)$^+$; $^1$H NMR (500MHz, DMSO-d$_6$) δ 9.15 (s, 1H), 8.76 (s, 1H), 8.60 (d, *J*=4.9 Hz, 1H), 7.63 (d, *J*=2.1 Hz, 1H), 7.59 - 7.54 (m, 2H), 7.50 (dd, *J*=5.2, 0.9 Hz, 1H), 7.43 - 7.39 (m, 2H), 7.34 - 7.26 (m, 2H), 7.23 (s, 1H), 7.09 (d, *J*=7.3 Hz, 1H), 6.16 (s, 1H), 5.79 (d, *J*=11.0 Hz, 1H), 3.94 (s, 3H), 2.61 - 2.53 (m, 1H), 2.27 - 2.17 (m, 1H), 2.07 - 1.97 (m, 1H), 1.80 - 1.70 (m, 1H), 1.44 - 1.34 (m, 1H), 1.31 - 1.20 (m, 1H), 0.81 (d, *J*=6.7 Hz, 3H), 0.43 - 0.29 (m, 1H). Analytical HPLC (Method A): RT =2.03 min, purity =94.8%; Factor XIa Ki = 7,500 nM.

Example 324

Preparation of (9R,13S)-13-{4-[5-chloro-2-(3-methoxyphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

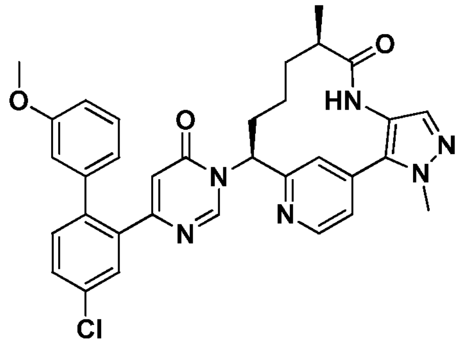

MS(ESI) *m/z:* 595.1 (M+H)$^+$.; $^1$H NMR (500MHz, DMSO-$d_6$) δ 9.15 (s, 1H), 8.81 (s, 1H), 8.61 (d, *J*=5.2 Hz, 1H), 7.61 (d, *J*=2.1 Hz, 1H), 7.58 (s, 1H), 7.55 - 7.49 (m, 2H), 7.41 - 7.37 (m, 2H), 7.18 (t, *J*=7.9 Hz, 1H), 6.80 (dd, *J*=8.2, 2.1 Hz, 1H), 6.73 (d, *J*=7.6 Hz, 1H), 6.69 (s, 1H), 6.04 (s, 1H), 5.78 (d, *J*=10.1 Hz, 1H), 3.94 (s, 3H), 3.58 (s, 3H), 2.60 - 2.53 (m, 1H), 2.27 - 2.18 (m, 1H), 2.07 - 1.98 (m, 1H), 1.81 - 1.71 (m, 1H), 1.44 - 1.34 (m, 1H), 1.31 - 1.21 (m, 1H), 0.81 (d, *J*=6.7 Hz, 3H), 0.43 - 0.29 (m, 1H); Analytical HPLC (Method A): RT =1.91 min, purity =97.5%; Factor XIa Ki = 550 nM.

Example 325

Preparation of (9R,13S)-13-{4-[5-chloro-2-(2-methylphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

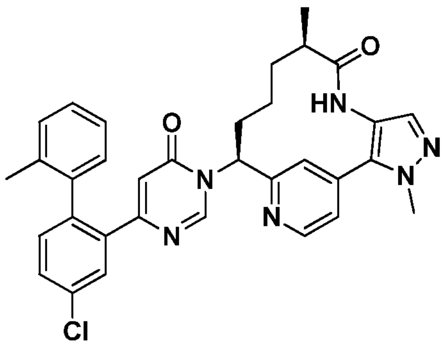

MS(ESI) *m/z*: 579.1 (M+H)$^+$; $^1$H NMR (500MHz, DMSO-$d_6$) δ 9.20 (s, 1H), 8.91 (s, 1H), 8.68 (d, *J*=5.2 Hz, 1H), 7.86 (d, *J*=2.4 Hz, 1H), 7.63 - 7.55 (m, 3H), 7.46 (s, 1H), 7.31 - 7.18 (m, 4H), 7.11 (s, 1H), 5.85 - 5.78 (m, 2H), 4.00 (d, *J*=3.1 Hz, 3H), 2.67 - 2.60 (m, 1H), 2.32 - 2.22 (m, 1H), 2.13 - 2.03 (m, 1H), 1.95 (d, *J*=12.8 Hz, 3H), 1.86 - 1.76 (m, 1H), 1.49 - 1.39 (m, 1H), 1.37 - 1.28 (m, 1H), 0.87 (d, *J*=6.7 Hz, 3H), 0.46 - 0.31 (m, 1H); Analytical HPLC (Method A): RT =2.05 min, purity =100%; Factor XIa Ki = 3,900.

Example 326

Preparation of (9R,13 S)-13-(4-{5-chloro-2-[4-(trifluoromethoxy)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one

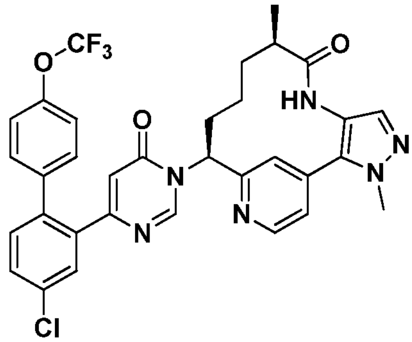

MS(ESI) *m/z*: 649 $(M+H)^+$; $^1H$ NMR (500MHz, DMSO-$d_6$) δ 9.21 (s, 1H), 8.83 (s, 1H), 8.66 (d, *J*=4.9 Hz, 1H), 7.71 (d, *J*=2.1 Hz, 1H), 7.67 - 7.61 (m, 2H), 7.59 (d, *J*=4.9 Hz, 1H), 7.51 - 7.46 (m, 2H), 7.38 - 7.30 (m, 4H), 6.22 (s, 1H), 5.88 (d, *J*=10.4 Hz, 1H), 4.01 (s, 3H), 2.69 - 2.60 (m, 1H), 2.32 - 2.23 (m, 1H), 2.16 - 2.06 (m, 1H), 1.86 - 1.77 (m, 1H), 1.52 - 1.42 (m, 1H), 1.38 - 1.28 (m, 1H), 0.88 (d, *J*=7.0 Hz, 3H), 0.48 - 0.34 (m, 1H); Analytical HPLC (Method A): RT =2.14 min, purity =100%.; Factor XIa Ki = 640.

Example 327

Preparation of (9R,13S)-13-{4-[5-chloro-2-(2-chlorophenyl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

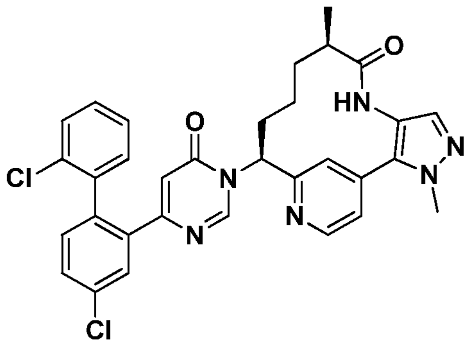

MS(ESI) *m/z:* 599 $(M+H)^+$; $^1H$ NMR (500MHz, DMSO-$d_6$) δ 9.20 (s, 1H), 8.89 (d, *J*=5.5 Hz, 1H), 8.70 - 8.66 (m, 1H), 7.85 (s, 1H), 7.66 - 7.61 (m, 2H), 7.57 (d, *J*=4.6 Hz, 1H), 7.52 - 7.46 (m, 2H), 7.43 - 7.29 (m, 4H), 5.93, 5.91 (2s, 1H), 5.84 (d, *J*=11.0 Hz, 1H), 4.01, 4.00 (2s, 3H), 2.66 - 2.60 (m, 1H), 2.32 - 2.22 (m, 1H), 2.13 - 2.04 (m, 1H), 1.85 - 1.75 (m, 1H), 1.50 - 1.40 (m, 1H), 1.37 - 1.27 (m, 1H), 0.87 (d, *J*=6.7 Hz, 3H), 0.45 - 0.31 (m, 1H); Analytical HPLC (Method A): RT =2.01 min, purity =100%; Factor XIa Ki = 3,300 nM.

Example 329

Preparation of (9R,13 S)-13-(4-{5-chloro-2-[4-(trifluoromethyl)phenyl]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one

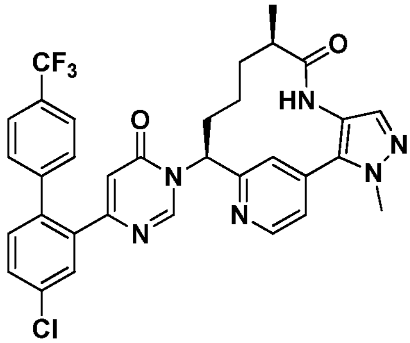

MS(ESI) *m/z*: 633.1 $(M+H)^+$; $^1H$ NMR (500MHz, DMSO-$d_6$) δ 9.21 (s, 1H), 8.82 (s, 1H), 8.64 (d, *J*=4.9 Hz, 1H), 7.74 (d, *J*=2.1 Hz, 1H), 7.71 - 7.64 (m, 4H), 7.59 (d, *J*=4.9 Hz, 1H), 7.53 - 7.44 (m, 4H), 6.26 (s, 1H), 5.88 (d, *J*=9.8 Hz, 1H), 4.01 (s, 3H), 2.68 - 2.59 (m, 1H), 2.32 - 2.24 (m, 1H), 2.15 - 2.06 (m, 1H), 1.86 - 1.77 (m, 1H), 1.51 - 1.42 (m, 1H), 1.38 - 1.28 (m, 1H), 0.88 (d, *J*=6.7 Hz, 3H), 0.48 - 0.32 (m, 1H); Analytical HPLC (Method A): RT =2.09 min, purity =100%; Factor XIa Ki = 380 nM.

Example 330

Preparation of (9R,13 S)-13-(4-{5-chloro-2-[4-(propan-2-ylsulfanyl)phenyl]phenyl}-6-oxo-1,6-dihydropyrimidin-1-yl)-3,9-dimethyl-3,4,7,15-tetraazatricyclo[12.3.1.0$^{2,6}$] octadeca-1(18),2(6),4,14,16-pentaen-8-one

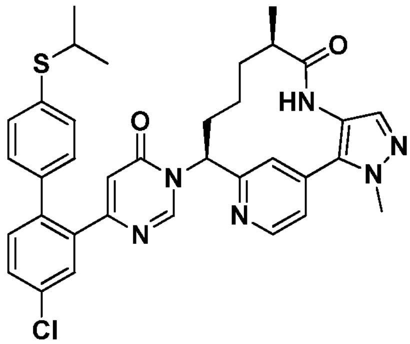

MS(ESI) *m/z:* 639.1 $(M+H)^+$; $^1H$ NMR (500MHz, DMSO-$d_6$) δ 9.15 (s, 1H), 8.80 (s, 1H), 8.62 (d, *J*=5.2 Hz, 1H), 7.65 (d, *J*=2.1 Hz, 1H), 7.60 (s, 1H), 7.57 - 7.52 (m, 2H), 7.43 - 7.38 (m, 2H), 7.25 (d, *J*=7.9 Hz, 2H), 7.12 (d, *J*=8.2 Hz, 2H), 6.08 (s, 1H), 5.82 (d, *J*=9.8 Hz, 1H), 3.96 (s, 3H), 3.49 - 3.39 (m, 1H), 2.62 - 2.55 (m, 1H), 2.27 - 2.17 (m, 1H), 2.10 - 2.01 (m, 1H), 1.81 - 1.72 (m, 1H), 1.46 - 1.36 (m, 1H), 1.33 - 1.23 (m, 1H), 1.18 (dd, *J*=6.6, 2.0 Hz, 6H), 0.83 (d, *J*=7.0 Hz, 3H), 0.42 - 0.28 (m, 1H); Analytical HPLC (Method A): RT =2.25 min, purity =100%; Factor XIa Ki = 400 nM.

Example 331

Preparation of 4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzene-1-sulfonamide

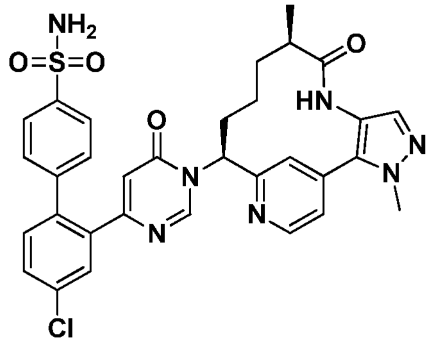

MS(ESI) *m/z:* 644 $(M+H)^+$; $^1H$ NMR (500MHz, DMSO-$d_6$) δ 9.21 (s, 1H), 8.85 (s, 1H), 8.69 (d, *J*=5.2 Hz, 1H), 7.78 (d, *J*=8.2 Hz, 2H), 7.73 (d, *J*=2.1 Hz, 1H), 7.67 - 7.64 (m, 2H), 7.58 (d, *J*=5.2 Hz, 1H), 7.52 - 7.40 (m, 6H), 6.25 (s, 1H), 5.89 (d, *J*=10.7 Hz, 1H), 4.01 (s, 3H), 2.68 - 2.60 (m, 1H), 2.33 - 2.24 (m, 1H), 2.15 - 2.06 (m, 1H), 1.88 - 1.78 (m, 1H), 1.51 - 1.42 (m, 1H), 1.39 - 1.28 (m, 1H), 0.88 (d, *J*=6.7 Hz, 3H), 0.48 - 0.33 (m, 1H); Analytical HPLC (Method A): RT =1.54 min, purity =100%; Factor XIa Ki = 130 nM.

Example 332

Preparation of (9R,13S)-13-(4-{5-chloro-2-[4-(difluoromethoxy)phenyl]phenyl}-6-oxo-1,6-dihydropyrimidin-1-yl)-3,9-dimethyl-3,4,7,15-tetraazatricyclo[12.3.1.$0^{2,6}$]octadeca-1(18),2(6),4,14,16-pentaen-8-one

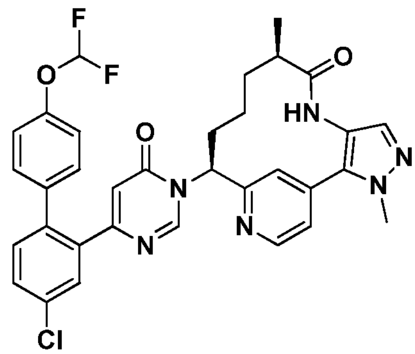

MS(ESI) *m/z:* 631 $(M+H)^+$; $^1H$ NMR (500MHz, DMSO-$d_6$) δ 9.21 (s, 1H), 8.86 (s, 1H), 8.67 (d, *J*=4.9 Hz, 1H), 7.70 (d, *J*=2.1 Hz, 1H), 7.65 (s, 1H), 7.63 - 7.57 (m, 2H), 7.49 - 7.42 (m, 2H), 7.31 - 7.26 (m, 3H), 7.17 - 7.11 (m, 2H), 6.17 (s, 1H), 5.88 (d, *J*=9.8 Hz, 1H), 4.01 (s, 3H), 2.69 - 2.60 (m, 1H), 2.34 - 2.24 (m, 1H), 2.16 - 2.06 (m, 1H), 1.88 - 1.79 (m, 1H), 1.51 - 1.42 (m, 1H), 1.39 - 1.28 (m, 1H), 0.88 (d, *J*=6.7 Hz, 3H), 0.48 - 0.33 (m, 1H); Analytical HPLC (Method A): RT =1.97 min, purity =100%; Factor XIa Ki = 180 nM.

Example 333

Preparation of N-[3-(4-chloro-2-{1-[(9R,13 S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)phenyl]methanesulfonamide

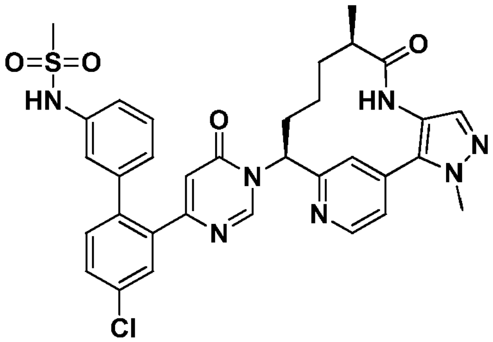

MS(ESI) *m/z:* 658 $(M+H)^+$; $^1H$ NMR (500MHz, DMSO-$d_6$) δ 9.20 (s, 1H), 8.87 (s, 1H), 8.69 (d, *J*=5.2 Hz, 1H), 7.70 (d, *J*=2.4 Hz, 1H), 7.66 - 7.60 (m, 3H), 7.58 (d, *J*=5.2 Hz, 1H), 7.48 - 7.44 (m, 2H), 7.38 - 7.33 (m, 1H), 7.16 (dd, *J*=8.1, 1.1 Hz, 1H), 7.06 (d, *J*=7.6 Hz, 1H), 7.03 (s, 1H), 6.10 (s, 1H), 5.86 (d, *J*=9.8 Hz, 1H), 4.01 (s, 3H), 2.80 (s, 3H), 2.70 - 2.60 (m, 1H), 2.34 - 2.24 (m, 1H), 2.15 - 2.05 (m, 1H), 1.88 - 1.78 (m, 1H), 1.51 - 1.42 (m, 1H), 1.39 - 1.28 (m, 1H), 0.89 (d, *J*=7.0 Hz, 3H), 0.49 - 0.35 (m, 1H); Analytical HPLC (Method A): RT =1.68 min, purity =100%; Factor XIa Ki = 1,200 nM.

Example 334

Preparation of 3-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzonitrile

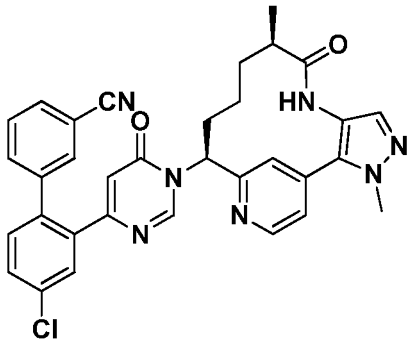

MS(ESI) *m/z:* 590.3 $(M+H)^+$; $^1H$ NMR (500MHz, DMSO-$d_6$) δ 9.21 (s, 1H), 8.78 (s, 1H), 8.68 (d, *J*=5.2 Hz, 1H), 7.80 (dd, *J*=5.3, 2.3 Hz, 1H), 7.74 - 7.70 (m, 2H), 7.68 - 7.64 (m, 2H), 7.59 - 7.51 (m, 4H), 7.47 (s, 1H), 6.31 (s, 1H), 5.86 (d, *J*=10.4 Hz, 1H), 4.01 (s, 3H), 2.70 - 2.59 (m, 1H), 2.33 - 2.23 (m, 1H), 2.14 - 2.04 (m, 1H), 1.86 - 1.77 (m, 1H), 1.51 - 1.41 (m, 1H), 1.38 - 1.28 (m, 1H), 0.88 (d, *J*=7.0 Hz, 3H), 0.50 - 0.36 (m, 1H); Analytical HPLC (Method A): RT =1.8 min, purity =100%; Factor XIa Ki = 1,600 nM.

Example 335

Preparation of (9R,13S)-13-(4-{5-chloro-2-[3-(trifluoromethoxy)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one

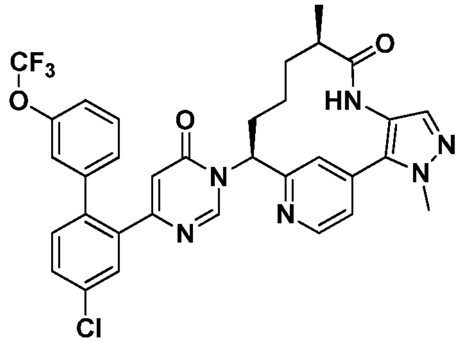

MS(ESI) *m/z*: 649 $(M+H)^+$; $^1H$ NMR (500MHz, DMSO-$d_6$) δ 9.20 (s, 1H), 8.84 (s, 1H), 8.67 (d, *J*=5.2 Hz, 1H), 7.69 (d, *J*=2.1 Hz, 1H), 7.67 - 7.62 (m, 2H), 7.58 (d, *J*=5.2 Hz, 1H), 7.52 (dt, *J*=8.1, 3.9 Hz, 2H), 7.47 (s, 1H), 7.36 (d, *J*=7.9 Hz, 1H), 7.32 (d, *J*=8.2 Hz, 1H), 7.09 (s, 1H), 6.23 (s, 1H), 5.88 (d, *J*=10.4 Hz, 1H), 4.00 (s, 3H), 2.69 - 2.60 (m, 1H), 2.33 - 2.24 (m, 1H), 2.14 - 2.05 (m, 1H), 1.82 - 1.72 (m, 1H), 1.51 - 1.41 (m, 1H), 1.37 - 1.27 (m, 1H), 0.88 (d, *J*=7.0 Hz, 3H), 0.49 - 0.36 (m, 1H); Analytical HPLC (Method A): RT =2.13 min, purity =100%; Factor XIa Ki = 3,700 nM.

Example 336

Preparation of (9R,13S)-13-{4-[5-chloro-2-(3-methanesulfonylphenyl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

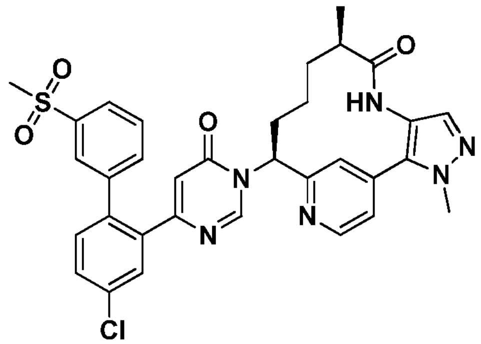

MS(ESI) *m/z*: 643 $(M+H)^+$; $^1H$ NMR (500MHz, DMSO-$d_6$) δ 9.24, 9.21 (s, 1H), 9.02, 8.79 (2s, 1H), 8.71 - 8.63 (m, *J*=14.0, 4.9 Hz, 1H), 8.17 - 7.43 (m, 9H), 7.07, 6.32 (2s, 1H), 5.99 - 5.78 (m, 1H), 4.01 (d, *J*=1.8 Hz, 3H), 3.91, 3.11 (2s, 3H), 2.70 - 2.59 (m, 1H), 2.41 - 2.21 (m, 1H), 2.18 - 2.04 (m, 1H), 1.99 - 1.75 (m, 1H), 1.54 - 1.26 (m, 2H), 0.93 - 0.85 (m, 3H), 0.55 - 0.35 (m, 1H); Analytical HPLC (Method A): RT =1.65 min, purity =100%; Factor XIa Ki = 1,200 nM.

Example 337

Preparation of methyl 4-(4-chloro-2-{1-[(9R,13S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)benzoate

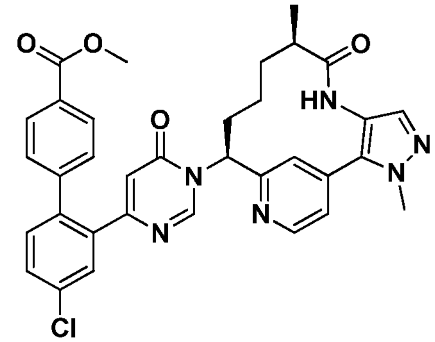

MS(ESI) *m/z:* 623.1 (M+H)$^+$; $^1$H NMR (500MHz, DMSO-$d_6$) δ 9.14 (s, 1H), 8.74 (s, 1H), 8.60 (d, *J*=4.9 Hz, 1H), 7.84 (d, *J*=8.2 Hz, 2H), 7.66 (d, *J*=2.1 Hz, 1H), 7.60 - 7.56 (m, 2H), 7.51 (d, *J*=5.2 Hz, 1H), 7.45 - 7.38 (m, 2H), 7.31 (d, *J*=8.2 Hz, 2H), 6.12 (s, 1H), 5.82 - 5.75 (m, 1H), 3.94 (s, 3H), 3.80 (s, 3H), 2.61 - 2.53 (m, 1H), 2.26 - 2.16 (m, 1H), 2.09 - 1.97 (m, 1H), 1.80 - 1.71 (m, 1H), 1.44 - 1.19 (m, 2H), 0.81 (d, *J*=6.7 Hz, 3H), 0.42 - 0.25 (m, 1H); Analytical HPLC (Method A): RT =1.91 min, purity =98.6%; Factor XIa Ki = 750 nM.

Example 338

Preparation of (9R,13S)-13-{4-[5-chloro-2-(3-methylphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

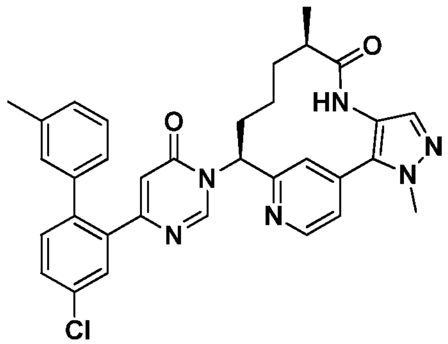

MS(ESI) *m/z*: 579.1 (M+H)$^+$; $^1$H NMR (500MHz, DMSO-$d_6$) δ 9.22 (s, 1H), 8.86 (s, 1H), 8.68 (d, *J*=5.2 Hz, 1H), 7.68 (d, *J*=2.1 Hz, 1H), 7.65 (s, 1H), 7.62 - 7.56 (m, 2H), 7.47 (s, 1H), 7.44 (d, *J*=8.5 Hz, 1H), 7.24 - 7.18 (m, 1H), 7.13 (d, *J*=7.6 Hz, 1H), 7.05 (s, 1H), 7.00 (d, *J*=7.6 Hz, 1H), 6.09 (s, 1H), 5.85 (d, *J*=10.4 Hz, 1H), 4.01 (s, 3H), 2.67 - 2.60 (m, 1H), 2.35 - 2.22 (m, 4H), 2.14 - 2.05 (m, 1H), 1.88 - 1.78 (m, 1H), 1.51 - 1.42 (m, 1H), 1.38 - 1.28 (m, 1H), 0.88 (d, *J*=7.0 Hz, 3H), 0.51 - 0.37 (m, 1H); Analytical HPLC (Method A): RT =2.03 min, purity =100%; Factor XIa Ki = 1,400 nM.

Example 339

Preparation of 4-(4-chloro-2-{1-[(9R,13S)-3,9-dimethyl-8-oxo-3,4,7,15-tetraazatricyclo [12.3.1.0$^{2,6}$]octadeca-1(18),2(6),4,14,16-pentaen-13-yl]-6-oxo-1,6-dihydropyrimidin-4-yl}phenyl)benzonitrile

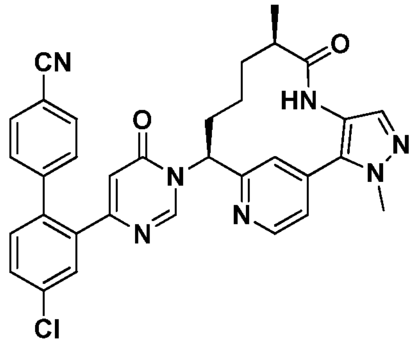

MS(ESI) *m/z:* 590.1 (M+H)$^+$; $^1$H NMR (500MHz, DMSO-$d_6$) δ 9.22 (s, 1H), 8.79 (s, 1H), 8.67 (d, *J*=4.9 Hz, 1H), 7.80 (d, *J*=8.2 Hz, 2H), 7.73 (d, *J*=2.1 Hz, 1H), 7.69 - 7.64 (m, 2H), 7.59 (d, *J*=5.2 Hz, 1H), 7.50 (d, *J*=8.2 Hz, 1H), 7.47 (s, 1H), 7.43 (d, *J*=8.5 Hz, 2H), 6.29 (s, 1H), 5.87 (d, *J*=10.1 Hz, 1H), 4.01 (s, 3H), 2.69 - 2.60 (m, 1H), 2.33 - 2.23 (m, 1H), 2.15 - 2.06 (m, 1H), 1.87 - 1.78 (m, 1H), 1.52 - 1.42 (m, 1H), 1.38 - 1.28 (m, 1H), 0.88 (d, *J*=6.7 Hz, 3H), 0.49 - 0.33 (m, 1H); Analytical HPLC (Method A): RT =1.8 min, purity =100%; Factor XIa Ki = 290 nM.

Example 340

Preparation of (9R,13S)-13-{4-[5-chloro-2-(1-methyl-1H-indol-5-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

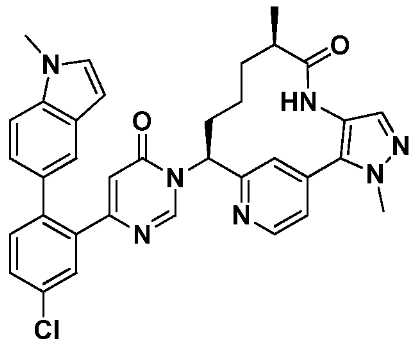

MS(ESI) *m/z:* 618.1 (M+H)$^+$; $^1$H NMR (500MHz, DMSO-$d_6$) δ 9.20 (s, 1H), 8.90 (s, 1H), 8.68 (d, *J*=5.2 Hz, 1H), 7.72 (d, *J*=2.1 Hz, 1H), 7.61 (s, 1H), 7.60 - 7.55 (m, 2H), 7.47 - 7.43 (m, 3H), 7.39 - 7.33 (m, 2H), 6.96 (dd, *J*=8.5, 1.2 Hz, 1H), 6.40 (d, *J*=3.1 Hz, 1H), 5.98 (s, 1H), 5.82 (d, *J*=10.7 Hz, 1H), 4.00 (s, 3H), 3.79 (s, 3H), 2.66 - 2.59 (m, 1H), 2.33 - 2.24 (m, 1H), 2.13 - 2.03 (m, 1H), 1.85 - 1.77 (m, 1H), 1.49 - 1.40 (m, 1H), 1.37 - 1.27 (m, 1H), 0.87 (d, *J*=7.0 Hz, 3H), 0.45 - 0.32 (m, 1H); Analytical HPLC (Method A): RT =2.02 min, purity=100%; Factor XIa Ki = 1,300 nM.

Example 341

Preparation of (9R,13S)-13-{4-[5-chloro-2-(isoquinolin-5-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

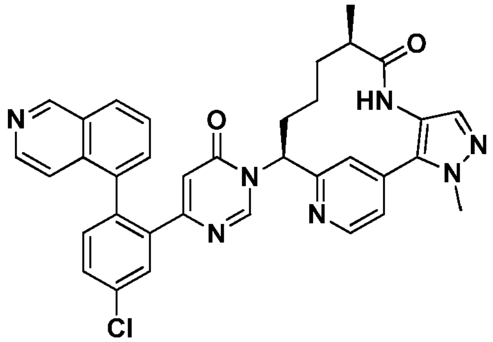

MS(ESI) *m/z:* 616 $(M+H)^+$; $^1H$ NMR (500MHz, DMSO-$d_6$) δ 9.48 (d, *J*=16.8 Hz, 1H), 9.17 (s, 1H), 8.66 - 8.58 (m, 2H), 8.43 (br. s., 1H), 8.24 (t, *J*=9.3 Hz, 1H), 7.90 (t, *J*=1.8 Hz, 1H), 7.82 - 7.69 (m, 3H), 7.56 - 7.51 (m, 2H), 7.48 (dd, *J*=8.2, 3.4 Hz, 1H), 7.45 - 7.38 (m, 2H), 6.00 (d, *J*=9.5 Hz, 1H), 5.69 (d, *J*=10.4 Hz, 1H), 3.99 (s, 3H), 2.63 - 2.57 (m, 1H), 2.19 - 1.96 (m, 2H), 1.73 - 1.59 (m, 1H), 1.45 - 1.34 (m, 1H), 1.31 - 1.20 (m, 1H), 0.85 (d, *J*=5.2 Hz, 3H), 0.41 - 0.28 (m, 1H); Analytical HPLC (Method B): RT =1.32 min, purity =100%; Factor XIa Ki = 6,500 nM.

Example 342

Preparation of methyl 3-(4-chloro-2-{1-[(9R,13S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)benzoate

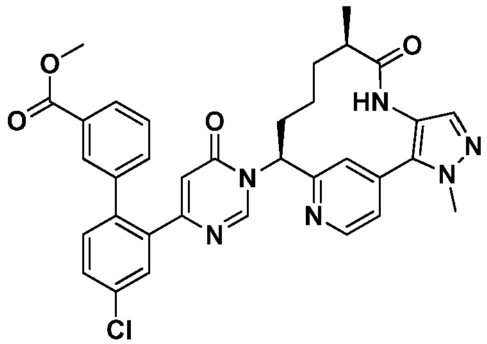

MS(ESI) *m/z:* 623.2 $(M+H)^+$; $^1H$ NMR (500MHz, DMSO-$d_6$) δ 9.21 (s, 1H), 8.80 (s, 1H), 8.65 (d, *J*=5.2 Hz, 1H), 7.93 - 7.89 (m, 1H), 7.79 (s, 1H), 7.71 (d, *J*=2.1 Hz, 1H), 7.66 - 7.63 (m, 2H), 7.58 (d, *J*=5.2 Hz, 1H), 7.54 - 7.46 (m, 4H), 6.23 (s, 1H), 5.86 (d, *J*=9.8 Hz, 1H), 4.01 (s, 3H), 3.78 (s, 3H), 2.67 - 2.59 (m, 1H), 2.31 - 2.22 (m, 1H), 2.14 - 2.05 (m, 1H), 1.83 - 1.74 (m, 1H), 1.51 - 1.41 (m, 1H), 1.37 - 1.27 (m, 1H), 0.88 (d, *J*=7.0 Hz, 3H), 0.50 - 0.36 (m, 1H); Analytical HPLC (Method A): RT =1.87 min, purity =100%; Factor XIa Ki = 2,200 nM.

Example 343

Preparation of N-[4-(4-chloro-2-{1-[(9R,13 S)-3,9-dimethyl-8-oxo-3,4,7,15-tetraazatricyclo[12.3.1.$0^{2,6}$]octadeca-1(18),2(6),4,14,16-pentaen-13-yl]-6-oxo-1,6-dihydropyrimidin-4-yl}phenyl)phenyl]methanesulfonamide

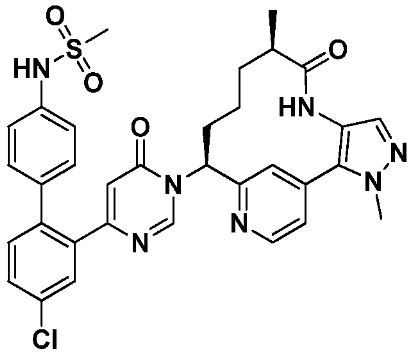

MS(ESI) *m/z:* 657.9 $(M+H)^+$; $^1H$ NMR (500MHz, DMSO-$d_6$) δ 9.21 (s, 1H), 8.86 (s, 1H), 8.70 (d, *J*=5.2 Hz, 1H), 7.70 (d, *J*=2.4 Hz, 1H), 7.65 (s, 1H), 7.62 - 7.56 (m, 2H), 7.50 - 7.42 (m, 2H), 7.30 - 7.25 (m, 1H), 7.22 - 7.14 (m, 4H), 6.11 (s, 1H), 5.86 (d, *J*=8.9 Hz, 1H), 4.01 (s, 3H), 2.99 (s, 3H), 2.68 - 2.61 (m, 1H), 2.34 - 2.23 (m, 1H), 2.16 - 2.06 (m, 1H), 1.88 - 1.78 (m, 1H), 1.53 - 1.28 (m, 2H), 0.88 (d, *J*=7.0 Hz, 3H), 0.51 - 0.33 (m, 1H); Analytical HPLC (Method B): RT =1.63 min, purity =97.8%; Factor XIa Ki = 450 nM.

Example 344

Preparation of (9R,13 S)-13-(4-{5-chloro-2-[3-(trifluoromethyl)phenyl]phenyl}-6-oxo-1,6-dihydropyrimidin-1-yl)-3,9-dimethyl-3,4,7,15-tetraazatricyclo[12.3.1.0$^{2,6}$]octadeca-1(18),2(6),4,14,16-pentaen-8-one

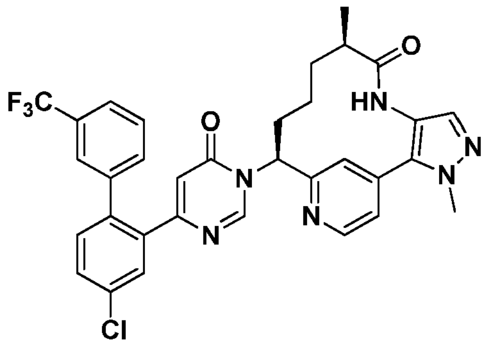

MS(ESI) *m/z:* 633 $(M+H)^+$; $^1H$ NMR (500MHz, DMSO-$d_6$) δ 9.21 (s, 1H), 8.79 (s, 1H), 8.65 (d, *J*=5.2 Hz, 1H), 7.73 - 7.64 (m, 4H), 7.61 - 7.54 (m, 4H), 7.52 - 7.46 (m, 2H), 6.30 (s, 1H), 5.86 (d, *J*=10.4 Hz, 1H), 4.01 (s, 3H), 2.67 - 2.59 (m, 1H), 2.33 - 2.23 (m, 1H), 2.14 - 2.05 (m, *J*=12.1, 12.1 Hz, 1H), 1.82 - 1.73 (m, 1H), 1.50 - 1.41 (m, 1H), 1.37 - 1.27 (m, 1H), 0.88 (d, *J*=6.7 Hz, 3H), 0.52 - 0.38 (m, 1H); Analytical HPLC (Method A): RT =2.06 min, purity =100%; Factor XIa Ki = 4,800 nM.

Example 345

Preparation of (9R,13S)-13-{4-[5-chloro-2-(4-methoxyphenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

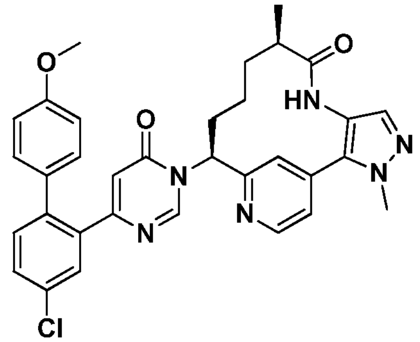

MS(ESI) *m/z:* 595 (M+H)$^+$; $^1$H NMR (500MHz, DMSO-$d_6$) δ 9.22 (s, 1H), 8.89 (s, 1H), 8.69 (d, *J*=5.2 Hz, 1H), 7.68 - 7.64 (m, 2H), 7.60 - 7.56 (m, 2H), 7.47 (s, 1H), 7.41 (d, *J*=8.2 Hz, 1H), 7.15 (d, *J*=8.5 Hz, 2H), 6.90 (d, *J*=8.9 Hz, 2H), 6.08 (s, 1H), 5.87 (d, *J*=10.1 Hz, 1H), 4.01 (s, 3H), 3.76 (s, 3H), 2.68 - 2.60 (m, 1H), 2.34 - 2.26 (m, 1H), 2.16 - 2.06 (m, 1H), 1.89 - 1.79 (m, 1H), 1.51 - 1.42 (m, 1H), 1.39 - 1.28 (m, 1H), 0.88 (d, *J*=7.0 Hz, 3H), 0.48 - 0.35 (m, 1H); Analytical HPLC (Method A): RT =1.89 min, purity =100%; Factor XIa Ki = 350 nM.

Example 346

Preparation of (9R,13S)-13-{4-[5-chloro-2-(4-chlorophenyl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

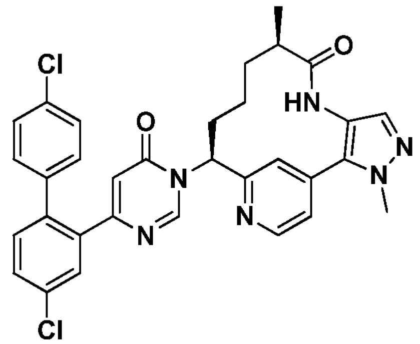

MS(ESI) *m/z*: 599 (M+H)$^+$; $^1$H NMR (500MHz, DMSO-$d_6$) δ 9.22 (s, 1H), 8.85 (s, 1H), 8.68 (d, *J*=4.9 Hz, 1H), 7.70 (d, *J*=2.1 Hz, 1H), 7.66 (s, 1H), 7.63 (dd, *J*=8.2, 2.1 Hz, 1H), 7.59 (d, *J*=5.2 Hz, 1H), 7.48 - 7.44 (m, 2H), 7.40 (d, *J*=8.5 Hz, 2H), 7.25 (d, *J*=8.2 Hz, 2H), 6.19 (s, 1H), 5.88 (d, *J*=9.8 Hz, 1H), 4.01 (s, 3H), 2.69 - 2.60 (m, 1H), 2.34 - 2.25 (m, 1H), 2.15 - 2.06 (m, 1H), 1.88 - 1.79 (m, 1H), 1.52 - 1.42 (m, 1H), 1.39 - 1.28 (m, 1H), 0.88 (d, *J*=6.7 Hz, 3H), 0.49 - 0.34 (m, 1H); Analytical HPLC (Method B): RT =2 min, purity =95.5%; Factor XIa Ki = 220 nM.

Example 347

Preparation of (9R,13S)-13-{4-[5-chloro-2-(pyridin-4-yl)phenyl]-6-oxo-1,6-dihydropyrimidin-1-yl}-3,9-dimethyl-3,4,7,15-tetraazatricyclo[12.3.1.0$^{2,6}$]octadeca-1(18),2(6),4,14,16-pentaen-8-one

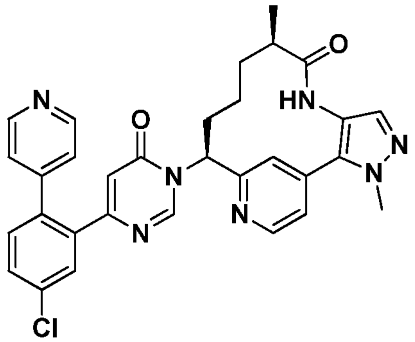

MS(ESI) *m/z:* 566.1 $(M+H)^+$; $^1H$ NMR (500MHz, DMSO-$d_6$) $\delta$ 9.21 (s, 1H), 8.80 (s, 1H), 8.68 (d, *J*=5.2 Hz, 1H), 8.55 (d, *J*=5.8 Hz, 2H), 7.74 (d, *J*=1.8 Hz, 1H), 7.69 (dd, *J*=8.2, 2.1 Hz, 1H), 7.66 (s, 1H), 7.58 (d, *J*=5.2 Hz, 1H), 7.53 (d, *J*=8.2 Hz, 1H), 7.47 (s, 1H), 7.30 (d, *J*=5.8 Hz, 2H), 6.34 (s, 1H), 5.87 (d, *J*=10.7 Hz, 1H), 4.01 (s, 3H), 2.69 - 2.60 (m, 1H), 2.33 - 2.23 (m, 1H), 2.15 - 2.06 (m, 1H), 1.88 - 1.78 (m, 1H), 1.52 - 1.42 (m, 1H), 1.38 - 1.28 (m, 1H), 0.88 (d, *J*=7.0 Hz, 3H), 0.48 - 0.35 (m, 1H); Analytical HPLC (Method A): RT =1.52 min, purity =98.9%; Factor XIa Ki = 2,000 nM.

Example 348

Preparation of (9R,13S)-13-{4-[5-chloro-2-(isoquinolin-7-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

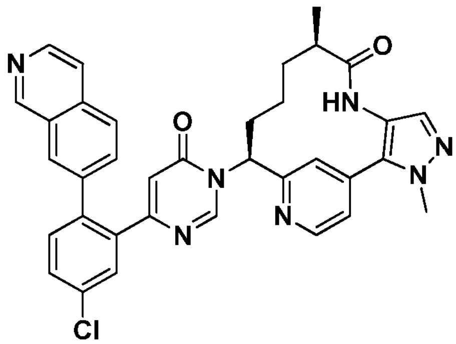

MS(ESI) *m/z:* 616 $(M+H)^+$; $^1H$ NMR (500MHz, DMSO-$d_6$) $\delta$ 9.25 (d, *J*=7.3 Hz, 2H), 8.73 (s, 1H), 8.60 (d, *J*=5.2 Hz, 1H), 8.50 (d, *J*=5.8 Hz, 1H), 8.03 (s, 1H), 7.90 (d, *J*=8.5 Hz, 1H), 7.86 (d, *J*=5.5 Hz, 1H), 7.75 (d, *J*=1.8 Hz, 1H), 7.70 - 7.66 (m, 1H), 7.63 - 7.56 (m, 3H), 7.53 (d, *J*=4.9 Hz, 1H), 7.45 (s, 1H), 6.22 (s, 1H), 5.82 - 5.74 (m, 1H), 3.98 (s, 3H), 2.66 - 2.59 (m, 1H), 2.25 - 2.16 (m, 1H), 2.07 - 1.99 (m, 1H), 1.83 - 1.74 (m, 1H), 1.48 - 1.38 (m, 1H), 1.35 - 1.25 (m, 1H), 0.85 (d, *J*=7.0 Hz, 3H), 0.47 - 0.32 (m, 1H); Analytical HPLC (Method A): RT =1.77 min, purity =94.8%; Factor XIa Ki = 260 nM, Plasma Kallikrein Ki = 3,600 nM.

Example 349

Preparation of (9R,13S)-13-{4-[5-chloro-2-(pyrimidin-5-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

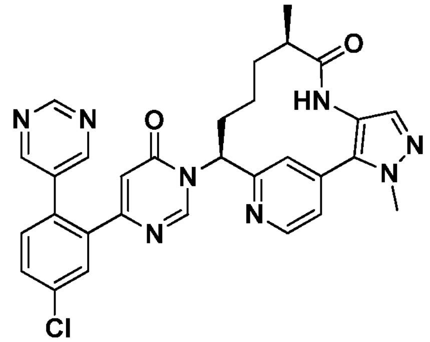

MS(ESI) *m/z:* 567 $(M+H)^+$; $^1H$ NMR (500MHz, DMSO-$d_6$) δ 9.22 (s, 1H), 9.16 (s, 1H), 8.80 (s, 1H), 8.71 - 8.66 (m, 3H), 7.79 (d, *J*=2.1 Hz, 1H), 7.73 (dd, *J*=8.2, 1.8 Hz, 1H), 7.67 (s, 1H), 7.62 (d, *J*=8.2 Hz, 1H), 7.58 (d, *J*=4.9 Hz, 1H), 7.48 (s, 1H), 6.46 (s, 1H), 5.89 (d, *J*=10.4 Hz, 1H), 4.02 (s, 3H), 2.69 - 2.61 (m, 1H), 2.33 - 2.23 (m, 1H), 2.16 - 2.07 (m, *J*=13.7 Hz, 1H), 1.87 - 1.78 (m, 1H), 1.52 - 1.43 (m, 1H), 1.38 - 1.28 (m, 1H), 0.89 (d, *J*=6.7 Hz, 3H), 0.48 - 0.35 (m, 1H); Analytical HPLC (Method A): RT =1.4 min, purity =100%; Factor XIa Ki = 16 nM, Plasma Kallikrein Ki = 3,000 nM.

Example 350

Preparation of

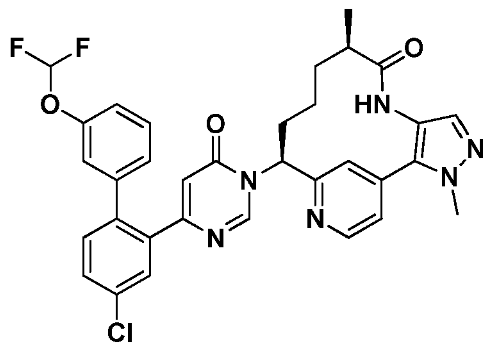

MS(ESI) *m/z:* 631.1 $(M+H)^+$; $^1H$ NMR (500MHz, DMSO-$d_6$) δ 9.25 (s, 1H), 8.84 (s, 1H), 8.67 (d, *J*=5.2 Hz, 1H), 7.69 (d, *J*=1.8 Hz, 1H), 7.66 - 7.46 (m, 6H), 7.43 - 7.38 (m, 1H), 7.16 - 7.11 (m, 2H), 7.00 (br. s., 1H), 6.20 (s, 1H), 5.89 - 5.82 (m, 1H), 4.01 (s, 3H), 2.68 - 2.60 (m, 1H), 2.33 - 2.23 (m, 1H), 2.13 - 2.04 (m, *J*=7.0 Hz, 1H), 1.86 - 1.77 (m, 1H), 1.52 - 1.42 (m, 1H), 1.38 - 1.28 (m, 1H), 0.89 (d, *J*=6.7 Hz, 3H), 0.51 - 0.37 (m, 1H); Analytical HPLC (Method A): RT =1.99 min, purity =96.9%; Factor XIa Ki = 600 nM.

Example 351

Preparation of (9R,13 S)-13-{4-[5-chloro-2-(1-ethyl-1H-pyrazol-4-yl)phenyl]-6-oxo-1,6-dihydropyrimidin-1-yl}-3,9-dimethyl-3,4,7,15-tetraazatricyclo[12.3.1.$0^{2,6}$]octadeca-1(18),2(6),4,14,16-pentaen-8-one

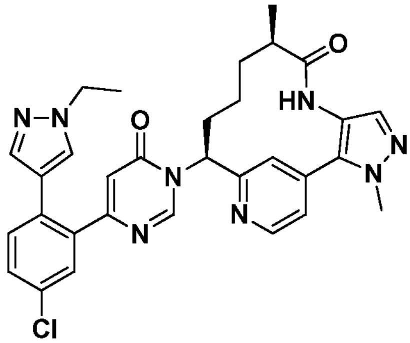

MS(ESI) *m/z:* 583.1 $(M+H)^+$; $^1H$ NMR (400MHz, $CD_3OD$) δ 8.98 (s, 1H), 8.75 (d, *J*=5.1 Hz, 1H), 7.72 (s, 1H), 7.60 (s, 1H), 7.56 - 7.52 (m, 2H), 7.49 (s, 1H), 7.48 - 7.47 (m, 2H), 7.36 (s, 1H), 6.39 (s, 1H), 6.02 (dd, *J*=12.7, 4.3 Hz, 1H), 4.12 (q, *J*=7.3 Hz, 2H), 4.05 (s, 3H), 2.76 - 2.67 (m, 1H), 2.39 - 2.29 (m, 1H), 2.14 - 2.00 (m, 2H), 1.67 - 1.43 (m, 2H), 1.37 (t, *J*=7.3 Hz, 3H), 1.01 (d, *J*=6.8 Hz, 3H), 0.79 - 0.64 (m, 1H); Analytical HPLC (Method A): RT =1.55 min, purity =95.5%; Factor XIa Ki = 96 nM.

Example 352

Preparation of (9R,13S)-13-(4-{5-chloro-2-[1-(4-fluorophenyl)-1H-pyrazol-4-yl]phenyl}-6-oxo-1,6-dihydropyrimidin-1-yl)-3,9-dimethyl-3,4,7,15-tetraazatricyclo [12.3.1.$0^{2,6}$]octadeca-1(18),2(6),4,14,16-pentaen-8-one

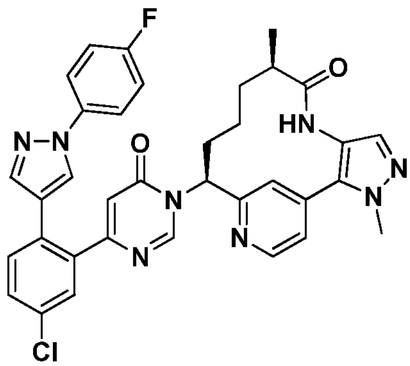

MS(ESI) *m/z*: 649.3 $(M+H)^+$; $^1H$ NMR (500MHz, DMSO-$d_6$) δ 9.24 (s, 1H), 8.95 (s, 1H), 8.62 (d, *J*=5.2 Hz, 1H), 8.53 (s, 1H), 7.83 (dd, *J*=8.9, 4.6 Hz, 2H), 7.70 (s, 1H), 7.67 - 7.56 (m, 5H), 7.49 (s, 1H), 7.36 (t, *J*=8.7 Hz, 2H), 6.46 (s, 1H), 5.96 (d, *J*=10.7 Hz, 1H), 4.03 (s, 3H), 2.70 - 2.62 (m, 1H), 2.38 - 2.28 (m, 1H), 2.18 - 2.10 (m, 1H), 1.94 - 1.85 (m, 1H), 1.54 - 1.45 (m, 1H), 1.41 - 1.31 (m, 1H), 0.90 (d, *J*=6.7 Hz, 3H), 0.51 - 0.38 (m, 1H); Analytical HPLC (Method A): RT =1.99 min, purity =100%; Factor XIa Ki = 7 nM.

## Claims

1. A compound having Formula (IIb)

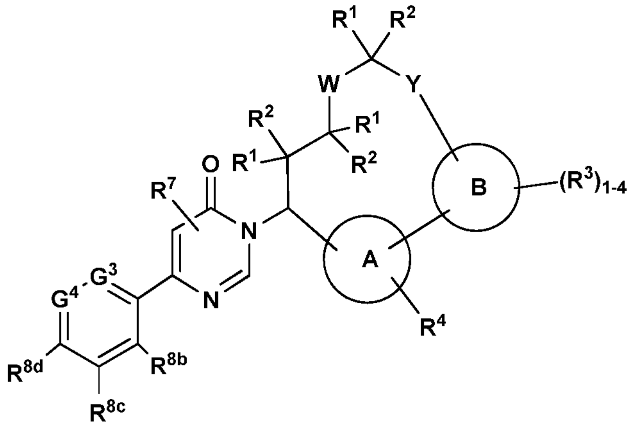

(IIb)

or a stereoisomer, a tautomer, or a pharmaceutically acceptable salt thereof, wherein:

ring A is independently selected from phenyl and 5- to 6-membered heterocyclyl;
ring B is 5- to 6-membered heteroaryl comprising carbon atoms and 1-4 heteroatoms selected from N and $NR^{3c}$;
W is independently selected from $(CR^1R^2)_{1-2}$, O, NH, and N($C_{1-4}$ alkyl);
Y is independently selected from $-CH_2NH-$, -NHC(=O)- and -C(=O)NH-;
$G^3$ is $CR^{8a}$;
$G^4$ is $CR^{8e}$;
$R^1$ and $R^2$ are independently selected from H, D, halogen, $CF_3$, $C_{1-6}$ alkyl, and hydroxyl;
$R^3$ is independently selected from H, halogen, $C_{1-4}$alkyl (optionally substituted with $R^6$), CN, $-(CH_2)_n-OR^5$, $-(CH_2)_n-C(=O)R^5$, and $-(CH_2)_n-C(=O)OR^5$;
$R^{3c}$ is independently selected from H, haloalkyl, $C_{1-4}$ alkyl (optionally substituted with $R^6$), $-(CH_2)_{1-2}-OH$, $C(=O)C_{1-4}$ alkyl, $-(CH_2)_{1-2}-C(=O)OH$, $-C(=O)OC_{1-4}$ alkyl, $S(=O)_pC_{1-6}$ alkyl, $-(CH_2)_n-C_{3-10}$ carbocyclyl and $-(CH_2)_n$-(4- to 10-membered heterocyclyl), wherein said carbocyclyl and heterocyclyl are optionally substituted with $R^6$;
$R^4$ is independently selected from H, OH, F, Cl, Br, $C_{1-4}$ alkyl, $C_{1-4}$ alkoxy, $CF_3$, CN, $C(=O)NH_2$, $C_{3-6}$ cycloalkyl, aryl, and 5- to 6-membered heterocyclyl, wherein said cycloalkyl, aryl and heterocyclyl are optionally substituted with $R^6$;
$R^5$ is independently selected from H and $C_{1-4}$ alkyl;
$R^6$ is independently selected from H, $-(CH_2)_n-OH$, =O, NH2, $-(CH_2)_n-CN$, halogen, $C_{1-6}$ alkyl, $-(CH_2)_n-C(=O)OH$, $-(CH_2)_n-C(=O)OC_{1-4}$ alkyl, $-(CH_2)_n-OC_{1-4}$ alkyl, $-(CH_2)_n-C_{3-6}$ cycloalkyl, $-(CH_2)_n$-(4- to 10-membered heterocyclyl), and $-O-(CH_2)_n$-(4- to 10-membered heterocyclyl), wherein said cycloalkyl and heterocyclyl are optionally substituted with $R^{10}$;
$R^7$ is independently selected from H, F, Cl, Br, and methyl;
$R^{8a}$ is independently selected from H, F, Cl, Br, I, $-(CH_2)_nCN$, $-(CH_2)_nNH_2$, $CH_3CHF_2$, $CCH_3F_2$, $CF_3$, OH, $OCH_3$, $OCF_3$, $OCHF_2$, $-C(=O)CH_3$, -C(=O)OH, $-C(=O)OCH_3$, $-C(=O)NH_2$, $-C(=O)NHCH_2CF_3$, $-C(=O)NHCH_2Ph$, $-NHC(=O)OCH_3$, $NHC(=O)CF_3$,

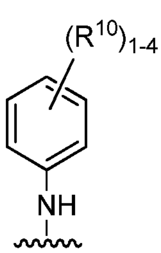 , 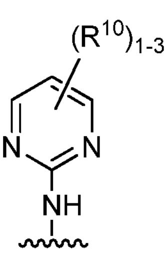 , 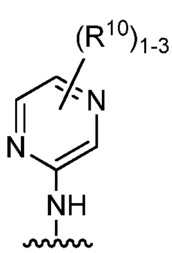 , 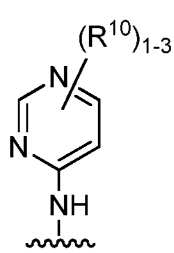 , 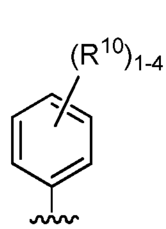 ,

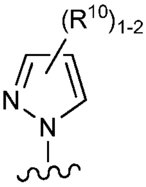, 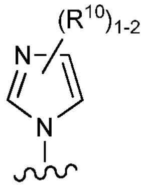, 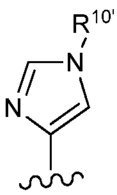, 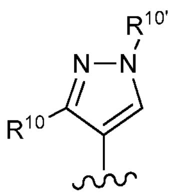, 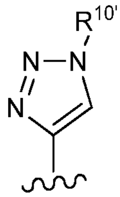, 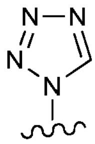, 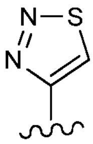,

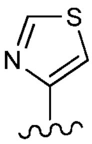, 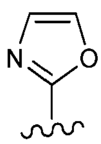, 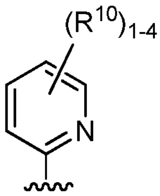, 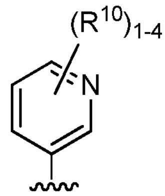, 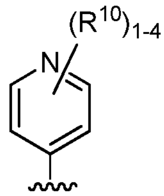, 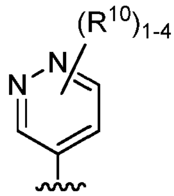, 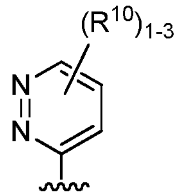,

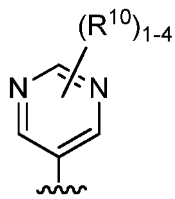, 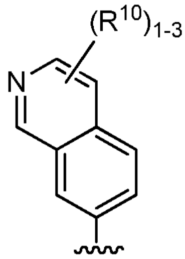, 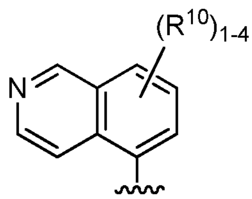, and 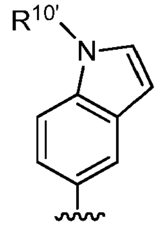;

$R^{8b}$ is independently selected from H and F;
$R^{8c}$ is independently selected from H, F, Cl, methyl, ethyl, isopropyl, $OCHF_2$ and $OCH_3$;
$R^{8d}$ is independently selected from H, F, and Cl;
$R^{8e}$ is independently selected from H, F, and Cl;
$R^{10}$ is independently selected from H, $C_{1-6}$ alkyl (optionally substituted with $R^{11}$), $C_{2-6}$ alkenyl, $C_{2-6}$ alkynyl, aryl (optionally substituted with $R^{11}$), $-(CH_2)_n-C_{3-6}$ cycloalkyl (optionally substituted with $R^{11}$), $-(CH_2)_n$-O-(4- to 10-membered heterocyclyl) (optionally substituted with $R^{11}$), F, Cl, Br, CN, $NO_2$, =O, $CONR^{12}R^{12}$, $-(CH_2)_n-C(=O)OR^{12}$, $Si(C_{1-4}\text{ alkyl})_3$, $-(CH_2)_n-OR^{12}$, $-(CH_2)_n-NR^{12}R^{12}$, $-S(=O)_pC_{1-6}$ alkyl, $NR^{12}S(=O)_pC_{1-6}$ alkyl, and $S(=O)_pNR^{12}R^{12}$;
$R^{10'}$ is independently selected from H, $C_{1-6}$ alkyl (optionally substituted with $R^{11}$), aryl, $-(CH_2)_n-C_{3-6}$ cycloalkyl (optionally substituted with $R^{11}$), and $-(CH_2)$n-O-(4- to 10-membered heterocyclyl) (optionally substituted with $R^{11}$);
$R^{11}$, at each occurrence, is independently selected from H, halogen, $C_{1-5}$ alkyl, $-(CH_2)_n-OH$, $C_{3-6}$ cycloalkyl, and phenyl;
$R^{12}$, at each occurrence, is independently selected from H, $C_{1-5}$ alkyl optionally substituted with $R^{11}$, $C_{3-6}$ cycloalkyl, phenyl, and heterocyclyl, or $R^{12}$ and $R^{12}$ together with the nitrogen atom to which they are both attached form a heterocyclic ring optionally substituted with $C_{1-4}$alkyl;
n, at each occurrence, is an integer independently selected from 0, 1, and 2; and
p, at each occurrence, is an integer independently selected from 0, 1, and 2.

**2.** A compound having Formula (IIIb):

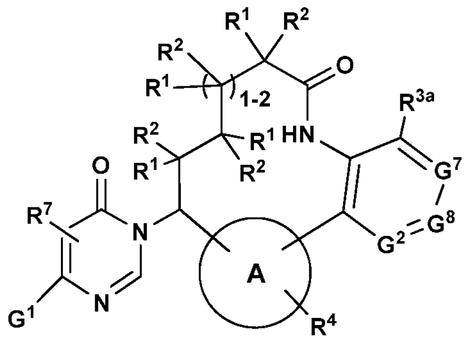

(IIIb)

or a stereoisomer, a tautomer, or a pharmaceutically acceptable salt thereof, wherein:

ring A is independently selected from phenyl and 5- to 6-membered heterocyclyl;
$G^1$ is independently selected from aryl, $C_{3-6}$cycloalkyl and 5- to 6-membered heterocyclyl, wherein said aryl, cycloalkyl and heterocyclyl are substituted with 1-4 $R^8$;
$G^2$ is independently selected from N and $CR^{3b}$;
$G^7$ is independently selected from N and $CR^3$;
$G^8$ is independently selected from N and $CR^3$;
provided at least one of $G^2$, $G^7$, and $G^8$ is N;
$R^1$ and $R^2$ are independently selected from H, halogen, $CF_3$, $C_{1-6}$ alkyl, and hydroxyl;
$R^3$ is independently selected from H, halogen, haloalkyl, $C_{1-4}$alkyl (optionally substituted with $R^6$), $C_{2-4}$alkenyl (optionally substituted with $R^6$), CN, $NO_2$, $-(CH_2)_n-OR^5$, $-(CH_2)_n-NR^5R^5$, $-(CH_2)_n-C(=O)OR^5$, $-(CH_2)_n-NHC(=O)OR^5$, $-(CH_2)_n-NH C(=O)R^5$, $-(CH_2)_n-NHC(N-CN)NHR^5$, $-(CH_2)_n-NHC$ , $-(CH_2)_n-N=CHNR^5R^5$, $-(CH_2)_n-NHC(=O)NR^5R^5$, $-(CH_2)_n-C(=O)NR^5R^5$, $-(CH_2)_n-NHC(S)NR^9C(=O)R^5$, $-(CH_2)_n-S(=O)_pC_{1-6}$ alkyl optionally substituted with $R^{11}$, $-(CH_2)_n-S(=O)_pNR^5R^5$, $-(CH_2)_n-NHS(=O)_pNR^5R^5$, $-(CH_2)_n-NHS(=O)_pC_{1-6}$ alkyl optionally substituted with $R^{11}$, $-(CH_2)_n-C_{3-10}$ carbocyclyl and $-(CH_2)_n-$(4- to 10-membered heterocyclyl), wherein said carbocyclyl and heterocyclyl are optionally substituted with $R^6$; optionally, two adjacent $R^3$ groups on the carbocyclyl and heterocyclyl may form a ring optionally substituted with $R^6$;
$R^{3a}$ is independently selected from H and halogen;
$R^{3b}$ is independently selected from H, halogen, methyl, and CN;
$R^4$ is independently selected from H, OH, F, Cl, Br, $C_{1-4}$ alkyl, $C_{1-4}$ alkoxy, $CF_3$, CN, $C_{3-6}$ cycloalkyl, aryl, and 5- to 6-membered heterocyclyl, wherein said cycloalkyl, aryl and heterocyclyl are optionally substituted with $R^6$;
$R^5$ is independently selected from H, $C_{1-4}$ alkyl (optionally substituted with halogen, hydroxyl, alkoxy, carboxy, alkoxycarbonyl, amino, substituted amino), $-(CH_2)_n-C_{3-10}$ carbocyclyl and $-(CH_2)_n-$(4- to 10-membered heterocyclyl), wherein said carbocyclyl and heterocyclyl are optionally substituted with $R^6$;
$R^6$ is independently selected from $-(CH_2)_n-OH$, =O, $NH_2$, $-(CH_2)_n-CN$, halogen, $C_{1-6}$ alkyl, $-(CH_2)_n-C(=O)OH$, $-(CH_2)_n-C(=O)OC_{1-4}$ alkyl, $-(CH_2)_n-OC_{1-4}$ alkyl, $-(CH_2)_n-C_{3-6}$ cycloalkyl, $-(CH_2)_n-$(4- to 10-membered heterocyclyl), and $-O-(CH_2)_n-$(4- to 10-membered heterocyclyl), wherein said cycloalkyl and heterocyclyl are optionally substituted with $R^{10}$;
$R^7$ is independently selected from H, F, Cl, and methyl;
$R^8$ is independently selected from H, halogen, CN, $NH_2$, $C_{1-6}$ alkyl, haloalkyl, alkylcarbonyl, alkoxy, haloalkoxy, aryl, $C_{3-6}$ cycloalkyl, and 4- to 12-membered heterocyclyl, wherein said aryl, cycloalkyl, and heterocyclyl are optionally substituted with $R^{10}$;
$R^9$ is H or $C_{1-6}$ alkyl;
$R^{10}$ is independently selected from H, $C_{1-6}$ alkyl (optionally substituted with $R^{11}$), $C_{2-6}$ alkenyl, $C_{2-6}$ alkynyl, aryl, $-(CH_2)_n-C_{3-6}$ cycloalkyl (optionally substituted with $R^{11}$), $-(CH_2)_n-O-$(4- to 10-membered heterocyclyl) (optionally substituted with $R^{11}$), F, Cl, Br, CN, $NO_2$, =O, $-C(=O)NR^{12}R^{12}$, $-C(=O)OR^{12}$, $-Si(C_{1-4}$ alkyl)3, $-(CH_2)_n-OR^{12}$, $-(CH_2)_n-NR^{12}R^{12}$, $-S(=O)_pC_{1-6}$ alkyl, $NR^{12}S(=O)_pC_{1-6}$ alkyl, and $S(=O)_pNR^{12}R^{12}$;
$R^{11}$, at each occurrence, is independently selected from H, halogen, $C_{1-5}$ alkyl, $-(CH_2)_n-OH$, $C_{3-6}$ cycloalkyl, and phenyl;
$R^{12}$, at each occurrence, is independently selected from H, $C_{1-5}$ alkyl, $C_{3-6}$ cycloalkyl, phenyl, and heterocyclyl, or $R^{12}$ and $R^{12}$ together with the nitrogen atom to which they are both attached form a heterocyclic ring optionally substituted with $C_{1-4}$alkyl;
n, at each occurrence, is an integer independently selected from 0, 1, and 2; and
p, at each occurrence, is an integer independently selected from 0, 1, and 2.

**3.** A compound having Formula (IVa):

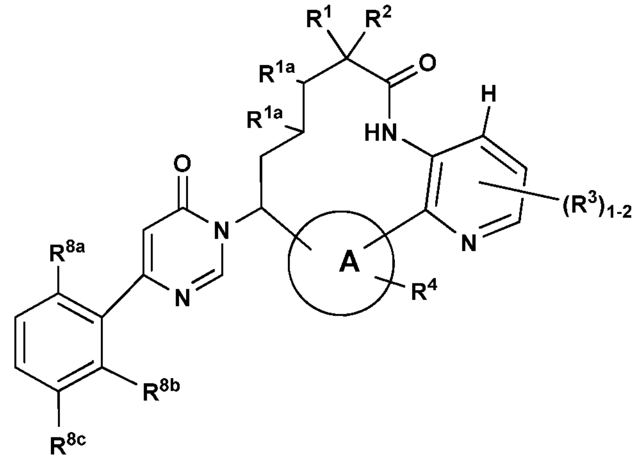

(IVa)

or a stereoisomer, a tautomer, or a pharmaceutically acceptable salt thereof, wherein:

ring A is independently selected from

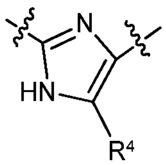 , 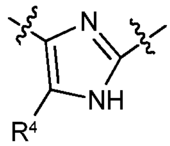 , 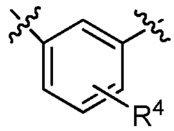 ,

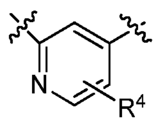 , 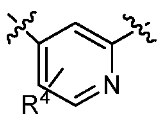 , 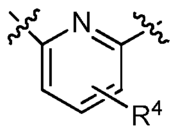 , 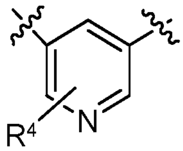 , 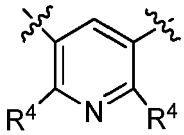 , 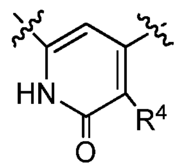 ,

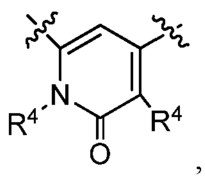 , 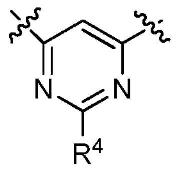 , 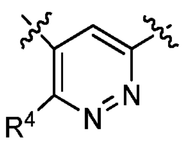 , and 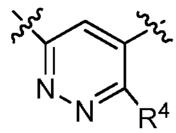 ;

$R^1$ and $R^2$ are independently selected from H, F, $C_{1-4}$ alkyl, and OH;
$R^{1a}$, at each occurrence, is independently selected from H, F, $CH_3$, and OH;
$R^3$ is independently selected from H, F, Cl, Br, I, $C_{2-4}$alkenyl (optionally substituted with C(=O)OH), CN, and $-(CH_2)_n-OH$;
$R^4$ is independently selected from H, OH, F, $OC_{1-4}$ alkyl, $C_{1-4}$ alkyl, CN, $C_{3-6}$ cycloalkyl, aryl, and 5- to 6-membered heterocyclyl, wherein said cycloalkyl, aryl and heterocyclyl are optionally substituted with $R^6$;
$R^6$ is independently selected from OH, $NH_2$, halogen, $C_{1-6}$ alkyl, $C_{3-6}$ cycloalkyl, $-(CH_2)_n-C(=O)OH$, $-(CH_2)_n-C(=O)OC_{1-4}$ alkyl, $-(CH_2)_n-OC_{1-4}$ alkyl, =O, $C_{3-6}$ cycloalkyl, 4- to 10-membered heterocyclyl, and -O-(4- to 10-membered heterocyclyl), wherein said cycloalkyl and heterocyclyl are optionally substituted with $R^{10}$;
$R^{8a}$ is independently selected from H, F, Cl, Br, CN, $OCH_3$, $OCF_3$, $CH_3$, $C(=O)CH_3$, $CF_3$, $OCHF_2$, $NHC(=O)C_{1-4}$ alkyl, aryl, $C_{3-6}$ cycloalkyl, and 4- to 12-membered heterocyclyl, wherein said aryl, cycloalkyl, and heterocyclyl are optionally substituted with $R^{10}$;
$R^{8b}$ is independently selected from H and F;
$R^{8c}$ is independently selected from H, F, Cl, $CH_3$, and $OCH_3$;

$R^{10}$ is independently selected from $C_{1-6}$ alkyl, $-C_{3-6}$ cycloalkyl, F, Cl, Br, $CF_3$, $CHF_2$, CN, and $OC_{1-5}$ alkyl; and, n, at each occurrence, is an integer independently selected from 0, 1, and 2.

**4.** The compound of claim 1, or a stereoisomer, a tautomer, or a pharmaceutically acceptable salt thereof, wherein:

ring A is independently selected from

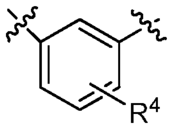, 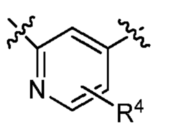, 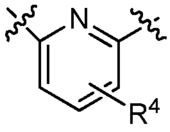,

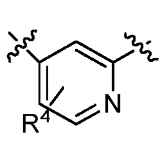, and 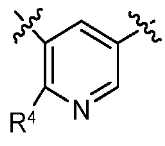;

ring B is independently selected from

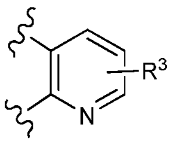, 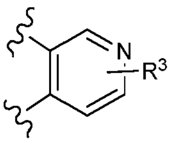, 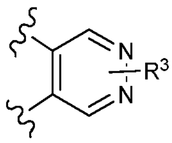,

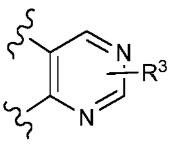, 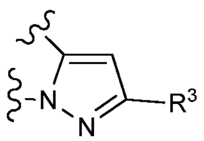, 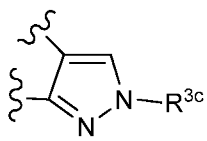, 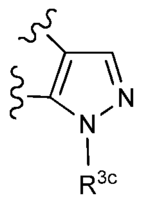

and

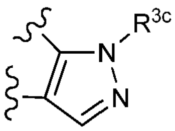;

W is independently selected from $CHR^{1a}$, O, NH, and $N(C_{1-4}$ alkyl);
$R^1$ is independently selected from H and $C_{1-4}$ alkyl;
$R^{1a}$ is independently selected from H, F, $CH_3$, and hydroxyl;
$R^2$ is independently selected from H and hydroxyl;
$R^3$ is independently selected from H, F, $CHF_2$, $CF_3$, $CH_3$, CN, $-(CH_2)_{0-2}-OH$, $OC_{1-4}$ alkyl, $C(=O)C_{1-4}$ alkyl, $-(CH_2)_{0-1}-C(=O)OH$, and $-C(=O)OC_{1-4}$ alkyl;
$R^{3c}$ is independently selected from H, $CF_2H$, $CF_3$, and $C_{1-4}$ alkyl;
$R^4$ is independently selected from H and F;
$R^{8b}$ is independently selected from H and F;
$R^{8c}$ is independently selected from H and Cl;
$R^{10}$ is independently selected from H, $C_{1-6}$ alkyl (optionally substituted with $R^{11}$), aryl, $-(CH_2)_n-C_{3-6}$ cycloalkyl (optionally substituted with $R^{11}$), $-(CH_2)_n$-O-(4- to 10-membered heterocyclyl), F, Cl, Br, CN, $-C(=O)NR^{12}R^{12}$, $-Si(C_{1-4}$ alkyl$)_3$, and $-(CH_2)_n-OR^{12}$;
$R^{11}$, at each occurrence, is independently selected from H, halogen, and $C_{1-5}$ alkyl; and
n, at each occurrence, is an integer independently selected from 0, 1, and 2.

**5.** The compound of claim 1, or a stereoisomer, a tautomer, or a pharmaceutically acceptable salt thereof, wherein:

ring A is independently selected from

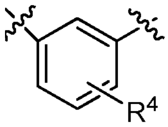 , 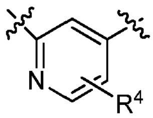 , 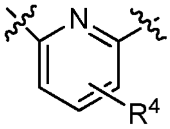 ,

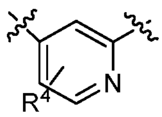 , and 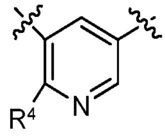 ;

ring B is independently selected from

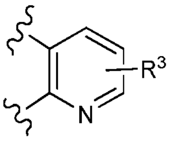 , 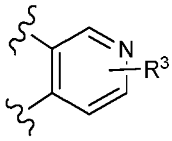 , 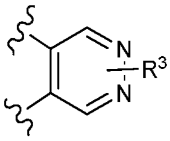 ,

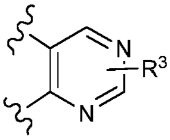 , 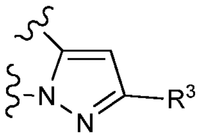 , 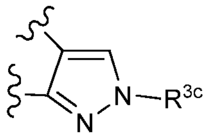 , 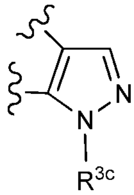

and

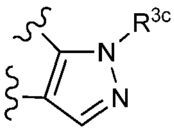 ;

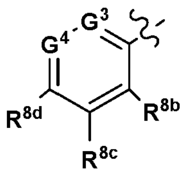

is independently selected from

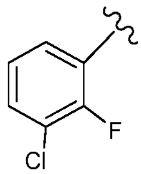 , 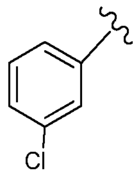 , 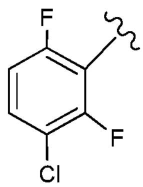 ,

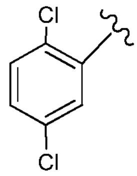,

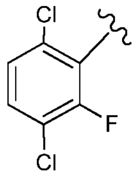, 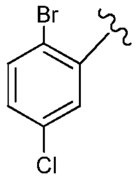, 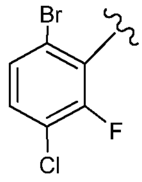, 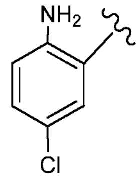, 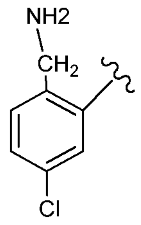, 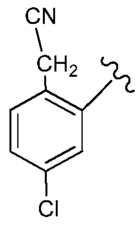, 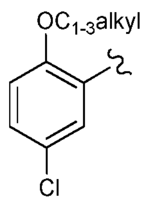,

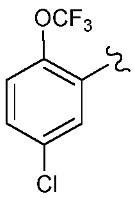, 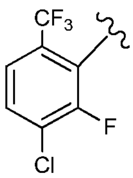, 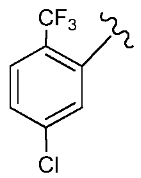, 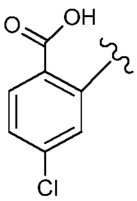, 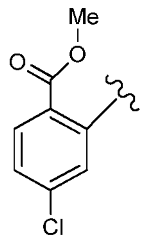, 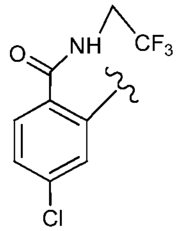, 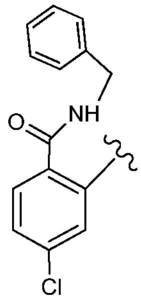,

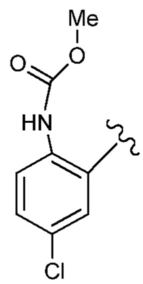, 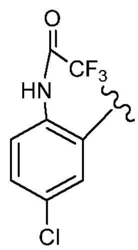, 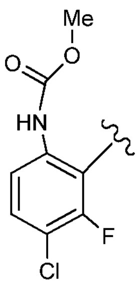, 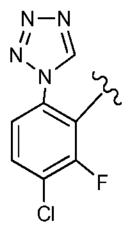, 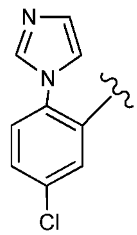,

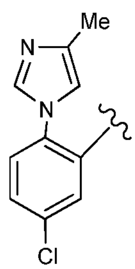, 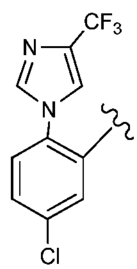, 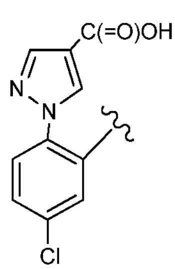, 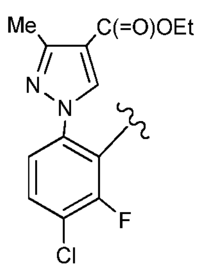, 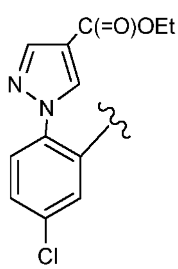,

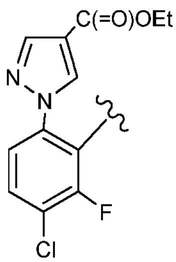, 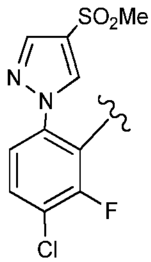, 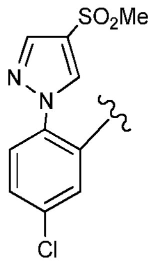, 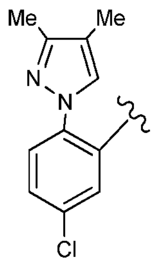, 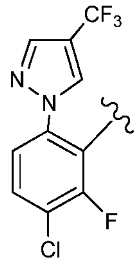, 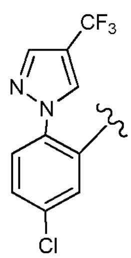, 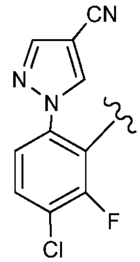,

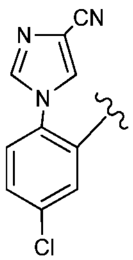, 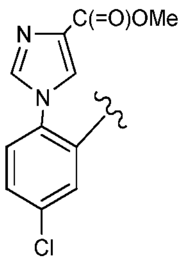, 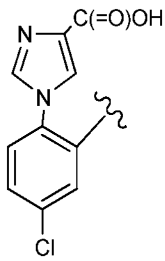, 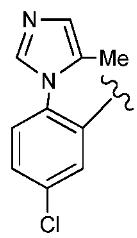, 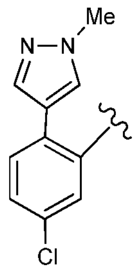, 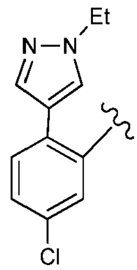, 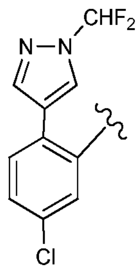,

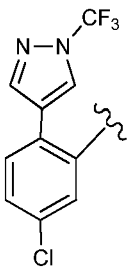, 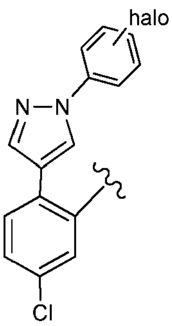, 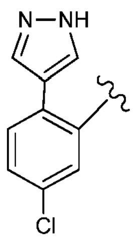 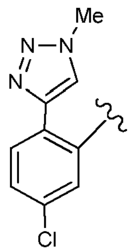, 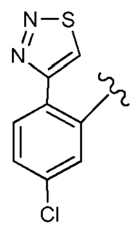, 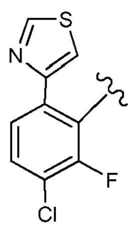, 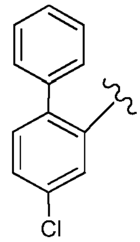, 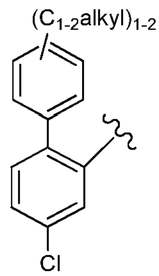

, 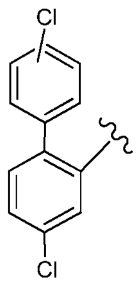, 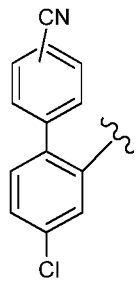,

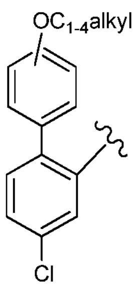, 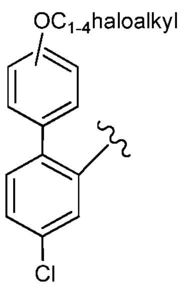, 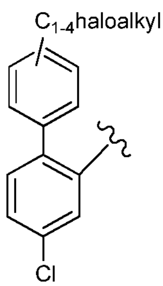, 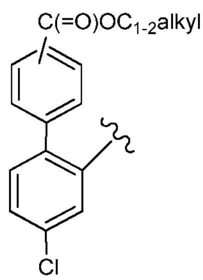, 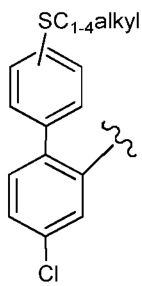, 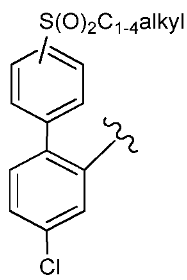,

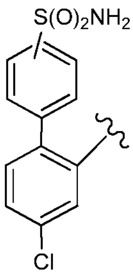 , 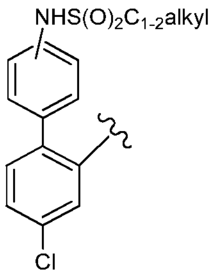 , 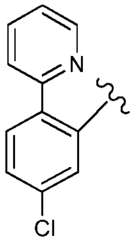 , 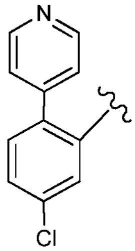 , 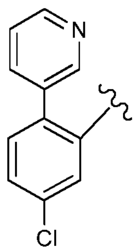 , 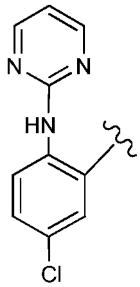 , 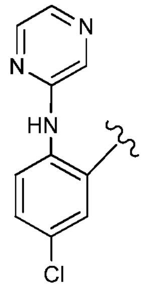 ,

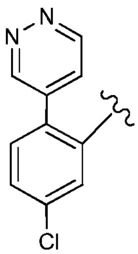 , 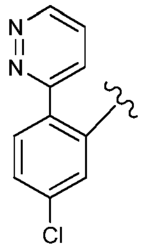 , 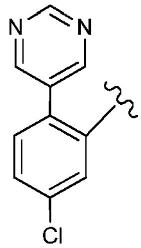 , 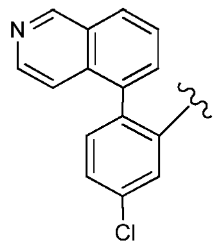 , and 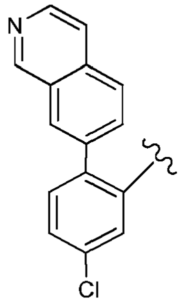 ;

W is independently selected from $CHR^1$, O, NH, and N($C_{1-4}$ alkyl);
Y is independently selected from $-CH_2NH-$, -NHC(=O)- and -C(=O)NH-;
$R^1$ and $R^2$ are independently selected from H, F, $C_{1-4}$ alkyl, and hydroxyl;
$R^3$ is independently selected from H, =O, F, $CHF_2$, $CF_3$, $CH_3$, CN, $-(CH_2)_{0-2}$-OH, $OC_{1-4}$ alkyl, C(=O)$C_{1-4}$ alkyl, $-(CH_2)_{0-1}$-C(=O)OH, and -C(=O)$OC_{1-4}$ alkyl,;
$R^{3c}$ is independently selected from H, $CF_2H$, $CF_3$ and $C_{1-4}$ alkyl;
$R^4$ is independently selected from H, F, and $C_{1-4}$ alkyl; and
$R^7$ is H.

**6.** A pharmaceutical composition comprising one or more compounds according to any one of claims 1-5 and a pharmaceutically acceptable carrier or diluent.

**7.** A compound of any one of claims 1-5, or a stereoisomer, a tautomer, or a pharmaceutically acceptable salt thereof, or the pharmaceutical composition according to claim 6, for use in therapy.

**8.** A compound of any one of claims 1-5, or a stereoisomer, a tautomer, or a pharmaceutically acceptable salt thereof, or the pharmaceutical composition according to claim 6, for use in the treatment and/or prophylaxis of a thromboembolic disorder, comprising: administering to a patient in need thereof a therapeutically effective amount of the compound, stereoisomer, tautomer, or pharmaceutically acceptable salt, or the pharmaceutical composition, wherein the thromboembolic disorder is selected from arterial cardiovascular thromboembolic disorders, venous cardiovascular thromboembolic disorders, and thromboembolic disorders in the chambers of the heart or in the peripheral circulation.

**9.** The compound or pharmaceutical composition for use according to claim 8, wherein the thromboembolic disorder is selected from unstable angina, an acute coronary syndrome, atrial fibrillation, myocardial infarction, transient ischemic attack, stroke, atherosclerosis, peripheral occlusive arterial disease, venous thrombosis, deep vein thrombosis, thrombophlebitis, arterial embolism, coronary arterial thrombosis, cerebral arterial thrombosis, cerebral embolism, kidney embolism, pulmonary embolism, and thrombosis resulting from medical implants, devices, or procedures in which blood is exposed to an artificial surface that promotes thrombosis.

## Patentansprüche

**1.** Verbindung der Formel (IIb)

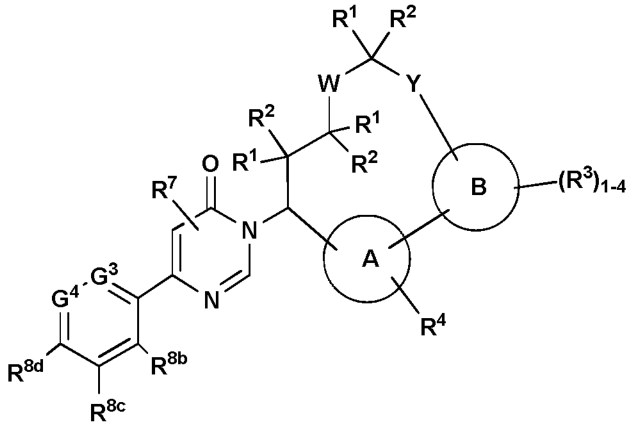

(IIb)

oder ein Stereoisomer, ein Tautomer oder ein pharmazeutisch verträgliches Salz davon, wobei:

Ring A unabhängig ausgewählt ist aus Phenyl und 5- bis 6-gliedrigem Heterocyclyl;
Ring B 5- bis 6-gliedriges Heteroaryl ist, umfassend Kohlenstoffatome und 1 bis 4 Heteroatome, die ausgewählt sind aus N und $NR^{3c}$;
W unabhängig ausgewählt ist aus $(CR^1R^2)_{1-2}$, O, NH und N($C_{1-4}$-Alkyl);
Y unabhängig ausgewählt ist aus $-CH_2NH-$, -NHC(=O)- und -C(=O)NH-;
$G^3$ $CR^{8a}$ ist;
$G^4$ $CR^{8e}$ ist;
$R^1$ und $R^2$ unabhängig ausgewählt sind aus H, D, Halogen, $CF_3$, $C_{1-6}$-Alkyl und Hydroxyl;
$R^3$ unabhängig ausgewählt ist aus H, Halogen, $C_{1-4}$-Alkyl (wahlweise substituiert mit $R^6$), CN, $-(CH_2)_n-OR^5$, $-(CH_2)_n-C(=O)R^5$ und $-(CH_2)_n-C(=O)OR^5$;
$R^{3c}$ unabhängig ausgewählt ist aus H, Halogenalkyl, $C_{1-4}$-Alkyl (wahlweise substituiert mit $R^6$), $-(CH_2)_{1-2}-OH$, $C(=O)C_{1-4}$-Alkyl, $-(CH_2)_{1-2}-C(=O)OH$, $-C(=O)OC_{1-4}$-Alkyl, $S(=O)_pC_{1-6}$-Alkyl, $-(CH_2)_n-C_{3-10}$-Carbocyclyl und $-(CH_2)_n$-(4- bis 10-gliedrigem Heterocyclyl), wobei das Carbocyclyl und Heterocyclyl wahlweise substituiert sind mit $R^6$;
$R^4$ unabhängig ausgewählt ist aus H, OH, F, Cl, Br, $C_{1-4}$-Alkyl, $C_{1-4}$-Alkoxy, $CF_3$, CN, $C(=O)NH_2$, $C_{3-6}$-Cycloalkyl, Aryl und 5- bis 6-gliedrigem Heterocyclyl, wobei das Cycloalkyl, Aryl und Heterocyclyl wahlweise substituiert sind mit $R^6$;
$R^5$ unabhängig ausgewählt ist aus H und $C_{1-4}$-Alkyl;
$R^6$ unabhängig ausgewählt ist aus H, $-(CH_2)_n-OH$, =O, $NH_2$, $-(CH_2)_n-CN$, Halogen, $C_{1-6}$-Alkyl, $-(CH_2)_n-C(=O)OH$, $-(CH_2)_n-C(=O)OC_{1-4}$-Alkyl, $-(CH_2)_n-OC_{1-4}$-Alkyl, $-(CH_2)n-C_{3-6}$-Cycloalkyl, $-(CH_2)_n$-(4- bis 10-gliedrigem Heterocyclyl) und $-O-(CH_2)_n$-(4- bis 10-gliedrigem Heterocyclyl), wobei das Cycloalkyl und Heterocyclyl wahlweise substituiert sind mit $R^{10}$;
$R^7$ unabhängig ausgewählt ist aus H, F, Cl, Br und Methyl;
$R^{8a}$ unabhängig ausgewählt ist aus H, F, Cl, Br, I, $-(CH_2)_nCN$, $-(CH_2)_nNH_2$, $CH_3CHF_2$, $CCH_3F_2$, $CF_3$, OH, $OCH_3$, $OCF_3$, $OCHF_2$, $-C(=O)CH_3$, -C(=O)OH, $-C(=O)OCH_3$, $-C(=O)NH_2$, $-C(=O)NHCH_2CF_3$, -C(=O) $NHCH_2Ph$, $-NHC(=O)OCH_3$, $NHC(=O)CF_3$,

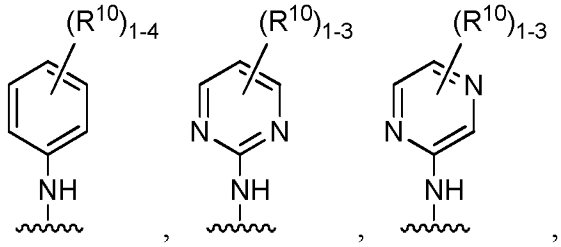

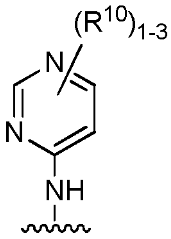 , 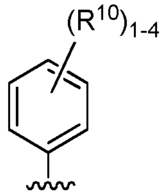 ,

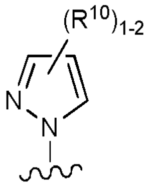 , 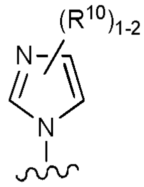 , 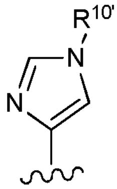 , 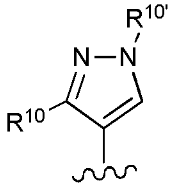 , 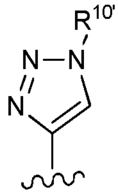 , 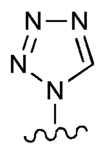 , 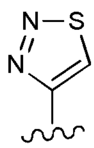 ,

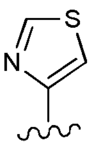 , 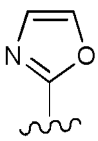 , 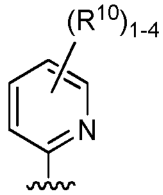 , 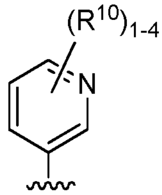 , 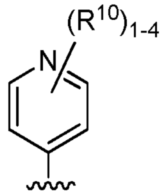 , 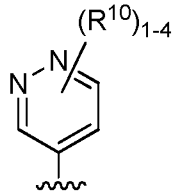 , 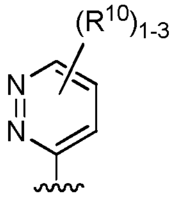 ,

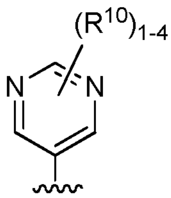 , 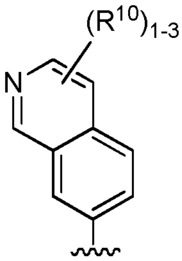 , 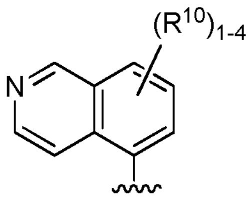 und 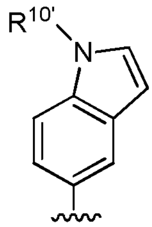 ;

$R^{8B}$ unabhängig ausgewählt ist aus H und F;

$R^{8c}$ unabhängig ausgewählt ist aus H, F, Cl, Methyl, Ethyl, Isopropyl, $OCHF_2$ und $OCH_3$;

$R^{8d}$ unabhängig ausgewählt ist aus H, F und Cl;

$R^{8e}$ unabhängig ausgewählt ist aus H, F und Cl;

$R^{10}$ unabhängig ausgewählt ist aus H, $C_{1-6}$-Alkyl (wahlweise substituiert mit $R^{11}$), $C_{2-6}$-Alkenyl, $C_{2-6}$-Alkinyl, Aryl (wahlweise substituiert mit $R^{11}$), $-(CH_2)_n-C_{3-6}$-Cycloalkyl (wahlweise substituiert mit $R^{11}$), $-(CH_2)_n$-O-(4- bis 10-gliedrigem Heterocyclyl) (wahlweise substituiert mit $R^{11}$), F, Cl, Br, CN, $NO_2$, =O, $CONR^{12}R^{12}$, $-(CH_2)_n-C(=O)OR^{12}$, $Si(C_{1-4}\text{-Alkyl})_3$, $-(CH_2)_n-OR^{12}$, $-(CH_2)_n-NR^{12}R^{12}$, $-S(=O)_pC_{1-6}$-Alkyl, $NR^{12}S(=O)_pC_{1-6}$-Akyl und $S(=O)_pNR^{12}R^{12}$;

$R^{10'}$ unabhängig ausgewählt ist aus H, $C_{1-6}$-Alkyl (wahlweise substituiert mit $R^{11}$), Aryl, $-(CH_2)_n-C_{3-6}$-Cycloalkyl (wahlweise substituiert mit $R^{11}$) und $-(CH_2)_n$-O-(4- bis 10-gliedrigem Heterocyclyl) (wahlweise substituiert mit $R^{11}$);

$R^{11}$ beim jeweiligen Auftreten unabhängig ausgewählt ist aus H, Halogen, $C_{1-5}$-Akyl, $-(CH_2)_n$-OH, $C_{3-6}$-Cycloalkyl und Phenyl;

$R^{12}$ beim jeweiligen Auftreten unabhängig ausgewählt ist aus H, $C_{1-5}$-Akyl, wahlweise substituiert mit $R^{11}$, $C_{3-6}$-Cycloalkyl, Phenyl, und Heterocyclyl, oder $R^{12}$ und $R^{12}$ können zusammen mit dem Stickstoffatom, an dem sie beide gebunden sind einen heterocyclischen Ring bilden, der wahlweise substituiert ist mit $C_{1-4}$-Alkyl;

n beim jeweiligen Auftreten eine ganze Zahl ist, die unabhängig ausgewählt ist aus 0, 1 und 2; und

p beim jeweiligen Auftreten eine ganze Zahl ist, die unabhängig ausgewählt ist aus 0, 1 und 2.

**2.** Verbindung der Formel (IIIb):

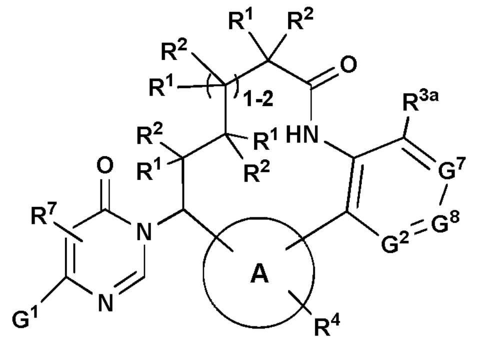

(IIIb)

oder ein Stereoisomer, ein Tautomer oder ein pharmazeutisch verträgliches Salz davon, wobei:

Ring A unabhängig ausgewählt ist aus Phenyl und 5- bis 6-gliedrigem Heterocyclyl;
$G^1$ unabhängig ausgewählt ist aus Aryl, $C_{3-6}$-Cycloalkyl und 5- bis 6-gliedrigem Heterocyclyl, wobei das Aryl, Cycloalkyl und Heterocyclyl substituiert sind mit 1 bis 4 $R^8$;
$G^2$ unabhängig ausgewählt ist aus N und $CR^{3b}$;
$G^7$ unabhängig ausgewählt ist aus N und $CR^3$;
$G^8$ unabhängig ausgewählt ist aus N und $CR^3$;
mit der Maßgabe, dass mindestens eines von $G^2$, $G^7$ und $G^8$ N ist;
$R^1$ und $R^2$ unabhängig ausgewählt sind aus H, Halogen, $CF_3$, $C_{1-6}$-Akyl und Hydroxyl;
$R^3$ unabhängig ausgewählt ist aus H, Halogen, Halogenalkyl, $C_{1-4}$-Alkyl (wahlweise substituiert mit $R^6$), $C_{2-4}$-Alkenyl (wahlweise substituiert mit $R^6$), CN, $NO_2$, $-(CH_2)_n-OR^5$, $-(CH_2)_n-NR^5R^5$, $-(CH_2)_n-C(=O)OR^5$, $-(CH_2)_n-NHC(=O)OR^5$, $-(CH_2)_n-NHC(=O)R^5$, $-(CH_2)_n-NHC(N-CN)NHR^5$, $-(CH_2)n-NHC(NH)NHR^5$, $-(CH_2)_n-N=CHNR^5R^5$, $-(CH_2)_n-NHC(=O)NR^5R^5$, $-(CH_2)_n-C(=O)NR^5R^5$, $-(CH_2)_n-NHC(S)NR^9C(=O)R^5$, $-(CH_2)_n-S(=O)_pC_{1-6}$-Akyl, das wahlweise ist substituiert mit $R^{11}$, $-(CH_2)_n-S(=O)_pNR^5R^5$, $-(CH_2)_n-NHS(=O)_pNR^5R^5$, $-(CH_2)_n-NHS(=O)_pC_{1-6}$-Akyl, das wahlweise substituiert ist mit $R^{11}$, $-(CH_2)_n-C_{3-10}$-Carbocyclyl und $-(CH_2)_n$-(4- bis 10-gliedrigem Heterocyclyl), wobei das Carbocyclyl und Heterocyclyl wahlweise substituiert ist mit $R^6$; wahlweise zwei benachbarte $R^3$-Gruppe an dem Carbocyclyl und Heterocyclyl einen Ring bilden können, der wahlweise substituiert ist mit $R^6$;
$R^{3a}$ unabhängig ausgewählt ist aus H und Halogen;
$R^{3b}$ unabhängig ausgewählt ist aus H, Halogen, Methyl und CN;
$R^4$ unabhängig ausgewählt ist aus H, OH, F, Cl, Br, $C_{1-4}$-Akyl, $C_{1-4}$-Alkoxy, $CF_3$, CN, $C_{3-6}$-Cycloalkyl, Aryl und 5- bis 6-gliedrigem Heterocyclyl, wobei das Cycloalkyl, Aryl und Heterocyclyl wahlweise substituiert ist mit $R^6$;
$R^5$ unabhängig ausgewählt ist aus H, $C_{1-4}$-Akyl (das wahlweise substituiert ist mit Halogen, Hydroxyl, Alkoxy, Carboxy, Alkoxycarbonyl, Amino, substituiertem Amino), $-(CH_2)_n-C_{3-10}$-Carbocyclyl und $-(CH_2)_n$-(4- bis 10-gliedrigem Heterocyclyl), wobei das Carbocyclyl und Heterocyclyl wahlweise substituiert sind mit $R^6$;
$R^6$ unabhängig ausgewählt ist aus $-(CH_2)_n-OH$, =O, $NH_2$, $-(CH_2)_n-CN$, Halogen, $C_{1-6}$-Akyl, $-(CH_2)_n-C(=O)OH$, $-(CH_2)_n-C(=O)OC_{1-4}$-Akyl, $-(CH_2)_n-OC_{1-4}$-Akyl, $-(CH_2)_n-C_{3-6}$-Cycloalkyl, $-(CH_2)_n$-(4- bis 10-gliedrigem Heterocyclyl) und $-O-(CH_2)_n$-(4- bis 10-gliedrigem Heterocyclyl), wobei das Cycloalkyl und Heterocyclyl wahlweise substituiert sind mit $R^{10}$;
$R^7$ unabhängig ausgewählt ist aus H, F, Cl und Methyl;
$R^8$ unabhängig ausgewählt ist aus H, Halogen, CN, $NH_2$, $C_{1-6}$-Akyl, Halogenalkyl, Alkylcarbonyl, Alkoxy, Halogenalkoxy, Aryl, $C_{3-6}$-Cycloalkyl und 4- bis 12-gliedrigem Heterocyclyl, wobei das Aryl, Cycloalkyl und Heterocyclyl wahlweise substituiert sind mit $R^{10}$;
$R^9$ H oder $C_{1-6}$-Akyl ist;
$R^{10}$ unabhängig ausgewählt ist aus H, $C_{1-6}$-Akyl (wahlweise substituiert mit $R^{11}$), $C_{2-6}$-Akenyl, $C_{2-6}$-Akinyl, Aryl, $-(CH_2)_n-C_{3-6}$-Cycloalkyl (das wahlweise substituiert ist mit $R^{11}$), $-(CH_2)_n-O$-(4- bis 10-gliedrigem Heterocyclyl) (wahlweise substituiert mit $R^{11}$), F, Cl, Br, CN, $NO_2$, =O, $-C(=O)NR^{12}R^{12}$, $-C(=O)OR^{12}$, $-Si(C_{1-4}\text{-Akyl})_3$, $-(CH_2)_n-OR^{12}$, $-(CH_2)_n-NR^{12}R^{12}$, $-S(=O)_pC_{1-6}$-Akyl, $NR^{12}S(=O)_pC_{1-6}$-Akyl und $S(=O)_pNR^{12}R^{12}$;
$R^{11}$ beim jeweiligen Auftreten unabhängig ausgewählt ist aus H, Halogen, $C_{1-5}$-Akyl, $-(CH_2)_n-OH$, $C_{3-6}$-Cycloalkyl und Phenyl;
$R^{12}$ beim jeweiligen Auftreten unabhängig ausgewählt ist aus H, $C_{1-5}$-Akyl, $C_{3-6}$-Cycloalkyl, Phenyl und Heterocyclyl, oder $R^{12}$ und $R^{12}$ bilden zusammen mit dem Stickstoffatom, an dem sie beide gebunden sind, einen heterocyclischen Ring, der wahlweise substituiert ist mit $C_{1-4}$-Alkyl;
n beim jeweiligen Auftreten eine ganze Zahl ist, die unabhängig ausgewählt ist aus 0, 1 und 2; und

p beim jeweiligen Auftreten eine ganze Zahl ist, die unabhängig ausgewählt ist aus 0, 1 und 2.

**3.** Verbindung der Formel (IVa):

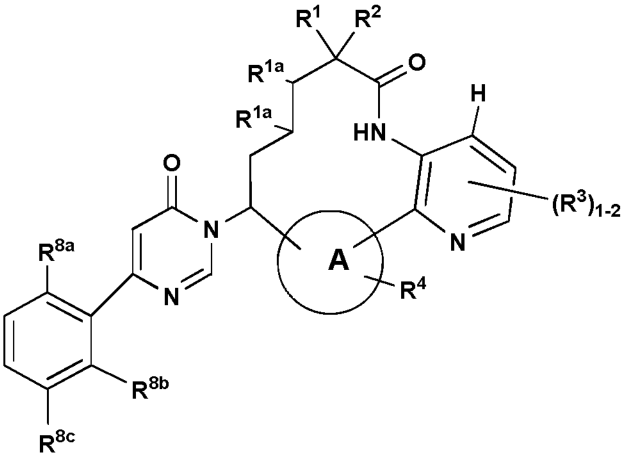

(IVa)

oder ein Stereoisomer, ein Tautomer oder ein pharmazeutisch verträgliches Salz davon, wobei:

Ring A unabhängig ausgewählt ist aus

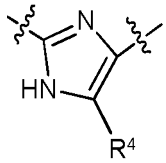 , 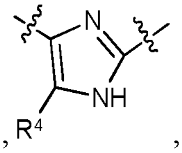 , 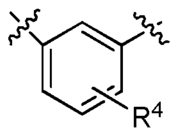 ,

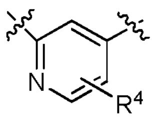 , 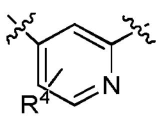 , 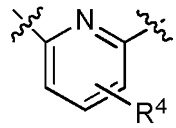 , 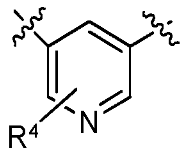 , 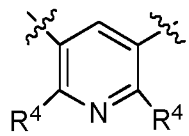 , 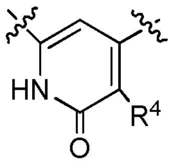 ,

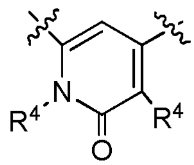 , 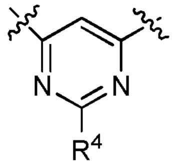 , 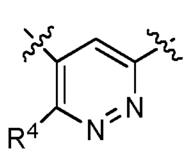 und 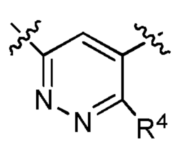 ;

$R^1$ und $R^2$ unabhängig ausgewählt sind aus H, F, $C_{1-4}$-Akyl und OH;
$R^{1a}$ beim jeweiligen Auftreten unabhängig ausgewählt ist aus H, F, $CH_3$ und OH;
$R^3$ unabhängig ausgewählt ist aus H, F, Cl, Br, I, $C_{2-4}$-Alkenyl (das wahlweise substituiert ist mit C(=O)OH), CN und $-(CH_2)_n$-OH;
$R^4$ unabhängig ausgewählt ist aus H, OH, F, $OC_{1-4}$-Akyl, $C_{1-4}$-Akyl, CN, $C_{3-6}$-Cycloalkyl, Aryl und 5- bis 6-gliedrigem Heterocyclyl, wobei das Cycloalkyl, Aryl und Heterocyclyl wahlweise substituiert sind mit $R^6$;
$R^6$ unabhängig ausgewählt ist aus OH, $NH_2$, Halogen, $C_{1-6}$-Akyl, $C_{3-6}$-Cycloalkyl, $-(CH_2)_n$-C(=O)OH, $-(CH_2)_n$-C(=O)$OC_{1-4}$-Akyl, $-(CH_2)_n$-$OC_{1-4}$-Akyl, =O, $C_{3-6}$-Cycloalkyl, 4- bis 10-gliedrigem Heterocyclyl und -O-(4- bis 10-gliedrigem Heterocyclyl), wobei das Cycloalkyl und Heterocyclyl wahlweise substituiert ist mit $R^{10}$;
$R^{8a}$ unabhängig ausgewählt ist aus H, F, Cl, Br, CN, $OCH_3$, $OCF_3$, $CH_3$, C(=O)$CH_3$, $CF_3$, $OCHF_2$, NHC(=O)$C_{1-4}$-Akyl, Aryl, $C_{3-6}$-Cycloalkyl, und 4- bis 12-gliedrigem Heterocyclyl, wobei das Aryl, Cycloalkyl und Heterocyclyl wahlweise substituiert ist mit $R^{10}$;

$R^{8b}$ unabhängig ausgewählt ist aus H und F;
$R^{8c}$ unabhängig ausgewählt ist aus H, F, Cl, $CH_3$ und $OCH_3$;
$R^{10}$ unabhängig ausgewählt ist aus $C_{1-6}$-Akyl, -$C_{3-6}$-Cycloalkyl, F, Cl, Br, $CF_3$, $CHF_2$, CN und $OC_{1-5}$-Akyl; und
n beim jeweiligen Auftreten eine ganze Zahl ist, die unabhängig ausgewählt ist aus 0, 1 und 2.

**4.** Verbindung nach Anspruch 1 oder ein Stereoisomer, ein Tautomer oder ein pharmazeutisch verträgliches Salz davon, wobei:

Ring A unabhängig ausgewählt ist aus

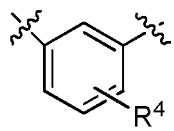, 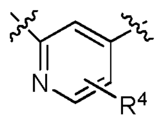, 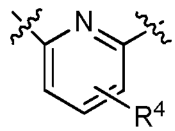,

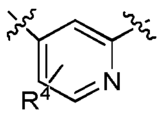 und 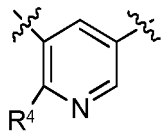;

Ring B unabhängig ausgewählt ist aus

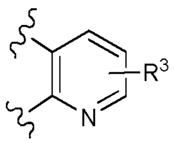, 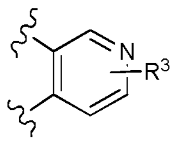, 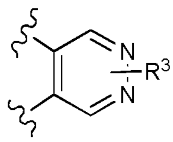,

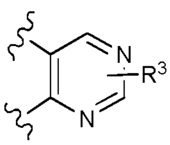, 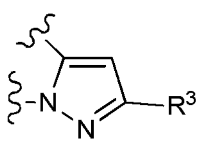, 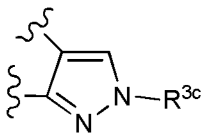, 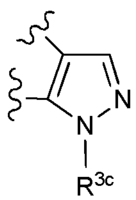 und 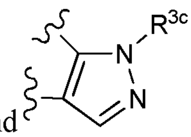;

W unabhängig ausgewählt ist aus $CHR^{1a}$, O, NH und N($C_{1-4}$-Akyl);
$R^1$ unabhängig ausgewählt ist aus H und $C_{1-4}$-Akyl;
$R^{1a}$ unabhängig ausgewählt ist aus H, F, $CH_3$ und Hydroxyl;
$R^2$ unabhängig ausgewählt ist aus H und Hydroxyl;
$R^3$ unabhängig ausgewählt ist aus H, F, $CHF_2$, $CF_3$, $CH_3$, CN, -$(CH_2)_{0-2}$-OH, $OC_{1-4}$-Akyl, C(=O)$C_{1-4}$-Akyl, -$(CH_2)_{0-1}$-C(=O)OH und -C(=O)$OC_{1-4}$-Akyl;
$R^{3c}$ unabhängig ausgewählt ist aus H, $CF_2H$, $CF_3$ und $C_{1-4}$-Akyl;
$R^4$ unabhängig ausgewählt ist aus H und F;
$R^{8b}$ unabhängig ausgewählt ist aus H und F;
$R^{8c}$ unabhängig ausgewählt ist aus H und Cl;
$R^{10}$ unabhängig ausgewählt ist aus H, $C_{1-6}$-Akyl (wahlweise substituiert mit $R^{11}$), Aryl, -$(CH_2)_n$-$C_{3-6}$Cycloalkyl (wahlweise substituiert mit $R^{11}$), -$(CH_2)_n$-O-(4- bis 10-gliedrigem Heterocyclyl), F, Cl, Br, CN, -C(=O)$NR^{12}R^{12}$, -Si($C_{1-4}$-Akyl)$_3$ und -$(CH_2)_n$-$OR^{12}$;
$R^{11}$ beim jeweiligen Auftreten unabhängig ausgewählt ist aus H, Halogen, und $C_{1-5}$-Akyl; und
n beim jeweiligen Auftreten eine ganze Zahl ist, die unabhängig ausgewählt ist aus 0, 1 und 2.

**5.** Verbindung nach Anspruch 1 oder ein Stereoisomer, ein Tautomer oder ein pharmazeutisch verträgliches Salz davon, wobei:

Ring A unabhängig ausgewählt ist aus

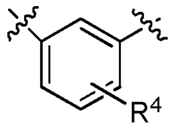, 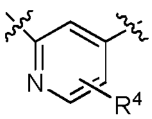, 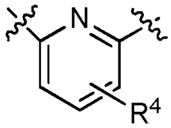,

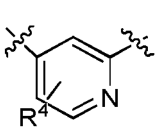 und 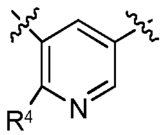;

Ring B unabhängig ausgewählt ist aus

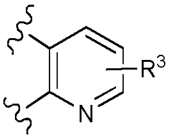, 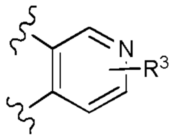, 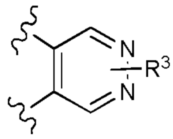,

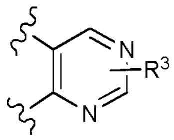, 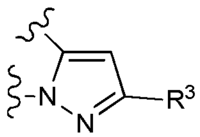, 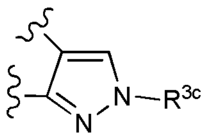, 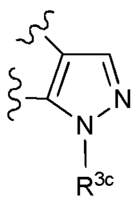 und 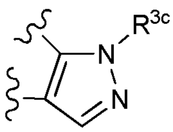;

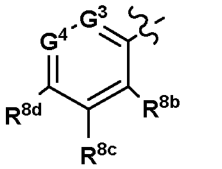

unabhängig ausgewählt ist aus

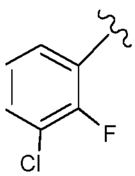, 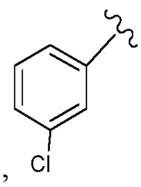, 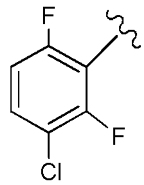,

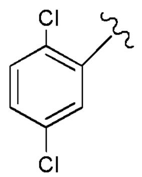,

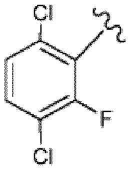, 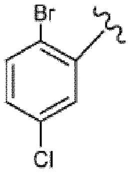, 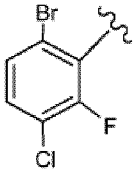, 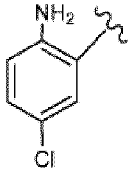, 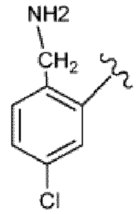, 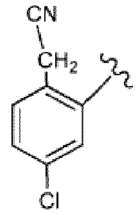, 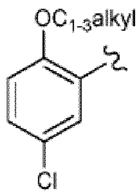,

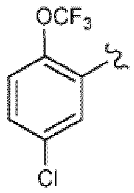, 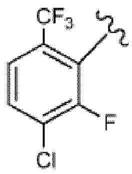, 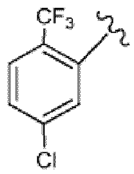, 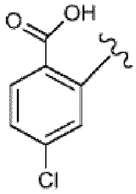, 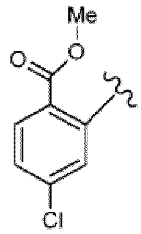, 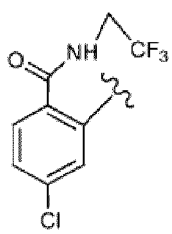, 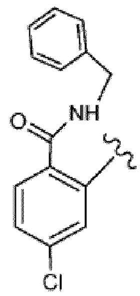,

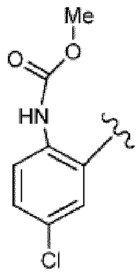, 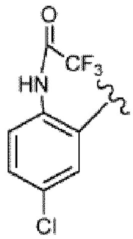, 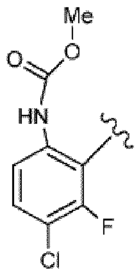, 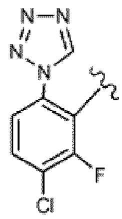, 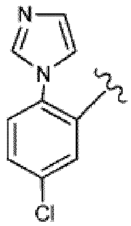,

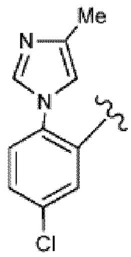, 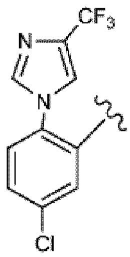, 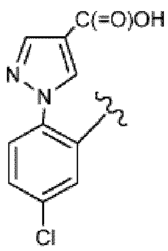, 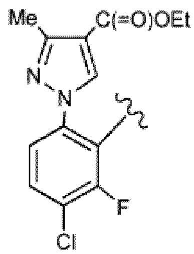, 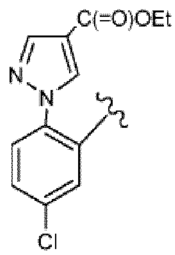,

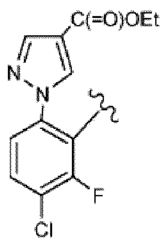, 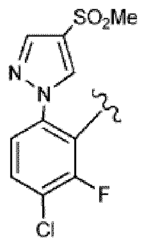, 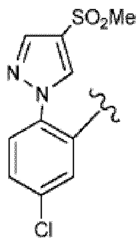, 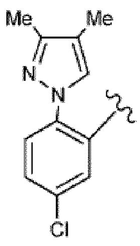, 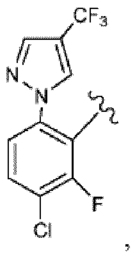, 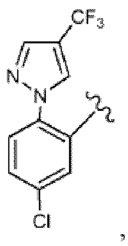, 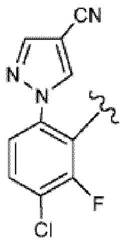,

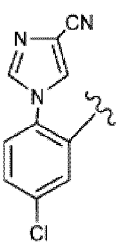, 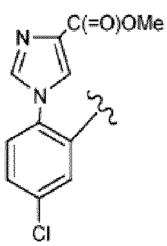, 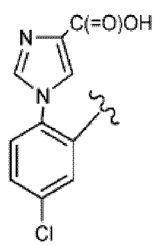, 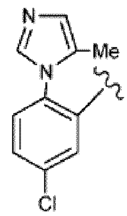, 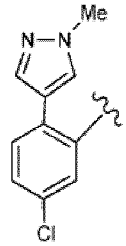, 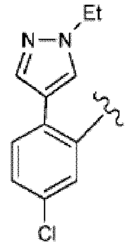, 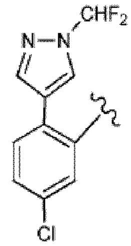,

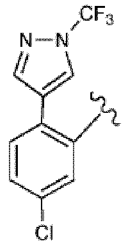, 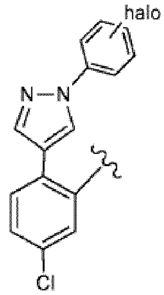, 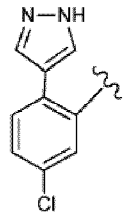 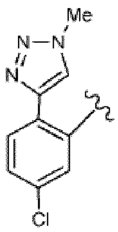, 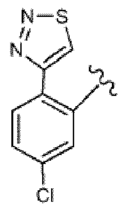, 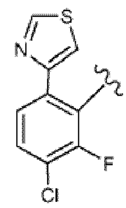, 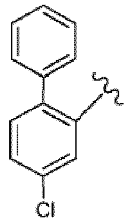, 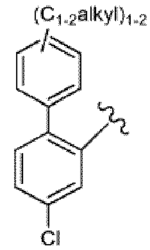

, 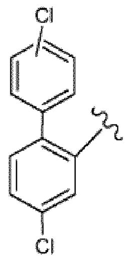, 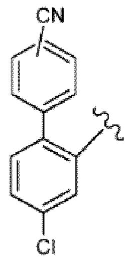,

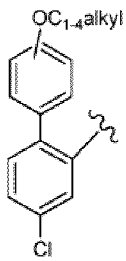, 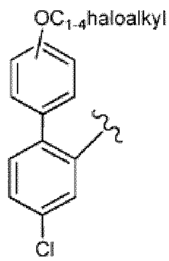, 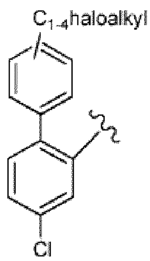, 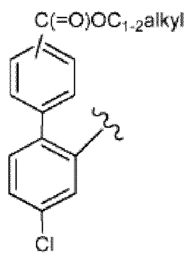, 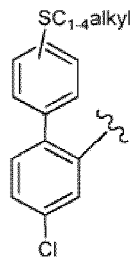, 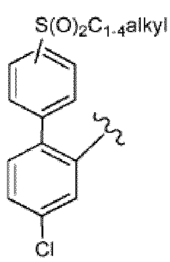,

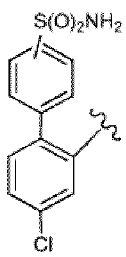, 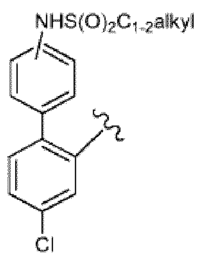, 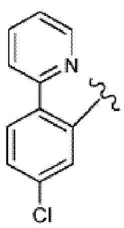, 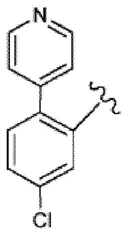, 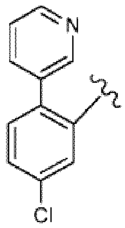, 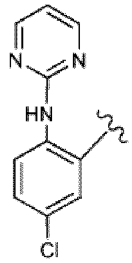, 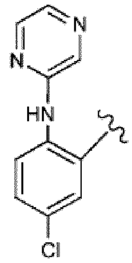,

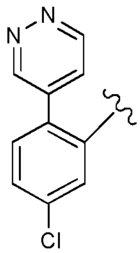, 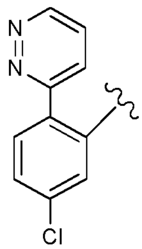, 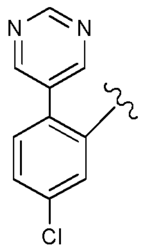, 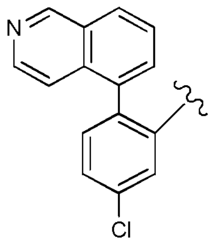 und 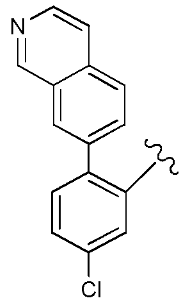;

W unabhängig ausgewählt ist aus $CHR^1$, O, NH und N($C_{1-4}$-Akyl);
Y unabhängig ausgewählt ist aus $-CH_2NH-$, -NHC(=O)- und -C(=O)NH-;
$R^1$ und $R^2$ unabhängig ausgewählt sind aus H, F, $C_{1-4}$-Akyl und Hydroxyl;
$R^3$ unabhängig ausgewählt ist aus H, =O, F, $CHF_2$, $CF_3$, $CH_3$, CN, $-(CH_2)_{0-2}$-OH, $OC_{1-4}$-Akyl, C(=O)$C_{1-4}$-Akyl, $-(CH_2)_{0-1}$-C(=O)OH und -C(=O)$OC_{1-4}$-Akyl;
$R^{3c}$ unabhängig ausgewählt ist aus H, $CF_2H$, $CF_3$ und $C_{1-4}$-Akyl;
$R^4$ unabhängig ausgewählt ist aus H, F, und $C_{1-4}$-Akyl;und
$R^7$ H ist.

6. Pharmazeutische Zusammensetzung, umfassend eine oder mehrere Verbindungen nach einem der Ansprüche 1 bis 5 und einen pharmazeutisch verträglichen Träger oder Verdünnungsmittel dafür.

7. Verbindung nach einem der Ansprüche 1 bis 5 oder ein Stereoisomer, ein Tautomer oder ein pharmazeutisch verträgliches Salz davon oder die pharmazeutische Zusammensetzung nach Anspruch 6 zur Verwendung in der Therapie.

8. Verbindung nach einem der Ansprüche 1 bis 5 oder ein Stereoisomer, ein Tautomer oder ein pharmazeutisch verträgliches Salz davon oder die pharmazeutische Zusammensetzung nach Anspruch 6 zur Verwendung in der Behandlung und/oder Prophylaxe einer thromboembolischen Erkrankung, umfassend: Verabreichen an einen Patient mit Bedarf dafür einer therapeutisch wirksamen Menge der Verbindung, des Stereoisomers, Tautomers oder des pharmazeutischen verträglichen Salzes oder der pharmazeutischen Zusammensetzung, wobei die thromboembolische Erkrankung ausgewählt ist aus arteriellen kardiovaskulären thromboembolischen Erkrankungen, venösen kardiovaskulären thromboembolischen Erkrankungen und thromboembolischen Erkrankungen in den Herzkammern oder in dem peripheren Kreislauf.

9. Verbindung oder pharmazeutische Zusammensetzung zur Verwendung nach Anspruch 8, wobei die thromboembolische Erkrankung ausgewählt ist aus instabiler Angina, einem akutem koronarem Syndrom, Vorhofflimmern, Myokardinfarkt, transientem ischämischem Anfall, Schlaganfall, Atherosklerose, peripherer arterieller Verschlusskrankheit, venöser Thrombose, tiefer Venenthrombose, Thrombophlebitis, arterieller Embolie, koronarer arterieller Thrombose, zerebraler arterieller Thrombose, Hirnembolie, Nierenembolie, Lungenembolie und Thrombose aus medizinischen Implantaten, Vorrichtungen oder Verfahren, bei denen Blut einer Thrombose fördernden künstlichen Oberfläche ausgesetzt wird.

## Revendications

1. Composé ayant la Formule (IIb)

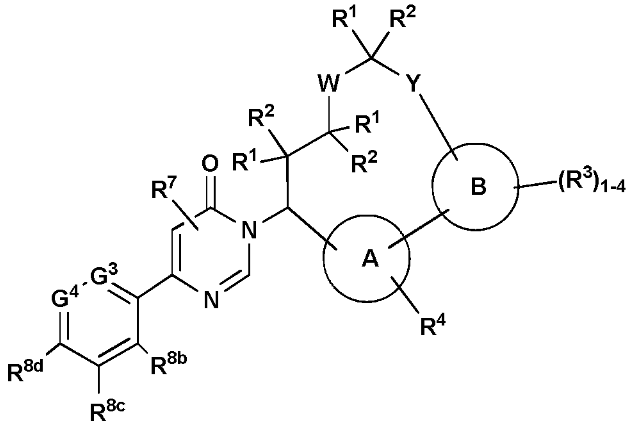

(IIb)

ou un stéréoisomère, un tautomère, ou un sel pharmaceutiquement acceptable de celui-ci, dans lequel :

le cycle A est indépendamment choisi parmi un phényle et un hétérocyclyle de 5 à 6 chaînons ;
le cycle B est un hétéroaryle de 5 à 6 chaînons comprenant des atomes de carbone et 1-4 hétéroatomes choisis parmi N et $NR^{3c}$ ;
W est indépendamment choisi parmi $(CR^1R^2)_{1-2}$, O, NH et $N(C_{1-4}$ alkyle) ;
Y est indépendamment choisi parmi $-CH_2NH-$, -NHC(=O)- et -C(=O)NH- ;
$G^3$ est un $CR^{8a}$ ;
$G^4$ est un $CR^{8e}$ ;
$R^1$ et $R^2$ sont indépendamment choisis parmi H, D, halogène, $CF_3$, $C_{1-6}$ alkyle et hydroxyle ;
$R^3$ est indépendamment choisi parmi H, halogène, $C_{1-4}$ alkyle (optionnellement substitué par $R^6$), CN, $-(CH_2)_n-OR^5$, $-(CH_2)_n-C(=O)R^5$ et $-(CH_2)_n-C(=O)OR^5$ ;
$R^{3c}$ est indépendamment choisi parmi H, haloalkyle, $C_{1-4}$ alkyle (optionnellement substitué par $R^6$), $-(CH_2)_{1-2}-OH$, $C(=O)C_{1-4}$ alkyle, $-(CH_2)_{1-2}-C(=O)OH$, $-C(=O)OC_{1-4}$ alkyle, $S(=O)_pC_{1-6}$ alkyle, $-(CH_2)_n-C_{3-10}$ carbocyclyle et $-(CH_2)_n-$(hétérocyclyle de 4 à 10 chaînons), dans lequel lesdits carbocyclyle et hétérocyclyle sont optionnellement substitués par $R^6$ ;
$R^4$ est indépendamment choisi parmi H, OH, F, Cl, Br, $C_{1-4}$ alkyle, $C_{1-4}$ alcoxy, $CF_3$, CN, $C(=O)NH_2$, $C_{3-6}$ cycloalkyle, aryle et hétérocyclyle de 5 à 6 chaînons, dans lequel lesdits cycloalkyle, aryle et hétérocyclyle sont optionnellement substitués par $R^6$ ;
$R^5$ est indépendamment choisi parmi H et $C_{1-4}$ alkyle ;
$R^6$ est indépendamment choisi parmi H, $-(CH_2)_n-OH$, =O, $NH_2$, $-(CH_2)_n-CN$, halogène, $C_{1-6}$ alkyle, $-(CH_2)_n-C(=O)OH$, $-(CH_2)_n-C(=O)OC_{1-4}$ alkyle, $-(CH_2)_n-OC_{1-4}$ alkyle, $-(CH_2)_n-C_{3-6}$ cycloalkyle, $-(CH_2)_n-$(hétérocyclyle de 4 à 10 chaînons) et $-O-(CH_2)_n-$(hétérocyclyle de 4 à 10 chaînons), dans lequel lesdits cycloalkyle et hétérocyclyle sont optionnellement substitués par $R^{10}$ ;
$R^7$ est indépendamment choisi parmi H, F, Cl, Br et méthyle ;
$R^{8a}$ est indépendamment choisi parmi H, F, Cl, Br, I, $-(CH_2)_nCN$, $-(CH_2)_nNH_2$, $CH_3CHF_2$, $CCH_3F_2$, $CF_3$, OH, $OCH_3$, $OCF_3$, $OCHF_2$, $-C(=O)CH_3$, -C(=O)OH, $-C(=O)OCH_3$, $-C(=O)NH_2$, $-C(=O)NHCH_2CF_3$, $-C(=O)NHCH_2Ph$, $-NHC(=O)OCH_3$, $NHC(=O)CF_3$,

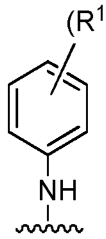 , 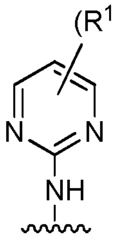 , 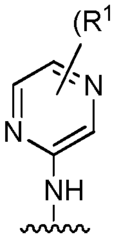 ,

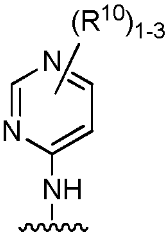, 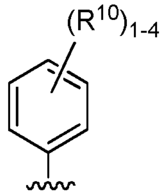,

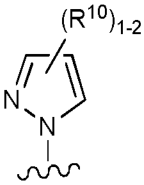, 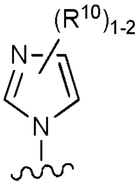, 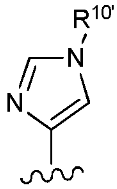, 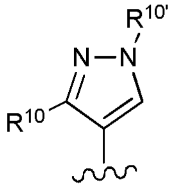, 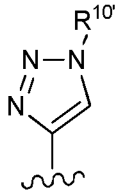, 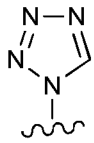, 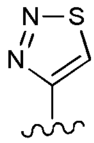,

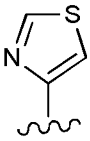, 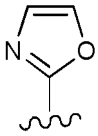, 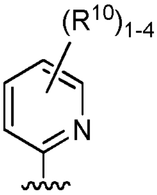, 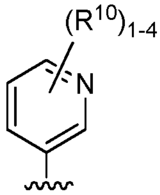, 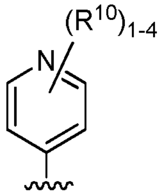, 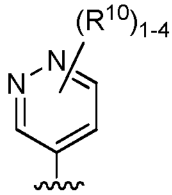, 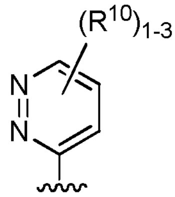,

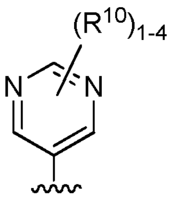, 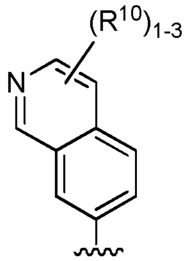, 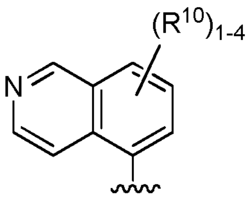 et 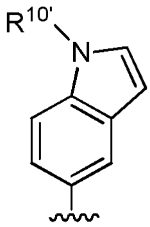 ;

$R^{8b}$ est indépendamment choisi parmi H et F ;

$R^{8c}$ est indépendamment choisi parmi H, F, Cl, méthyle, éthyle, isopropyle, $OCHF_2$ et $OCH_3$ ;

$R^{8d}$ est indépendamment choisi parmi H, F et Cl ;

$R^{8e}$ est indépendamment choisi parmi H, F et Cl ;

$R^{10}$ est indépendamment choisi parmi H, $C_{1-6}$ alkyle (optionnellement substitué par $R^{11}$), $C_{2-6}$ alcényle, $C_{2-6}$ alcynyle, aryle (optionnellement substitué par $R^{11}$), $-(CH_2)_n-C_{3-6}$ cycloalkyle (optionnellement substitué par $R^{11}$), $-(CH_2)_n$-O-(hétérocyclyle de 4 à 10 chaînons) (optionnellement substitué par $R^{11}$), F, Cl, Br, CN, $NO_2$, =O, $CONR^{12}R^{12}$, $-(CH_2)_n-C(=O)OR^{12}$, $Si(C_{1-4}\ alkyle)_3$, $-(CH_2)_n-OR^{12}$, $-(CH_2)_n-NR^{12}R^{12}$, $-S(=O)_pC_{1-6}$ alkyle, $NR^{12}S(=0)_pC_{1-6}$ alkyle et $S(=O)_pNR^{12}R^{12}$ ;

$R^{10}$ est indépendamment choisi parmi H, $C_{1-6}$ alkyle (optionnellement substitué par $R^{11}$), aryle, $-(CH_2)_n-C_{3-6}$ cycloalkyle (optionnellement substitué par $R^{11}$) et $-(CH_2)_n$-O-(hétérocyclyle de 4 à 10 chaînons) (optionnellement substitué par $R^{11}$) ;

$R^{11}$, à chaque occurrence, est indépendamment choisi parmi H, halogène, $C_{1-5}$ alkyle, $-(CH_2)_n-OH$, $C_{3-6}$ cycloalkyle et phényle ;

$R^{12}$, à chaque occurrence, est indépendamment choisi parmi H, $C_{1-5}$ alkyle optionnellement substitué par $R^{11}$, $C_{3-6}$ cycloalkyle, phényle et hétérocyclyle, ou $R^{12}$ et $R^{12}$ avec l'atome d'azote auquel ils sont liés tous les deux forment un cycle hétérocyclique optionnellement substitué par $C_{1-4}$ alkyle ;

n, à chaque occurrence, est un entier indépendamment choisi parmi 0, 1 et 2 ; et

p, à chaque occurrence, est un entier indépendamment choisi parmi 0, 1 et 2.

**2.** Composé ayant la Formule (IIIb) :

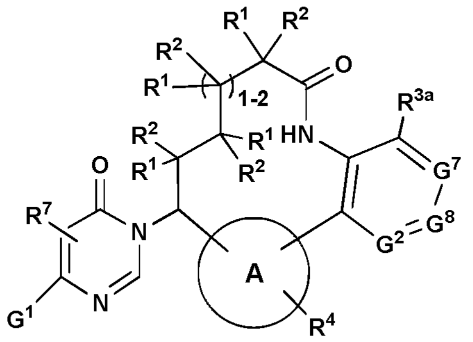

(IIIb)

ou un stéréoisomère, un tautomère, ou un sel pharmaceutiquement acceptable de celui-ci, dans lequel :

le cycle A est indépendamment choisi parmi phényle et un hétérocyclyle de 5 à 6 chaînons ;
$G^1$ est indépendamment choisi parmi aryle, $C_{3-6}$ cycloalkyle et hétérocyclyle de 5 à 6 chaînons, dans lequel lesdits aryle, cycloalkyle et hétérocyclyle sont substitués par 1-4 $R^8$ ;
$G^2$ est indépendamment choisi parmi N et $CR^{3b}$ ;
$G^7$ est indépendamment choisi parmi N et $CR^3$ ;
$G^8$ est indépendamment choisi parmi N et $CR^3$ ;
à condition qu'au moins l'un de $G^2$, $G^7$ et $G^8$ soit N ;
$R^1$ et $R^2$ sont indépendamment choisis parmi H, halogène, $CF_3$, $C_{1-6}$ alkyle et hydroxyle ;
$R^3$ est indépendamment choisi parmi H, halogène, haloalkyle, $C_{1-4}$ alkyle (optionnellement substitué par $R^6$), $C_{2-4}$ alcényle (optionnellement substitué par $R^6$), CN, $NO_2$, $-(CH_2)_n-OR^5$, $-(CH_2)_n-NR^5R^5$, $-(CH_2)_n-C(=O)OR^5$, $-(CH_2)_n-NHC(=O)OR^5$, $-(CH_2)_n-NHC(=O)R^5$ $-(CH_2)_n-NHC(N-CN)NHR^5$, $-(CH_2)_n-NHC(NH)NHR^5$, $-(CH_2)_n-N=CHNR^5R^5$, $-(CH_2)_n-NHC(=O)NR^5R^5$, $-(CH_2)_n-C(=O)NR^5R^5$, $-(CH_2)_n-NHC(S)NR^9C(=O)R^5$, $-(CH_2)_n-S(=O)_pC_{1-6}$ alkyle optionnellement substitué par $R^{11}$, $-(CH_2)_n-S(=O)_pNR^5R^5$, $-(CH_2)_n-NHS(=O)_pNR^5R^5$, $-(CH_2)_n-NHS(=O)_pC_{1-6}$ alkyle optionnellement substitué par $R^{11}$, $-(CH_2)_n-C_{3-10}$ carbocyclyle et $-(CH_2)_n-$(hétérocyclyle de 4 à 10 chaînons), dans lequel lesdits carbocyclyle et hétérocyclyle sont optionnellement substitués par $R^6$, deux groupes $R^3$ adjacents sur le carbocyclyle et l'hétérocyclyle peuvent former un cycle optionnellement substitué par $R^6$ ;
$R^{3a}$ est indépendamment choisi parmi H et halogène ;
$R^{3b}$ est indépendamment choisi parmi H, halogène, méthyle et CN ;
$R^4$ est indépendamment choisi parmi H, OH, F, Cl, Br, $C_{1-4}$ alkyle, $C_{1-4}$ alcoxy, $CF_3$, CN, $C_{3-6}$ cycloalkyle, aryle et hétérocyclyle de 5 à 6 chaînons, dans lequel lesdits cycloalkyle, aryle et hétérocyclyle sont optionnellement substitués par $R^6$ ;
$R^5$ est indépendamment choisi parmi H, $C_{1-4}$ alkyle (optionnellement substitué par halogène, hydroxyle, alcoxy, carboxy, alcoxycarbonyle, amino, amino substitué), $-(CH_2)-_n-C_{3-10}$ carbocyclyle et $-(CH_2)_n-$(hétérocyclyle de 4 à 10 chaînons), dans lequel lesdits carbocyclyle et hétérocyclyle sont optionnellement substitué par $R^6$ ;
$R^6$ est indépendamment choisi parmi $-(CH_2)_n-OH$, =O, $NH_2$, $-(CH_2)_n-CN$, halogène, $C_{1-6}$ alkyle, $-(CH_2)_n-C(=O)OH$, $-(CH_2)_n-C(=O)OC_{1-4}$ alkyle, $-(CH_2)_n-OC_{1-4}$ alkyle, $-(CH_2)_n-C_{3-6}$ cycloalkyle, $-(CH_2)_n-$(hétérocyclyle de 4 à 10 chaînons) et $-O-(CH_2)_n-$(hétérocyclyle de 4 à 10 chaînons), dans lequel lesdits cycloalkyle et hétérocyclyle sont optionnellement substitués par $R^{10}$ ;
$R^7$ est indépendamment choisi parmi H, F, Cl et méthyle ;
$R^8$ est indépendamment choisi parmi H, halogène, CN, $NH_2$, $C_{1-6}$ alkyle, haloalkyle, alkylcarbonyle, alcoxy, haloalcoxy, aryle, $C_{3-6}$ cycloalkyle et hétérocyclyle de 4 à 12 chaînons, dans lequel lesdits aryle, cycloalkyle et hétérocyclyle sont optionnellement substitués par $R^{10}$ ;
$R^9$ est H ou $C_{1-6}$ alkyle ;
$R^{10}$ est indépendamment choisi parmi H, $C_{1-6}$ alkyle (optionnellement substitué par $R^{11}$), $C_{2-6}$ alcényle, $C_{2-6}$ alcynyle, aryle, $-(CH_2)_n-C_{3-6}$ cycloalkyle (optionnellement substitué par $R^{11}$), $-(CH_2)_n-O-$(hétérocyclyle de 4 à 10 chaînons) (optionnellement substitué par $R^{11}$), F, Cl, Br, CN, $NO_2$, =O, $-C(=O)NR^{12}R^{12}$, $-C(=O)OR^{12}$, $-Si(C_{1-4}$ alkyle)3, $-(CH_2)_n-OR^{12}$, $-(CH_2)_n-NR^{12}R^{12}$, $-S(=O)_pC_{1-6}$ alkyle, $NR^{12}S(=O)_pC_{1-6}$ alkyle et $S(=O)_pNR^{12}R^{12}$ ;
$R^{11}$, à chaque occurrrence, est indépendamment choisi parmi H, halogène, $C_{1-5}$ alkyle, $-(CH_2)_n-OH$, $C_{3-6}$ cycloalkyle et phényle ;
$R^{12}$, à chaque occurrence, est indépendamment choisi parmi H, $C_{1-5}$ alkyle, $C_{3-6}$ cycloalkyle, phényle et hétérocyclyle, ou $R^{12}$ et $R^{12}$ avec l'atome d'azote auquel ils sont liés tous les deux forment un cycle hétérocyclique optionnellement substitué par $C_{1-4}$ alkyle ;

n, à chaque occurrence, est un entier indépendamment choisi parmi 0, 1 et 2 ; et
p, à chaque occurrence, est un entier indépendamment choisi parmi 0,1 et 2.

**3.** Composé ayant la Formule (IVa) :

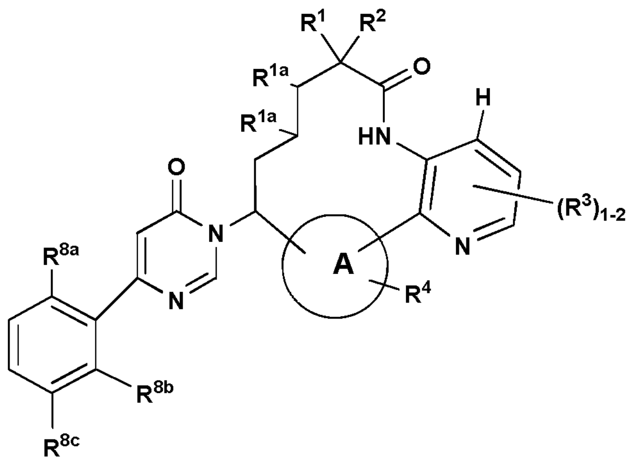

(IVa)

ou un stéréoisomère, un tautomère, ou un sel pharmaceutiquement acceptable de celui-ci, dans lequel :

le cycle A est indépendamment choisi parmi

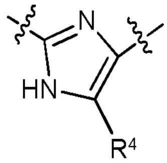
,
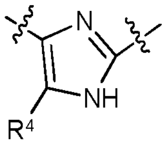
,

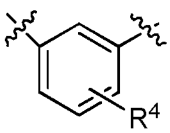
,
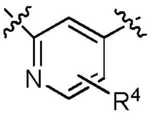
,
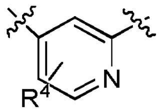
,
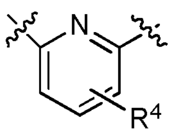
,
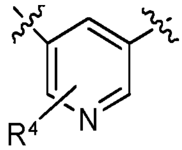
,
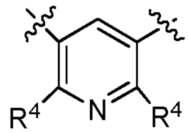
,

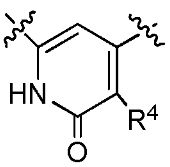
,
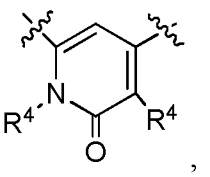
,
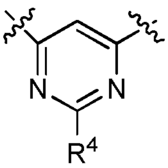
,
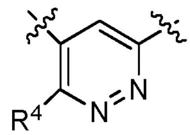
et
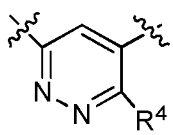
;

$R^1$ et $R^2$ sont indépendamment choisis parmi H, F, $C_{1-4}$ alkyle et OH ;
$R^{1a}$, à chaque occurrence, est indépendamment choisi parmi H, F, $CH_3$ et OH ;
$R^3$ est indépendamment choisi parmi H, F, Cl, Br, I, $C_{2-4}$ alcényle (optionnellement substitué par C(=O)OH), CN et $-(CH_2)n-OH$ ;
$R^4$ est indépendamment choisi parmi H, OH, F, $OC_{1-4}$ alkyle, $C_{1-4}$ alkyle, CN, $C_{3-6}$ cycloalkyle, aryle et hétérocyclyle de 5 à 6 chaînons, dans lequel lesdits cycloalkyle, aryle et hétérocyclyle sont optionnellement substitués par $R^6$ ;
$R^6$ est indépendamment choisi parmi OH, $NH_2$, halogène, $C_{1-6}$ alkyle, $C_{3-6}$ cycloalkyle, $-(CH_2)_n-C(=O)OH$, $-(CH_2)_n-C(=O)OC_{1-4}$ alkyle, $-(CH_2)_n-OC_{1-4}$ alkyle, =O, $C_{3-6}$ cycloalkyle, hétérocyclyle de 4 à 10 chaînons et -O-(hétérocyclyle de 4 à 10 chaînons), dans lequel lesdits cycloalkyle et hétérocyclyle sont optionnellement substitués par $R^{10}$ ;
$R^{8a}$ est choisi indépendamment parmi H, F, Cl, Br, CN, $OCH_3$, $OCF_3$, $CH_3$, $C(=O)CH_3$, $CF_3$, $OCHF_2$,

$NHC(=O)C_{1-4}$ alkyle, aryle, $C_{3-6}$ cycloalkyle et hétérocyclyle de 4 à 12 chaînons, dans lequel lesdits aryle, cycloalkyle et hétérocyclyle sont optionnellement substitués par $R^{10}$ ;
$R^{8b}$ est indépendamment choisi parmi H et F ;
$R^{8c}$ est indépendamment choisi parmi H, F, Cl, $CH_3$ et $OCH_3$ ;
$R^{10}$ est indépendamment choisi parmi $C_{1-6}$ alkyle, $-C_{3-6}$ cycloalkyle, F, Cl, Br, $CF_3$, $CHF_2$, CN et $OC_{1-5}$ alkyle ; et
n, à chaque occurrence, est un entier indépendamment choisi parmi 0, 1 et 2.

**4.** Composé selon la revendication 1, ou un stéréoisomère, un tautomère, ou un sel pharmaceutiquement acceptable de celui-ci, dans lequel :

le cycle A est indépendamment choisi parmi

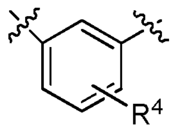 , 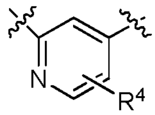 ,

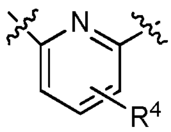 , 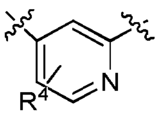 et 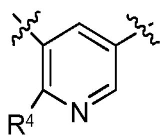 ;

le cycle B est indépendamment choisi parmi

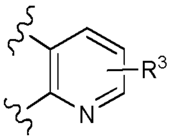 , 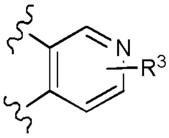 ,

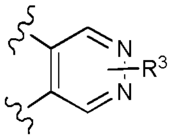 , 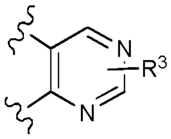 , 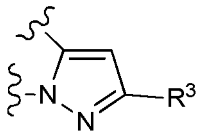 , 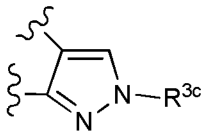 , 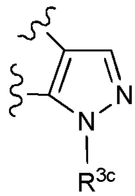 et 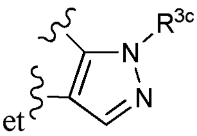 ;

W est indépendamment choisi parmi $CHR^{1a}$, O, NH et $N(C_{1-4}$ alkyle) ;
$R^1$ est indépendamment choisi parmi H et $C_{1-4}$ alkyle ;
$R^{1a}$ est indépendamment choisi parmi H, F, $CH_3$ et hydroxyle ;
$R^2$ est indépendamment choisi parmi H et hydroxyle ;
$R^3$ est indépendamment choisi parmi H, F, $CHF_2$, $CF_3$, $CH_3$, CN, $-(CH_2)_{0-2}-OH$, $OC_{1-4}$ alkyle, $C(=O)C_{1-4}$ alkyle, $-(CH_2)_{0-1}-C(=O)OH$ et $-C(=O)OC_{1-4}$ alkyle ;
$R^{3c}$ est indépendamment choisi parmi H, $CF_2H$, $CF_3$ et $C_{1-4}$ alkyle ;
$R^4$ est indépendamment choisi parmi H et F ;
$R^{8b}$ est indépendamment choisi parmi H et F ;
$R^{8c}$ est indépendamment choisi parmi H et Cl ;
$R^{10}$ est indépendamment choisi parmi H, $C_{1-6}$ alkyle (optionnellement substitué par $R^{11}$), aryle, $-(CH_2)_n-C_{3-6}$ cycloalkyle (optionnellement substitué par $R^{11}$), $-(CH_2)_n-O-$(hétérocyclyle de 4 à 10 chaînons), F, Cl, Br, CN, $-C(=O)NR^{12}R^{12}$, $-Si(C_{1-4}$ alkyle$)_3$ et $-(CH_2)_n-OR^{12}$ ;
$R^{11}$, à chaque occurrence, est indépendamment choisi parmi H, halogène et $C_{1-5}$ alkyle ; et
n, à chaque occurrence, est un entier indépendamment choisi parmi 0, 1 et 2.

**5.** Composé selon la revendication 1, ou un stéréoisomère, un tautomère, ou un sel pharmaceutiquement acceptable de celui-ci, dans lequel :

le cycle A est indépendamment choisi parmi

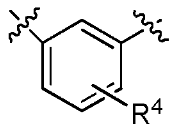, 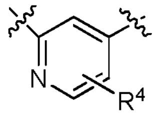,

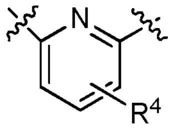, 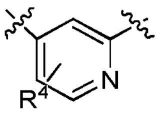 et 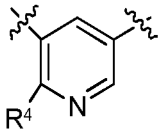;

le cycle B est indépendamment choisi parmi

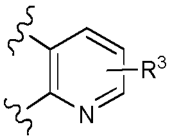, 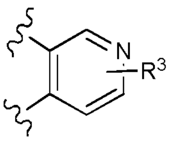,

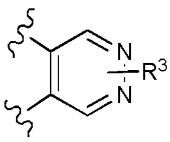, 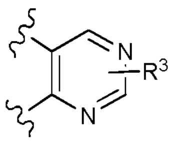, 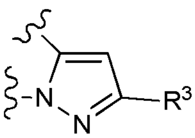, 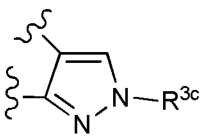, 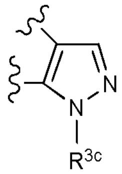 et 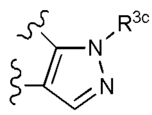;

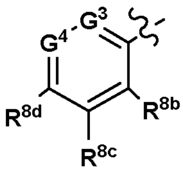

est indépendamment choisi parmi

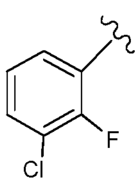, 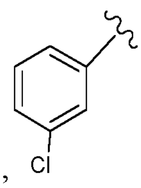, 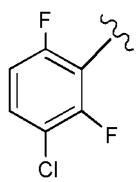

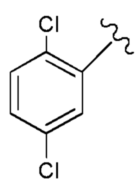,

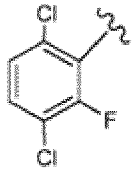 , 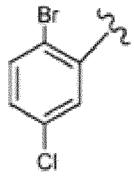 , 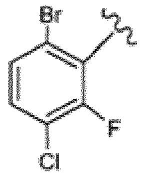 , 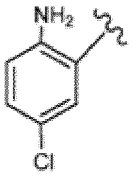 , 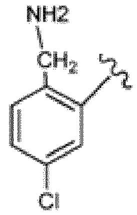 , 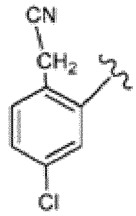 , 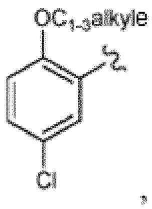 ,

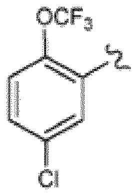 , 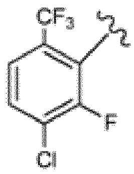 , 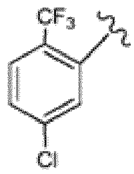 , 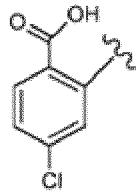 , 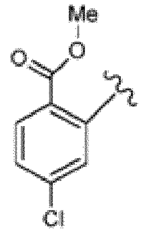 , 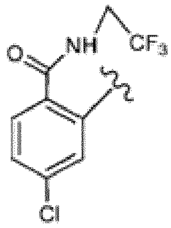 , 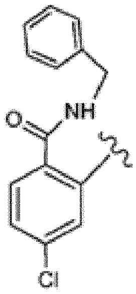 ,

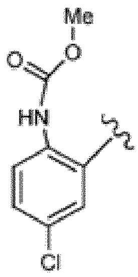 , 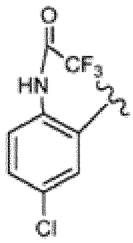 , 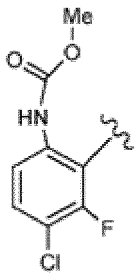 , 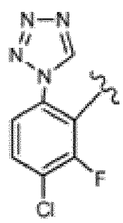 , 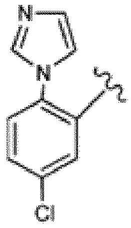 ,

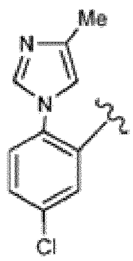 , 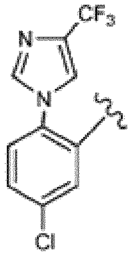 , 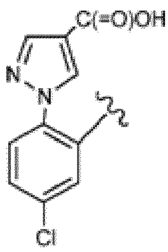 , 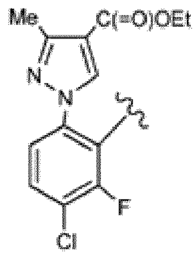 , 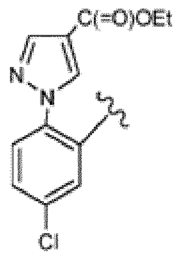 ,

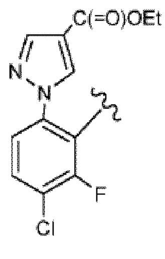 , 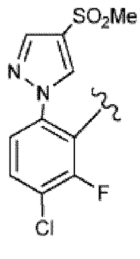 , 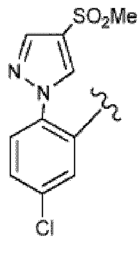 , 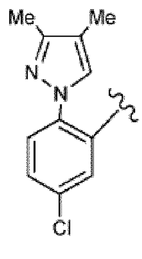 , 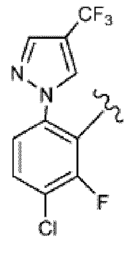 , 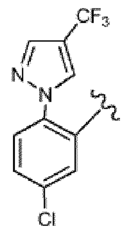 , 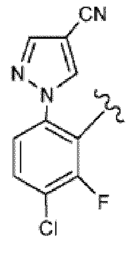 ,

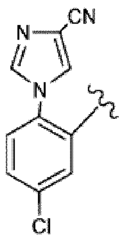 , 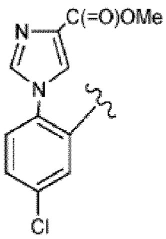 , 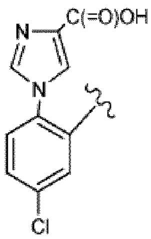 , 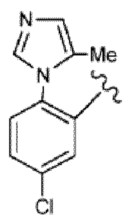 , 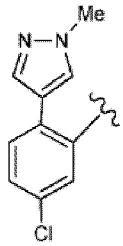 , 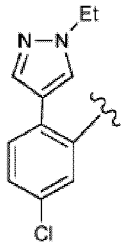 , 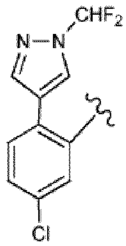 ,

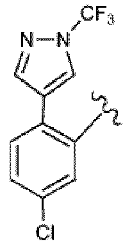 , 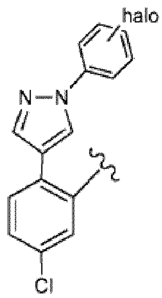 , 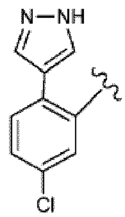 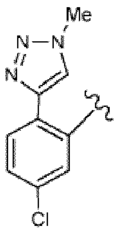 , 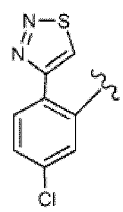 , 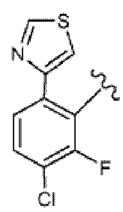 , 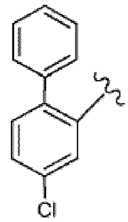 , 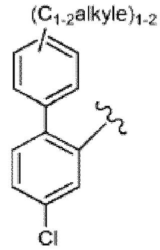

, 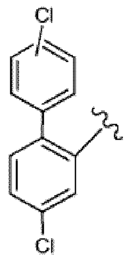 , 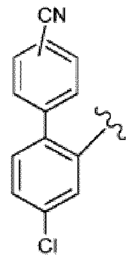 ,

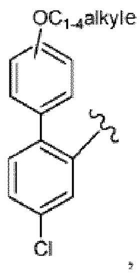 , 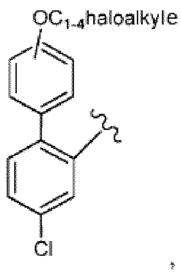 , 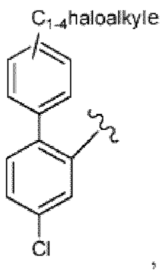 , 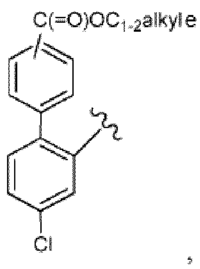 , 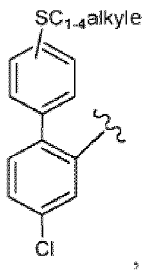 , 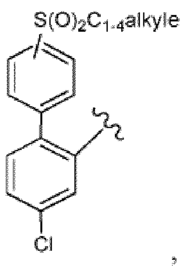 ,

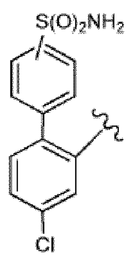 , 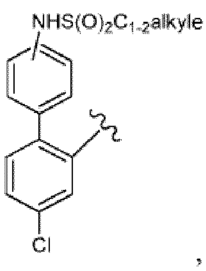 , 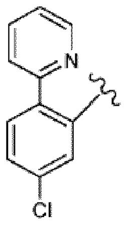 , 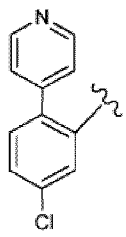 , 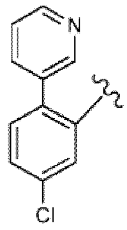 , 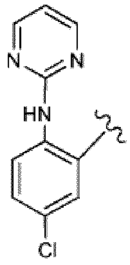 , 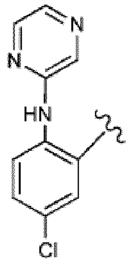 ,

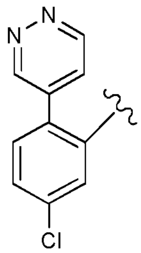 , 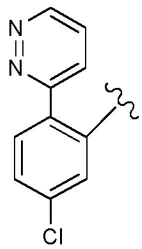 , 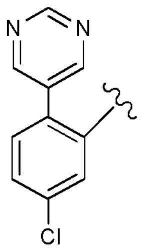 , 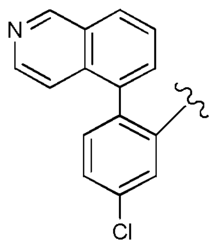 et 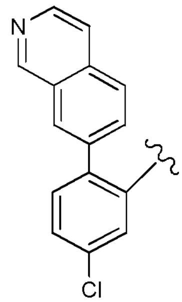 ;

W est indépendamment choisi parmi $CHR^1$, O, NH et N($C_{1-4}$ alkyle) ;
Y est indépendamment choisi parmi $-CH_2NH-$, -NHC(=O)- et -C(=O)NH- ;
$R^1$ et $R^2$ sont indépendamment choisis parmi H, F, $C_{1-4}$ alkyle et hydroxyle ;
$R^3$ est indépendamment choisi parmi H, =O, F, $CHF_2$, $CF_3$, $CH_3$, CN, $-(CH_2)_{0-2}-OH$, $OC_{1-4}$ alkyle, C(=O)$C_{1-4}$ alkyle, $-(CH_2)_{0-1}-C(=O)OH$ et -C(=O)$OC_{1-4}$ alkyle ;
$R^{3c}$ est indépendamment choisi parmi H, $CF_2H$, $CF_3$ et $C_{1-4}$ alkyle ;
$R^4$ est indépendamment choisi parmi H, F et $C_{1-4}$ alkyle ; et
$R^7$ est H.

**6.** Composition pharmaceutique comprenant un ou plusieurs composés selon l'une quelconque des revendications 1-5 et un véhicule ou diluant pharmaceutiquement acceptable.

**7.** Composé selon l'une quelconque des revendications 1-5, ou un stéréoisomère, un tautomère ou un sel pharmaceutiquement acceptable de celui-ci, ou la composition selon la revendication 6, pour une utilisation en thérapie.

**8.** Composé selon l'une quelconque des revendications 1-5, ou un stéréoisomère, un tautomère ou un sel pharmaceutiquement acceptable de celui-ci, ou la composition selon la revendication 6, pour une utilisation dans le traitement et/ou la prophylaxie d'un trouble thromboembolique, comprenant : l'administration à un patient en ayant besoin d'une quantité thérapeutiquement efficace du composé, stéréoisomère, tautomère, ou sel pharmaceutiquement acceptable de celui-ci, ou de la composition pharmaceutique, dans lequel le trouble thromboembolique est choisi parmi des troubles thromboemboliques cardiovasculaires artériels, des troubles thromboemboliques cardiovasculaires veineux et des troubles thromboemboliques dans les chambres du cœur ou dans la circulation périphérique.

**9.** Composé ou composition pharmaceutique pour une utilisation selon la revendication 8, dans lequel le trouble thromboembolique est choisi parmi un angor instable, un syndrome coronarien aigu, une fibrillation auriculaire, un infarctus du myocarde, une attaque ischémique transitoire, un accident vasculaire cérébral, l'athérosclérose, une maladie artérielle occlusive périphérique, une thrombose veineuse, une thrombose veineuse profonde, une thrombophlébite, une embolie artérielle, une thrombose artérielle coronarienne, une thrombose artérielle cérébrale, une embolie cérébrale, une embolie rénale, une embolie pulmonaire et une thrombose résultant d'implants, dispositifs ou protocoles médicaux dans lesquels du sang est exposé à une surface artificielle qui favorise une thrombose.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040180855 A1
- WO 9857951 A
- WO 03026652 A
- WO 01047919 A
- WO 00076970 A
- WO 9837075 A
- WO 02044145 A
- EP 028489 A


### Non-patent literature cited in the description


- **GAILANI, D. et al.** *Arterioscler. Thromb. Vasc. Biol.,* 2007, vol. 27, 2507-2513
- **HOFFMAN, M.** *Blood Reviews,* 2003, vol. 17, S1-S5
- **LEHMANN, A.** Ecallantide (DX-88), a plasma kallikrein inhibitor for the treatment of hereditary angioedema and the prevention of blood loss in on-pump cardiothoracic surgery. *Expert Opin. Biol. Ther.,* 2008, vol. 8, 1187-1199
- **CLERMONT, A. et al.** Plasma kallikrein mediates retinal vascular dysfunction and induces retinal thickening in diabetic rats. *Diabetes,* 2011, vol. 60, 1590-1598
- IUPAC Recommendations 1996. *Pure and Applied Chemistry,* 1996, vol. 68, 2193-2222
- Hawley's Condensed Chemical Dictionary. John Wiley & Sons, Inc, 1997
- Remington's Pharmaceutical Sciences. Mack Publishing Company, 1990
- Design of Prodrugs. Elsevier, 1985
- Methods in Enzymology. Academic Press, 1985, vol. 112, 309-396
- Design and Application of Prodrugs. **BUNDGAARD, H. et al.** A Textbook of Drug Design and Development. Harwood Academic Publishers, 1991, 113-191
- **BUNDGAARD, H.** *Adv. Drug Deliv. Rev.,* 1992, vol. 8, 1-38
- **BUNDGAARD, H. et al.** *J. Pharm. Sci.,* 1988, vol. 77, 285
- **KAKEYA, N. et al.** *Chem. Pharm. Bull.,* 1984, vol. 32, 692
- Medicinal Chemistry: Principles and Practice. The Royal Society of Chemistry, 1994
- Hydrolysis in Drug and Prodrug Metabolism. **TESTA, B. et al.** Chemistry, Biochemistry and Enzymology. VCHA and Wiley-VCH, 2003
- The Practice of Medicinal Chemistr. Academic Press, 1999
- Hemostasis and Thrombosis, Basic Principles and Clinical Practice. Lippincott Williams & Wilkins, 2006, 853
- **HIRSH, J. et al.** *Blood,* 2005, vol. 105, 453-463
- **SHARIAT-MADAR et al.** *Blood,* 2006, vol. 108, 192-199
- Contact Activation Pathway. **COLEMAN, R.** Hemostasis and Thrombosis. Lippincott Williams & Wilkin, 2001, 103-122
- **SCHMAIER, A.H.** Contact Activation. *Thrombosis and Hemorrhage,* 1998, 105-128
- **RENNE et al.** *J. Exp. Med.,* 2005, vol. 202, 271-281
- **KLEINSCHMITZ et al.** *J. Exp. Med.,* 2006, vol. 203, 513-518
- **GALIANI, D.** *Trends Cardiovasc. Med.,* 2000, vol. 10, 198-204
- **BOUMA, B.N. et al.** *Thromb. Res.,* 2001, vol. 101, 329-354
- **ROSEN et al.** *Thromb. Haemost.,* 2002, vol. 87, 774-777
- **WANG et al.** *J. Thromb. Haemost.,* 2005, vol. 3, 695-702
- **CHAN et al.** *Amer. J. Pathology,* 2001, vol. 158, 469-479
- **GRUBER et al.** *Blood,* 2003, vol. 102, 953-955
- **GAILANI, D.** *Frontiers in Bioscience,* 2001, vol. 6, 201-207
- **GAILANI, D. et al.** *Blood Coagulation and Fibrinolysis,* 1997, vol. 8, 134-144
- **MINNEMA, M.C. et al.** *Arterioscler. Thromb. Vasc. Biol.,* 2000, vol. 20, 2489-2493
- **MURAKAMI, T. et al.** *Arterioscler. Thromb. Vasc. Biol.,* 1995, vol. 15, 1107-1113
- **MEIJERS, J.C.M. et al.** *N. Engl. J. Med.,* 2000, vol. 342, 696-701
- Screening Tests of Hemostasis. **GOODNIGHT, S.H. et al.** Disorders of Thrombosis and Hemostasis: A Clinical Guide. McGraw-Hill, 2001, 41-51

- **WONG P.C. et al.** *Journal of Thrombosis and Thrombolysis,* August 2011, vol. 32 (2), 129-137
- **SCHUMACHER, W. et al.** *Journal of Thrombosis and Haemostasis,* 2005, vol. 3 (1), 1228
- **SCHUMACHER, W.A. et al.** *Eur. J. Pharmacol.,* 2007, 167-174
- **LEVITAN, N. et al.** *Medicine (Baltimore),* 1999, vol. 78 (5), 285-291
- **LEVINE M. et al.** *N. Engl. J. Med.,* 1996, vol. 334 (11), 677-681
- **BLOM, J.W. et al.** *JAMA,* 2005, vol. 293 (6), 715-722
- **MISMETTI, P. et al.** *British Journal of Surgery,* 2001, vol. 88, 913-930
- **WONG et al.** *J. Pharmacol. Exp. Ther.,* 2000, vol. 295, 212-218
- **WONG, P.C. et al.** *J. Pharmacol. Exp. Ther.,* 2000, vol. 292, 351-357
- Remington's Pharmaceutical Sciences. 1990
- Remington's Pharmaceutical Sciences. Mack Publishing Company
- **MAFFRAND, J.P. et al.** *Heterocycles,* 1981, vol. 16 (1), 35-37
- **GREENE et al.** Protective Groups in Organic Synthesis. Wiley-Interscience, 2006
- *Organic Letters,* 2009, vol. 11, 1421
- *Synthesis,* 1996, vol. 991
- **ELLMAN, J.** *J. Org. Chem.,* 1999, vol. 64, 1278
- **KUDUK.** *Tetrahedron Letters,* 2004, vol. 45, 6641
- **XU, M-H.** *Organic Letters,* 2008, vol. 10 (6), 1259
- **LOVELY.** *Tetrahedron Letters,* 2003, vol. 44, 1379
- **KROEHNKE, F.** *Synthesis,* 1976, vol. 1
- Pyridine and Its Derivatives. The Chemistry of Heterocyclic Compounds. John Wiley & Sons, 1974, vol. 14
- Comprehensive Heterocyclic Chemistry. Pergamon Press, 1984, vol. 2, 165-524
- Comprehensive Heterocyclic Chemistry. Pergamon Press, 1996, vol. 5, 1-300
- **ISHIYAMA, T. et al.** *J. Org. Chem.,* 1995, vol. 60 (23), 7508-7510
- **MURATA et al.** *J. Org. Chem.,* 1997, vol. 62 (19), 6458-6459
- **MIYAURA, N. et al.** *Chem. Rev.,* 1995, vol. 95, 2457
- **TSUJI, J.** Transition Metal Reagents and Catalysts: Innovations in Organic Synthesis. John Wiley & Sons, 2000
- **TSUJI, J.** Palladium Reagents and Catalysts: Innovations in Organic Synthesis. John Wiley & Sons, 1996
- **CONTOUR-GALCERA et al.** *Bioorg. Med. Chem. Lett.,* 2001, vol. 11 (5), 741-745
- **GOIKHMAN, R. ; JACQUES, T.L. ; SAMES, D.** *J. Am. Chem. Soc.,* 2009, vol. 131, 3042